# EUROPEAN PATENT APPLICATION

(11) **EP 4 700 478 A1**
(43) Date of publication of application: **25.02.2026**
(21) Application number: 25196346.8
(22) Date of filing: 18.08.2025
(51) Int. Cl.: G03F 7/004, G03F 7/039, C07D 347/00, C07C 65/24, C07C 309/65

(54) **IODONIUM SALT, CHEMICALLY AMPLIFIED POSITIVE RESIST COMPOSITION AND RESIST PATTERN FORMING PROCESS**

(30) Priority: 20.08.2024 JP 2024139110
(71) Applicant: Shin-Etsu Chemical Co., Ltd., Chiyoda-ku Tokyo 100-0005 (JP)
(72) Inventor: FUKUSHIMA, Masahiro, Joetsu-shi (JP)
(74) Representative: Ter Meer Steinmeister & Partner

(57) **Abstract**

A betaine type iodonium salt containing a C6-C18 alkyl or C4-C18 fluorinated alkyl group and having a iodonium cation and a carboxylate anion within a common molecule is a useful quencher. A chemically amplified positive resist composition comprising the iodonium salt exhibits improved lithography properties such as resolution, LER and pattern profile when processed by photolithography.

## Description

### TECHNICAL FIELD

This invention relates to a iodonium salt, a chemically amplified positive resist composition, and a resist pattern forming process.

### BACKGROUND ART

Pattern formation to a smaller feature size is required to meet the recent demand for higher integration in integrated circuits. Acid-catalyzed chemically amplified resist compositions are most often used in forming resist patterns with a feature size of 0.2 µm or less. For exposure of these resist compositions, high-energy radiation such as UV, deep-UV or EB is used as the energy source. In particular, the EB lithography, which is utilized as the ultra-fine microfabrication technique, is also indispensable in processing a photomask blank into a photomask for use in the fabrication of semiconductor devices. The resist compositions for use in the photolithography include positive ones where exposed regions are dissolved to form patterns and negative ones where exposed regions are retained to form patterns. Of these, either one which is easy to use depending on the desired shape of resist pattern is selected.

In general, the EB lithography is by writing an image with EB, without using a mask. In the case of positive resist, those regions of a resist film other than the regions to be retained are successively irradiated with EB having a minute area. In the case of negative resist, those regions of a resist film to be retained are successively irradiated with EB. The operation of successively scanning all finely divided regions on the work surface takes a long time as compared with full wafer exposure through a photomask. To prevent any throughput decline, a resist film having a high sensitivity is required. Because of the long image-writing time, there is a likelihood of a difference arising between the initially written portion and the later written portion. Thus the stability with time of exposed regions in vacuum is one of important performance requirements. One of the important applications of chemically amplified resist material resides in processing of photomask blanks. Some photomask blanks have a surface material that can have an impact on the pattern profile of the overlying chemically amplified resist film, for example, a layer of a chromium compound, typically chromium oxide deposited on a photomask substrate. For high resolution and profile retention after etching, it is one important performance factor to maintain the profile of a resist film pattern rectangular independent of the type of substrate.

Attempts were made to ameliorate resist sensitivity and pattern profile in a controlled way by properly selecting and combining components used in resist compositions and adjusting processing conditions. One outstanding problem is the diffusion of acid, which has a significant impact on the resolution of a chemically amplified resist film. In the processing of photomasks, it is required that the profile of the resist pattern resulting from exposure does not change depending on the time taken until PEB. The major cause for time-dependent changes is the diffusion of acid generated upon exposure. Since the problem of acid diffusion has large impacts on sensitivity and resolution not only in the photomask processing, but also in general resist compositions, many studies are made thereon.

Patent Documents 1 and 2 describe acid generators capable of generating bulky acids upon exposure, for thereby controlling acid diffusion and reducing roughness. Since these acid generators are still insufficient to control acid diffusion, it is desired to have an acid generator with more controlled diffusion.

Patent Documents 3 to 6 describe that a sulfonic acid to be generated upon exposure is bound to a polymer for use in a resist composition for controlling acid diffusion. This approach of controlling acid diffusion by introducing repeat units capable of generating acid upon exposure into a base polymer is effective in forming a pattern with reduced LER. However, the base polymer having bound therein repeat units capable of generating acid upon exposure encounters a problem with respect to its solubility in organic solvent, depending on the structure and proportion of the relevant units.

Polymers comprising a major proportion of aromatic structure having an acidic side chain, for example, polyhydroxystyrene, are useful in resist materials for the KrF lithography. Since the aromatic ring in the base polymer exhibits substantial absorption to light around wavelength 200 nm, such polymers are not used as the base polymer in resist materials for the ArF lithography. These polymers, however, are expected to form useful resist materials for the EB and EUV lithography for forming patterns of smaller size than the processing limit of ArF lithography because they offer high etching resistance.

Positive resist compositions for EB and EUV lithography use as the base polymer a polymer having an acidic functional group on phenol side chain masked with an acid labile group (or acid-decomposable protective group). Upon exposure to high-energy radiation, the acid labile group is deprotected by the catalysis of an acid generated from a photoacid generator so that the polymer may turn soluble in alkaline developer. Typical of the acid labile group are tertiary alkyl, tert-butoxycarbonyl and acetal groups. When acetal groups are used as the acid labile group requiring a relatively low level of activation energy for deprotection, a resist film having a high sensitivity is advantageously obtained. However, if the control of diffusion of generated acid is insufficient, deprotection reaction can occur even in the regions of resist film which are not exposed. This raises such problems as degradation of LER and a lowering of CDU of pattern line width.

Under the current demand for further pattern miniaturization, it becomes more important to control the diffusion of acid generated by a photoacid generator. Accordingly, a variety of acid diffusion inhibitors or quenchers have been proposed. As the quencher, quenchers of onium salt type such as sulfonium or iodonium salts are proposed in addition to the conventional amine compounds. Patent Document 7 describes a carboxylic acid onium salt having a nitrogen-containing heterocyclic structure, typically indole. Also, Patent Documents 8 to 10 disclose quenchers derived from onium salts of betaine type having a sulfonium or iodonium cation and a phenoxide or carboxylate anion within a common molecule. It is confirmed that using these quenchers, lithographic performance is improved to some extent. However, the results are still unsatisfactory. To meet the demand for further miniaturization, it is desired to develop a resist composition capable of forming patterns of satisfactory profile having improved lithography properties.

### [Citation List]

| | |
|---|---|
| Patent Document 1: | JP-A 2009-053518 |
| Patent Document 2: | JP-A 2010-100604 |
| Patent Document 3: | JP 4425776 |
| Patent Document 4: | JP 5201363 |
| Patent Document 5: | JP 5231357 |
| Patent Document 6: | WO 2008/081832 |
| Patent Document 7: | JP 6515831 |
| Patent Document 8: | JP 6848776 |
| Patent Document 9: | JP 6583136 |
| Patent Document 10: | JP 6246480 |

### SUMMARY OF THE INVENTION

Resist compositions are recently demanded which are capable of forming not only line-and-space (LS), isolated line (IL) and isolated space (IS) patterns of satisfactory profile, but also hole patterns of satisfactory profile.

An object of the invention is to provide is a iodonium salt having satisfactory solvent solubility and being useful as a quencher, a chemically amplified positive resist composition comprising the iodonium salt which exhibits improved lithography properties such as resolution, LER and pattern profile when processed by photolithography using high-energy radiation such as KrF or ArF excimer laser, EB or EUV, and a resist pattern forming process using the composition.

The inventor has found that a iodonium salt of betaine type containing a C₆-C₁₈ chainlike alkyl or C₄-C₁₈ chainlike fluorinated alkyl group and having a iodonium cation and a carboxylate anion within a common molecule has satisfactory solvent solubility. A chemically amplified positive resist composition comprising the iodonium salt exhibits improved lithography properties such as resolution, LER and pattern profile particularly when processed by the EB or EUV lithography.

In one aspect, the invention provides a iodonium salt having the formula (A).

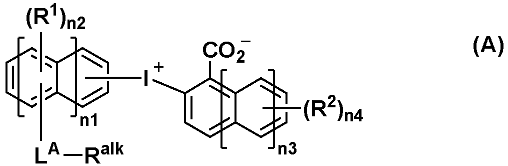

Herein n1 is 0 or 1, n2 is 0, 1, 2, 3 or 4, n3 is 0 or 1, n4 is 0, 1, 2, 3 or 4,
L^{A} is a single bond, ether bond, ester bond, sulfonic ester bond, carbonate bond or carbamate bond,
R^{alk} is a C₆-C₁₈ alkyl group or C₄-C₁₈ fluorinated alkyl group; when R^{alk} is a C₆-C₁₈ alkyl group, the alkyl group has at least one straight chain structure of 6 or more carbon atoms; when R^{alk} is a C₄-C₁₈ fluorinated alkyl group, the fluorinated alkyl group has at least two groups selected from -CF₂- and -CF₃; the alkyl or fluorinated alkyl group may have some -CH₂- replaced by an ether bond or carbonyl group and may contain a cyclic structure selected from cyclopentane, cyclohexane, adamantane, norbornyl and benzene rings at its end or in a carbon-carbon bond,
R¹ is halogen, nitro, cyano, hydroxy, a C₁-C₂₀ hydrocarbyl group which may contain a heteroatom, C₁-C₂₀ hydrocarbyloxy group which may contain a heteroatom, or C₁-C₂₀ hydrocarbylthio group which may contain a heteroatom; when n2 is 2, 3 or 4, a plurality of R¹ may be identical or different and may bond together to form a ring with the carbon atoms to which they are attached,
R² is halogen, nitro, cyano, hydroxy, a C₁-C₂₀ hydrocarbyl group which may contain a heteroatom, C₁-C₂₀ hydrocarbyloxy group which may contain a heteroatom, or C₁-C₂₀ hydrocarbylthio group which may contain a heteroatom; when n4 is 2, 3 or 4, a plurality of R² may be identical or different and may bond together to form a ring with the carbon atoms to which they are attached,
two aromatic rings attached to I⁺ in the formula may bond together to form a ring with the iodine atom to which they are attached.

The preferred iodonium salt has the formula (A1): 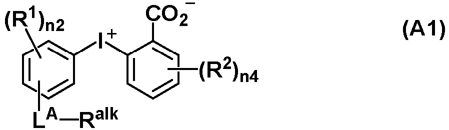 wherein n2, n4, L^{A}, R^{alk}, R¹ and R² are as defined above.

In another aspect, the invention provides a quencher in the form of the iodonium salt defined herein.

In a further aspect, the invention provides a chemically amplified positive resist composition comprising the quencher defined herein.

In a preferred embodiment, the resist composition further comprises a base polymer containing a polymer adapted to be decomposed under the action of acid to increase its solubility in alkaline developer.

The polymer may comprise repeat units having the formula (B 1).

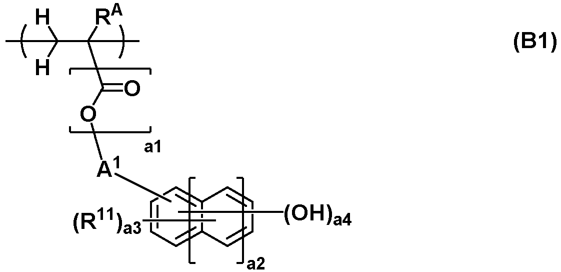

Herein a1 is 0 or 1, a2 is 0, 1 or 2, a3 is an integer meeting 0 ≤ a3 ≤ 5+2(a2)-a4, a4 is 1, 2 or 3,
R^{A} is hydrogen, fluorine, methyl or trifluoromethyl,
R¹¹ is halogen, nitro group, carboxy group, an optionally halogenated C₁-C₆ saturated hydrocarbyl group, optionally halogenated C₁-C₆ saturated hydrocarbyloxy group, or optionally halogenated C₂-C₈ saturated hydrocarbylcarbonyloxy group, and
A¹ is a single bond or C₁-C₁₀ saturated hydrocarbylene group in which some -CH₂-may be replaced by -O-.

The polymer may comprise repeat units having the formula (B2-1).

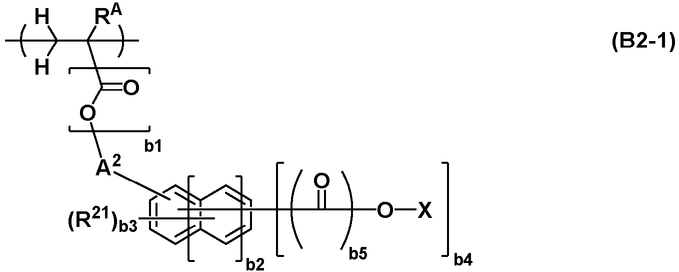

Herein R^{A} is hydrogen, fluorine, methyl or trifluoromethyl,
b1 is 0 or 1, b2 is 0, 1 or 2, b3 is an integer meeting 0 ≤ b3 ≤ 5+2(b2)-b4, b4 is 1, 2 or 3, b5 is 0 or 1,
R²¹ is halogen, an optionally halogenated C₁-C₆ saturated hydrocarbyl group, optionally halogenated C₁-C₆ saturated hydrocarbyloxy group, or optionally halogenated C₂-C₈ saturated hydrocarbylcarbonyloxy group,
A² is a single bond or C₁-C₁₀ saturated hydrocarbylene group in which some -CH₂-may be replaced by -O-,
X is an acid labile group when b4=1, and X is hydrogen or an acid labile group, at least one being an acid labile group, when b4=2 or 3.

The polymer may comprise repeat units having the formula (B2-2).

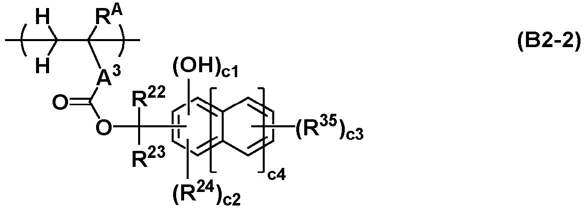

Herein c1 is 0, 1 or 2, c2 is 0, 1 or 2, c3 is 0, 1, 2, 3, 4 or 5, c4 is 0, 1 or 2,
R^{A} is hydrogen, fluorine, methyl or trifluoromethyl,
R²² and R²³ are each independently a C₁-C₁₀ hydrocarbyl group which may contain a heteroatom, R²² and R²³ may bond together to form a ring with the carbon atom to which they are attached,
R²⁴ is each independently fluorine, a C₁-C₅ fluorinated alkyl group or C₁-C₅ fluorinated alkoxy group,
R²⁵ is each independently a C₁-C₁₀ hydrocarbyl group which may contain a heteroatom, and
A³ is a single bond, phenylene, naphthylene or *-C(=O)-O-A³¹-, A³¹ is a C₁-C₂₀ aliphatic hydrocarbylene group which may contain a hydroxy moiety, ether bond, ester bond or lactone ring, a phenylene group or a naphthylene group, * designates a point of attachment to the carbon atom in the backbone.

The polymer may comprise repeat units of at least one type selected from repeat units having the formula (B3), repeat units having the formula (B4), and repeat units having the formula (B5).

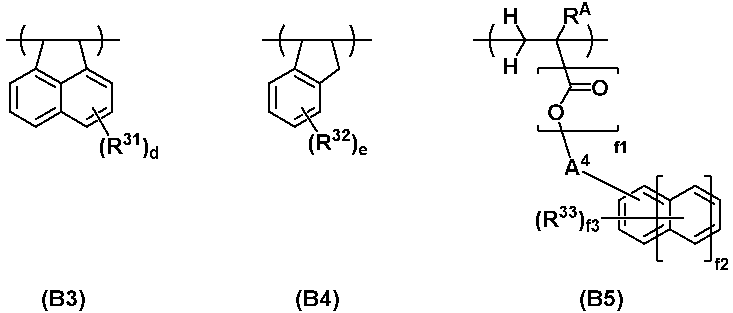

Herein d is 0, 1, 2, 3, 4, 5 or 6, e is 0, 1, 2, 3 or 4, f1 is 0 or 1, f2 is 0, 1 or 2, f3 is 0, 1, 2, 3, 4 or 5,
R^{A} is hydrogen, fluorine, methyl or trifluoromethyl,
R³¹ and R³² are each independently hydroxy, halogen, an optionally halogenated C₁-C₆ saturated hydrocarbyl group, optionally halogenated C₁-C₆ saturated hydrocarbyloxy group, or optionally halogenated C₂-C₈ saturated hydrocarbylcarbonyloxy group,
R³³ is a C₁-C₂₀ saturated hydrocarbyl group, C₁-C₂₀ saturated hydrocarbyloxy group, C₂-C₂₀ saturated hydrocarbylcarbonyloxy group, C₂-C₂₀ saturated hydrocarbyloxyhydrocarbyl group, C₂-C₂₀ saturated hydrocarbylthiohydrocarbyl group, halogen, nitro, or cyano, R³³ may also be hydroxy when f2=1 or 2, and
A⁴ is a single bond or C₁-C₁₀ saturated hydrocarbylene group in which some -CH₂-may be replaced by -O-.

The polymer may comprise repeat units of at least one type selected from repeat units having the formula (B6), repeat units having the formula (B7), repeat units having the formula (B8), repeat units having the formula (B9), and repeat units having the formula (B10).

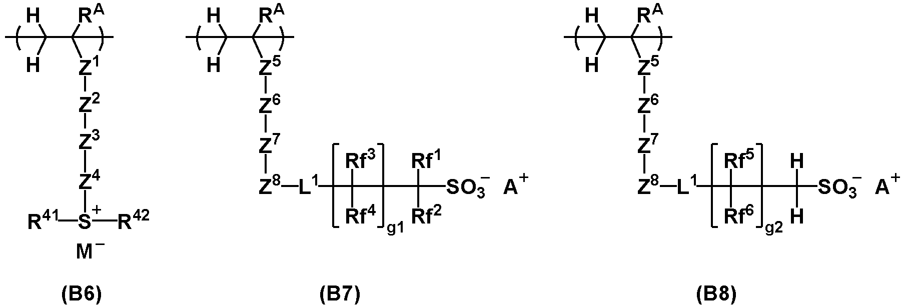

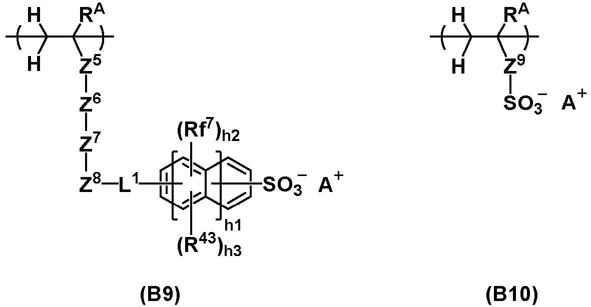

Herein g1 and g2 are each independently 0, 1, 2 or 3, h1 is 0 or 1, h2 is 0, 1, 2, 3 or 4, h3 is 0, 1, 2, 3 or 4, with the proviso that when h1=0, 0 ≤ h2+h3 ≤ 4, and when h1=1, 0 ≤ h2+h3 ≤ 6,
R^{A} is hydrogen, fluorine, methyl or trifluoromethyl,
Z¹ is a single bond or an optionally substituted phenylene group,
Z² is a single bond, **-C(=O)-O-Z²¹-, **-C(=O)-NH-Z²¹-, or **-O-Z²¹-, Z²¹ is a C₁-C₆ aliphatic hydrocarbylene group, phenylene group or a divalent group obtained by combining the foregoing, which may contain halogen, carbonyl moiety, ester bond, ether bond or hydroxy moiety,
Z³ is a single bond, ether bond, ester bond, amide bond, sulfonate ester bond, sulfonamide bond, carbonate bond or carbamate bond,
Z⁴ is a single bond, or a C₁-C₆ aliphatic hydrocarbylene group, phenylene group or a divalent group obtained by combining the foregoing, which may contain halogen, carbonyl moiety, ester bond, ether bond or hydroxy moiety,
Z⁵ is each independently a single bond, an optionally substituted phenylene, naphthylene, or *-C(=O)-O-Z⁵¹-, Z⁵¹ is a C₁-C₁₀ aliphatic hydrocarbylene group which may contain halogen, hydroxy moiety, ether bond, ester bond or lactone ring, or phenylene or naphthylene group,
Z⁶ is a single bond, ether bond, ester bond, amide bond, sulfonate ester bond, sulfonamide bond, carbonate bond or carbamate bond,
Z⁷ is each independently a single bond, ***-Z⁷¹-C(=O)-O-, ***-C(=O)-NH-Z⁷¹-, or ***-O-Z⁷¹-, Z⁷¹ is a C₁-C₂₀ hydrocarbylene group which may contain a heteroatom,
Z⁸ is each independently a single bond, ****-Z⁸¹-C(=O)-O-, ****-C(=O)-NH-Z⁸¹-, or ****-O-Z⁸¹-, Z⁸¹ is a C₁-C₂₀ hydrocarbylene group which may contain a heteroatom,
Z⁹ is a single bond, methylene, ethylene, phenylene, fluorinated phenylene, trifluoromethyl-substituted phenylene, *-C(=O)-O-Z⁹¹-, *-C(=O)-N(H)-Z⁹¹-, or *-O-Z⁹¹-, Z⁹¹ is a C₁-C₆ aliphatic hydrocarbylene group, phenylene group, fluorinated phenylene group or trifluoromethyl-substituted phenylene group, which may contain a carbonyl moiety, ester bond, ether bond or hydroxy moiety,
* is a point of attachment to the carbon atom in the backbone, ** is a point of attachment to Z¹, *** is a point of attachment to Z⁶, **** is a point of attachment to Z⁷,
L¹ is a single bond, ether bond, ester bond, carbonyl group, sulfonate ester bond, sulfonamide bond, carbonate bond or carbamate bond,
Rf¹ and Rf² are each independently fluorine or a C₁-C₆ fluorinated saturated hydrocarbyl group,
Rf³ and Rf⁴ are each independently hydrogen, fluorine, or a C₁-C₆ fluorinated saturated hydrocarbyl group,
Rf⁵ and Rf⁶ are each independently hydrogen, fluorine, or a C₁-C₆ fluorinated saturated hydrocarbyl group, excluding that all Rf⁵ and Rf⁶ are hydrogen at the same time,
Rf⁷ is fluorine, a C₁-C₆ fluorinated alkyl group, C₁-C₆ fluorinated alkoxy group, or C₁-C₆ fluorinated alkylthio group,
R⁴¹ and R⁴² are each independently a C₁-C₂₀ hydrocarbyl group which may contain a heteroatom, R⁴¹ and R⁴² may bond together to form a ring with the sulfur atom to which they are attached,
R⁴³ is halogen exclusive of fluorine, or a C₁-C₂₀ hydrocarbyl group which may contain a heteroatom; when h3 is 2, 3 or 4, a plurality of R⁴³ may be identical or different and may bond together to form a ring with the carbon atoms to which they are attached,
M⁻ is a non-nucleophilic counter ion, and
A⁺ is an onium cation.

In a preferred embodiment, repeat units having an aromatic ring structure account for at least 60 mol% of the overall repeat units of the polymer in the base polymer.

Typically, the resist composition further comprises (C) an organic solvent and/or (D) a photoacid generator.

The resist composition may further comprise (E) a fluorinated polymer comprising repeat units of at least one type selected from repeat units having the formula (E1), repeat units having the formula (E2), repeat units having the formula (E3) and repeat units having the formula (E4) and optionally repeat units of at least one type selected from repeat units having the formula (E5) and repeat units having the formula (E6).

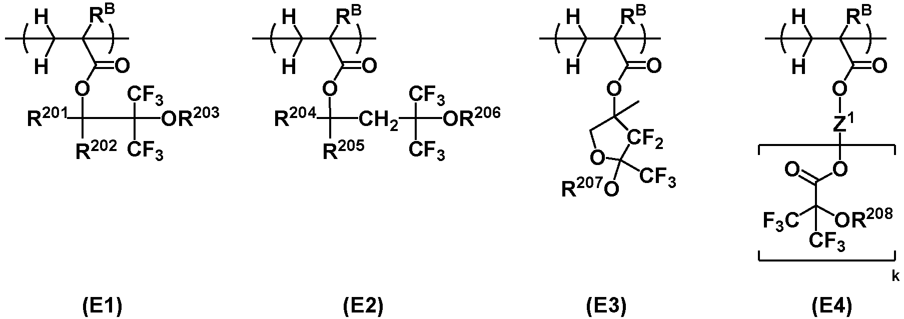

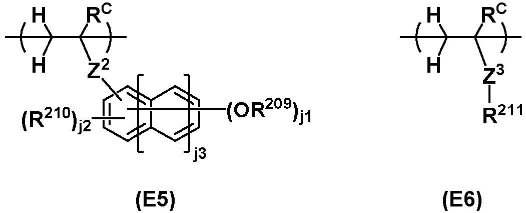

Herein j1 is 1, 2 or 3, j2 is an integer meeting 0 ≤ j2 ≤ 5+2(j3)-j1, j3 is 0 or 1, k is 1, 2 or 3,
R^{B} is each independently hydrogen, fluorine, methyl or trifluoromethyl,
R^{C} is each independently hydrogen or methyl,
R²⁰¹, R²⁰², R²⁰⁴ and R²⁰⁵ are each independently hydrogen or a C₁-C₁₀ saturated hydrocarbyl group,
R²⁰³, R²⁰⁶, R²⁰⁷ and R²⁰⁸ are each independently hydrogen, a C₁-C₁₅ hydrocarbyl group, C₁-C₁₅ fluorinated hydrocarbyl group, or acid labile group, and when R²⁰³, R²⁰⁶, R²⁰⁷ and R²⁰⁸ each are a hydrocarbyl or fluorinated hydrocarbyl group, an ether bond or carbonyl moiety may intervene in a carbon-carbon bond,
R²⁰⁹ is hydrogen or a C₁-C₅ straight or branched hydrocarbyl group in which a heteroatom-containing moiety may intervene in a carbon-carbon bond,
R²¹⁰ is a C₁-C₅ straight or branched hydrocarbyl group in which a heteroatom-containing moiety may intervene in a carbon-carbon bond,
R²¹¹ is a C₁-C₂₀ saturated hydrocarbyl group in which at least one hydrogen is substituted by fluorine, and in which some constituent -CH₂- may be replaced by an ester bond or ether bond,
Z¹ is a C₁-C₂₀ (k+1)-valent hydrocarbon group or C₁-C₂₀ (k+1)-valent fluorinated hydrocarbon group,
Z² is a single bond, *-C(=O)-O- or *-C(=O)-NH-, * designates a point of attachment to the carbon atom in the backbone,
Z³ is a single bond, -O-, *-C(=O)=O-Z³¹-Z³²- or *-C(=O)-NH-Z³¹-Z³²-, Z³¹ is a single bond or C₁-C₁₀ saturated hydrocarbylene group, Z³² is a single bond, ester bond, ether bond, or sulfonamide bond, and * designates a point of attachment to the carbon atom in the backbone.

The resist composition may further comprise a quencher other than the quencher of claim 3.

In a still further aspect, the invention provides a resist pattern forming process comprising the steps of:
applying the chemically amplified positive resist composition defined above onto a substrate to form a resist film thereon,
exposing the resist film patternwise to high-energy radiation, and
developing the exposed resist film in an alkaline developer.

Most often, the high-energy radiation is EUV of wavelength 3 to 15 nm or EB.

In one embodiment, the substrate has the outermost surface of a chromium-containing material. Typically, the substrate is a photomask blank.

### ADVANTAGEOUS EFFECTS OF THE INVENTION

The chemically amplified positive resist composition of the invention exhibits a very high resolution and forms a pattern of satisfactorily rectangular profile with reduced LER when processed by the microfabrication technology, especially EB or EUV lithography.

### DESCRIPTION OF THE PREFERRED EMBODIMENT

As used herein, the singular forms "a," "an" and "the" include plural referents unless the context clearly dictates otherwise. "Optional" or "optionally" means that the subsequently described event or circumstances may or may not occur, and that description includes instances where the event or circumstance occurs and instances where it does not. The notation (Cn-Cm) means a group containing from n to m carbon atoms per group.

In chemical formulae, the broken line (---) and asterisk (*) each designate a point of attachment, namely valence bond. Me stands for methyl and Ac for acetyl. As used herein, the term "halogenated" refers to a halogen-substituted or halogen-containing compound or group. For example, "fluorinated" refers to a fluorine-substituted or fluorine-containing compound or group. The terms "group" and "moiety" are interchangeable.

The abbreviations and acronyms have the following meaning.
- PAG:: photoacid generator
- Mw:: weight average molecular weight
- Mn:: number average molecular weight
- Mw/Mn:: molecular weight distribution or dispersity
- GPC:: gel permeation chromatography
- PEB:: post-exposure baking
- LER:: line edge roughness
- CDU:: critical dimension uniformity

It is understood that for some structures represented by chemical formulae, there can exist enantiomers and diastereomers because of the presence of asymmetric carbon atoms. In such a case, a single formula collectively represents all such isomers. The isomers may be used alone or in admixture.

### [Iodonium salt]

One embodiment of the invention is a iodonium salt having the formula (A).

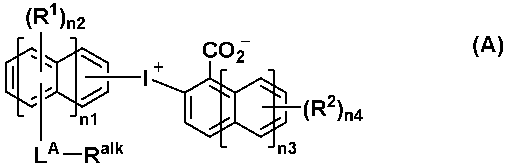

In formula (A), n1 is 0 or 1. The relevant structure is a benzene ring when n1=0, and a naphthalene ring when n1=1. The benzene ring corresponding to n1=0 is preferred from the aspect of solvent solubility. The subscript n2 is 0, 1, 2, 3 or 4. It is preferred from the aspect of reactant availability that n2 be 0, 1 or 2. The subscript n3 is 0 or 1. The relevant structure is a benzene ring when n3=0, and a naphthalene ring when n3=1. The benzene ring corresponding to n3=0 is preferred from the aspect of solvent solubility. The subscript n4 is 0, 1, 2, 3 or 4. It is preferred from the aspect of reactant availability that n4 be 0, 1 or 2.

In formula (A), L^{A} is a single bond, ether bond, ester bond, sulfonic ester bond, carbonate bond or carbamate bond. Inter alia, a single bond, ether bond, ester bond or sulfonic ester bond is preferred. An ether bond or ester bond is more preferred.

In formula (A), R^{alk} is a C₆-C₁₈ alkyl group or C₄-C₁₈ fluorinated alkyl group.

When R^{alk} is a C₆-C₁₈ alkyl group, the alkyl group has at least one straight chain structure of 6 or more carbon atoms. Examples of the C₆-C₁₈ alkyl group R^{alk} include 1-hexyl, 1-heptyl, 1-octyl, 1-nonyl, 1-decyl, 1-undecyl, 1-dodecyl, 1-tridecyl, 1-tetradecyl, 1-hexadecyl, 1-octadecyl, octan-2-yl, decan-2-yl, decan-4-yl, octadecan-8-yl, 7,7-dimethyloctyl, 7,7-diethylnonyl and 4-butyldodecyl. In the alkyl group, some -CH₂-may be replaced by an ether bond or carbonyl group. The alkyl group may contain a cyclic structure such as cyclopentane, cyclohexane, adamantane, norbornane or benzene ring at its end or in a carbon-carbon bond. R^{alk} is preferably a straight alkyl group or straight glyme chain.

When R^{alk} is a C₄-C₁₈ fluorinated alkyl group, the fluorinated alkyl group has at least two groups selected from -CF₂- and -CF₃. Examples of the C₄-C₁₈ fluorinated alkyl group R^{alk} include 1-butyl, 1-pentyl, 1-hexyl, 1-heptyl, 1-octyl, 1-nonyl, 1-decyl, 1-undecyl, 1-dodecyl, 1-tridecyl, 1-tetradecyl, 1-hexadecyl, 1-octadecyl, octan-2-yl, decan-2-yl, decan-4-yl, octadecan-8-yl, 7,7-dimethyloctyl, 7,7-diethylnonyl, and 4-butyldodecyl in which some or all of the hydrogen atoms are substituted by fluorine. In the fluorinated alkyl group, some -CH₂- may be replaced by an ether bond or carbonyl group. The fluorinated alkyl group may contain a cyclic structure such as cyclopentane, cyclohexane, adamantane, norbornyl or benzene ring at its end or in a carbon-carbon bond.

Preferred examples of R^{alk} are shown below, but not limited thereto.

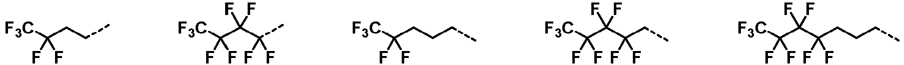

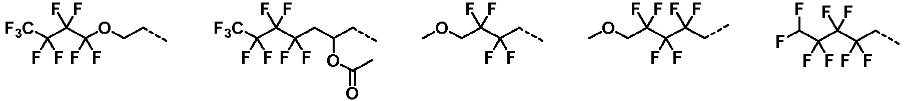

**In** formula (A), R¹ is halogen, nitro, cyano, hydroxy, a C₁-C₂₀ hydrocarbyl group which may contain a heteroatom, C₁-C₂₀ hydrocarbyloxy group which may contain a heteroatom, or C₁-C₂₀ hydrocarbylthio group which may contain a heteroatom. Suitable halogen atoms include fluorine, chlorine, bromine and iodine, with fluorine and iodine being preferred. The hydrocarbyl group may be saturated or unsaturated and straight, branched or cyclic. Examples thereof include C₁-C₂₀ alkyl groups such as methyl, ethyl, n-propyl, isopropyl, n-butyl, isobutyl, sec-butyl, tert-butyl, n-pentyl, n-hexyl, n-octyl, n-nonyl, n-decyl, undecyl, dodecyl, tridecyl, tetradecyl, pentadecyl, heptadecyl, octadecyl, nonadecyl, icosyl; C₃-C₂₀ cyclic saturated hydrocarbyl groups such as cyclopropyl, cyclopentyl, cyclohexyl, cyclopropylmethyl, 4-methylcyclohexyl, cyclohexylmethyl, norbornyl, adamantyl; C₂-C₂₀ alkenyl groups such as vinyl, allyl, propenyl, butenyl, hexenyl; C₃-C₂₀ cyclic unsaturated hydrocarbyl groups such as cyclohexenyl; C₆-C₂₀ aryl groups such as phenyl and naphthyl; C₇-C₂₀ aralkyl groups such as benzyl, 1-phenylethyl, 2-phenylethyl, and combinations thereof. Inter alia, aryl groups are preferred. In the hydrocarbyl groups, some or all hydrogen may be substituted by a moiety containing a heteroatom such as oxygen, sulfur, nitrogen or halogen, and some -CH₂- may be replaced by a moiety containing a heteroatom such as oxygen, sulfur or nitrogen, so that the group may contain a hydroxy, cyano, fluorine, chlorine, bromine, iodine, carbonyl, ether bond, ester bond, sulfonate ester bond, carbonate bond, lactone ring, sultone ring, carboxylic anhydride (-C(=O)-O-C(=O)-) or haloalkyl moiety. When n2 is 2, 3 or 4, a plurality of R¹ may be identical or different.

When n2 is 2, 3 or 4, a plurality of R¹ may bond together to form a ring with the carbon atoms to which they are attached. Examples of the ring include cyclopropane, cyclobutene, cyclopentane, cyclohexane, norbornane and adamantane rings. In the ring, some or all hydrogen may be substituted by a moiety containing a heteroatom such as oxygen, sulfur, nitrogen or halogen, and some -CH₂- may be replaced by a moiety containing a heteroatom such as oxygen, sulfur or nitrogen, so that the group may contain a hydroxy, fluorine, chlorine, bromine, iodine, cyano, carbonyl, ether bond, ester bond, sulfonate ester bond, carbonate bond, lactone ring, sultone ring, carboxylic anhydride (-C(=O)-O-C(=O)-) or haloalkyl moiety.

In formula (A), R² is halogen, nitro, cyano, hydroxy, a C₁-C₂₀ hydrocarbyl group which may contain a heteroatom, C₁-C₂₀ hydrocarbyloxy group which may contain a heteroatom, or C₁-C₂₀ hydrocarbylthio group which may contain a heteroatom. Suitable halogen atoms include fluorine, chlorine, bromine and iodine, with fluorine and iodine being preferred. The hydrocarbyl group may be saturated or unsaturated and straight, branched or cyclic. Examples thereof are as exemplified above for the hydrocarbyl group R¹, but not limited thereto. When n4 is 2, 3 or 4, a plurality of R² may be identical or different.

When n4 is 2, 3 or 4, a plurality of R² may bond together to form a ring with the carbon atoms to which they are attached. Examples of the ring are as exemplified above for the ring that a plurality of R¹, taken together, form with the carbon atoms to which they are attached, but not limited thereto.

Two aromatic rings attached to I⁺ in formula (A) may bond together to form a ring with the iodine atom to which they are attached. Exemplary structures of the ring are shown below.

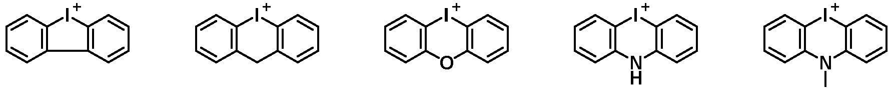

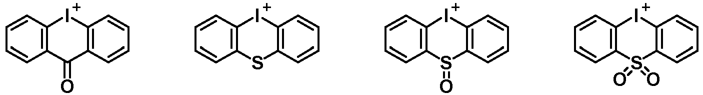

The preferred iodonium salt of formula (A) has the formula (A1).

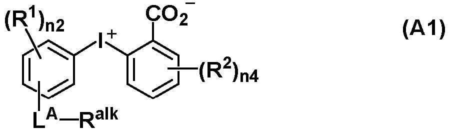

Herein n2, n4, L^{A}, R^{alk}, R¹ and R² are as defined above.

Preferred examples of the iodonium salt having formula (A) are shown below, but not limited thereto.

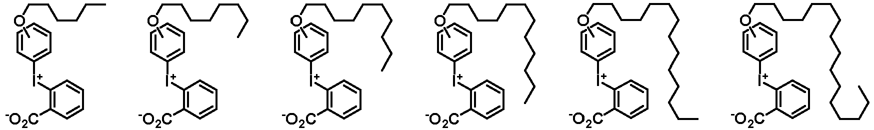

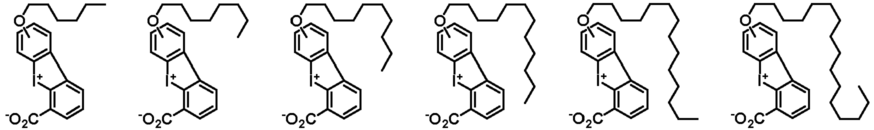

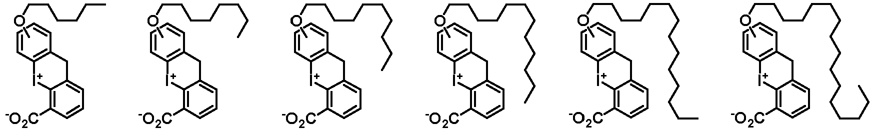

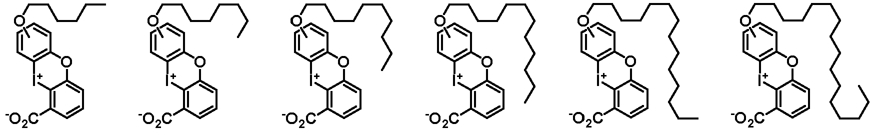

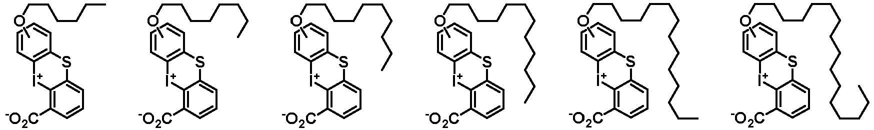

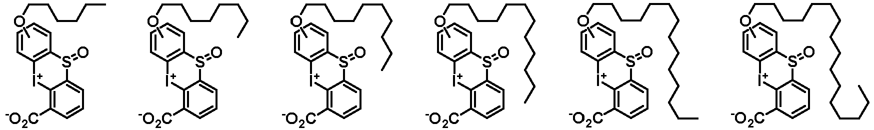

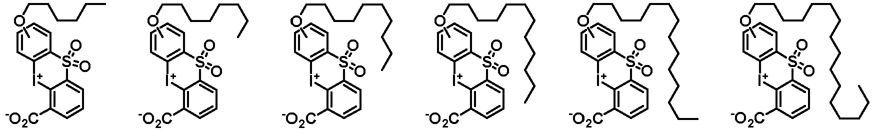

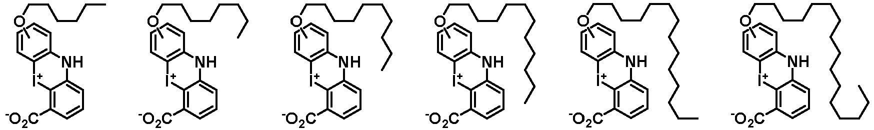

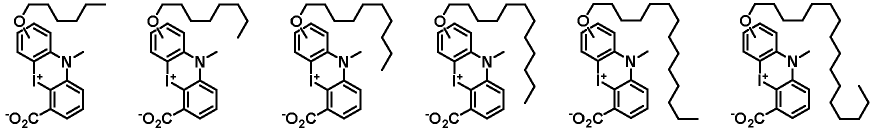

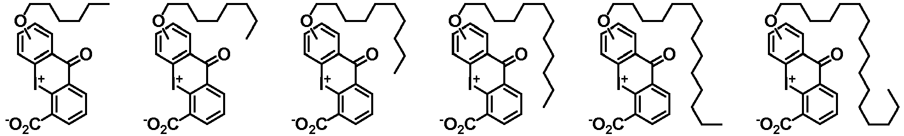

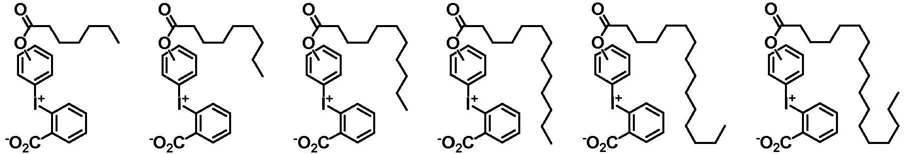

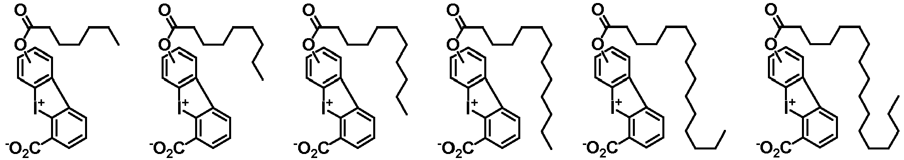

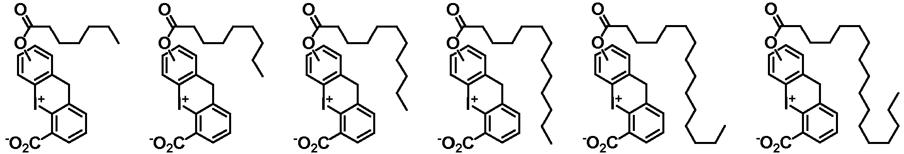

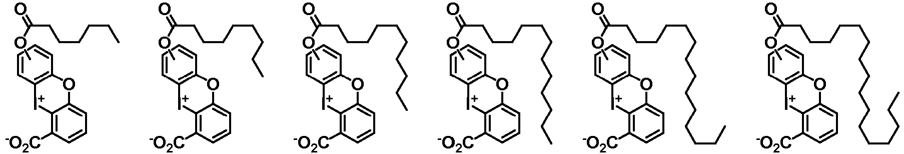

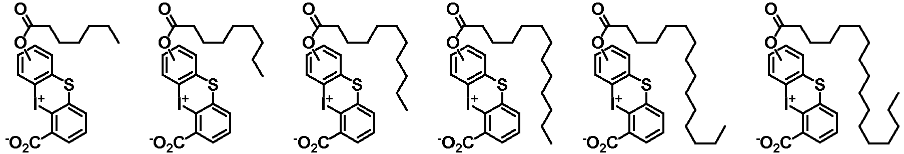

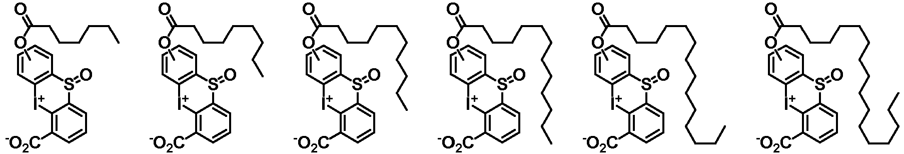

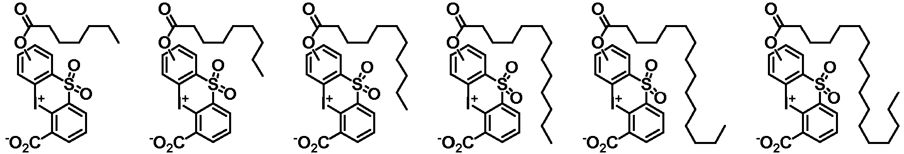

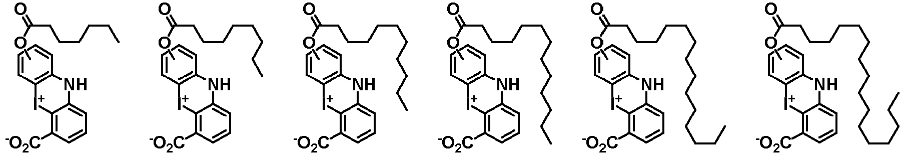

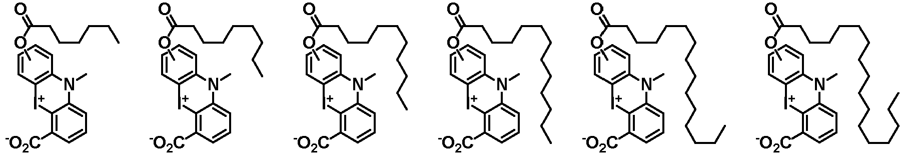

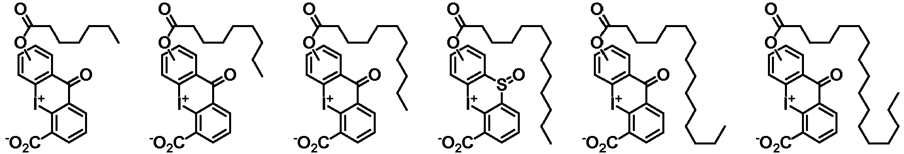

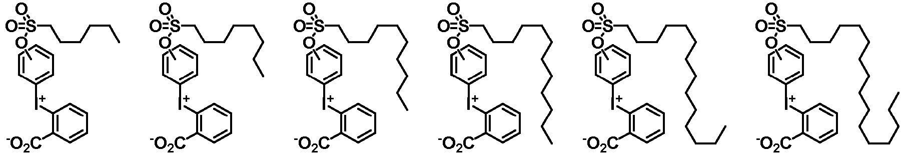

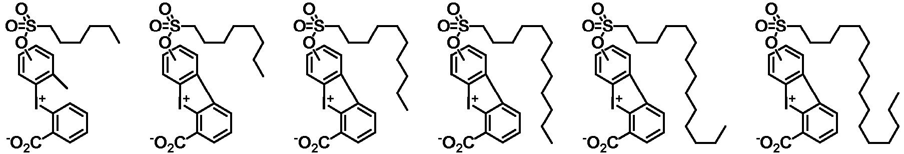

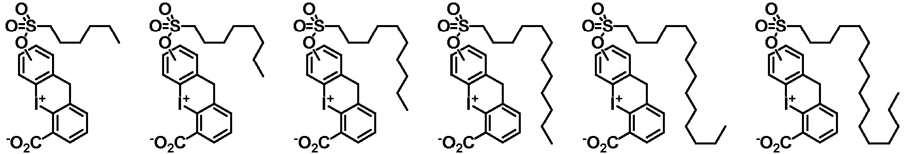

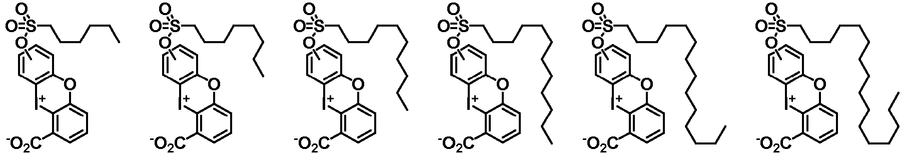

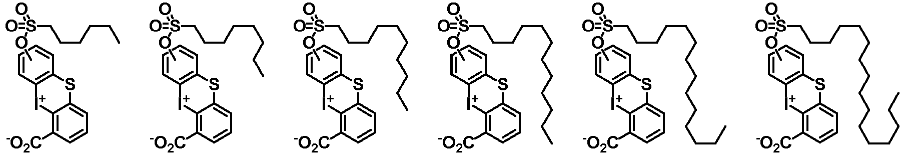

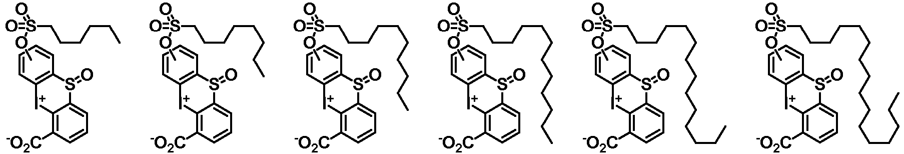

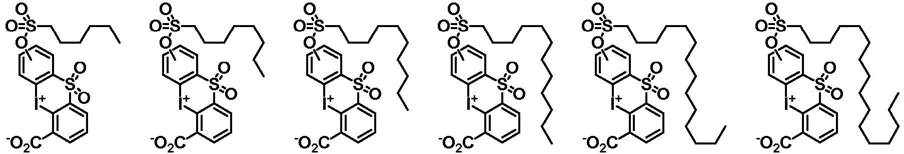

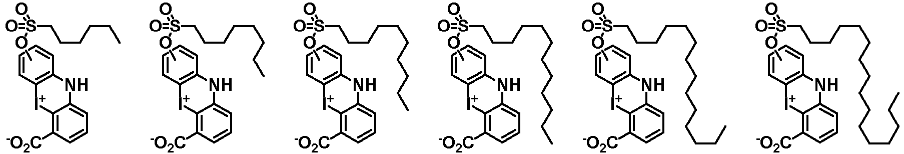

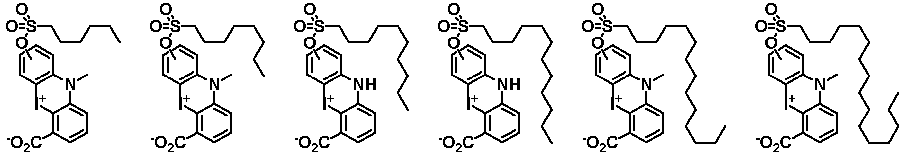

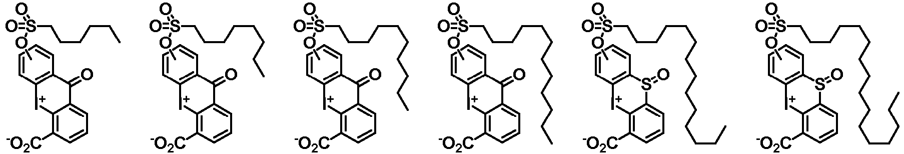

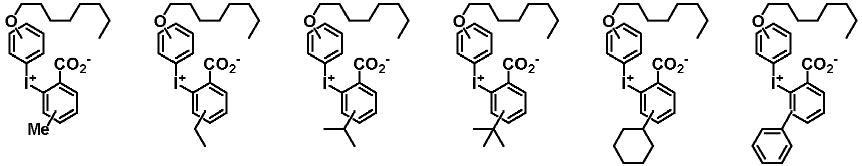

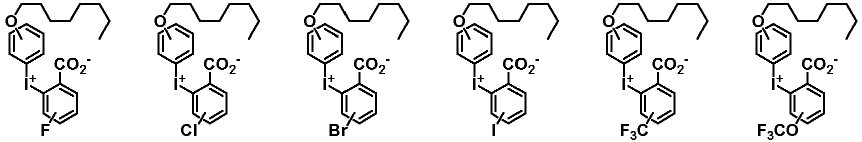

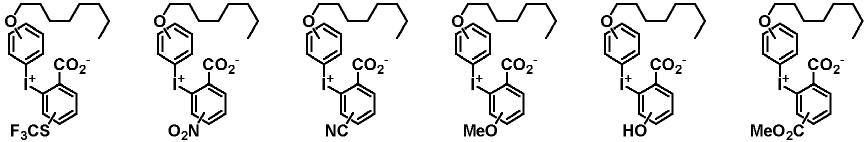

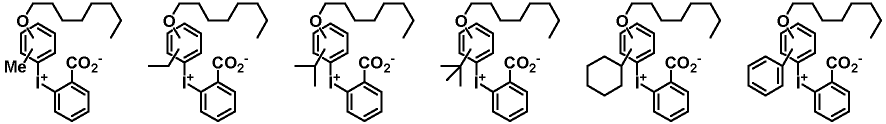

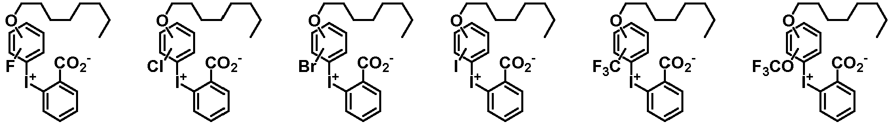

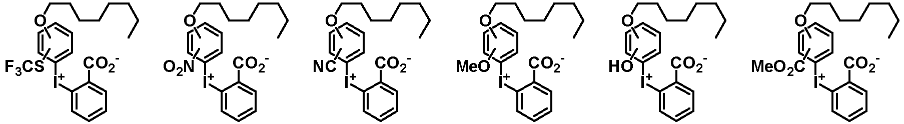

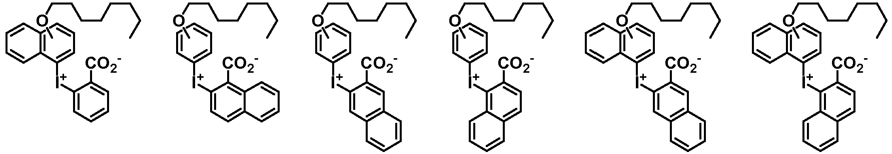

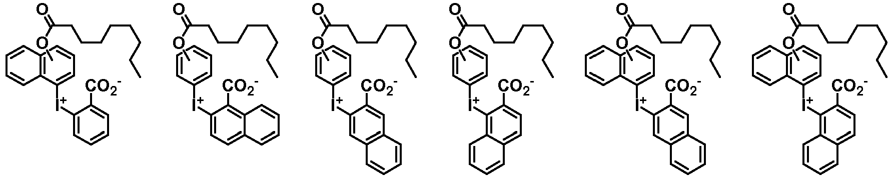

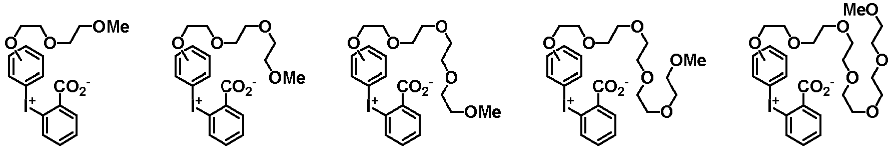

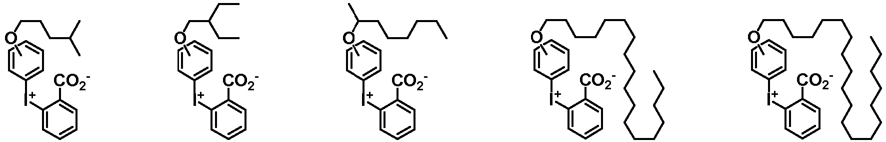

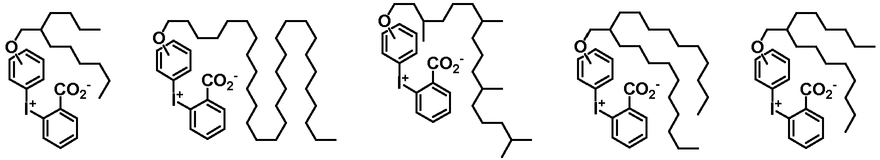

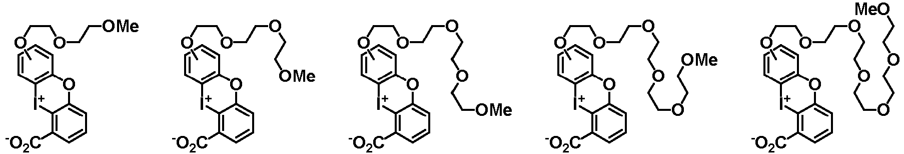

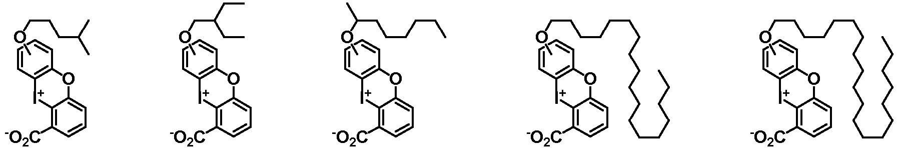

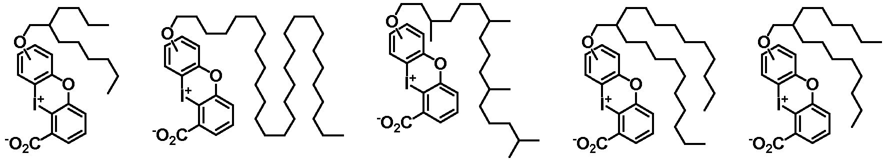

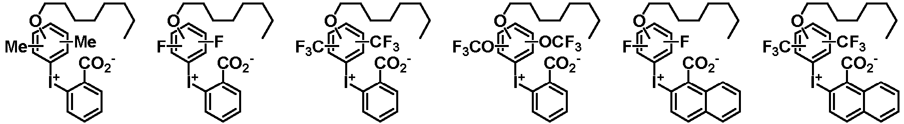

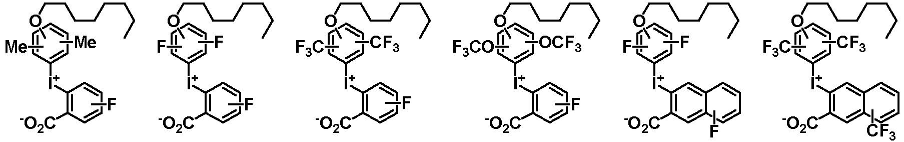

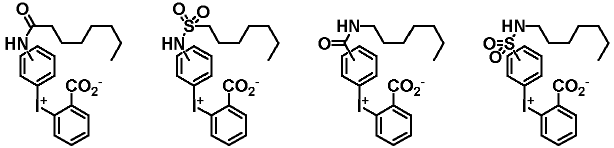

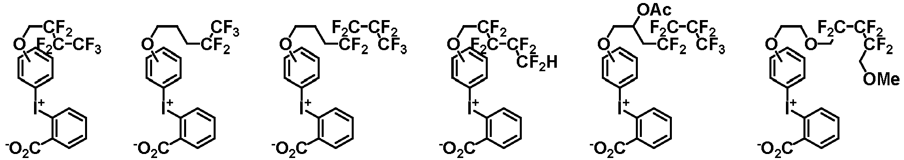

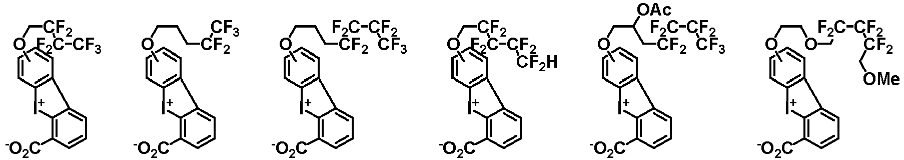

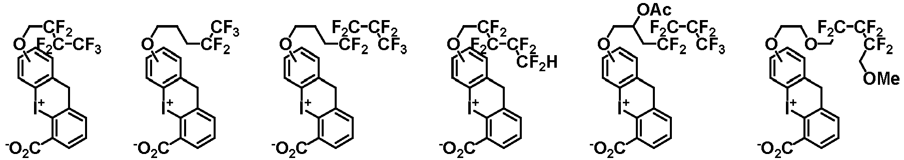

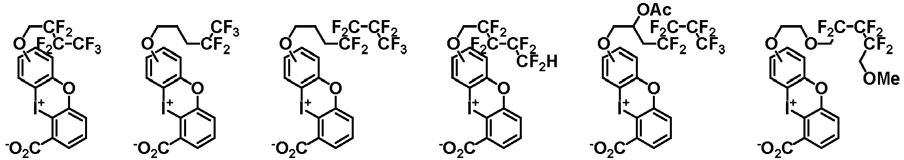

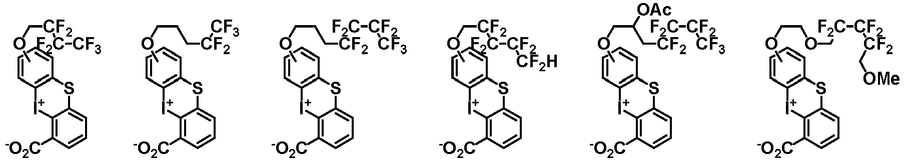

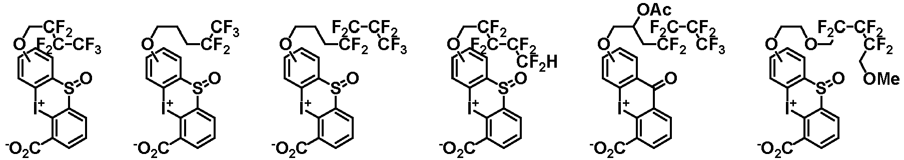

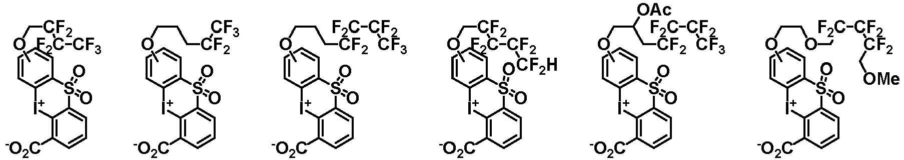

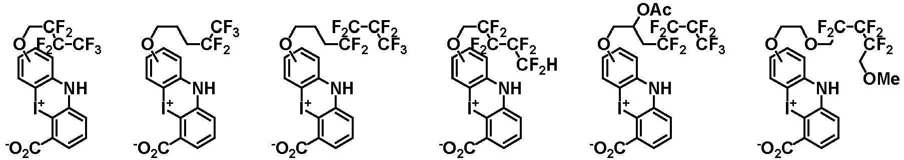

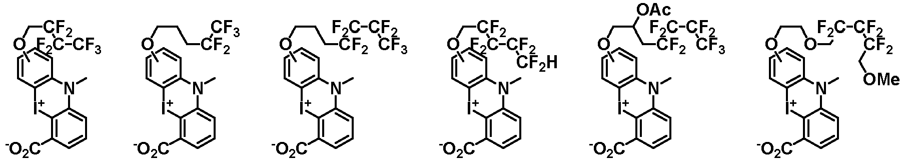

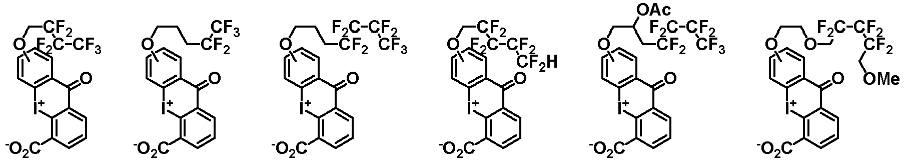

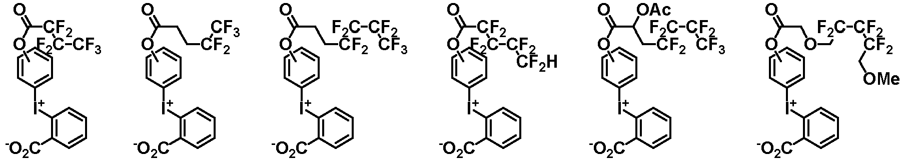

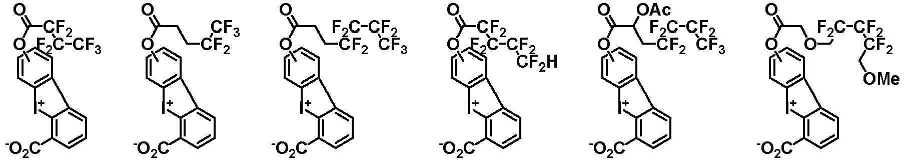

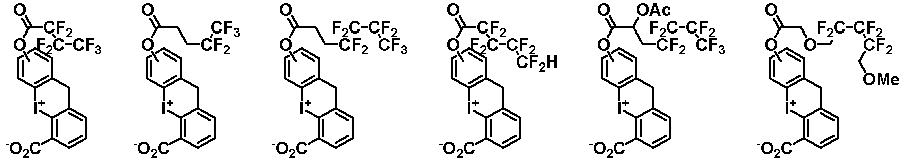

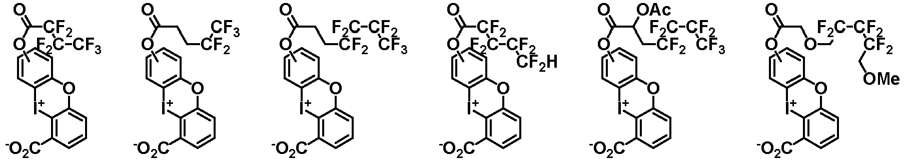

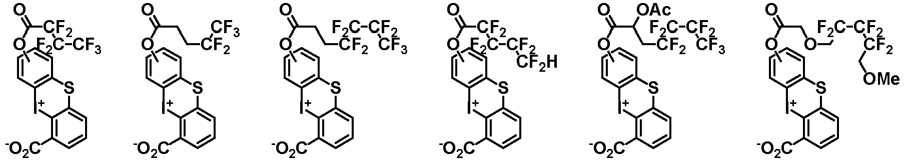

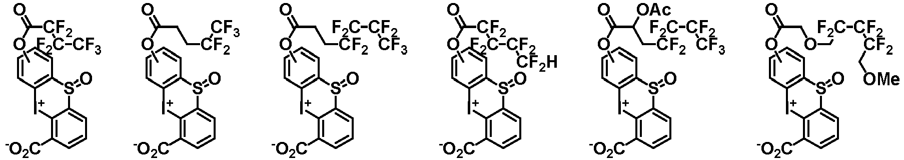

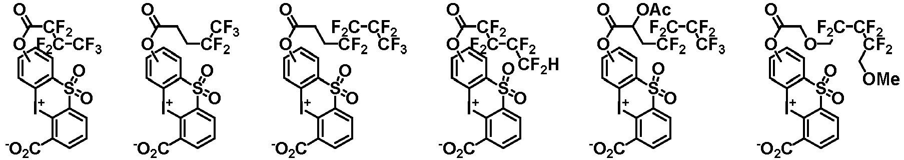

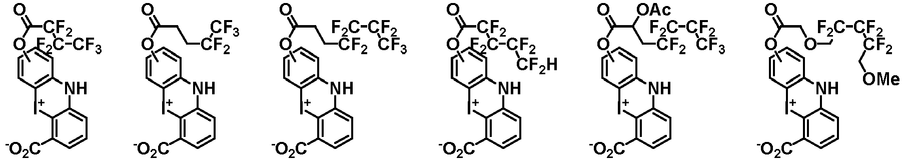

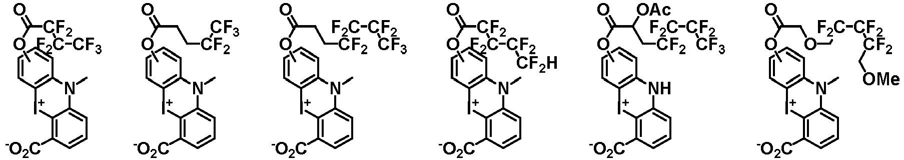

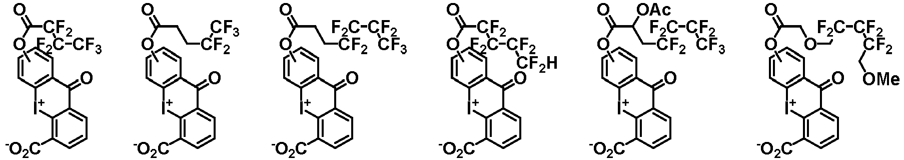

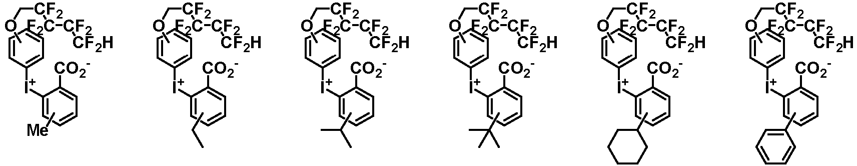

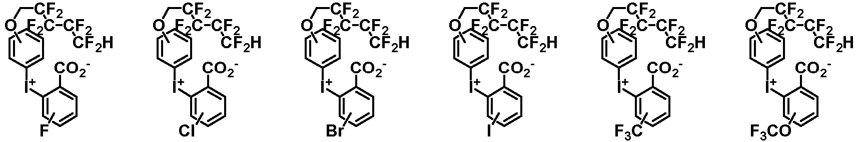

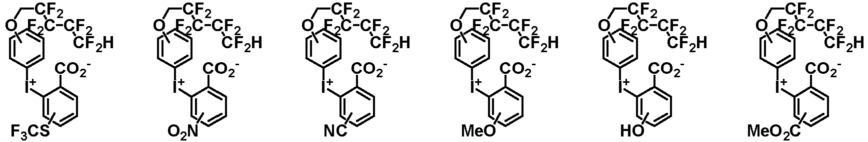

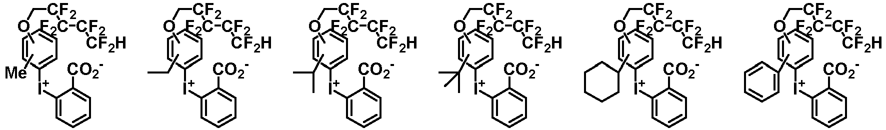

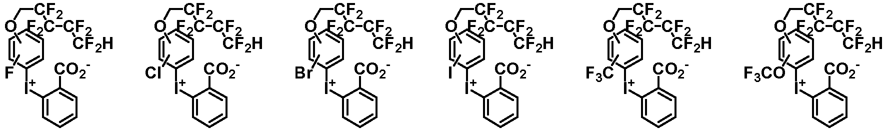

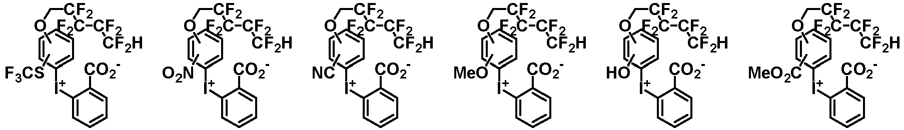

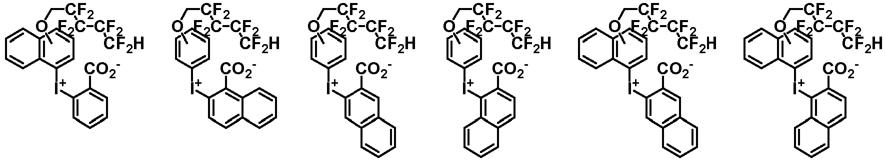

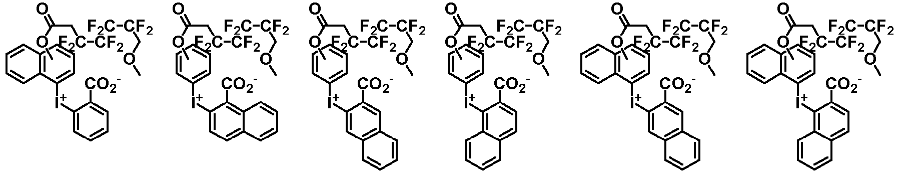

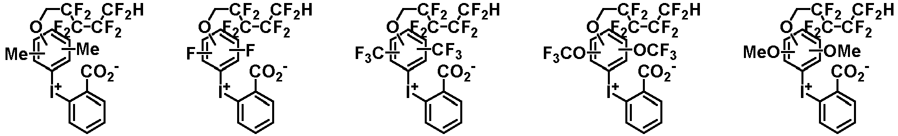

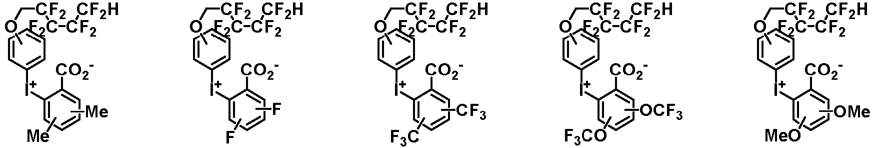

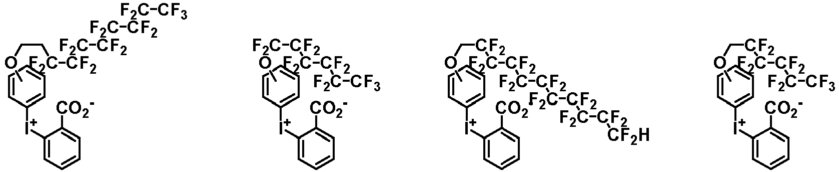

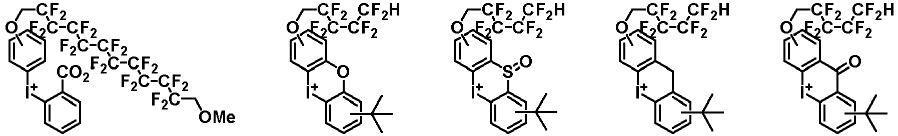

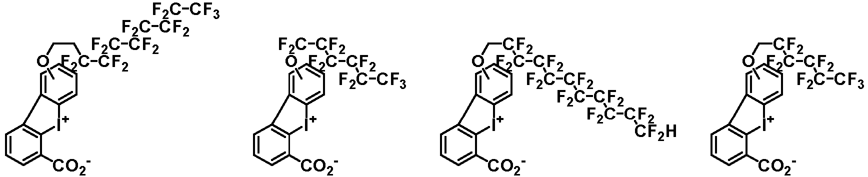

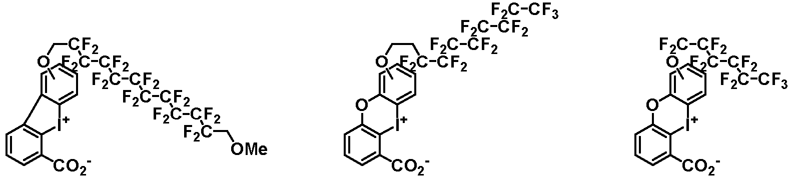

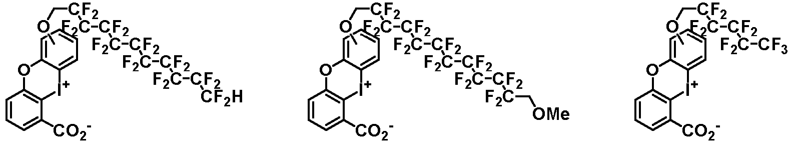

The iodonium salt can be synthesized by any well-known methods. For example, the iodonium salt may be synthesized by constructing a iodonium cation skeleton according to the synthesis method of JP 5062996, and effecting transformation reaction to convert it to the desired compound. The synthesis method is not limited thereto.

The iodonium salt of formula (A) is useful as a quencher. As seen from formula (A), the iodonium salt is characterized by having a basic carboxylate structure and a C₆-C₁₈ chainlike alkyl or C₄-C₁₈ chainlike fluorinated alkyl group on aromatic rings which form a iodonium cation. It is believed that since the alkyl or fluorinated alkyl group serves to increase solvent solubility and is highly lipophilic, the iodonium salt having formula (A) is distributed in an upper layer of the resist film. Particularly in the EB lithography, when a pattern is written with an electron beam, the influence of forward scattering of electron beam is unavoidable in the exposed region. If a conventional quencher is used, deprotection reaction runs to an excessive extent at the top of the pattern, leaving a concern that the pattern is rounded at the top, that is, takes a round-top profile. Using the inventive quencher, the excessive deprotection reaction at the top of the pattern is effectively suppressed and the pattern is corrected to be more rectangular. The carboxylate anion in the common molecule has a high basicity enough to control the diffusion of the generated acid whereby effective quenching is achieved. By virtue of the synergy of these effects, the iodonium salt ensures to form a pattern of rectangular profile having a satisfactory resolution, high contrast, improved CDU, and reduced LER.

### [Chemically amplified positive resist composition]

### (A) Quencher

Another embodiment of the invention is a chemically amplified positive resist composition comprising a quencher in the form of the iodonium salt having formula (A) as essential component (A). As used herein, the "quencher" refers to a compound capable of trapping an acid generated from the acid generator upon exposure. The quencher is effective for holding down the rate of diffusion of the acid (generated by the acid generator) in the resist film. Even when a substrate whose outermost surface is made of a chromium-containing material is used, the quencher is effective for suppressing the influence of the acid (generated in the resist film) on the chromium-containing material.

In the chemically amplified positive resist composition, the quencher (A) is preferably added in an amount of 0 to 50 parts, more preferably 0.1 to 40 parts by weight per 80 parts by weight of the base polymer (B) described just below. An amount of the quencher within the range is effective for trapping the generated acid and thus forming a pattern of satisfactory profile and provides for storage stability. The quencher may be used alone or in admixture of two or more.

### (B) Base polymer

In one preferred embodiment, the resist composition further comprises (B) a base polymer containing a polymer which is decomposed under the action of acid to increase its solubility in alkaline developer.

The polymer preferably contains repeat units having the formula (B1), which are also referred to as repeat units B1.

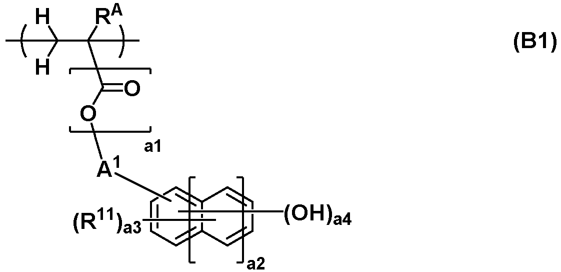

In formula (B1), a1 is 0 or 1. The subscript a2 is 0, 1 or 2. The relevant structure is a benzene ring when a2=0, a naphthalene ring when a2=1, and an anthracene ring when a2=2. The subscript a3 is an integer meeting 0 ≤ a3 ≤ 5+2(a2)-a4, and a4 is 1, 2 or 3. When a2 is 0, preferably a3 is 0, 1, 2 or 3 and a4 is 1, 2 or 3. When a2 is 1 or 2, preferably a3 is 0, 1, 2, 3 or 4 and a4 is 1, 2 or 3.

In formula (B1), R^{A} is hydrogen, fluorine, methyl or trifluoromethyl.

In formula (B1), R¹¹ is halogen, nitro, carboxy, an optionally halogenated C₁-C₆ saturated hydrocarbyl group, optionally halogenated C₁-C₆ saturated hydrocarbyloxy group or optionally halogenated C₂-C₈ saturated hydrocarbylcarbonyloxy group. The saturated hydrocarbyl group and saturated hydrocarbyl moiety in the saturated hydrocarbyloxy and saturated hydrocarbylcarbonyloxy groups may be straight, branched or cyclic. Examples thereof include alkyl groups such as methyl, ethyl, n-propyl, isopropyl, butyl, pentyl, hexyl, and structural isomers thereof; cycloalkyl groups such as cyclopropyl, cyclobutyl, cyclopentyl and cyclohexyl; and combinations thereof. A carbon count within the upper limit ensures a satisfactory solubility in alkaline developer. A plurality of R¹¹ may be identical or different when a3 is 2 or more.

In formula (B1), A¹ is a single bond or C₁-C₁₀ saturated hydrocarbylene group in which some constituent -CH₂- may be replaced by -O-. The saturated hydrocarbylene group may be straight, branched or cyclic and examples thereof include C₁-C₁₀ alkanediyl groups such as methylene, ethane-1,2-diyl, propane-1,3-diyl, butane-1,4-diyl, pentane-1,5-diyl, hexane-1,6-diyl, and structural isomers thereof; C₃-C₁₀ cyclic saturated hydrocarbylene groups such as cyclopropanediyl, cyclobutanediyl, cyclopentanediyl, and cyclohexanediyl; and combinations thereof. For the saturated hydrocarbylene group containing an ether bond, in case of a1=1 in formula (B1), the ether bond may be incorporated at any position excluding the position between the α- and β-carbons relative to the ester oxygen. In case of a1=0, the atom bonding to the backbone becomes an ether oxygen atom, and a second ether bond may be incorporated at any position excluding the position between the α- and β-carbons relative to the ether oxygen. Saturated hydrocarbylene groups having no more than 10 carbon atoms are desirable because of a sufficient solubility in alkaline developer.

Preferred examples of the repeat units B1 wherein a1=0 and A¹ is a single bond (meaning that the aromatic ring is directly bonded to the backbone of the polymer), that is, repeat units free of a linker: -C(=O)-O-A¹- include units derived from 3-hydroxystyrene, 4-hydroxystyrene, 5-hydroxy-2-vinylnaphthalene, and 6-hydroxy-2-vinylnaphthalene. Exemplary units are shown below, but not limited thereto. Herein R^{A} is as defined above.

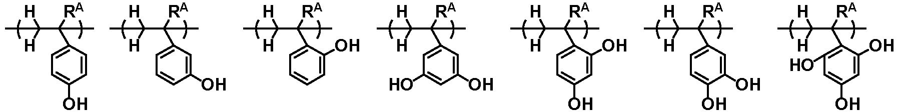

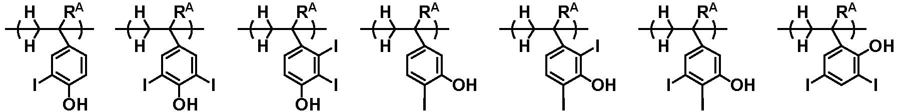

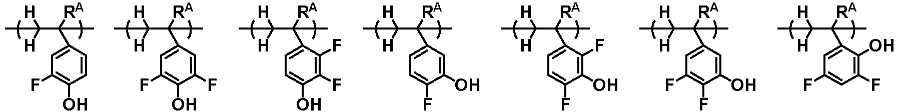

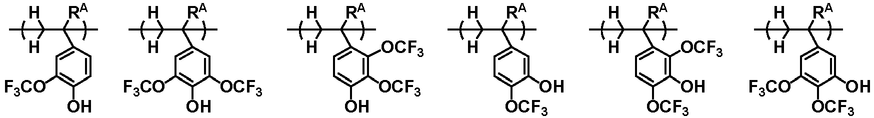

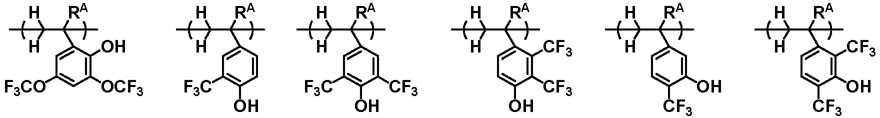

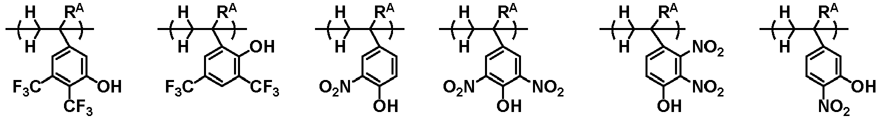

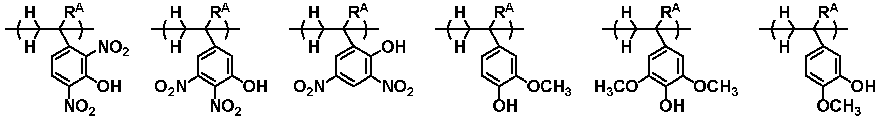

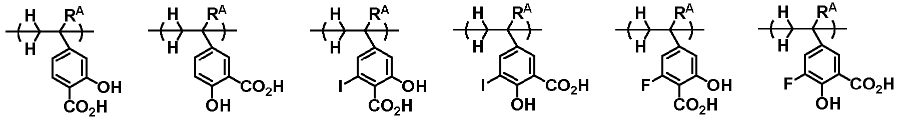

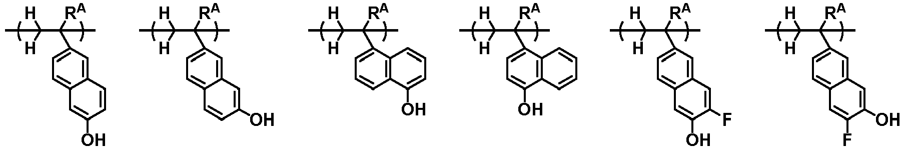

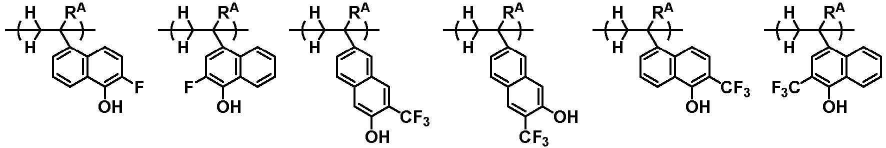

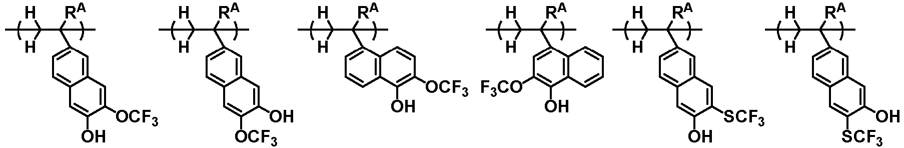

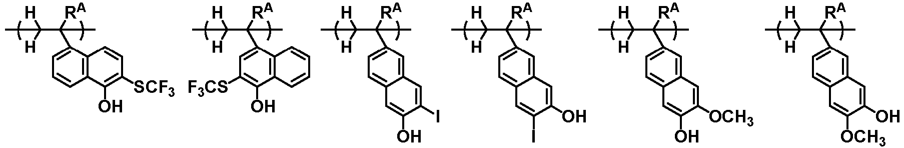

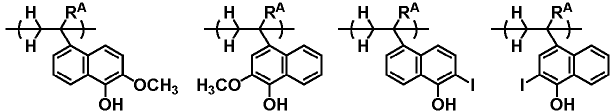

Preferred examples of the repeat units B1 wherein a1=1, that is, having a linker: -C(=O)-O-A¹- are shown below, but not limited thereto. Herein R^{A} is as defined above.

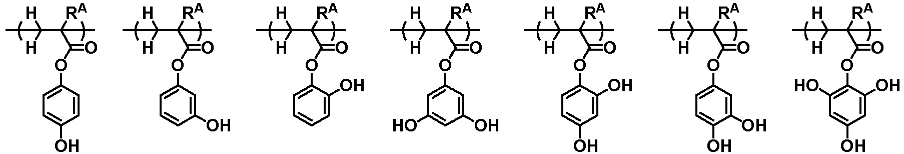

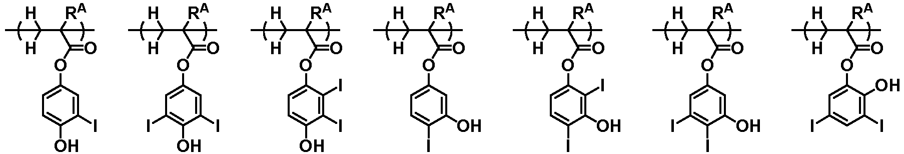

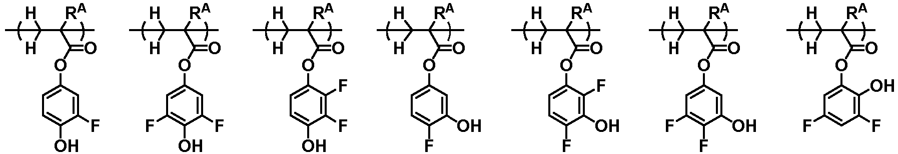

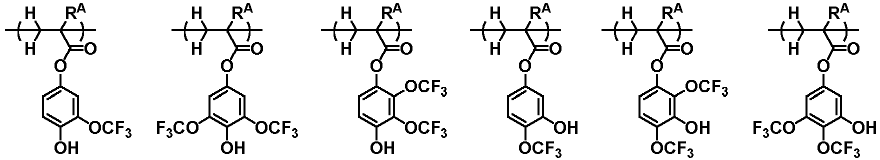

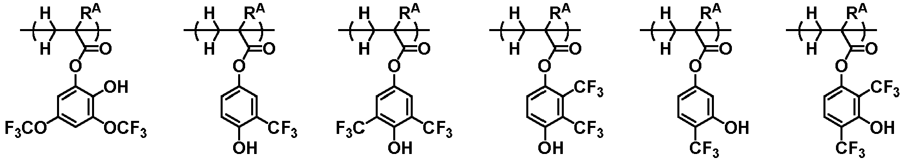

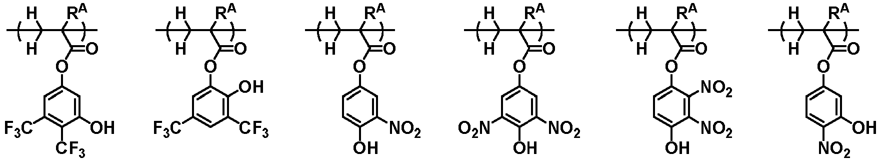

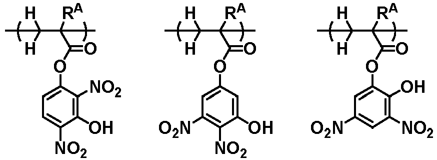

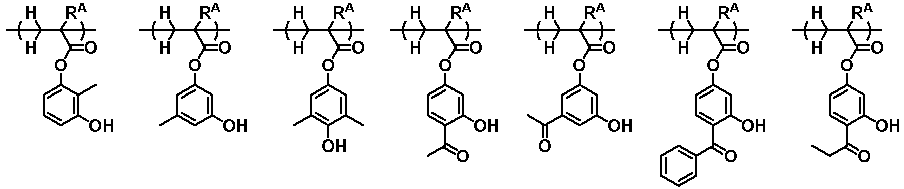

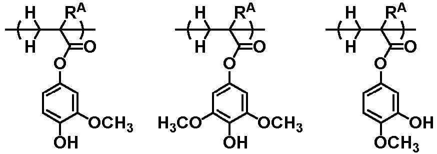

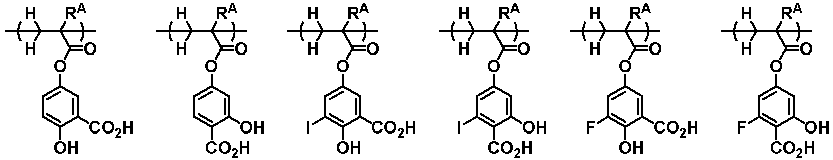

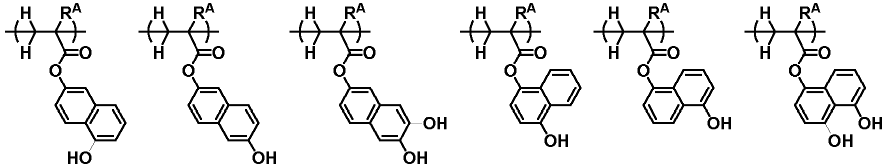

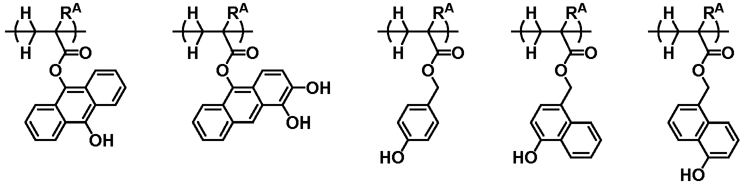

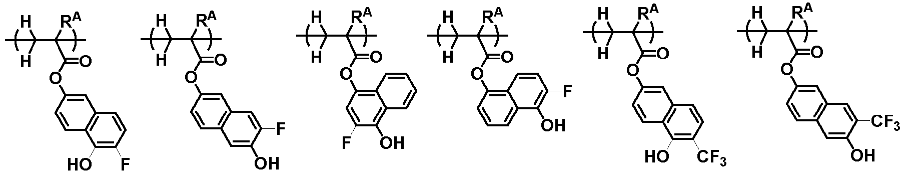

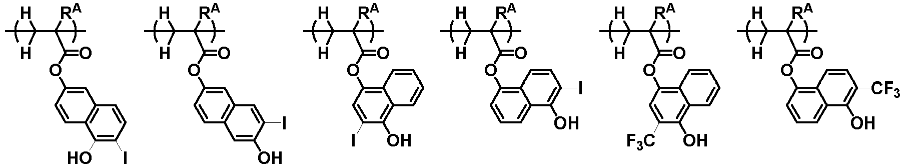

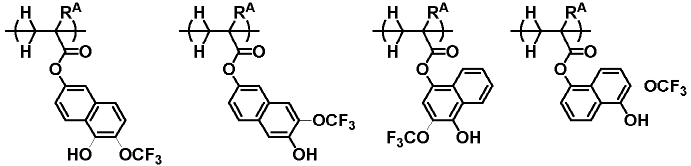

The content of repeat units B1 is preferably 15 to 90 mol%, more preferably 15 to 80 mol% of the overall units of the polymer. When the polymer further comprises repeat units of at least one type selected from repeat units having formula (B3) and repeat units having formula (B4), which provide the polymer with higher etch resistance, the repeat units containing a phenolic hydroxy group as a substituent, the total content of repeat units B1 and repeat units B3 and/or B4 should preferably fall in the range. The repeat units B1 may be of one type or a combination of plural types.

In a preferred embodiment, the polymer further contains repeat units B2 having an acidic functional group protected with an acid labile group (i.e., repeat units protected with an acid labile group and adapted to turn alkali soluble under the action of acid) in order that the positive resist composition in an exposed region turn soluble in alkaline developer.

Typical of the repeat unit B2 is a unit having the formula (B2-1), also referred to as repeat unit B2-1.

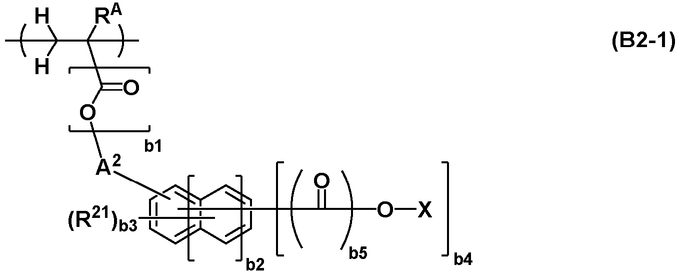

In formula (B2-1), b1 is 0 or 1. The subscript b2 is 0, 1 or 2. The structure represents a benzene skeleton when b2=0, a naphthalene skeleton when b2=1, and an anthracene skeleton when b2=2. The subscript b3 is an integer meeting 0 ≤ b3 ≤ 5+2(b2)-b4. The subscript b4 is 1, 2 or 3, and b5 is 0 or 1. When b2=0, preferably b3 is 0, 1, 2 or 3 and b4 is 1, 2 or 3. When b2=1 or 2, preferably b3 is 0, 1, 2, 3 or 4 and b4 is 1, 2 or 3.

In formula (B2-1), R^{A} is hydrogen, fluorine, methyl or trifluoromethyl.

In formula (B2-1), R²¹ is halogen, an optionally halogenated C₁-C₆ saturated hydrocarbyl group, optionally halogenated C₁-C₆ saturated hydrocarbyloxy group or optionally halogenated C₂-C₈ saturated hydrocarbylcarbonyloxy group. The saturated hydrocarbyl group and saturated hydrocarbyl moiety in the saturated hydrocarbyloxy group and saturated hydrocarbylcarbonyloxy group may be straight, branched or cyclic, and examples thereof include alkyl groups such as methyl, ethyl, n-propyl, isopropyl, butyl, pentyl, hexyl, and structural isomers thereof, cycloalkyl groups such as cyclopropyl, cyclobutyl, cyclopentyl and cyclohexyl, and combinations thereof. A carbon count within the upper limit ensures good solubility in alkaline developer. A plurality of R²¹ may be identical or different when b3 is 2 or more.

In formula (B2-1), A² is a single bond or a C₁-C₁₀ saturated hydrocarbylene group in which some constituent -CH₂- may be replaced by -O-. The saturated hydrocarbylene group may be straight, branched or cyclic and examples thereof include C₁-C₁₀ alkanediyl groups such as methylene, ethane-1,2-diyl, propane-1,3-diyl, butane-1,4-diyl, pentane-1,5-diyl, hexane-1,6-diyl, and structural isomers thereof; C₃-C₁₀ cyclic saturated hydrocarbylene groups such as cyclopropanediyl, cyclobutanediyl, cyclopentanediyl, and cyclohexanediyl; and combinations thereof. For the saturated hydrocarbylene group containing an ether bond, in case of b1=1 in formula (B2-1), the ether bond may be incorporated at any position excluding the position between the α-carbon and β-carbon relative to the ester oxygen. In case of b1=0, the atom that bonds with the backbone becomes an ethereal oxygen, and a second ether bond may be incorporated at any position excluding the position between the α-carbon and β-carbon relative to that ethereal oxygen. Saturated hydrocarbylene groups having no more than 10 carbon atoms are desirable because of a sufficient solubility in alkaline developer.

In formula (B2-1), X is an acid labile group when b4=1. X is hydrogen or an acid labile group, at least one X being an acid labile group, when b4=2 or 3. That is, repeat units B2-1 have phenolic hydroxy groups bonded to an aromatic ring, at least one of which is protected with an acid labile group, or repeat units B2-1 have a carboxy group bonded to an aromatic ring, which is protected with an acid labile group. The acid labile group used herein is not particularly limited as long as it is commonly used in a number of well-known chemically amplified resist compositions and eliminated under the action of acid to release an acidic group.

Typical of the acid labile group is a tertiary saturated hydrocarbyl group. The tertiary saturated hydrocarbyl group is preferably of 4 to 18 carbon atoms because a monomer for use in polymerization is recoverable by distillation.

The saturated hydrocarbyl group bonded to the tertiary carbon atom in the tertiary saturated hydrocarbyl group is preferably of 1 to 15 carbon atoms. The C₁-C₁₅ saturated hydrocarbyl group may be straight, branched or cyclic and contain an oxygen-containing functional group such as an ether bond or carbonyl group in its carbon-carbon bond. The saturated hydrocarbyl groups bonded to the tertiary carbon atom may bond together to form a ring with the tertiary carbon atom to which they are attached.

Examples of the alkyl substituent include methyl, ethyl, propyl, adamantyl, norbornyl, tetrahydrofuran-2-yl, 7-oxanorbornan-2-yl, cyclopentyl, 2-tetrahydrofuryl, tricyclo[5.2.1.0^{2,6}]decyl, 8-ethyl-8-tricyclo[5.2.1.0^{2,6}]decyl, 3-methyl-3-tetracyclo[4.4.0.1^{2,5}.1^{7,10}]dodecyl, tetracyclo[4.4.0.1^{2,5}.1^{7,10}]dodecyl, and 3-oxo-1-cyclohexyl.

Examples of the tertiary saturated hydrocarbyl group include, but are not limited to, tert-butyl, tert-pentyl, 1-ethyl-1-methylpropyl, 1,1-diethylpropyl, 1,1,2-trimethylpropyl, 1-adamantyl-1-methylethyl, 1-methyl-1-(2-norbornyl)ethyl, 1-methyl-1-(tetrahydrofuran-2-yl)ethyl, 1-methyl-1-(7-oxanorbornan-2-yl)ethyl, 1-methylcyclopentyl, 1-ethylcyclopentyl, 1-propylcyclopentyl, 1-cyclopentylcyclopentyl, 1-cyclohexylcyclopentyl, 1-(2-tetrahydrofuryl)cyclopentyl, 1-(7-oxanorbornan-2-yl)cyclopentyl, 1-methylcyclohexyl, 1-ethylcyclohexyl, 1-cyclopentylcyclohexyl, 1-cyclohexylcyclohexyl, 2-methyl-2-norbornyl, 2-ethyl-2-norbornyl, 8-methyl-8-tricyclo[5.2.1.0^{2,6}]decyl, 8-ethyl-8-tricyclo[5.2.1.0^{2,6}]decyl, 3-methyl-3-tetracyclo[4.4.0.1^{2,5}.1^{7,10}]dodecyl, 3-ethyl-3-tetracyclo[4.4.0.1^{2,5}.1^{7,10}]dodecyl, 2-methyl-2-adamantyl, 2-ethyl-2-adamantyl, 1-methyl-3-oxo-1-cyclohexyl, 1-methyl-1-(tetrahydrofuran-2-yl)ethyl, 5-hydroxy-2-methyl-2-adamantyl, and 5-hydroxy-2-ethyl-2-adamantyl.

A group having the following formula (B2-1-1) is also suitable as the acid labile group. The group having formula (B2-1-1) is often used as the acid labile group. It is a good choice of the acid labile group that ensures to form a pattern having a relatively rectangular pattern-substrate interface in a consistent manner. An acetal structure is formed when X is a group having formula (B2-1-1).

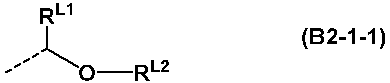

In formula (B2-1-1), R^{L1} is hydrogen or a C₁-C₁₀ saturated hydrocarbyl group. The saturated hydrocarbyl group may be straight, branched or cyclic.

A choice of R^{L1} may depend on the designed sensitivity of labile group to acid. For example, hydrogen or a group in which the carbon atom bonded to acetal carbon is tertiary is selected when the acid labile group is designed to ensure relatively high stability and to be decomposed with strong acid. Examples of R^{L1} bonded to acetal carbon via tertiary carbon include tert-butyl, tert-pentyl, and 1-adamantyl, but are not limited thereto. A straight alkyl group is selected when the acid labile group is designed to have relatively high reactivity and high sensitivity to pH changes. Although the choice varies with a particular combination of acid generator and quencher in the resist composition, R^{L1} is preferably a group in which the carbon in bond with acetal carbon is secondary, when R^{L2} is a relatively large alkyl group substituted at the end and the acid labile group is designed to undergo a substantial change of solubility by decomposition. Examples of R^{L1} bonded to acetal carbon via secondary carbon include isopropyl, sec-butyl, cyclopentyl, and cyclohexyl, but are not limited thereto.

In formula (B2-1-1), R^{L2} is a C₁-C₃₀ hydrocarbyl group. The hydrocarbyl group may be saturated or unsaturated and straight, branched or cyclic. Some constituent -CH₂-in the hydrocarbyl group may be replaced by a heteroatom such as oxygen or sulfur so that the group may contain an ether bond or sulfide bond. Illustrative are C₁-C₃₀ saturated hydrocarbyl groups and C₆-C₃₀ aryl groups. R^{L2} is preferably a C₁-C₆ hydrocarbyl group for acquiring a higher resolution in forming small-size patterns. When R^{L2} is a C₁-C₆ hydrocarbyl group, the alcohol created after a progress of acid-aided deprotection reaction is water soluble. Then, when a positive pattern is formed using an alkaline developer, the alcohol is dissolved in the developer so that defects remaining in the exposed region are minimized.

Preferred examples of the group having formula (B2-1-1) are given below, but not limited thereto. Herein R^{L1} is as defined above.

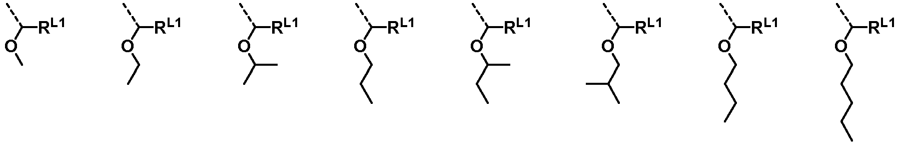

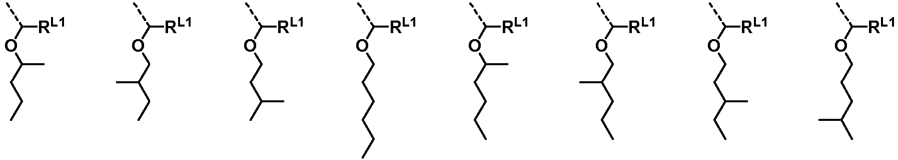

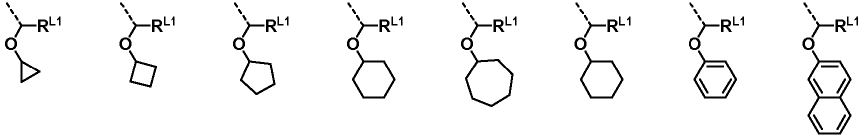

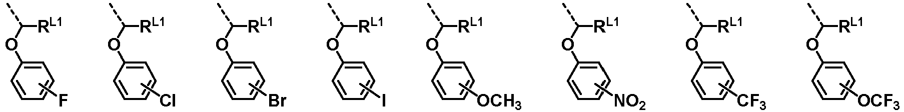

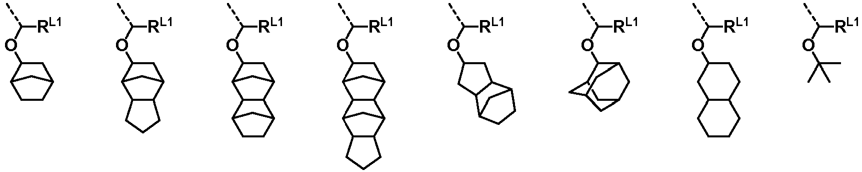

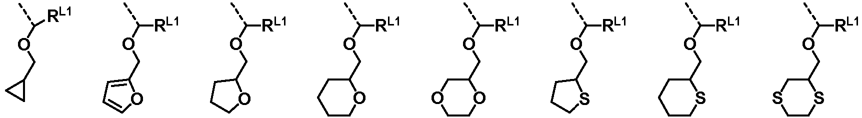

Another acid labile group which can be used herein is a phenolic hydroxy group whose hydrogen is substituted by -CH₂COO-(tertiary saturated hydrocarbyl group). The tertiary saturated hydrocarbyl group may be the same as the foregoing tertiary saturated hydrocarbyl group used for the protection of a phenolic hydroxy group.

Another example of repeat unit B2 is a repeat unit having the following formula (B2-2), referred to as repeat unit B2-2. The repeat unit B2-2 which enables to increase the dissolution rate in the exposed region is a useful choice of the acid labile group-containing unit which affords satisfactory performance against line width variations during develop loading.

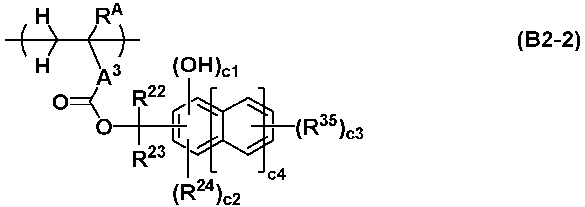

In formula (B2-2), c1 is 0, 1 or 2, c2 is 0, 1 or 2, c3 is 0, 1, 2, 3, 4 or 5, and c4 is 0, 1 or 2.

In formula (B2-2), R^{A} is hydrogen, fluorine, methyl or trifluoromethyl.

In formula (B2-2), R²² and R²³ are each independently a C₁-C₁₀ hydrocarbyl group which may contain a heteroatom. R²² and R²³ may bond together to form a ring with the carbon atom to which they are attached.

In formula (B2-2), R²⁴ is each independently fluorine, C₁-C₅ fluorinated alkyl group or C₁-C₅ fluorinated alkoxy group.

In formula (B2-2), R²⁵ is each independently a C₁-C₁₀ hydrocarbyl group which may contain a heteroatom.

In formula (B2-2), A³ is a single bond, phenylene group, naphthylene group, or *-C(=O)-O-A³¹-. A³¹ is a C₁-C₂₀ aliphatic hydrocarbylene group which may contain hydroxy, ether bond, ester bond or lactone ring, or phenylene or naphthylene group, and * is a point of attachment to the carbon atom in the backbone.

Preferred examples of the repeat unit B2-2 are shown below, but not limited thereto. Herein R^{A} is as defined above.

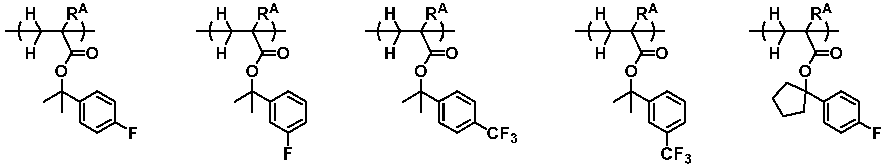

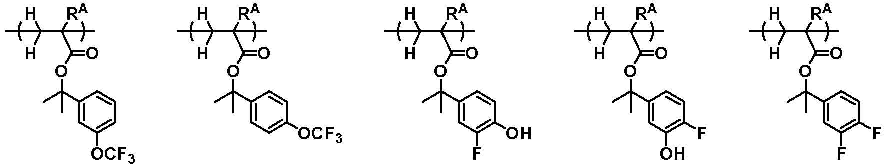

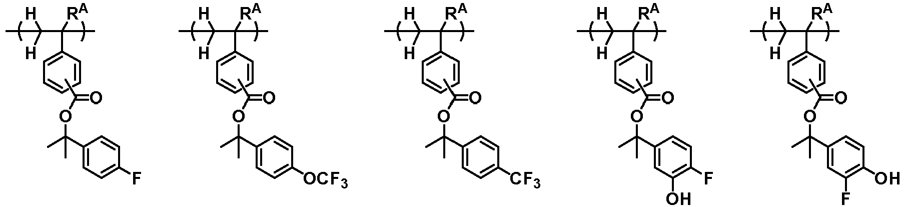

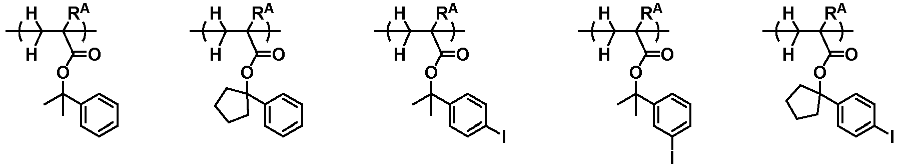

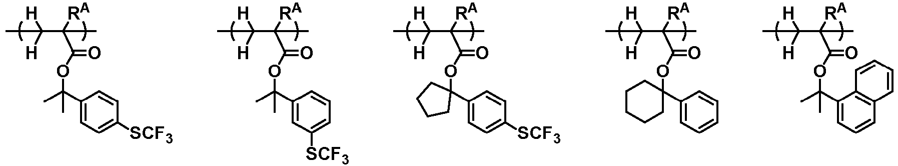

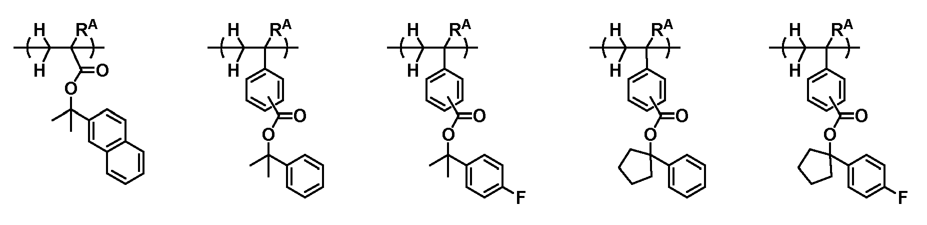

The content of repeat units B2 is preferably 5 to 95 mol%, more preferably 20 to 80 mol% based on the overall repeat units of the polymer. Each of repeat units B2 may be of one type or a mixture of two or more types.

In a preferred embodiment, the polymer further comprises repeat units of at least one type selected from units having the formulae (B3), (B4) and (B5). These repeat units are simply referred to as repeat units B3, B4 and B5, respectively.

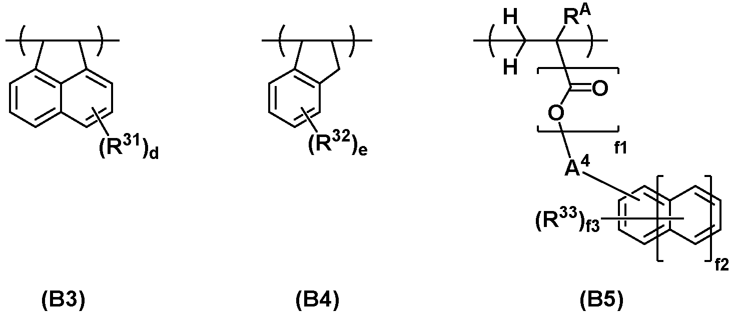

In formulae (B3) and (B4), d is 0, 1, 2, 3, 4, 5 or 6 and e is 0, 1, 2, 3 or 4.

In formulae (B3) and (B4), R³¹ and R³² are each independently hydroxy, halogen, an optionally halogenated C₁-C₆ saturated hydrocarbyl group, optionally halogenated C₁-C₆ saturated hydrocarbyloxy group, or optionally halogenated C₂-C₈ saturated hydrocarbylcarbonyloxy group. The saturated hydrocarbyl group, saturated hydrocarbyloxy group and saturated hydrocarbylcarbonyloxy group may be straight, branched or cyclic. When d is 2 or more, a plurality of R³¹ may be identical or different. When e is 2 or more, a plurality of R³² may be identical or different.

In formula (B5), f1 is 0 or 1. The subscript f2 is 0, 1 or 2. The relevant structure represents a benzene skeleton when f2=0, a naphthalene skeleton when f2=1, and an anthracene skeleton when f2=2. The subscript f3 is 0, 1, 2, 3, 4 or 5. When f2=0, preferably f3 is 0, 1, 2 or 3. When f2=1 or 2, preferably f3 is 0, 1, 2, 3 or 4.

In formula (B5), R^{A} is hydrogen, fluorine, methyl or trifluoromethyl.

In formula (B5), R³³ is a C₁-C₂₀ saturated hydrocarbyl group, C₁-C₂₀ saturated hydrocarbyloxy group, C₂-C₂₀ saturated hydrocarbylcarbonyloxy group, C₂-C₂₀ saturated hydrocarbyloxyhydrocarbyl group, C₂-C₂₀ saturated hydrocarbylthiohydrocarbyl group, halogen atom, nitro group, or cyano group. When f2 is 1 or 2, R³³ may also be hydroxy. The saturated hydrocarbyl group, saturated hydrocarbyloxy group, saturated hydrocarbylcarbonyloxy group, saturated hydrocarbyloxyhydrocarbyl group, and saturated hydrocarbylthiohydrocarbyl group may be straight, branched or cyclic. When f3 is 2 or more, a plurality of R³³ may be identical or different.

In formula (B5), A⁴ is a single bond or a C₁-C₁₀ saturated hydrocarbylene group in which some constituent -CH₂- may be replaced by -O-. The saturated hydrocarbylene group may be straight, branched or cyclic. Examples thereof are as exemplified above for A¹ in formula (B 1).

When repeat units of at least one type selected from repeat units B3 to B5 are incorporated, better performance is obtained because not only the aromatic ring possesses etch resistance, but the cyclic structure incorporated into the backbone also exerts the effect of improving etch resistance and resistance to EB irradiation during pattern inspection step.

The content of repeat units B3 to B5 is preferably at least 5 mol% based on the overall repeat units of the polymer for obtaining the effect of improving etch resistance. Also, the content of repeat units B3 to B5 is preferably up to 25 mol%, more preferably up to 20 mol% based on the overall repeat units of the polymer. When the relevant units are free of functional groups or have a functional group other than hydroxy, their content of up to 25 mol% is preferred because the risk of forming development defects is eliminated. Each of the repeat units B3 to B5 may be of one type or a combination of plural types.

It is preferred that the polymer comprise repeat units B1, repeat units B2, and repeat units of at least one type selected from repeat units B3 to B5, because both etch resistance and high resolution are achievable. The total content of these repeat units is preferably at least 60 mol%, more preferably at least 70 mol%, even more preferably at least 80 mol%, most preferably at least 90 mol% based on the overall repeat units of the polymer.

In another preferred embodiment, the polymer further comprises repeat units of at least one type selected from repeat units having the formula (B6), repeat units having the formula (B7), repeat units having the formula (B8), repeat units having the formula (B9), and repeat units having the formula (B10), shown below. Notably these repeat units are also referred to as repeat units B6 to B10.

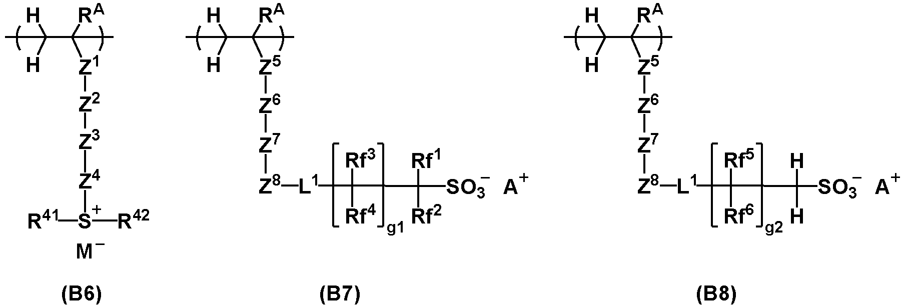

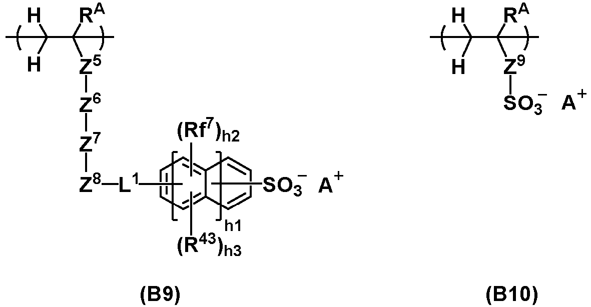

In formulae (B6) to (B10), R^{A} is each independently hydrogen, fluorine, methyl or trifluoromethyl. Z¹ is a single bond or an optionally substituted phenylene group. Z² is a single bond, **-C(=O)-O-Z²¹-, **-C(=O)-NH-Z²¹-, or **-O-Z²¹-, wherein Z²¹ is a C₁-C₆ aliphatic hydrocarbylene group, phenylene group or a divalent group obtained by combining the foregoing, which may contain halogen, carbonyl moiety, ester bond, ether bond or hydroxy moiety. Z³ is a single bond, ether bond, ester bond, amide bond, sulfonate ester bond, sulfonamide bond, carbonate bond or carbamate bond. Z⁴ is a single bond, or a C₁-C₆ aliphatic hydrocarbylene group, phenylene group or a divalent group obtained by combining the foregoing, which may contain halogen, carbonyl moiety, ester bond, ether bond or hydroxy moiety. Z⁵ is each independently a single bond, an optionally substituted phenylene, naphthylene, or *-C(=O)-O-Z⁵¹-, wherein Z⁵¹ is a C₁-C₁₀ aliphatic hydrocarbylene group which may contain halogen, hydroxy moiety, ether bond, ester bond or lactone ring, or phenylene or naphthylene group. Z⁶ is a single bond, ether bond, ester bond, amide bond, sulfonate ester bond, sulfonamide bond, carbonate bond or carbamate bond. Z⁷ is each independently a single bond, ***-Z⁷¹-C(=O)-O-, ***-C(=O)-NH-Z⁷¹-, or ***-O-Z⁷¹-, wherein Z⁷¹ is a C₁-C₂₀ hydrocarbylene group which may contain a heteroatom. Z⁸ is each independently a single bond, ****-Z⁸¹-C(=O)-O-, ****-C(=O)-NH-Z⁸¹-, or ****-O-Z⁸¹-, wherein Z⁸¹ is a C₁-C₂₀ hydrocarbylene group which may contain a heteroatom. Z⁹ is a single bond, methylene, ethylene, phenylene, fluorinated phenylene, trifluoromethyl-substituted phenylene, *-C(=O)-O-Z⁹¹-, *-C(=O)-N(H)-Z⁹¹-, or *-O-Z⁹¹-, wherein Z⁹¹ is a C₁-C₆ aliphatic hydrocarbylene group, phenylene group, fluorinated phenylene group or trifluoromethyl-substituted phenylene group, which may contain a carbonyl moiety, ester bond, ether bond or hydroxy moiety. The asterisk * designates a point of attachment to the carbon atom in the backbone, ** is a point of attachment to Z¹, *** is a point of attachment to Z⁶, and **** is a point of attachment to Z⁷.

The aliphatic hydrocarbylene group represented by Z²¹, Z⁵¹ and Z⁹¹ may be straight, branched or cyclic. Examples thereof include alkanediyl groups such as methanediyl, ethane-1,1-diyl, ethane-1,2-diyl, propane-1,1-diyl, propane-1,2-diyl, propane-1,3-diyl, propane-2,2-diyl, butane-1,1-diyl, butane-1,2-diyl, butane-1,3-diyl, butane-2,3-diyl, butane-1,4-diyl, 1,1-dimethylethane-1,2-diyl, pentane-1,5-diyl, 2-methylbutane-1,2-diyl, and hexane-1,6-diyl; cycloalkanediyl groups such as cyclopropanediyl, cyclobutanediyl, cyclopentanediyl, and cyclohexanediyl; and combinations thereof.

The optionally heteroatom-containing hydrocarbylene group represented by Z⁷¹ and Z⁸¹ may be saturated or unsaturated and straight, branched or cyclic. Examples thereof are shown below, but not limited thereto.

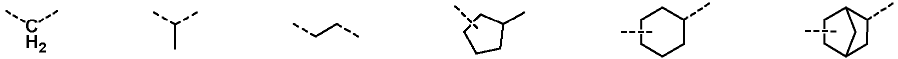

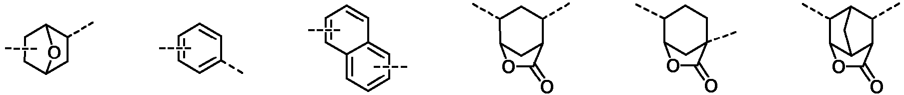

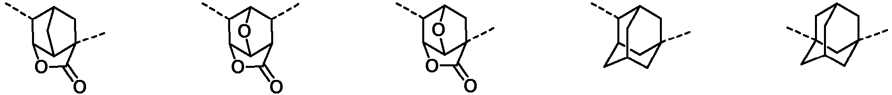

**In** formula (B6), R⁴¹ and R⁴² are each independently a C₁-C₂₀ hydrocarbyl group which may contain a heteroatom. The hydrocarbyl group may be saturated or unsaturated and straight, branched or cyclic. Examples thereof include C₁-C₂₀ alkyl groups such as methyl, ethyl, n-propyl, isopropyl, n-butyl, isobutyl, sec-butyl, and tert-butyl; C₃-C₂₀ cyclic saturated hydrocarbyl groups such as cyclopropyl, cyclopentyl, cyclohexyl, cyclopropylmethyl, 4-methylcyclohexyl, cyclohexylmethyl, norbornyl, and adamantyl; C₂-C₂₀ alkenyl groups such as vinyl, 1-propenyl, 2-propenyl, butenyl, and hexenyl; C₃-C₂₀ cyclic unsaturated hydrocarbyl groups such as cyclohexenyl; C₆-C₂₀ aryl groups such as phenyl, naphthyl and thienyl; C₇-C₂₀ aralkyl groups such as benzyl, 1-phenylethyl and 2-phenylethyl, and combinations thereof. Inter alia, aryl groups are preferred. **In** the hydrocarbyl group, some or all of the hydrogen atoms may be substituted by a moiety containing a heteroatom such as oxygen, sulfur, nitrogen or halogen and some constituent -CH₂- may be replaced by a moiety containing a heteroatom such as oxygen, sulfur or nitrogen, so that the group may contain a hydroxy, fluorine, chlorine, bromine, iodine, cyano, carbonyl, ether bond, ester bond, sulfonate ester bond, carbonate bond, lactone ring, sultone ring, carboxylic anhydride (-C(=O)-O-C(=O)-), or haloalkyl moiety.

R⁴¹ and R⁴² may bond together to form a ring with the sulfur atom to which they are attached. Examples of the ring are shown below.

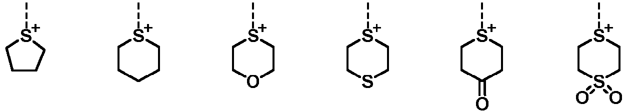

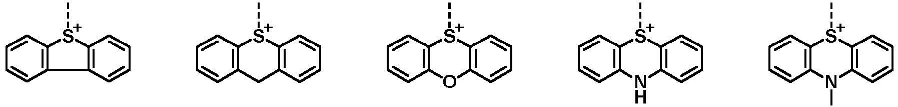

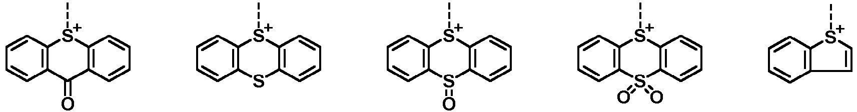

Herein the broken line designates a point of attachment to Z⁴.

Examples of the cation in repeat units B6 are shown below, but not limited thereto. Herein R^{A} is as defined above.

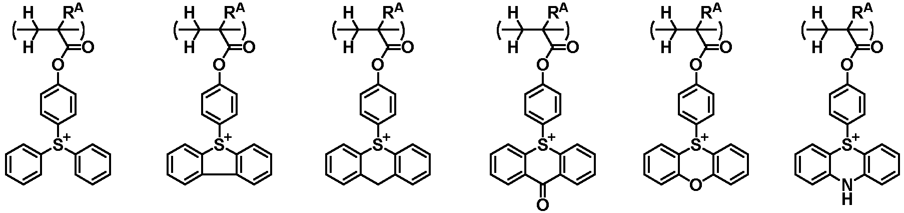

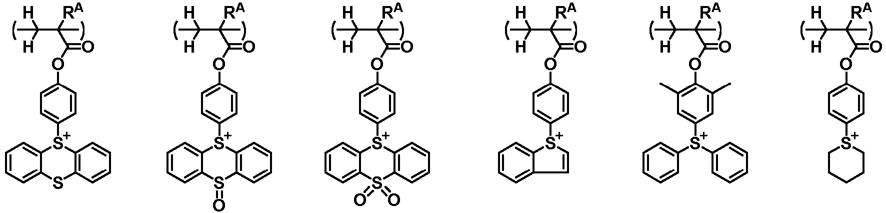

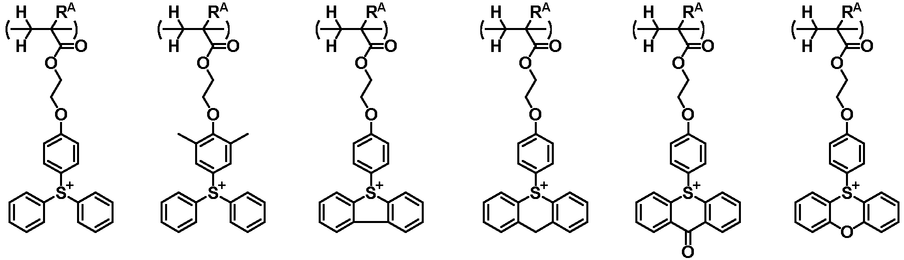

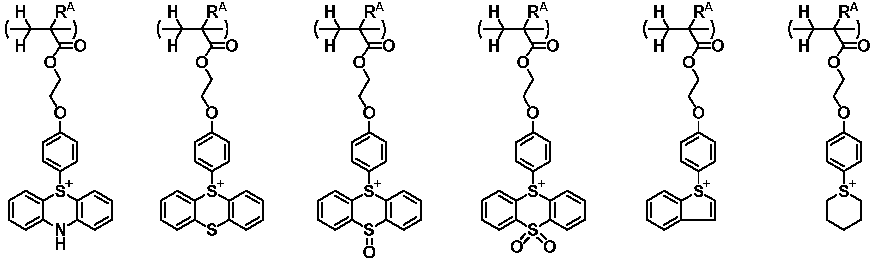

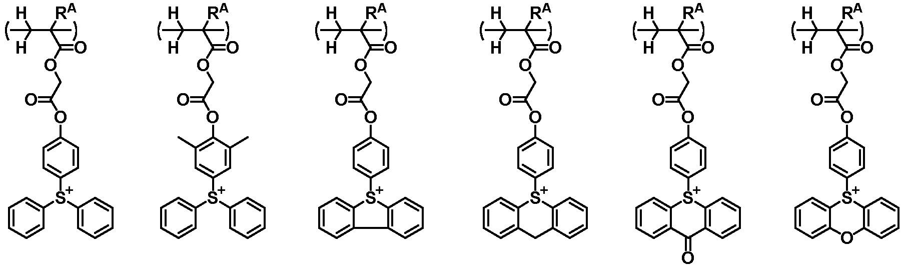

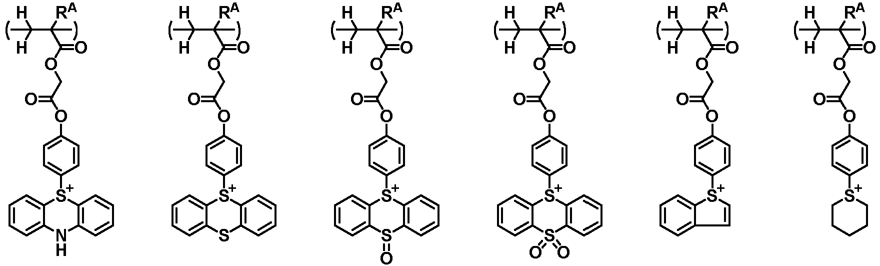

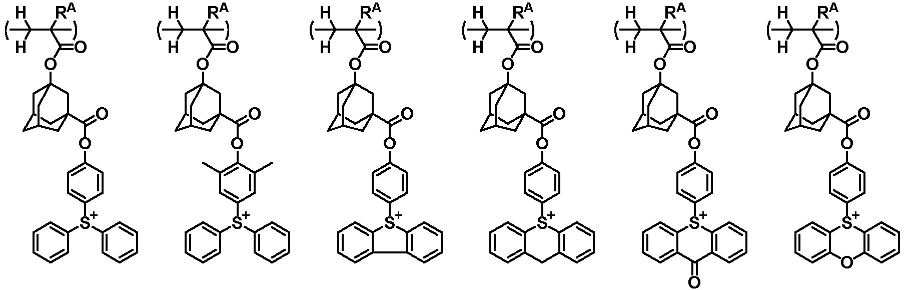

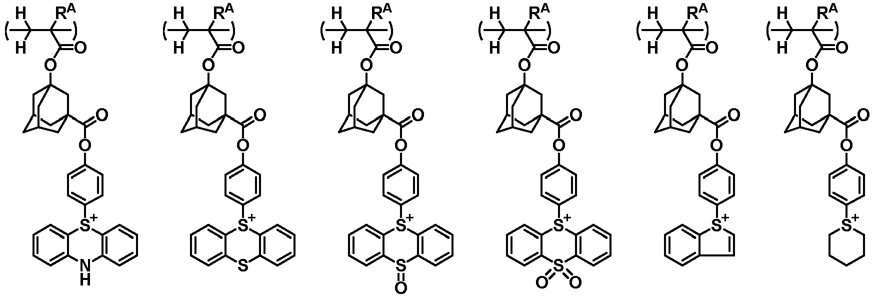

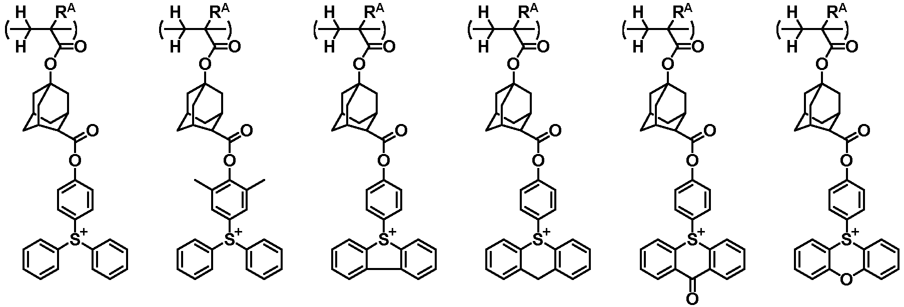

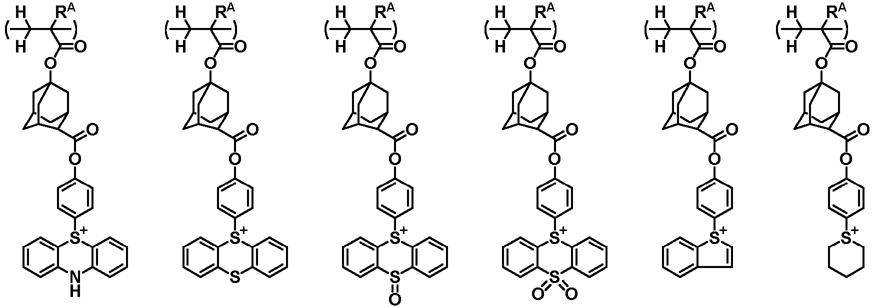

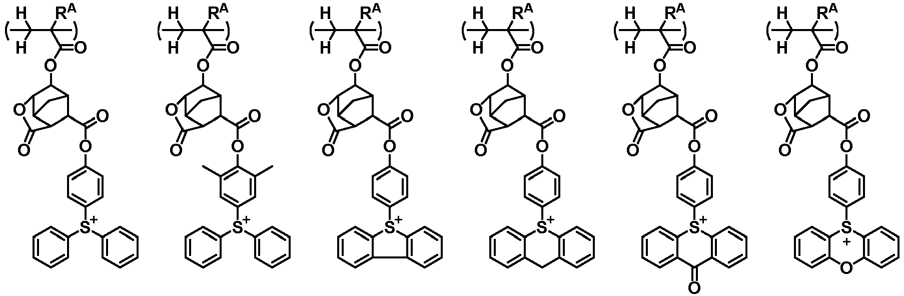

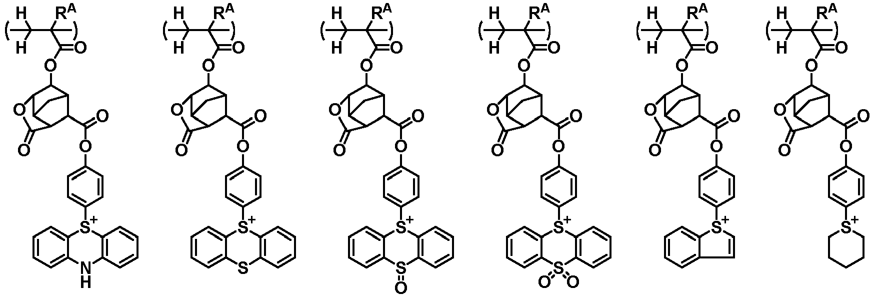

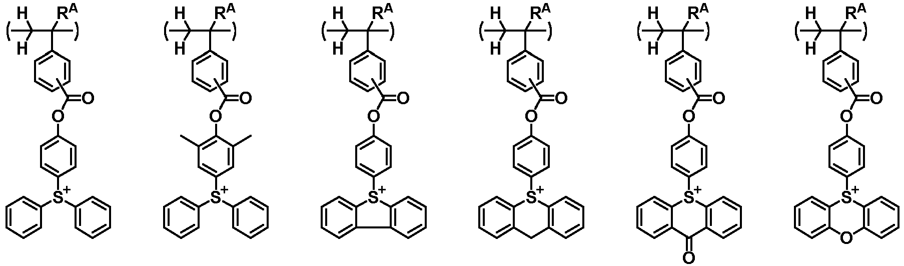

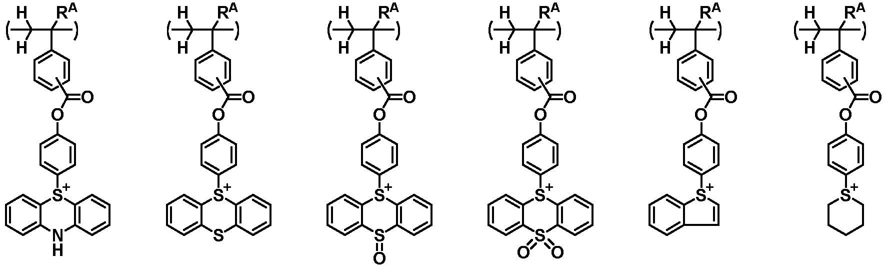

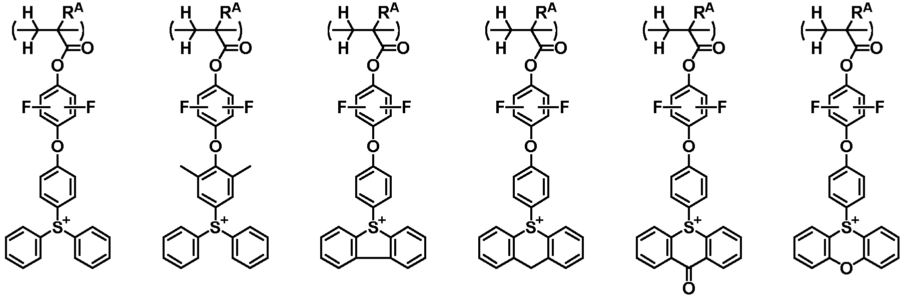

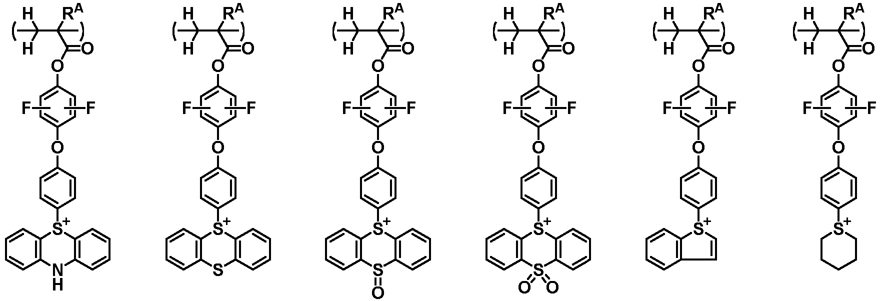

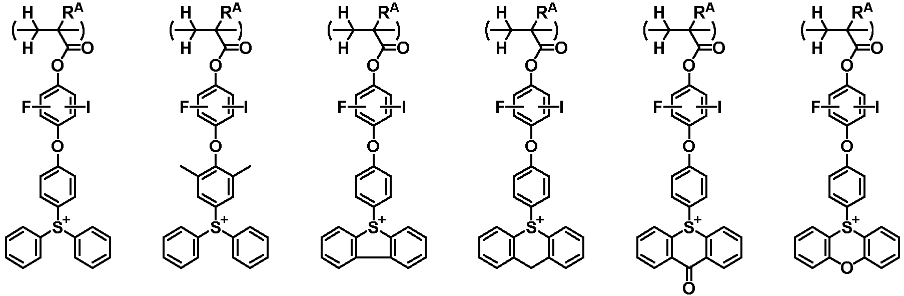

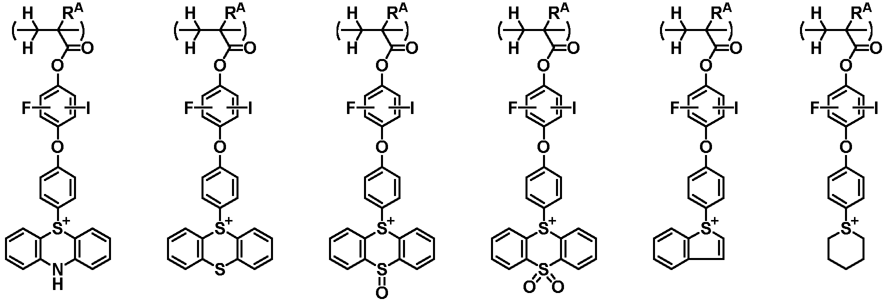

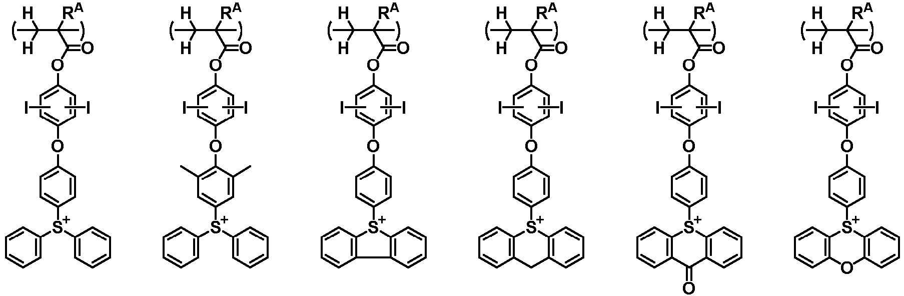

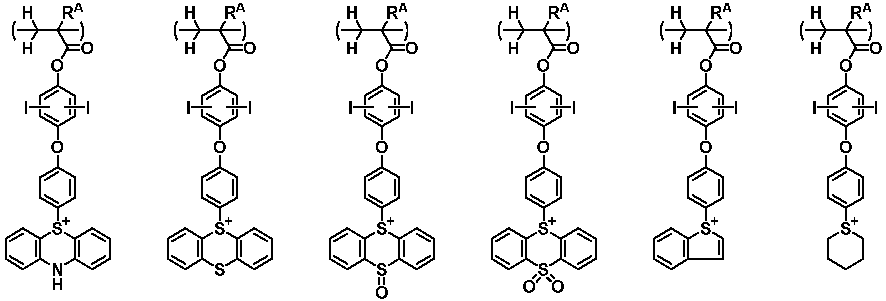

**In** formula (B6), M⁻ is a non-nucleophilic counter ion. Halide ions, sulfonate anions, imide anions, and methide anions are preferred. Examples of the non-nucleophilic counter ion include halide ions such as chloride and bromide ions; sulfonate anions, specifically fluoroalkylsulfonate ions such as triflate, 1,1,1-trifluoroethanesulfonate, and nonafluorobutanesulfonate, arylsulfonate ions such as tosylate, benzenesulfonate, 4-fluorobenzenesulfonate, and 1,2,3,4,5-pentafluorobenzenesulfonate, alkylsulfonate ions such as mesylate and butanesulfonate; imide ions such as bis(trifluoromethylsulfonyl)imide, bis(perfluoroethylsulfonyl)imide and bis(perfluorobutylsulfonyl)imide; and methide ions such as tris(trifluoromethylsulfonyl)methide and tris(perfluoroethylsulfonyl)methide.

Anions having the following formulae (B6-1) to (B6-4) are also useful as the non-nucleophilic counter ion.

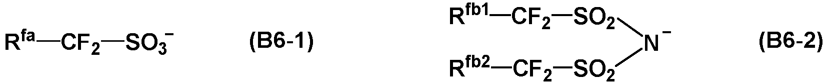

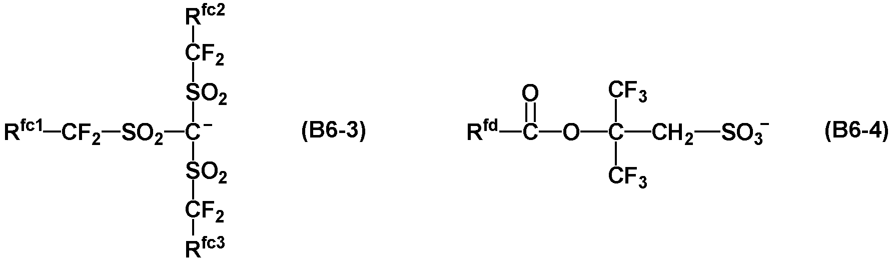

In formula (B6-1), R^{fa} is fluorine or a C₁-C₄₀ hydrocarbyl group which may contain a heteroatom. The hydrocarbyl group may be saturated or unsaturated and straight, branched or cyclic. Examples thereof are as will be exemplified below for the hydrocarbyl group R^{fa1} in formula (B6-1-1).

Of the anions of formula (B6-1), an anion having the formula (B6-1-1) is preferred.

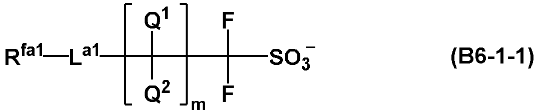

In formula (B6-1-1), Q¹ and Q² are each independently hydrogen, fluorine or a C₁-C₆ fluorinated saturated hydrocarbyl group. It is preferred for solvent solubility that at least one of Q¹ and Q² be trifluoromethyl. The subscript m is 0, 1, 2, 3 or 4, preferably 1.

R^{fa1} is a C₁-C₃₅ hydrocarbyl group which may contain a heteroatom. As the heteroatom, oxygen, nitrogen, sulfur and halogen atoms are preferred, with oxygen being most preferred. Of the hydrocarbyl groups, those groups of 6 to 30 carbon atoms are preferred from the aspect of achieving a high resolution in forming patterns of small feature size. The hydrocarbyl group may be saturated or unsaturated and straight, branched or cyclic. Examples thereof include C₁-C₃₅ alkyl groups such as methyl, ethyl, n-propyl, isopropyl, n-butyl, isobutyl, sec-butyl, tert-butyl, pentyl, neopentyl, hexyl, heptyl, 2-ethylhexyl, nonyl, undecyl, tridecyl, pentadecyl, heptadecyl, and icosyl; C₃-C₃₅ cyclic saturated hydrocarbyl groups such as cyclopentyl, cyclohexyl, 1-adamantyl, 2-adamantyl, 1-adamantylmethyl, norbornyl, norbornylmethyl, tricyclodecyl, tetracyclododecyl, tetracyclododecylmethyl, and dicyclohexylmethyl; C₂-C₃₅ unsaturated aliphatic hydrocarbyl groups such as 2-propenyl and 3-cyclohexenyl; C₆-C₃₅ aryl groups such as phenyl, 1-naphthyl, 2-naphthyl and 9-fluorenyl; and C₇-C₃₅ aralkyl groups such as benzyl and diphenylmethyl, and combinations thereof.

In the foregoing hydrocarbyl groups, some or all hydrogen may be substituted by a moiety containing a heteroatom such as oxygen, sulfur, nitrogen or halogen, or some constituent -CH₂- may be replaced by a moiety containing a heteroatom such as oxygen, sulfur or nitrogen, so that the group may contain a hydroxy, fluorine, chlorine, bromine, iodine, cyano, nitro, carbonyl, ether bond, ester bond, sulfonate ester bond, carbonate bond, lactone ring, sultone ring, carboxylic anhydride (-C(=O)-O-C(=O)-) or haloalkyl moiety. Examples of the heteroatom-containing hydrocarbyl group include tetrahydrofuryl, methoxymethyl, ethoxymethyl, methylthiomethyl, acetamidomethyl, trifluoroethyl, (2-methoxyethoxy)methyl, acetoxymethyl, 2-carboxy-1-cyclohexyl, 2-oxopropyl, 4-oxo-1-adamantyl, and 3-oxocyclohexyl.

In formula (B6-1-1), L^{a1} is a single bond, ether bond, ester bond, sulfonate ester bond, carbonate bond or carbamate bond. From the aspect of synthesis, an ether bond or ester bond is preferred, with the ester bond being more preferred.

Examples of the anion having formula (B6-1) are shown below, but not limited thereto. Herein Q¹ is as defined above.

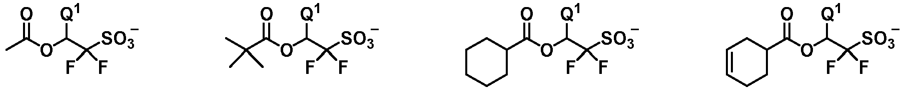

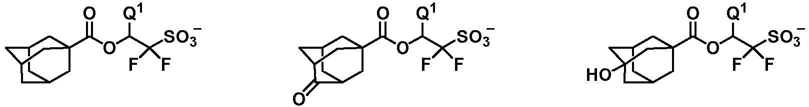

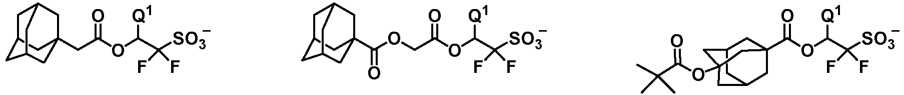

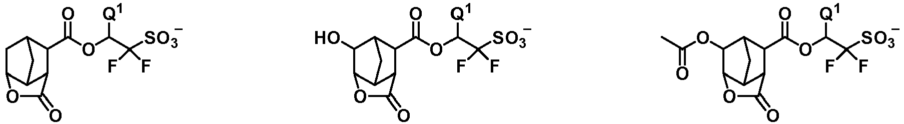

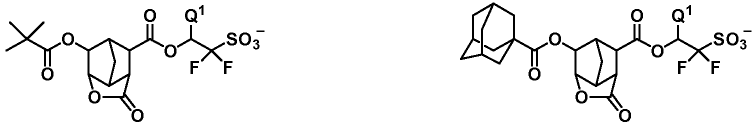

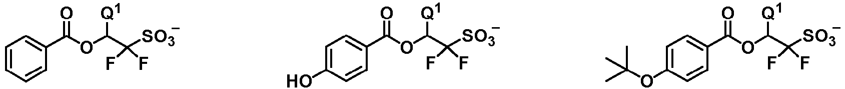

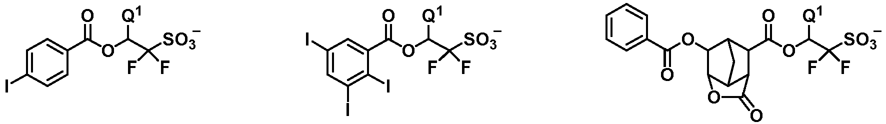

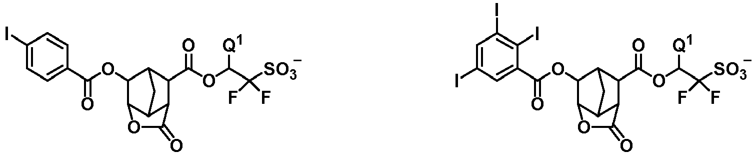

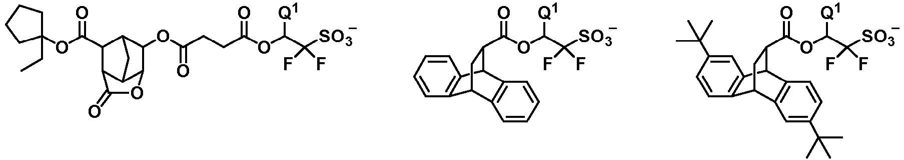

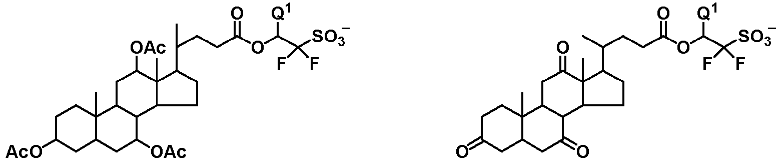

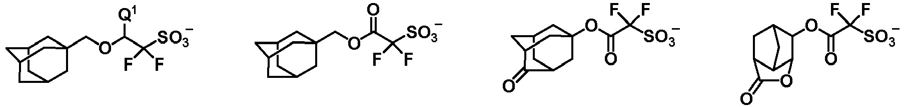

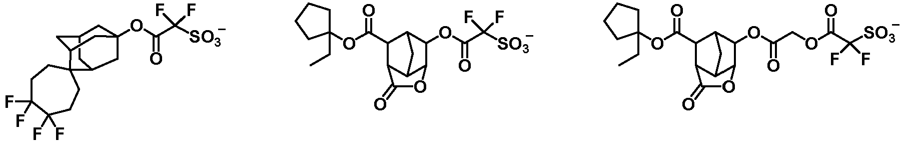

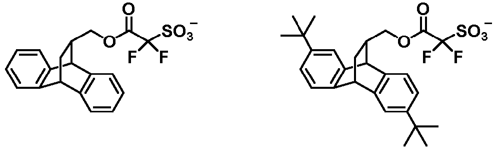

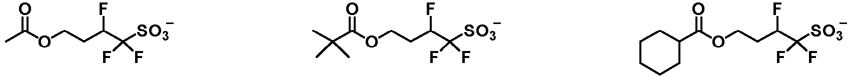

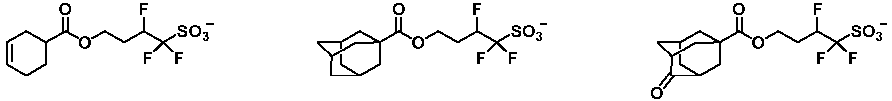

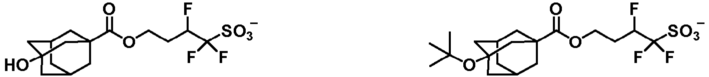

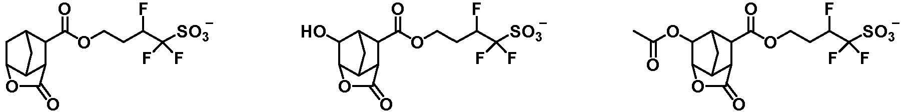

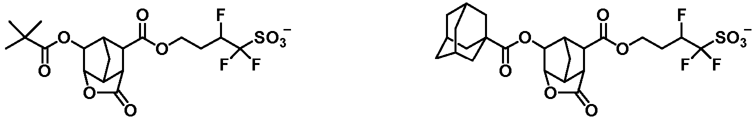

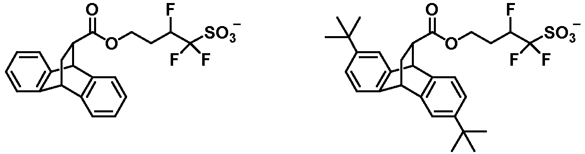

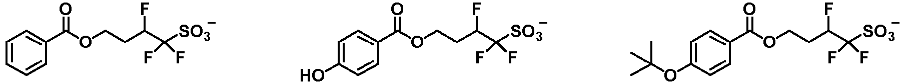

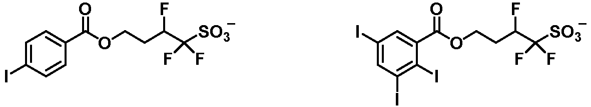

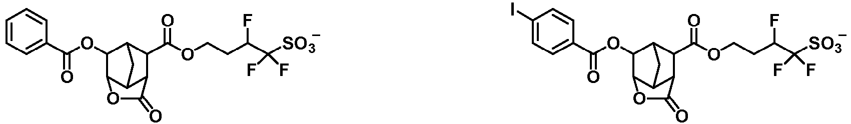

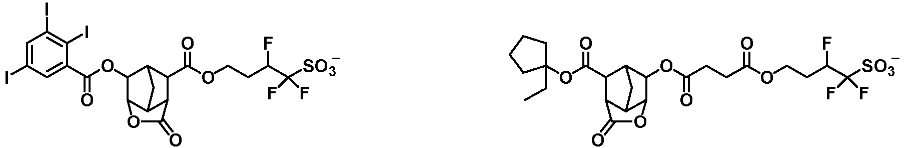

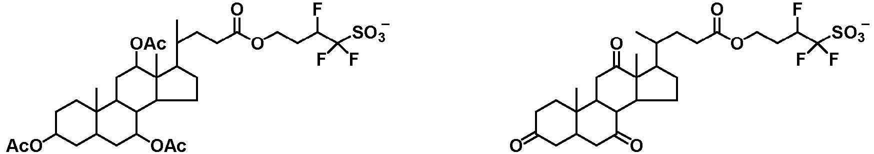

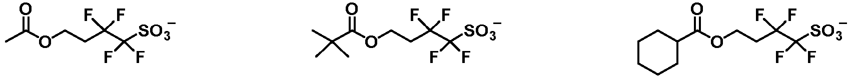

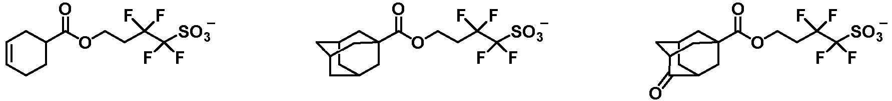

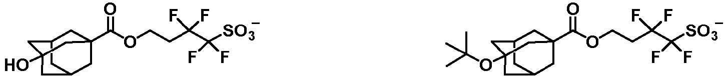

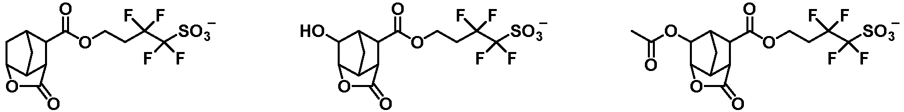

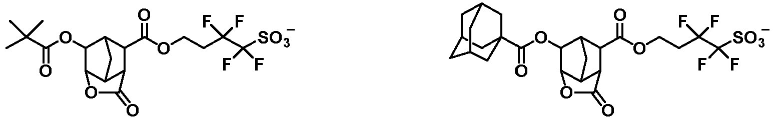

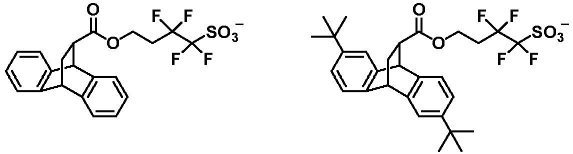

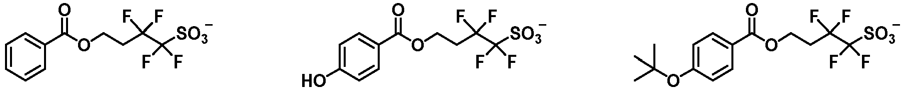

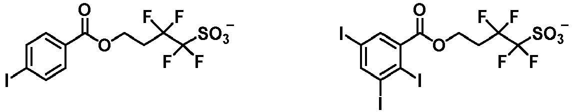

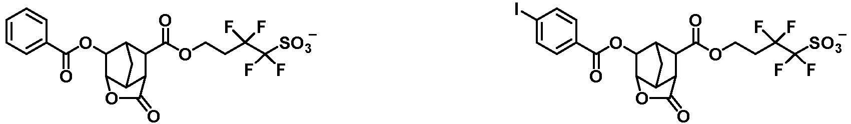

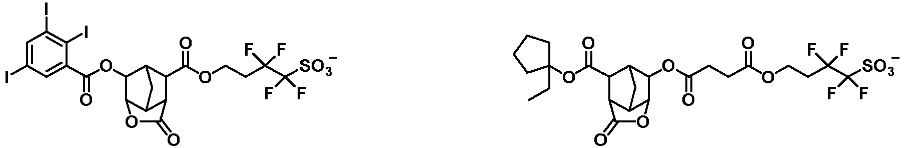

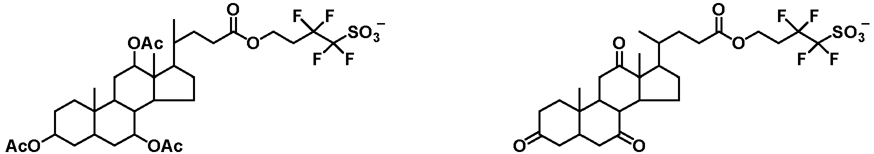

In formula (B6-2), R^{fb1} and R^{fb2} are each independently fluorine or a C₁-C₄₀ hydrocarbyl group which may contain a heteroatom. The hydrocarbyl group may be saturated or unsaturated and straight, branched or cyclic. Examples thereof are as exemplified above for R^{fa1} in formula (B6-1-1). Preferably R^{fb1} and R^{fb2} are fluorine or C₁-C₄ straight fluorinated alkyl groups. Also, R^{fb1} and R^{fb2} may bond together to form a ring with the linkage: -CF₂-SO₂-N⁻-SO₂-CF₂- to which they are attached. It is preferred that a combination of R^{fb1} and R^{fb2} be a fluorinated ethylene or fluorinated propylene group.

In formula (B6-3), R^{fc1}, R^{fc2} and R^{fc3} are each independently fluorine or a C₁-C₄₀ hydrocarbyl group which may contain a heteroatom. The hydrocarbyl group may be saturated or unsaturated and straight, branched or cyclic. Examples thereof are as exemplified for R^{fa1} in formula (B6-1-1). Preferably R^{fc1}, R^{fc2} and R^{fc3} are fluorine or C₁-C₄ straight fluorinated alkyl groups. Also, R^{fc1} and R^{fc2} may bond together to form a ring with the linkage: -CF₂-SO₂-C⁻-SO₂-CF₂- to which they are attached. It is preferred that a combination of R^{fc1} and R^{fc2} be a fluorinated ethylene or fluorinated propylene group.

In formula (B6-4), R^{fd} is a C₁-C₄₀ hydrocarbyl group which may contain a heteroatom. The hydrocarbyl group may be saturated or unsaturated and straight, branched or cyclic. Examples thereof are as exemplified above for R^{fa1} in formula (B6-1-1).

Examples of the anion having formula (B6-4) are shown below, but not limited thereto.

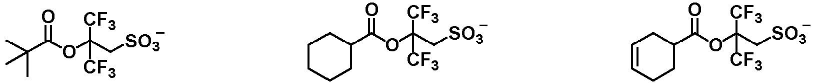

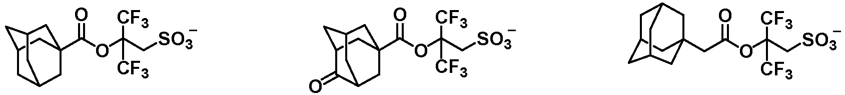

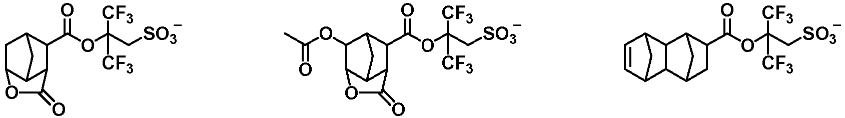

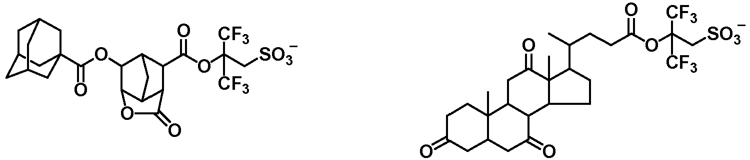

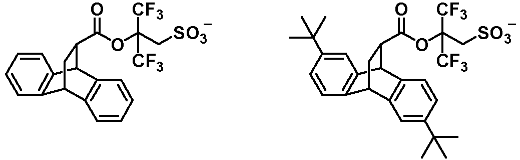

Anions having an iodized or brominated aromatic ring are also useful as the non-nucleophilic counter ion. These anions have the formula (B6-5).

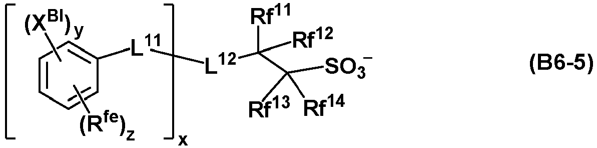

In formula (B6-5), x is 1, 2 or 3, y is 1, 2, 3, 4 or 5, z is 0, 1, 2 or 3, and y+z is from 1 to 5. Preferably, y is 1, 2 or 3, more preferably 2 or 3, and z is 0, 1 or 2.

In formula (B6-5), X^{BI} is iodine or bromine. A plurality of X^{BI} may be identical or different when x and/or y is 2 or more.

In formula (B6-5), L¹¹ is a single bond, ether bond, ester bond, or a C₁-C₆ saturated hydrocarbylene group which may contain an ether bond or ester bond. The saturated hydrocarbylene group may be straight, branched or cyclic.

In formula (B6-5), L¹² is a single bond or a C₁-C₂₀ divalent linking group when x=1, or a C₁-C₂₀ (x+1)-valent linking group when x=2 or 3. The linking group may contain an oxygen, sulfur or nitrogen atom.

In formula (B6-5), R^{fe} is hydroxy, carboxy, fluorine, chlorine, bromine, amino group, or a C₁-C₂₀ hydrocarbyl, C₁-C₂₀ hydrocarbyloxy, C₂-C₂₀ hydrocarbylcarbonyl, C₂-C₂₀ hydrocarbyloxycarbonyl, C₂-C₂₀ hydrocarbylcarbonyloxy, or C₁-C₂₀ hydrocarbylsulfonyloxy group, which may contain fluorine, chlorine, bromine, hydroxy, amino or ether bond, or -N(R^{feA})(R^{feB}), -N(R^{feC})-C(=O)-R^{feD} or -N(R^{feC})-C(=O)-O-R^{feD}. R^{feA} and R^{feB} are each independently hydrogen or a C₁-C₆ saturated hydrocarbyl group. R^{feC} is hydrogen, or a C₁-C₆ saturated hydrocarbyl group which may contain halogen, hydroxy, C₁-C₆ saturated hydrocarbyloxy, C₂-C₆ saturated hydrocarbylcarbonyl or C₂-C₆ saturated hydrocarbylcarbonyloxy moiety. R^{feD} is a C₁-C₁₆ aliphatic hydrocarbyl group, C₆-C₁₂ aryl group or C₇-C₁₅ aralkyl group, which may contain halogen, hydroxy, C₁-C₆ saturated hydrocarbyloxy, C₂-C₆ saturated hydrocarbylcarbonyl or C₂-C₆ saturated hydrocarbylcarbonyloxy moiety. The aliphatic hydrocarbyl group may be saturated or unsaturated and straight, branched or cyclic. The hydrocarbyl, hydrocarbyloxy, hydrocarbylcarbonyl, hydrocarbyloxycarbonyl, hydrocarbylcarbonyloxy, and hydrocarbylsulfonyloxy groups may be straight, branched or cyclic. A plurality of R^{fe} may be identical or different when x and/or z is 2 or more.

Of these, R^{fe} is preferably hydroxy, -N(R^{feC})-C(=O)-R^{feD}, -N(R^{feC})-C(=O)-O-R^{feD}, fluorine, chlorine, bromine, methyl or methoxy.

In formula (B6-5), Rf¹¹ to Rf¹⁴ are each independently hydrogen, fluorine or trifluoromethyl, at least one of Rf¹¹ to Rf¹⁴ is fluorine or trifluoromethyl. Rf¹¹ and Rf¹², taken together, may form a carbonyl group. More preferably, both Rf¹³ and Rf¹⁴ are fluorine.

Examples of the anion having formula (B6-5) are shown below, but not limited thereto. X^{BI} is as defined above.

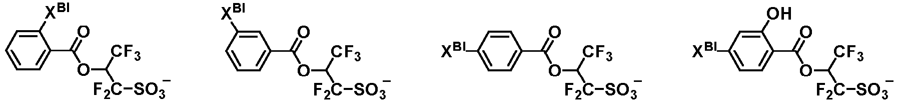

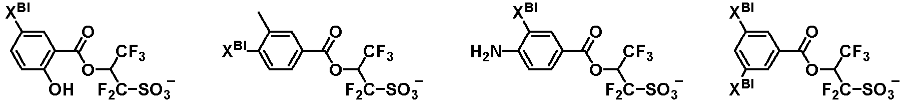

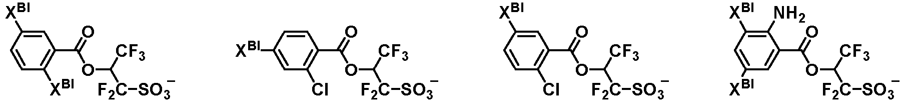

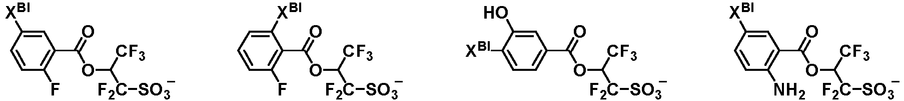

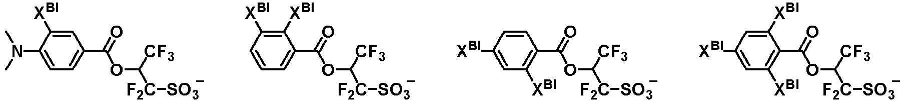

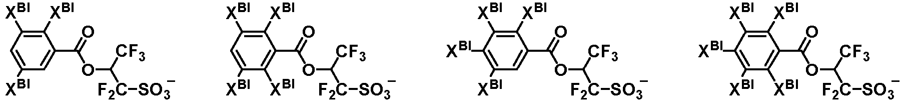

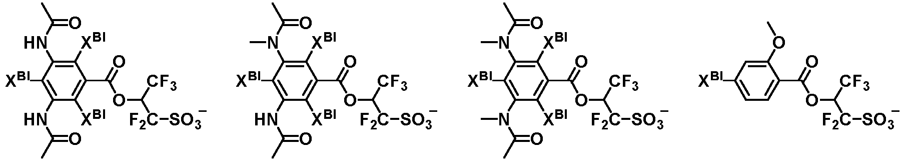

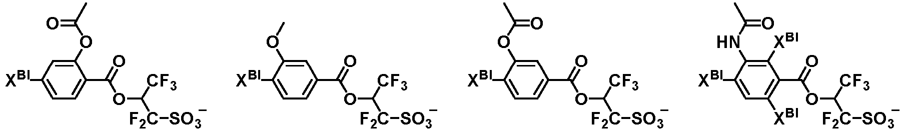

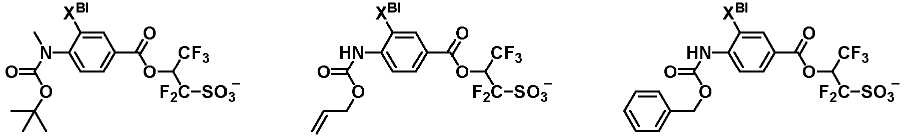

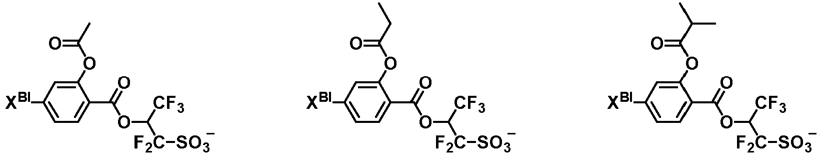

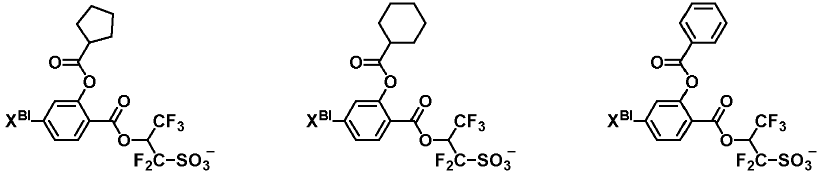

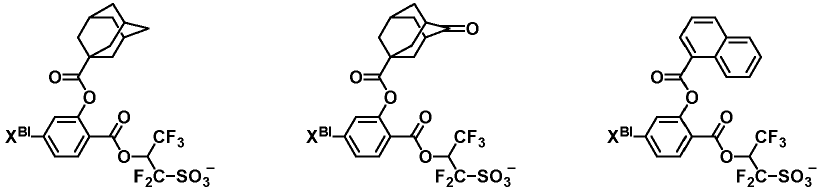

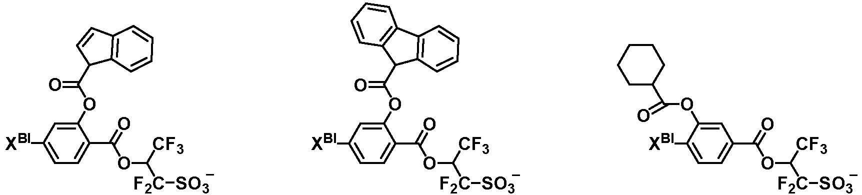

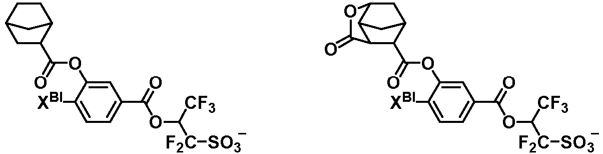

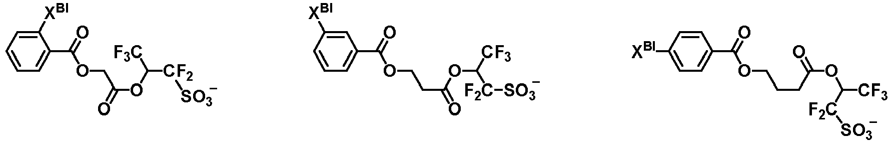

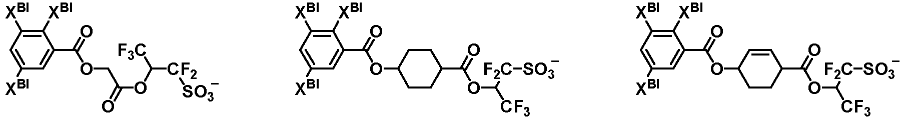

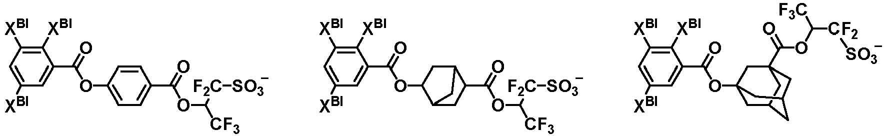

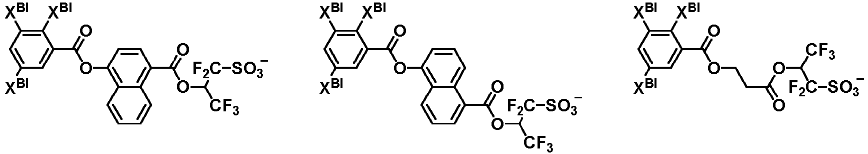

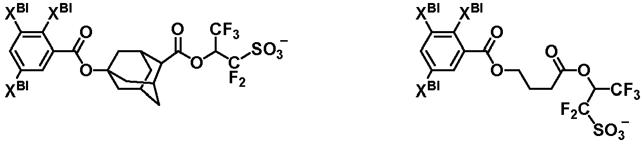

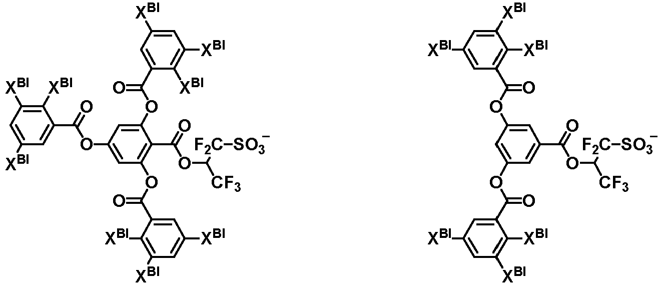

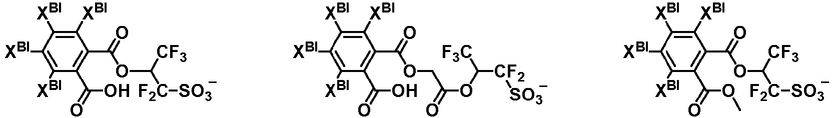

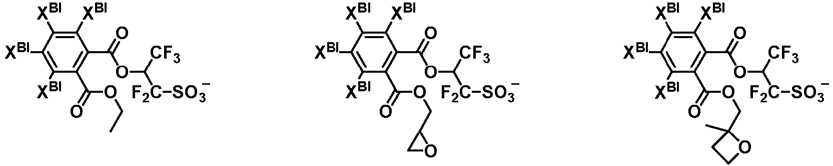

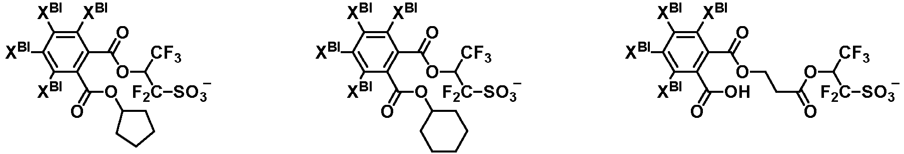

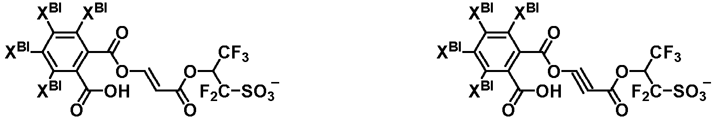

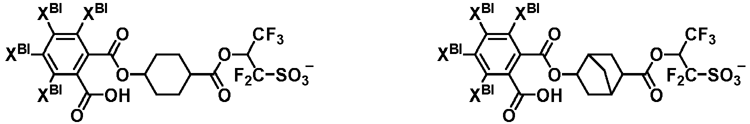

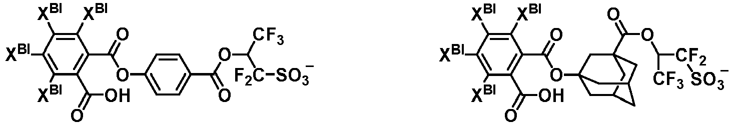

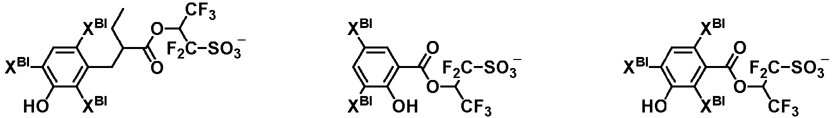

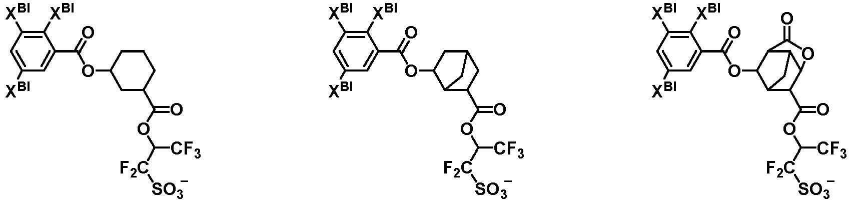

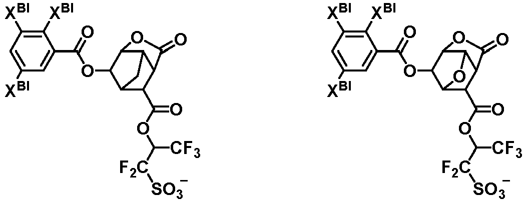

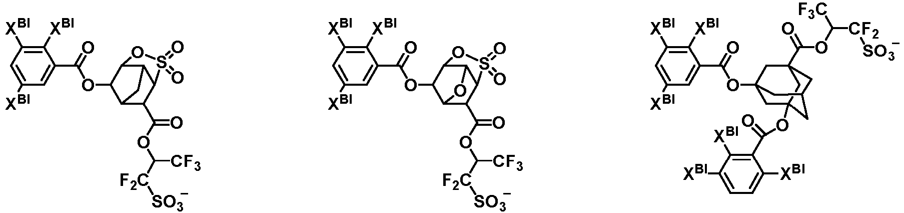

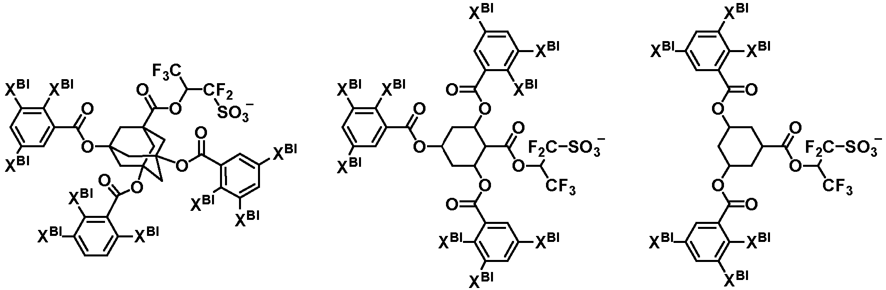

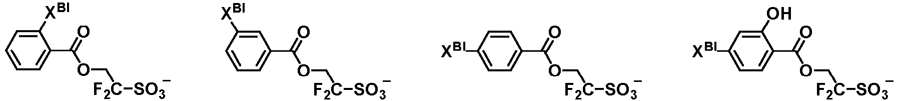

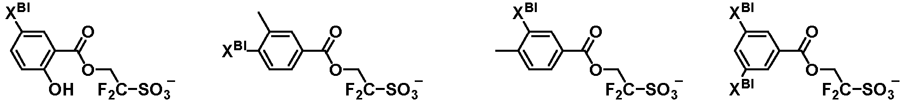

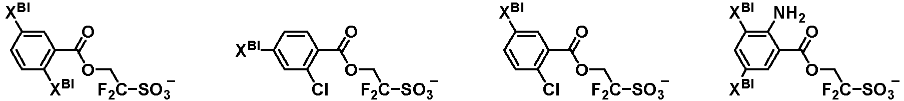

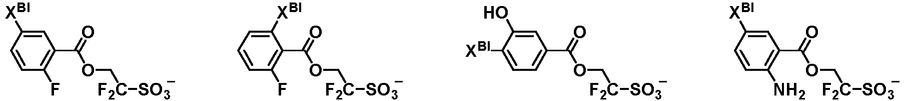

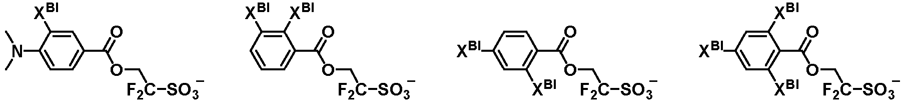

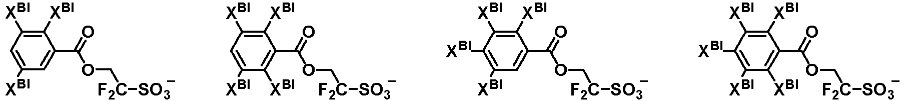

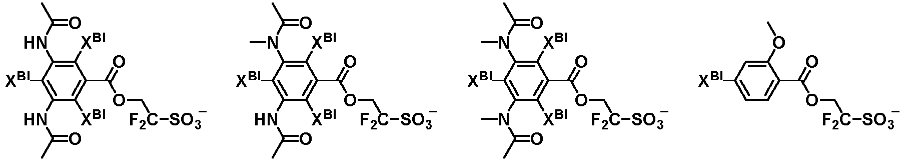

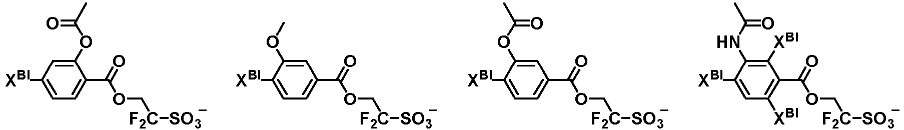

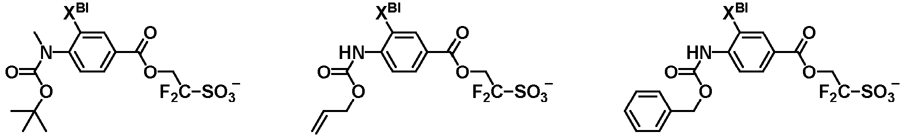

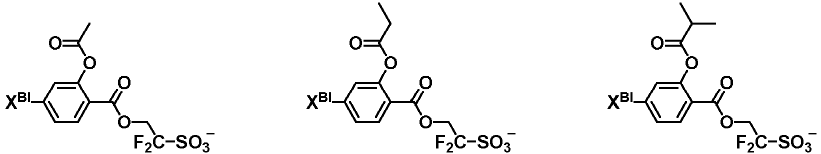

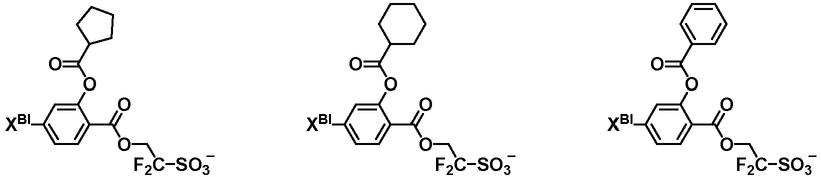

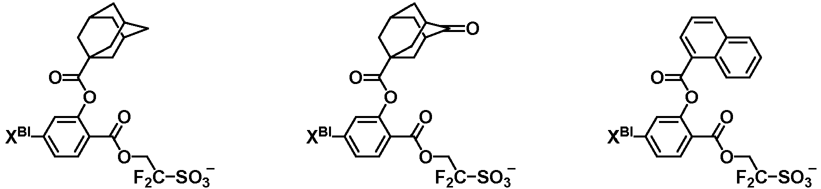

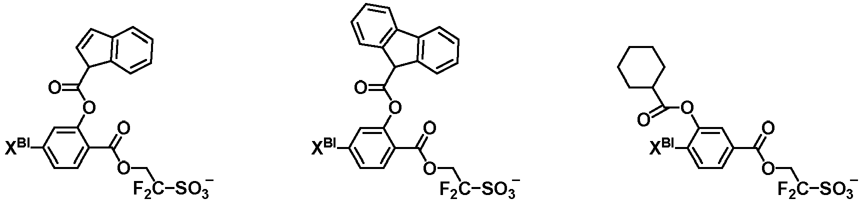

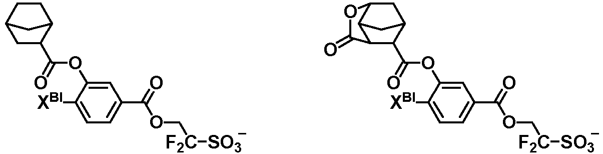

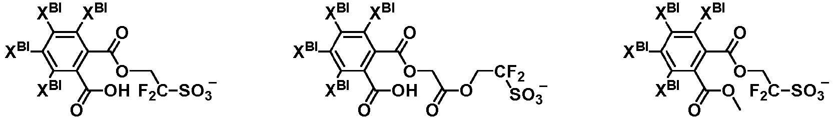

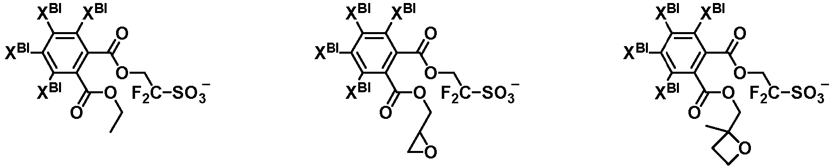

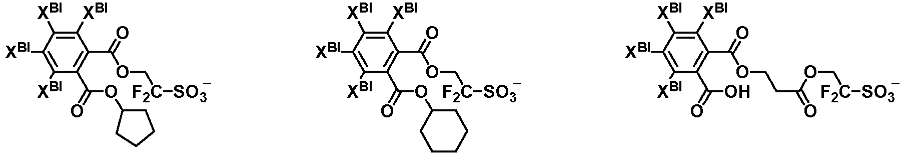

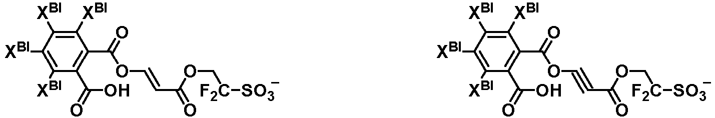

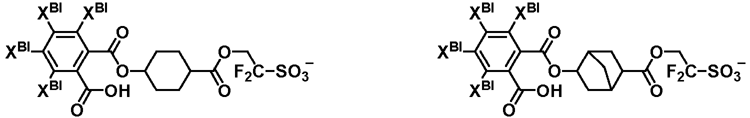

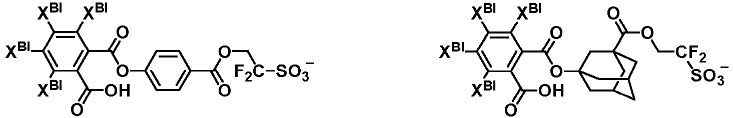

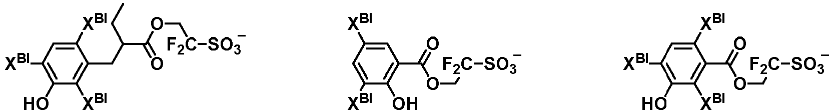

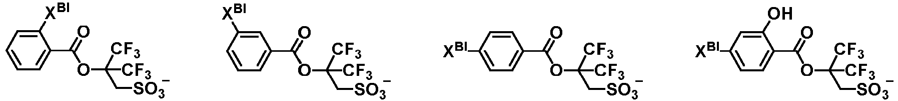

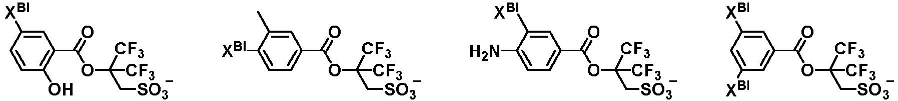

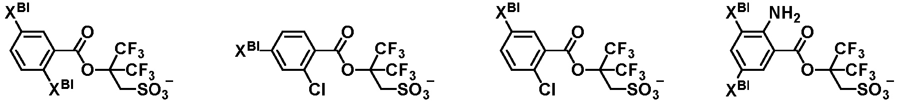

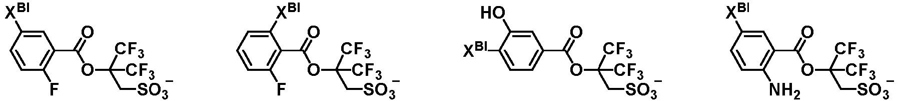

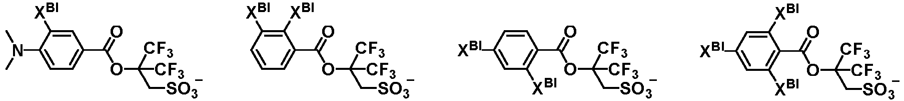

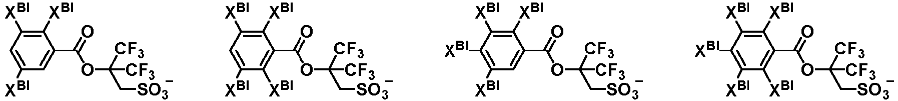

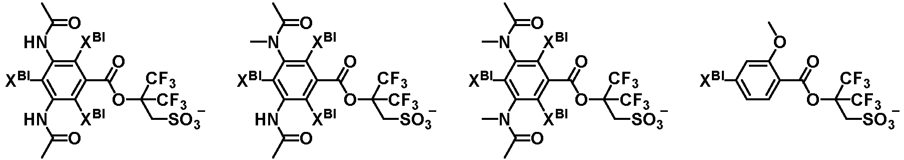

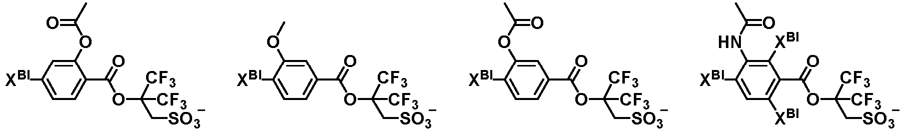

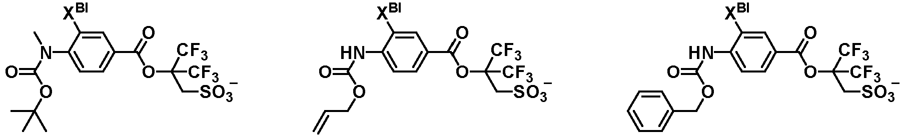

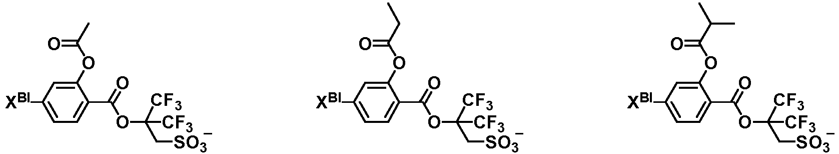

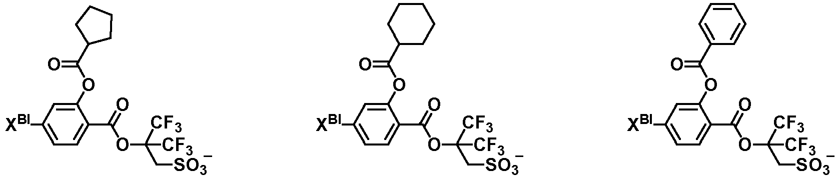

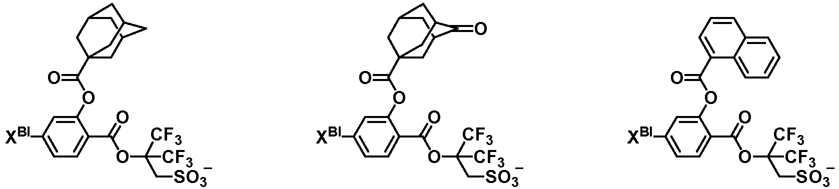

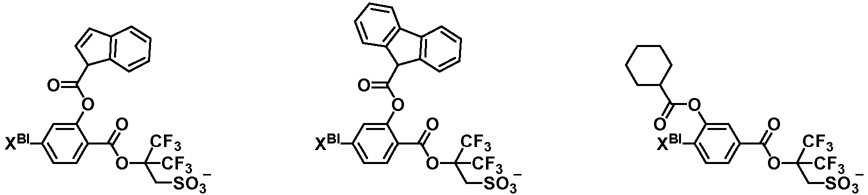

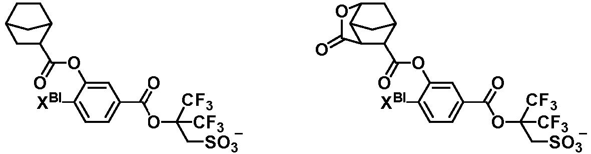

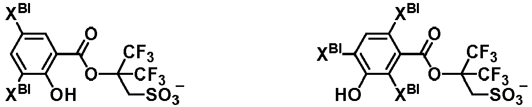

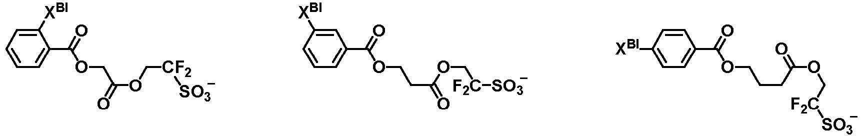

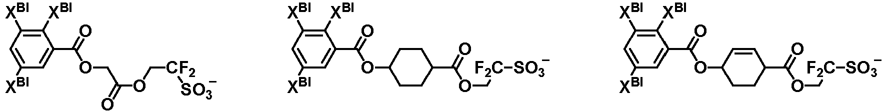

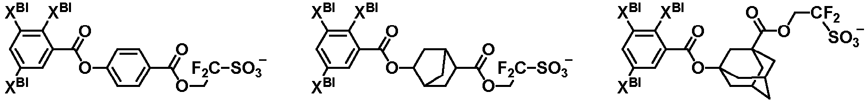

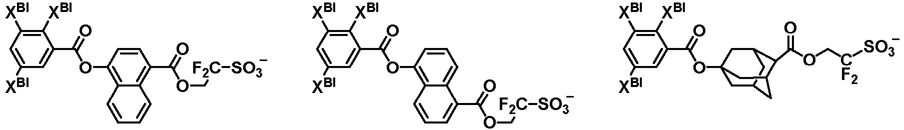

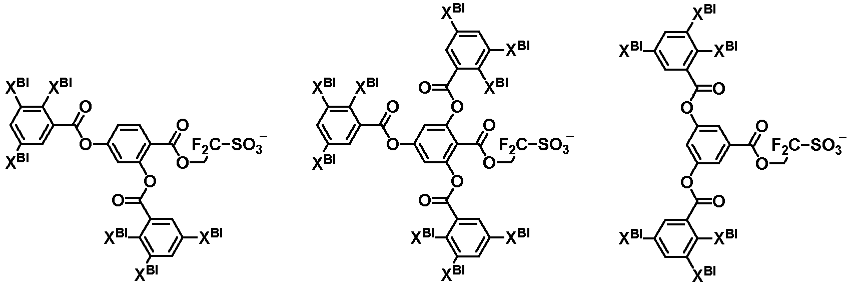

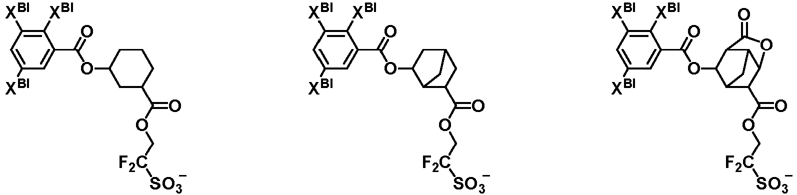

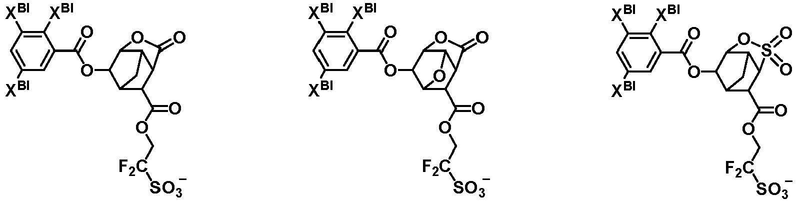

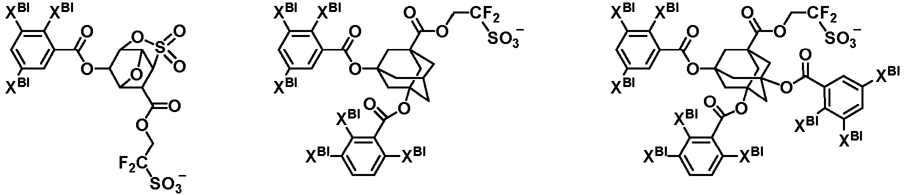

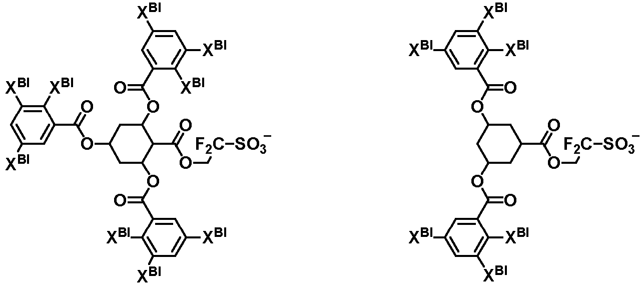

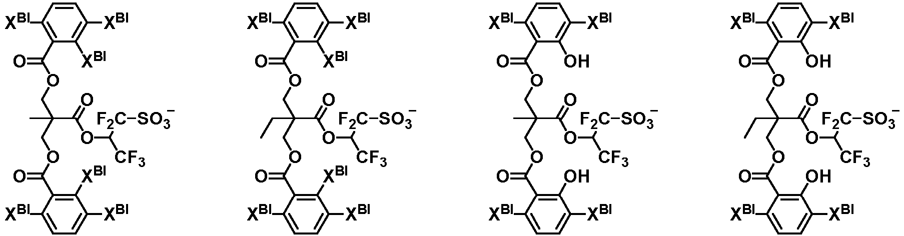

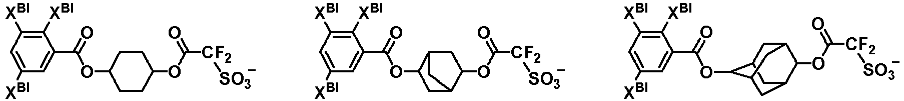

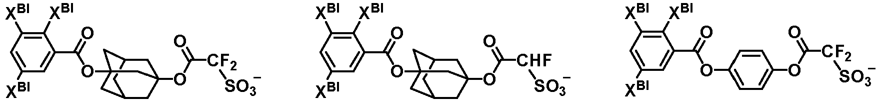

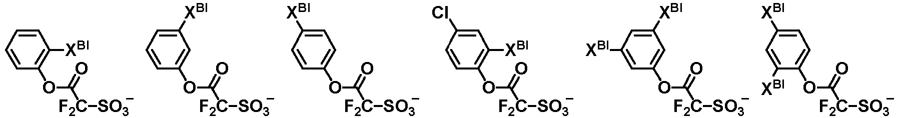

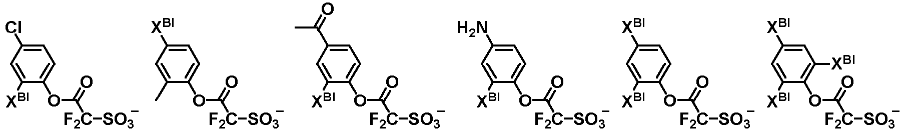

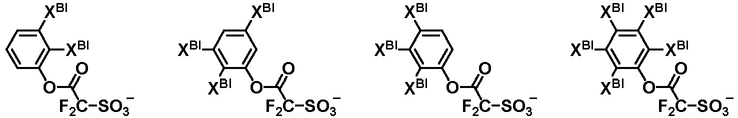

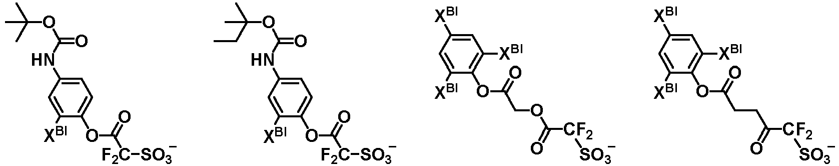

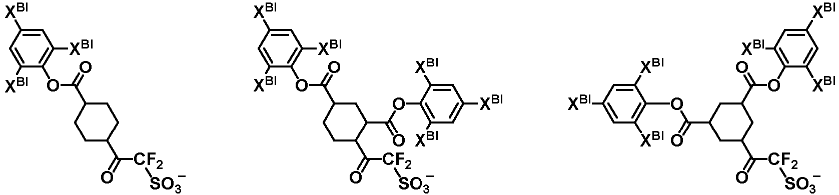

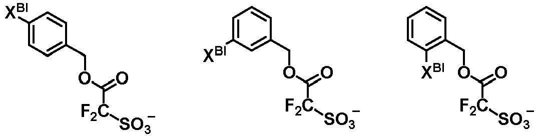

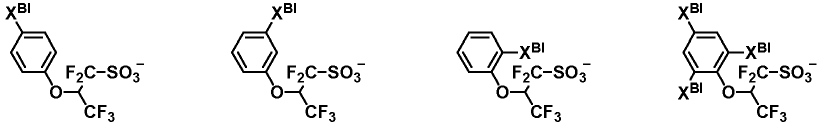

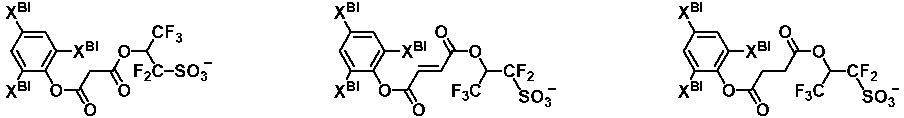

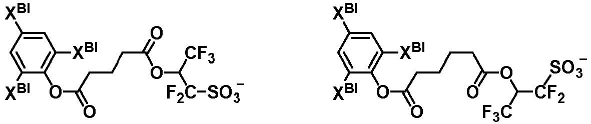

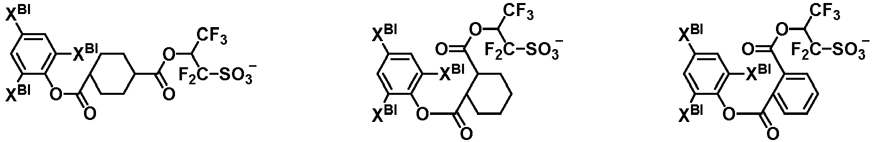

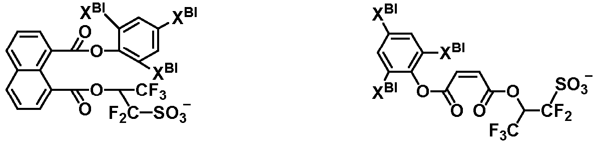

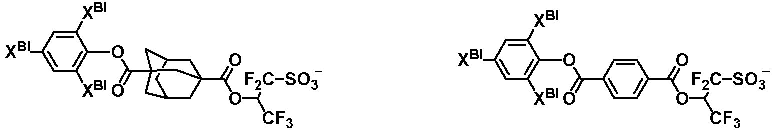

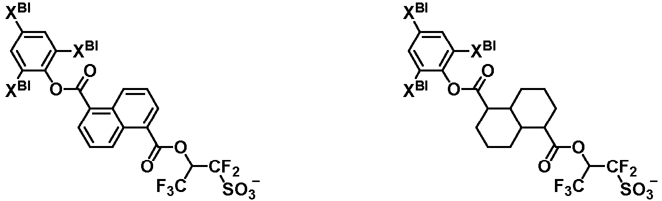

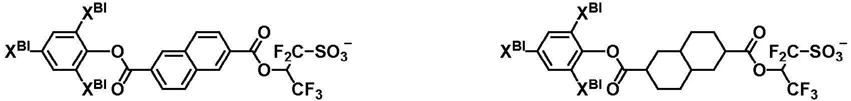

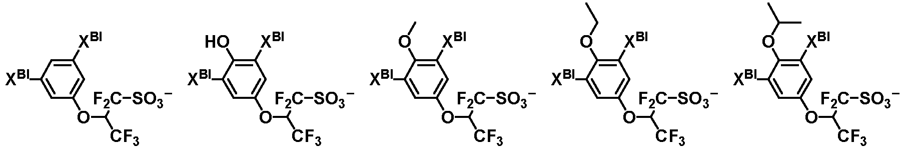

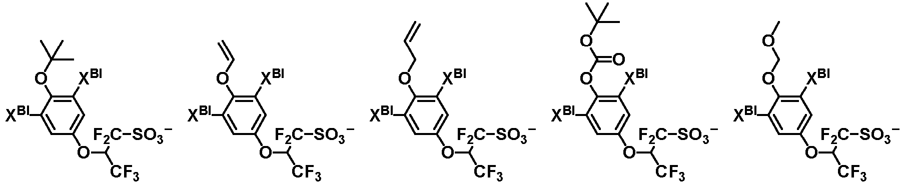

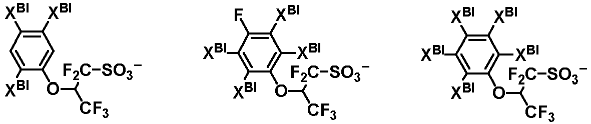

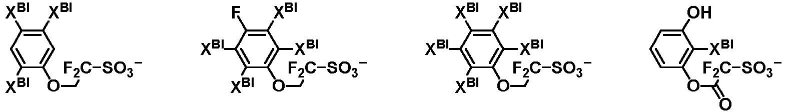

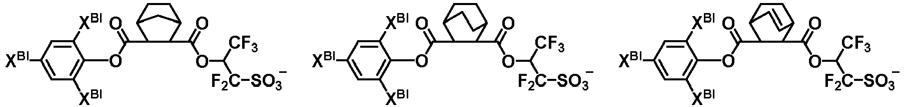

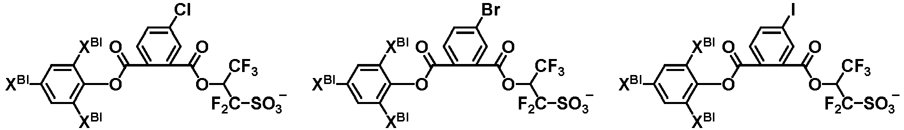

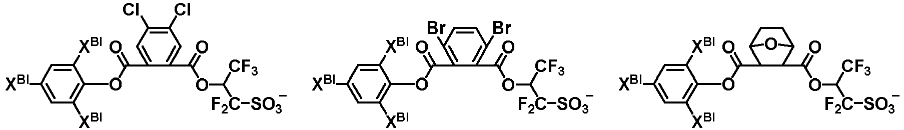

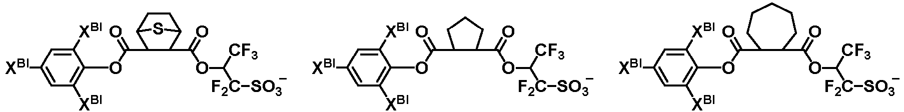

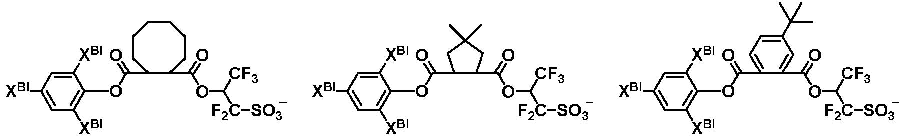

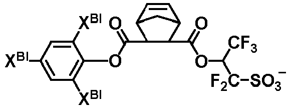

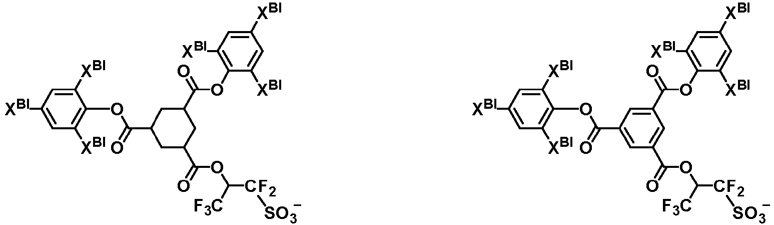

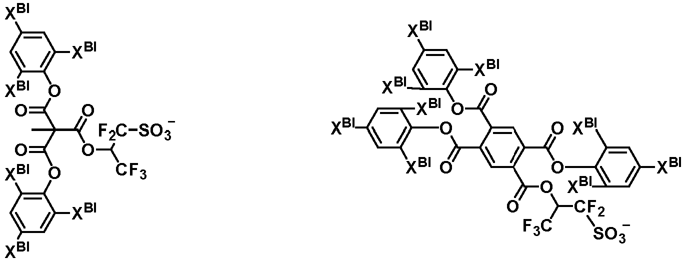

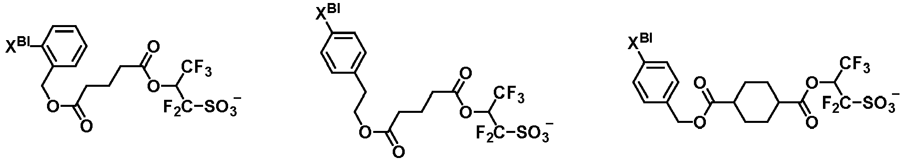

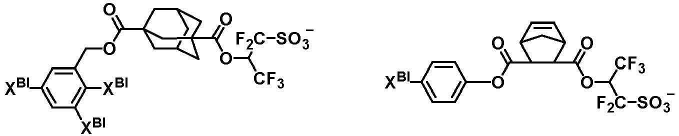

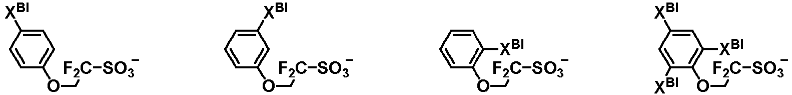

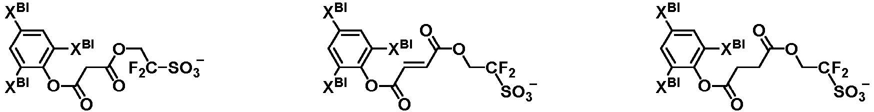

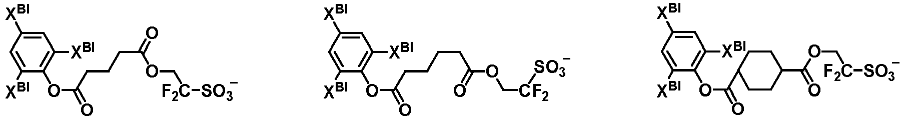

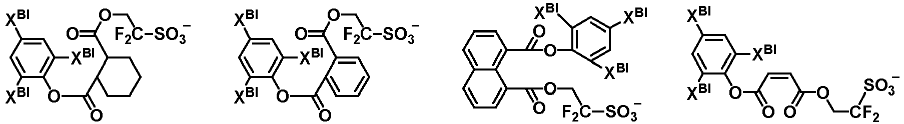

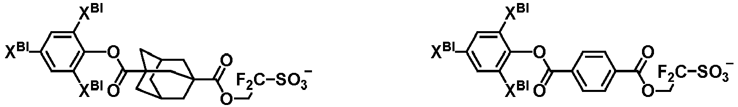

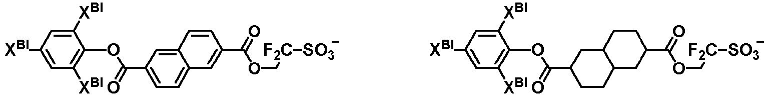

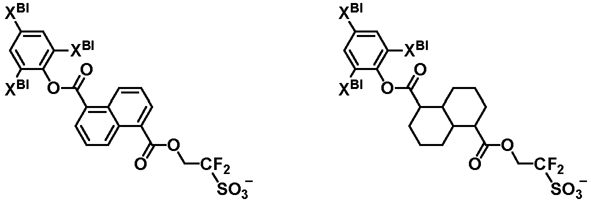

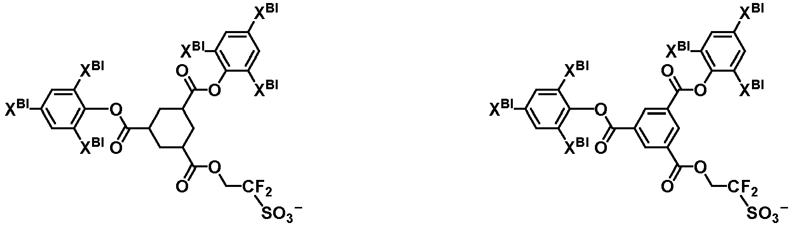

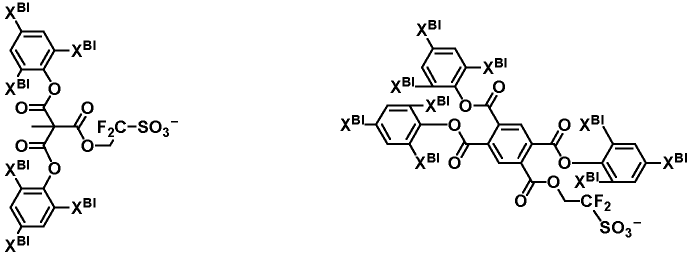

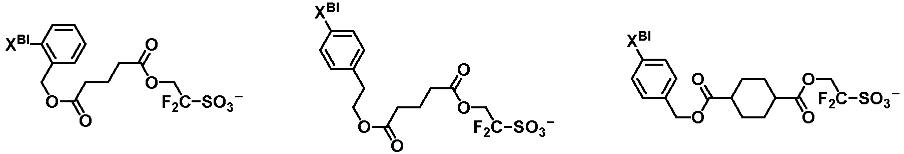

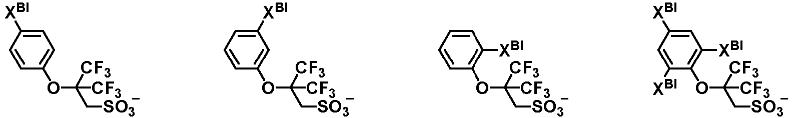

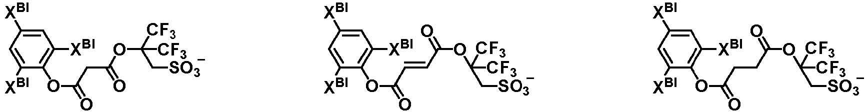

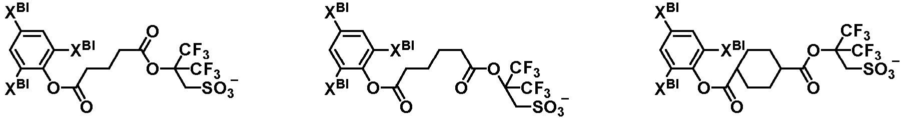

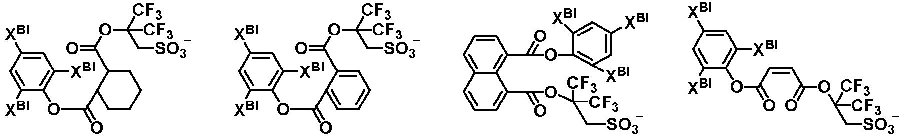

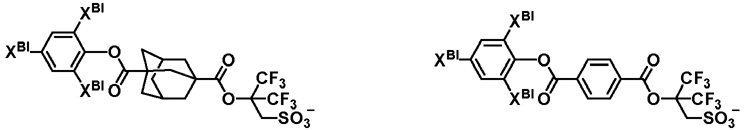

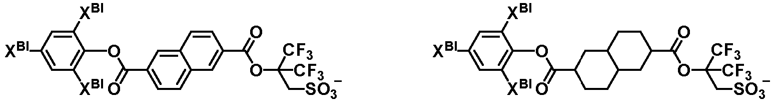

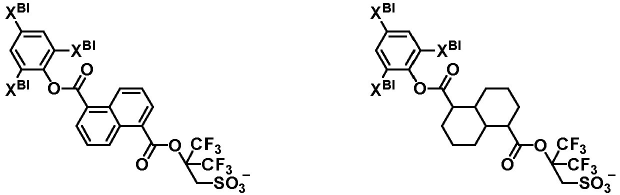

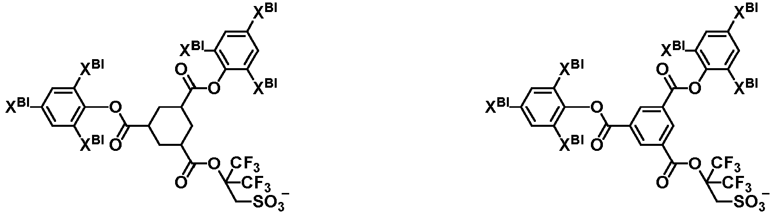

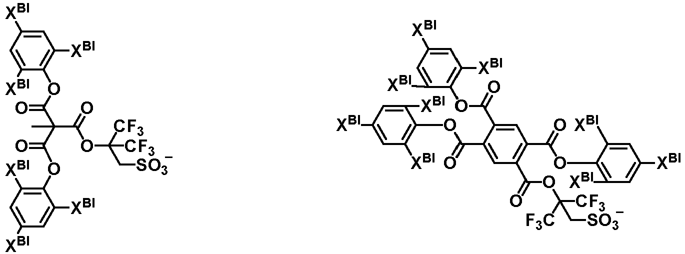

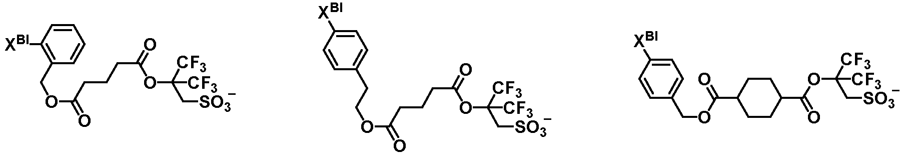

Other useful examples of the non-nucleophilic counter ion include fluorobenzenesulfonic acid anions having an iodized aromatic ring bonded thereto as described in JP 6648726, anions having an acid-catalyzed decomposition mechanism as described in WO 2021/200056 and JP-A 2021-070692, anions having a cyclic ether group as described in JP-A 2018-180525 and JP-A 2021-035935, and anions as described in JP-A 2018-092159.

Further useful examples of the non-nucleophilic counter ion include fluorine-free bulky benzenesulfonic acid anions as described in JP-A 2006-276759, JP-A 2015-117200, JP-A 2016-065016, and JP-A 2019-202974; fluorine-free benzenesulfonic acid or alkylsulfonic acid anions having an iodized aromatic group bonded thereto as described in JP 6645464.

Also useful are bissulfonic acid anions as described in JP-A 2015-206932, sulfonamide or sulfonimide anions having sulfonic acid side and different side as described in WO 2020/158366, and anions having a sulfonic acid side and a carboxylic acid side as described in JP-A 2015-024989.

In formulae (B7) and (B8), g1 and g2 are each independently 0, 1, 2 or 3, preferably 1.

In formula (B9), h1 is 0 or 1, h2 is 0, 1, 2, 3 or 4, h3 is 0, 1, 2, 3 or 4, with the proviso that when h1=0, 0 ≤ h2+h3 ≤ 4, and when h1=1, 0 ≤ h2+h3 ≤ 6.

In formulae (B7), (B8) and (B9), L¹ is a single bond, ether bond, ester bond, carbonyl, sulfonate ester bond, carbonate bond or carbamate bond. From the aspect of synthesis, an ether bond, ester bond or carbonyl is preferred, with the ester bond or carbonyl being more preferred.

In formula (B7), Rf¹ and Rf² are each independently fluorine or a C₁-C₆ fluorinated saturated hydrocarbyl group. It is preferred that both Rf¹ and Rf² be fluorine because the generated acid has a higher acid strength. Rf³ and Rf⁴ are each independently hydrogen, fluorine or a C₁-C₆ fluorinated saturated hydrocarbyl group. It is preferred for solvent solubility that at least one of Rf³ and Rf⁴ be trifluoromethyl.

In formula (B8), Rf⁵ and Rf⁶ are each independently hydrogen, fluorine or a C₁-C₆ fluorinated saturated hydrocarbyl group. It is excluded that all Rf⁵ and Rf⁶ are hydrogen at the same time. It is preferred for solvent solubility that at least one of Rf⁵ and Rf⁶ be trifluoromethyl.

In formula (B9), Rf⁷ is fluorine, a C₁-C₆ fluorinated alkyl group, C₁-C₆ fluorinated alkoxy group, or C₁-C₆ fluorinated alkylthio group. Rf⁷ is preferably fluorine, trifluoromethyl, difluoromethyl, trifluoromethoxy, difluoromethoxy, trifluoromethylthio or difluoromethylthio, more preferably fluorine, trifluoromethyl or trifluoromethoxy. When h2 is 2, 3 or 4, a plurality of Rf⁷ may be identical or different.

In formula (B9), R⁴³ is halogen exclusive of fluorine or a C₁-C₂₀ hydrocarbyl group which may contain a heteroatom. The hydrocarbyl group may be saturated or unsaturated and straight, branched or cyclic. Examples thereof are as exemplified above for the hydrocarbyl group R¹ in formula (A), but not limited thereto. When h3 is 2, 3 or 4, a plurality of R⁴³ may be identical or different.

When h3 is 2, 3 or 4, a plurality of R⁴³ may bond together to form a ring with the carbon atom to which they are attached. Examples of the ring thus formed include cyclopropane, cyclobutane, cyclopentane, cyclohexane, norbornane, and adamantane rings. In the ring, some or all of the hydrogen atoms may be substituted by a moiety containing a heteroatom such as oxygen, sulfur, nitrogen or halogen, and some constituent -CH₂-may be replaced by a moiety containing a heteroatom such as oxygen, sulfur or nitrogen, so that the ring may contain a hydroxy, fluorine, chlorine, bromine, iodine, cyano, carbonyl, ether bond, ester bond, sulfonate ester bond, carbonate bond, lactone ring, sultone ring, carboxylic anhydride (-C(=O)-O-C(=O)-) or haloalkyl moiety.

Examples of the anion in repeat unit B7 are shown below, but not limited thereto. Herein R^{A} is as defined above.

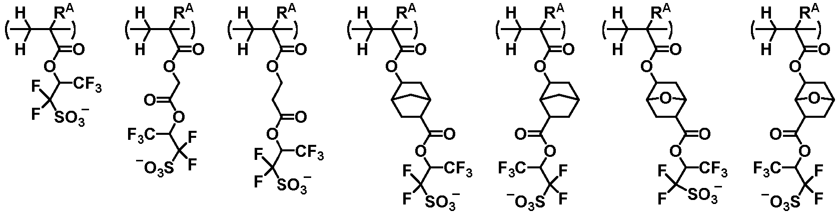

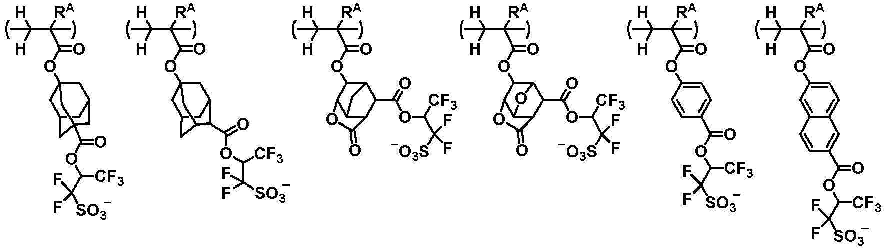

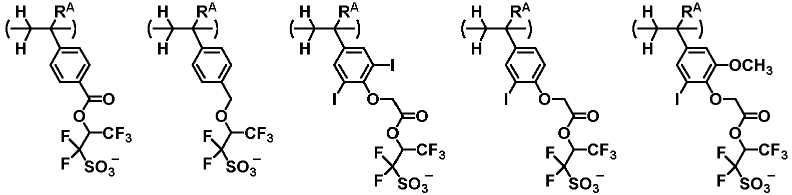

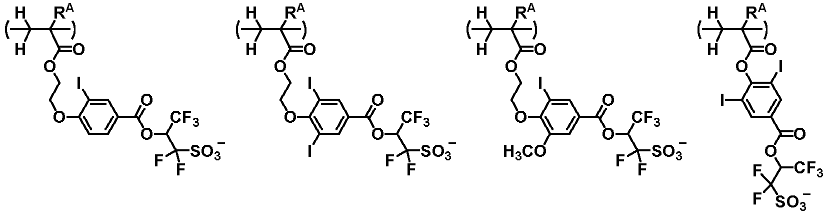

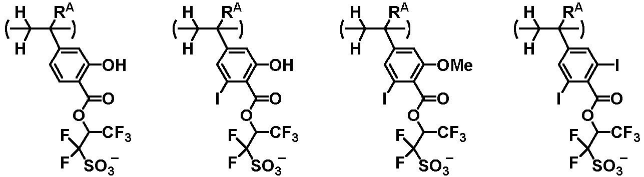

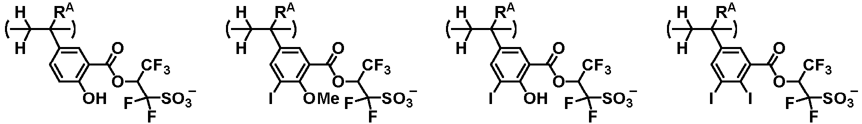

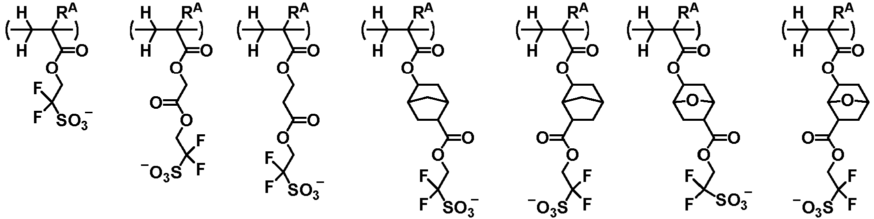

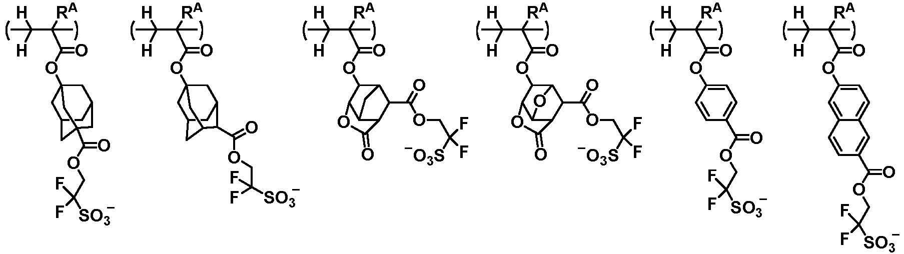

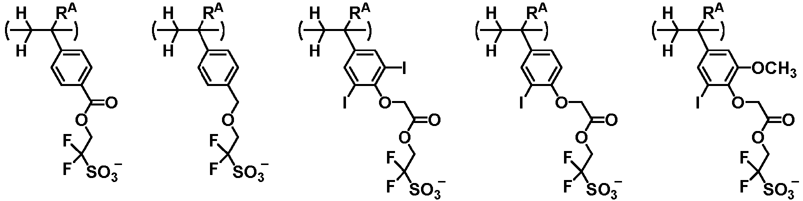

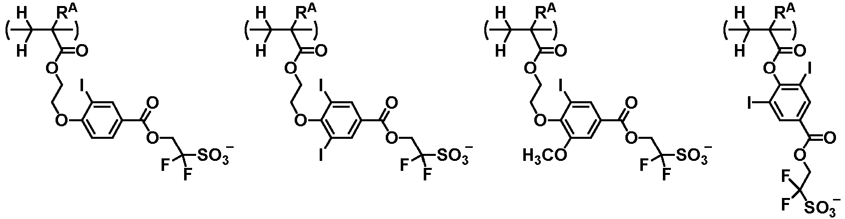

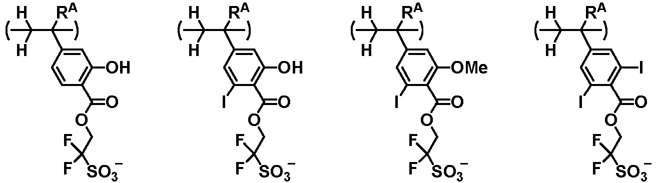

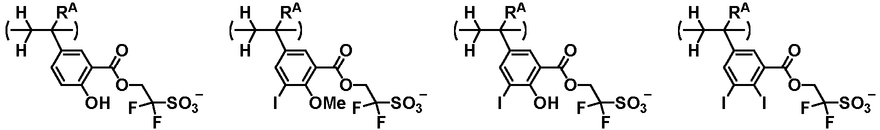

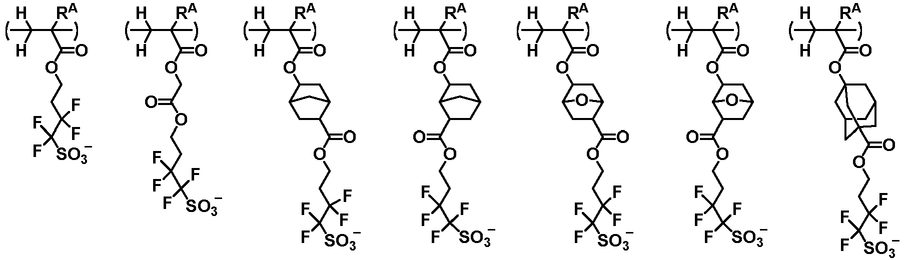

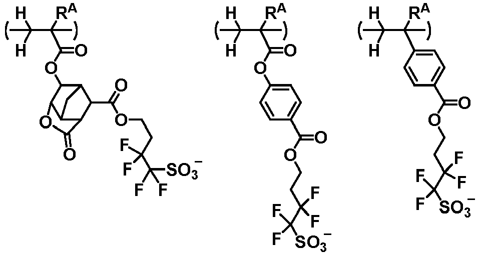

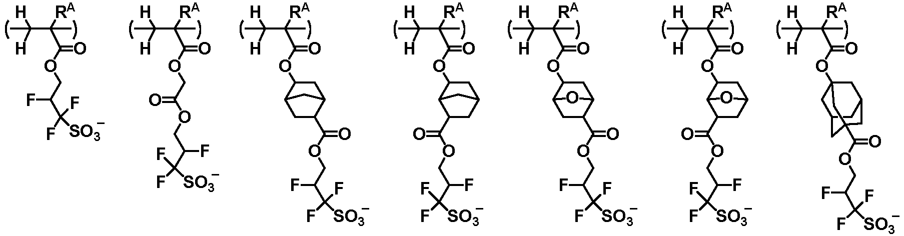

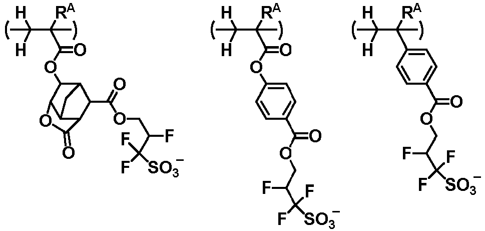

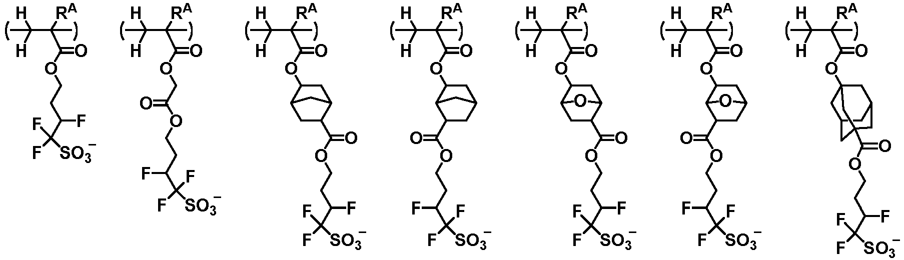

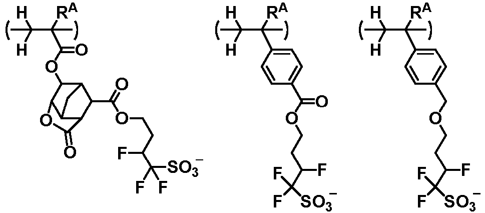

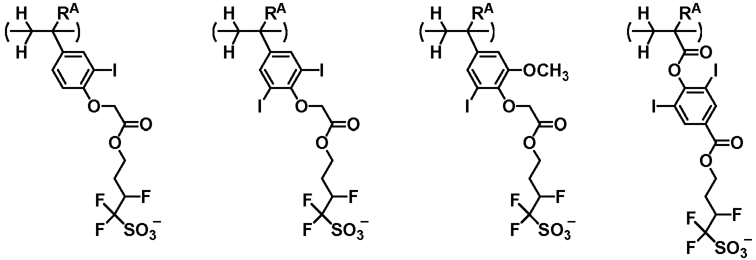

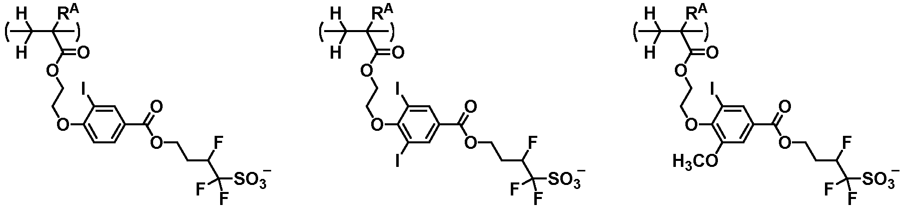

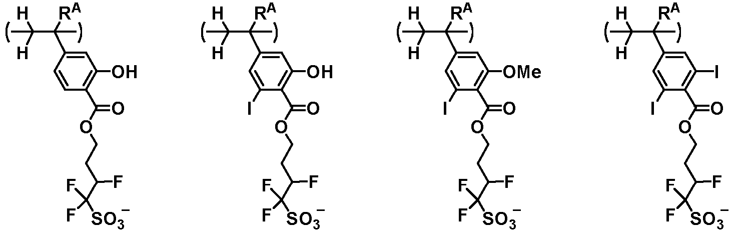

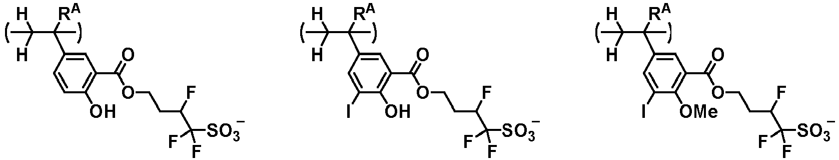

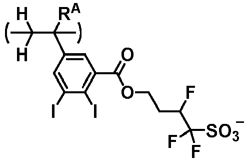

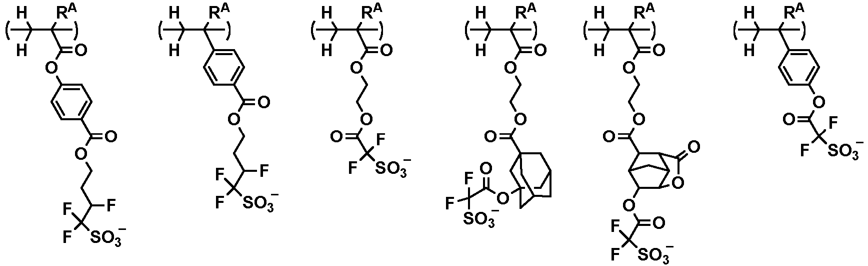

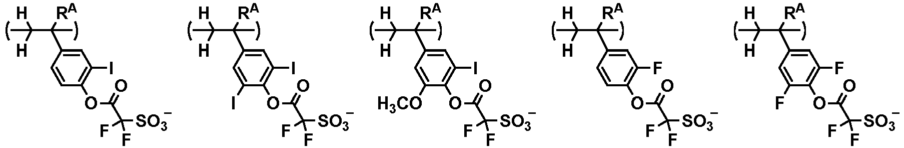

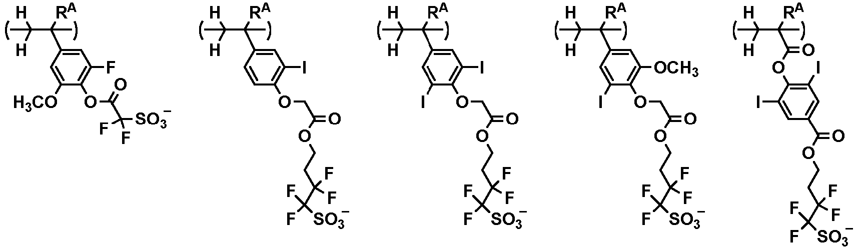

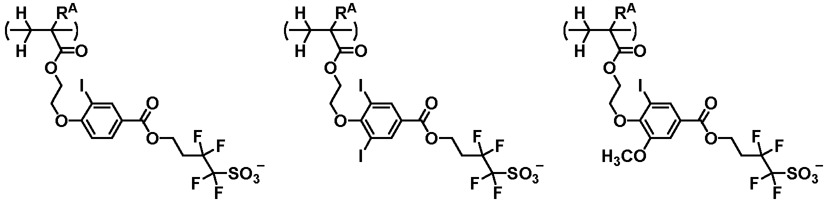

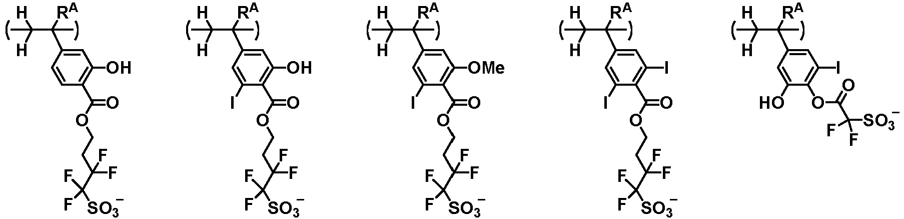

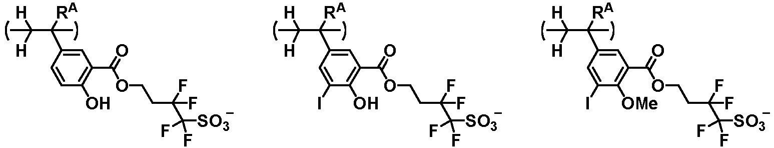

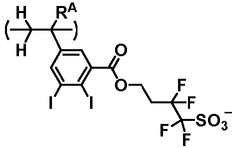

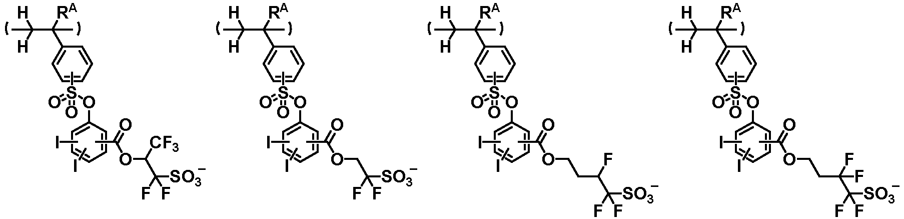

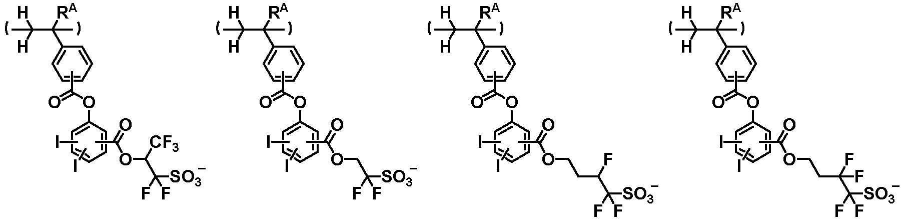

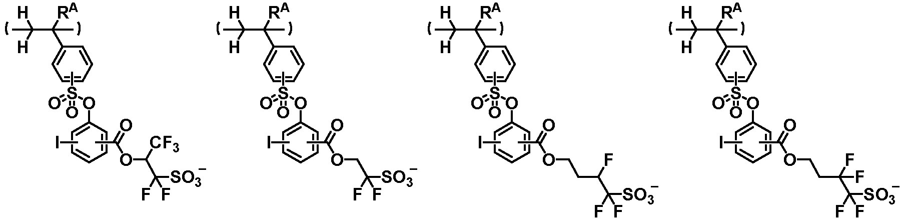

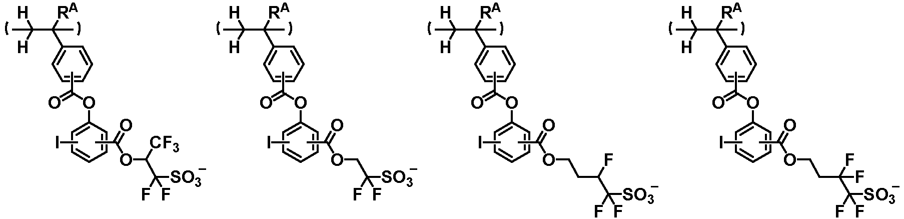

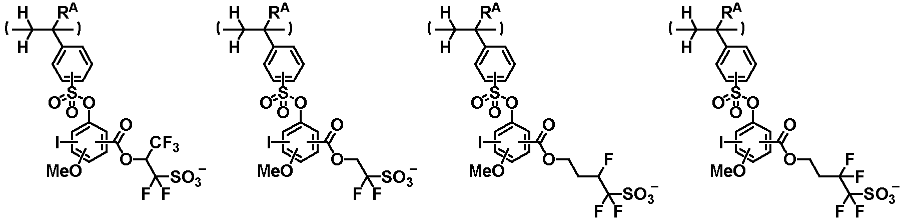

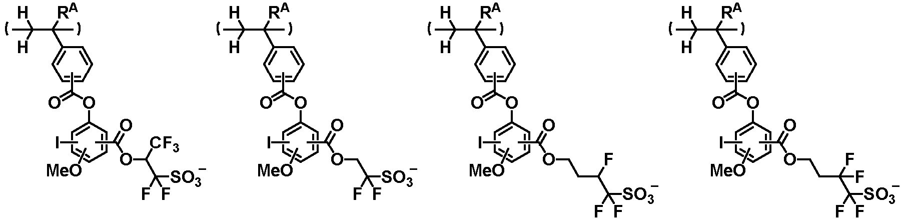

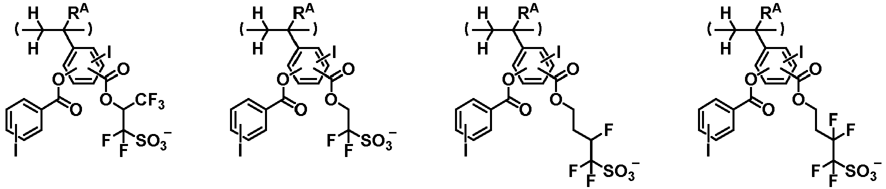

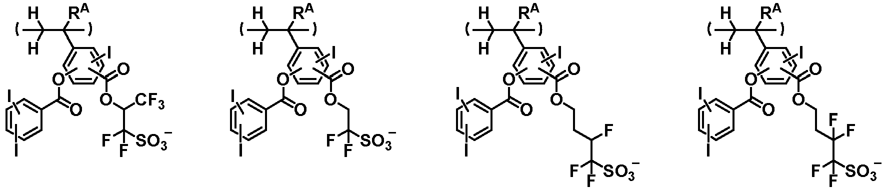

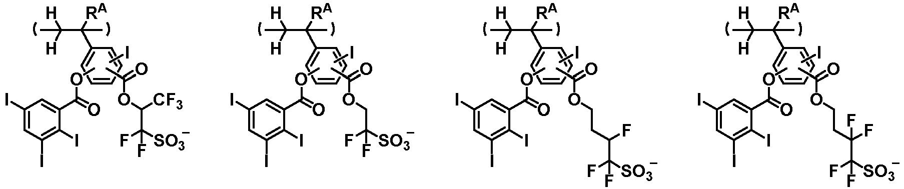

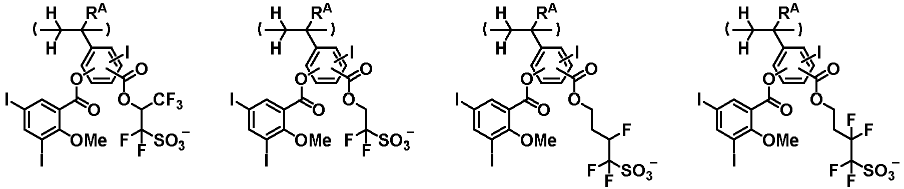

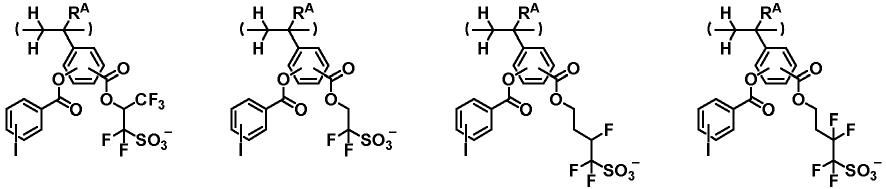

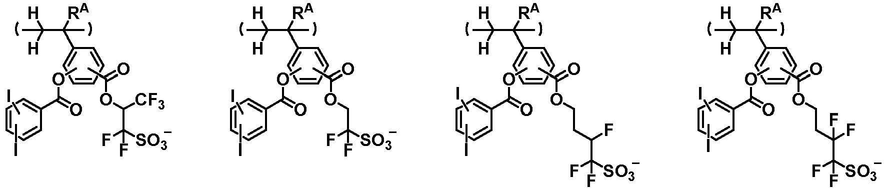

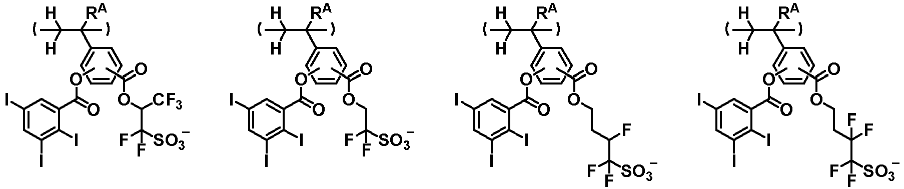

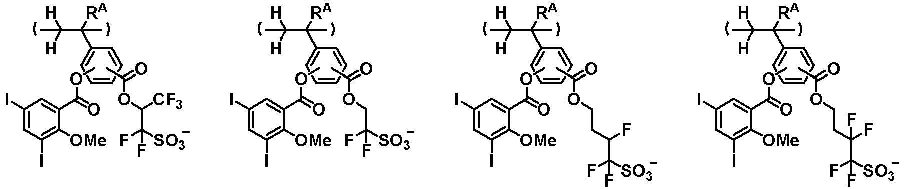

Examples of the anion in repeat unit B8 are shown below, but not limited thereto. Herein R^{A} is as defined above.

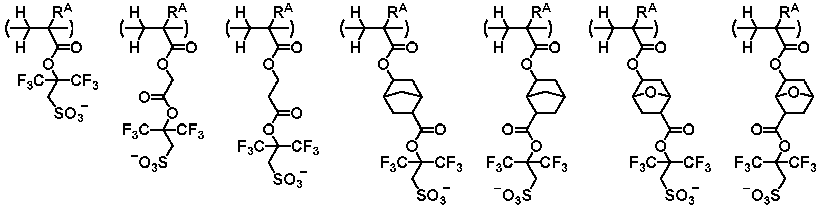

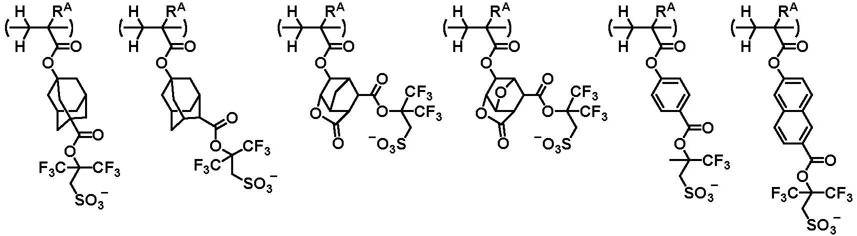

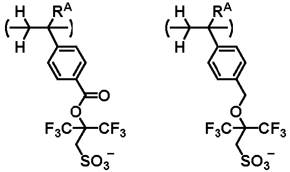

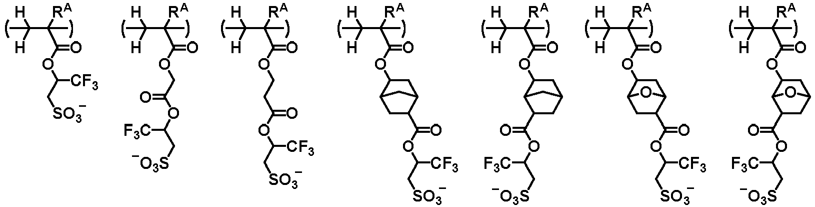

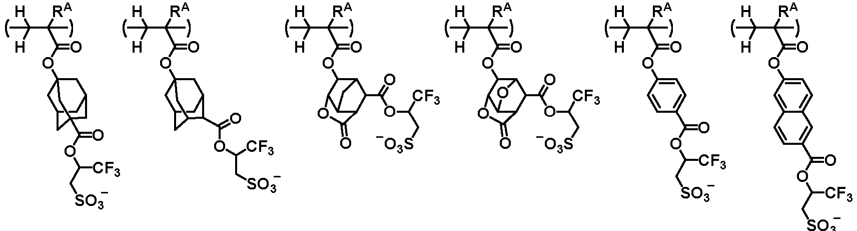

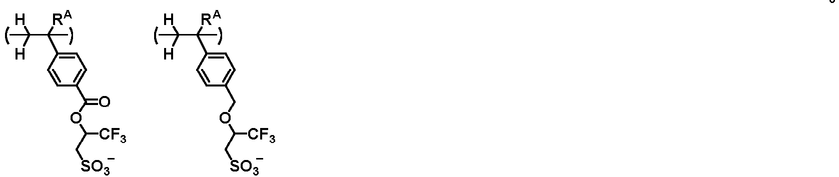

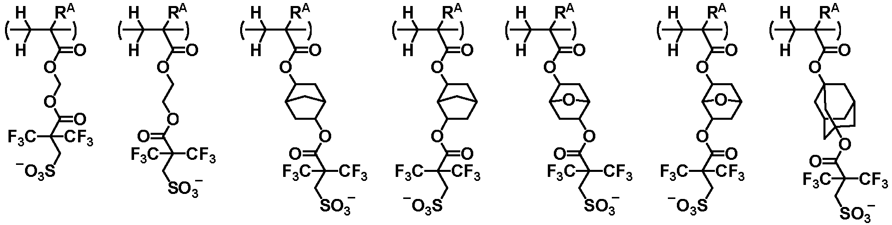

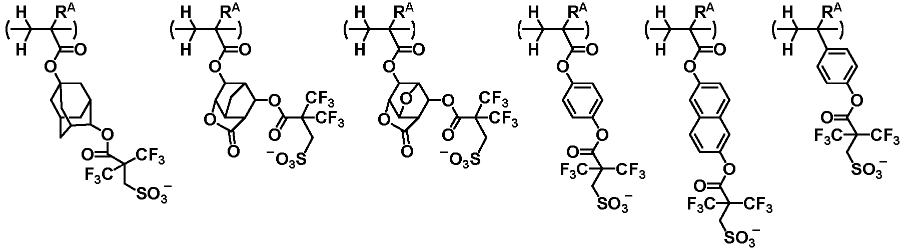

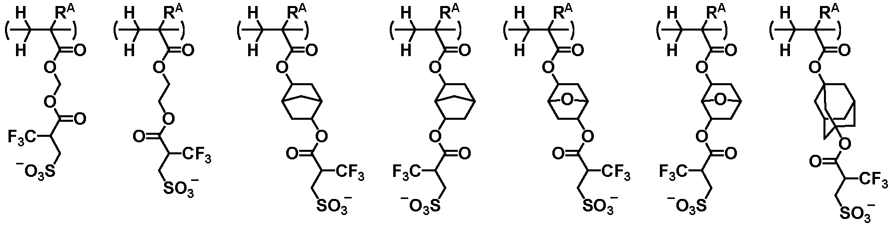

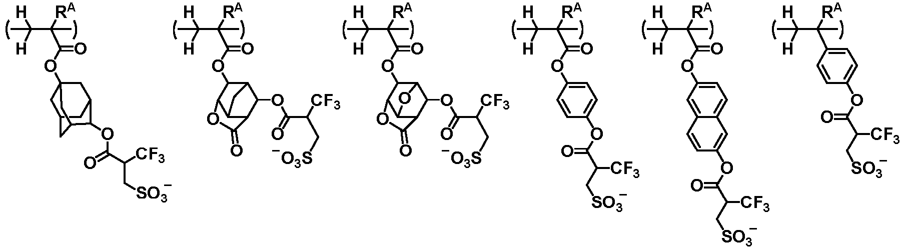

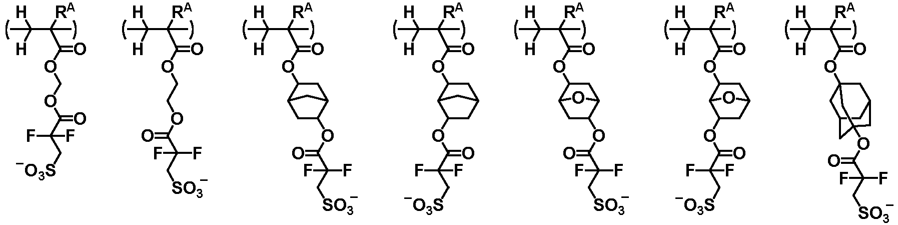

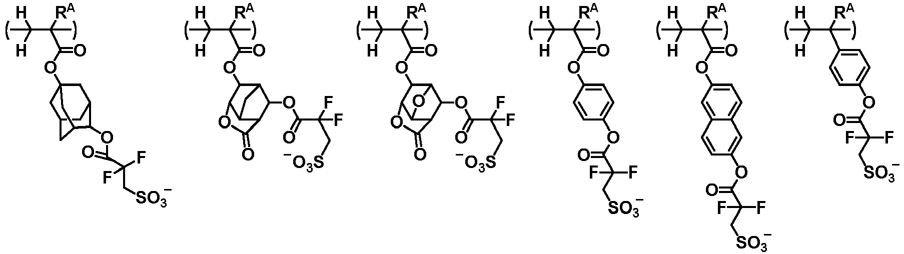

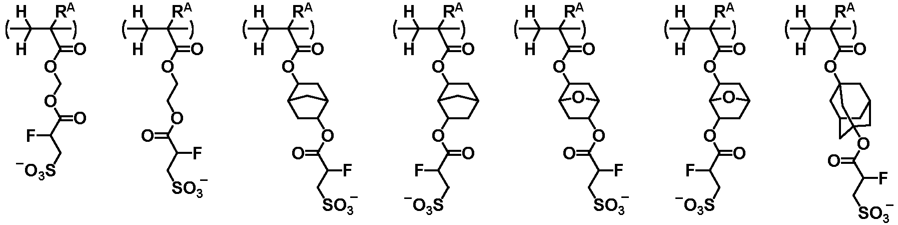

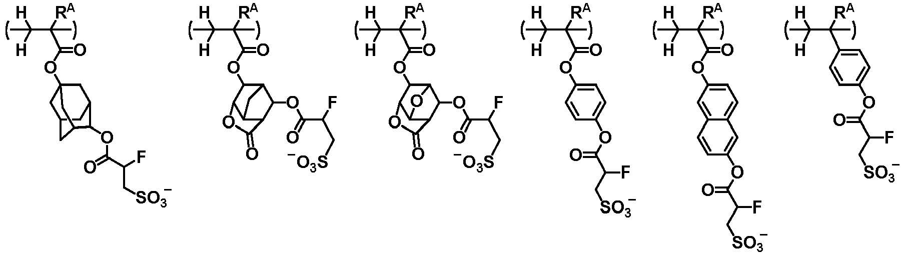

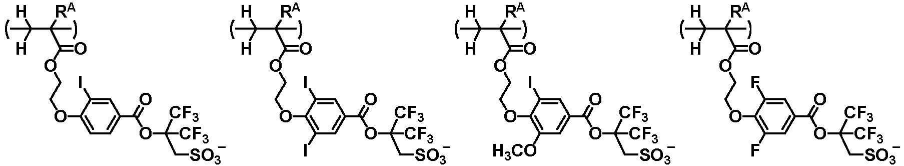

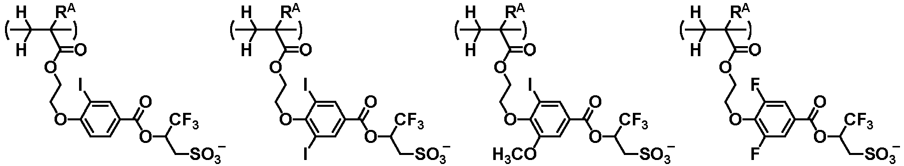

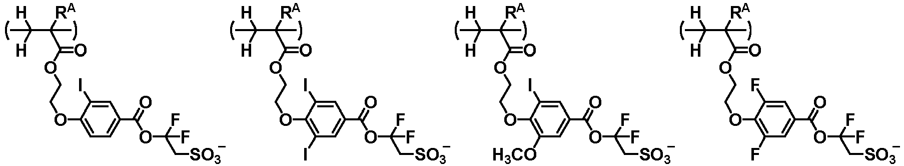

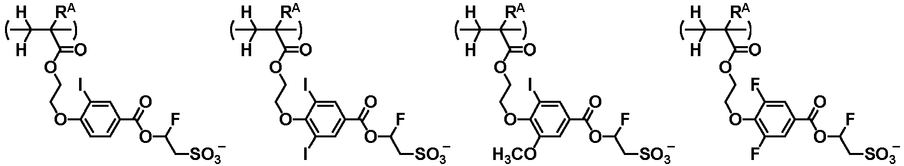

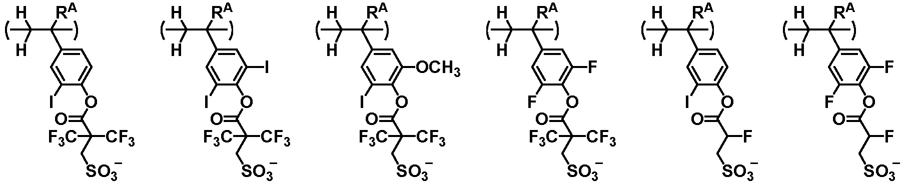

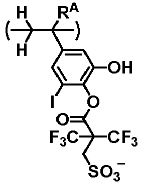

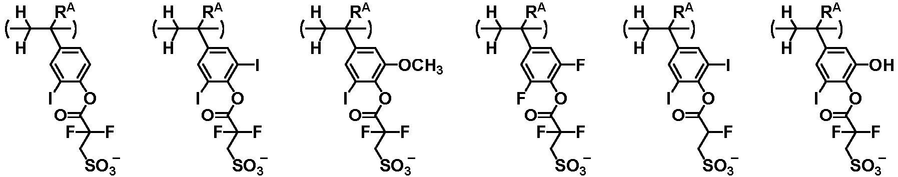

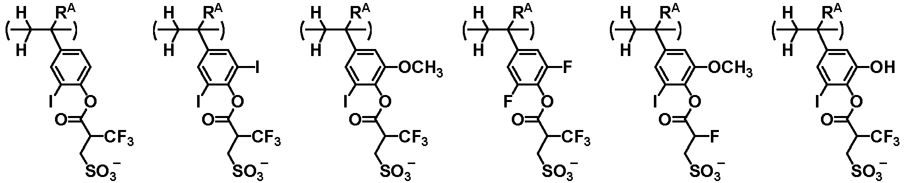

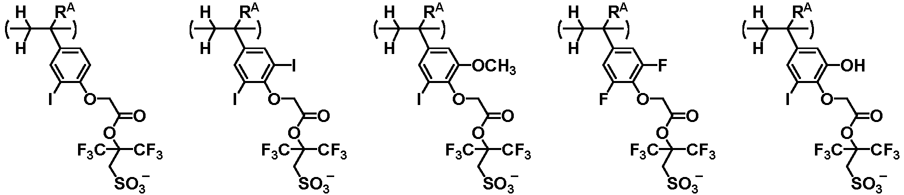

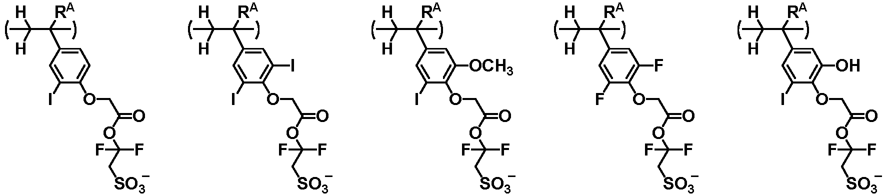

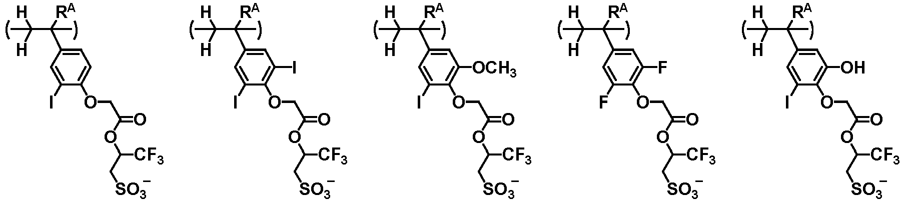

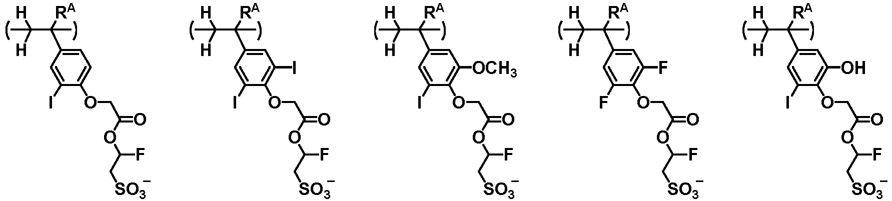

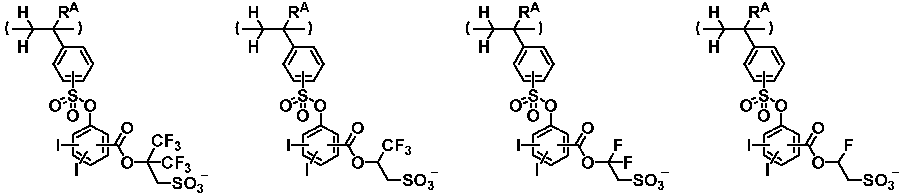

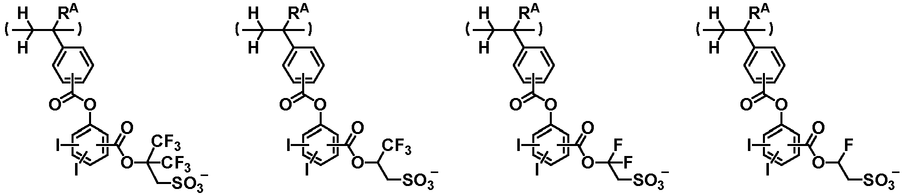

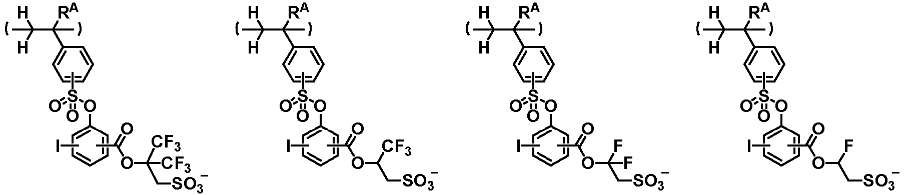

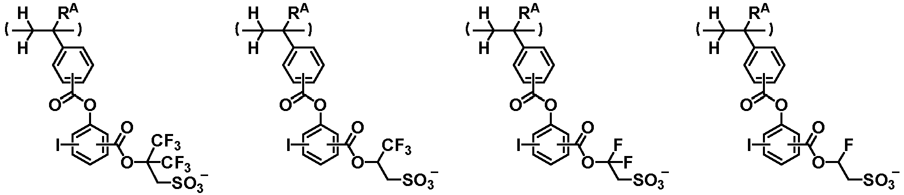

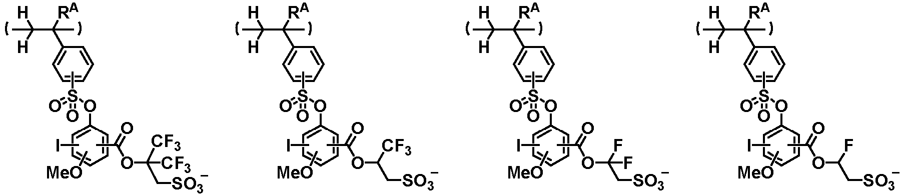

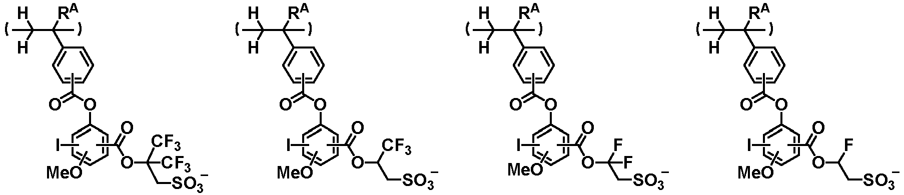

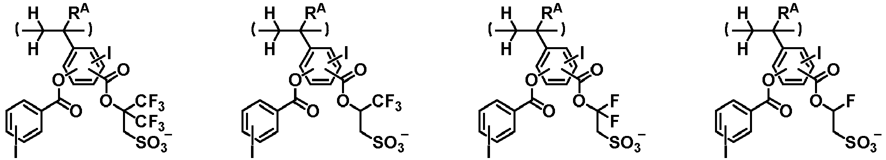

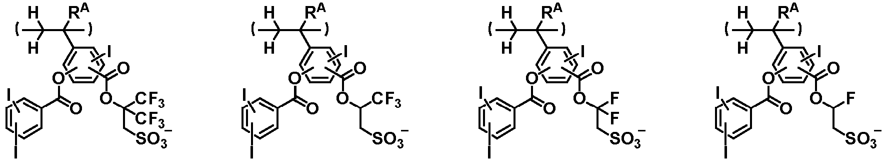

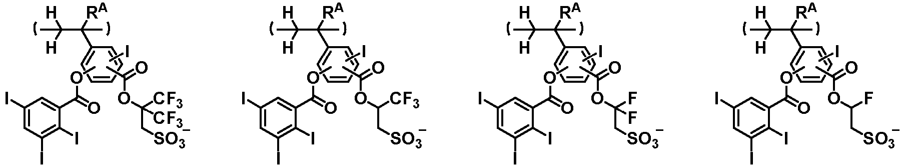

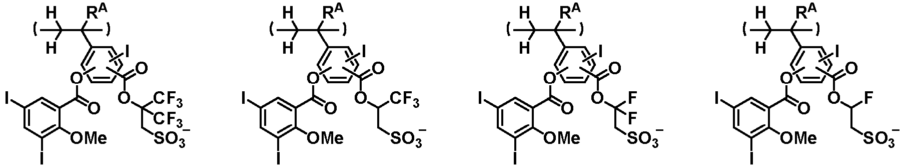

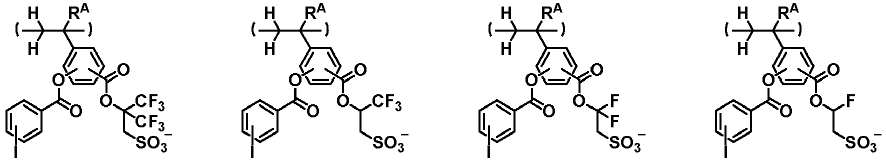

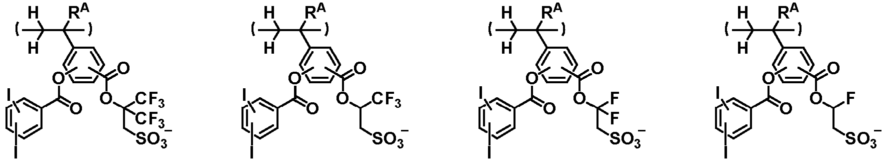

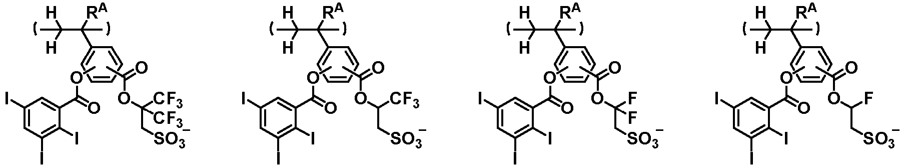

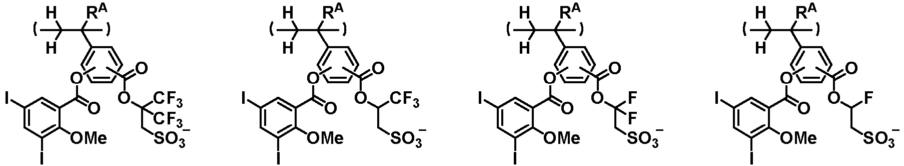

Examples of the anion in repeat unit B9 are shown below, but not limited thereto. Herein R^{A} is as defined above.

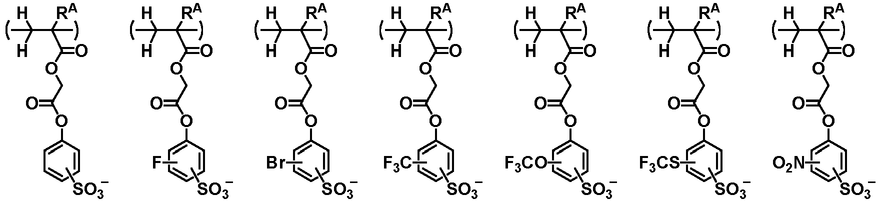

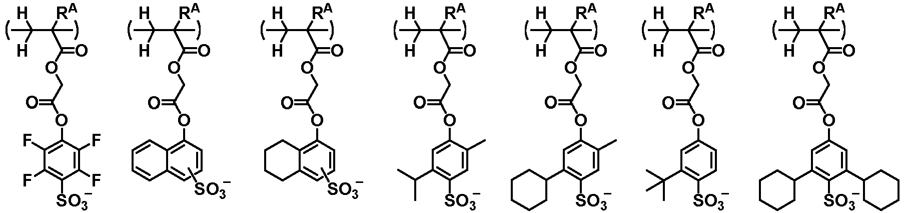

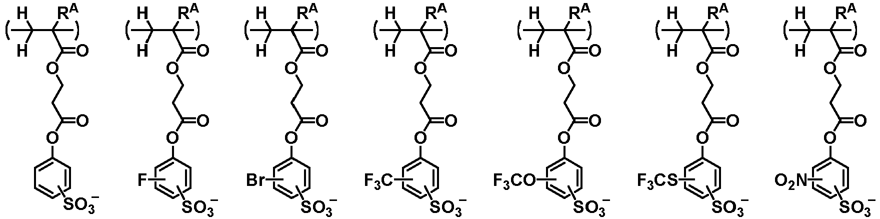

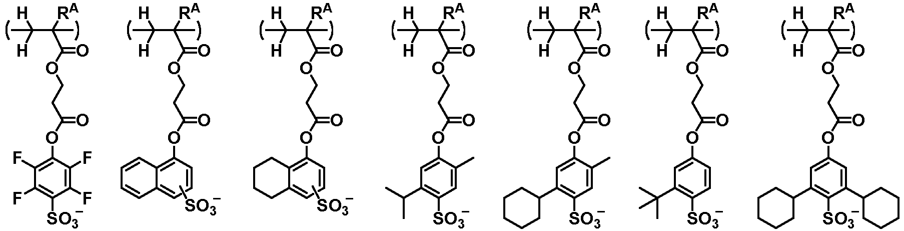

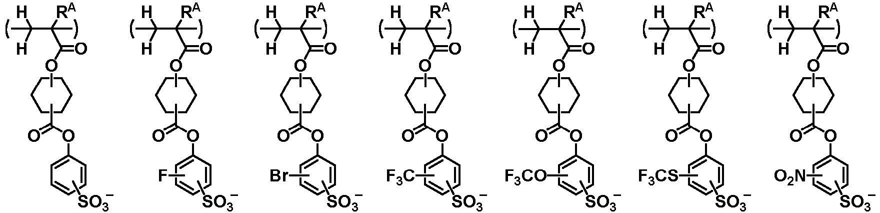

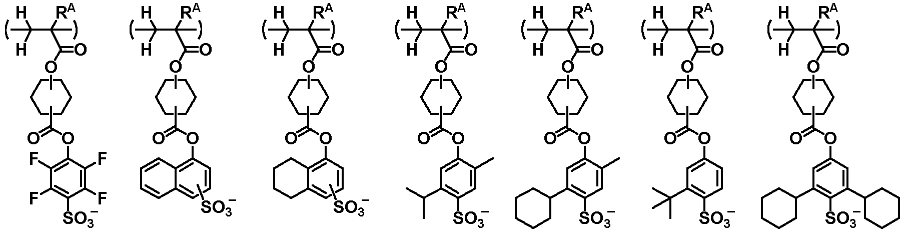

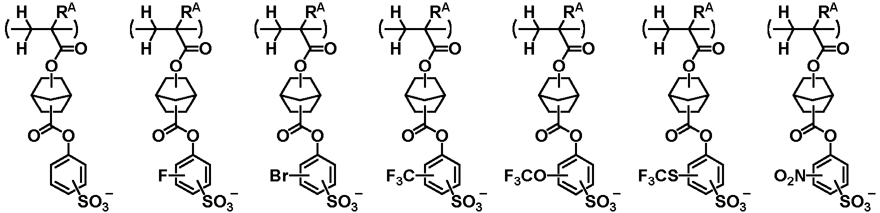

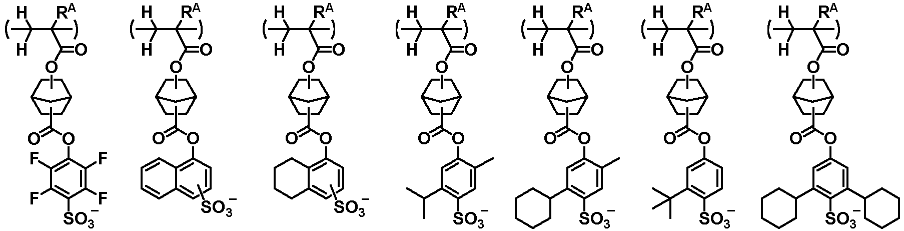

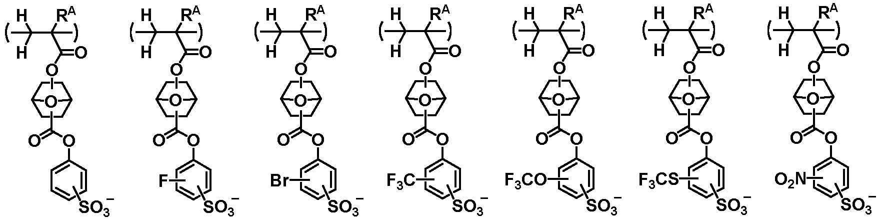

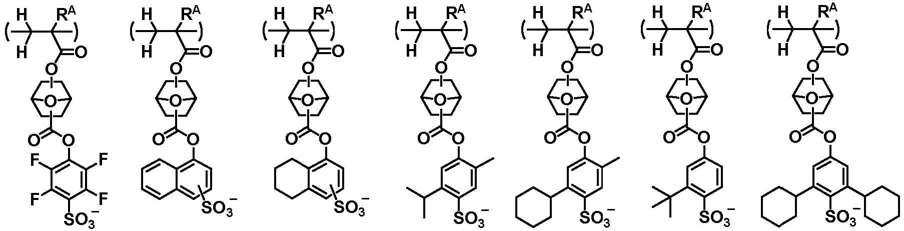

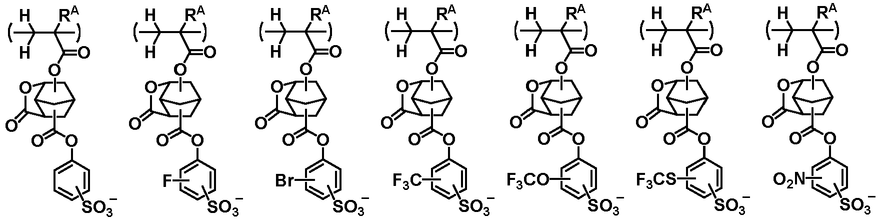

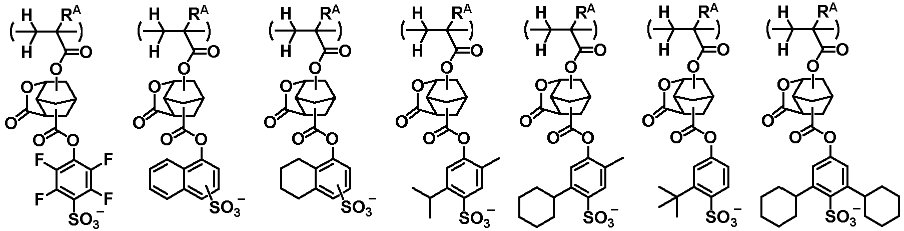

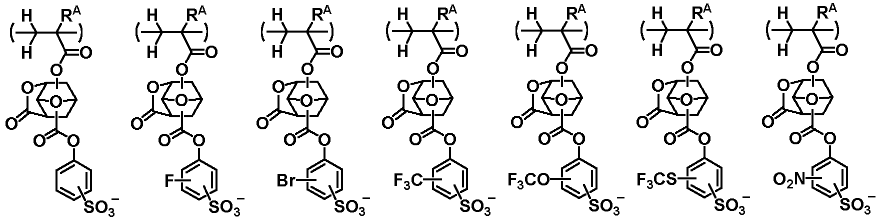

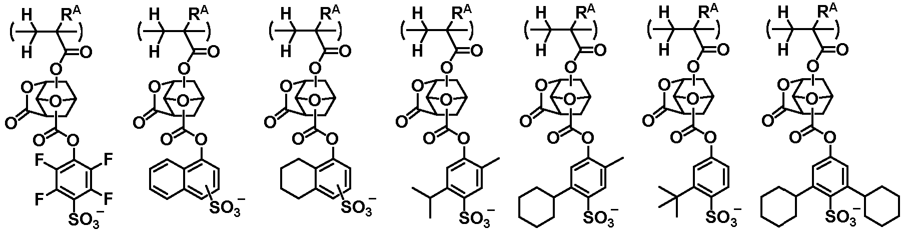

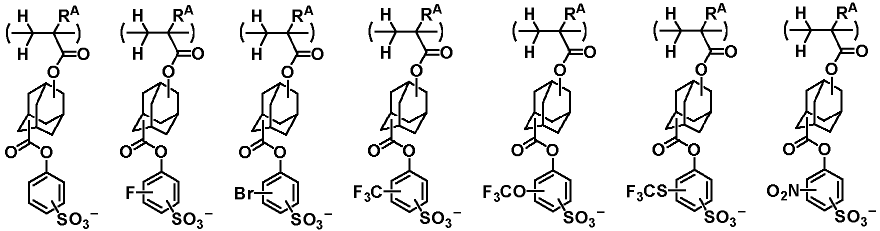

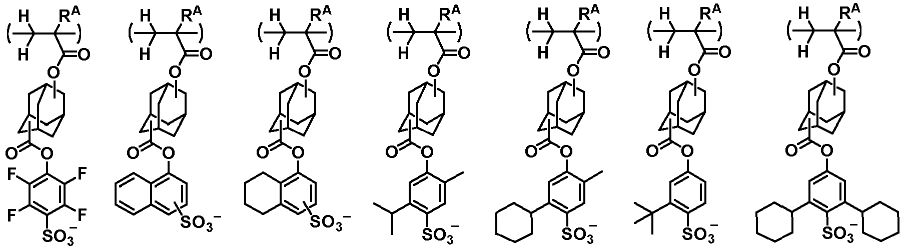

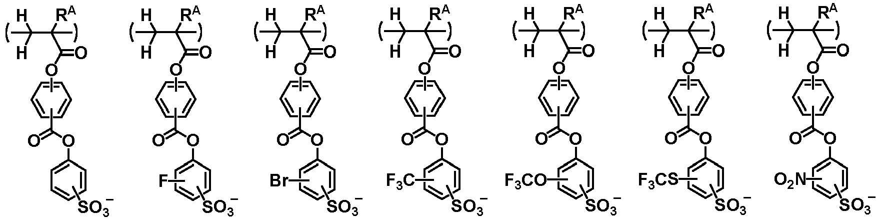

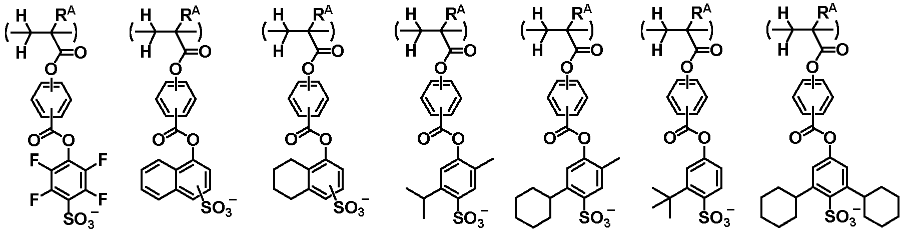

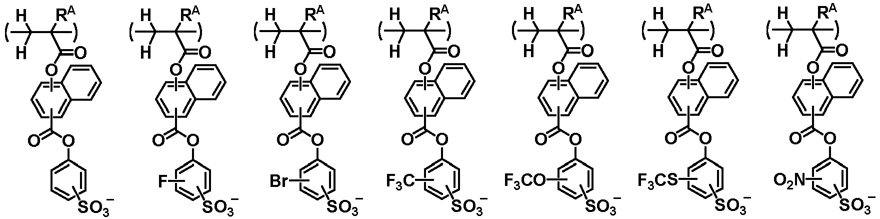

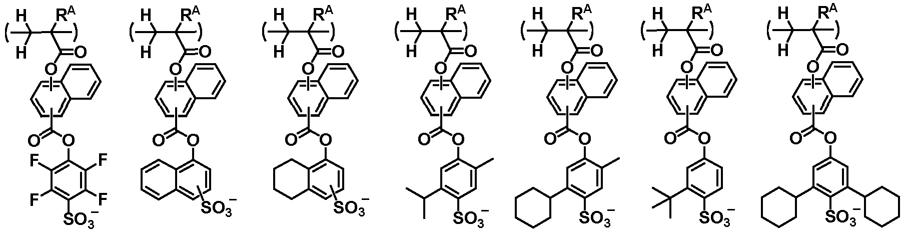

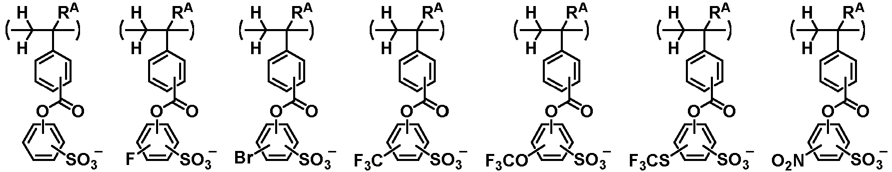

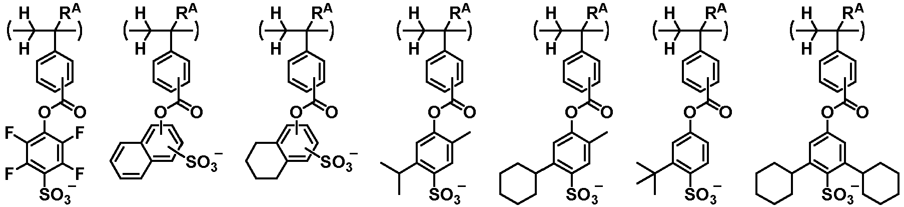

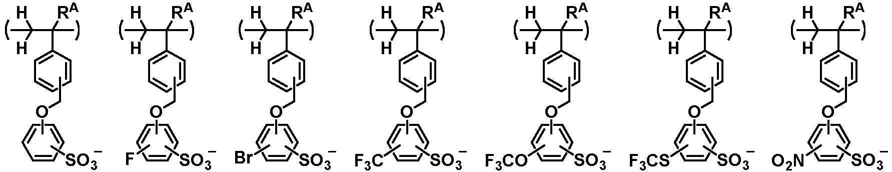

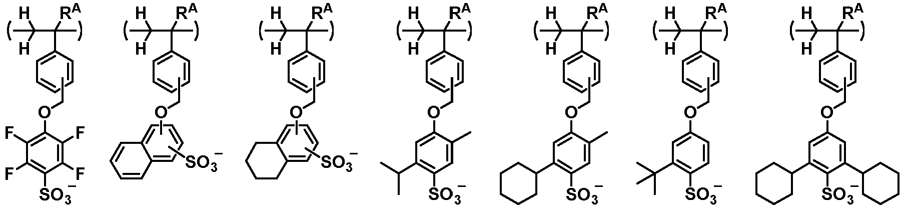

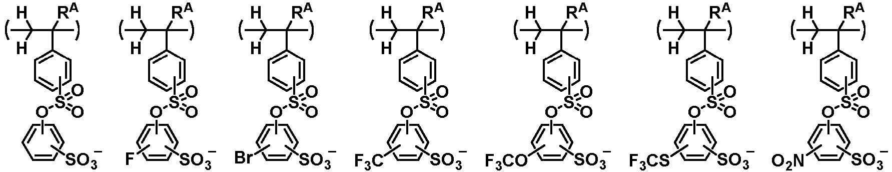

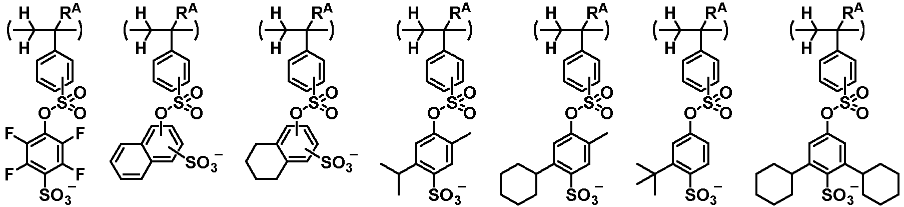

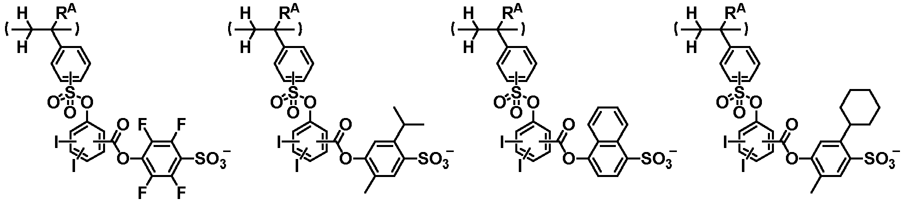

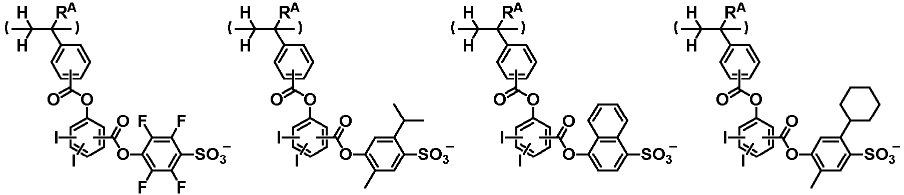

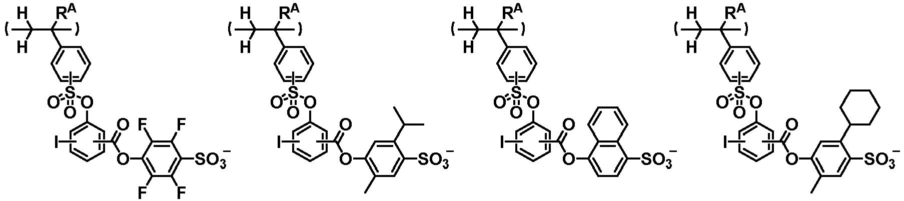

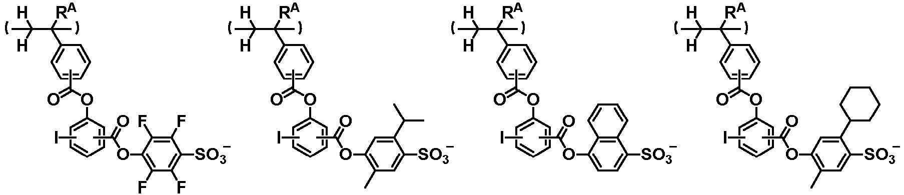

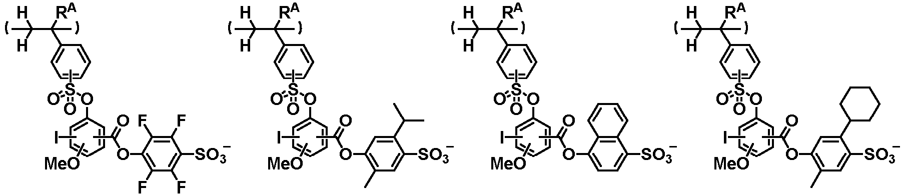

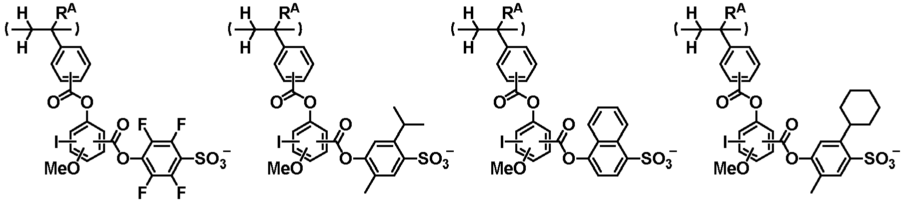

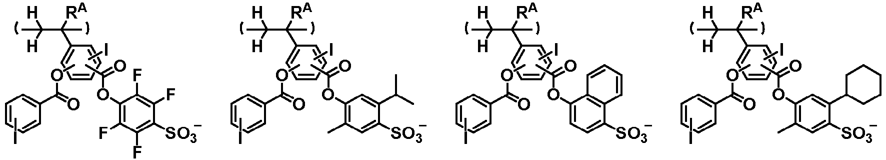

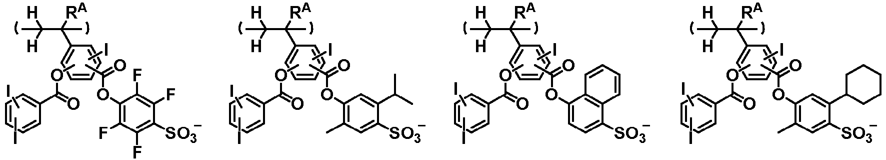

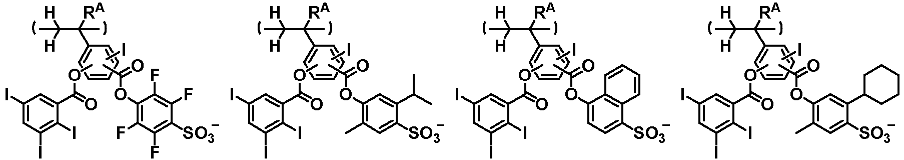

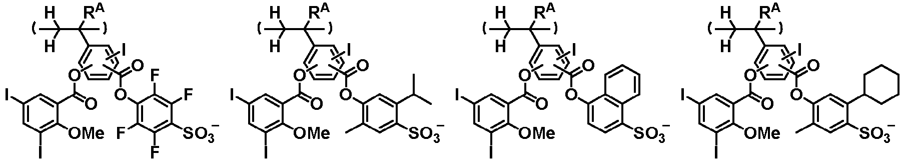

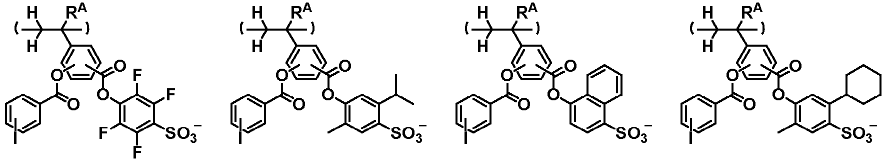

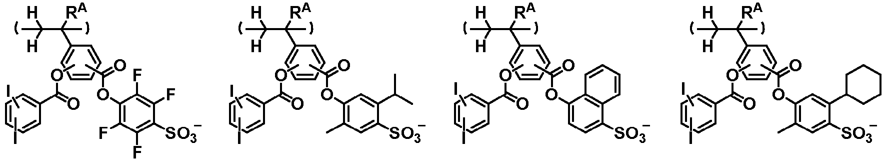

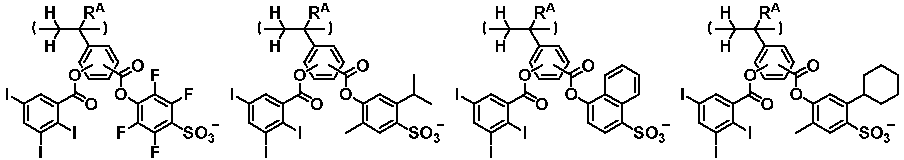

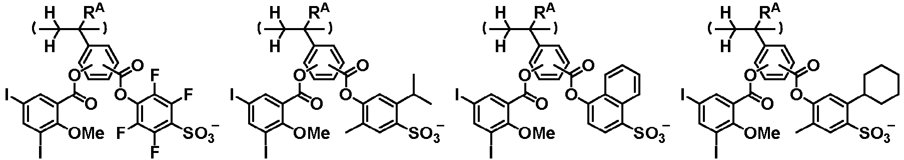

Examples of the anion in repeat unit B 10 are shown below, but not limited thereto. Herein R^{A} is as defined above.

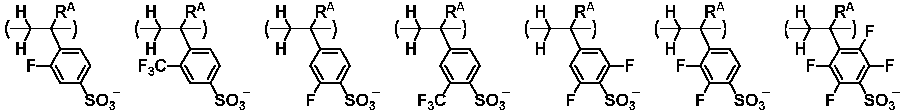

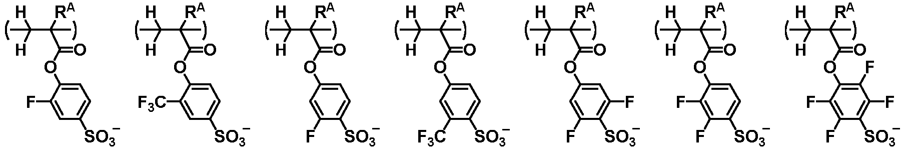

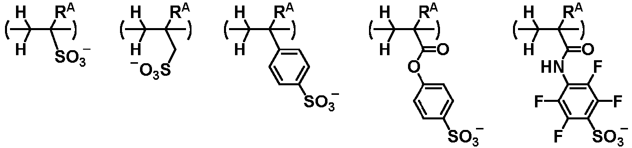

In formulae (B7) to (B10), A⁺ is an onium cation, preferably a sulfonium cation having the formula (Z-1) or iodonium cation having the formula (Z-2).

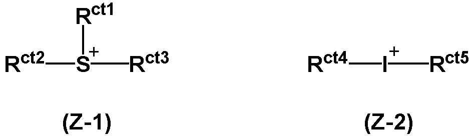

In formulae (Z-1) and (Z-2), R^{ct1} to R^{ct5} are each independently halogen or a C₁-C₃₀ hydrocarbyl group which may contain a heteroatom. Suitable halogen atoms include fluorine, chlorine, bromine and iodine. The hydrocarbyl group may be saturated or unsaturated and straight, branched or cyclic. Examples thereof include C₁-C₃₀ alkyl groups such as methyl, ethyl, n-propyl, isopropyl, n-butyl, isobutyl, sec-butyl, tert-butyl; C₃-C₃₀ cyclic saturated hydrocarbyl groups such as cyclopropyl, cyclopentyl, cyclohexyl, cyclopropylmethyl, 4-methylcyclohexyl, cyclohexylmethyl, norbornyl, adamantyl; C₂-C₃₀ alkenyl groups such as vinyl, 1-propenyl, 2-propenyl, butenyl, hexenyl; C₃-C₃₀ cyclic unsaturated hydrocarbyl groups such as cyclohexenyl; C₆-C₃₀ aryl groups such as phenyl, naphthyl, thienyl; C₇-C₃₀ aralkyl groups such as benzyl, 1-phenylethyl, 2-phenylethyl, and combinations thereof. Inter alia, the aryl groups are preferred. In the hydrocarbyl groups, some or all hydrogen may be substituted by a moiety containing a heteroatom such as oxygen, sulfur, nitrogen or halogen, and some -CH₂- may be replaced by a moiety containing a heteroatom such as oxygen, sulfur or nitrogen, so that the group may contain a hydroxy, fluorine, chlorine, bromine, iodine, cyano, nitro, carbonyl, ether bond, ester bond, sulfonate ester bond, carbonate bond, lactone ring, sultone ring, carboxylic anhydride (-C(=O)-O-C(=O)-) or haloalkyl moiety.

Also, R^{ct1} and R^{ct2} may bond together to form a ring with the sulfur atom to which they are attached. Exemplary structures of the ring are shown below.

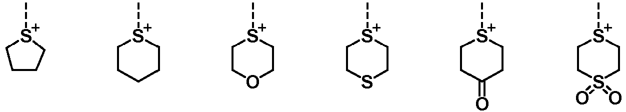

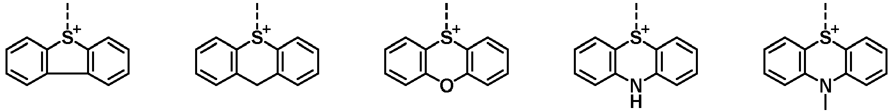

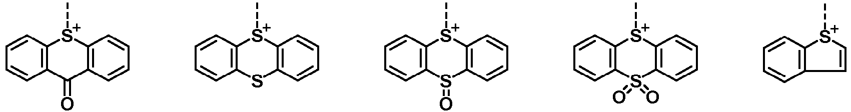

The broken line designates a point of attachment to R^{ct3}.

Examples of the sulfonium cation having formula (Z-1) include those described in JP-A 2024-003744, paragraphs [0102]-[0125] and JP-A 2023-169812, paragraphs [0070]-[0085], but are not limited thereto.

Examples of the iodonium cation having formula (Z-2) include those described in JP-A 2024-000259, paragraph [0181], but are not limited thereto.

As the onium cation Z⁺, a sulfonium cation having the formula (Z-3) is also preferred.

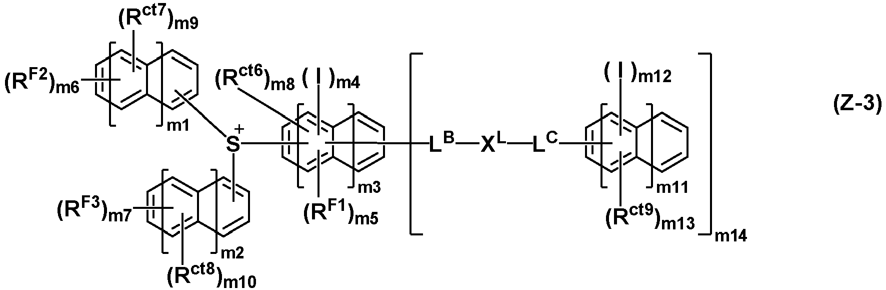

In formula (Z-3), m1 is 0 or 1. The relevant structure is a benzene ring when m1=0 and a naphthalene ring when m1=1. From the aspect of solvent solubility, the benzene ring corresponding to m1=0 is preferred. The subscript m2 is 0 or 1. The relevant structure is a benzene ring when m2=0 and a naphthalene ring when m2=1. From the aspect of solvent solubility, the benzene ring corresponding to m2=0 is preferred. The subscript m3 is 0 or 1. The relevant structure is a benzene ring when m3=0 and a naphthalene ring when m3=1. From the aspect of solvent solubility, the benzene ring corresponding to m3=0 is preferred.

In formula (Z-3), m4 is 0, 1, 2, 3 or 4. As the number of iodine atoms in the cation structure is more, the absorption of EUV becomes higher, but solvent solubility becomes poorer. There is left concern that the compound precipitates in the resist composition. Thus m4 is preferably 1, 2 or 3, more preferably 1 or 2.

In formula (Z-3), m5 is 0, 1, 2, 3 or 4. It is preferred from the aspect of reactant availability that m5 be 0, 1, 2 or 3, more preferably 0, 1 or 2. The subscript m6 is 0, 1, 2, 3, 4, 5 or 6. It is preferred from the aspect of reactant availability that m6 be 0, 1, 2 or 3, more preferably 0, 1 or 2. The subscript m7 is 0, 1, 2, 3, 4, 5 or 6. It is preferred from the aspect of reactant availability that m7 be 0, 1, 2 or 3, more preferably 0, 1 or 2.

In formula (Z-3), m8 is 0, 1 or 2. It is preferred from the aspect of reactant availability that m8 be 0 or 1. The subscript m9 is 0, 1 or 2. It is preferred from the aspect of reactant availability that m9 be 0 or 1. The subscript m10 is 0, 1 or 2. It is preferred from the aspect of reactant availability that m10 be 0 or 1.

In formula (Z-3), m11 is 0 or 1. The relevant structure is a benzene ring when m11=0 and a naphthalene ring when m11=1. From the aspect of solvent solubility, the benzene ring corresponding to m11=0 is preferred.

In formula (Z-3), m12 is 0, 1, 2, 3 or 4. As the number of iodine atoms in the cation structure is more, the absorption of EUV becomes higher, but solvent solubility becomes poorer. There is left concern that the compound precipitates in the resist composition. Thus m12 is preferably 0, 1, 2 or 3, more preferably 0, 1 or 2.

In formula (Z-3), m13 is 0, 1 or 2. It is preferred from the aspect of reactant availability that m13 be 0 or 1. The subscript m14 is 0, 1 or 2. It is preferred from the aspect of synthesis that m14 be 0 or 1.

It is noted that when m1=0, 0 ≤ m6+m9 ≤ 4, and when m1=1, 0 ≤ m6+m9 ≤ 6. When m2=0, 0 ≤ m7+m10 ≤ 4, and when m2=1, 0 ≤ m7+m10 ≤ 6. When m3=0, 1 ≤ m4+m5+m8+m14 ≤ 4, and when m3=1, 1 ≤ m4+m5+m8+m14 ≤ 6. When m11=0, 0 ≤ m12+m13 ≤ 4, and when m11=1, 0 ≤ m12+m13 ≤ 6. Also, m4+m12 ≥ 1.

In formula (Z-3), R^{F1} to R^{F3} each independently fluorine, a C₁-C₆ fluorinated saturated hydrocarbyl group, C₁-C₆ fluorinated saturated hydrocarbyloxy group, or C₁-C₆ fluorinated saturated hydrocarbylthio group. Of these, trifluoromethyl, trifluoromethoxy and trifluorothiomethoxy are preferred. When m5 is 2 or more, a plurality of R^{F1} may be identical or different. When m6 is 2 or more, a plurality of R^{F2} may be identical or different. When m7 is 2 or more, a plurality of R^{F3} may be identical or different.

In formula (Z-3), R^{ct6} to R^{ct9} are each independently halogen exclusive of iodine and fluorine, nitro, cyano, a C₁-C₂₀ hydrocarbyl group which may contain a heteroatom, C₁-C₂₀ hydrocarbyloxy group which may contain a heteroatom, or C₁-C₂₀ hydrocarbylthio group which may contain a heteroatom. The hydrocarbyl group and hydrocarbyl moiety in the hydrocarbyloxy and hydrocarbylthio groups may be saturated or unsaturated and straight, branched or cyclic. Examples thereof are as exemplified above for the hydrocarbyl group R¹ in formula (A). In the hydrocarbyl group and hydrocarbyl moiety in the hydrocarbyloxy and hydrocarbylthio groups, some or all of the hydrogen atoms may be substituted by a moiety containing a heteroatom such as oxygen, sulfur, nitrogen or halogen, and some constituent -CH₂- may be replaced by a moiety containing a heteroatom such as oxygen, sulfur or nitrogen, so that the group may contain a hydroxy moiety, cyano moiety, fluorine, chlorine, bromine, iodine, carbonyl moiety, ether bond, ester bond, sulfonate ester bond, carbonate bond, lactone ring, sultone ring, carboxylic anhydride (-C(=O)-O-C(=O)-) or haloalkyl moiety.

When m8=2, two R^{ct6} may be identical or different and two R^{ct6} may bond together to form a ring with the carbon atoms to which they are attached. When m9=2, two R^{ct7} may be identical or different and two R^{ct7} may bond together to form a ring with the carbon atoms to which they are attached. When m10=2, two R^{ct8} may be identical or different and two R^{ct8} may bond together to form a ring with the carbon atoms to which they are attached. When m13=2, two R^{ct9} may be identical or different and two R^{ct9} may bond together to form a ring with the carbon atoms to which they are attached. Examples of the ring thus formed include cyclopropane, cyclobutane, cyclopentane, cyclohexane, norbornane, and adamantane rings. In the ring, some or all of the hydrogen atoms may be substituted by a moiety containing a heteroatom such as oxygen, sulfur, nitrogen or halogen, and some constituent -CH₂- may be replaced by a moiety containing a heteroatom such as oxygen, sulfur or nitrogen, so that the ring may contain a hydroxy moiety, fluorine, chlorine, bromine, iodine, cyano moiety, carbonyl moiety, ether bond, ester bond, sulfonate ester bond, carbonate bond, lactone ring, sultone ring, carboxylic anhydride (-C(=O)-O-C(=O)-) or haloalkyl moiety.

The aromatic rings directly bonded to S⁺ in the sulfonium cation having formula (Z-3) may bond together to form a ring with S⁺. Exemplary structures of the ring are shown below.

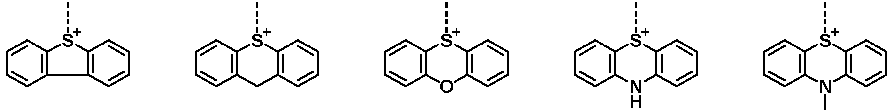

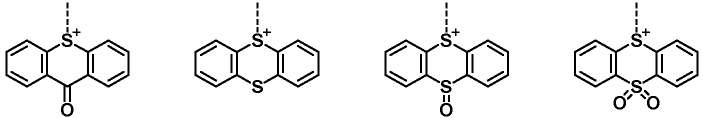

In formula (Z-3), L^{B} and L^{C} are each independently a single bond, ether bond, ester bond, sulfonate ester bond, amide bond, sulfonamide bond, carbonate bond or carbamate bond. L^{B} is preferably a single bond, ether bond, ester bond or sulfonate ester bond, more preferably an ester bond or sulfonate ester bond. L^{C} is preferably a single bond, ether bond or ester bond, more preferably a single bond.

In formula (Z-3), X^{L} is a single bond or a C₁-C₄₀ hydrocarbylene group which may contain a heteroatom. The C₁-C₄₀ hydrocarbylene group may be straight, branched or cyclic. Suitable hydrocarbylene groups include alkanediyl, cyclic saturated hydrocarbylene and arylene groups. Suitable heteroatoms include oxygen, nitrogen and sulfur atoms.

Examples of the optionally heteroatom-containing C₁-C₄₀ hydrocarbylene group X^{L} are shown below, but not limited thereto. Herein * each designates a point of attachment to L^{B} or L^{C}.

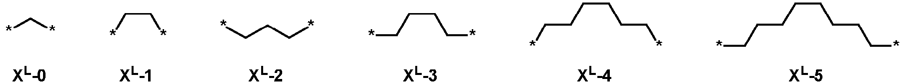

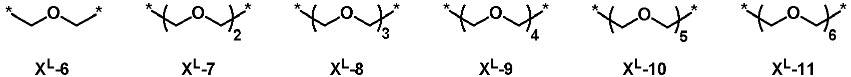

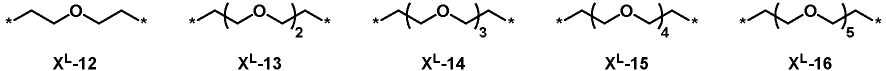

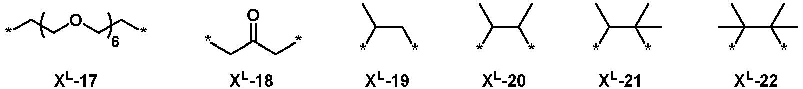

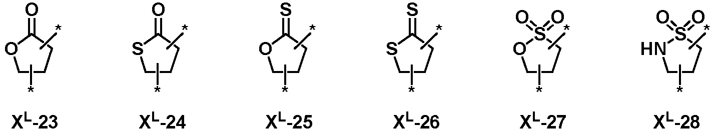

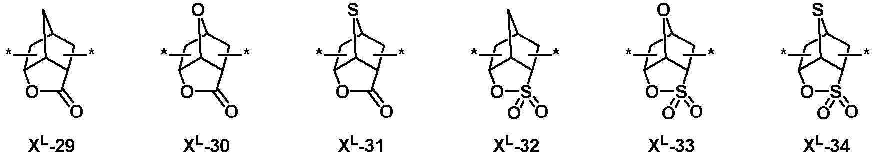

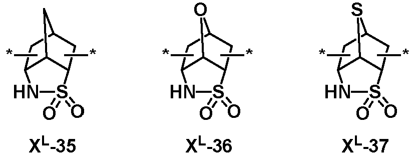

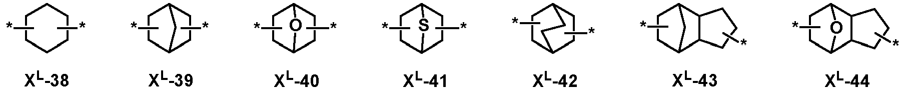

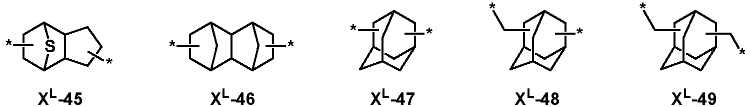

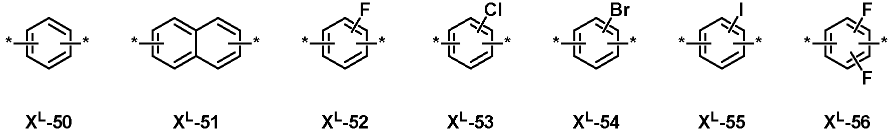

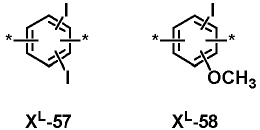

Of these, X^{L}-0 to X^{L}-22, X^{L}-29 to X^{L}-34, and X^{L}-47 to X^{L}-58 are preferred.

Of the sulfonium cations having formula (Z-3), a cation having the formula (Z-3-1) is preferred.

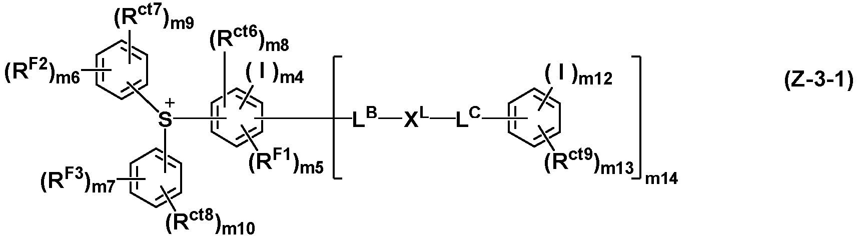

Herein m4 to m10, m12 to m14, R^{F1} to R^{F3}, R^{ct6} to R^{ct9}, L^{B}, L^{C}, and X^{L} are as defined above.

Of the sulfonium cations having formula (Z-3-1), a cation having the formula (Z-3-2) is preferred.

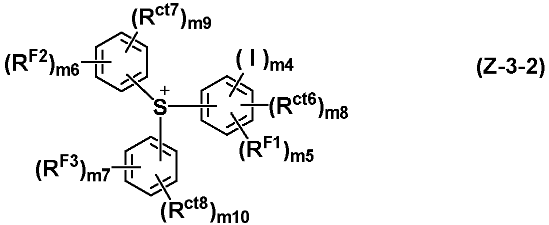

Herein m4 to m10, R^{F1} to R^{F3}, and R^{ct6} to R^{ct8} are as defined above.

Examples of the sulfonium cation having formula (Z-3) are shown below, but not limited thereto.

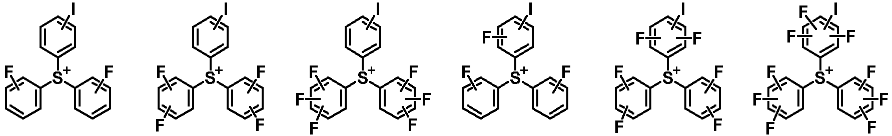

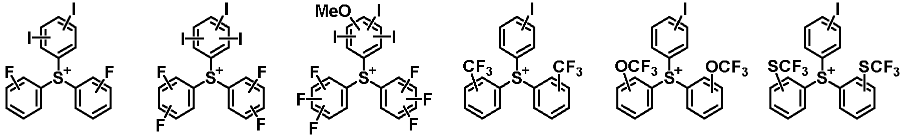

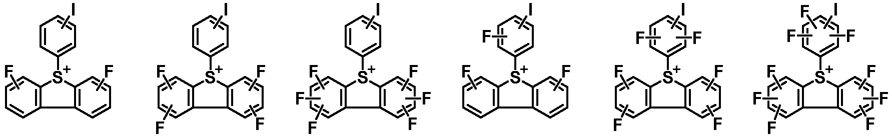

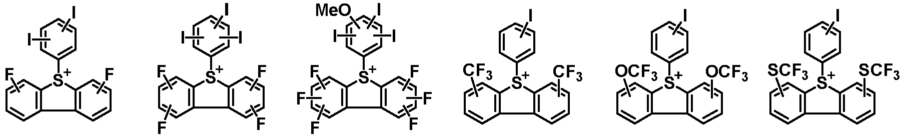

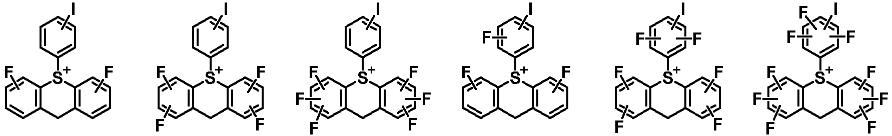

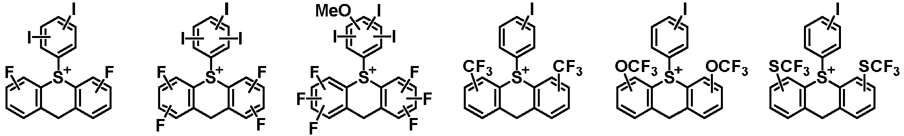

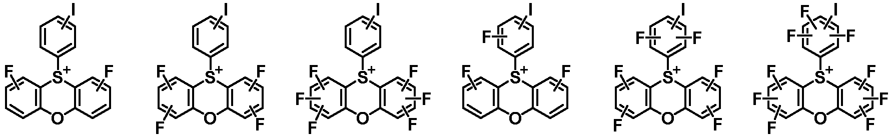

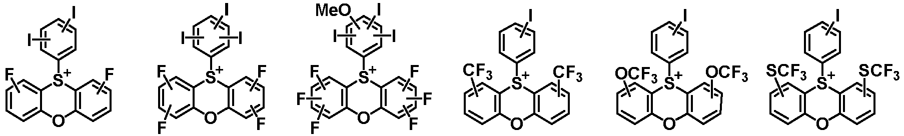

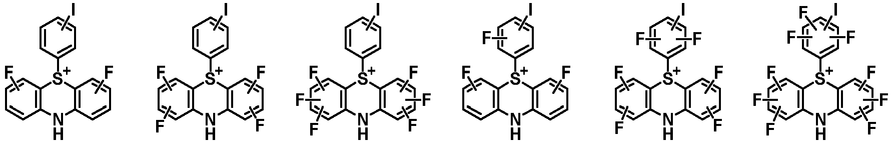

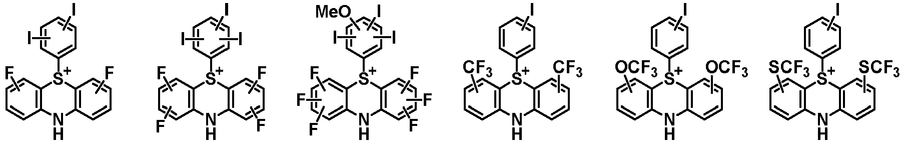

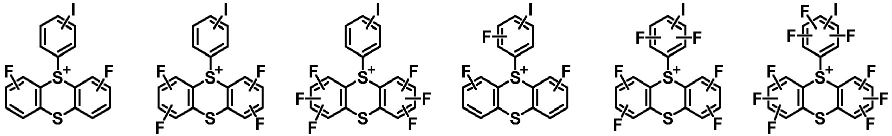

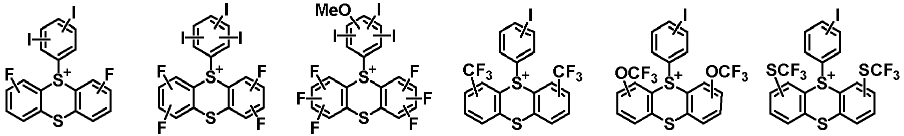

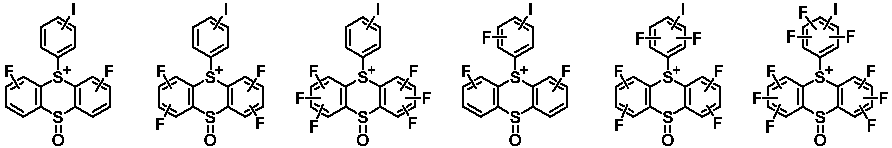

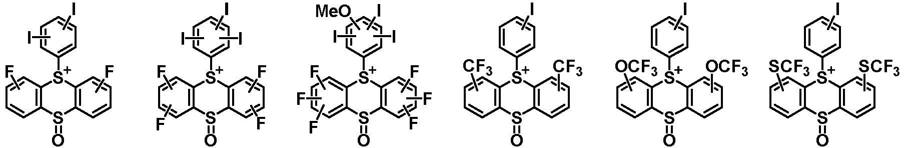

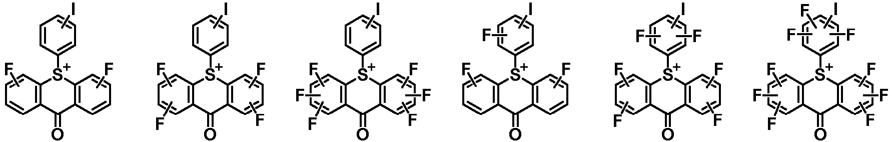

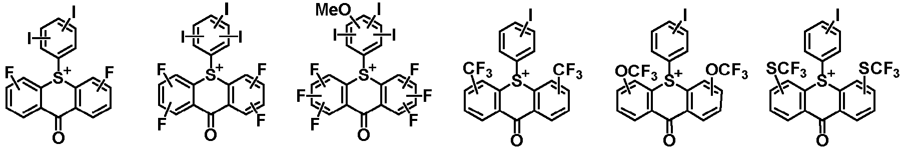

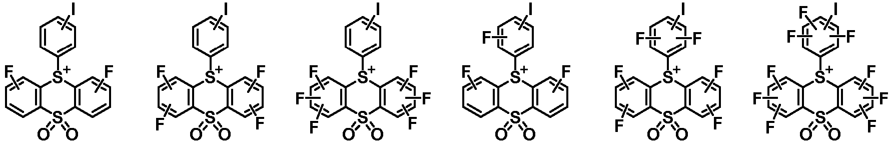

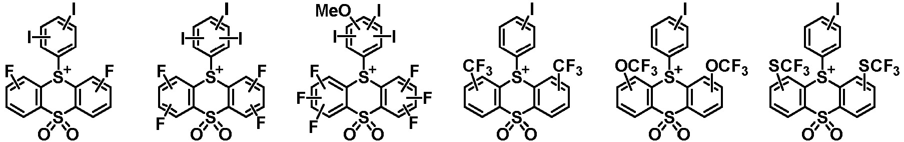

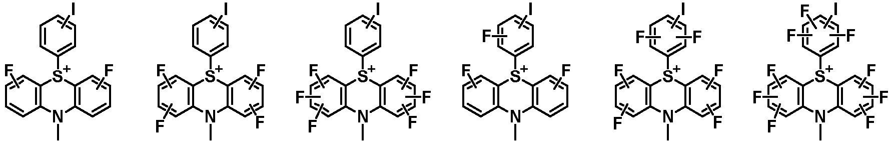

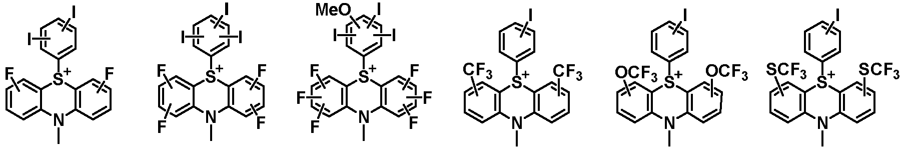

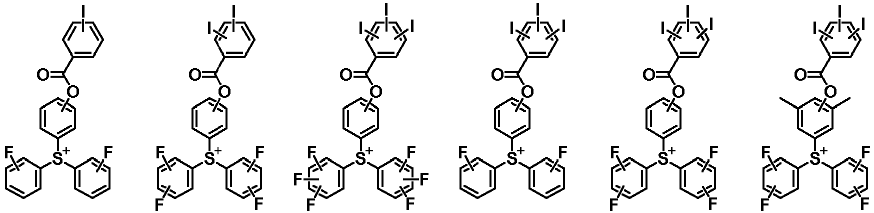

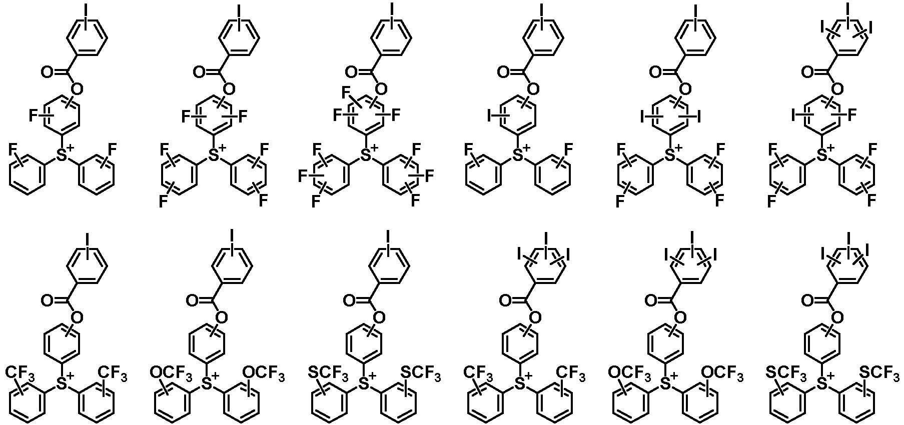

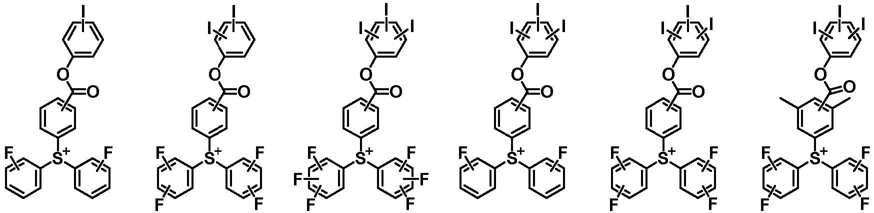

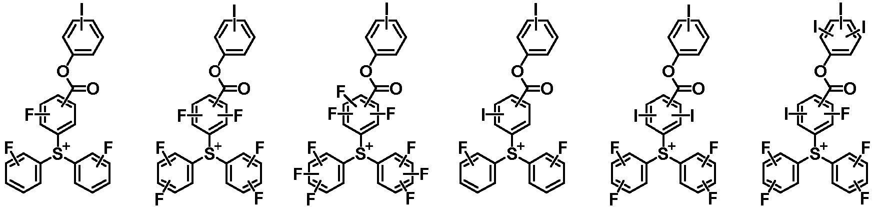

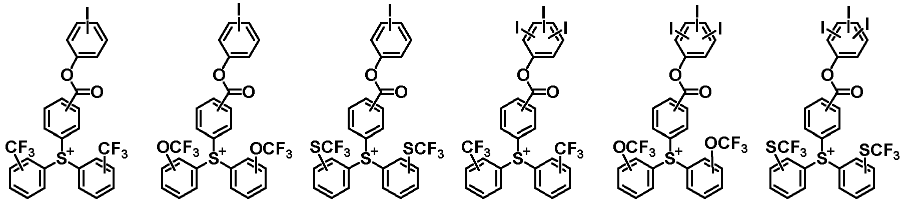

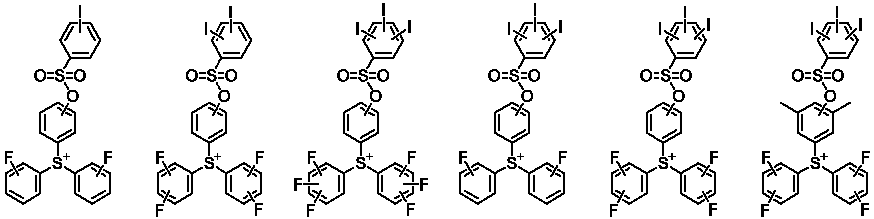

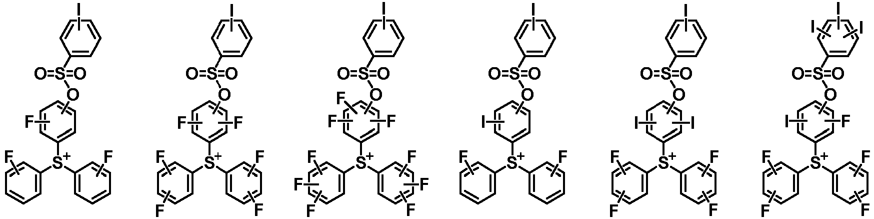

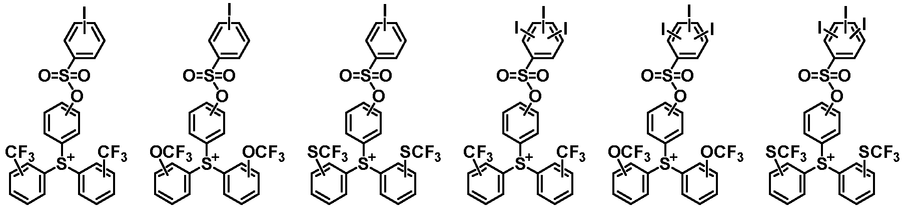

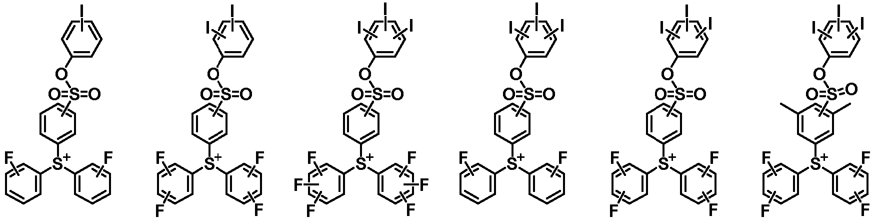

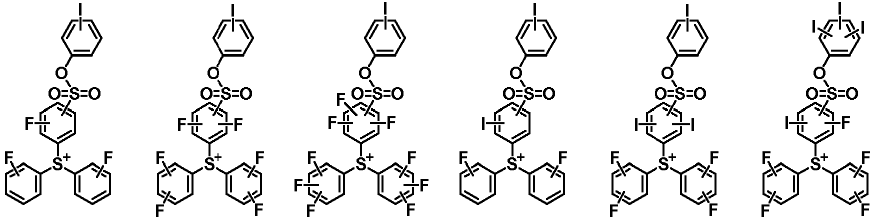

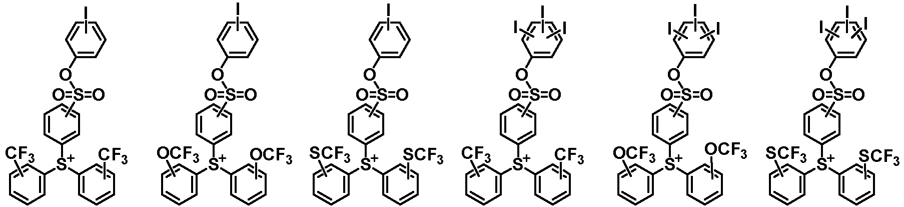

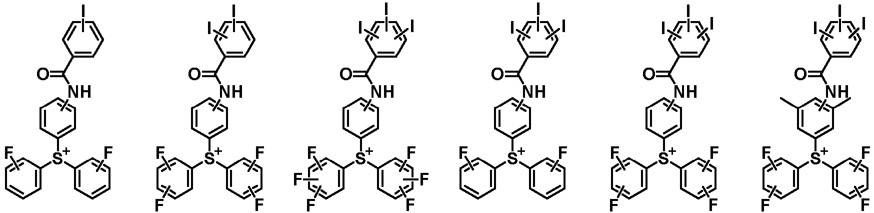

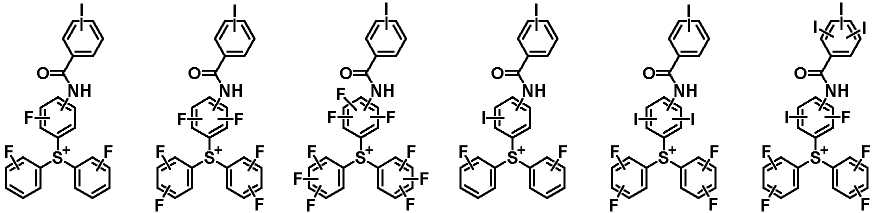

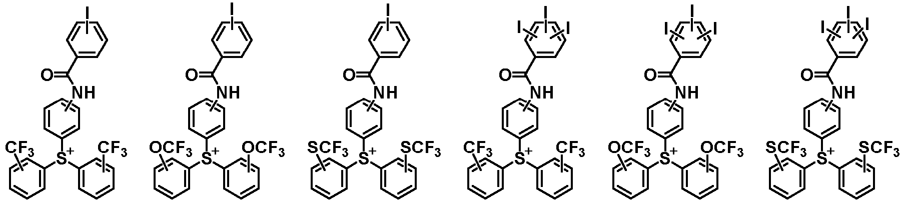

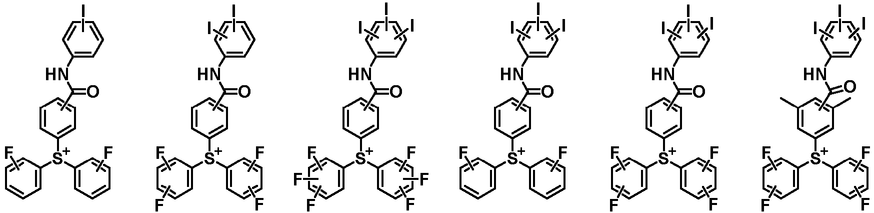

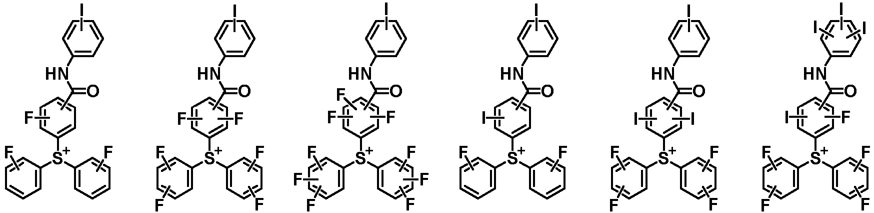

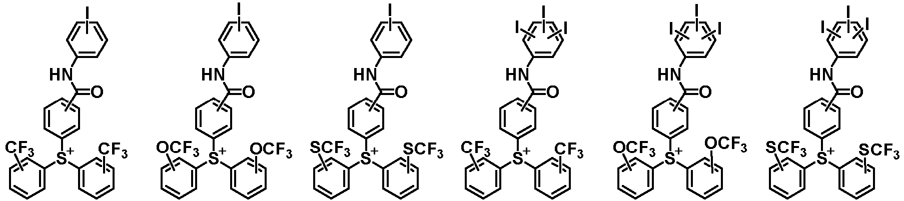

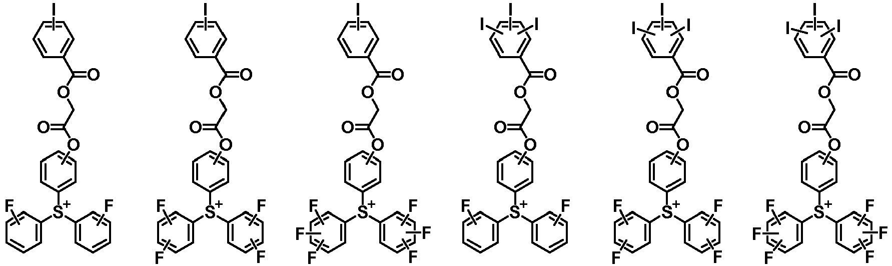

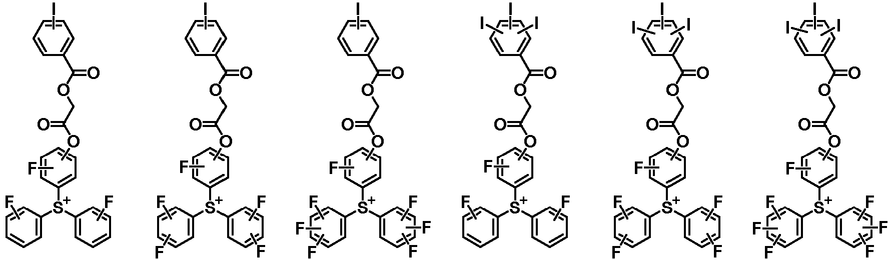

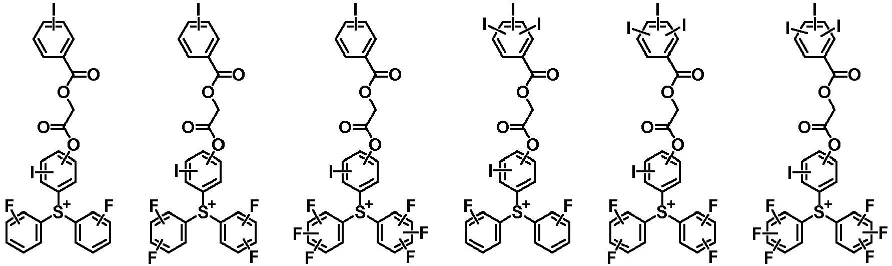

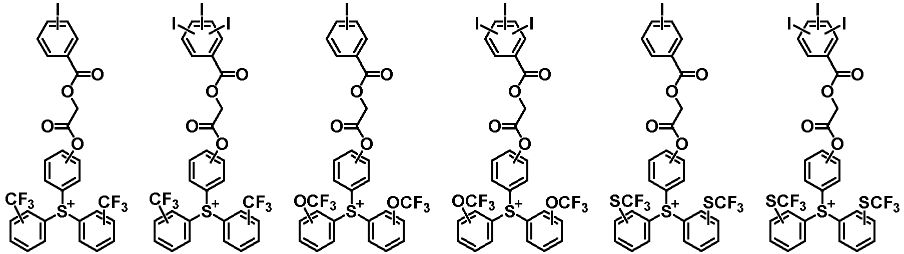

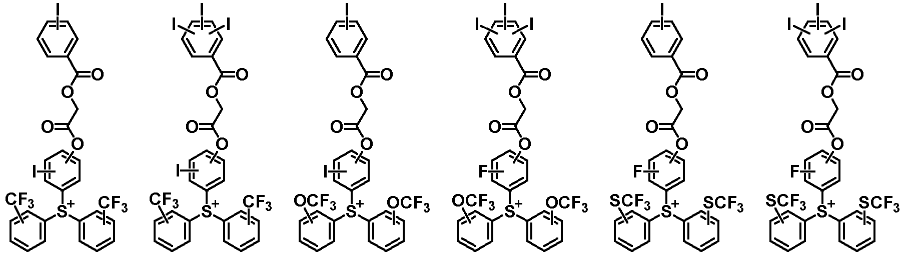

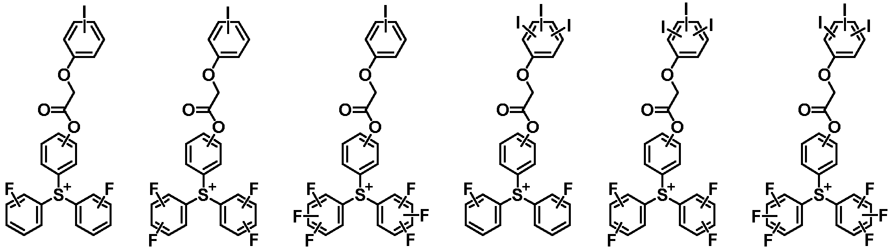

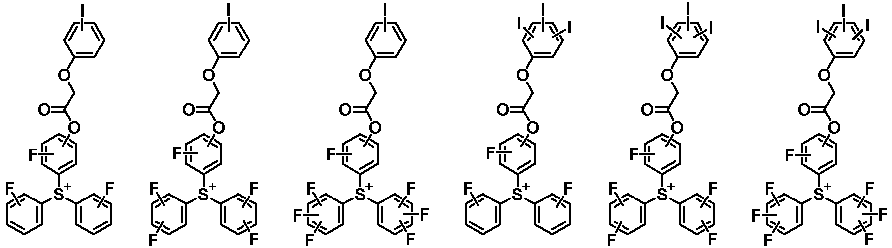

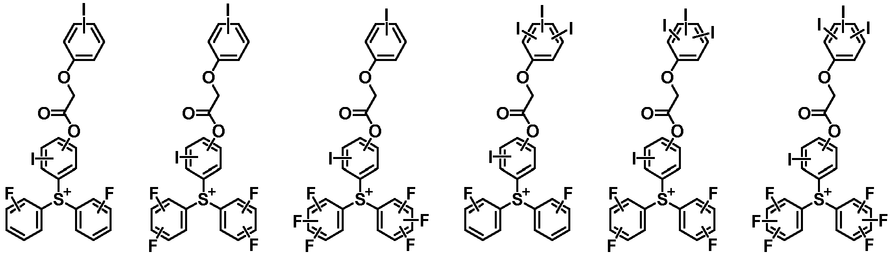

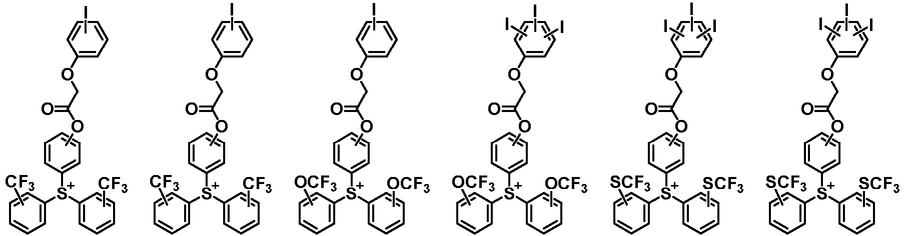

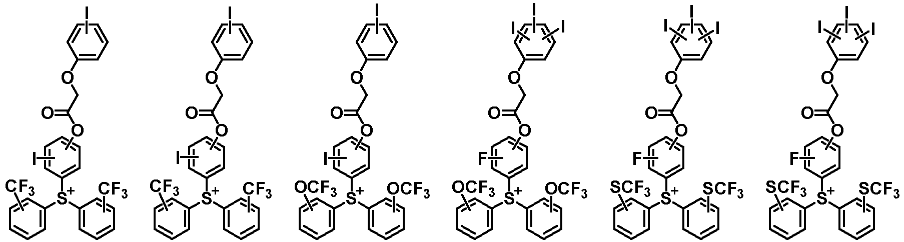

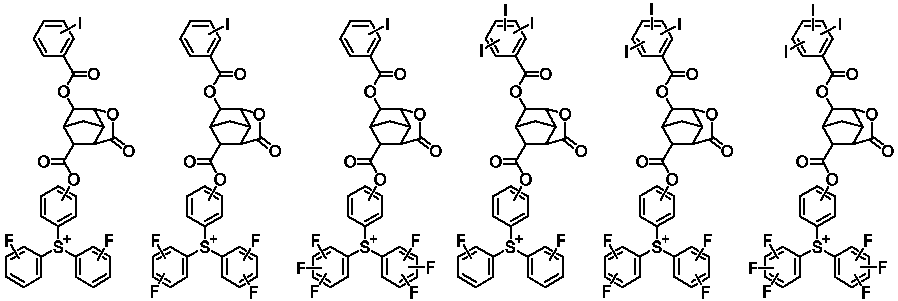

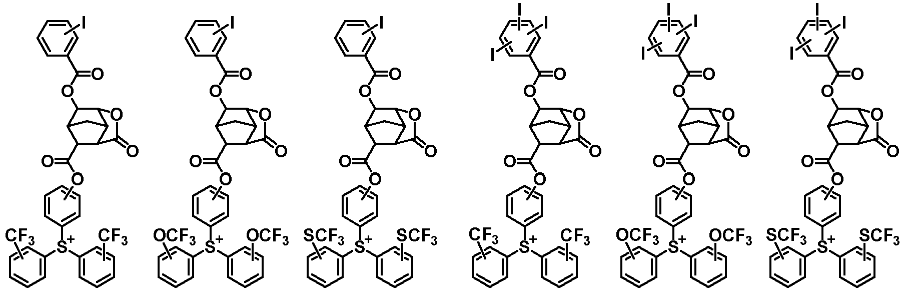

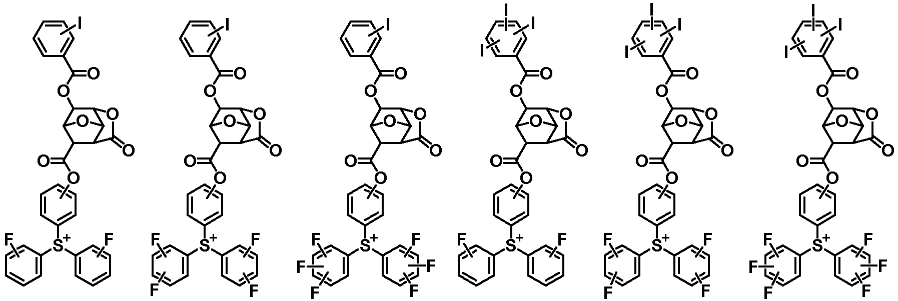

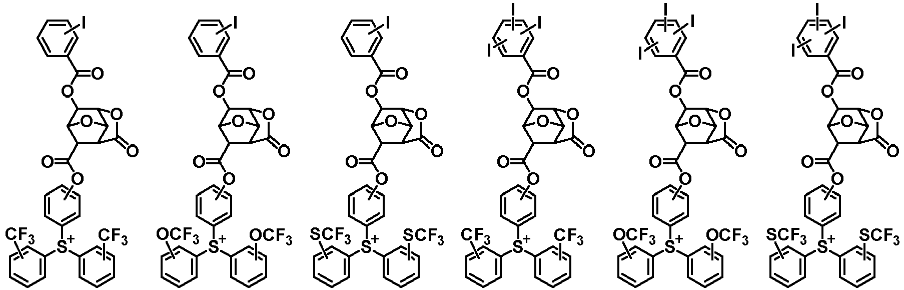

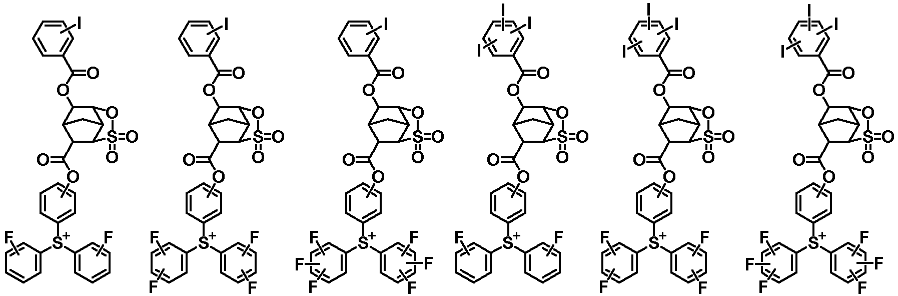

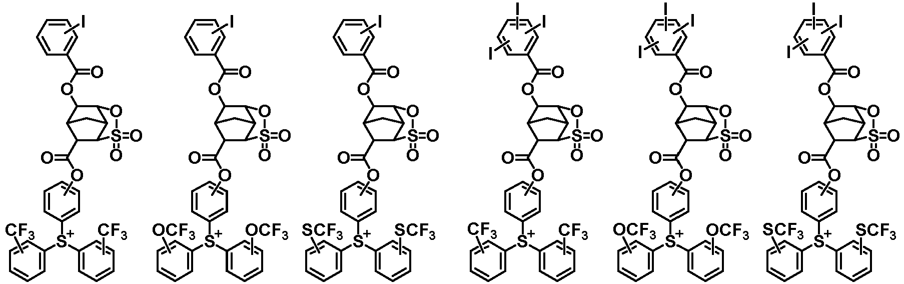

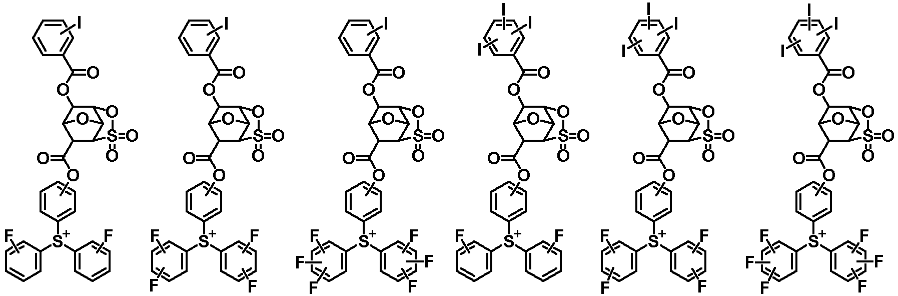

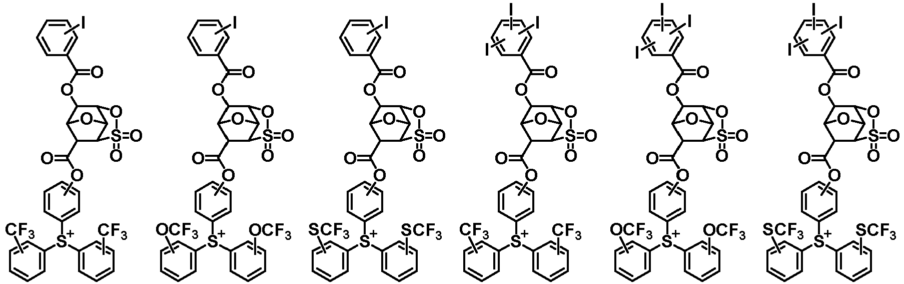

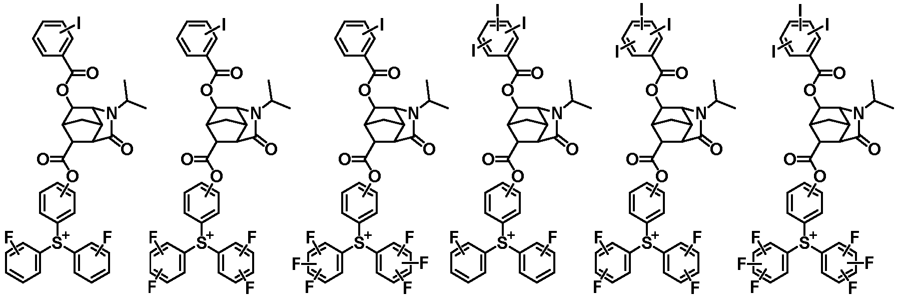

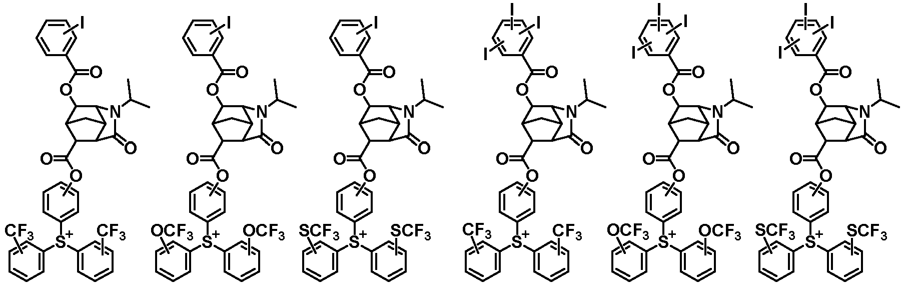

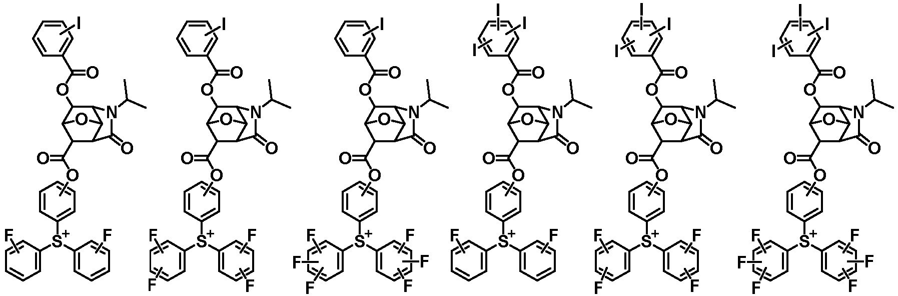

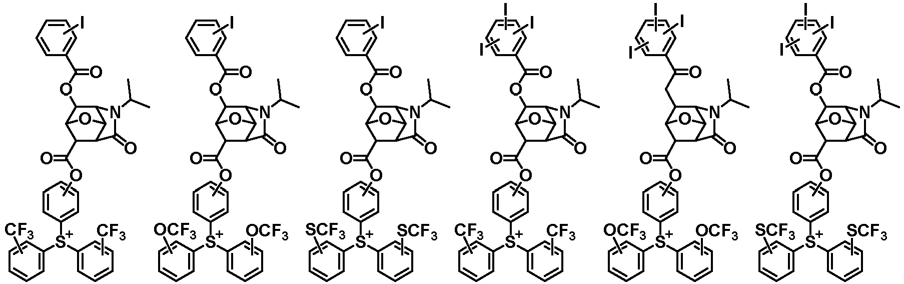

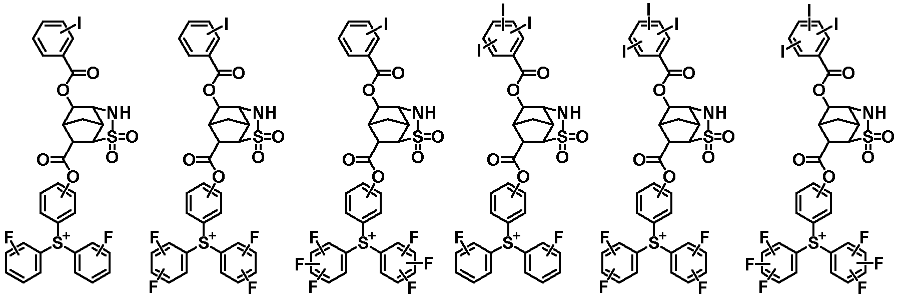

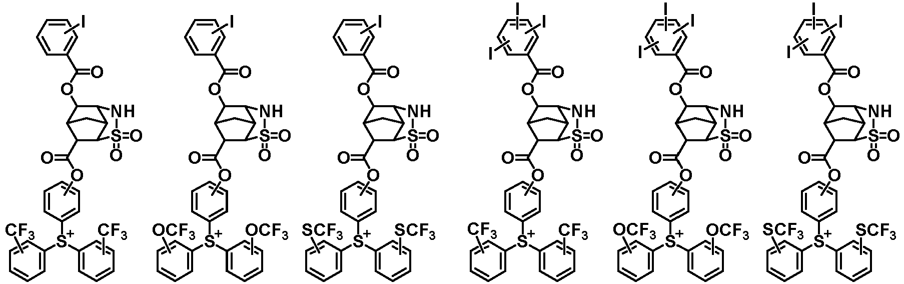

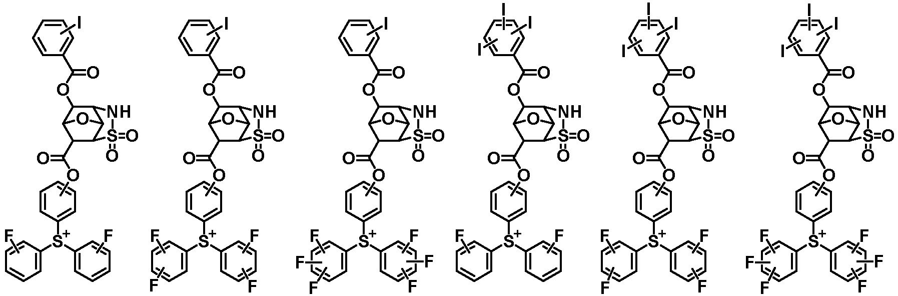

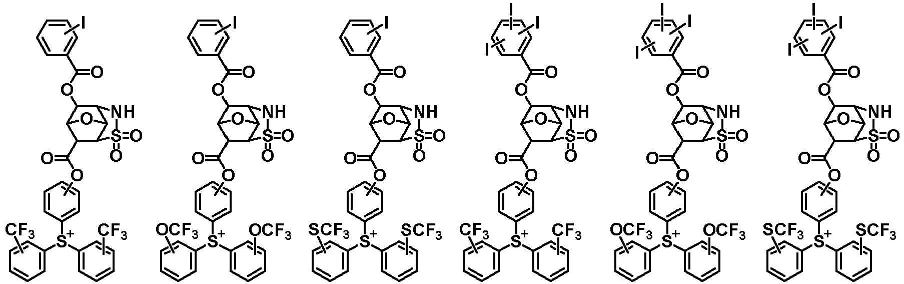

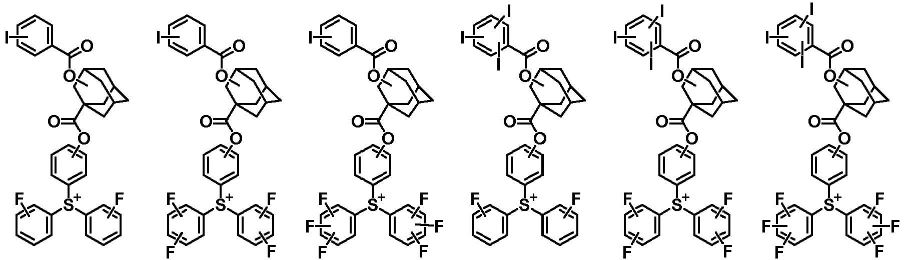

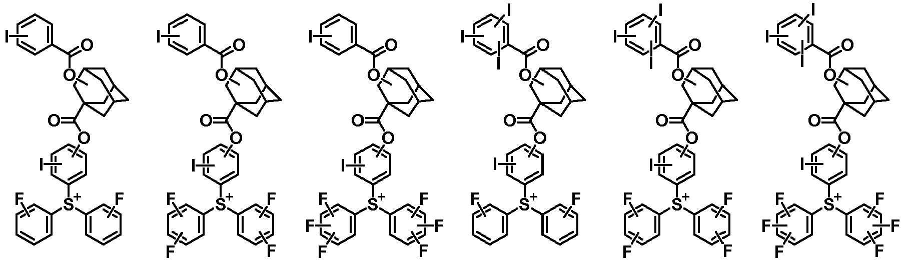

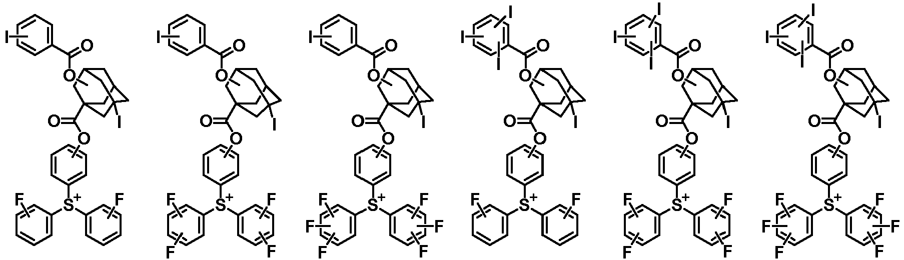

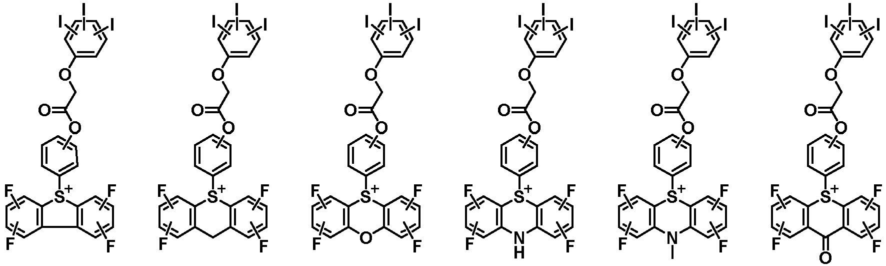

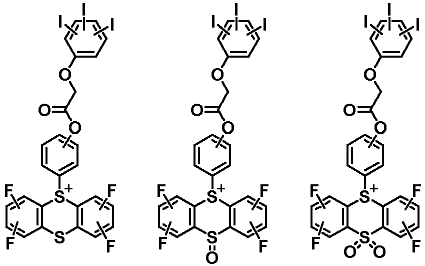

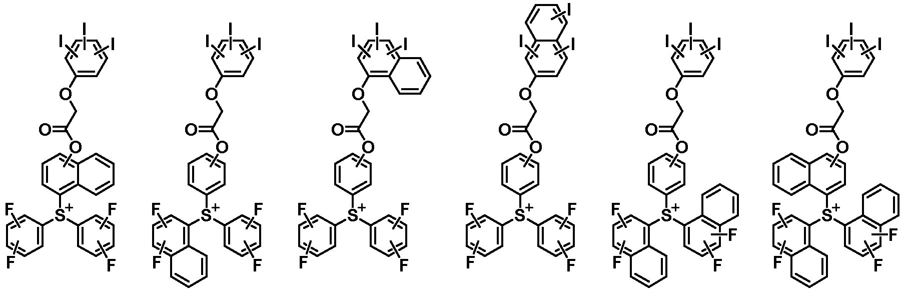

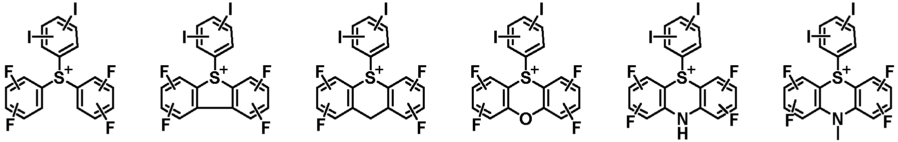

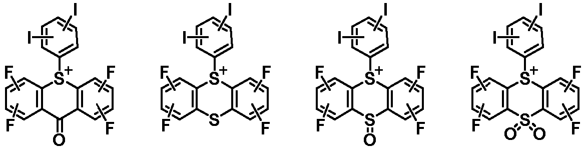

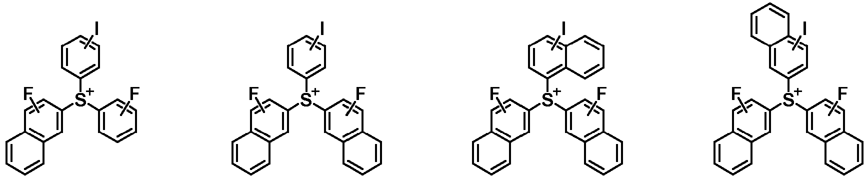

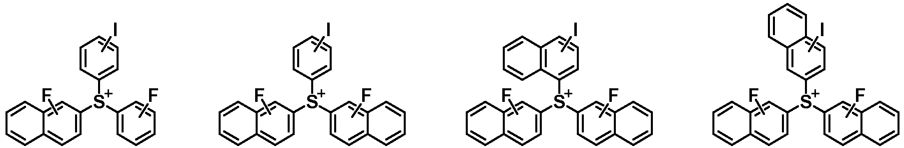

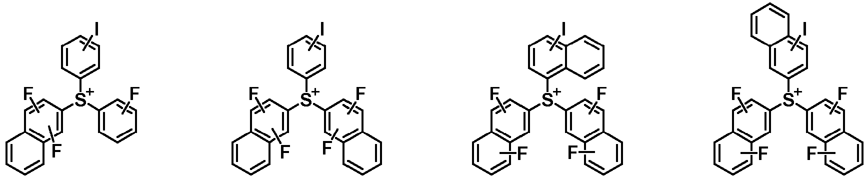

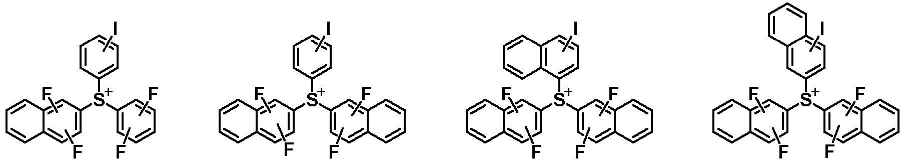

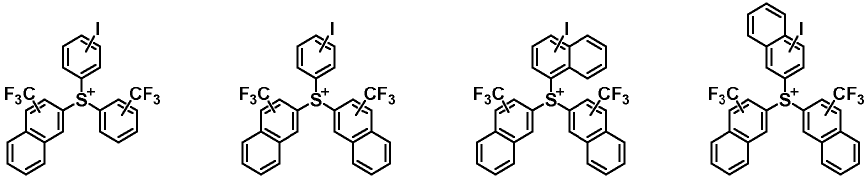

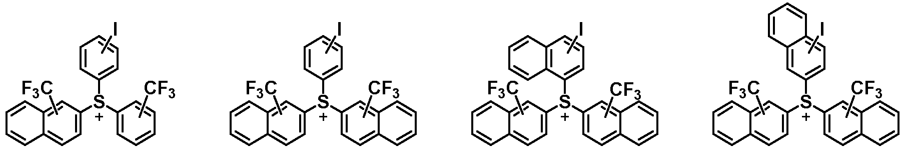

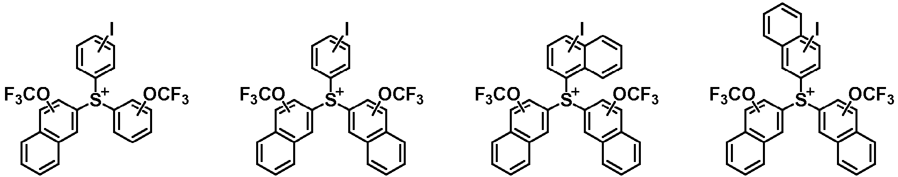

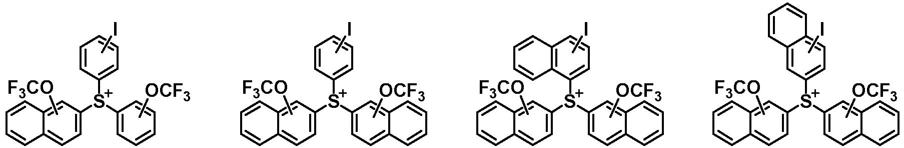

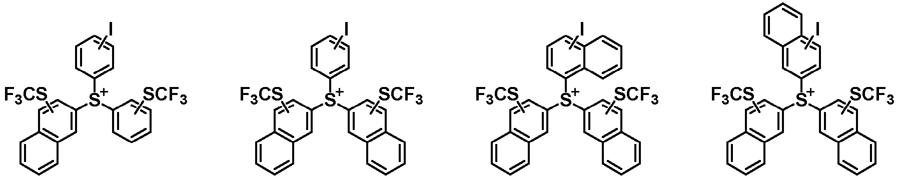

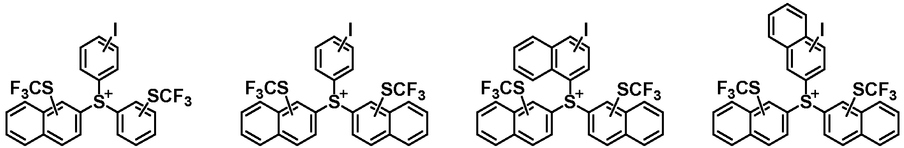

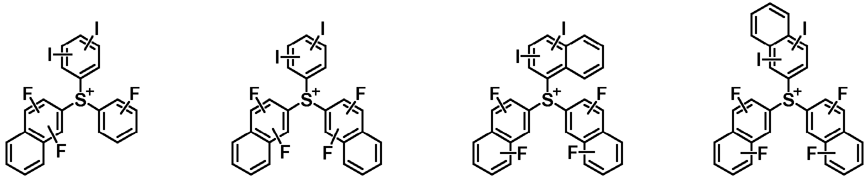

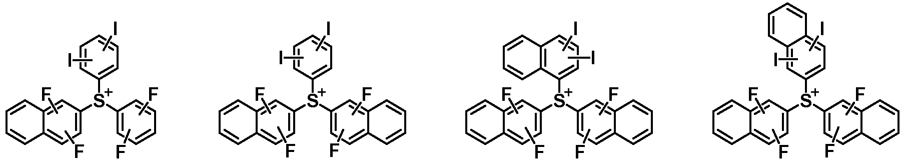

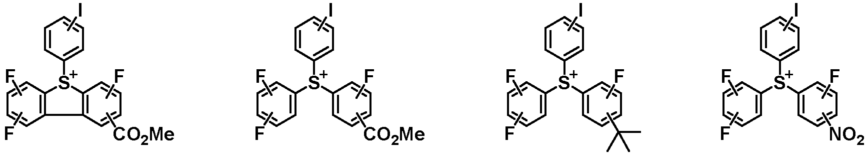

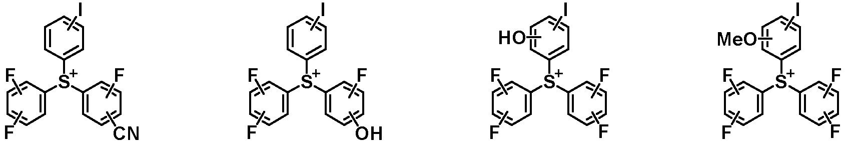

Illustrative structures of repeat units B6 to B10 include arbitrary combinations of anions with cations, both as mentioned above.

The repeat units B6 to B10 are capable of generating an acid upon exposure to high-energy radiation. It is believed that binding of the relevant units to a polymer enables to appropriately control acid diffusion and to form a pattern with reduced LER. Since the acid-generating unit is bound to a polymer, the phenomenon that acid volatilizes from the exposed region and re-deposits on the unexposed region during bake in vacuum is suppressed. This is effective for reducing LER and for suppressing profile degradation due to unwanted film thickness loss in the unexposed region.

Of repeat units B6 to B10, repeat units B7 to B 10 are preferred for the processing of photomask blanks because an optimum acid strength is available for the suppression of acid diffusion and the design of an acid labile group on the polymer. The repeat units B7, B8 and B9 are more preferred

When repeat units B6 to B10 are included, their content is preferably 0.1 to 30 mol%, more preferably 0.5 to 20 mol% based on the overall repeat units of the polymer. Each of repeat units B6 to B10 may be of one type or a combination of plural types.

The content of repeat units having an aromatic ring structure is preferably at least 65 mol%, more preferably at least 75 mol%, even more preferably at least 85 mol% based on the overall repeat units of the polymer. When the polymer does not contain repeat units B6 to B 10, it is preferred that all units have an aromatic ring structure.

The polymer may further comprise (meth)acrylate units protected with an acid labile group or (meth)acrylate units having an adhesive group such as lactone structure or hydroxy group other than phenolic hydroxy as commonly used in the art. These repeat units are effective for fine adjustment of properties of a resist film, but not essential.

Examples of the (meth)acrylate unit having an adhesive group include repeat units having the following formulae (B11) to (B13), which are also referred to as repeat units B11 to B13. While these units do not exhibit acidity, they may be used as auxiliary units for providing adhesion to substrates or adjusting solubility.

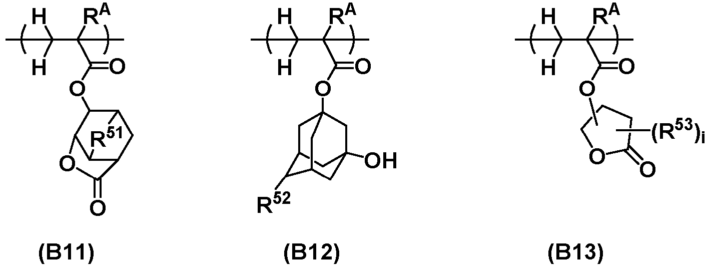

In formulae (B11) to (B13), R^{A} is each independently hydrogen, fluorine, methyl or trifluoromethyl. R⁵¹ is -O- or methylene. R⁵² is hydrogen or hydroxy. R⁵³ is a C₁-C₄ saturated hydrocarbyl group, and i is 0, 1, 2 or 3.

When repeat units B11 to B13 are included, their content is preferably 0 to 20 mol%, more preferably 0 to 10 mol% based on the overall repeat units of the polymer. Each of repeat units B11 to B13 may be of one type or a combination of plural types.

The polymer may be synthesized by combining suitable monomers optionally protected with a protective group, copolymerizing them in the standard way, and effecting deprotection reaction if necessary. The copolymerization reaction is preferably radical or anionic polymerization though not limited thereto. For the polymerization reaction, reference may be made to JP-A 2004-115630, for example.

The polymer should preferably have a Mw of 1,000 to 50,000, and more preferably 2,000 to 20,000. A Mw of at least 1,000 eliminates the risk that pattern features are rounded at their top to invite degradations of resolution and LER. A Mw of up to 50,000 eliminates the risk that LER is degraded when a pattern with a line width of up to 100 nm is formed. As used herein, Mw is measured by GPC versus polystyrene standards using tetrahydrofuran (THF) or dimethylformamide (DMF) solvent.

The polymer preferably has a narrow molecular weight distribution or dispersity (Mw/Mn) of 1.0 to 2.0, more preferably 1.0 to 1.9, even more preferably 1.0 to 1.8. A polymer with such a narrow dispersity eliminates the risk that foreign particles are left on the pattern after development and the pattern profile is aggravated.

The base polymer is designed such that the dissolution rate in alkaline developer is preferably up to 10 nm/min, more preferably up to 7 nm/min, even more preferably up to 5 nm/min. In the advanced generation of lithography wherein the coating film on the substrate is in a thin film range of up to 100 nm, the influence of pattern film thickness loss during alkaline development becomes strong. When the polymer has an alkaline dissolution rate of greater than 10 nm/min, pattern collapse occurs, i.e., a small-size pattern cannot be formed. The problem becomes outstanding in the fabrication of photomasks requiring to be defectless and having a tendency of strong development process. It is noted that the dissolution rate of a base polymer in alkaline developer is computed by spin coating a 16.7 wt% solution of a polymer in propylene glycol monomethyl ether acetate (PGMEA) solvent onto a 8-inch silicon wafer, baking at 100°C for 90 seconds to form a film of 1,000 nm thick, developing the film in a 2.38 wt% aqueous solution of tetramethylammonium hydroxide (TMAH) at 23°C for 100 seconds, and measuring a loss of film thickness.

In addition to the polymer defined above, the base polymer (B) may contain another polymer. The other polymer may be any of prior art well-known base polymers used in resist compositions. The content of the other polymer is not particularly limited as long as the benefits of the invention are not impaired.

### (C) Organic solvent

The resist composition may comprise an organic solvent as component (C). The organic solvent (C) is not particularly limited as long as the foregoing and other components are soluble therein. Suitable solvents include ketones such as cyclopentanone, cyclohexanone, methyl-2-n-pentyl ketone, and 2-heptanone; alcohols such as 3-methoxybutanol, 3-methyl-3-methoxybutanol, 1-methoxy-2-propanol, 1-ethoxy-2-propanol, and diacetone alcohol (DAA); ethers such as propylene glycol monomethyl ether (PGME), ethylene glycol monomethyl ether, propylene glycol monoethyl ether, ethylene glycol monoethyl ether, propylene glycol dimethyl ether, and diethylene glycol dimethyl ether; esters such as propylene glycol monomethyl ether acetate (PGMEA), propylene glycol monoethyl ether acetate, ethyl lactate (EL), ethyl pyruvate, butyl acetate, methyl 3-methoxypropionate, ethyl 3-ethoxypropionate, tert-butyl acetate, tert-butyl propionate, and propylene glycol mono-tert-butyl ether acetate; and lactones such as γ-butyrolactone (GBL), and mixtures thereof, as described in USP 7,537,880 (JP-A 2008-111103, paragraphs [0144]-[0145]). When an acid labile group of acetal type is used, a high-boiling alcohol solvent such as diethylene glycol, propylene glycol, glycerin, 1,4-butanediol or 1,3-butanediol may be added in order to accelerate the deprotection reaction of acetal.

Of the foregoing organic solvents, it is recommended to use 1-ethoxy-2-propanol, PGMEA, PGME, cyclohexanone, EL, GBL and mixtures thereof.

The organic solvent (C) is preferably added in an amount of 200 to 10,000 parts by weight, and more preferably 400 to 5,000 parts by weight per 80 parts by weight of the base polymer (B). The organic solvent may be used alone or in admixture.

### (D) Photoacid generator

The chemically amplified positive resist composition may further comprise a photoacid generator (PAG) as component (D). The PAG used herein may be any compound capable of generating an acid upon exposure to high-energy radiation. Suitable PAGs include sulfonium salts, iodonium salts, sulfonyldiazomethane, N-sulfonyloxyimide, and oxime-O-sulfonate acid generators.

Suitable PAGs include nonafluorobutane sulfonate, partially fluorinated sulfonates described in JP-A 2012-189977, paragraphs [0247]-[0251], partially fluorinated sulfonates described in JP-A 2013-101271, paragraphs [0261]-[0265], and those described in JP-A 2008-111103, paragraphs [0122]-[0142] and JP-A 2010-215608, paragraphs [0080]-[0081]. Among others, arenesulfonate and alkanesulfonate type PAGs are preferred because they generate acids having an appropriate strength to deprotect the acid labile group in repeat unit B2.

The preferred PAGs are salt compounds having a sulfonium anion of the structure shown below.

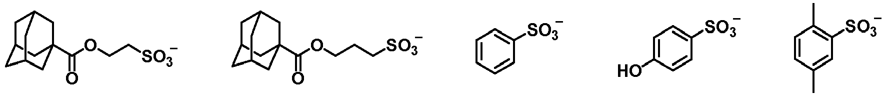

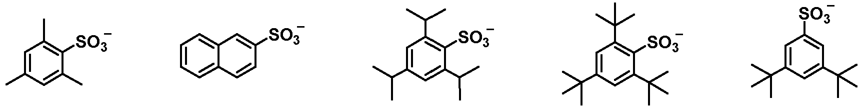

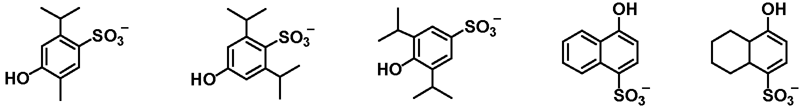

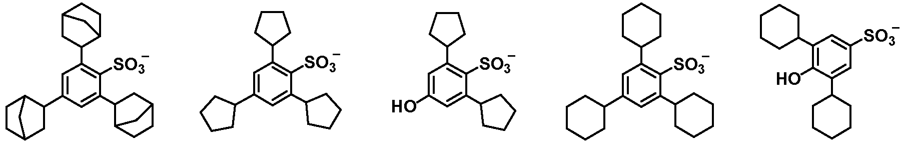

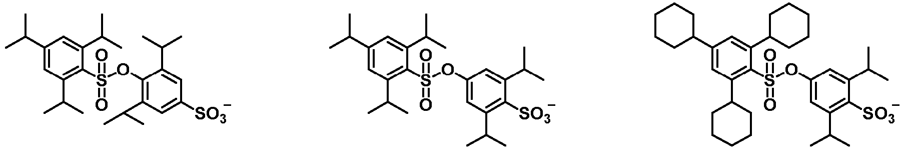

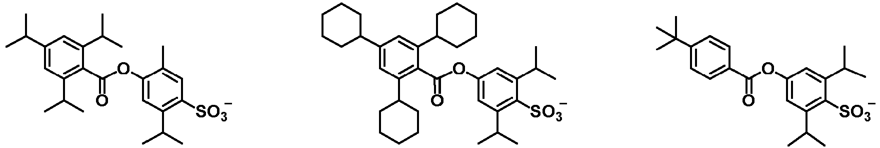

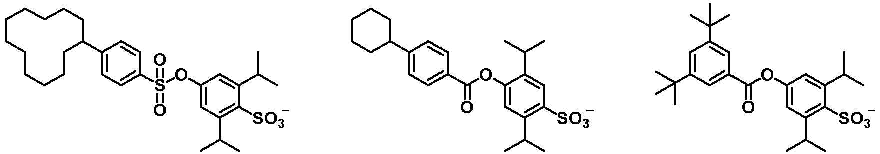

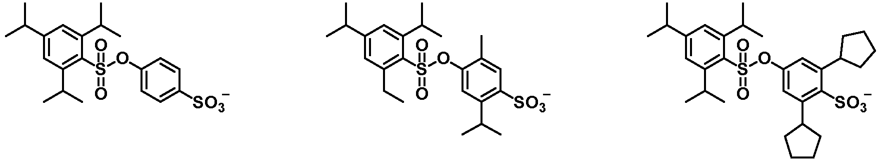

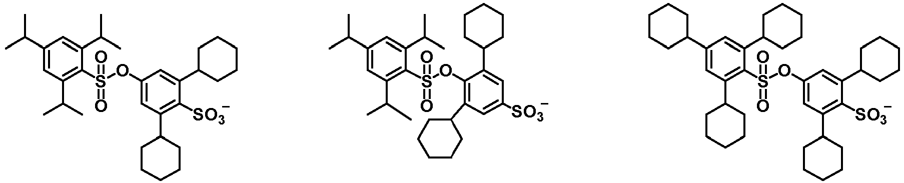

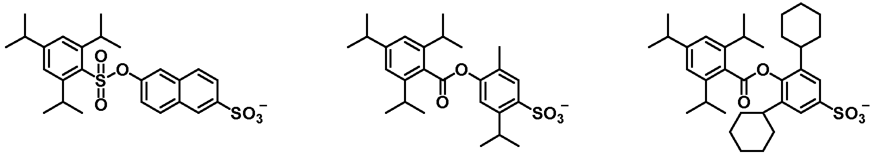

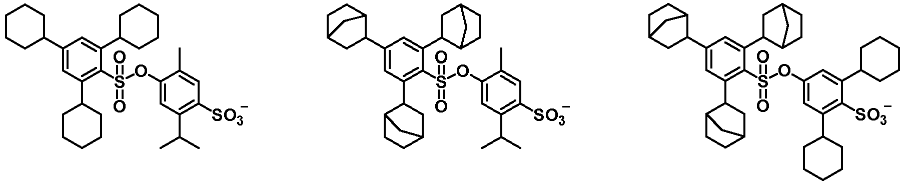

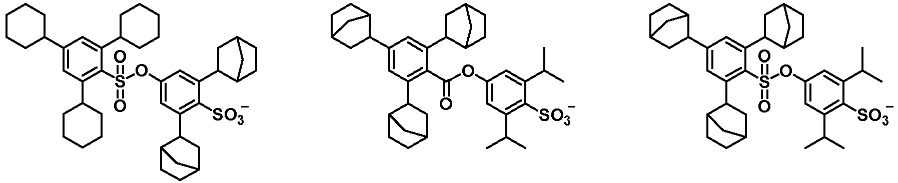

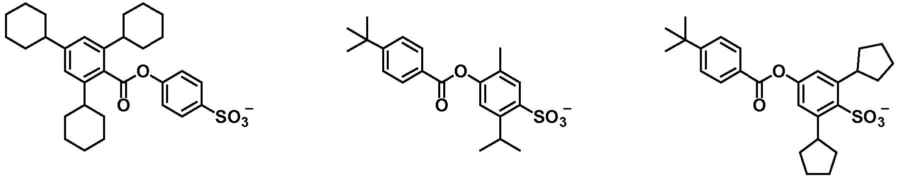

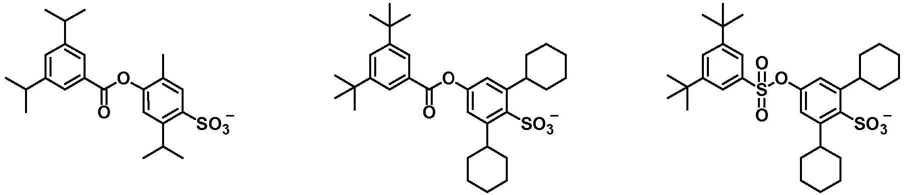

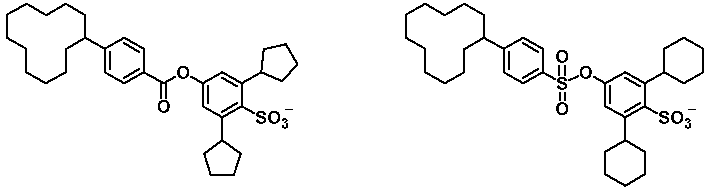

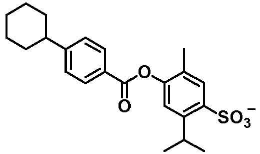

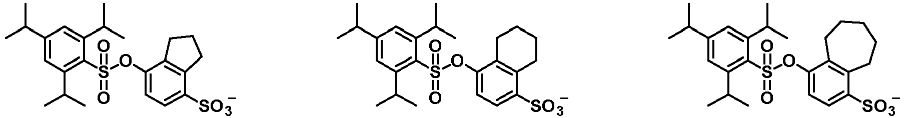

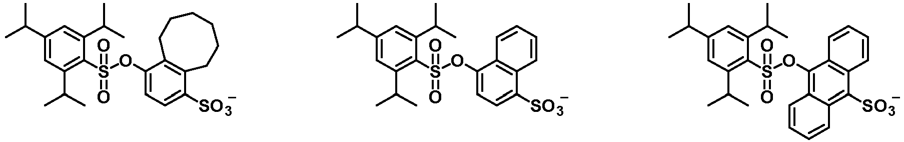

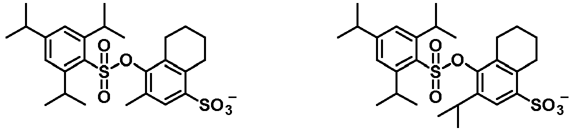

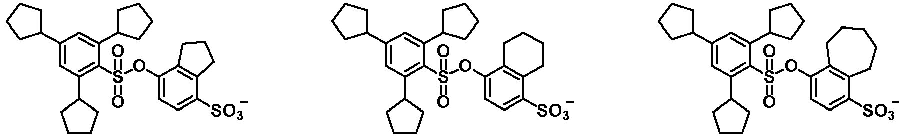

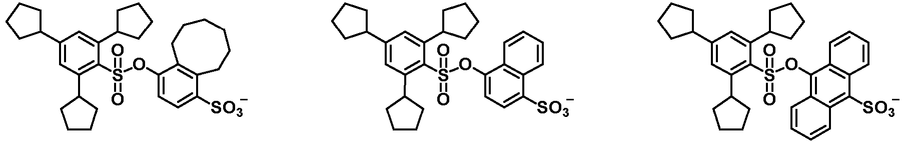

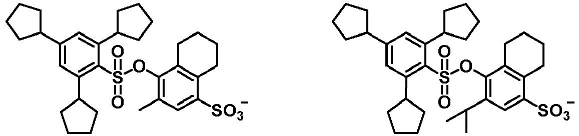

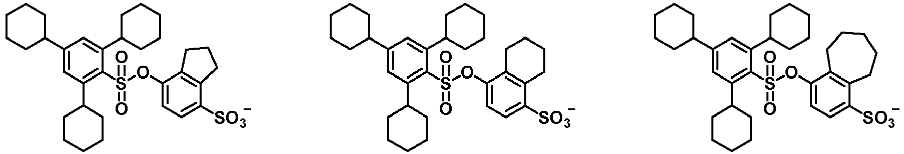

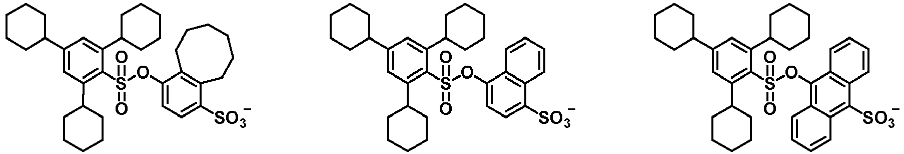

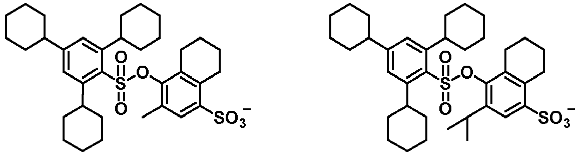

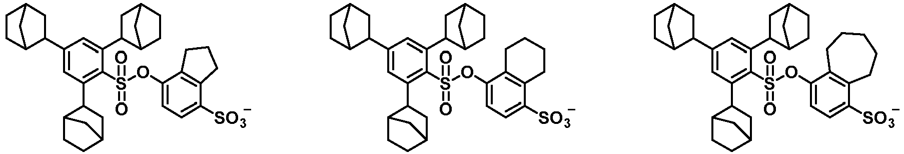

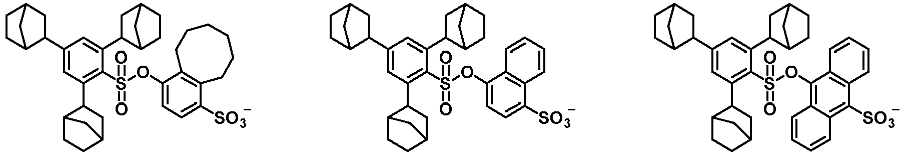

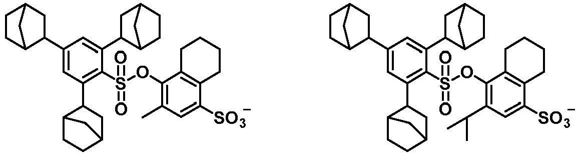

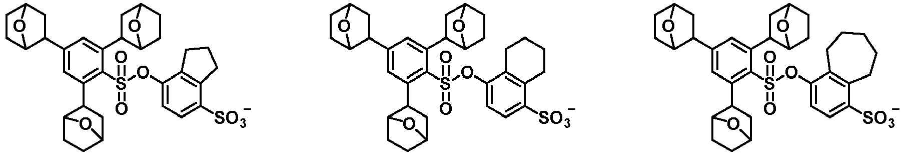

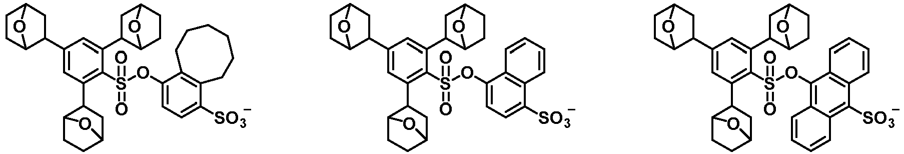

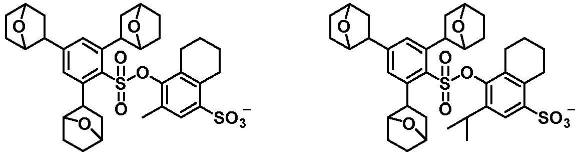

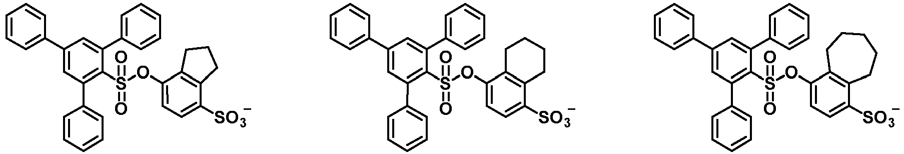

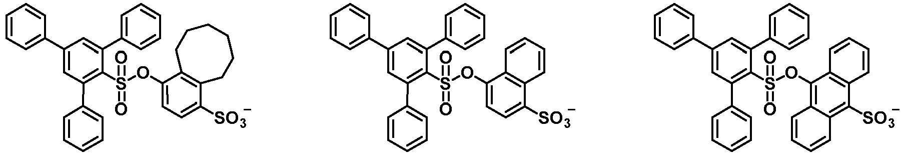

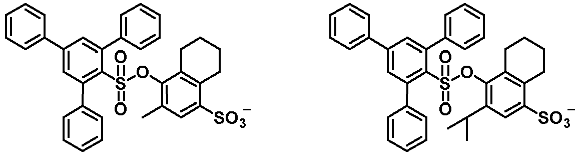

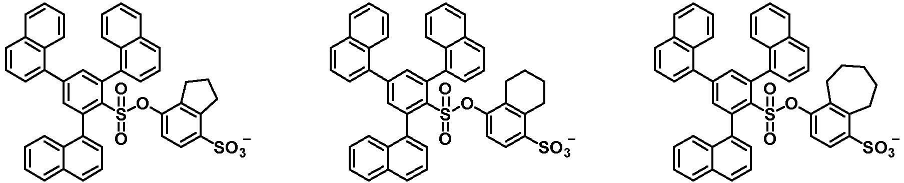

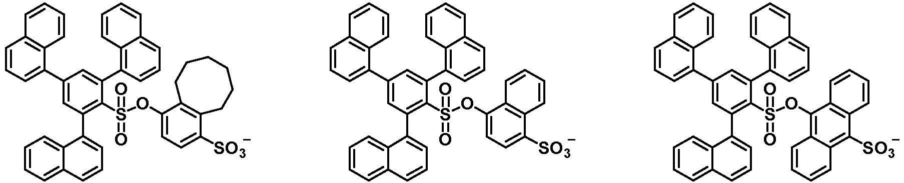

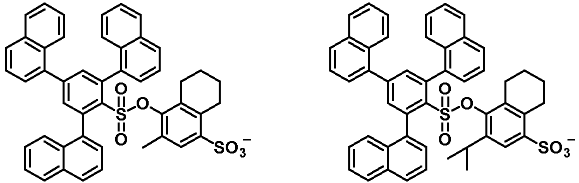

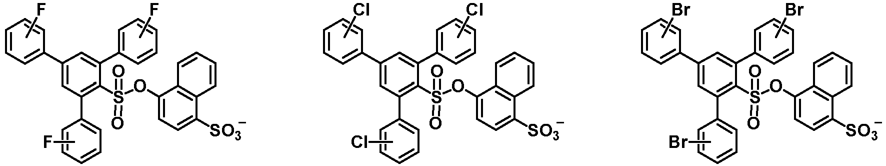

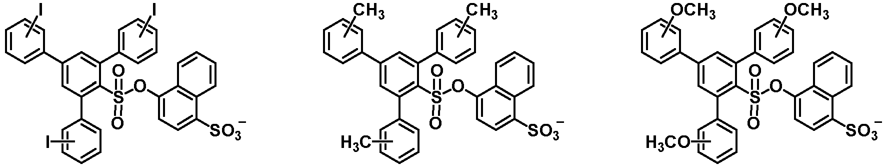

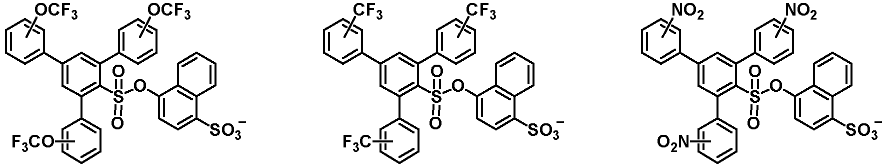

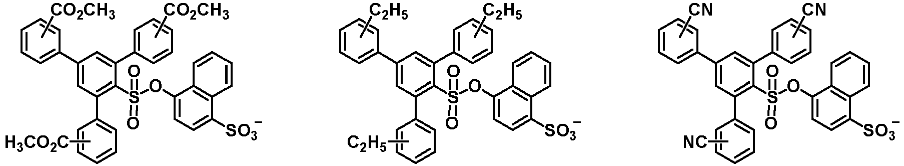

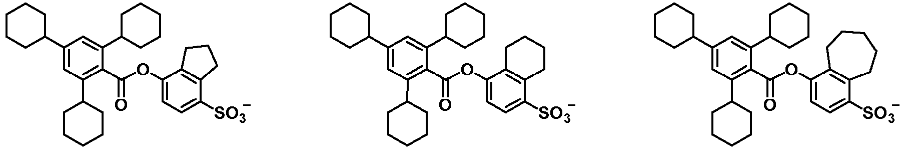

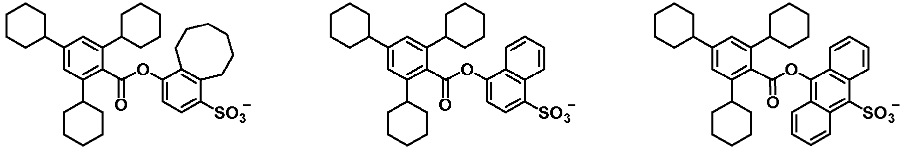

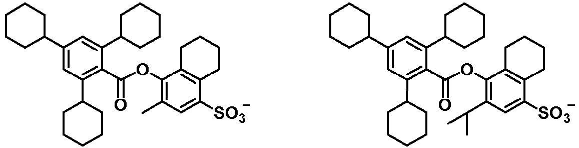

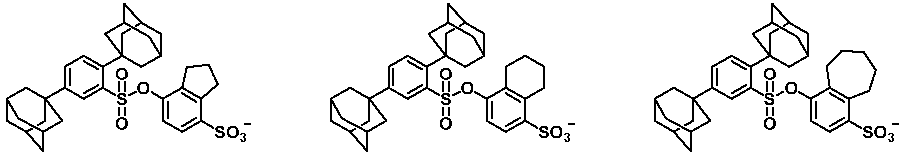

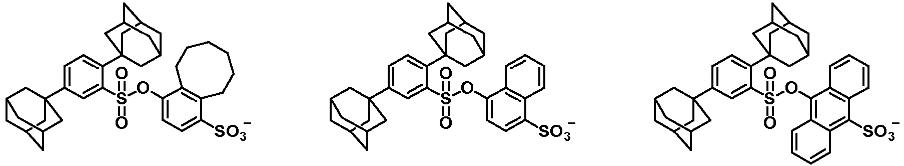

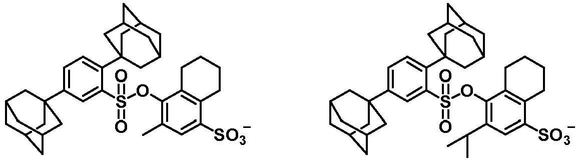

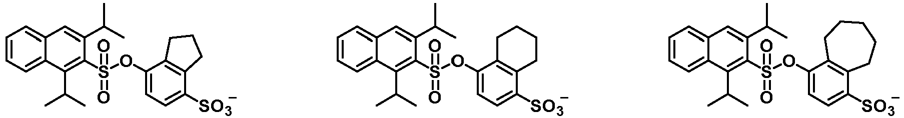

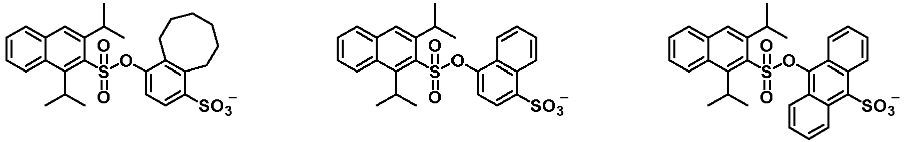

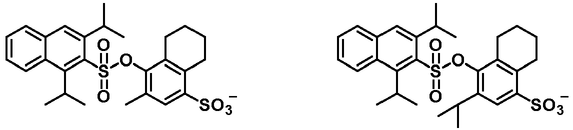

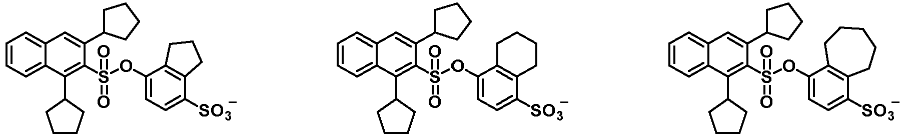

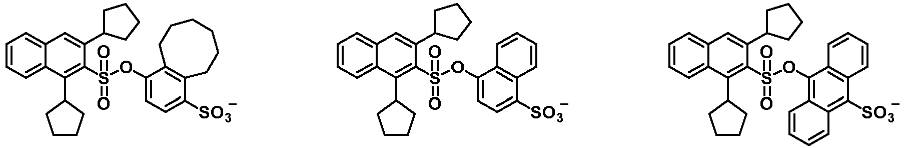

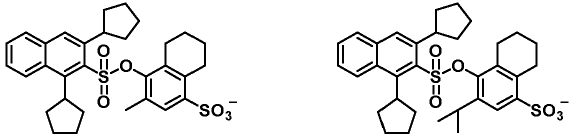

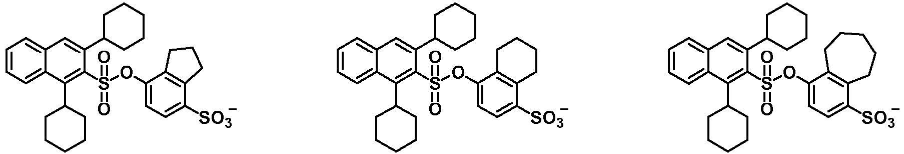

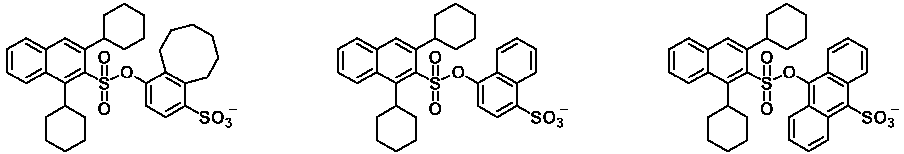

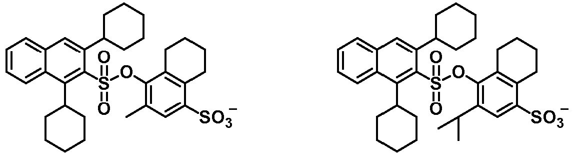

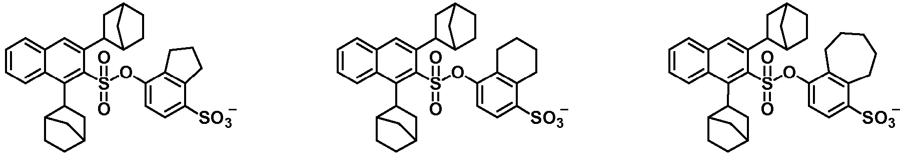

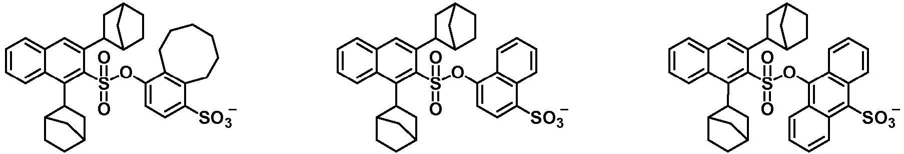

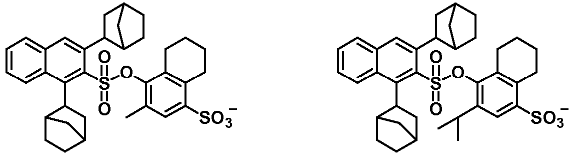

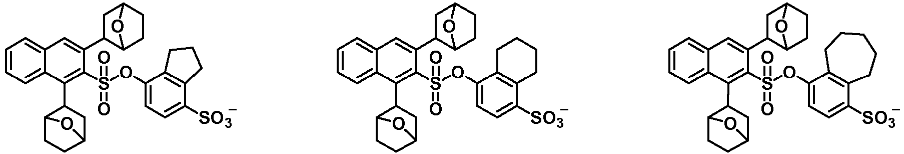

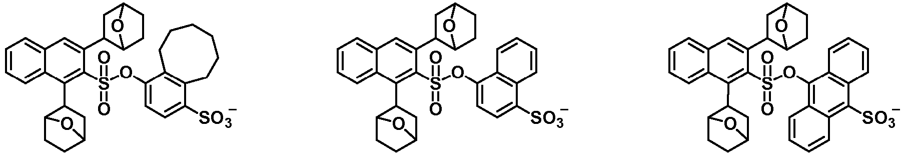

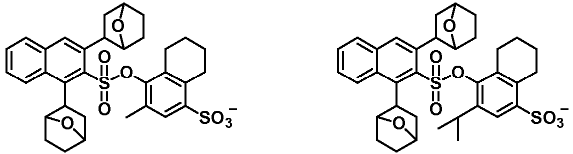

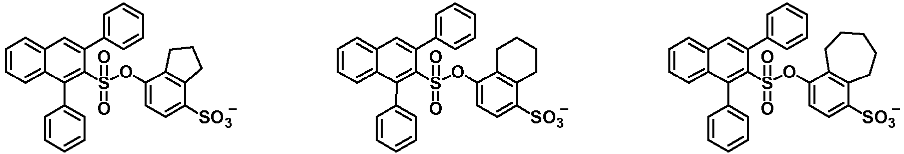

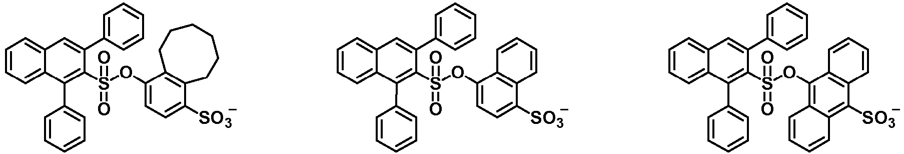

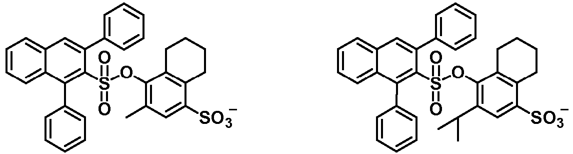

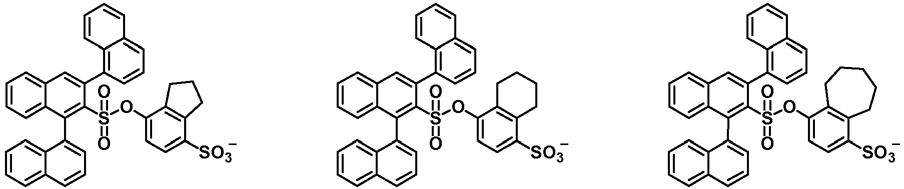

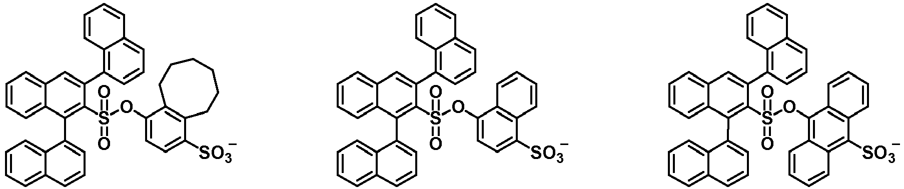

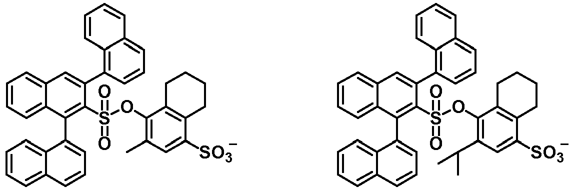

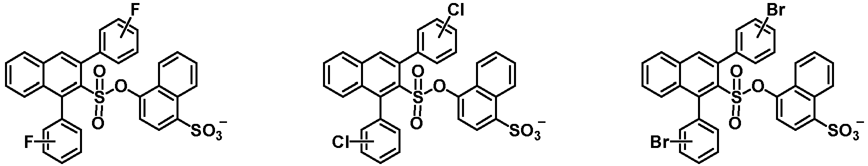

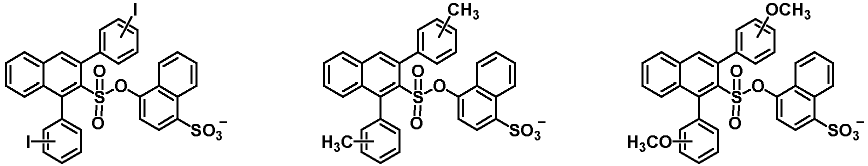

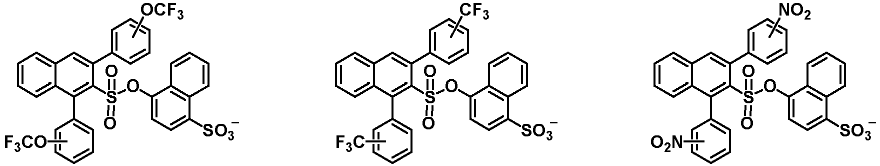

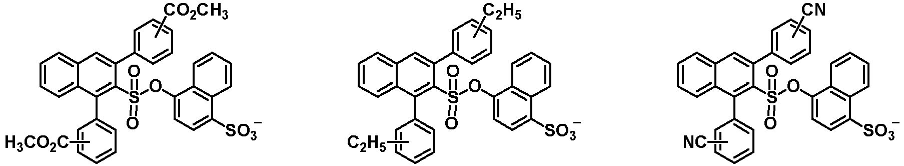

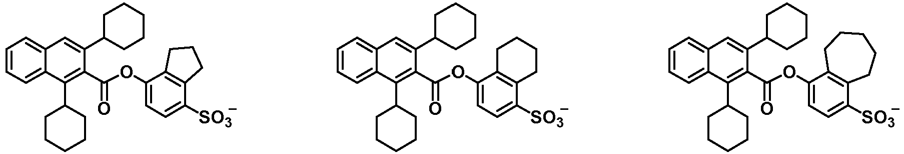

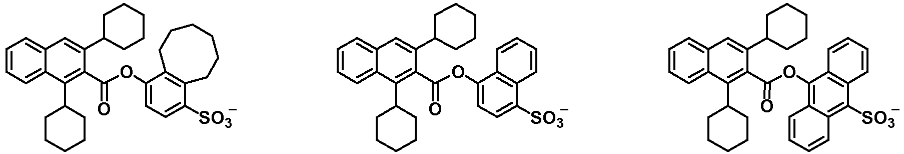

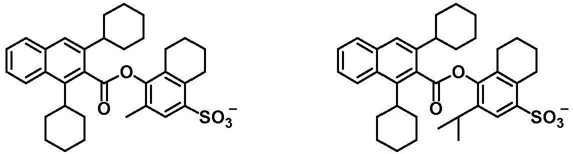

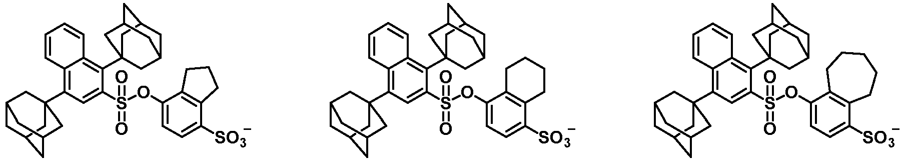

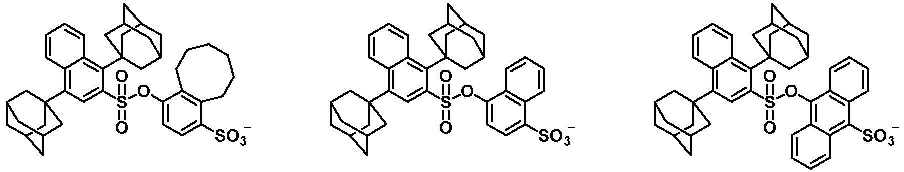

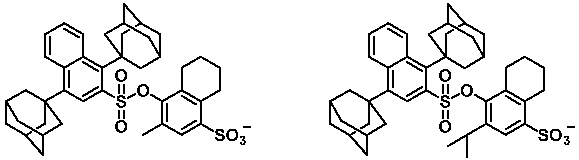

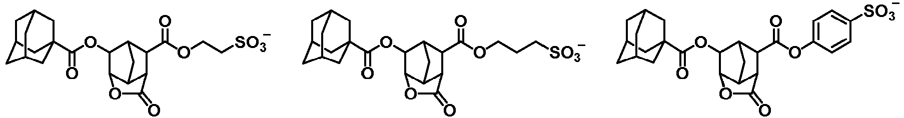

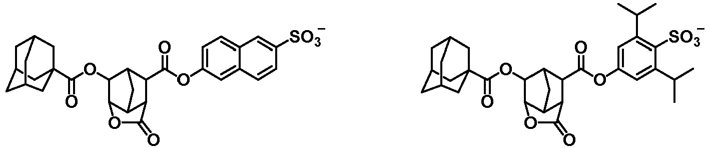

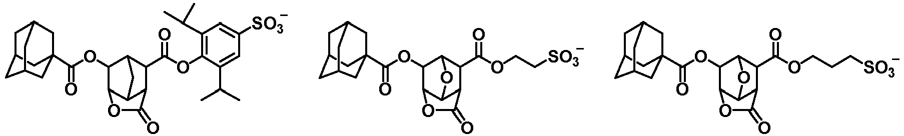

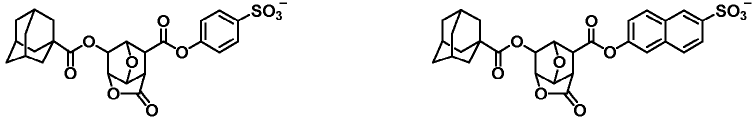

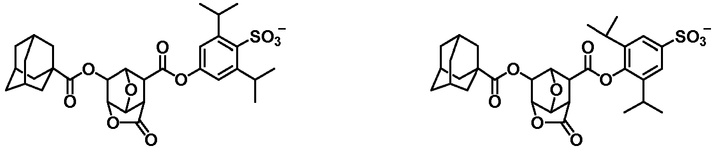

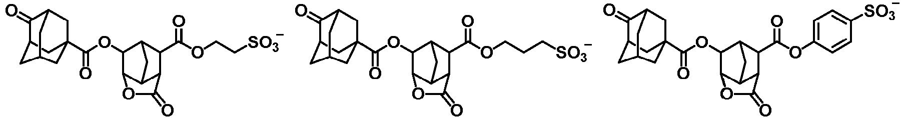

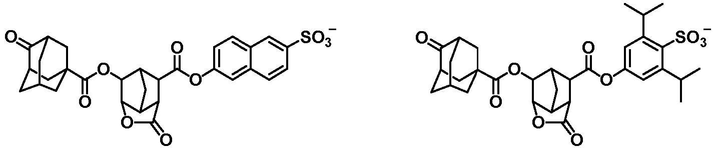

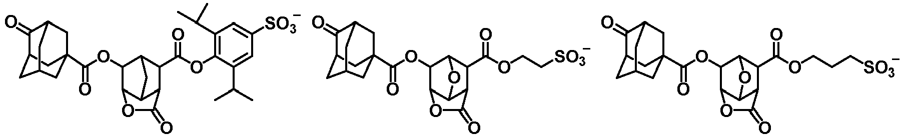

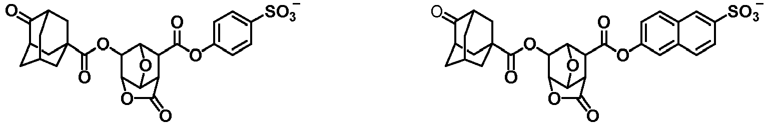

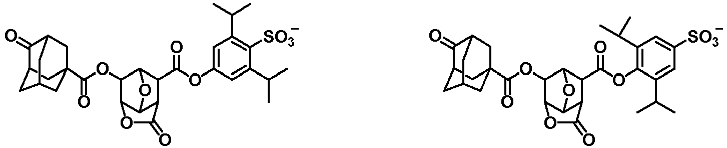

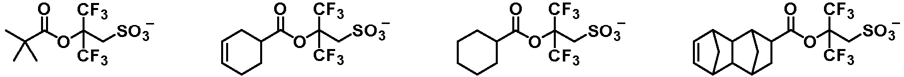

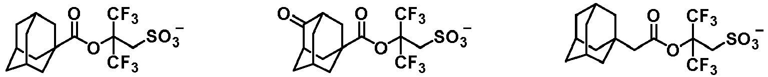

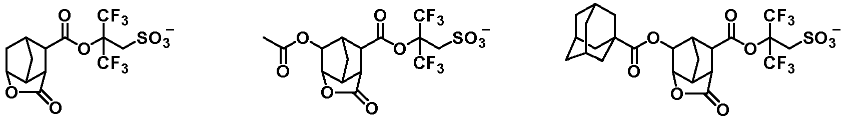

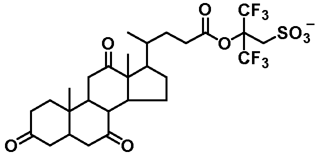

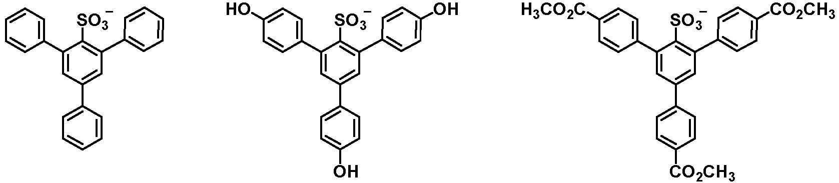

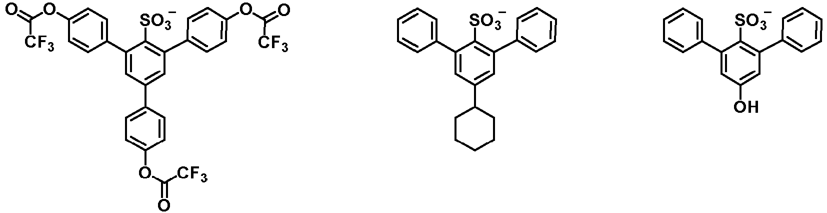

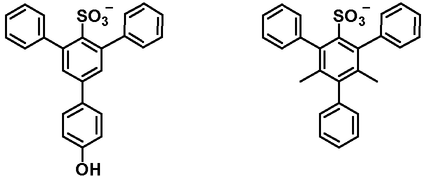

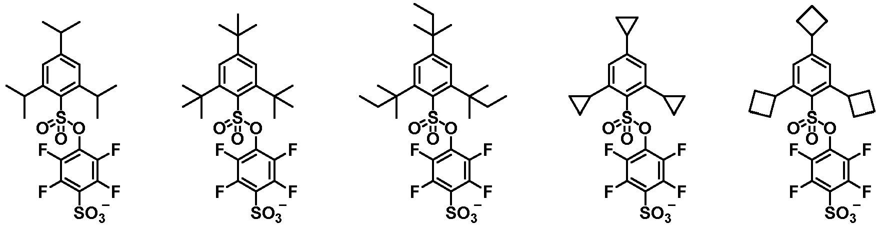

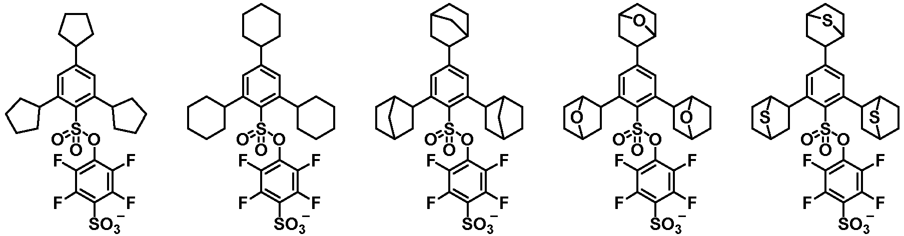

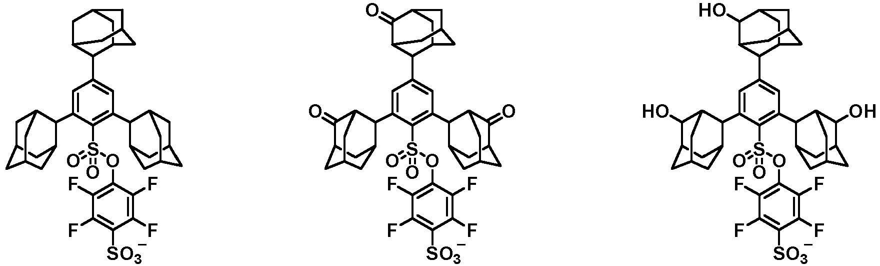

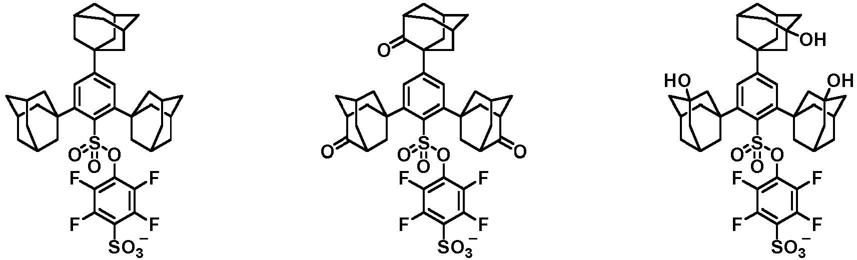

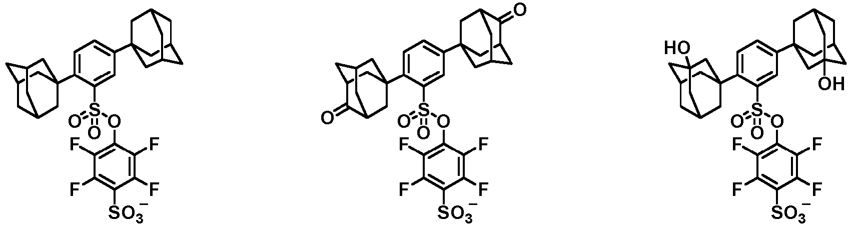

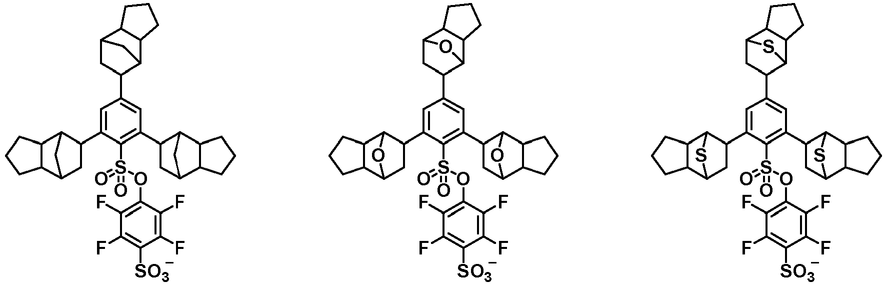

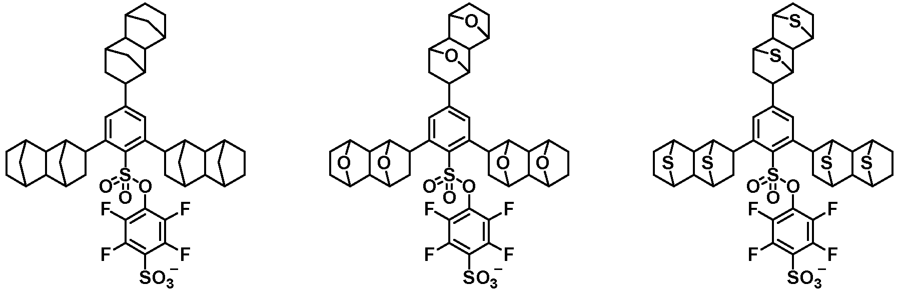

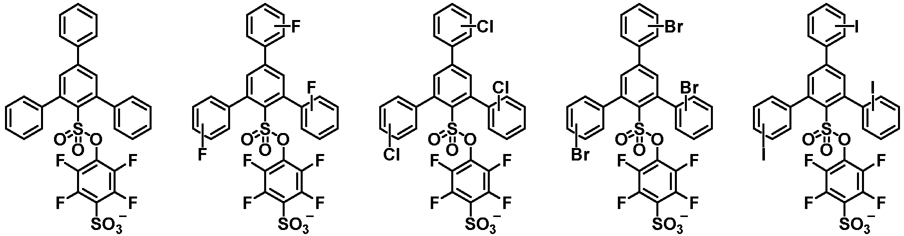

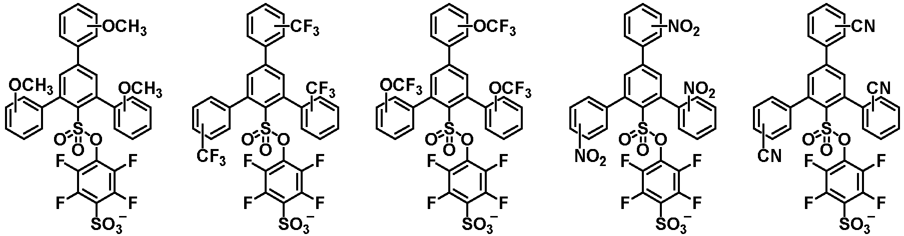

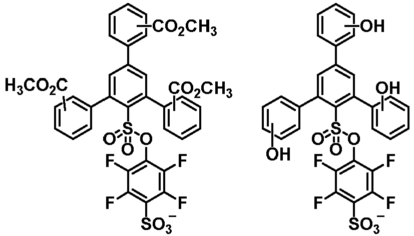

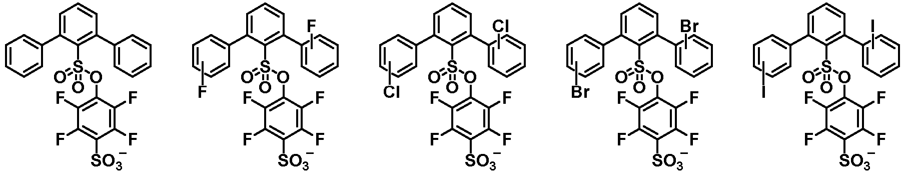

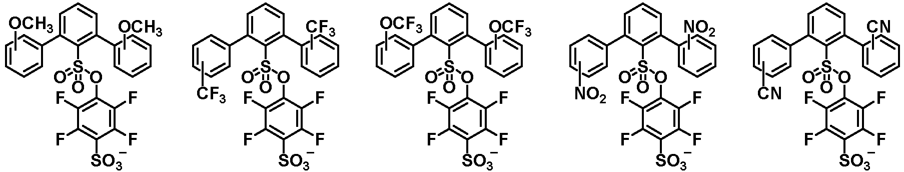

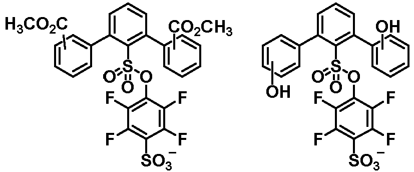

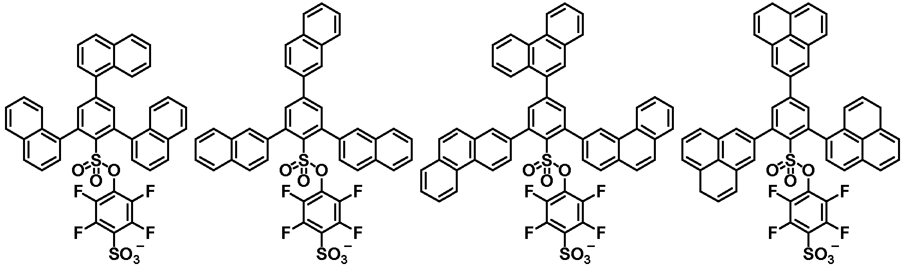

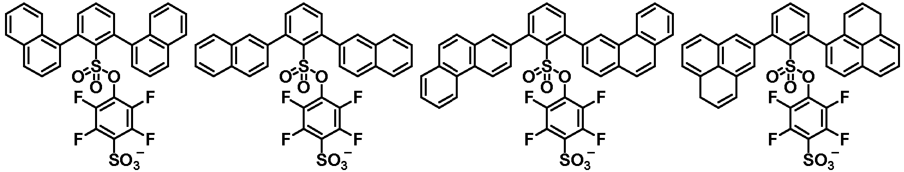

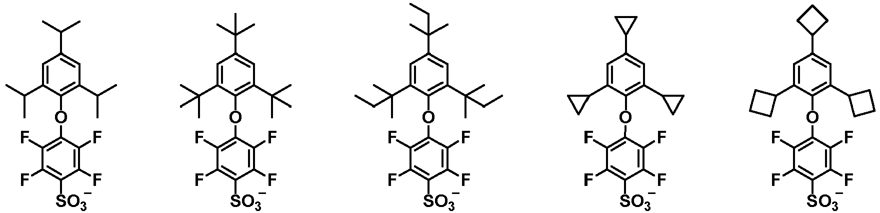

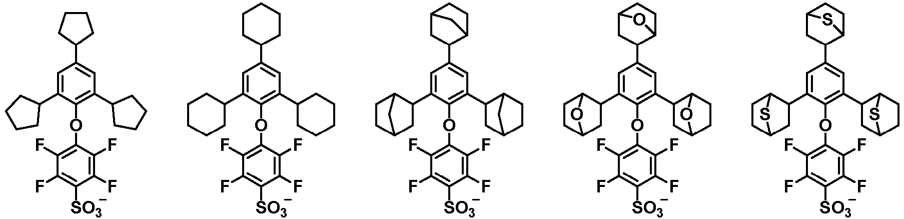

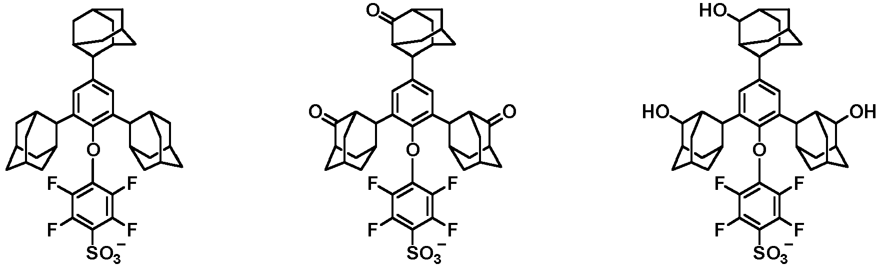

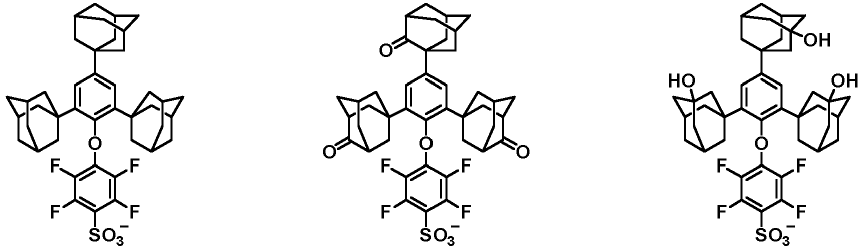

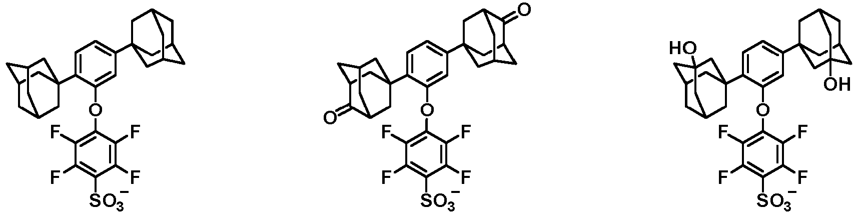

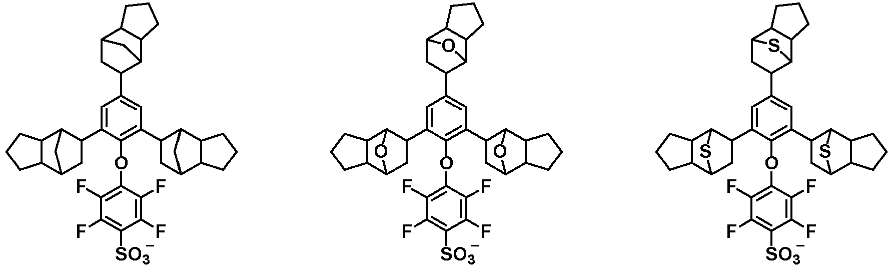

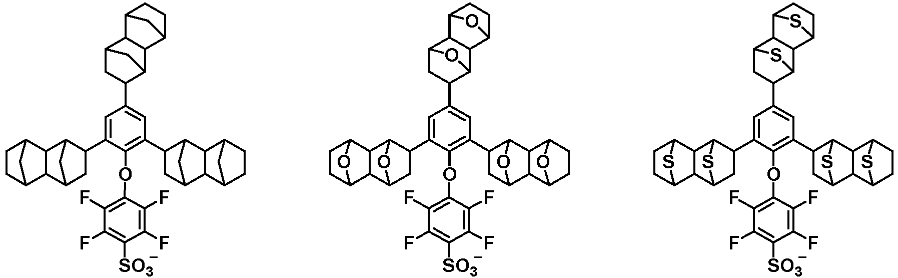

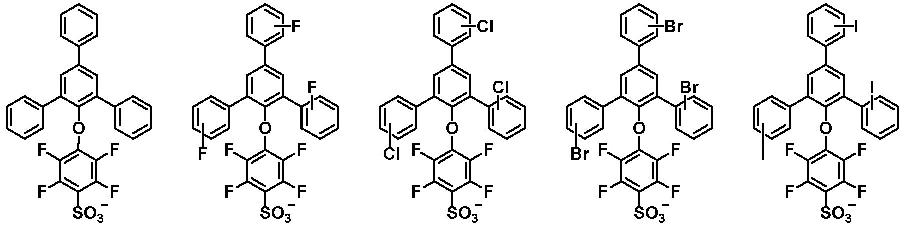

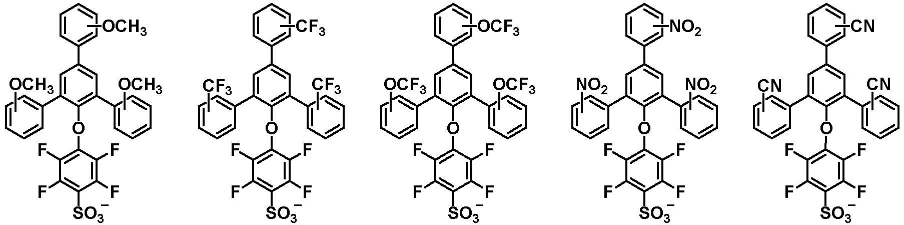

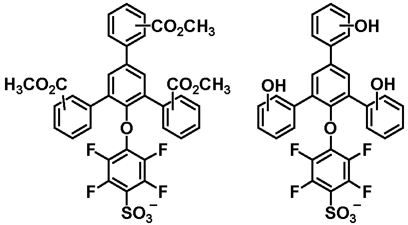

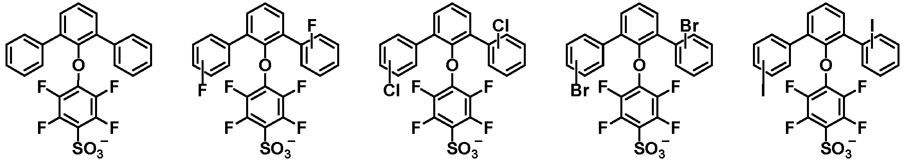

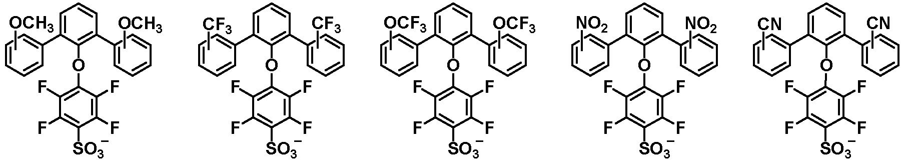

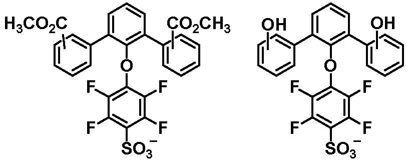

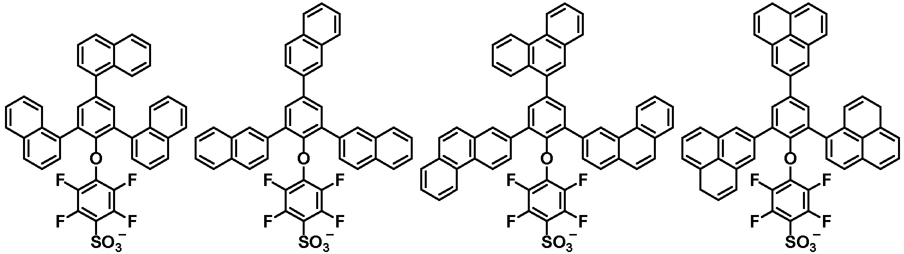

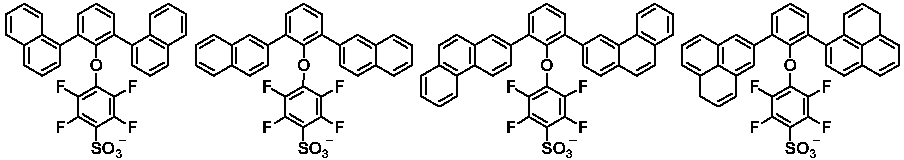

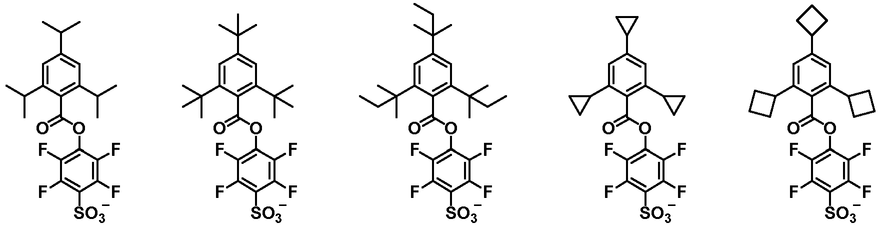

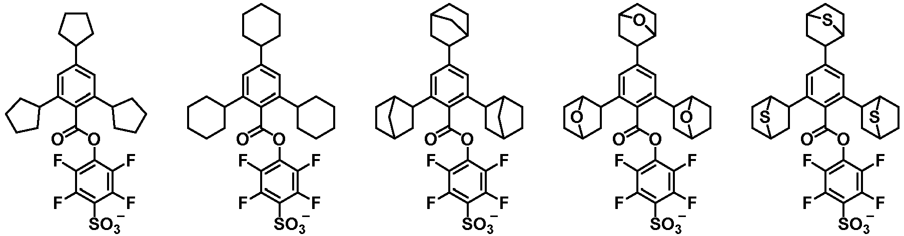

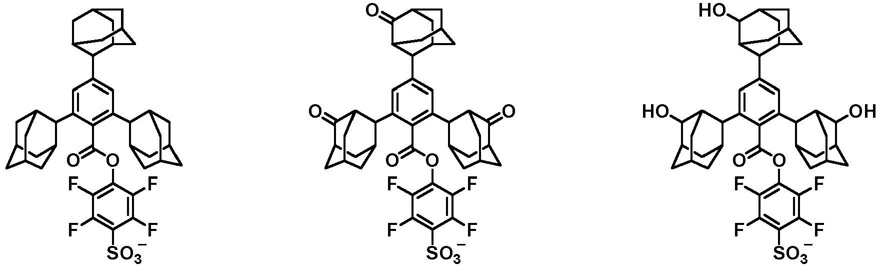

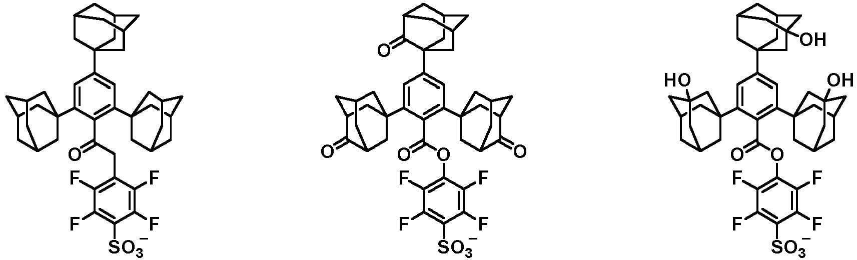

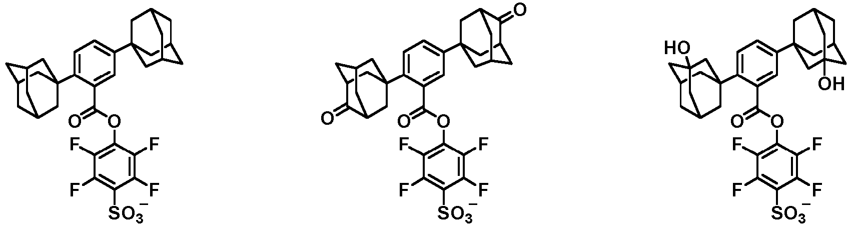

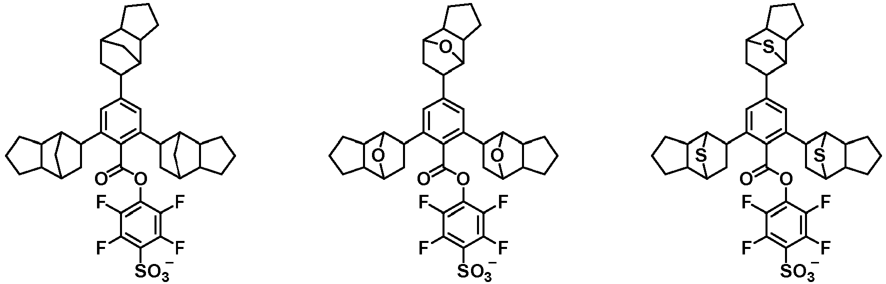

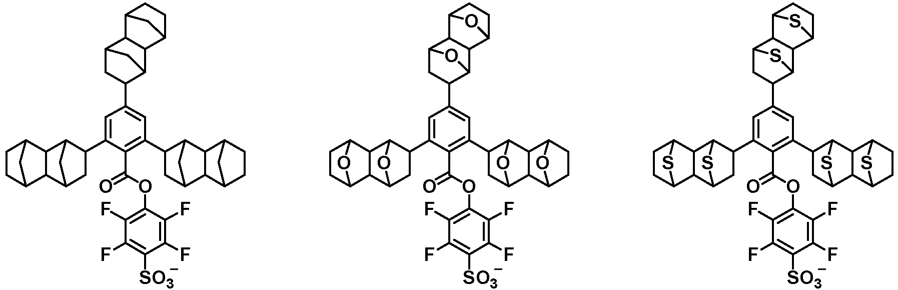

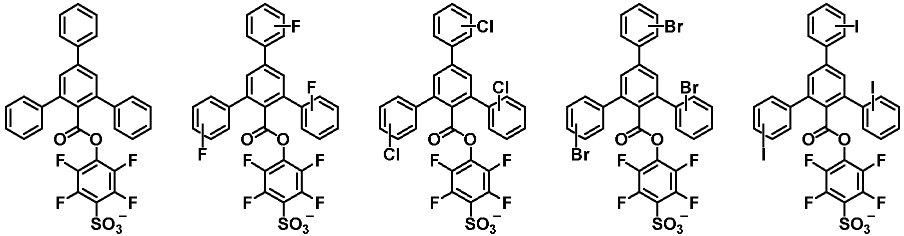

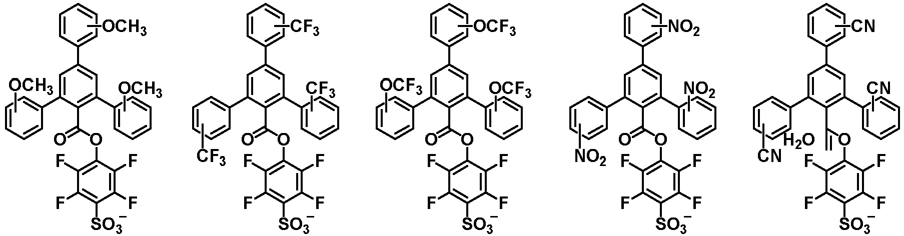

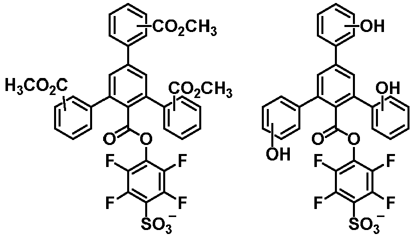

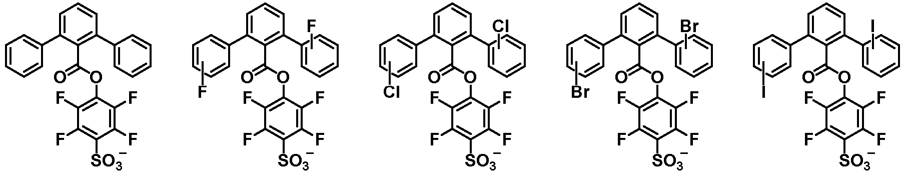

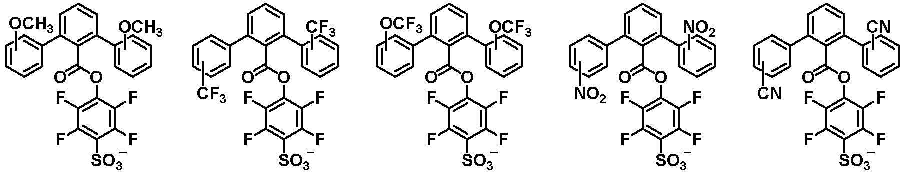

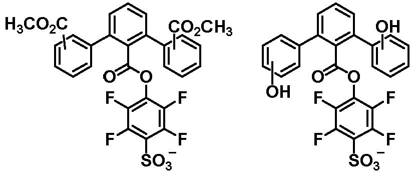

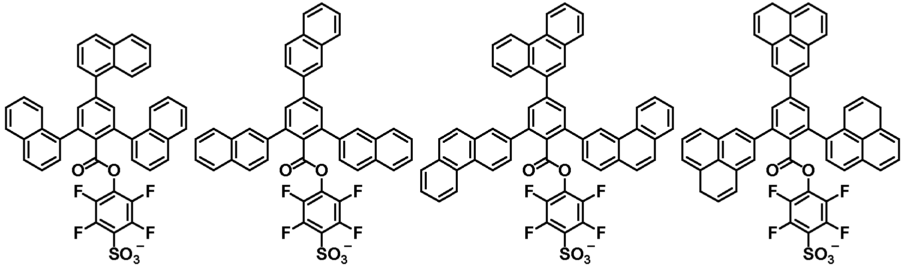

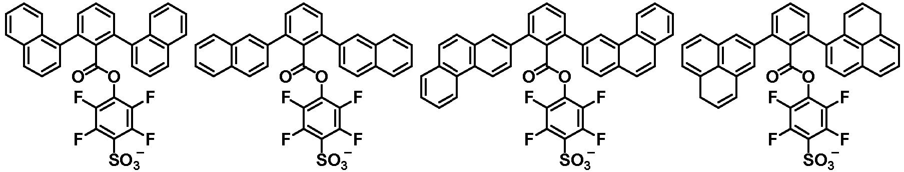

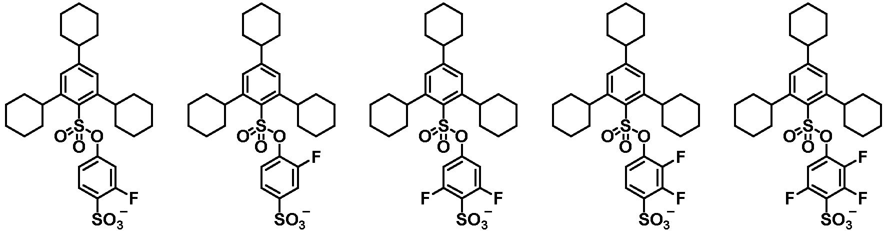

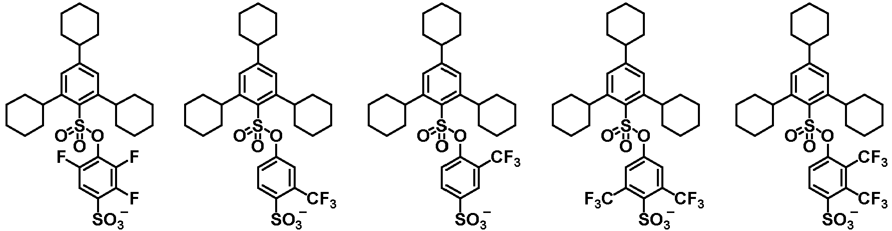

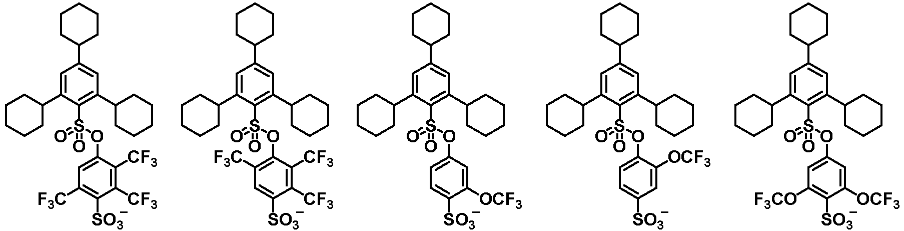

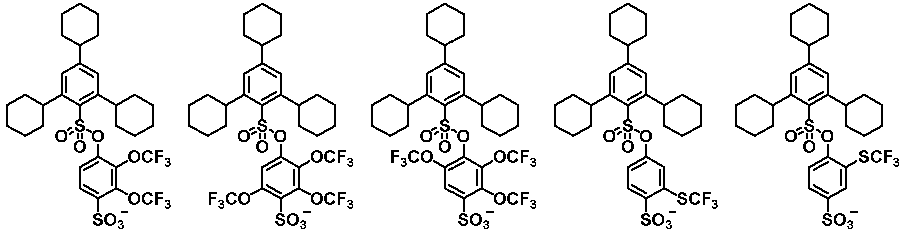

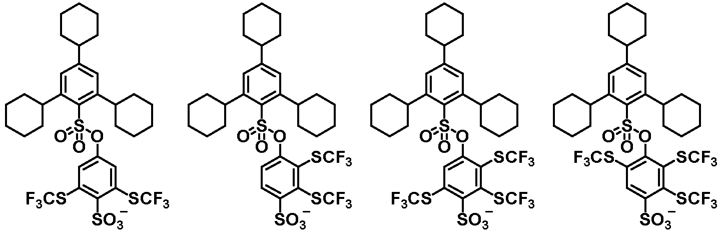

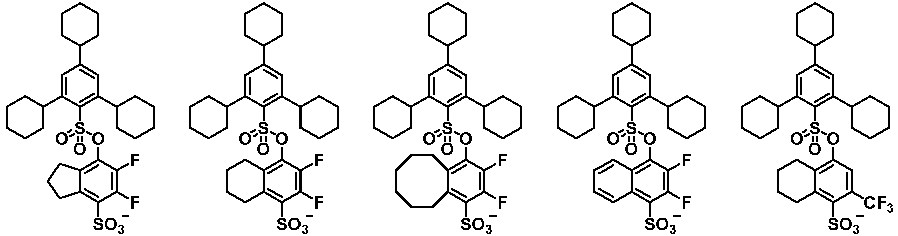

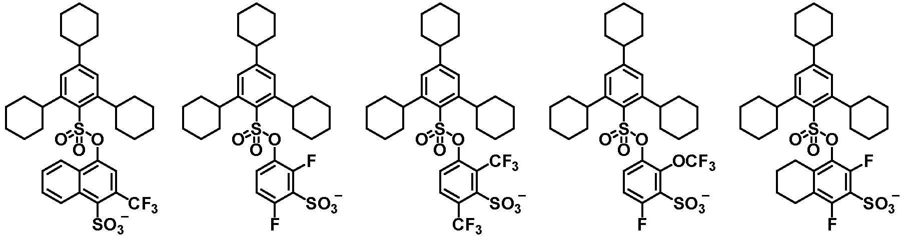

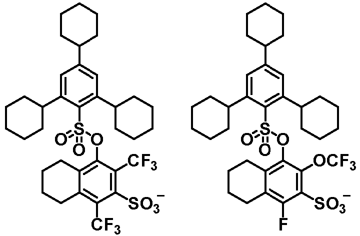

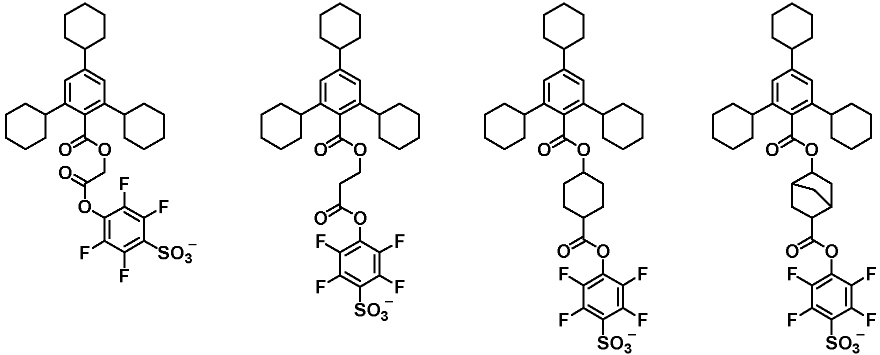

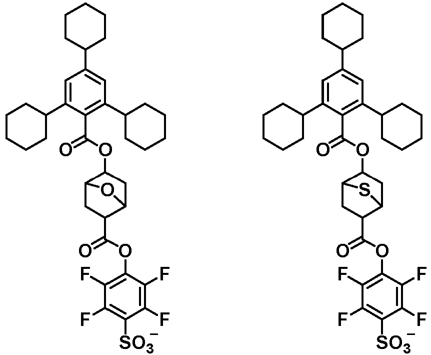

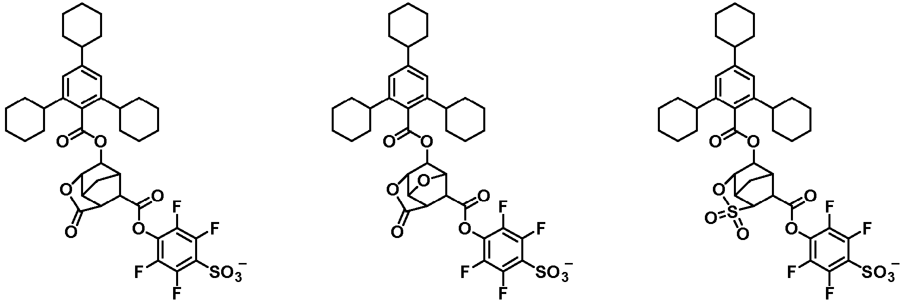

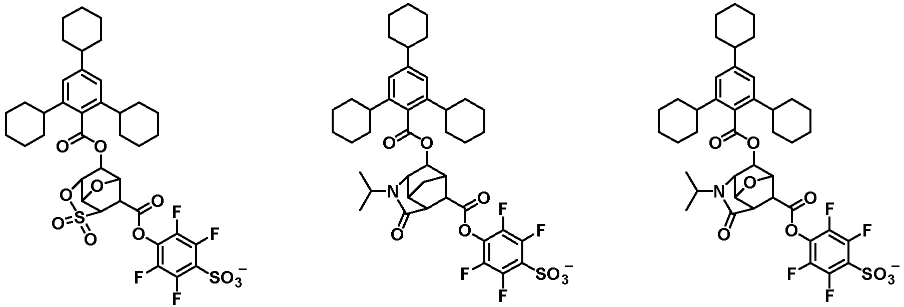

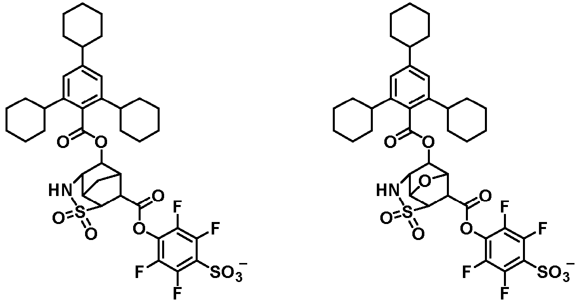

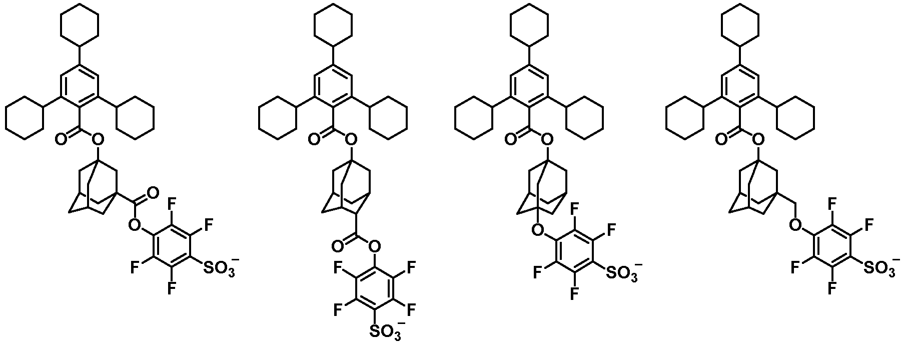

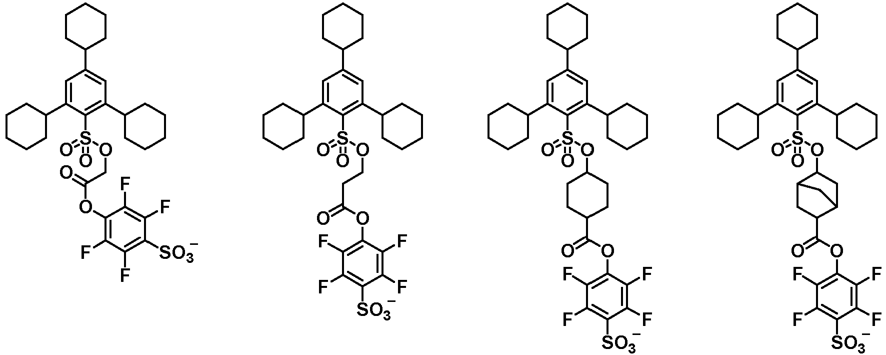

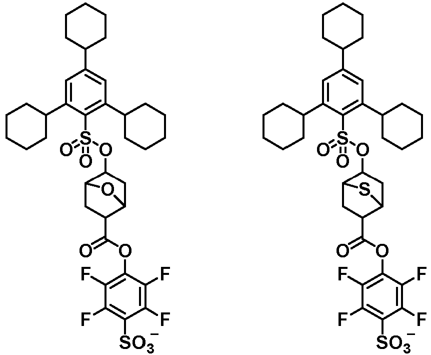

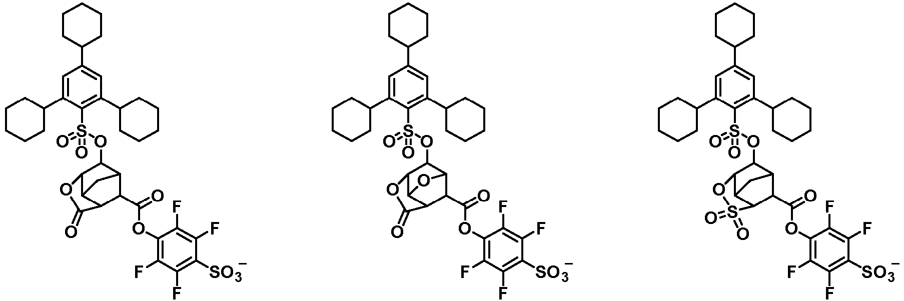

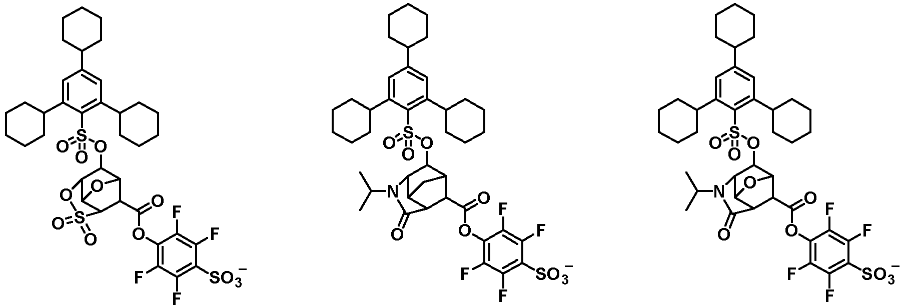

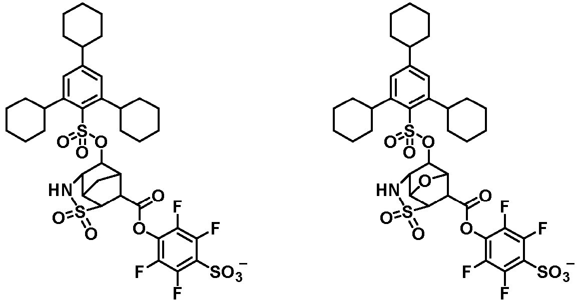

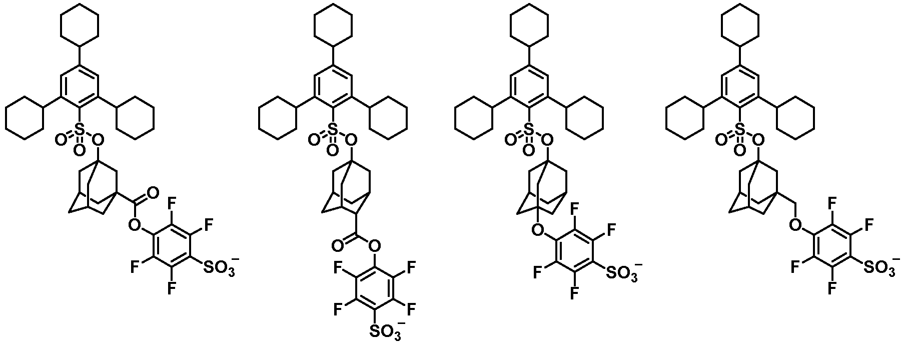

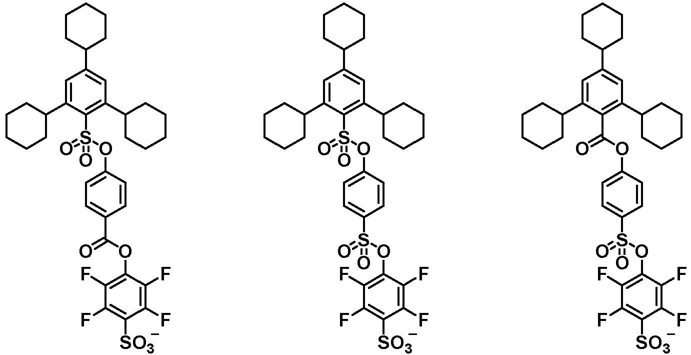

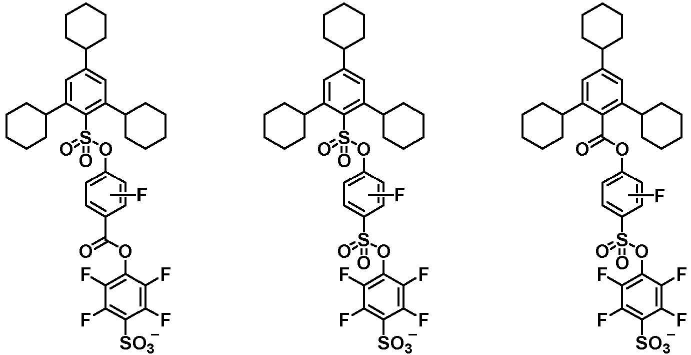

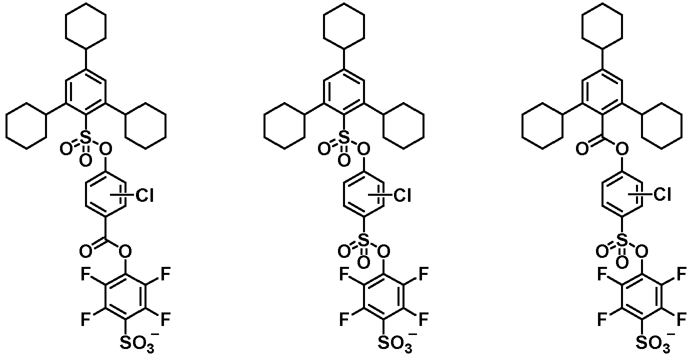

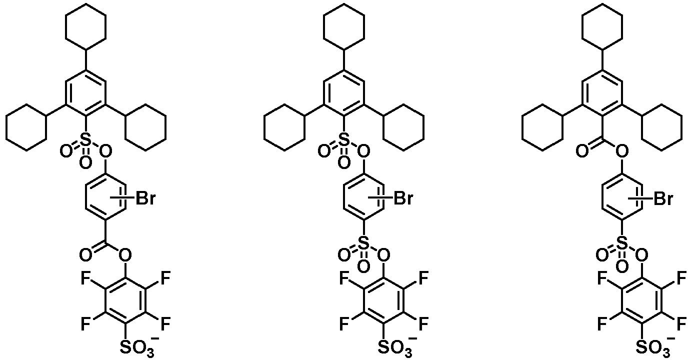

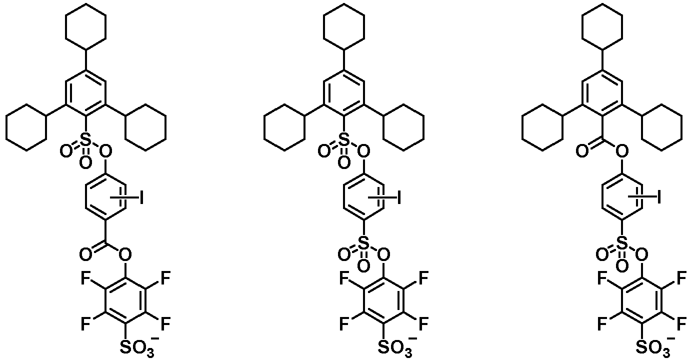

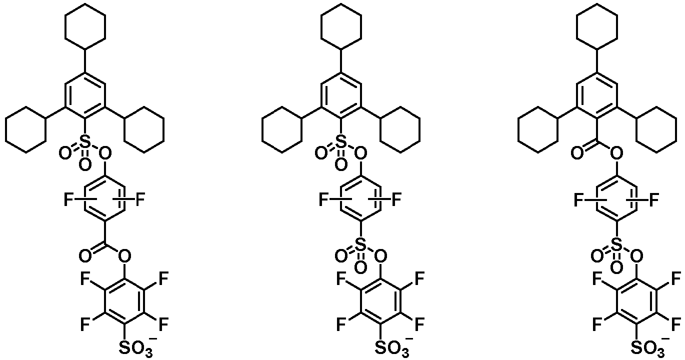

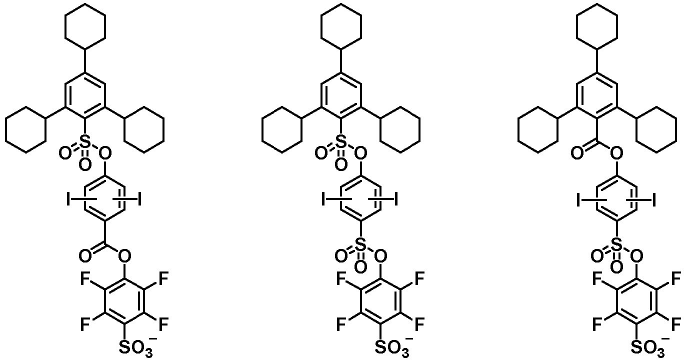

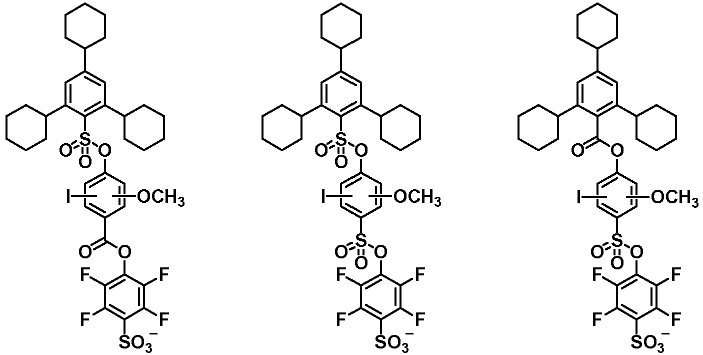

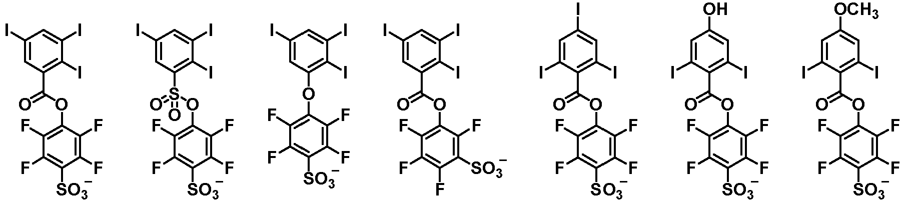

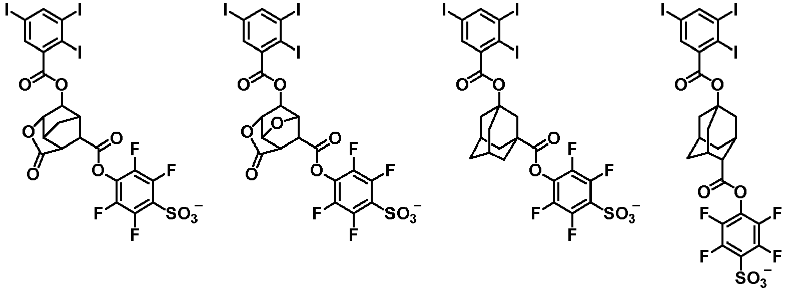

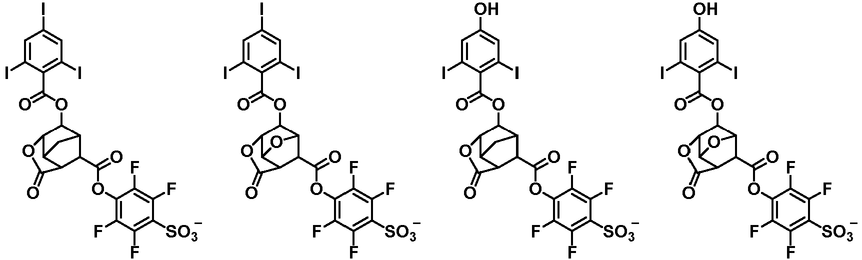

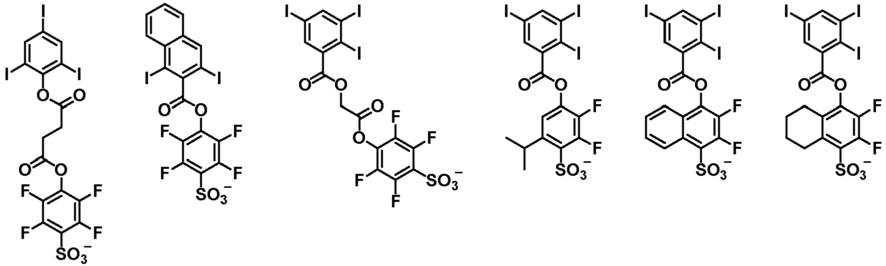

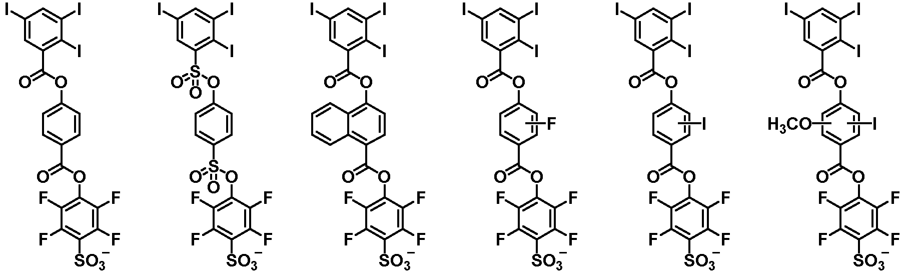

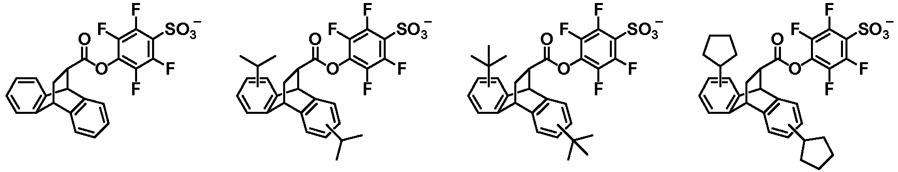

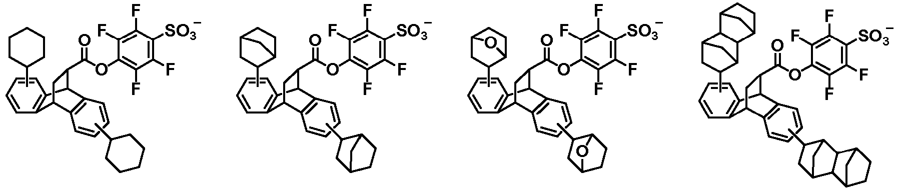

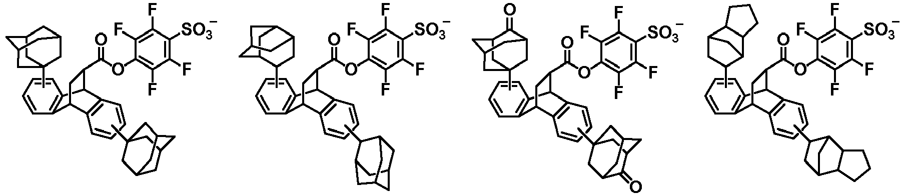

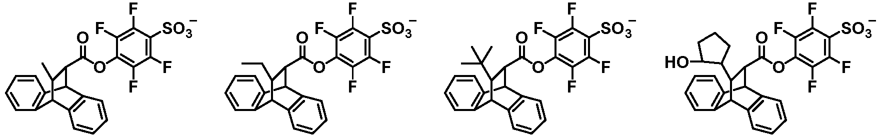

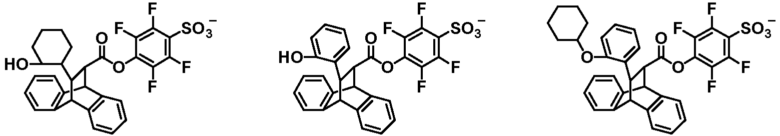

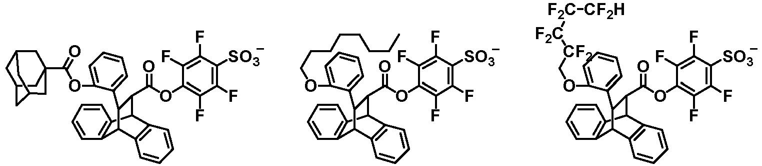

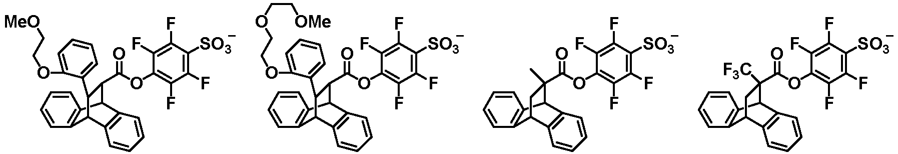

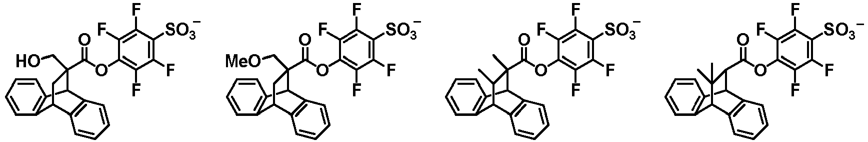

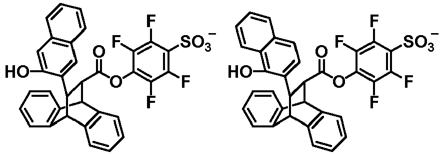

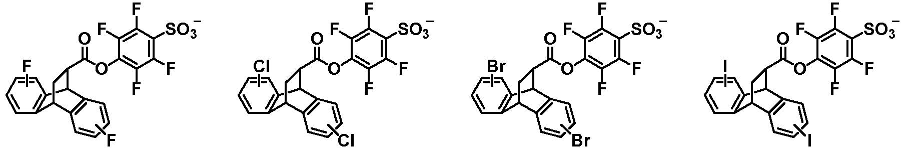

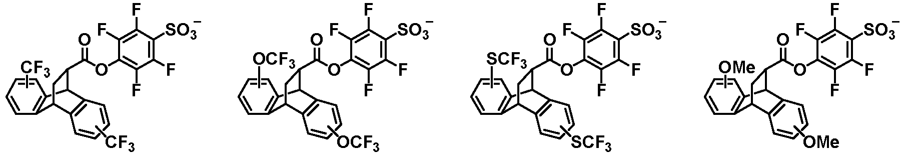

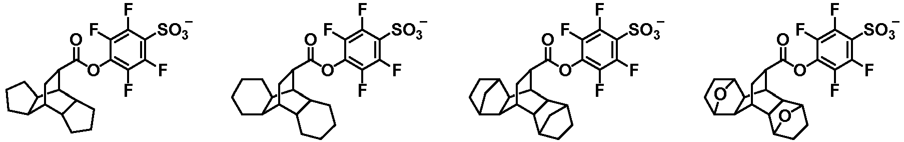

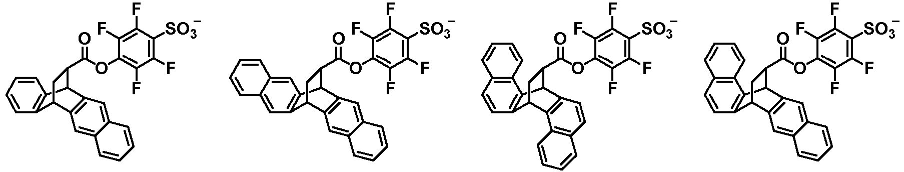

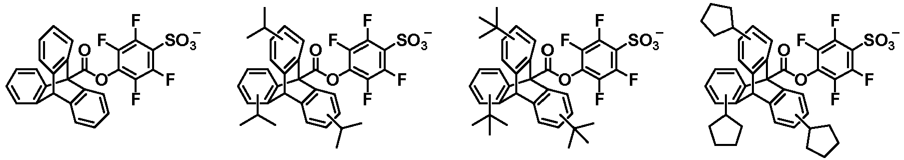

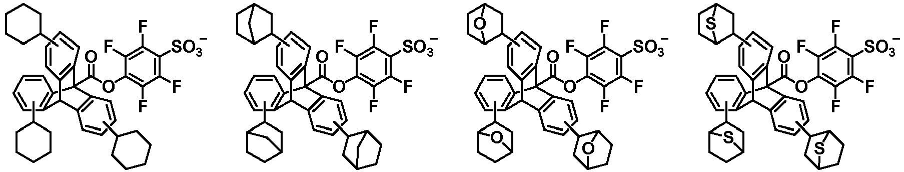

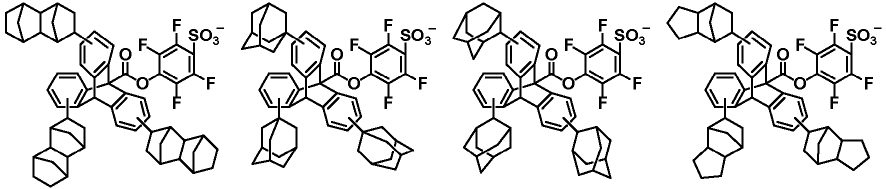

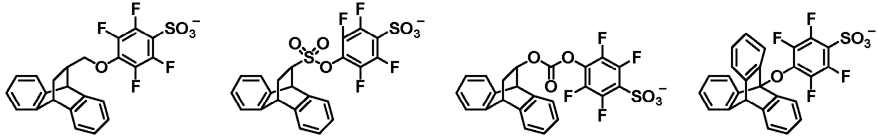

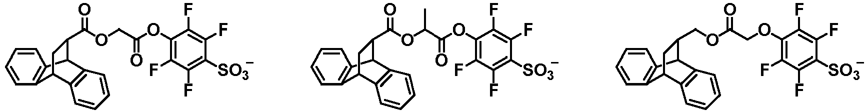

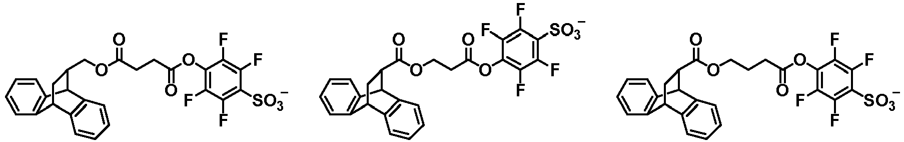

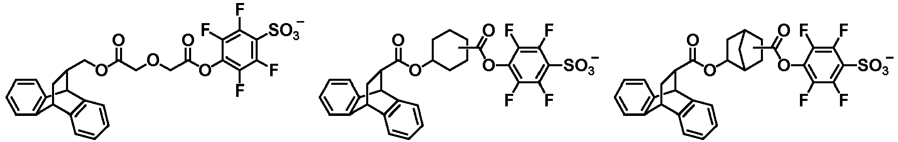

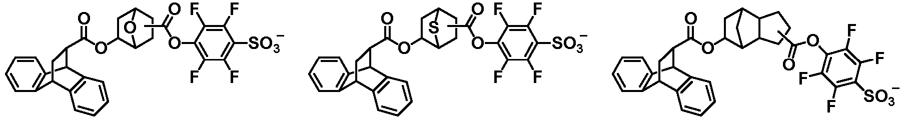

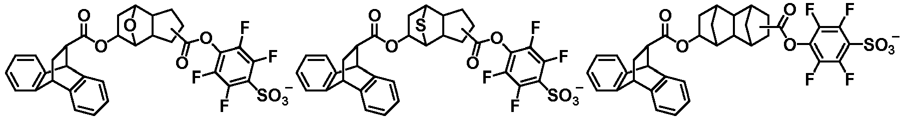

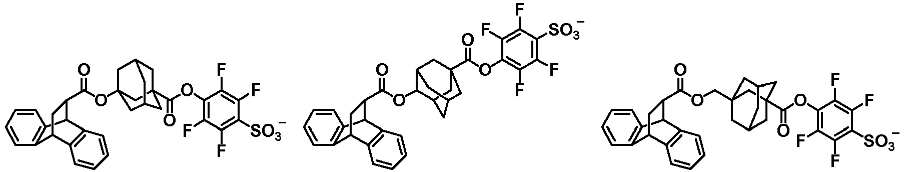

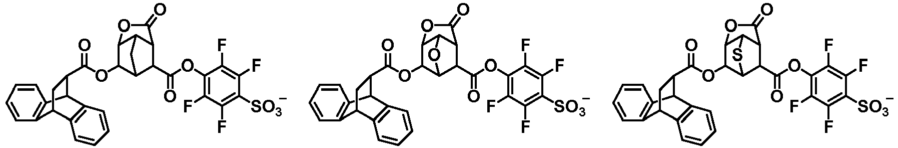

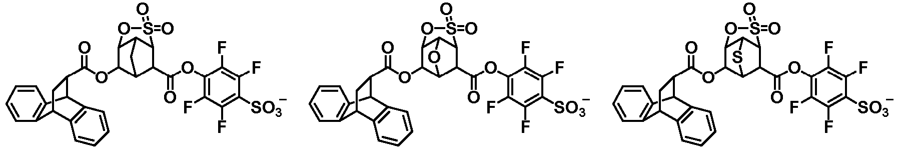

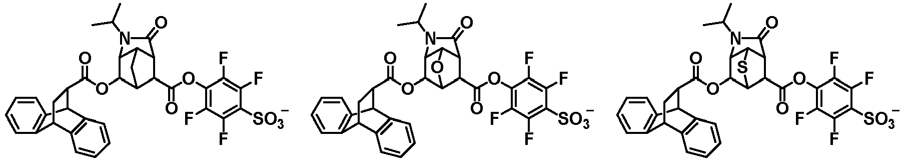

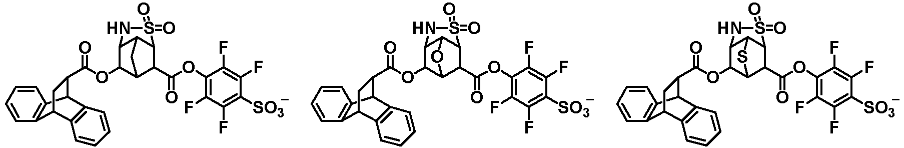

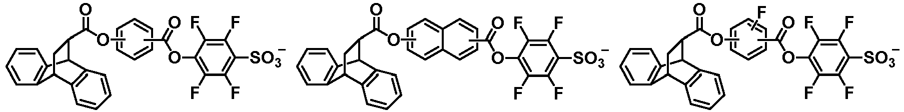

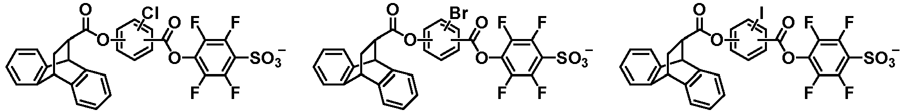

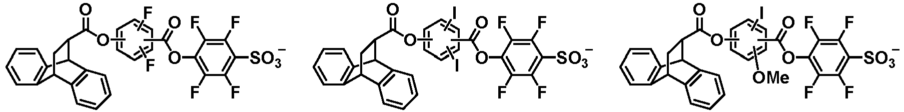

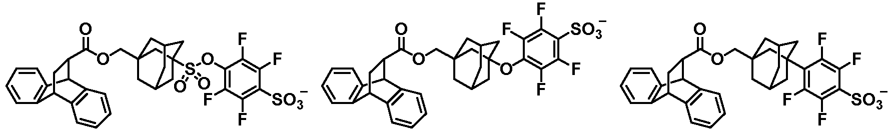

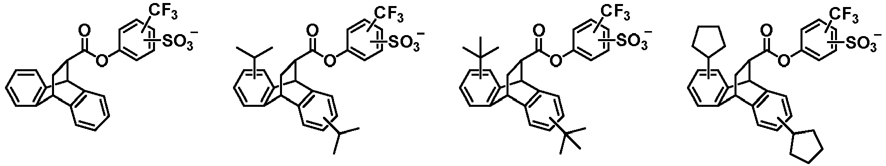

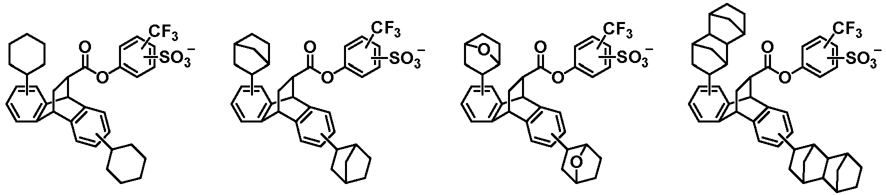

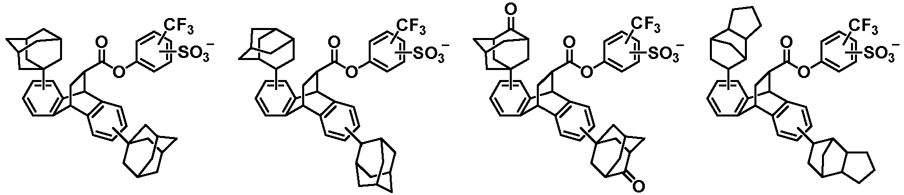

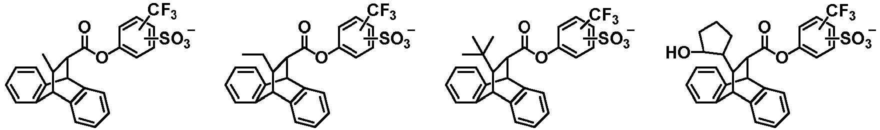

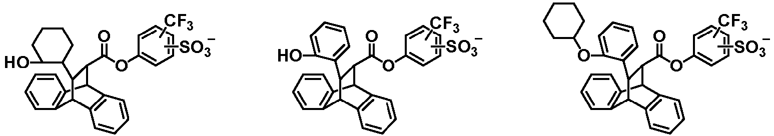

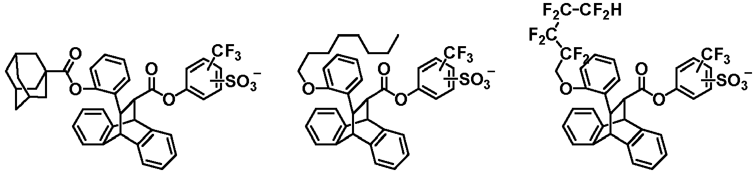

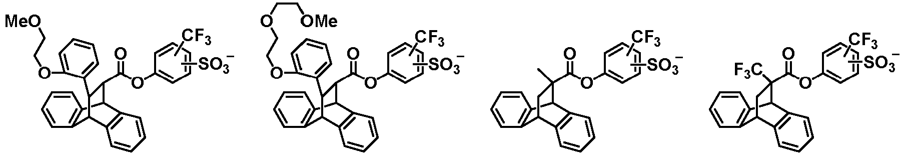

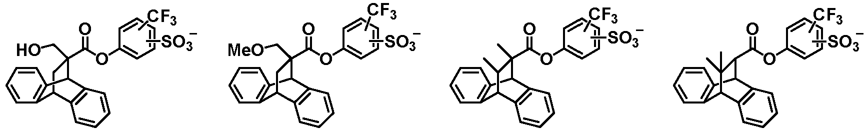

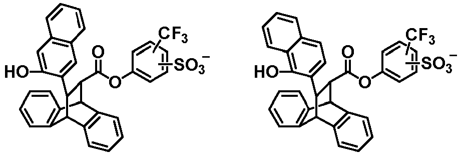

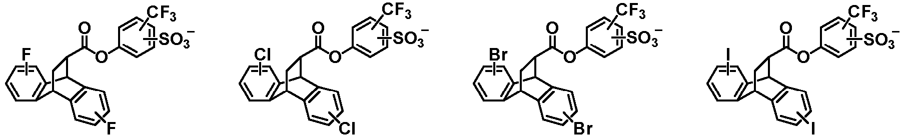

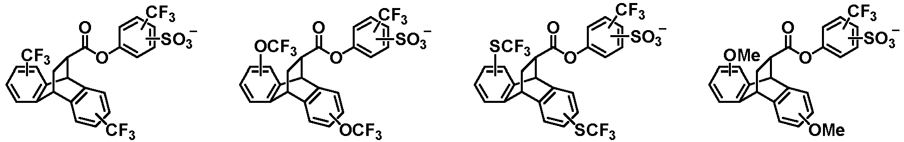

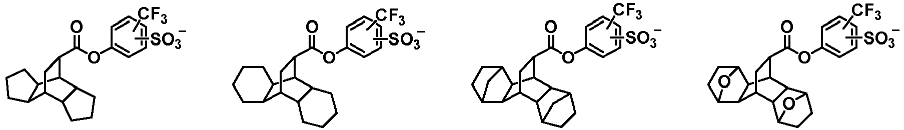

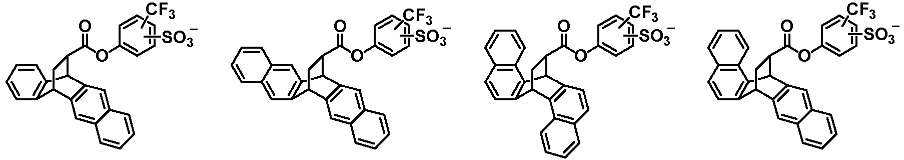

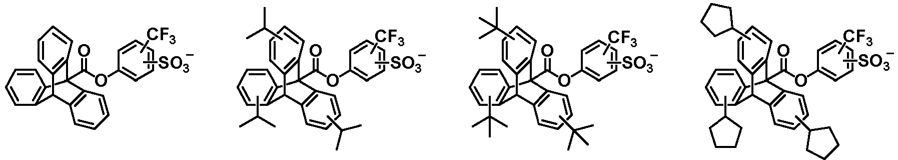

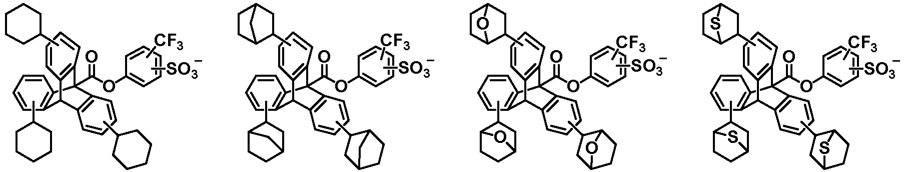

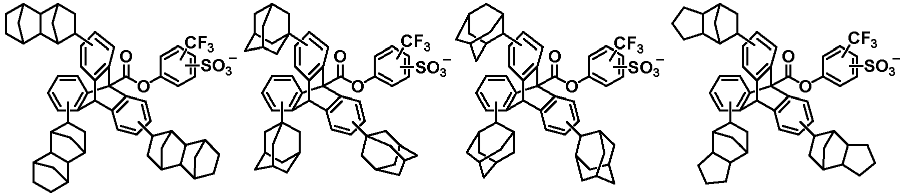

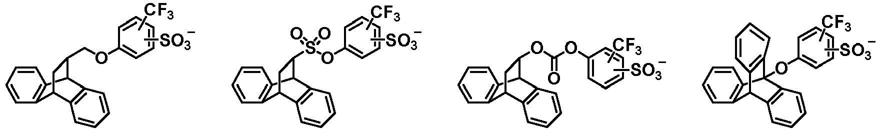

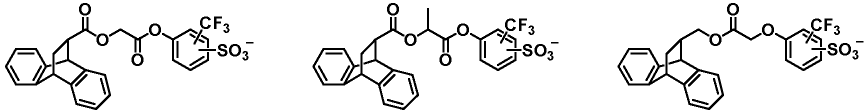

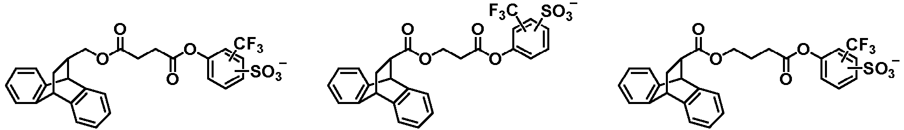

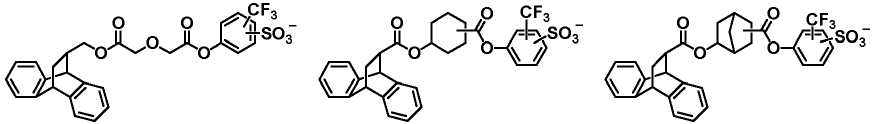

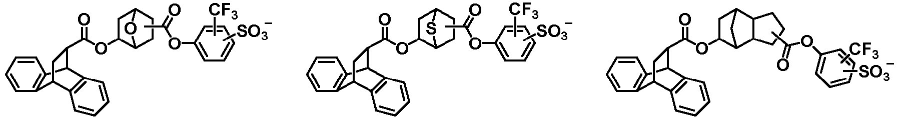

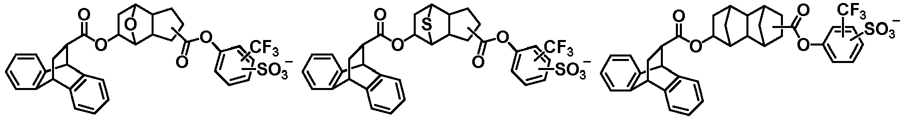

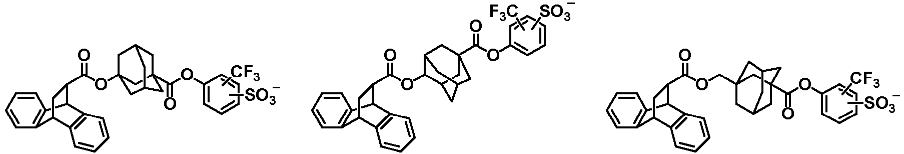

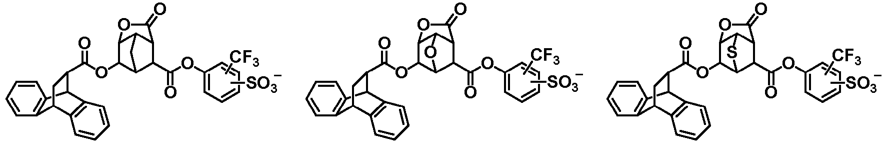

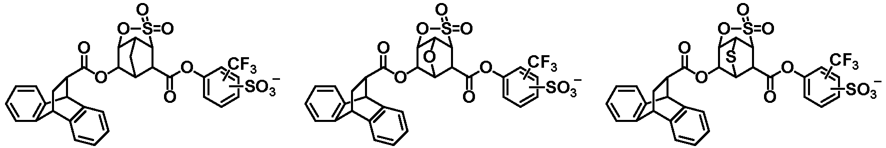

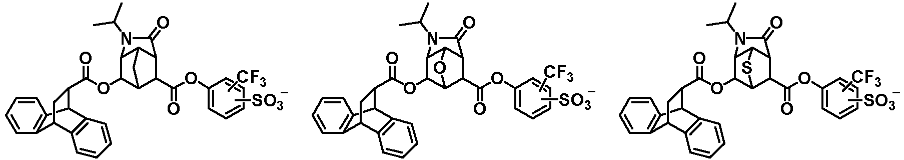

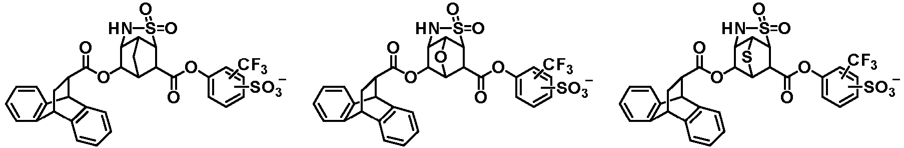

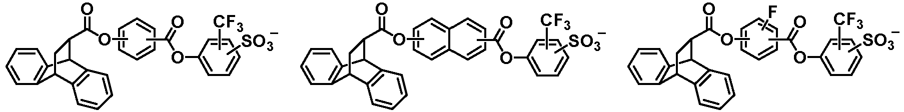

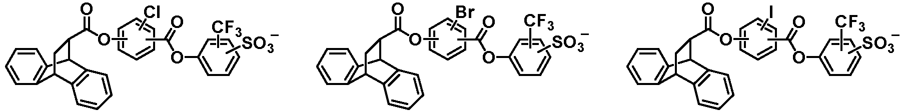

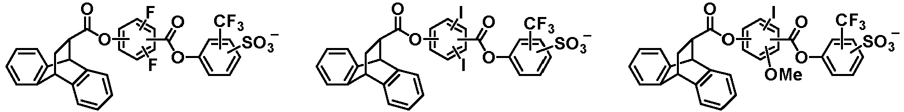

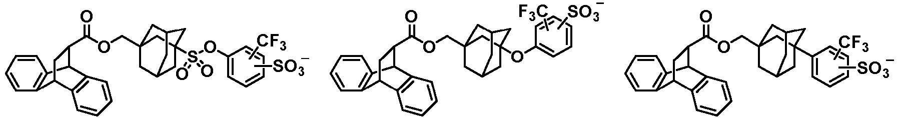

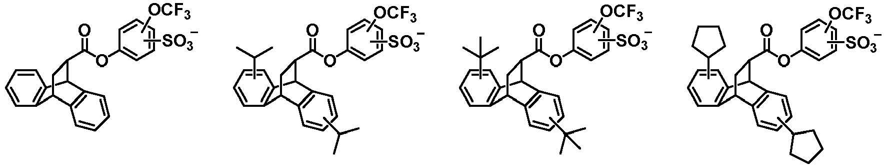

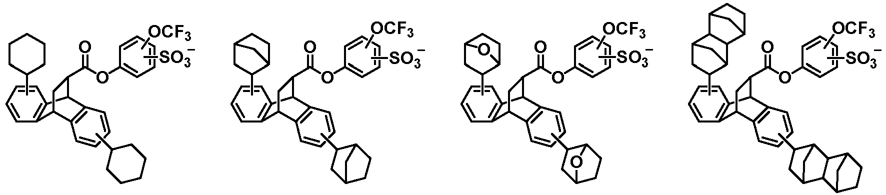

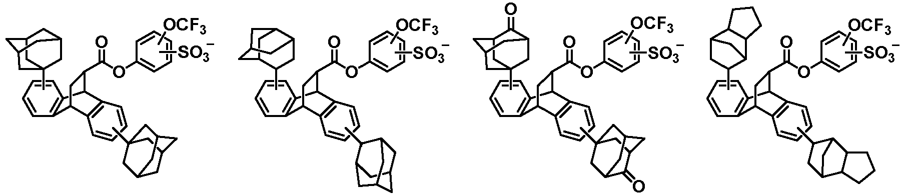

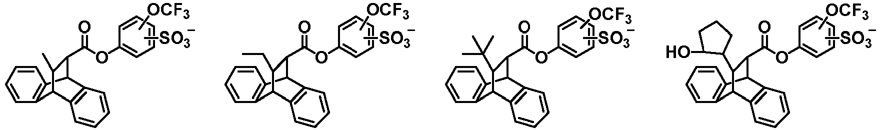

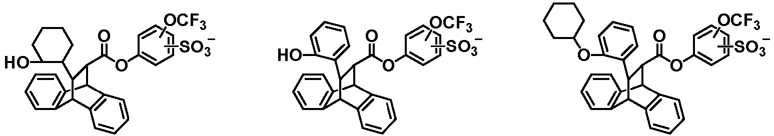

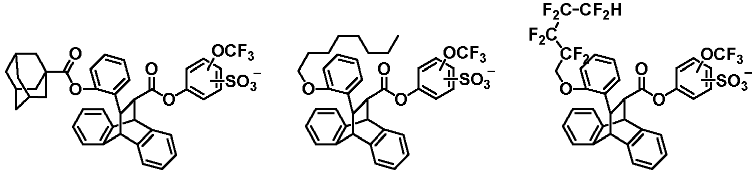

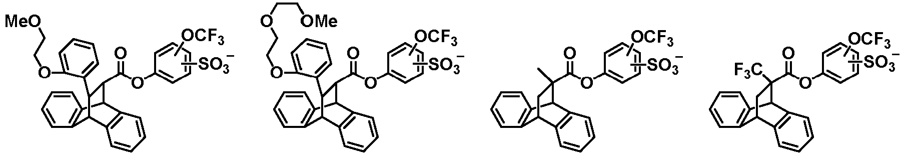

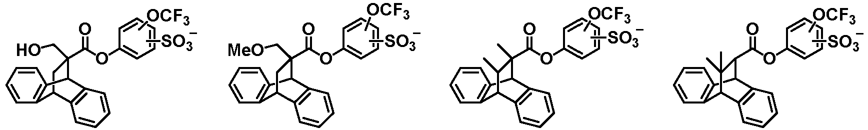

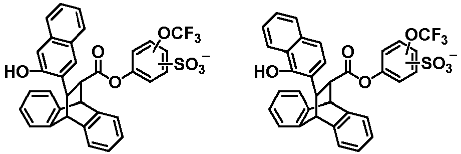

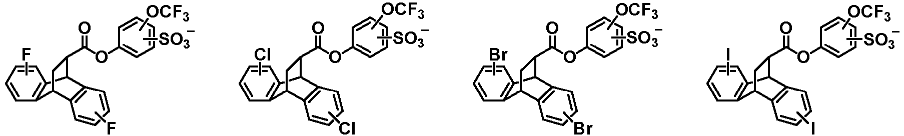

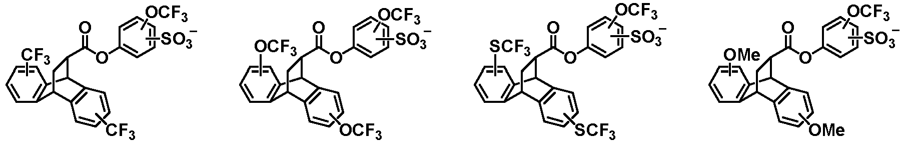

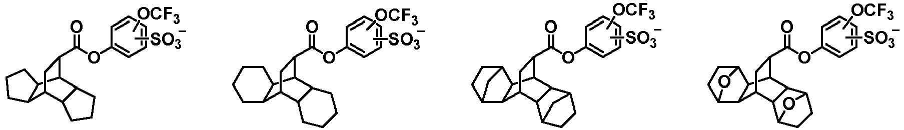

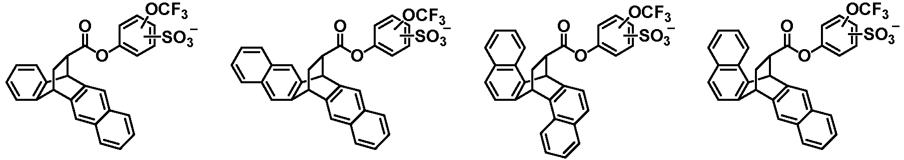

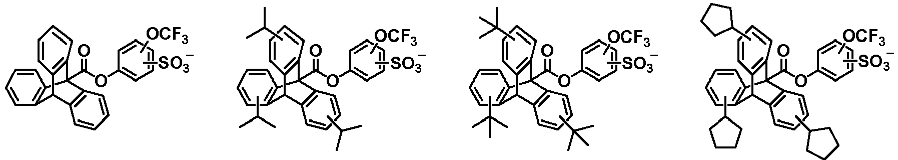

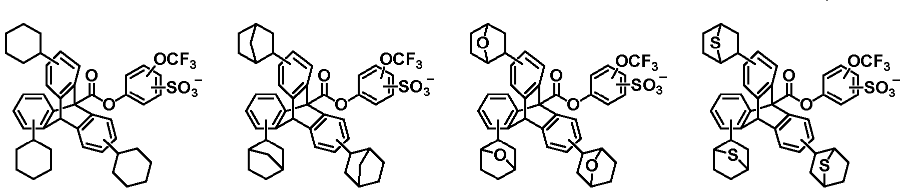

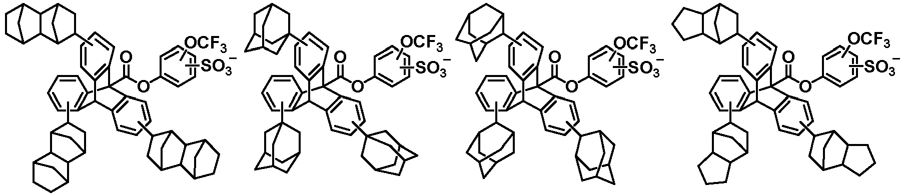

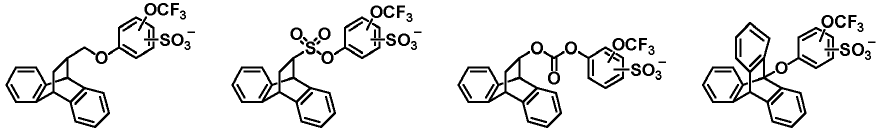

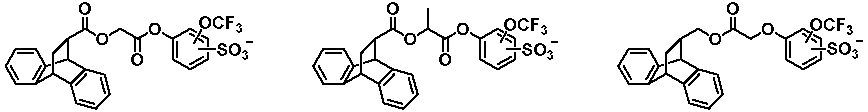

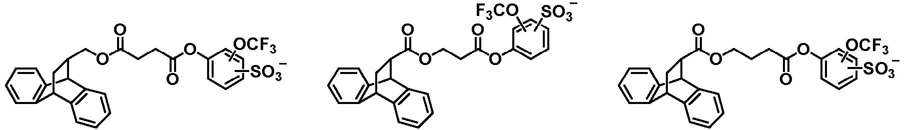

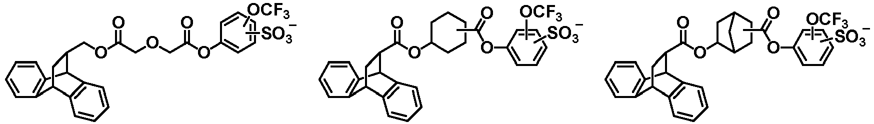

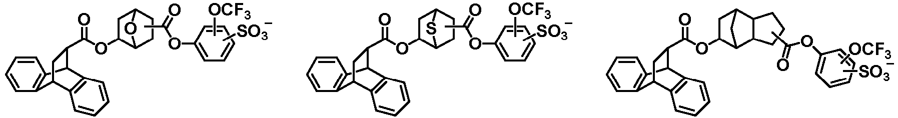

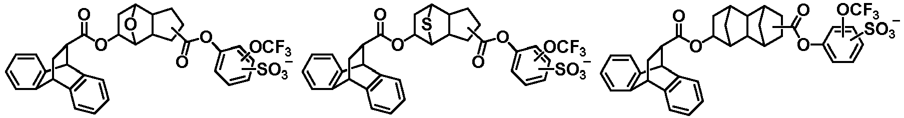

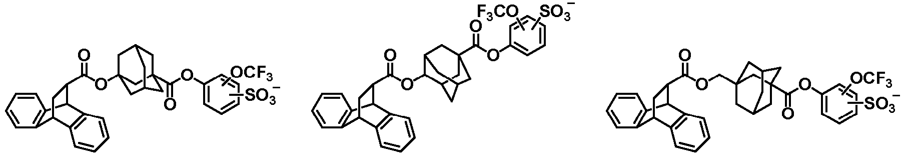

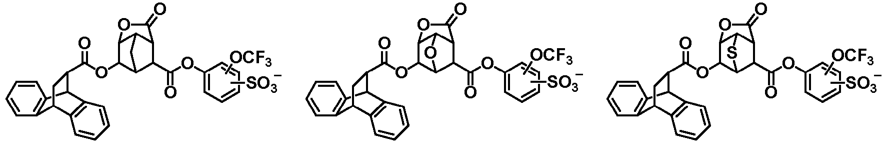

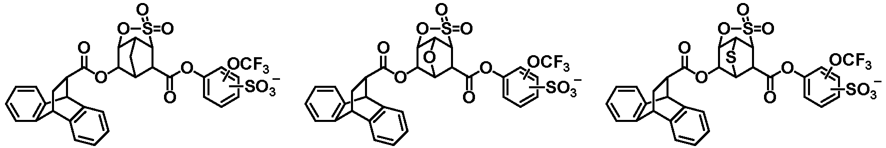

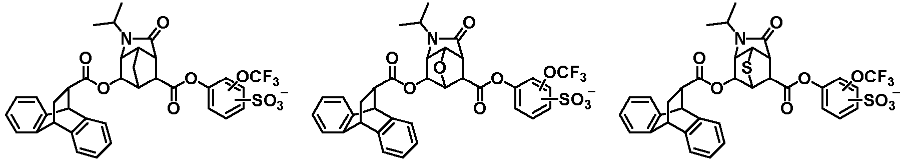

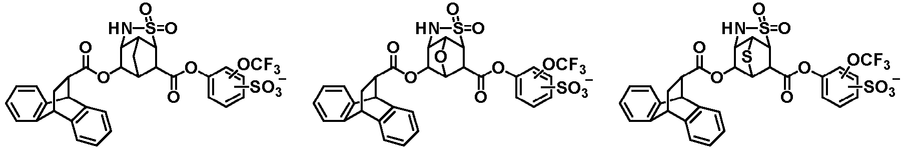

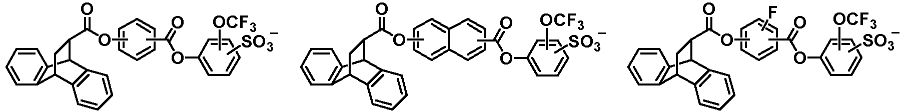

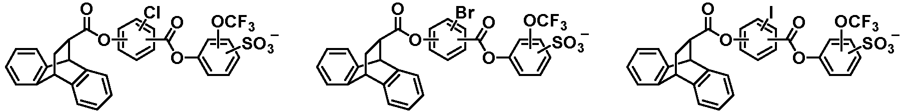

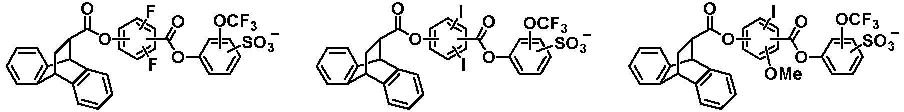

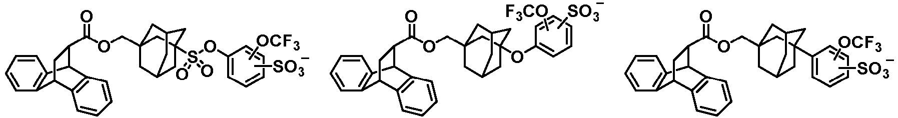

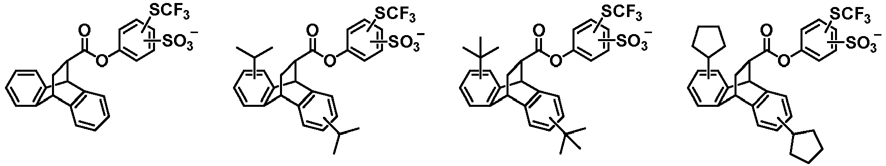

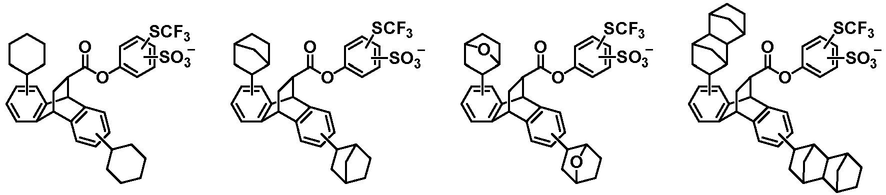

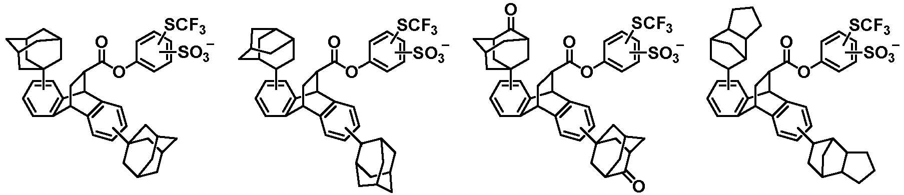

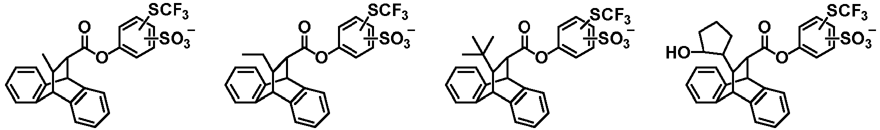

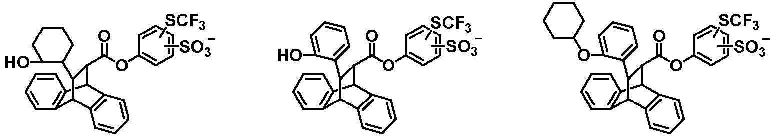

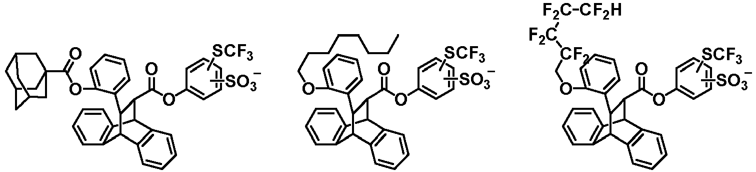

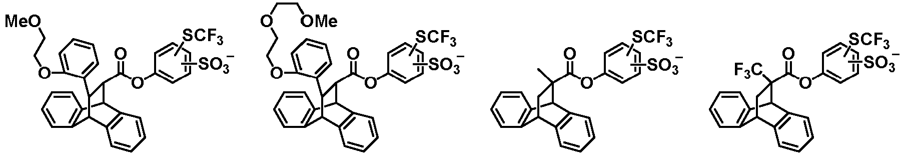

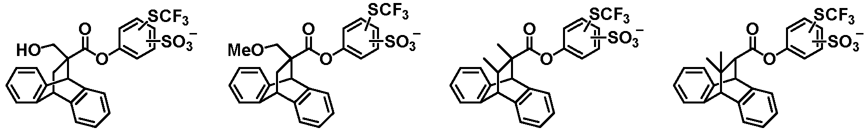

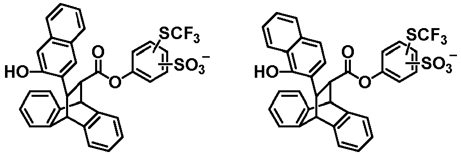

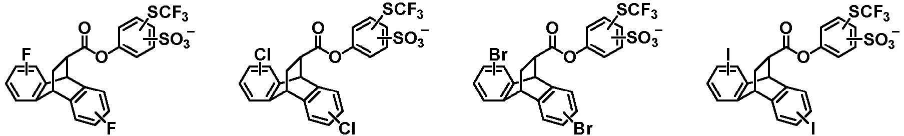

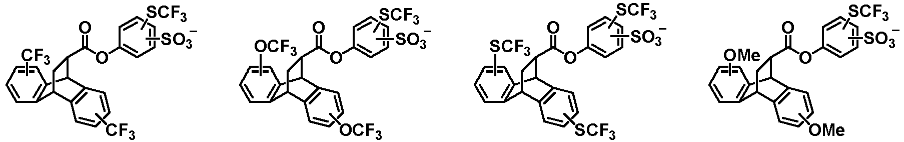

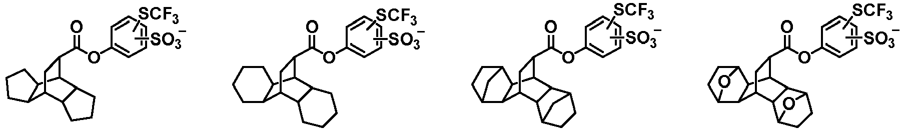

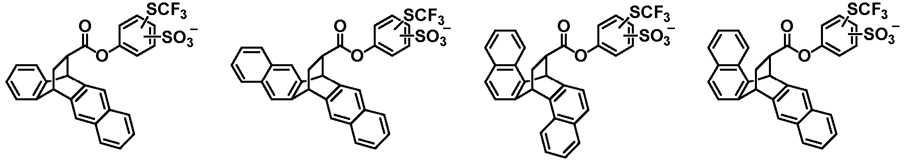

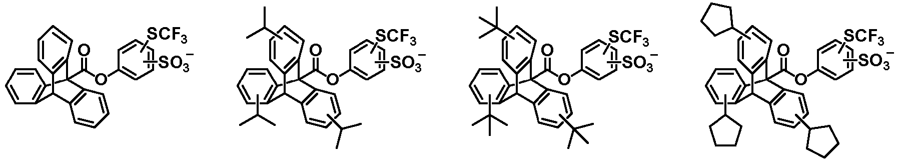

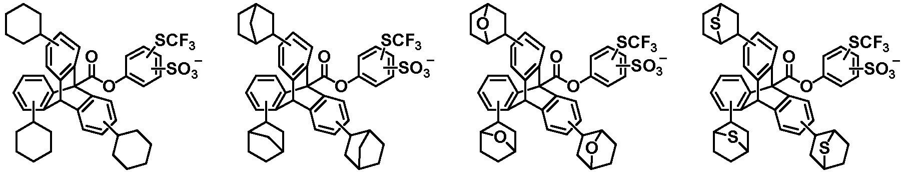

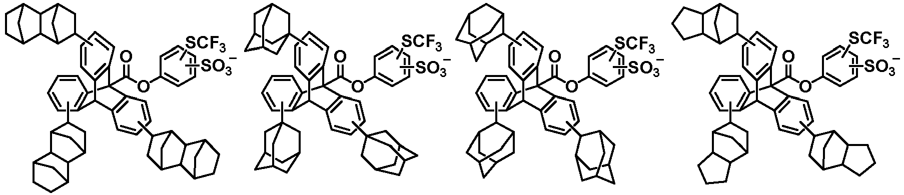

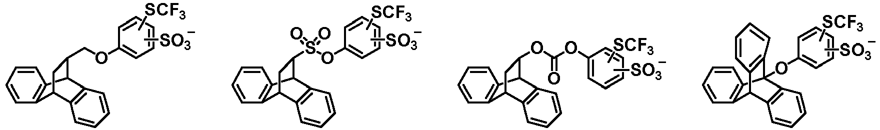

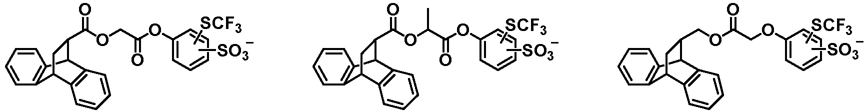

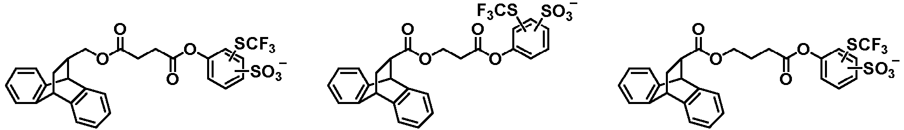

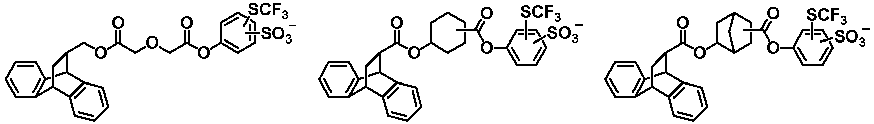

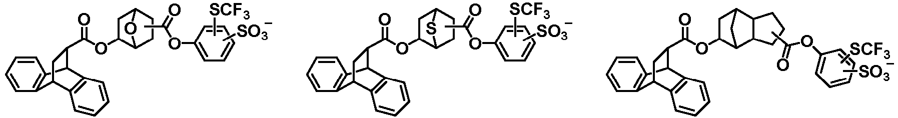

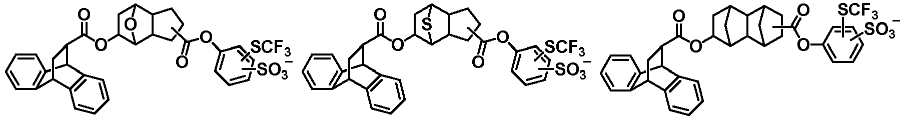

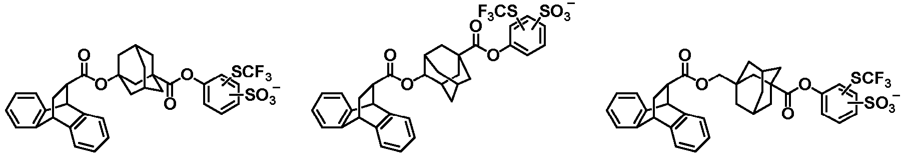

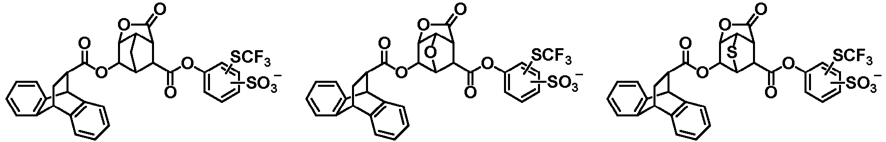

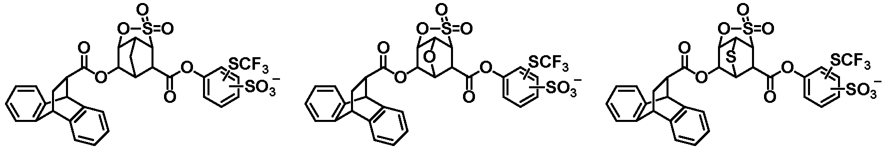

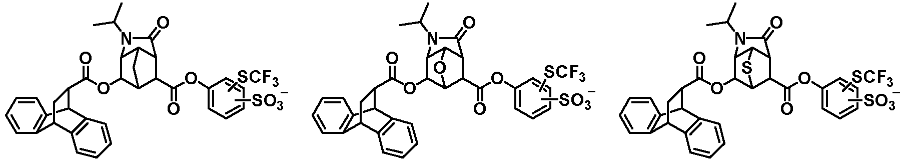

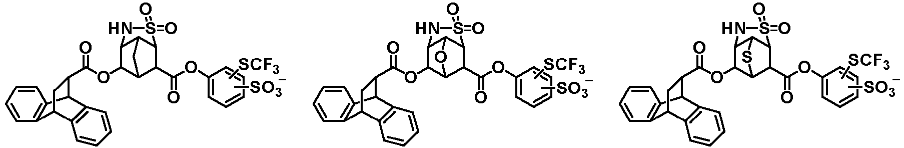

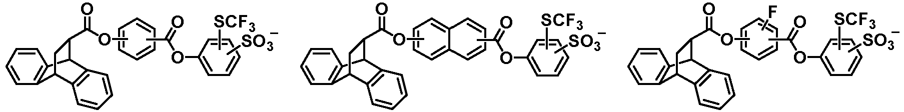

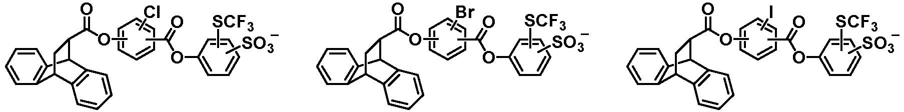

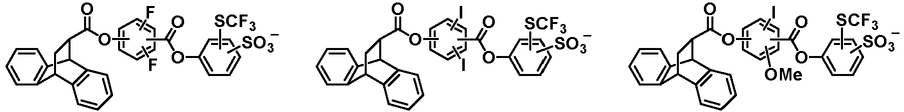

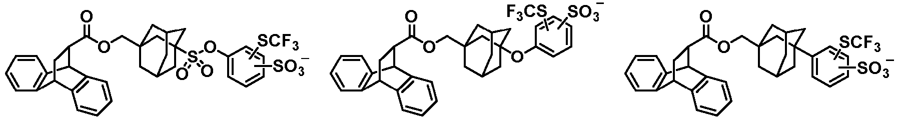

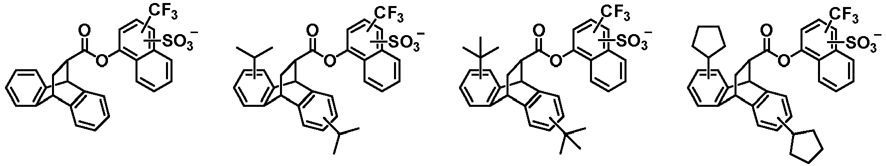

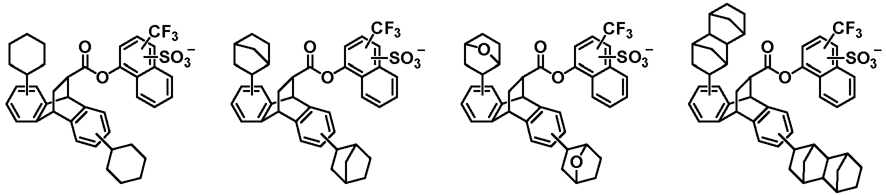

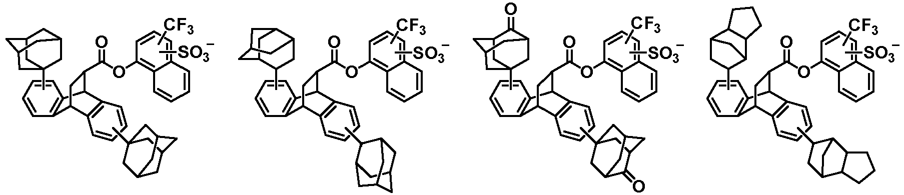

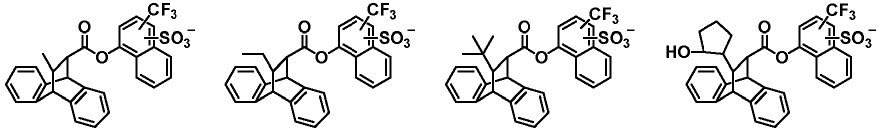

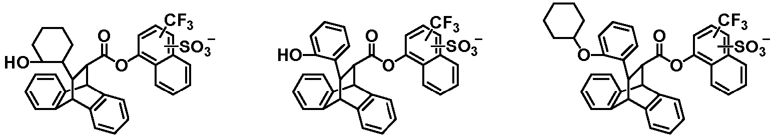

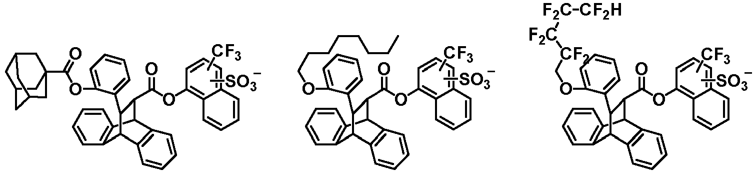

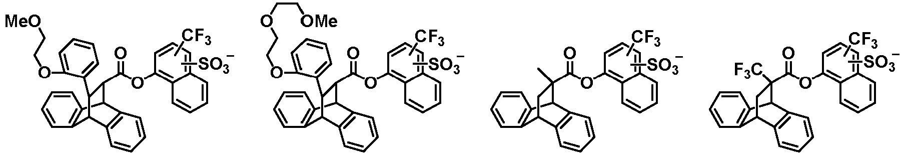

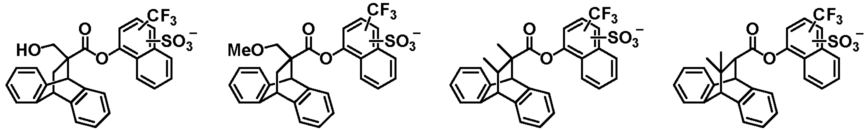

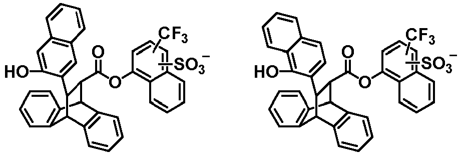

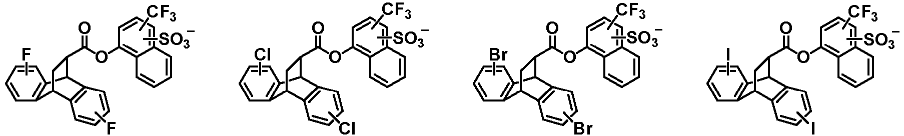

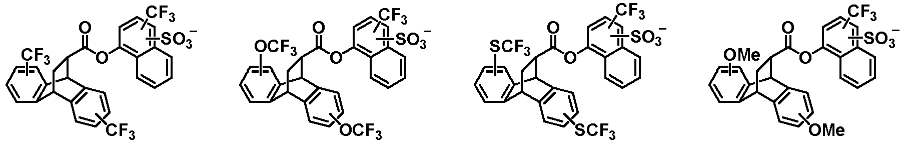

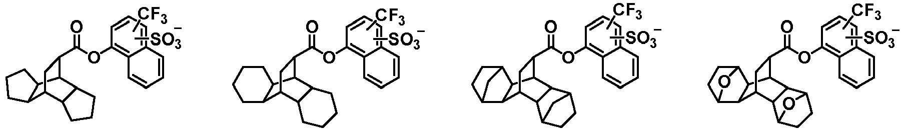

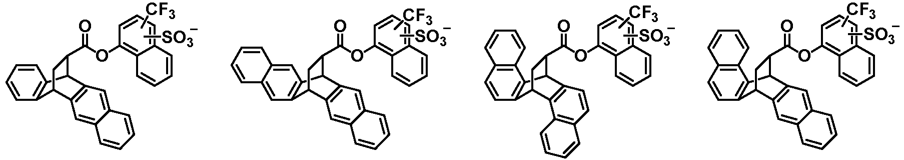

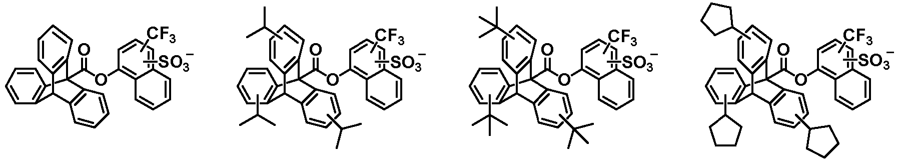

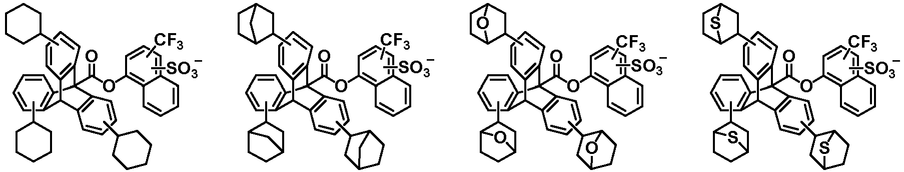

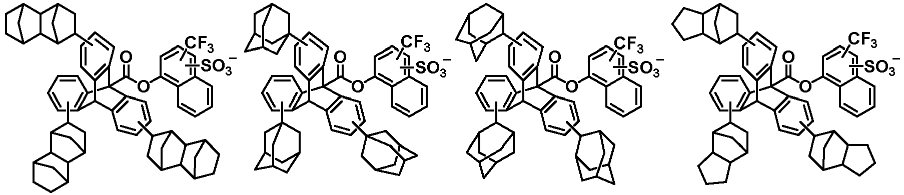

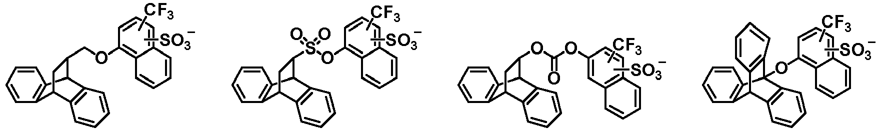

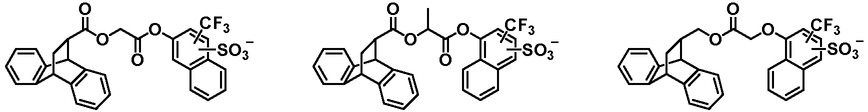

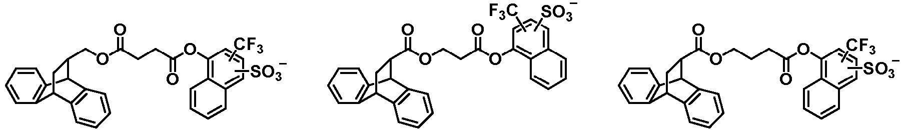

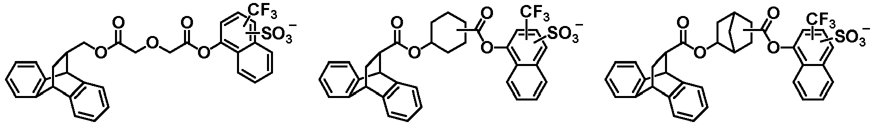

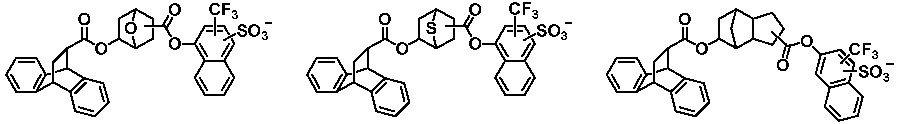

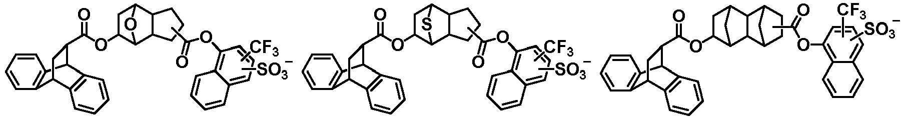

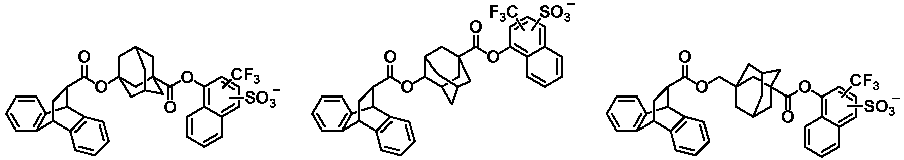

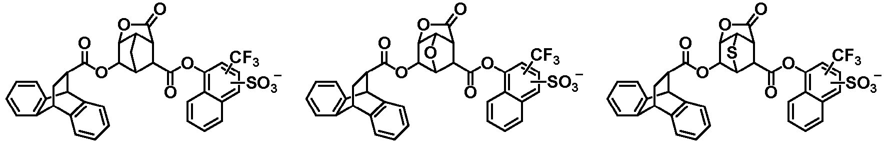

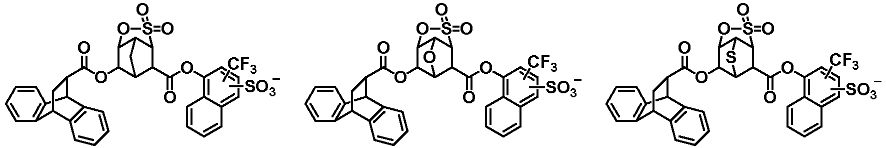

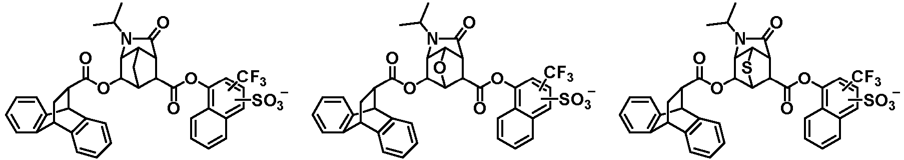

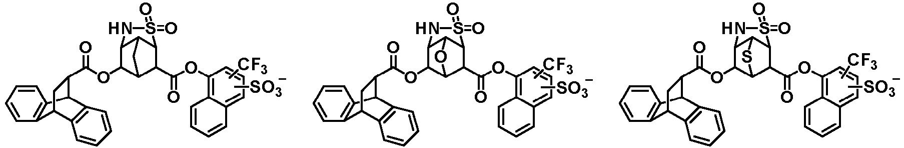

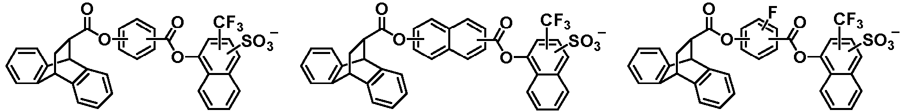

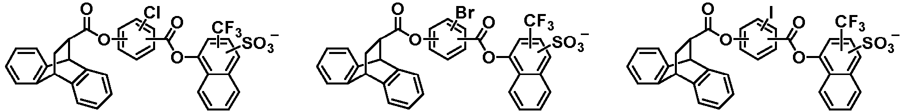

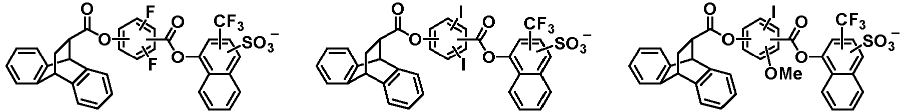

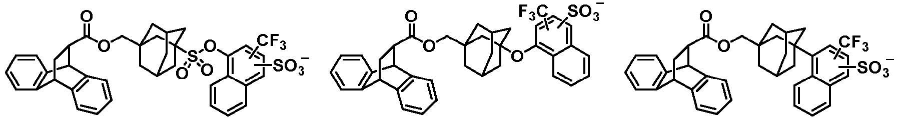

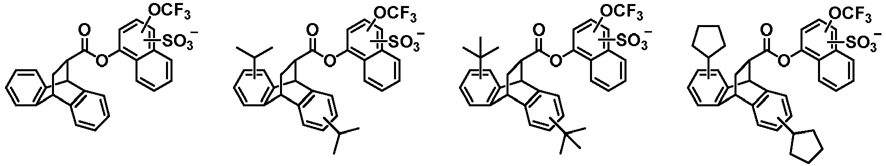

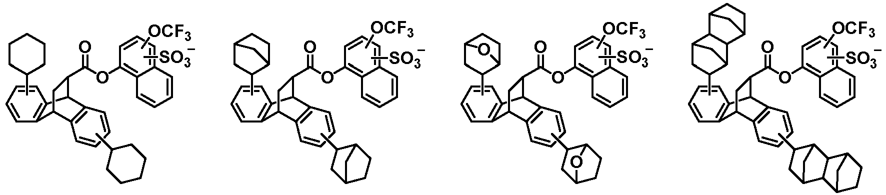

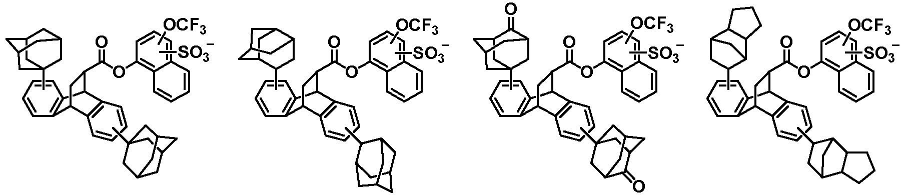

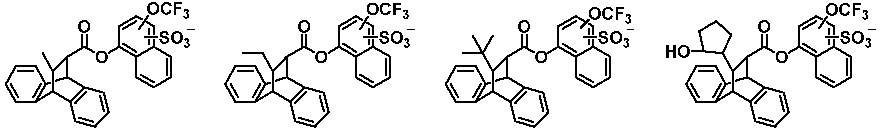

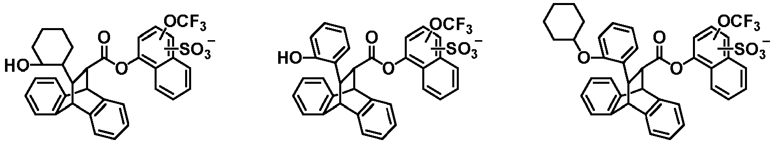

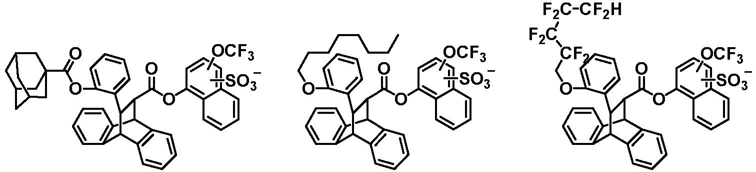

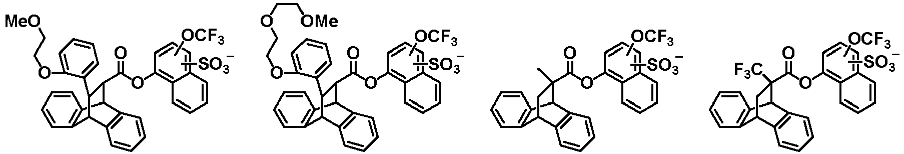

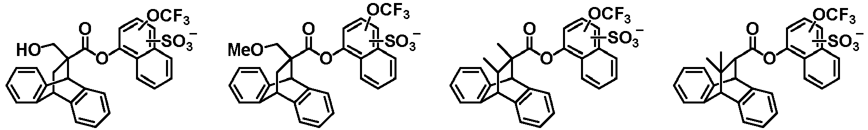

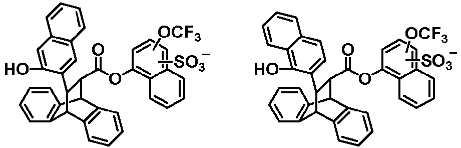

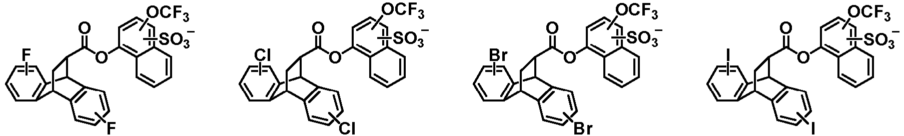

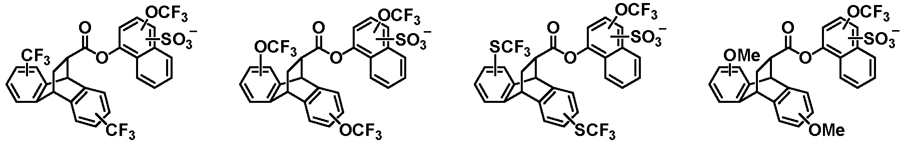

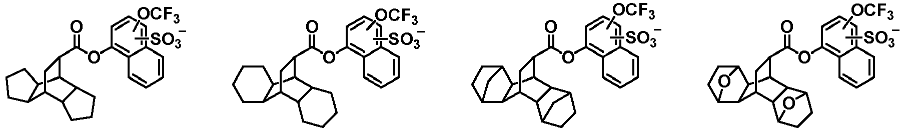

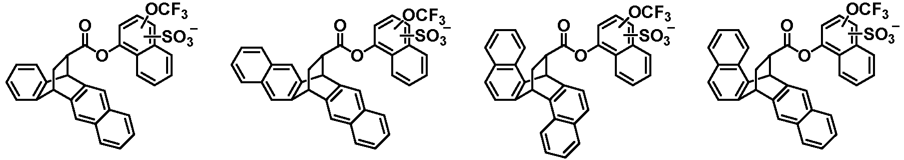

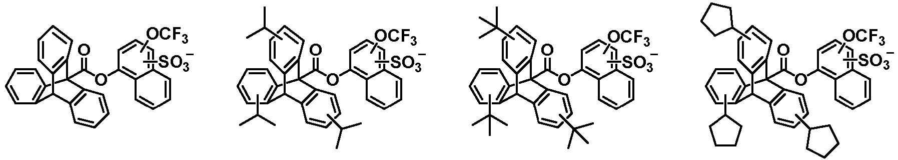

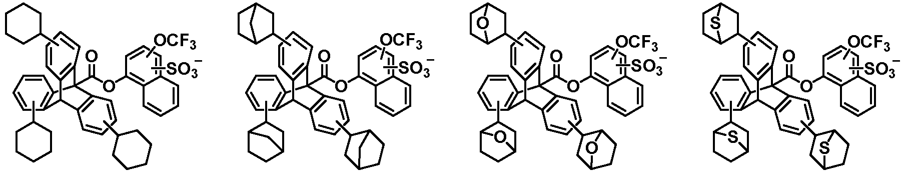

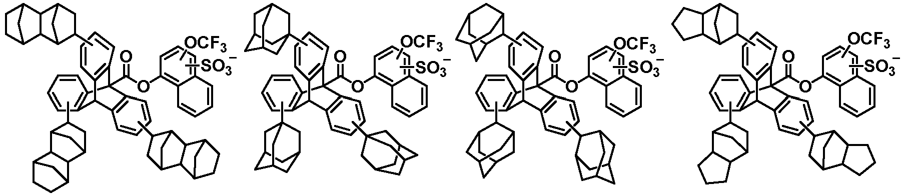

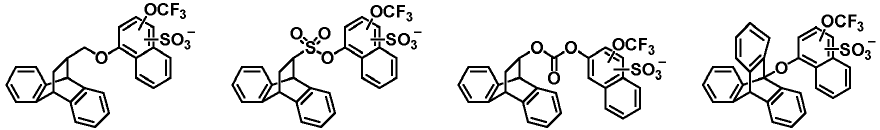

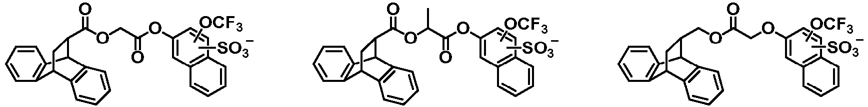

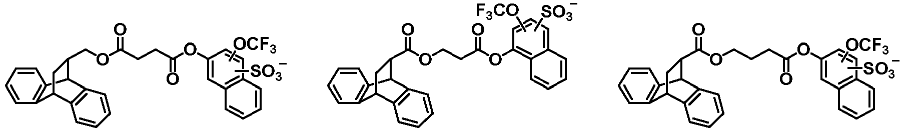

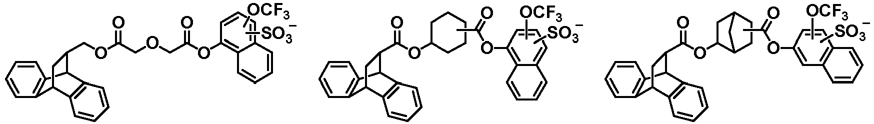

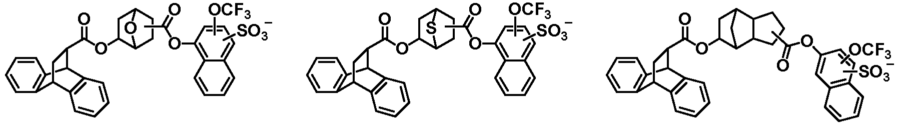

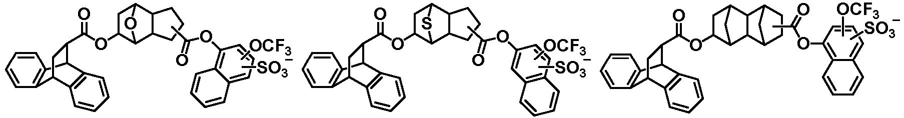

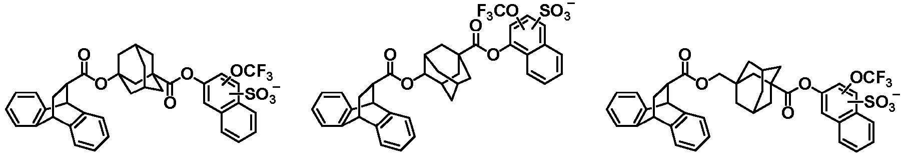

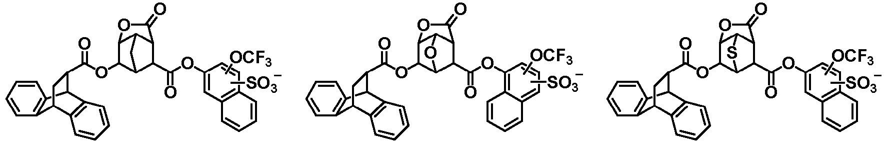

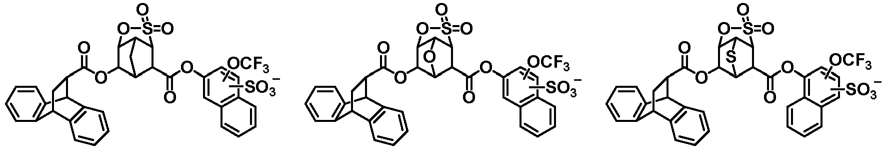

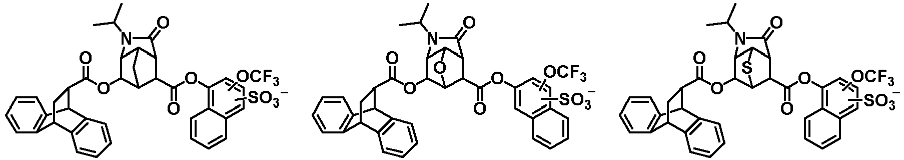

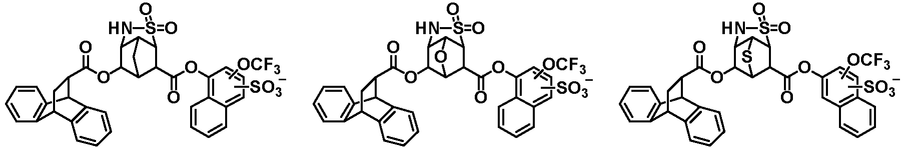

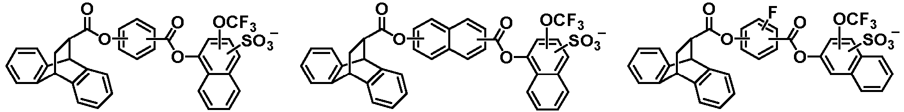

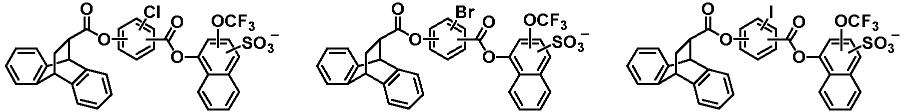

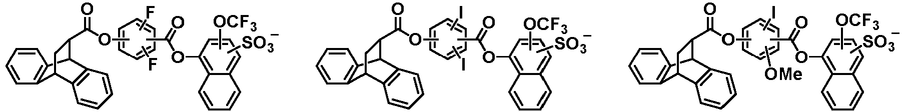

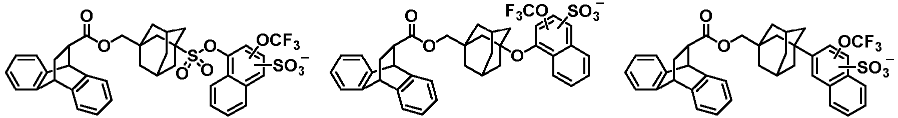

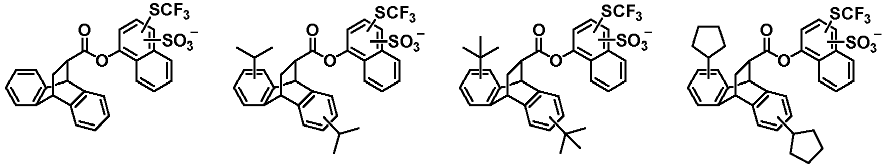

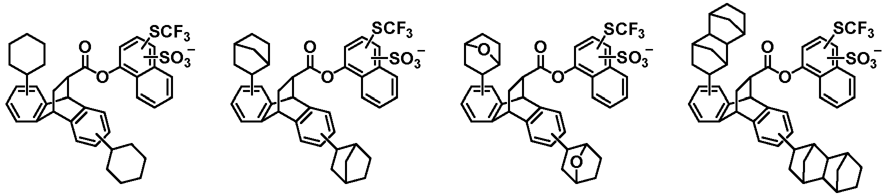

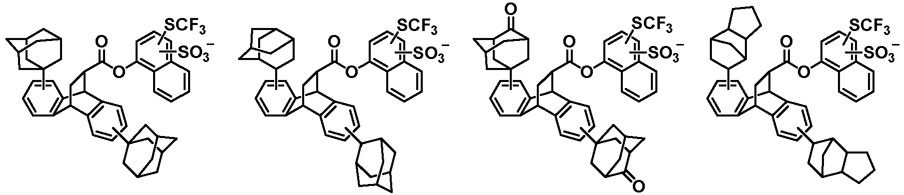

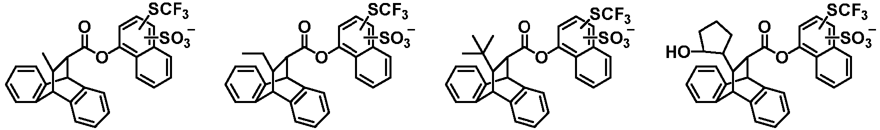

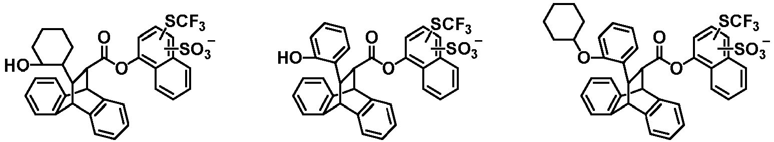

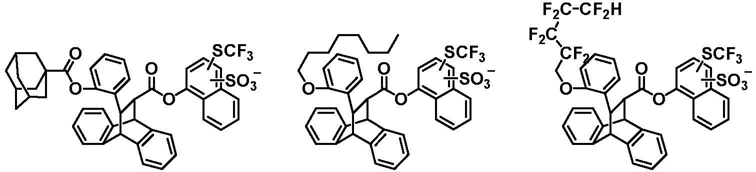

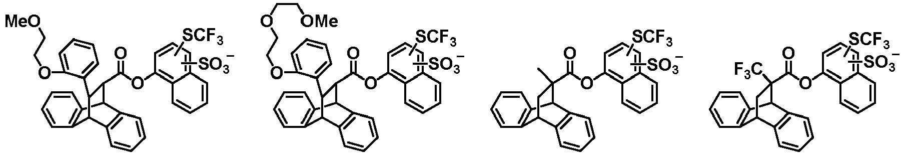

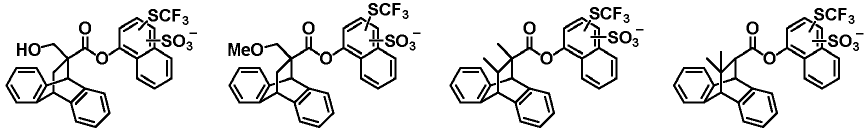

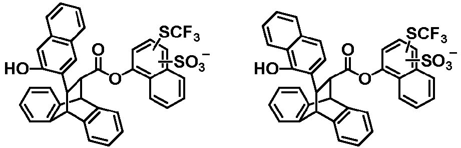

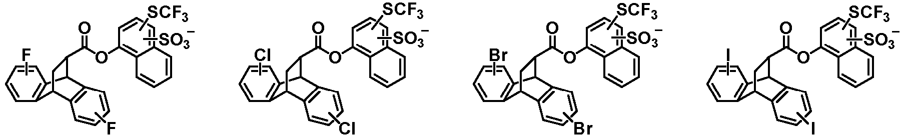

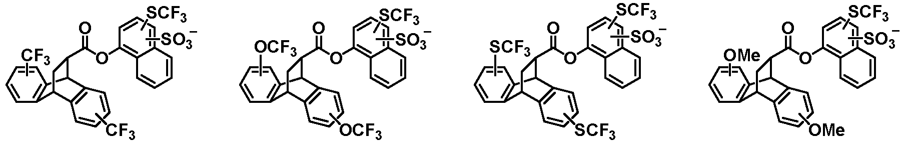

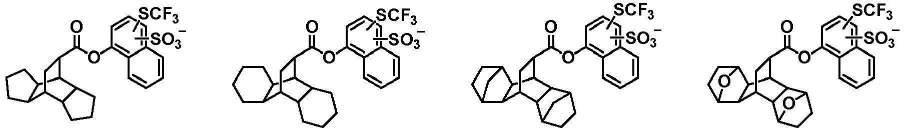

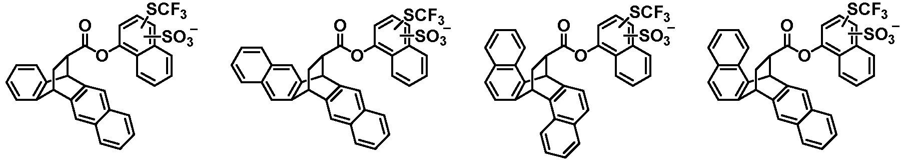

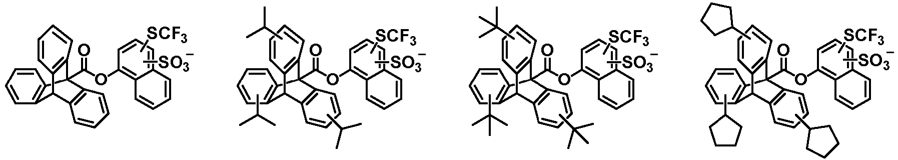

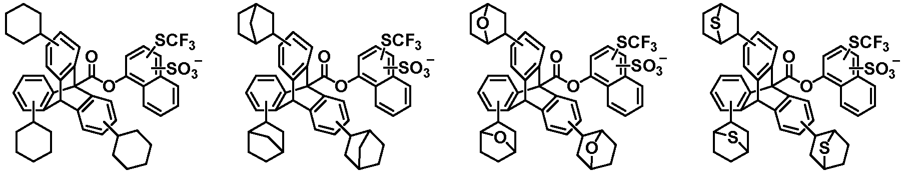

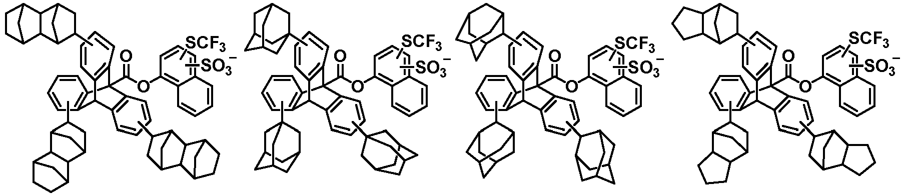

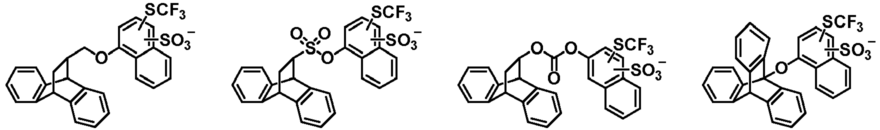

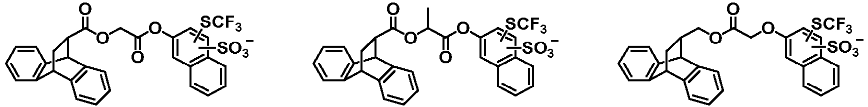

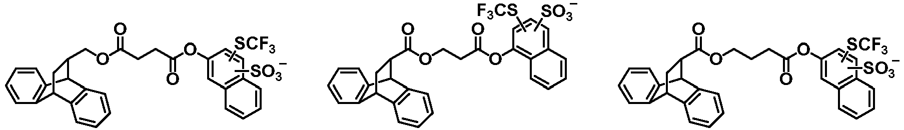

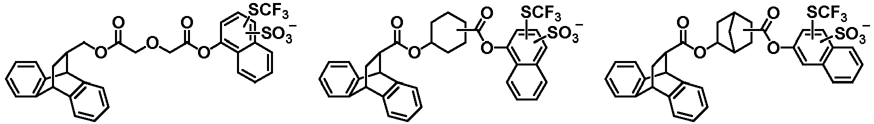

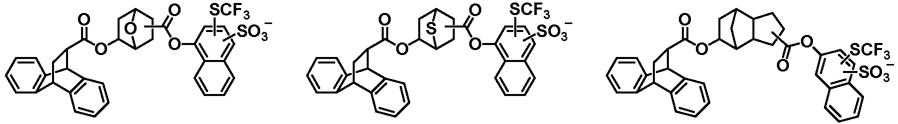

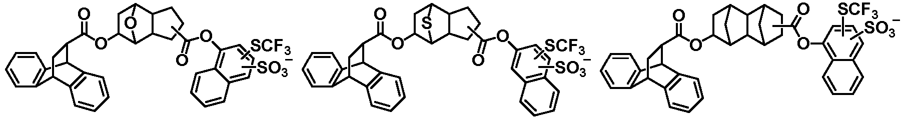

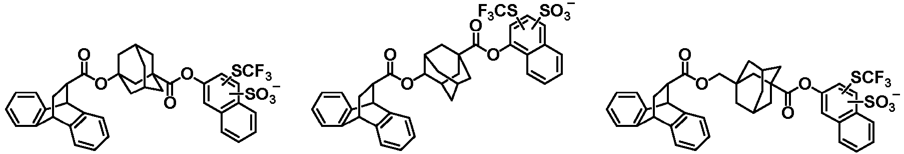

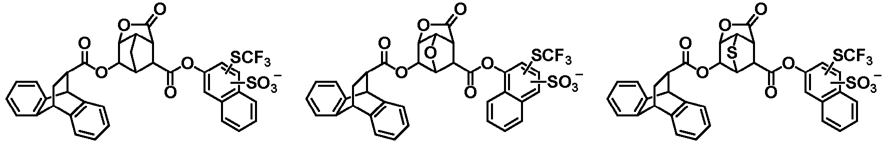

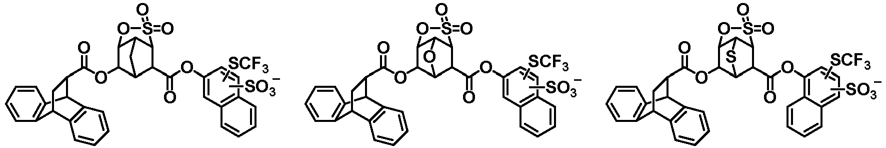

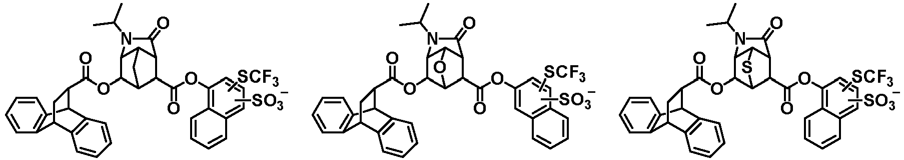

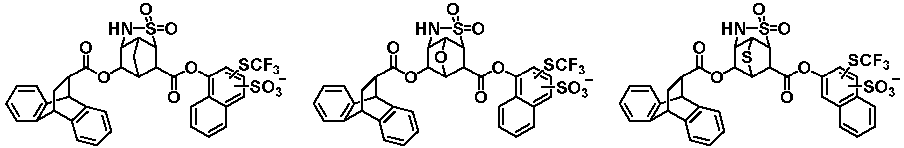

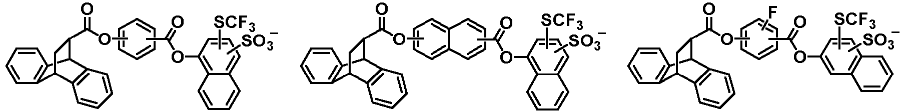

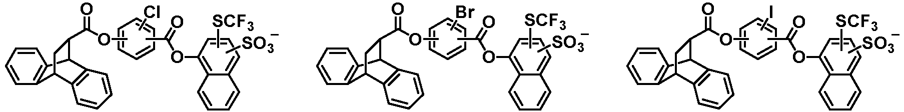

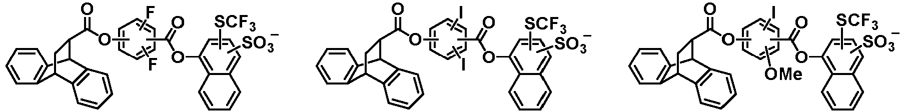

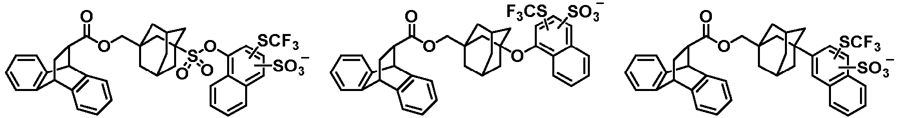

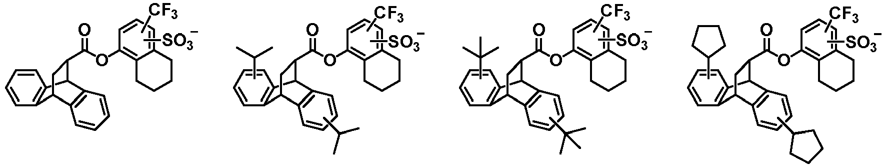

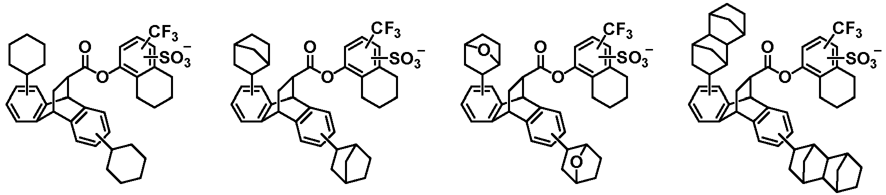

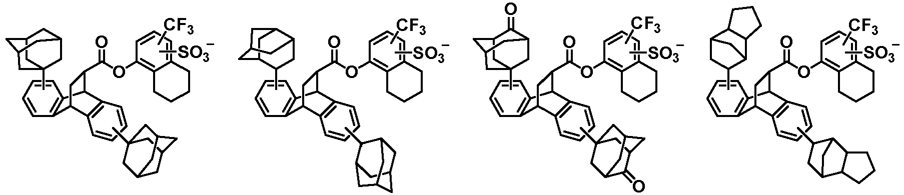

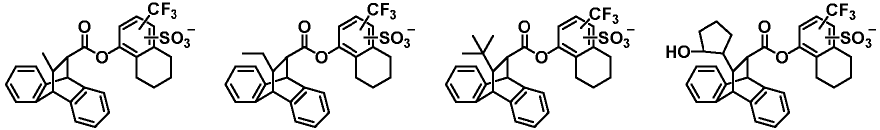

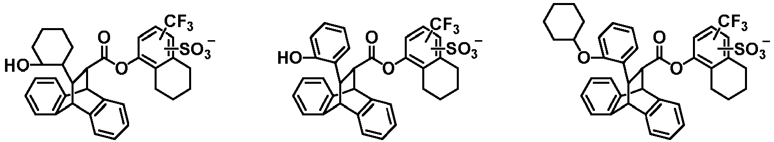

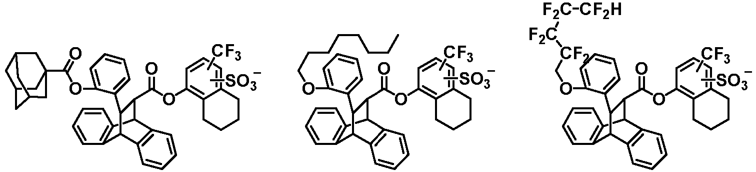

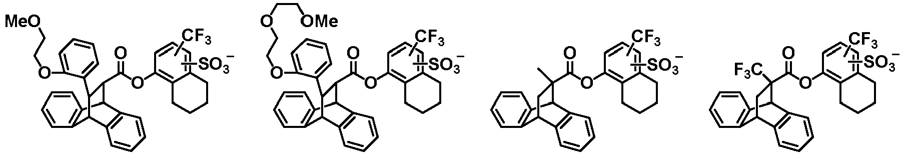

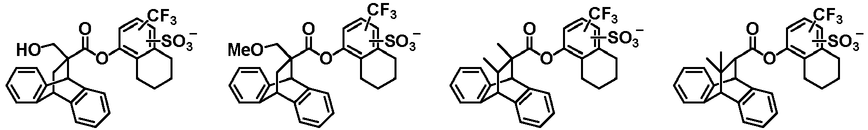

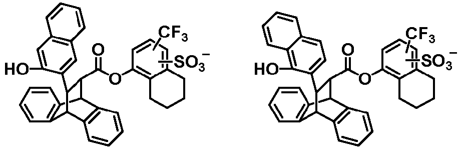

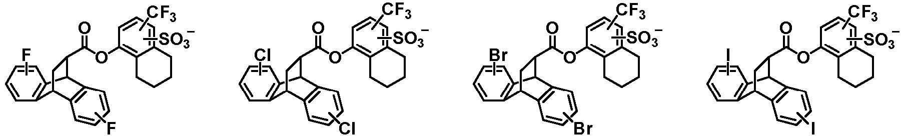

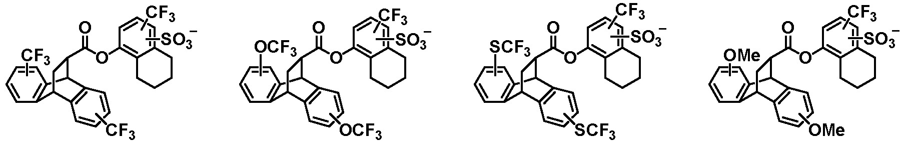

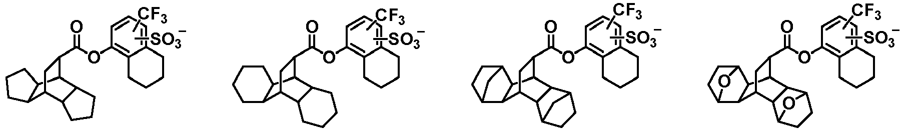

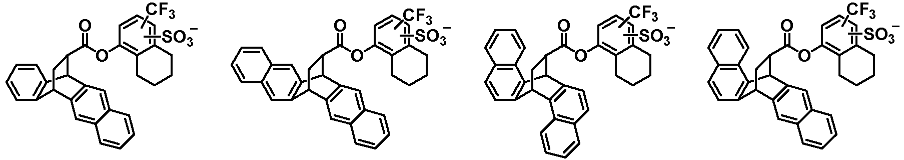

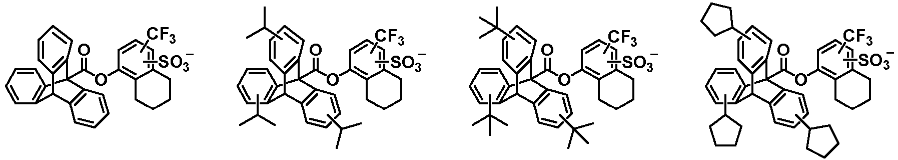

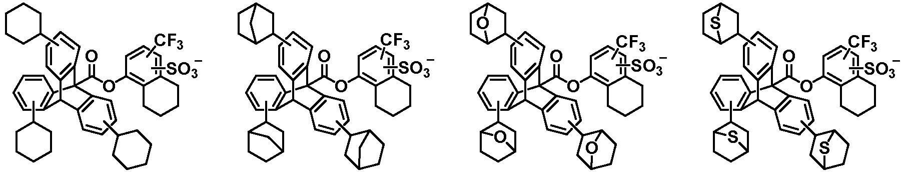

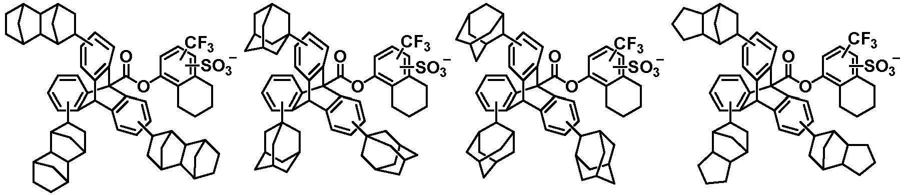

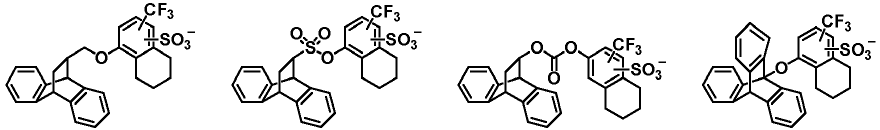

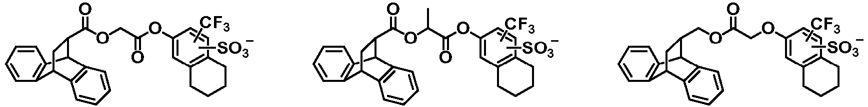

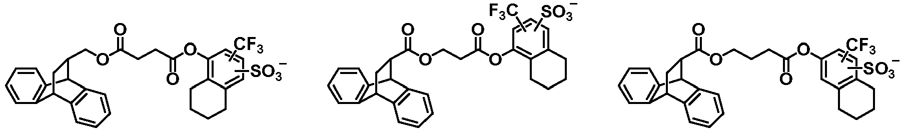

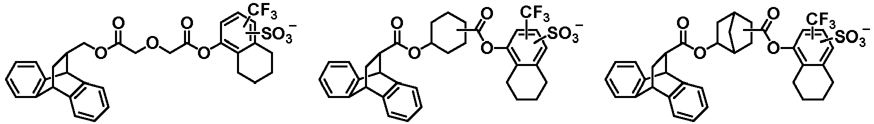

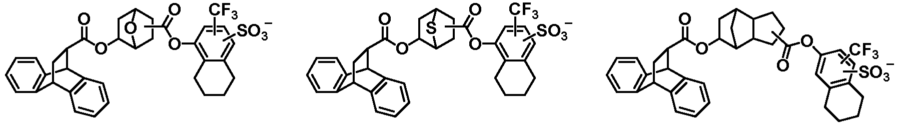

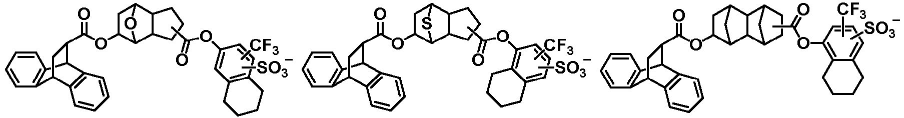

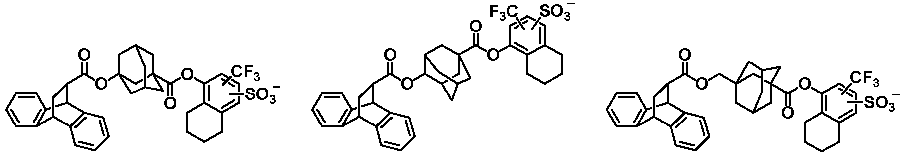

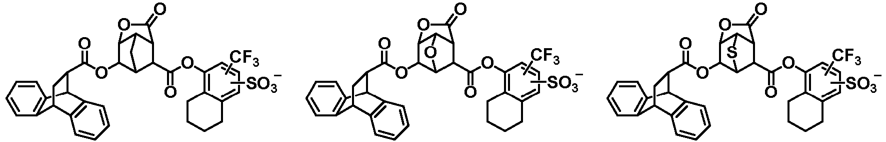

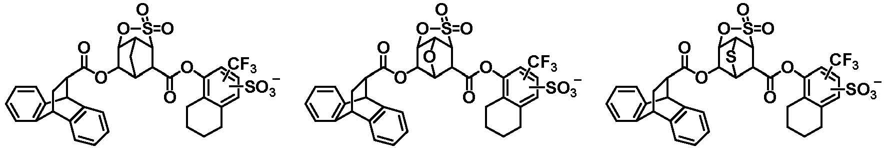

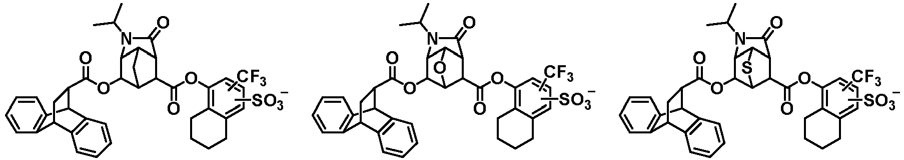

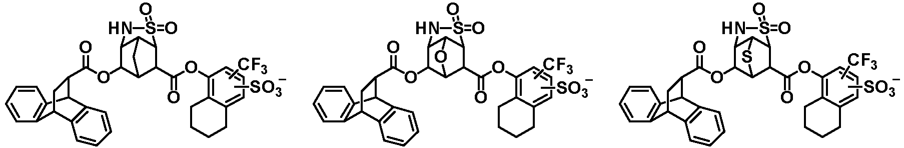

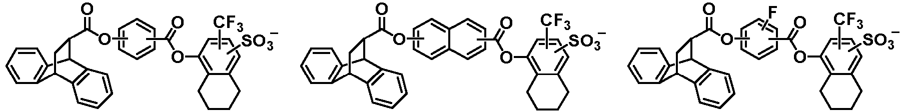

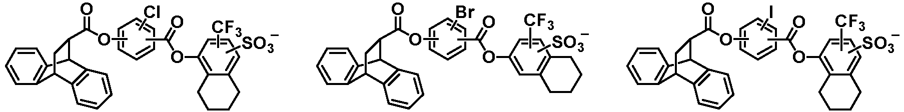

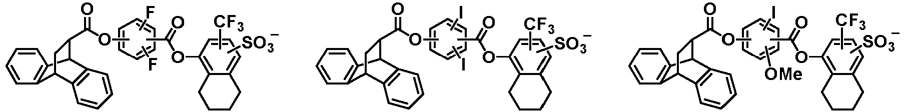

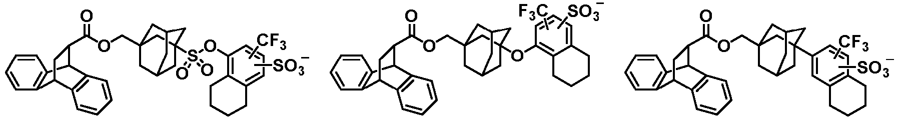

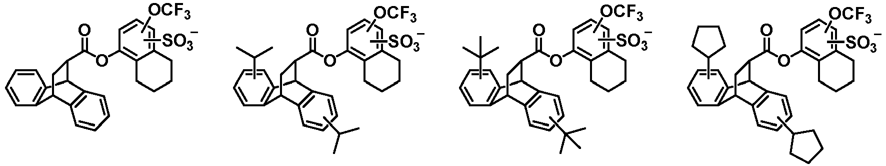

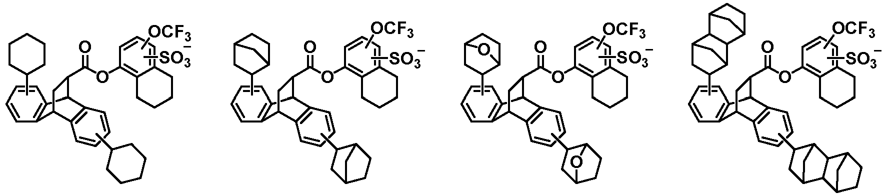

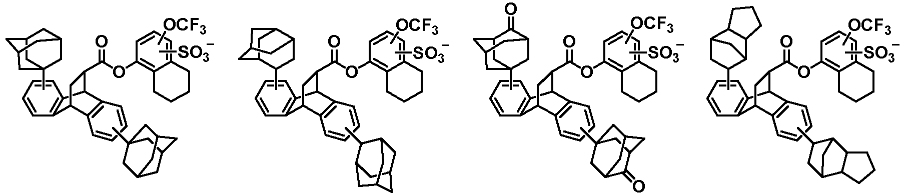

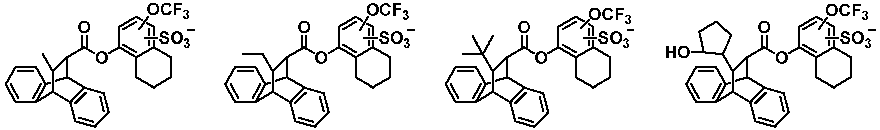

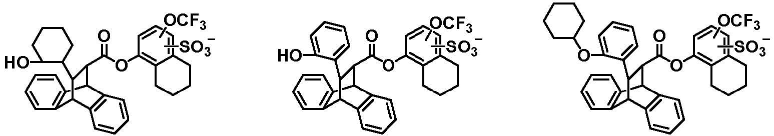

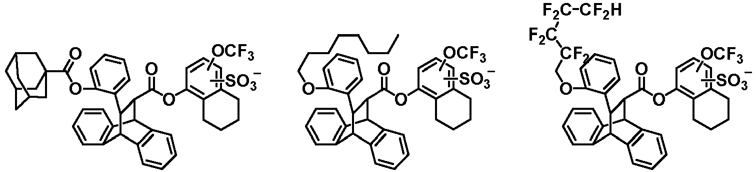

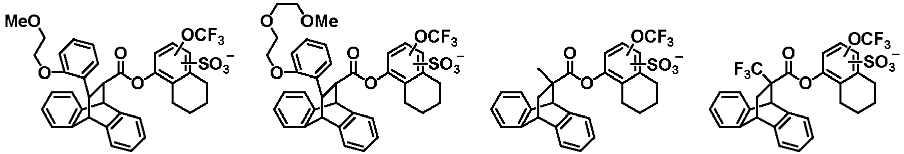

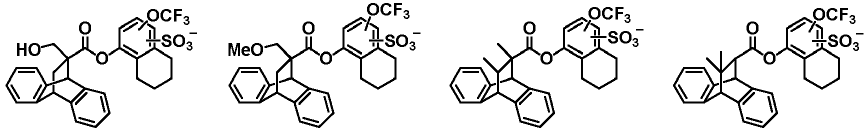

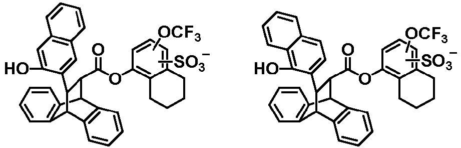

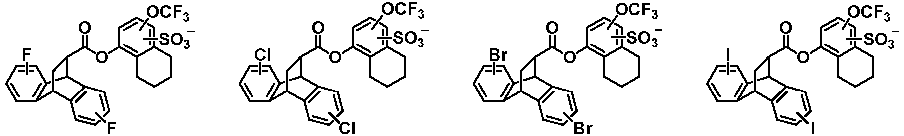

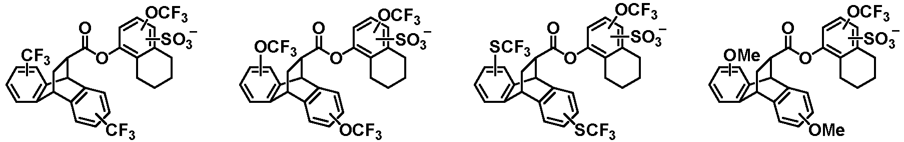

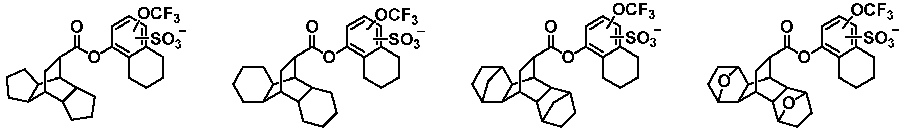

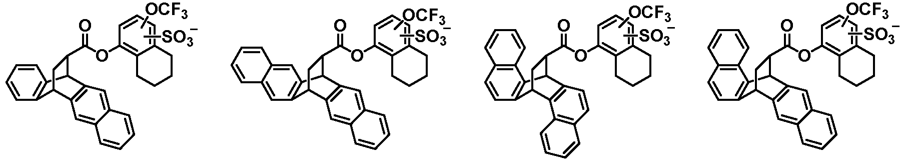

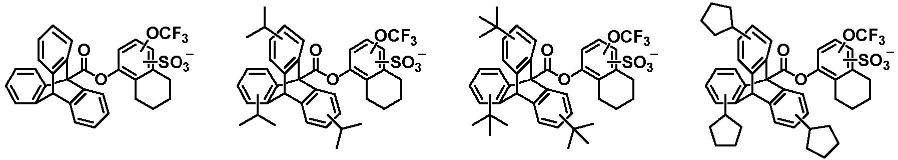

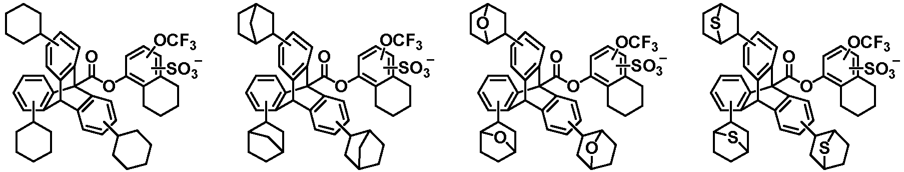

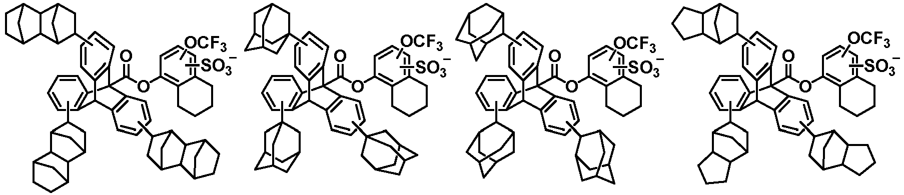

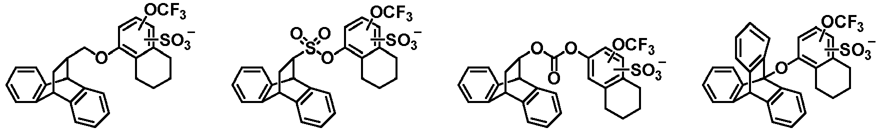

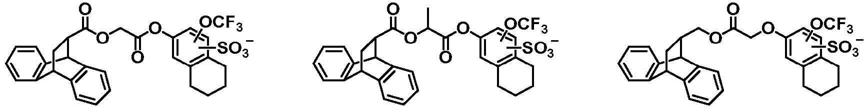

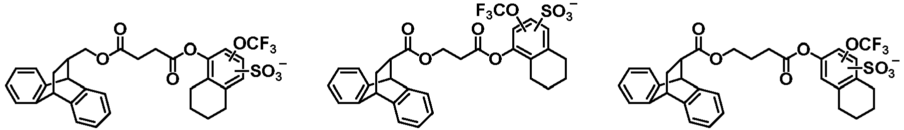

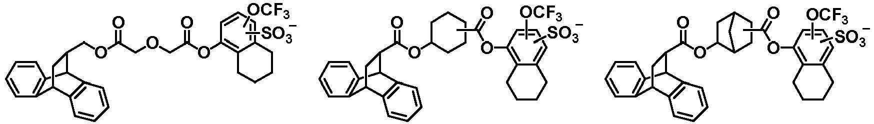

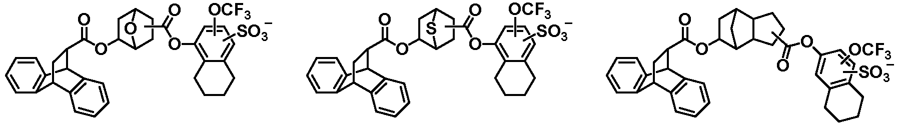

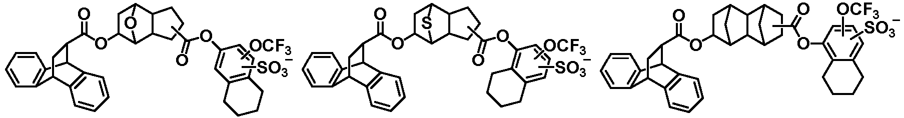

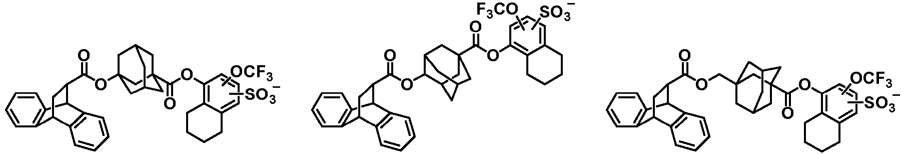

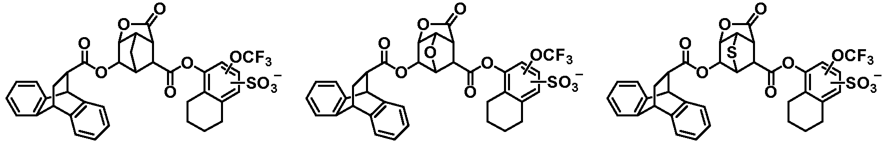

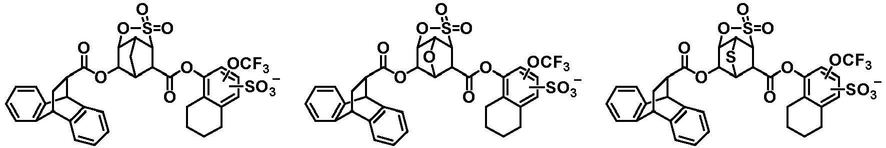

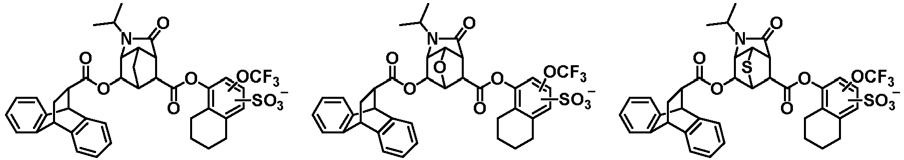

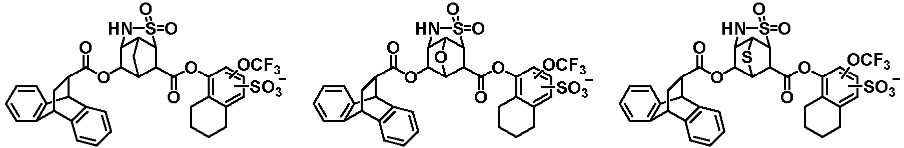

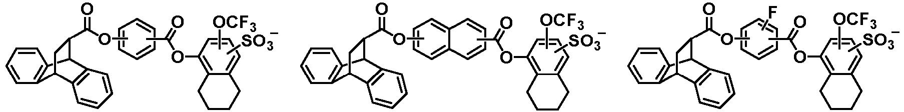

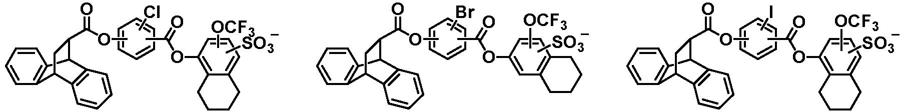

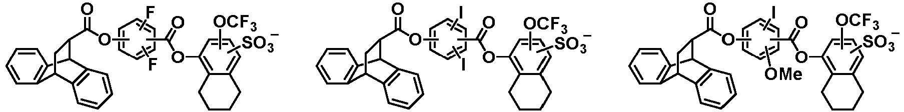

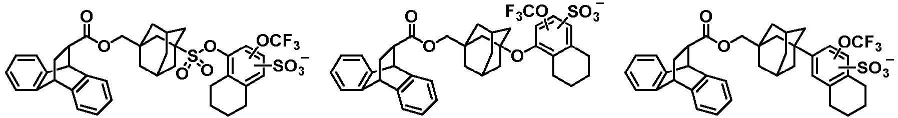

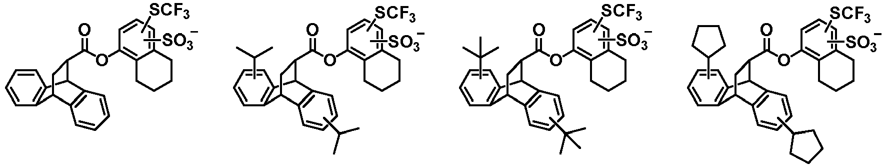

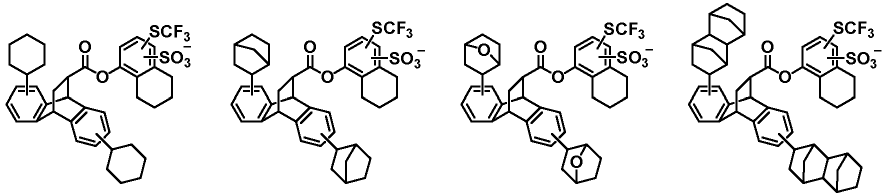

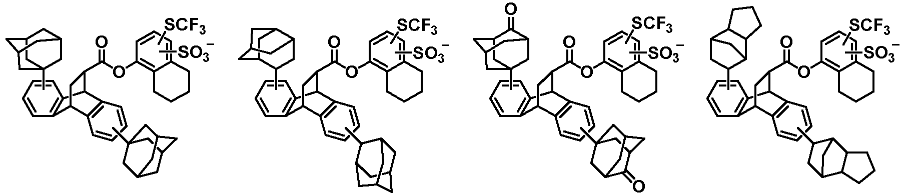

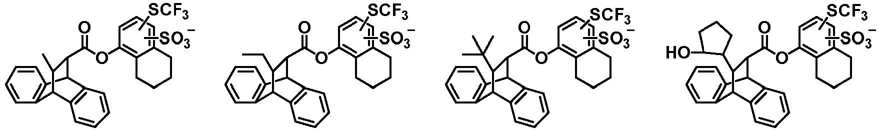

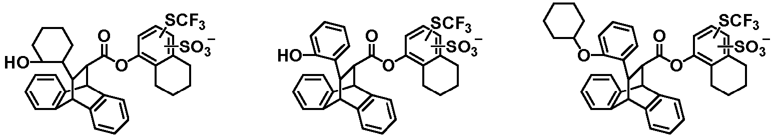

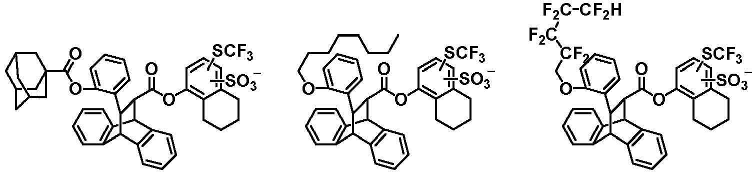

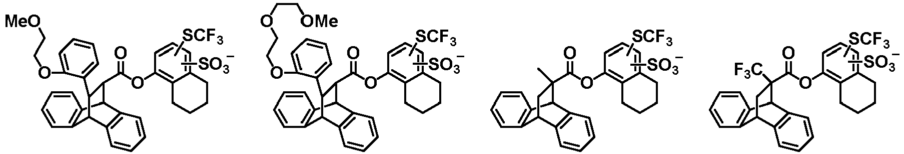

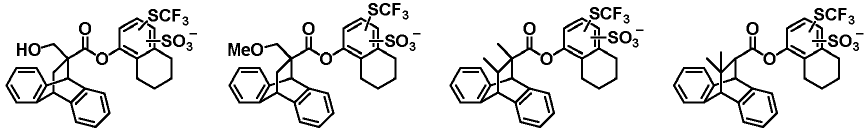

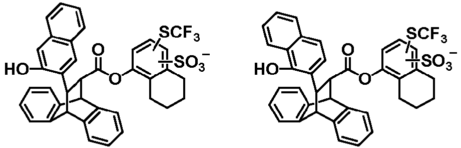

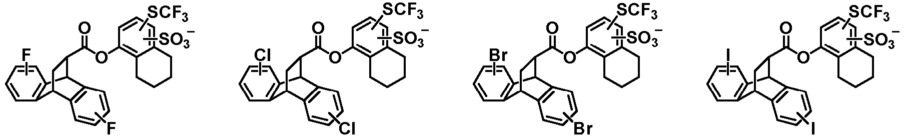

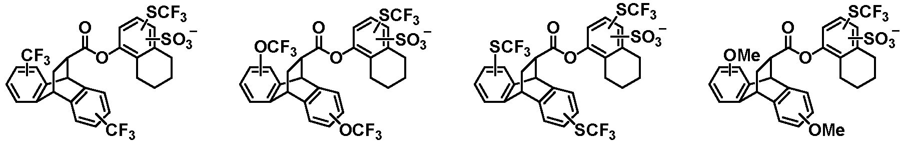

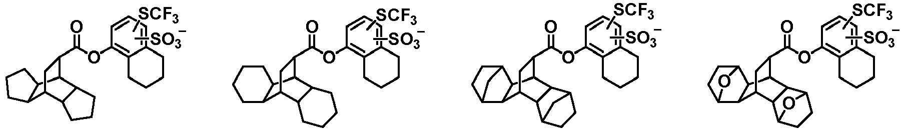

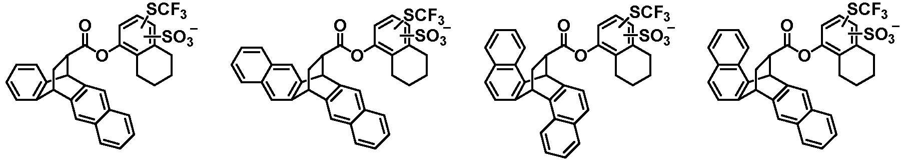

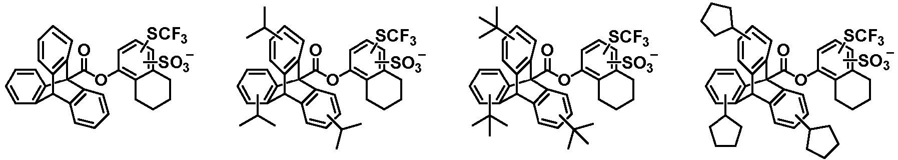

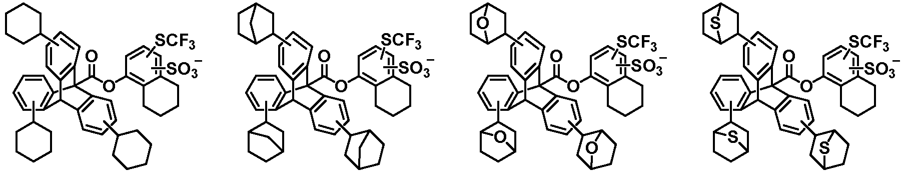

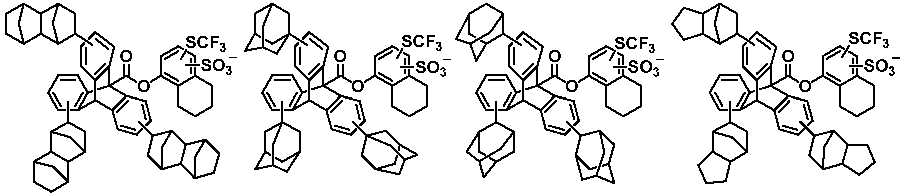

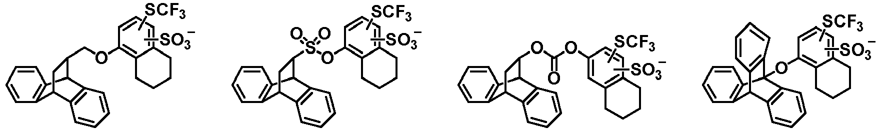

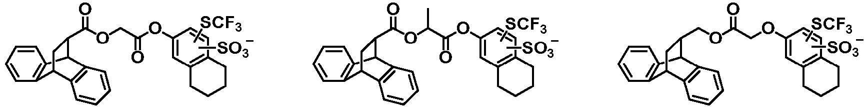

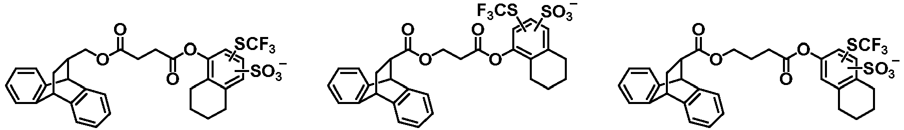

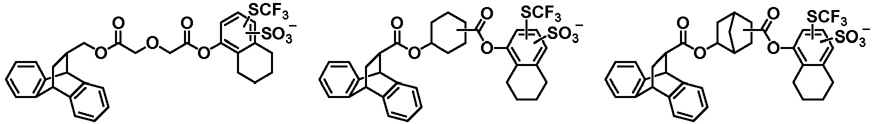

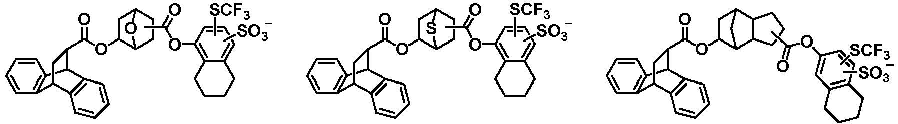

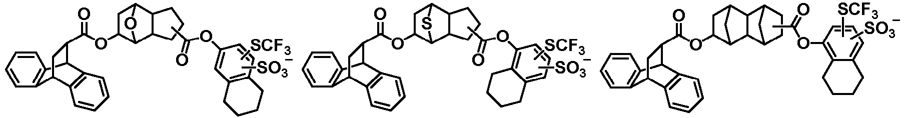

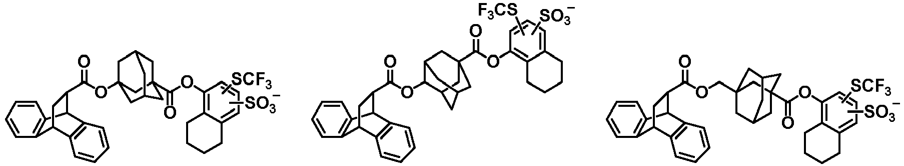

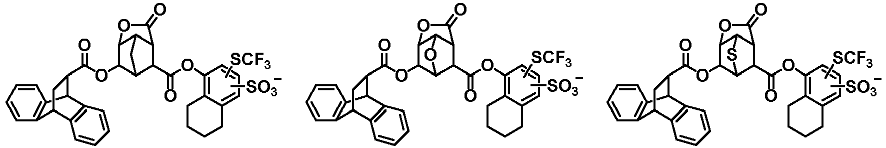

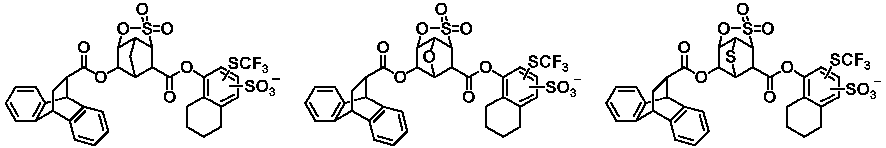

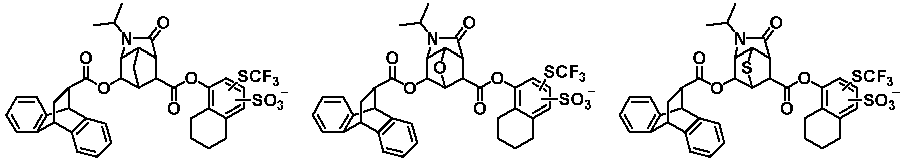

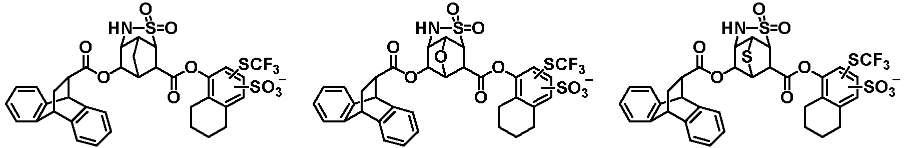

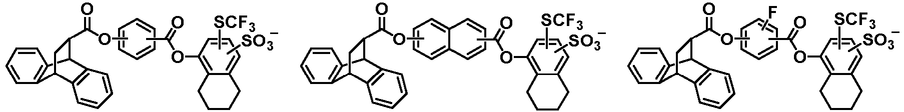

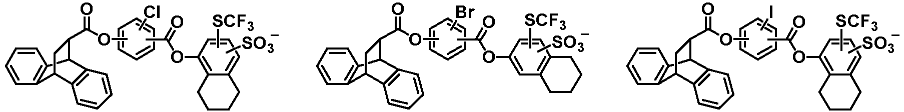

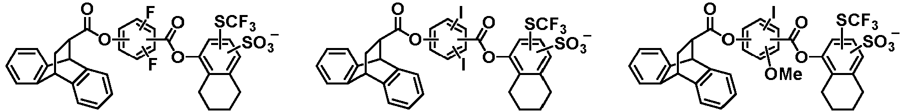

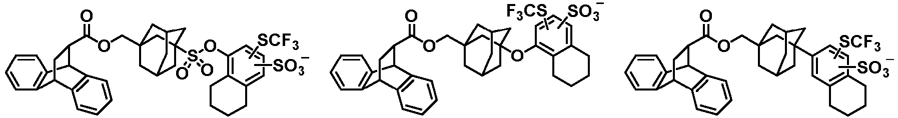

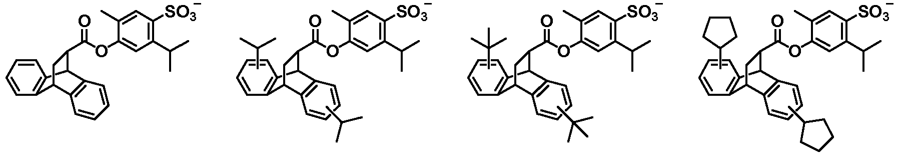

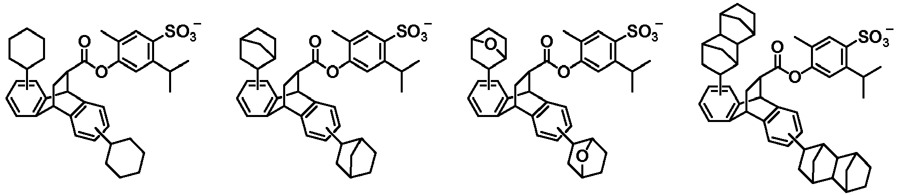

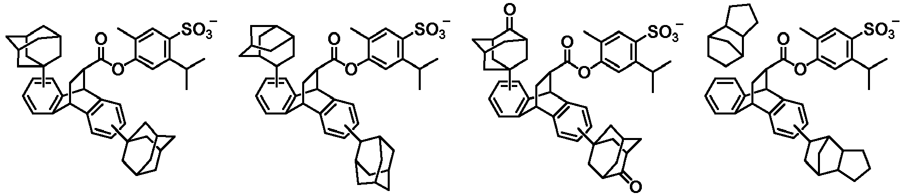

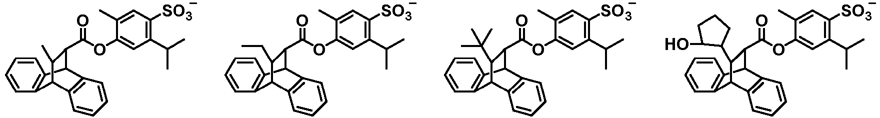

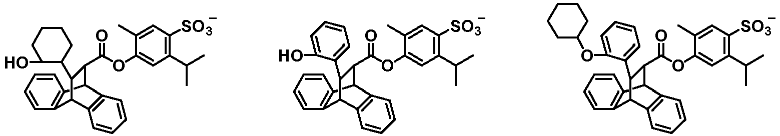

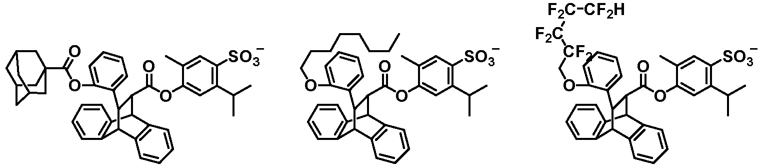

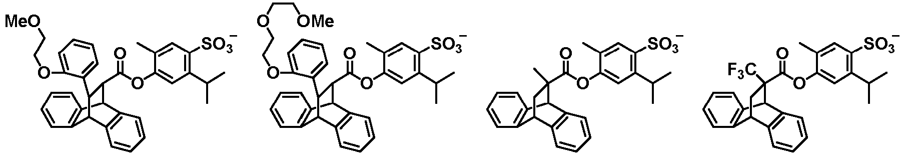

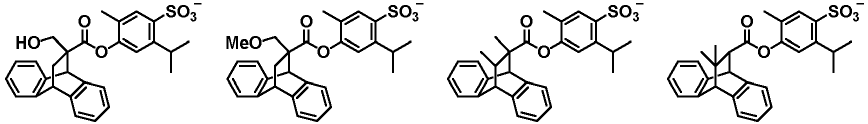

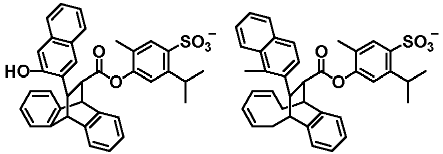

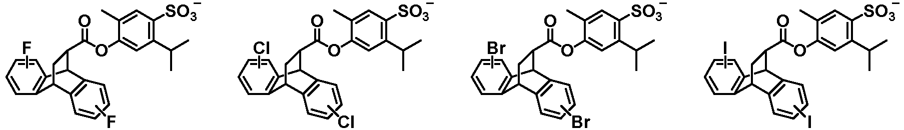

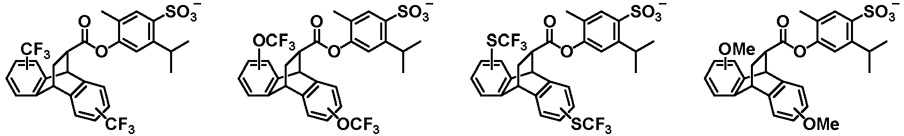

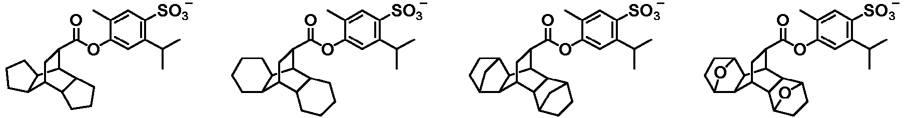

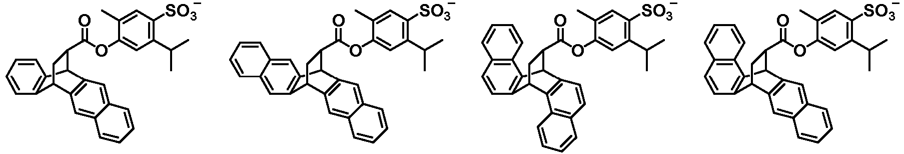

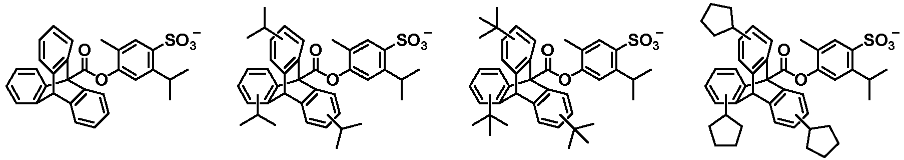

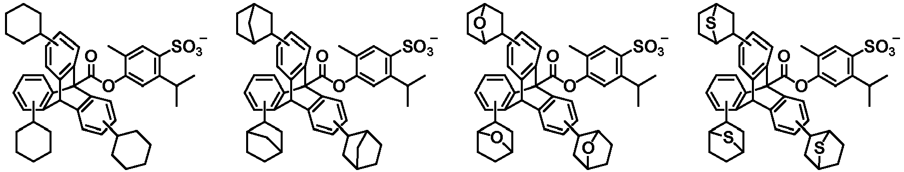

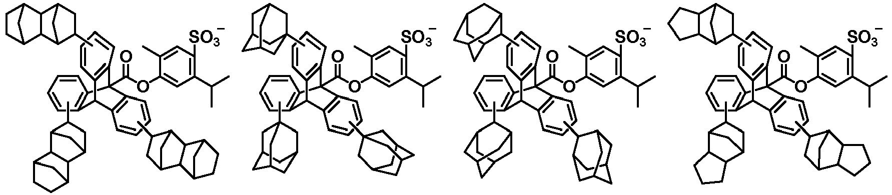

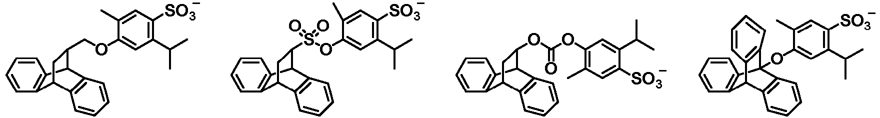

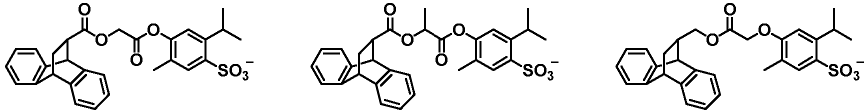

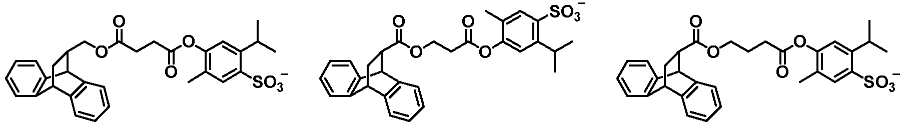

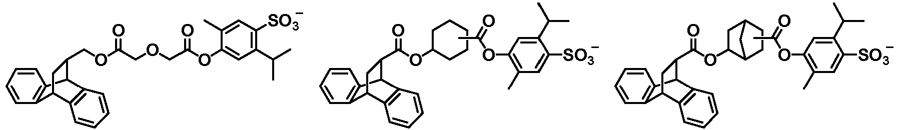

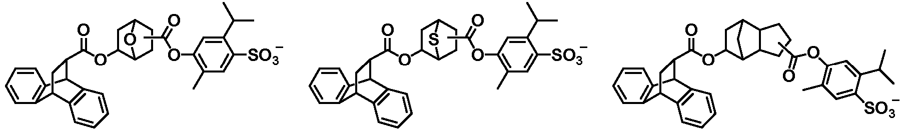

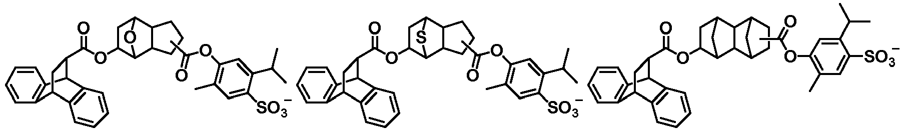

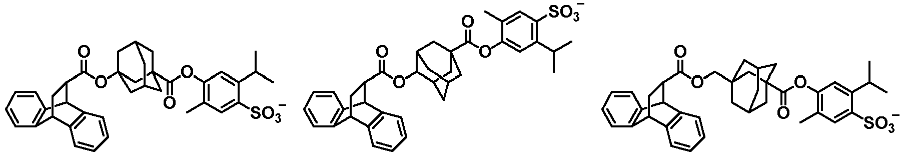

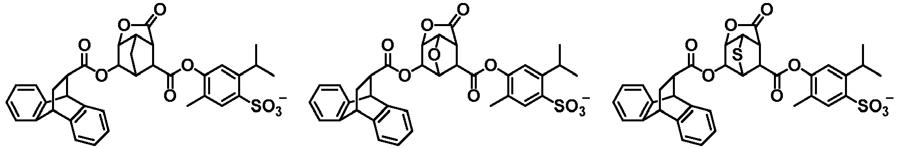

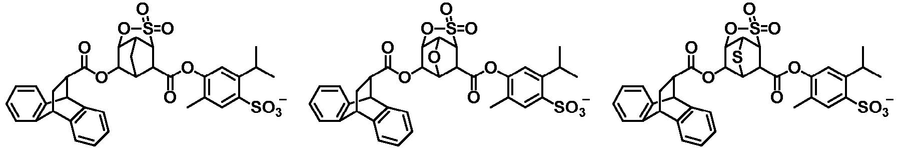

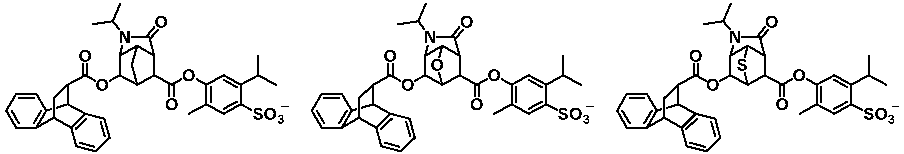

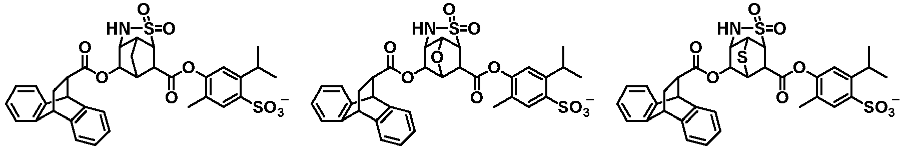

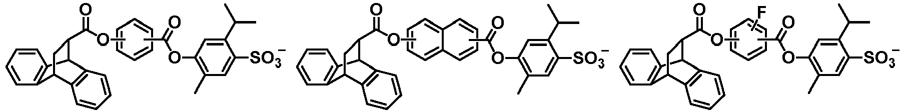

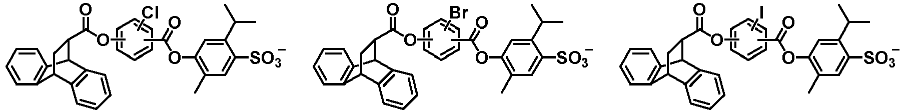

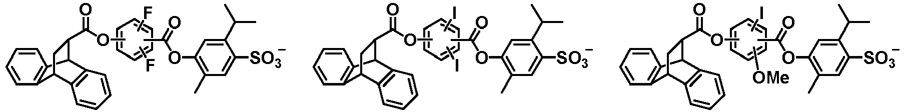

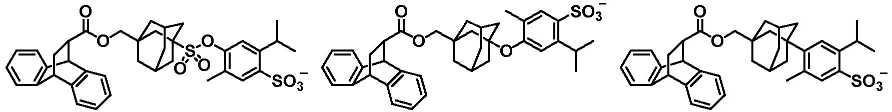

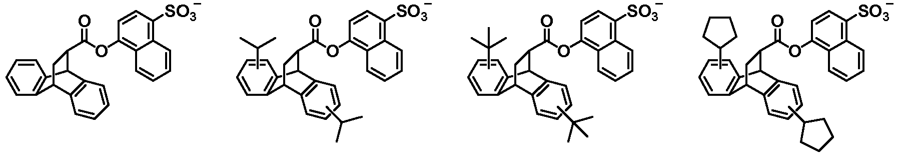

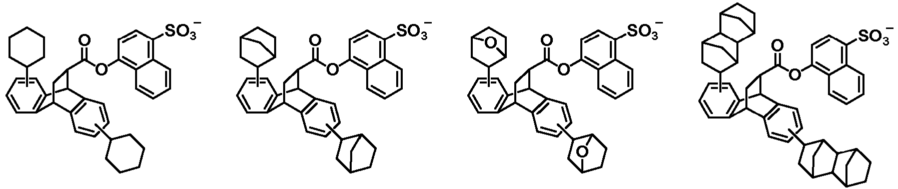

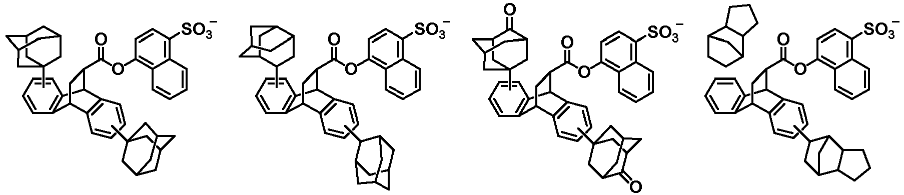

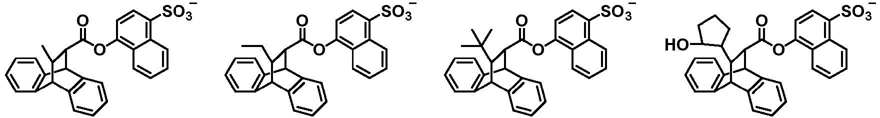

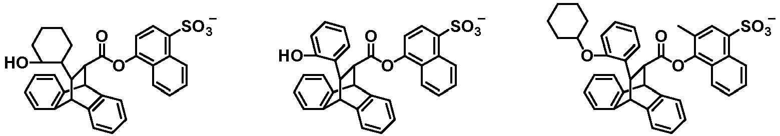

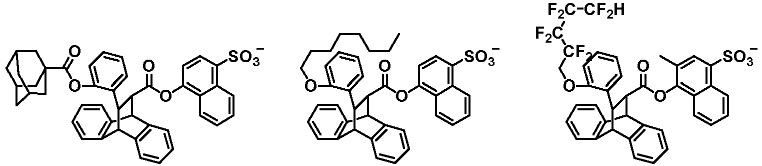

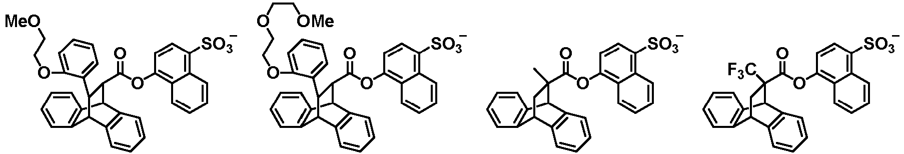

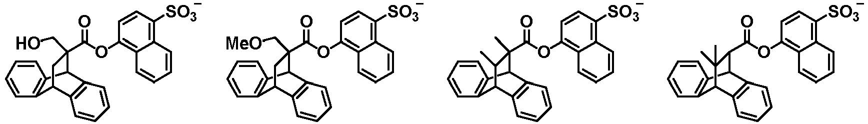

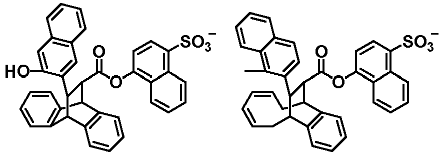

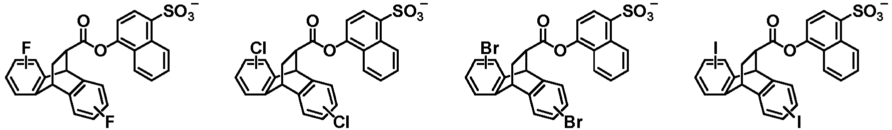

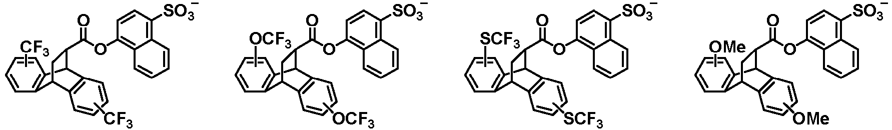

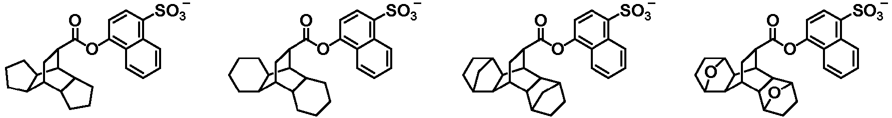

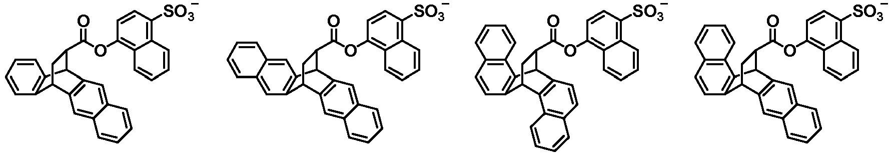

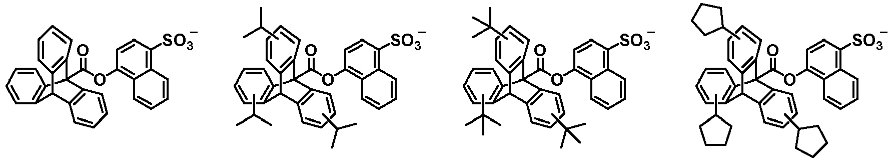

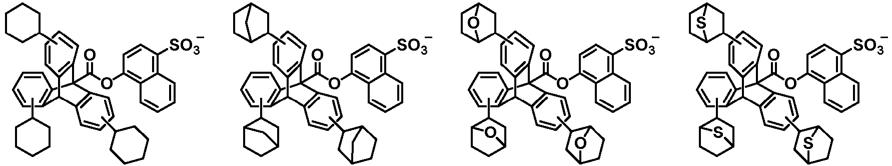

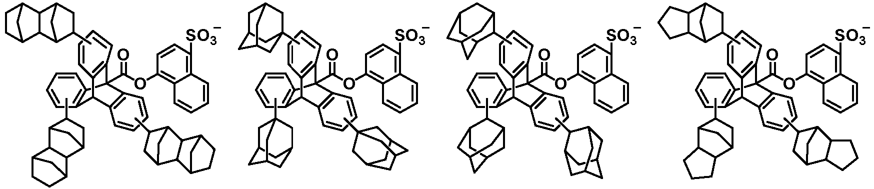

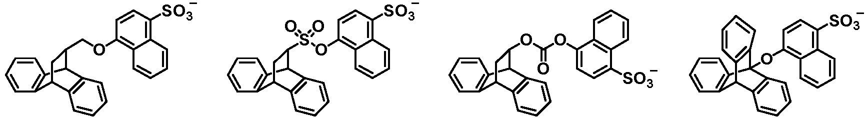

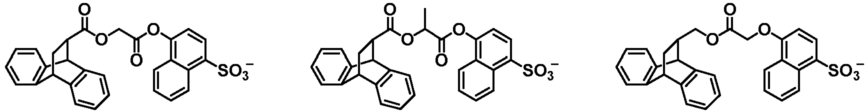

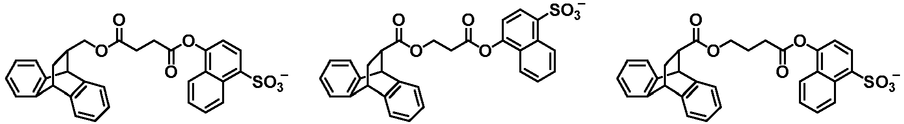

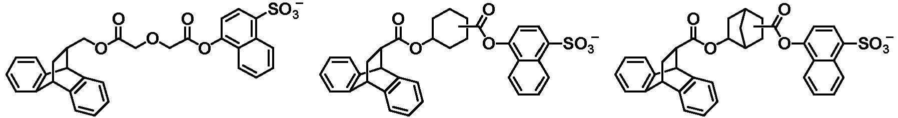

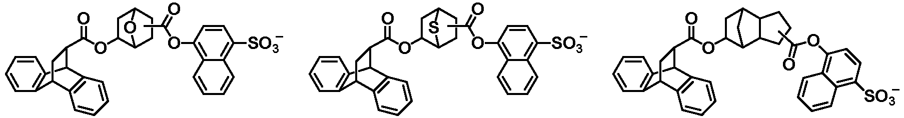

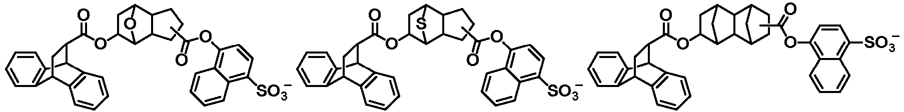

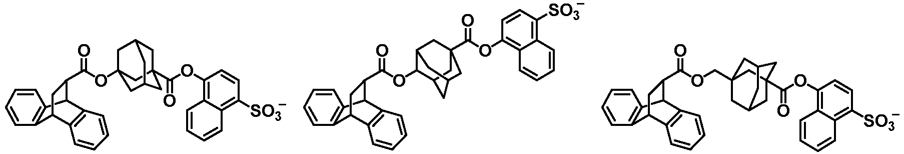

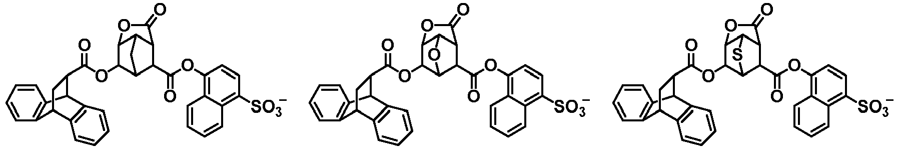

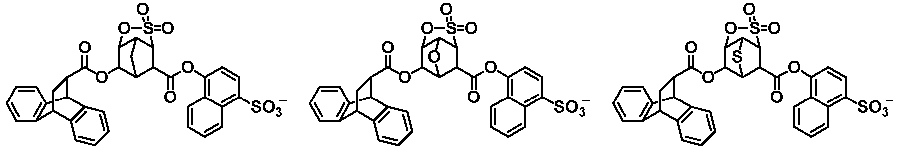

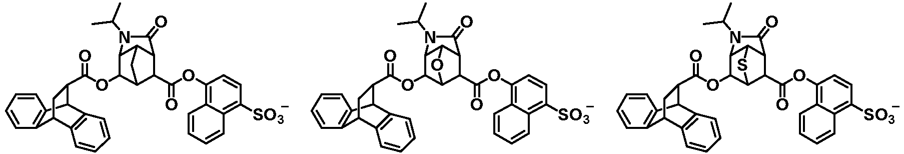

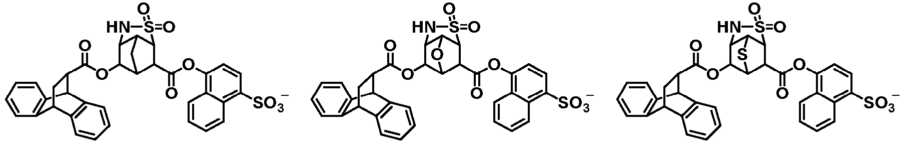

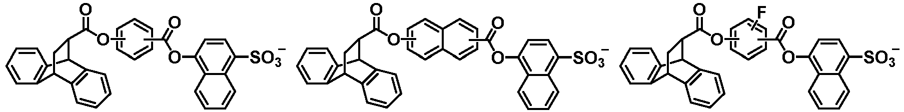

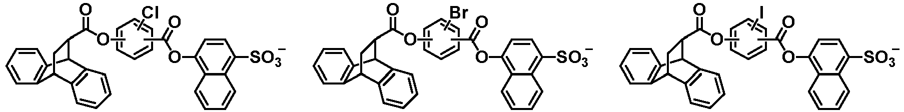

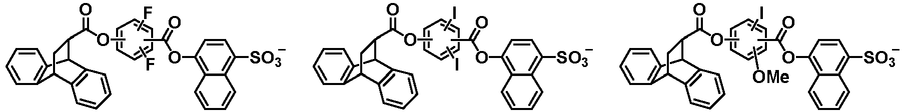

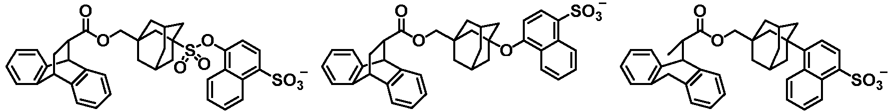

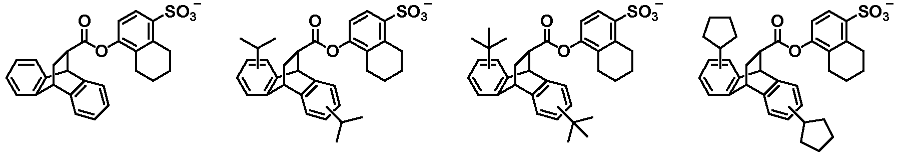

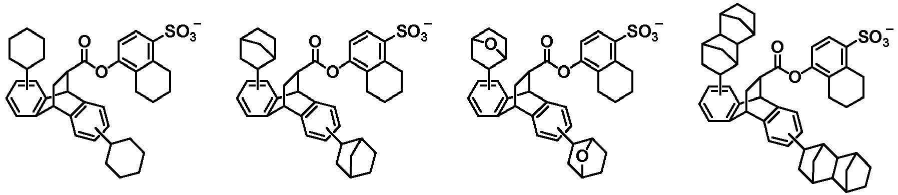

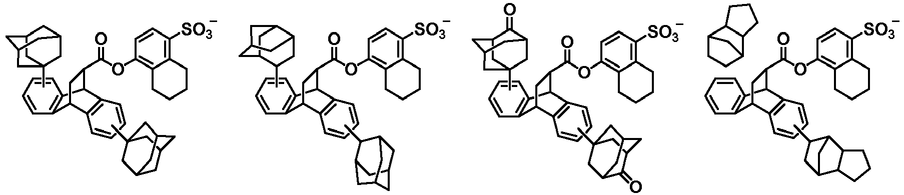

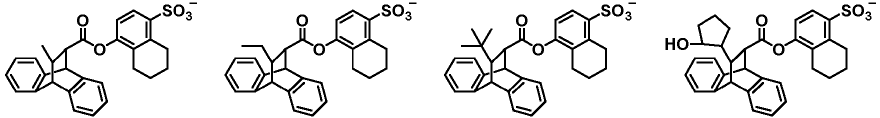

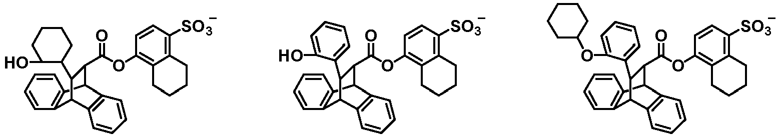

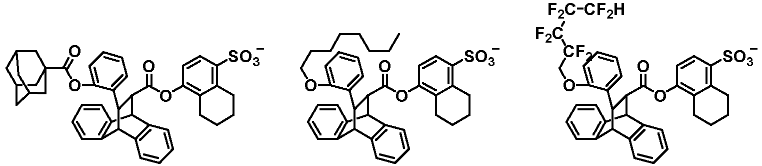

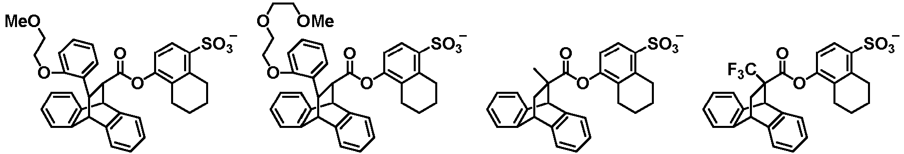

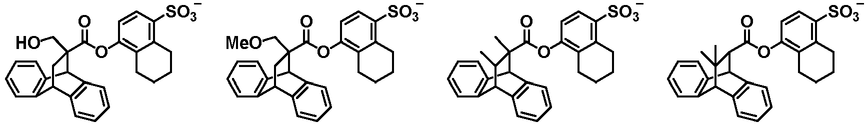

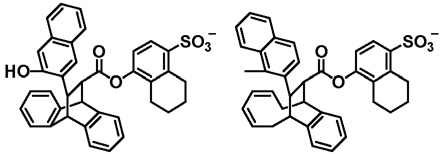

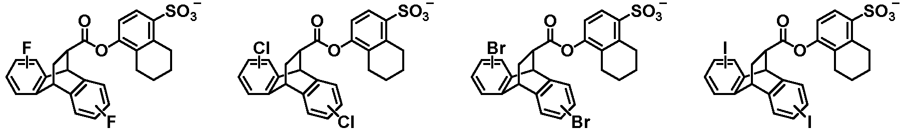

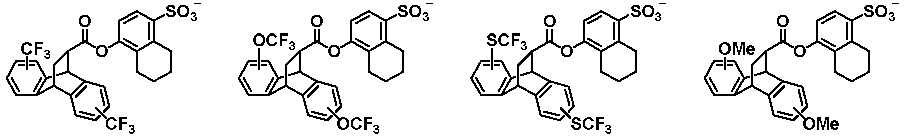

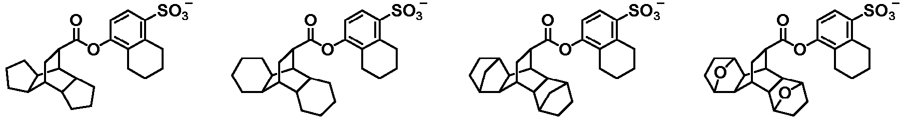

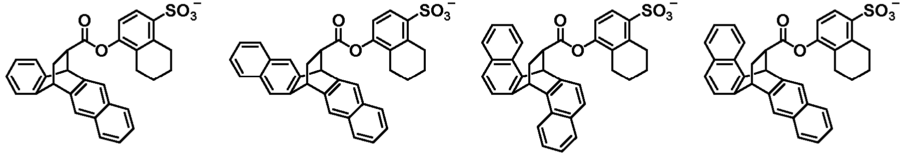

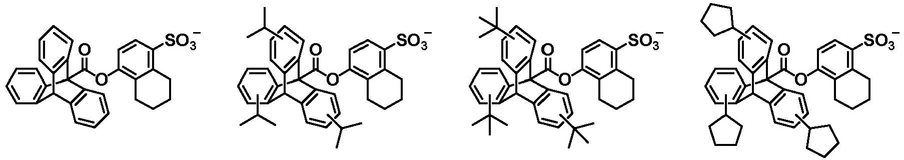

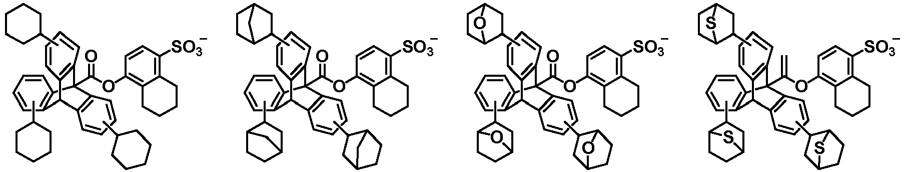

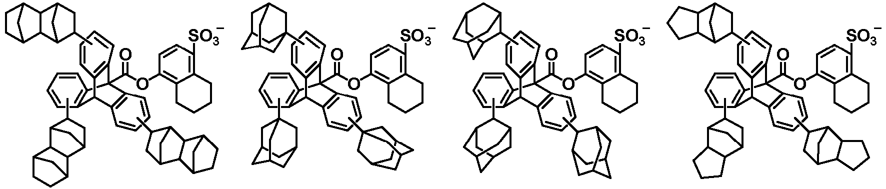

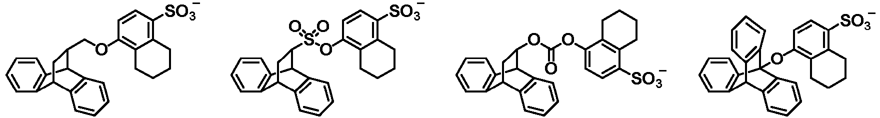

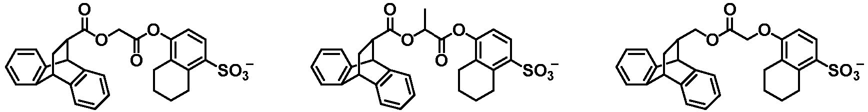

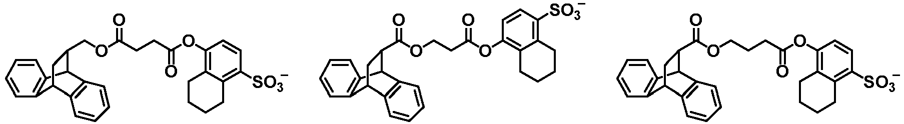

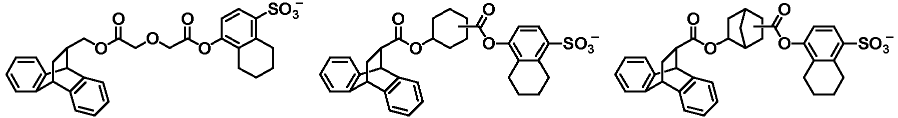

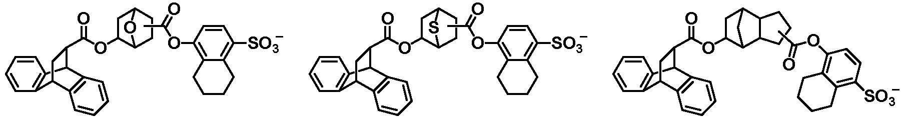

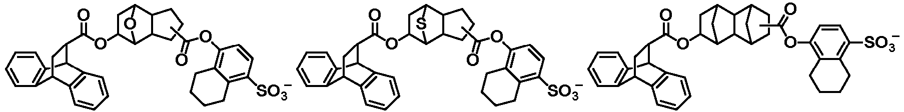

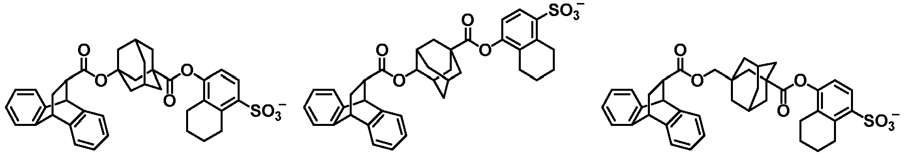

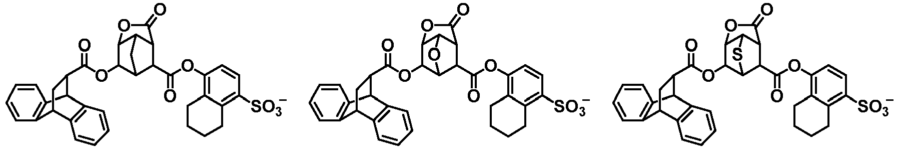

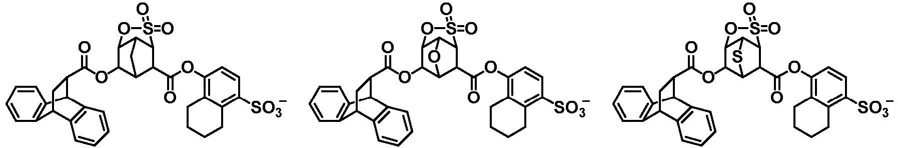

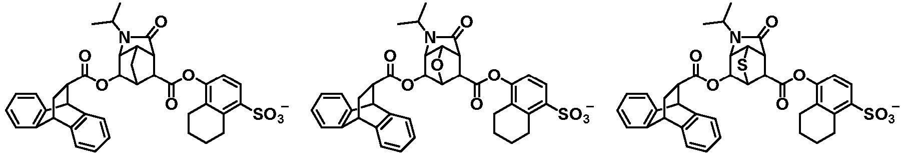

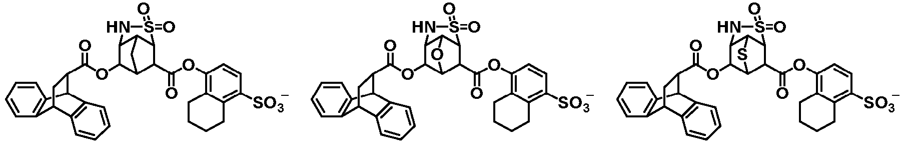

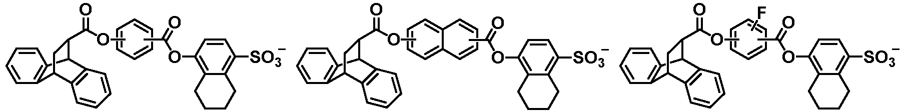

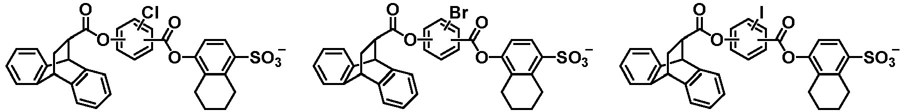

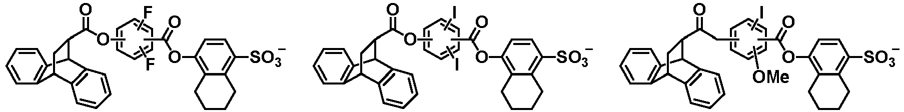

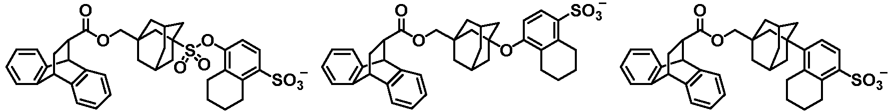

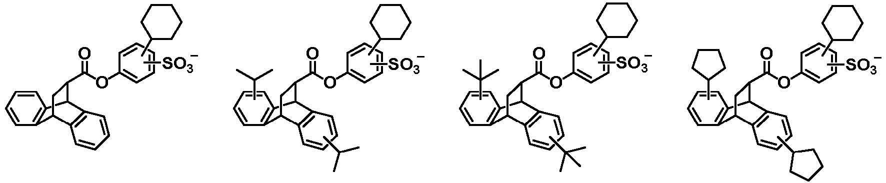

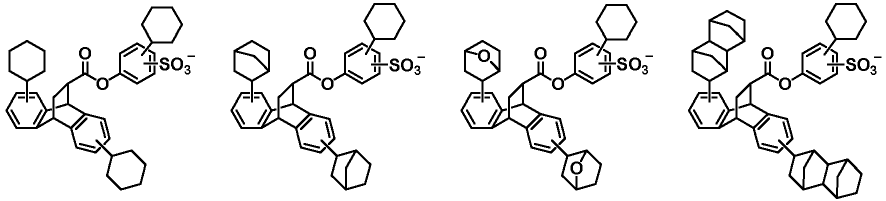

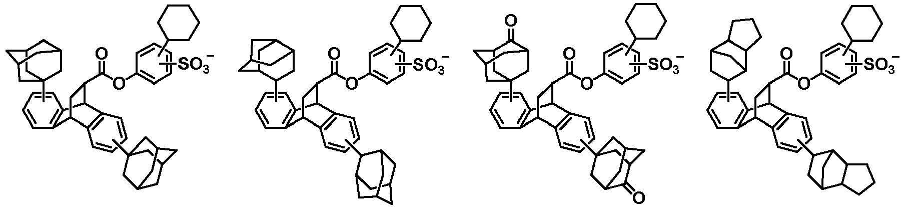

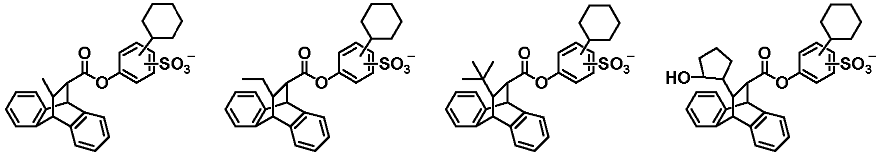

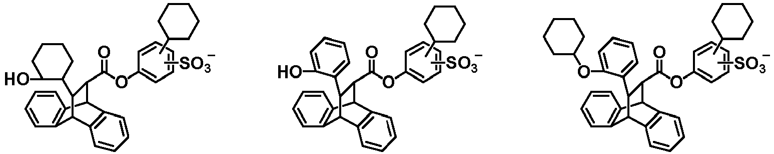

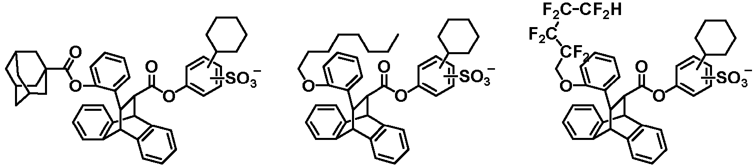

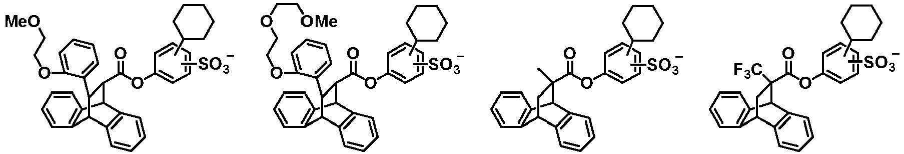

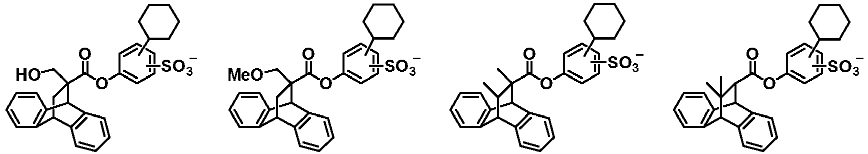

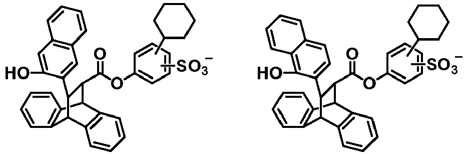

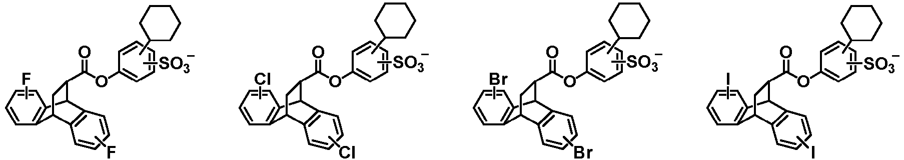

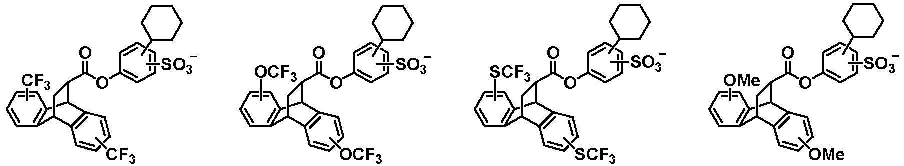

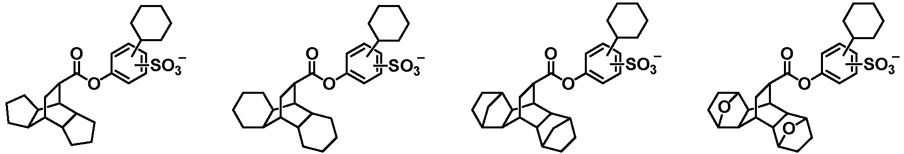

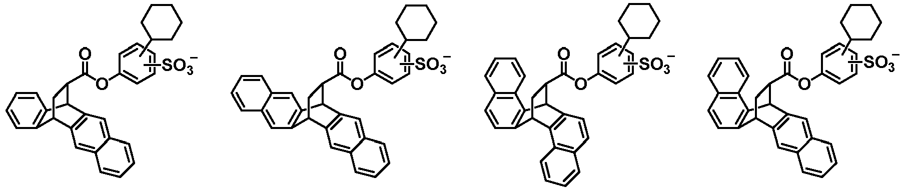

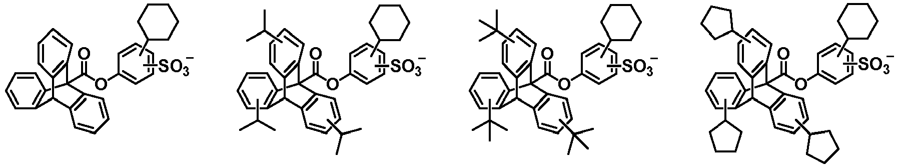

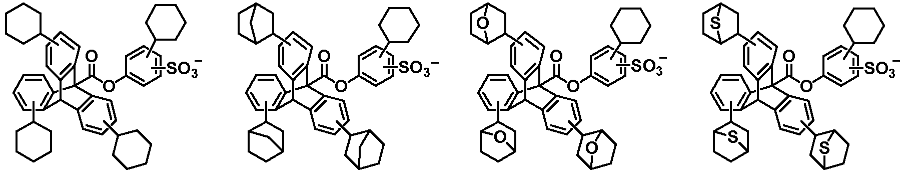

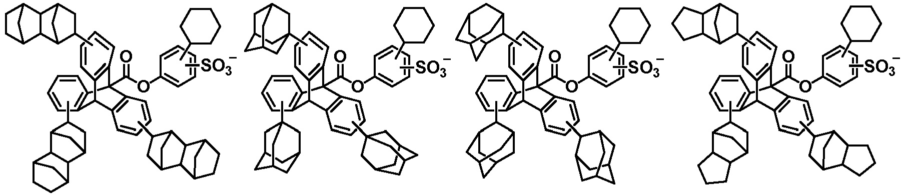

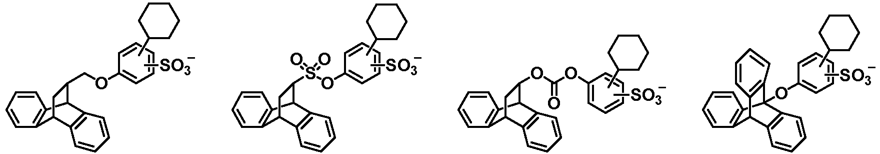

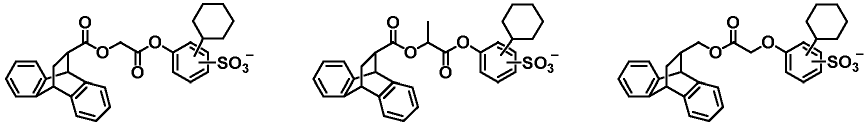

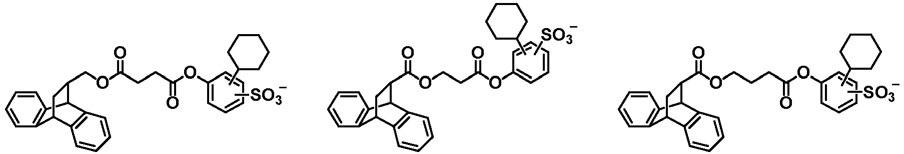

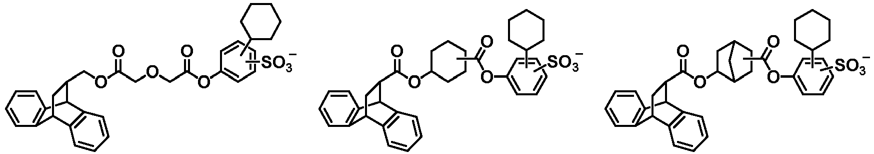

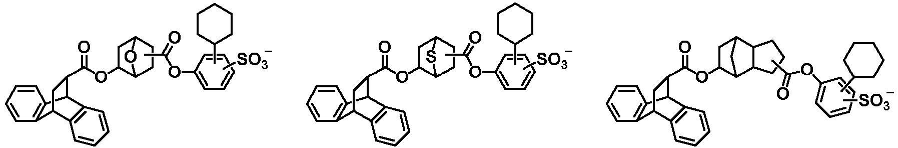

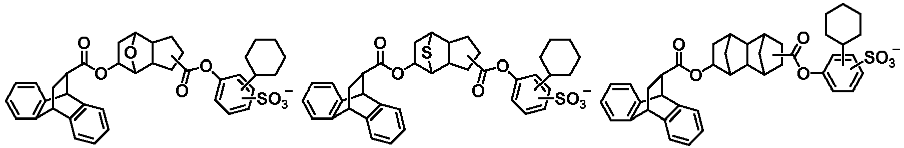

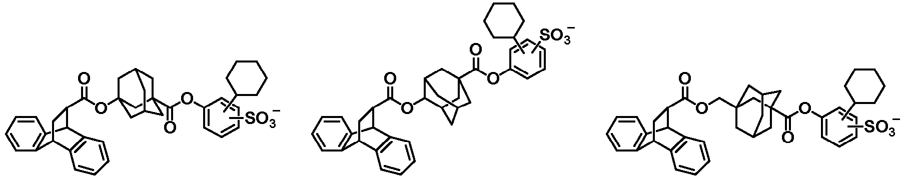

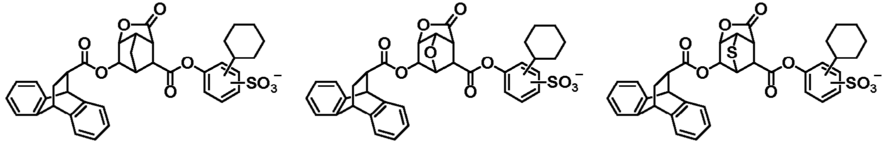

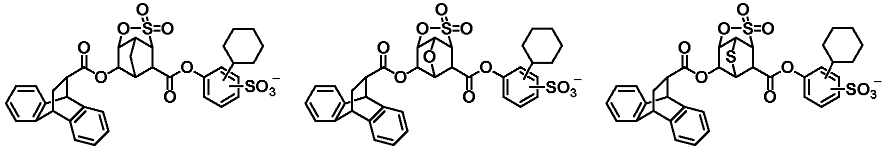

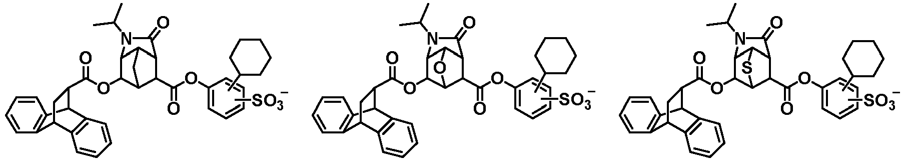

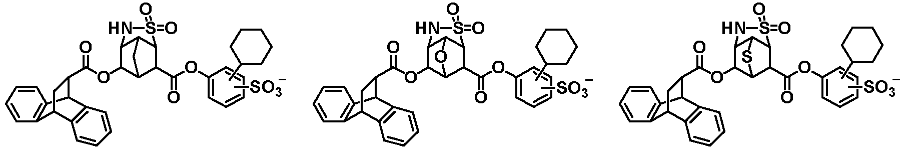

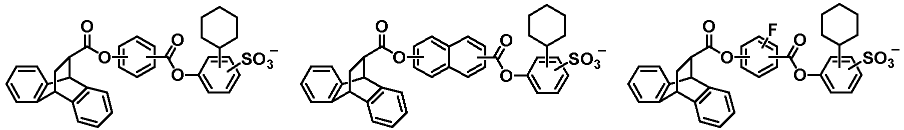

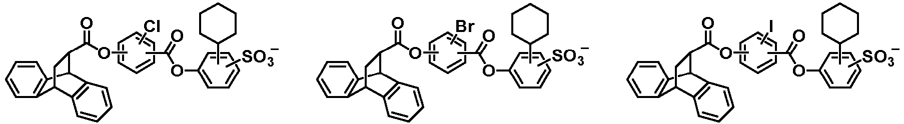

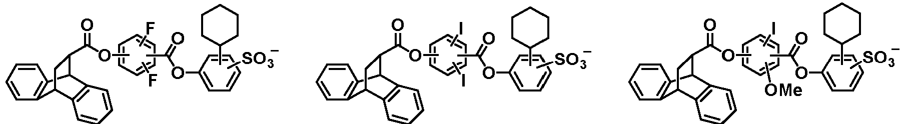

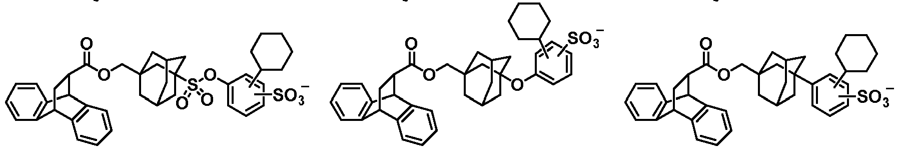

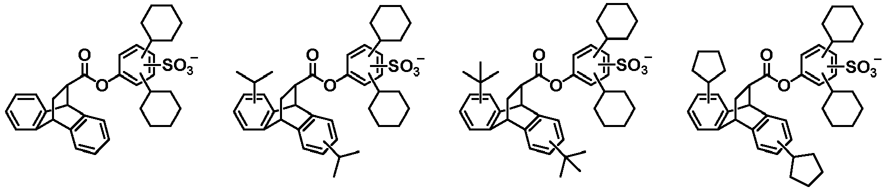

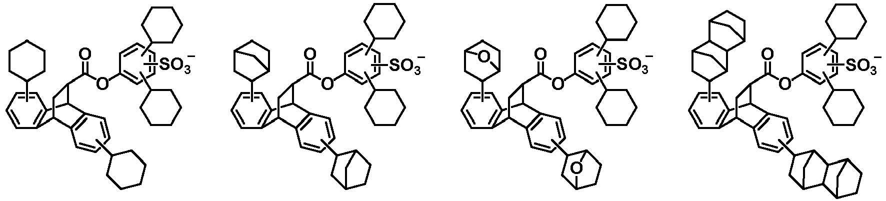

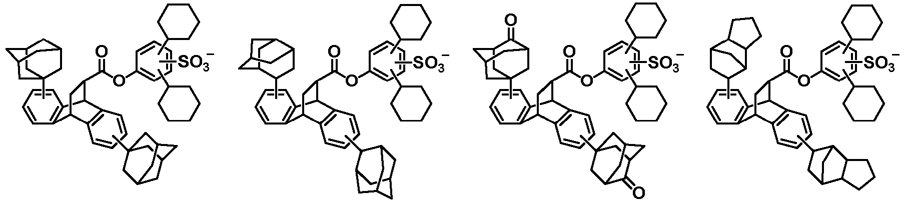

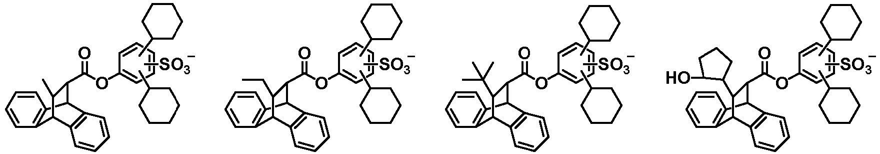

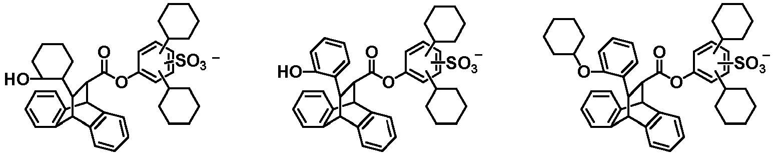

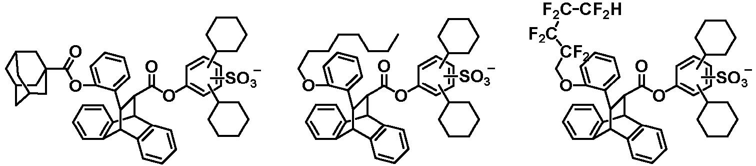

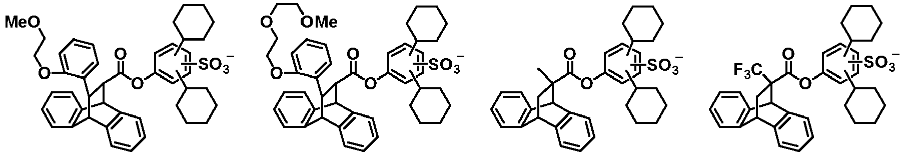

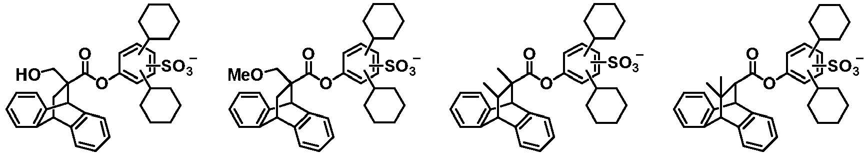

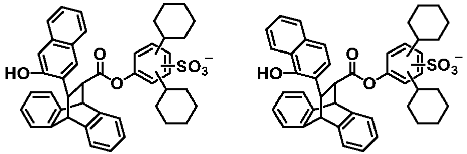

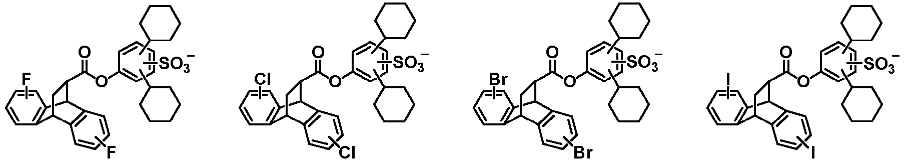

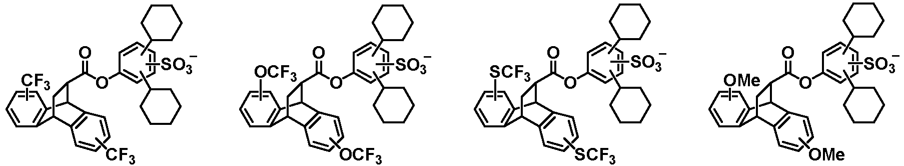

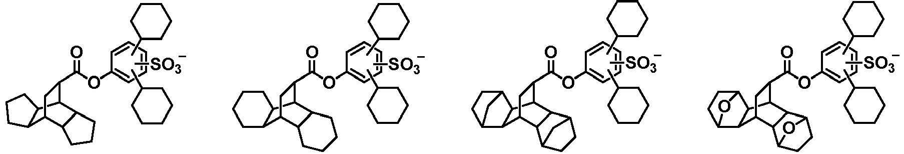

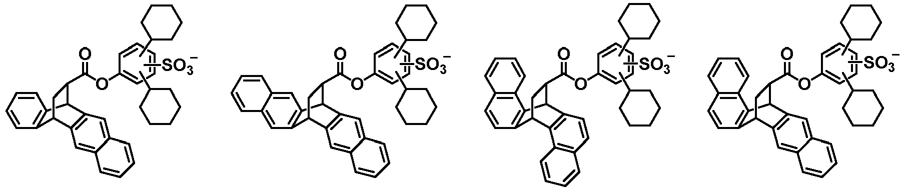

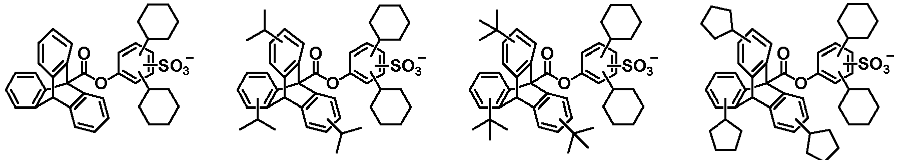

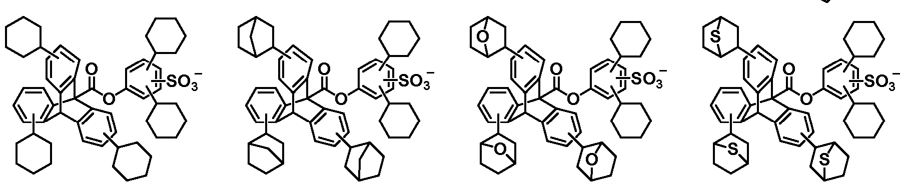

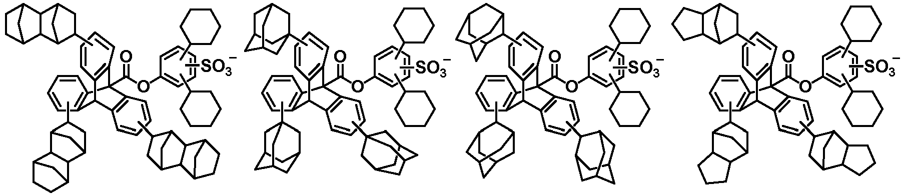

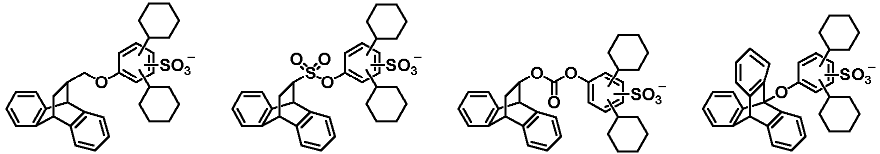

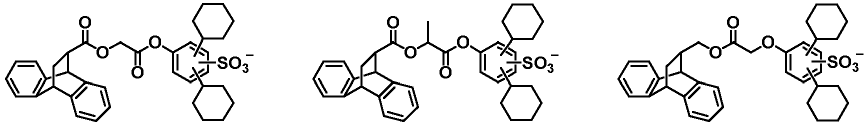

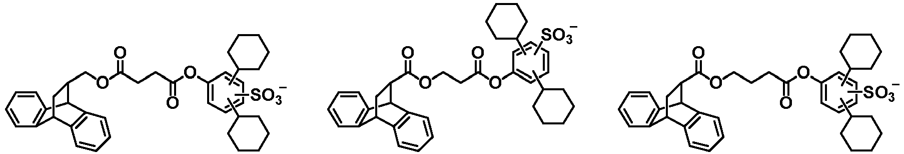

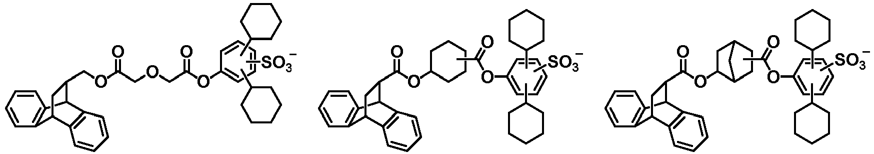

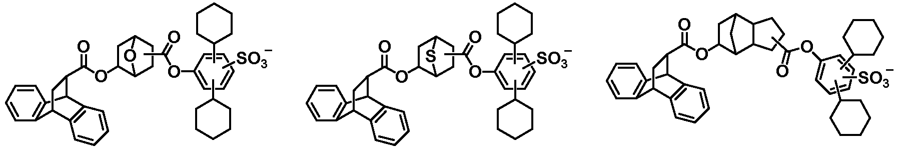

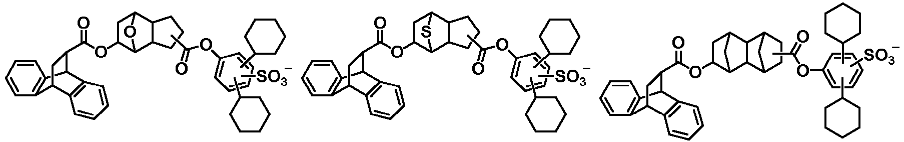

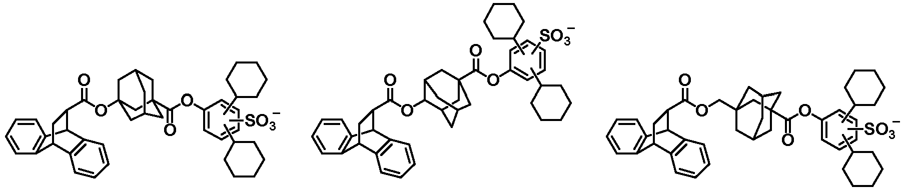

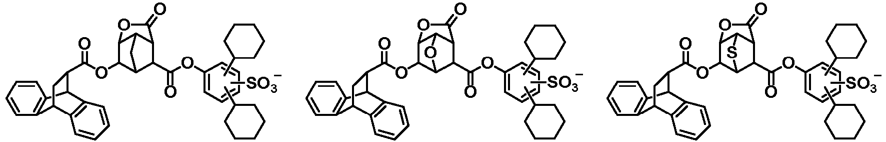

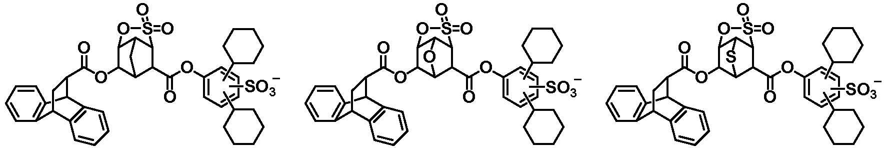

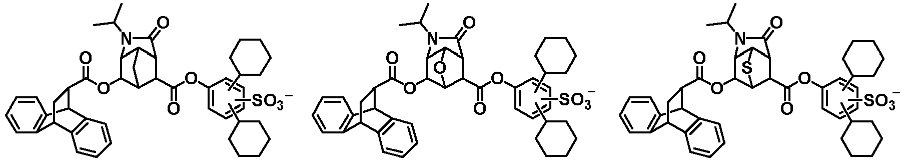

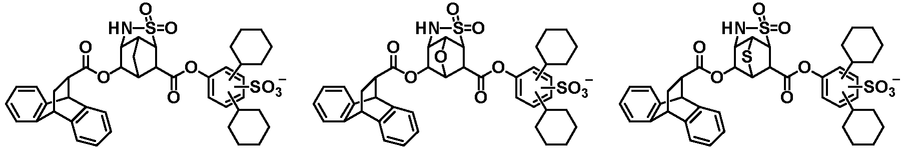

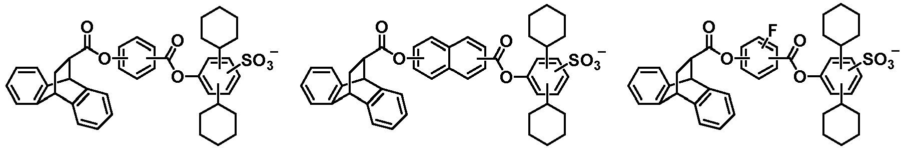

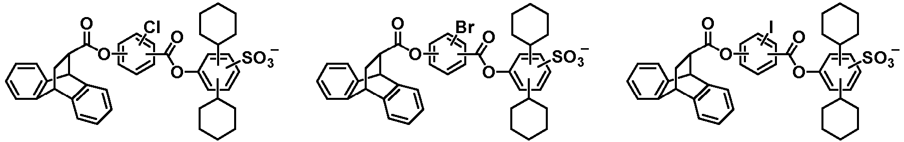

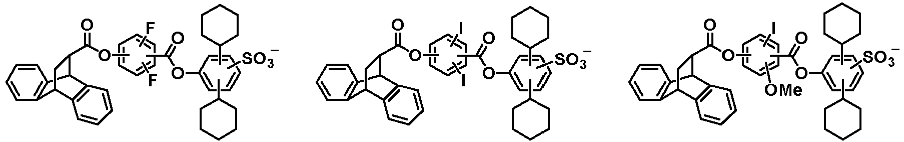

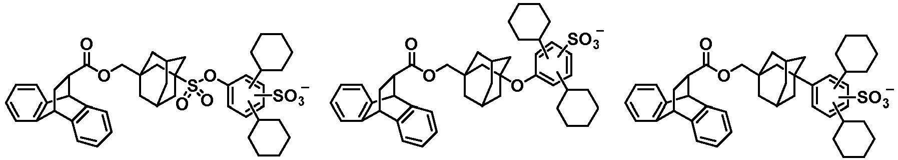

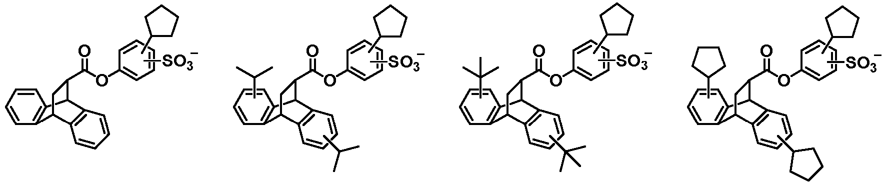

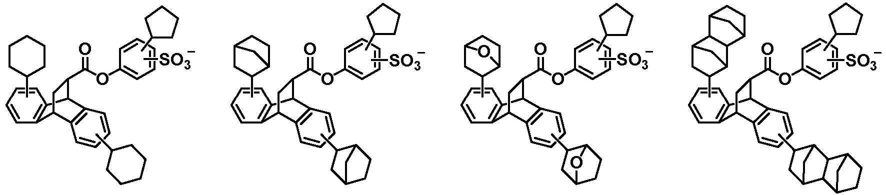

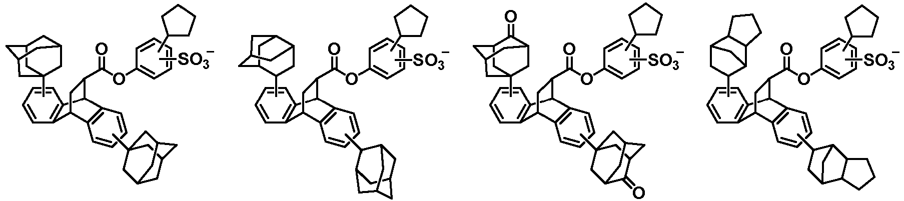

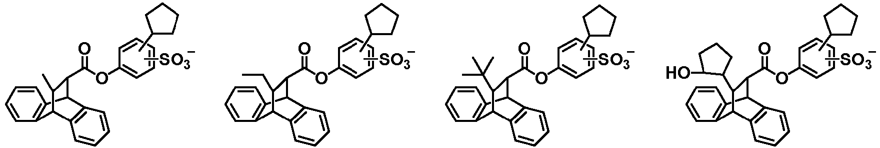

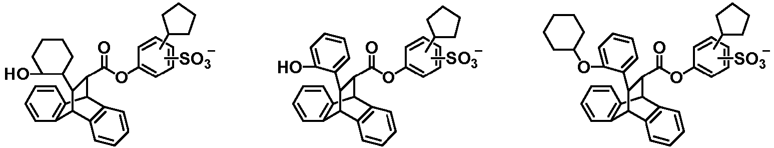

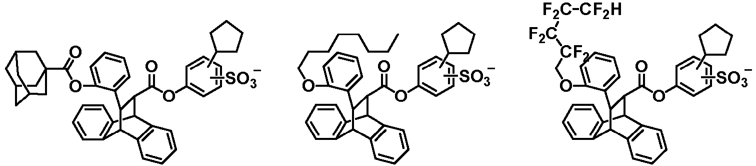

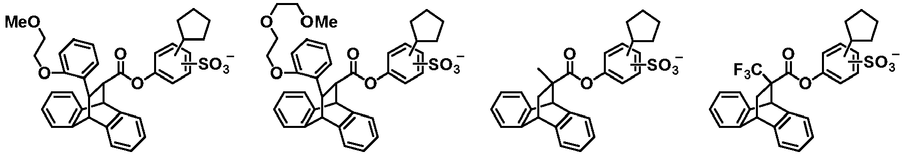

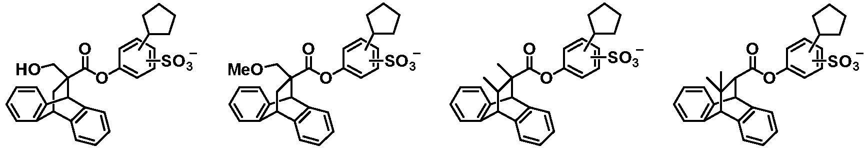

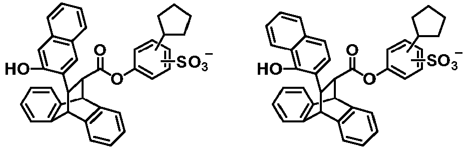

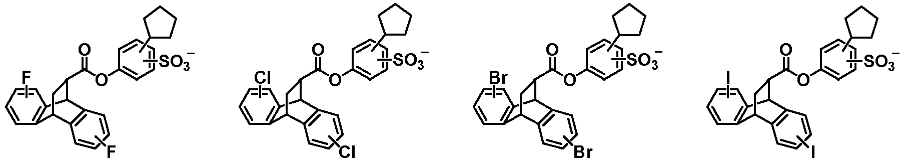

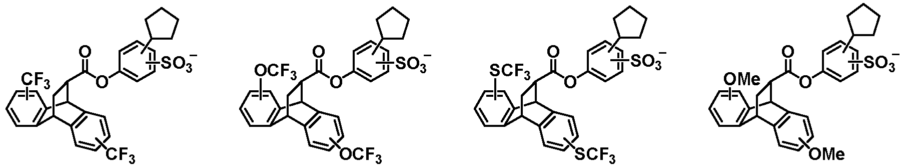

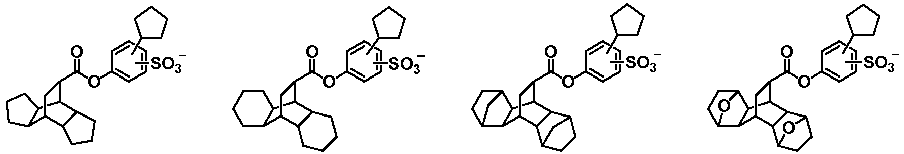

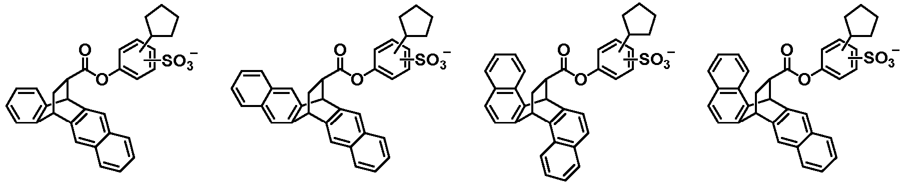

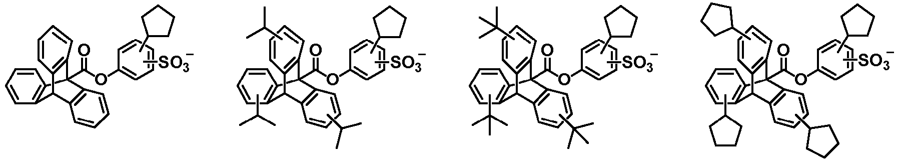

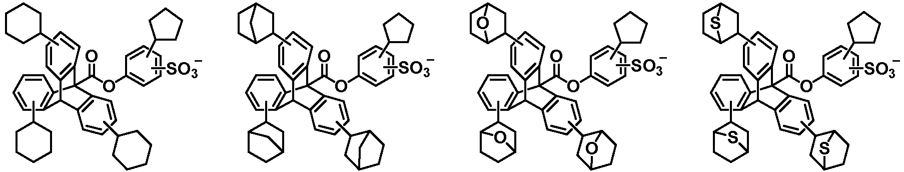

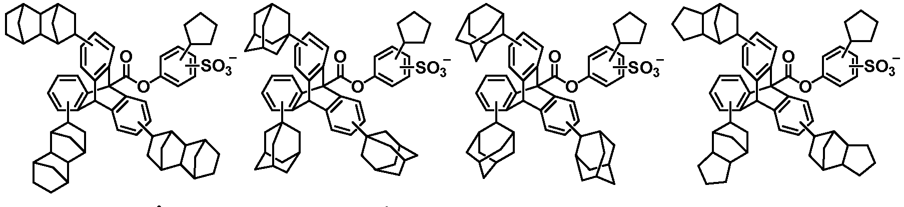

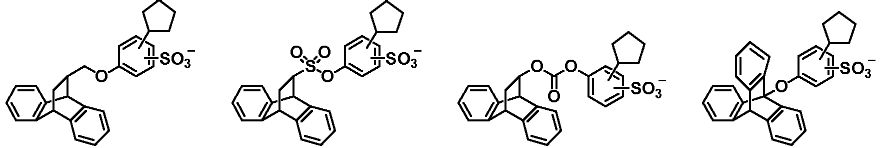

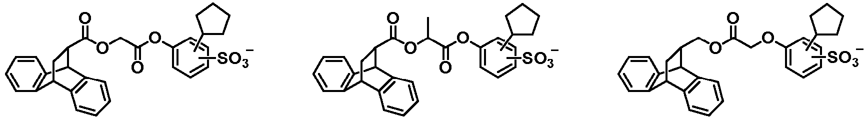

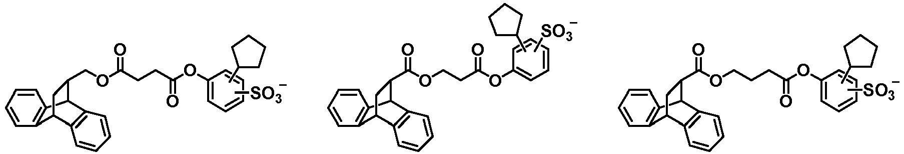

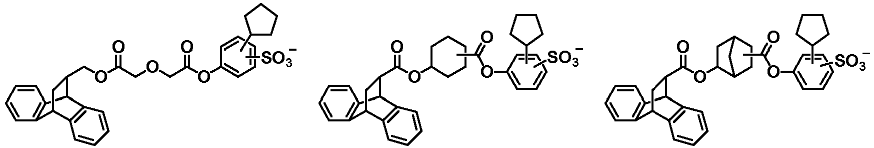

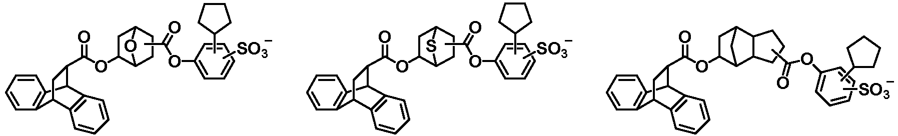

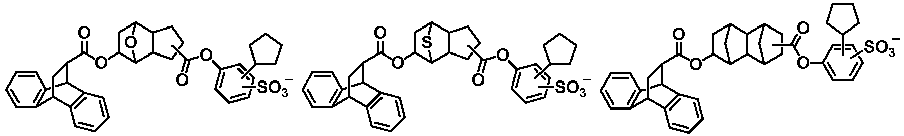

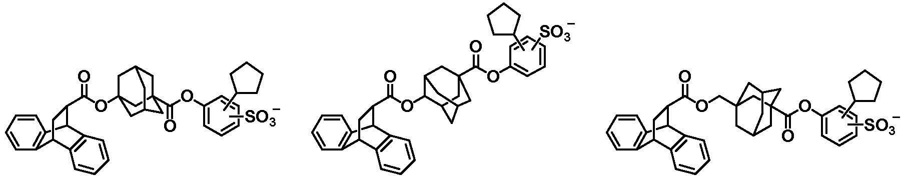

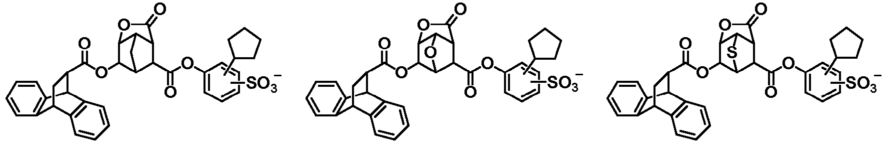

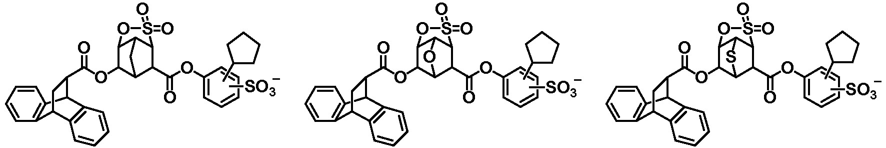

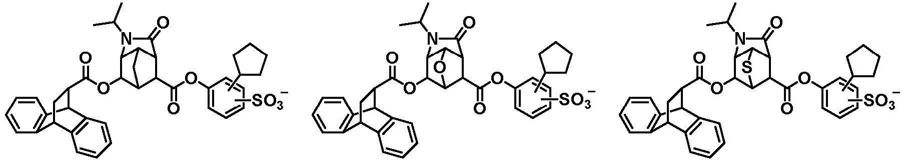

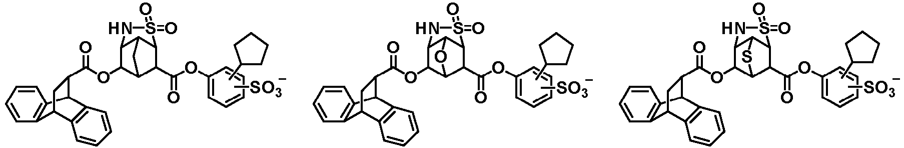

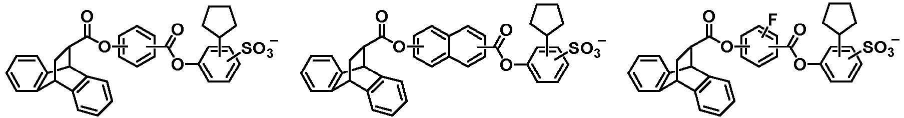

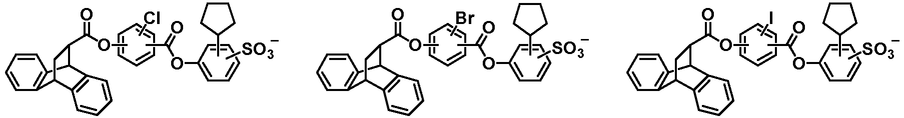

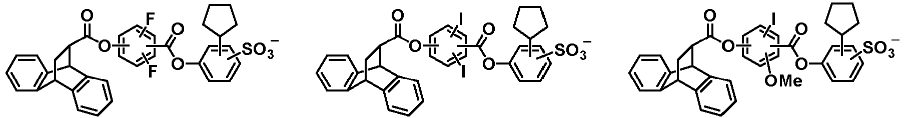

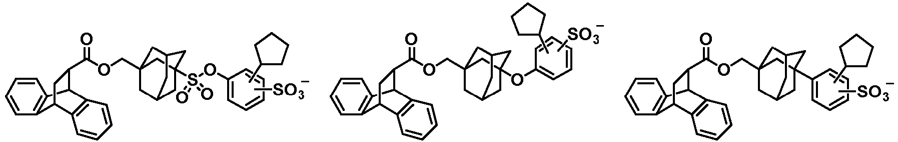

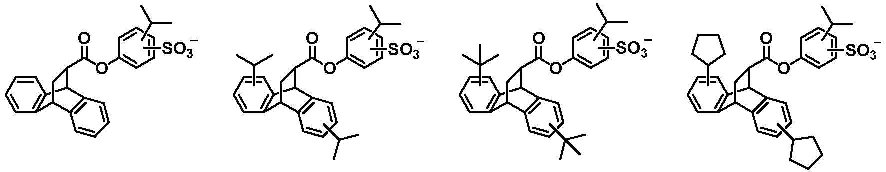

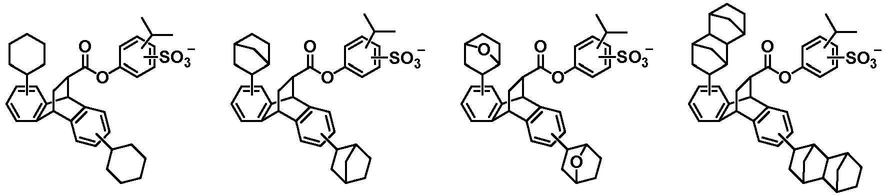

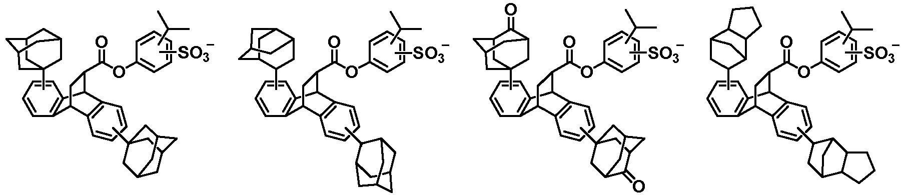

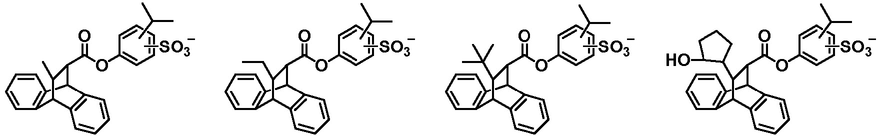

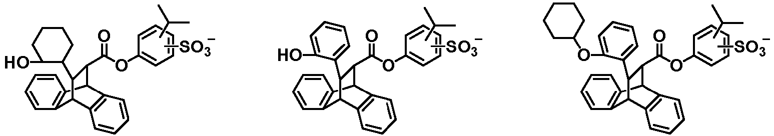

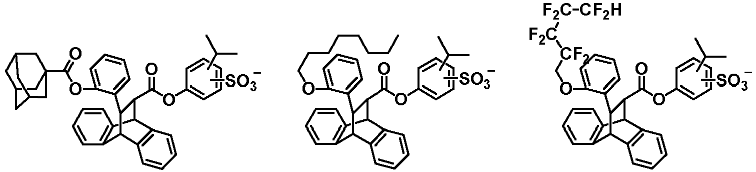

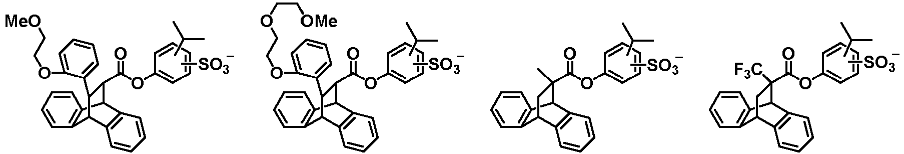

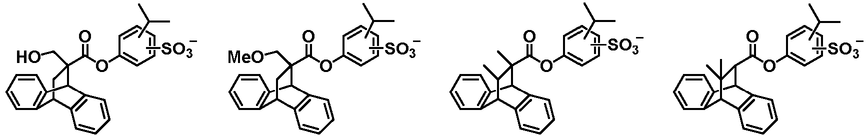

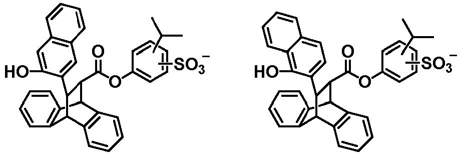

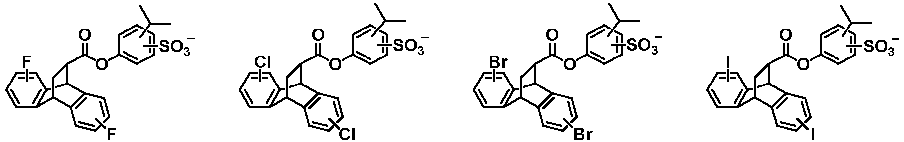

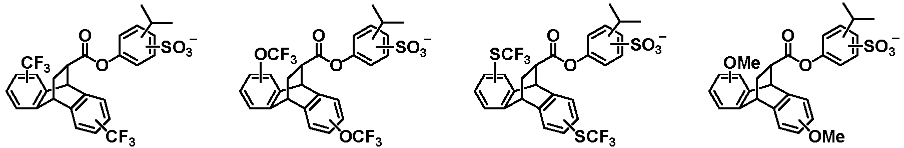

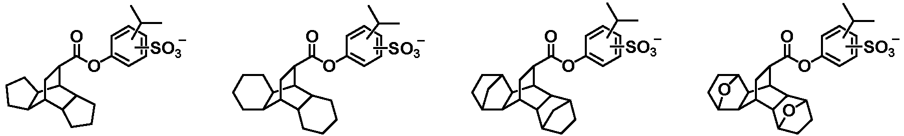

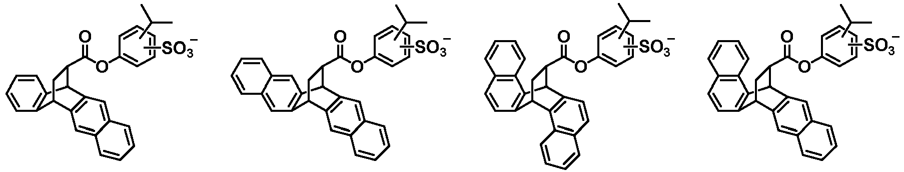

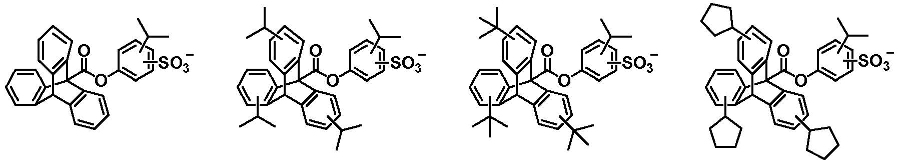

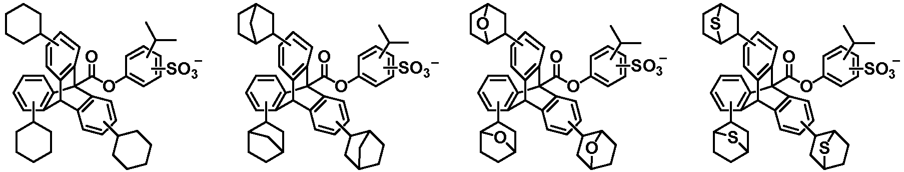

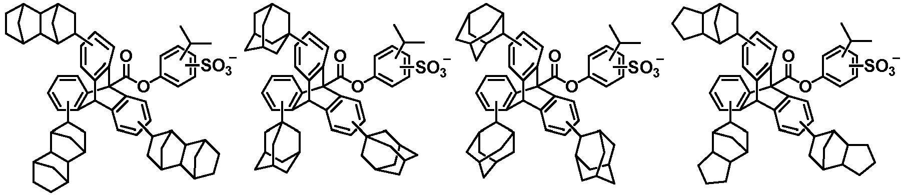

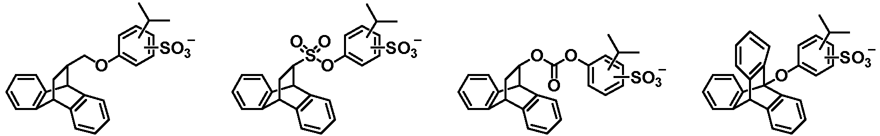

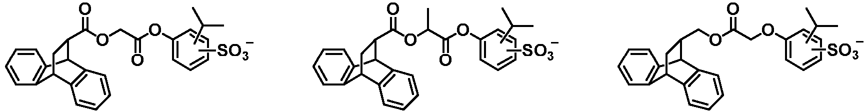

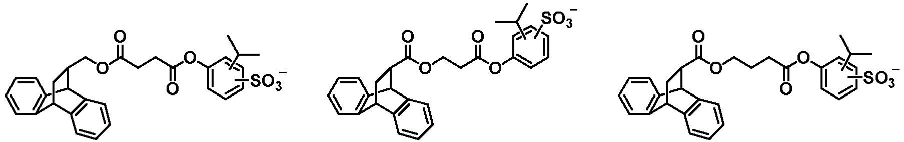

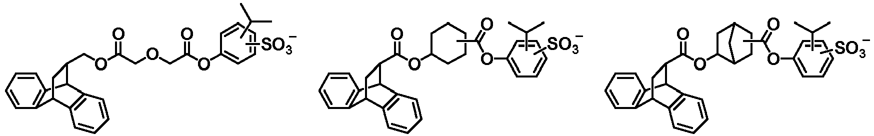

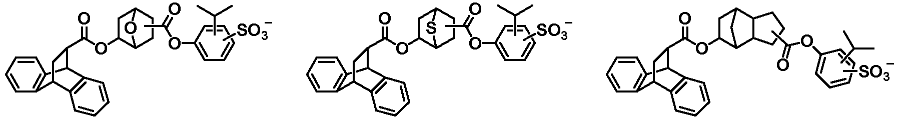

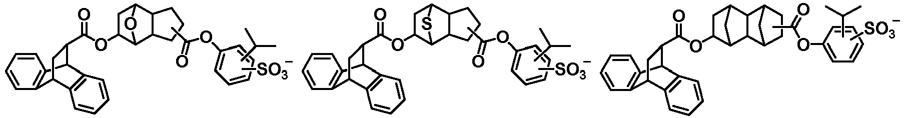

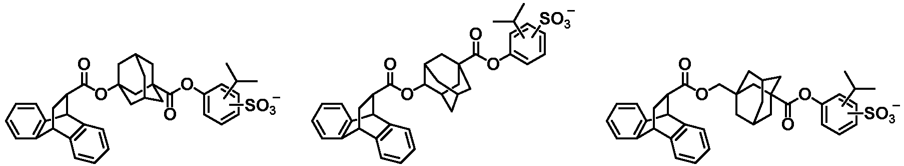

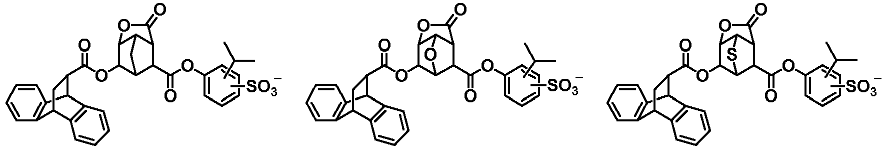

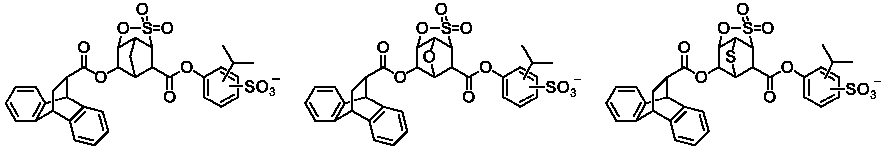

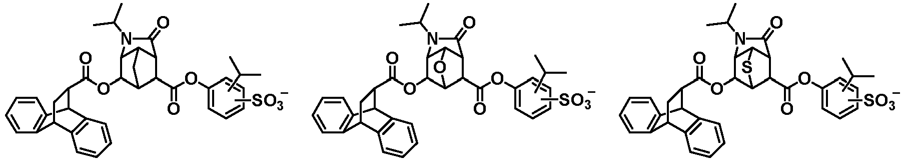

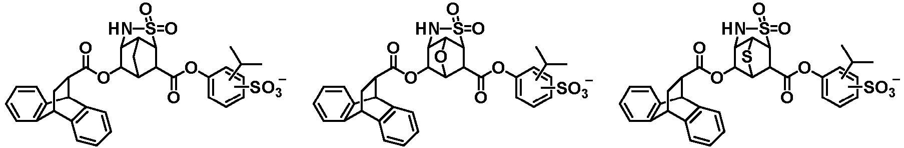

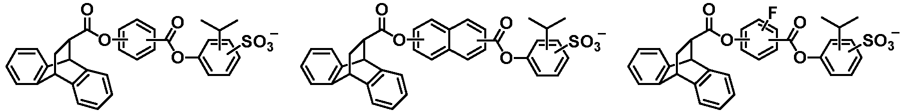

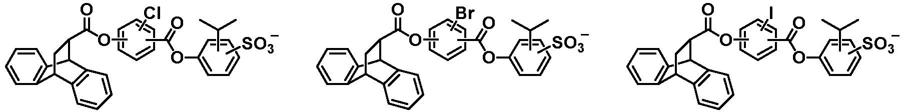

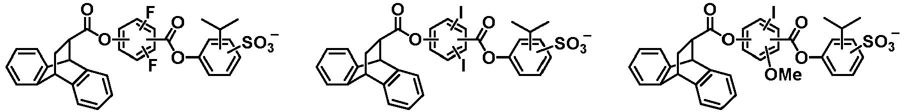

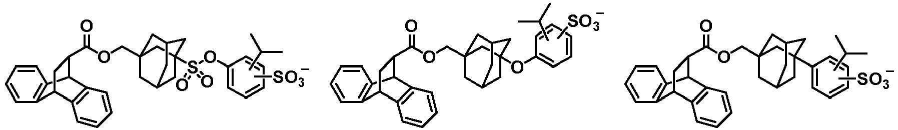

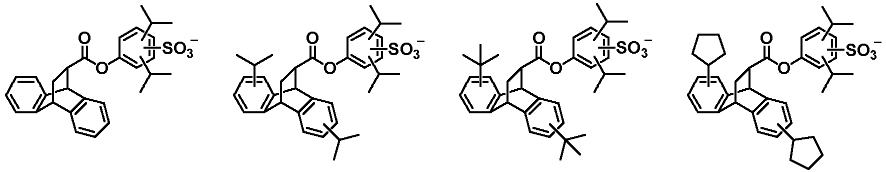

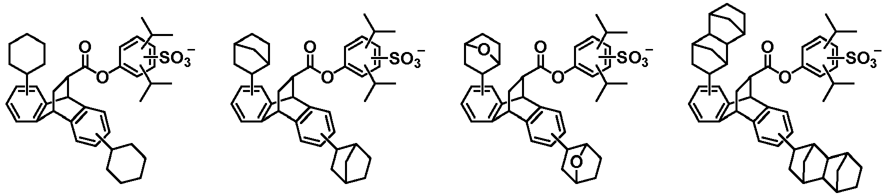

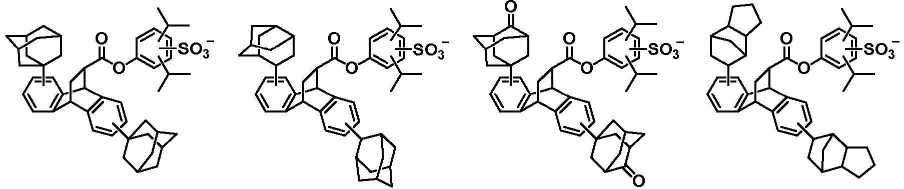

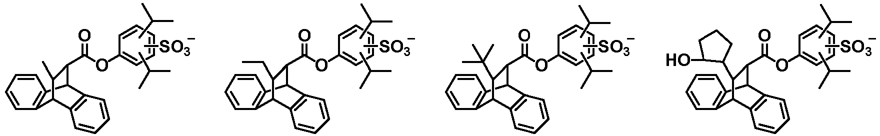

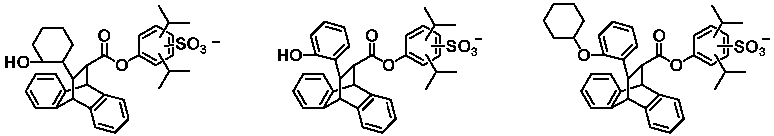

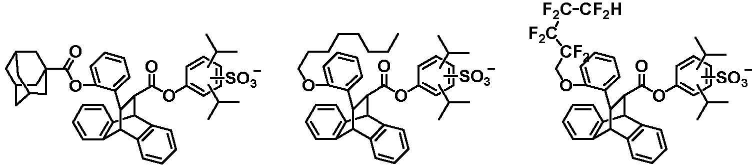

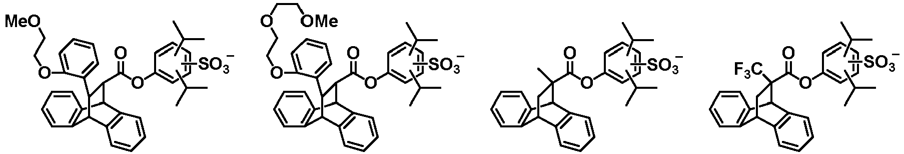

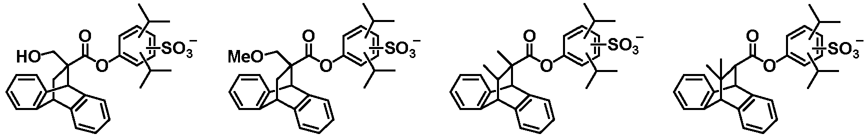

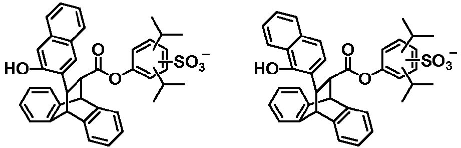

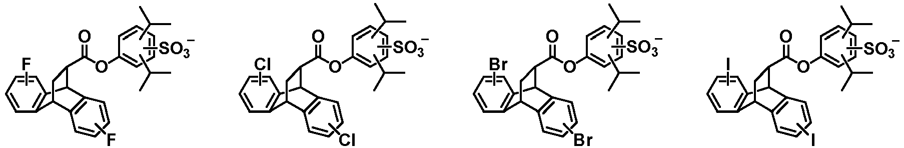

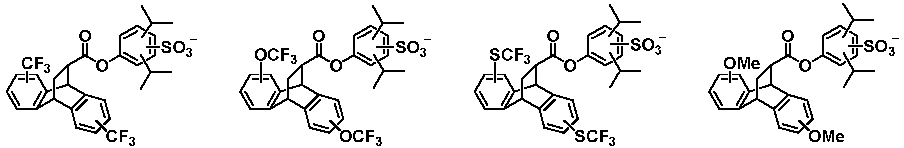

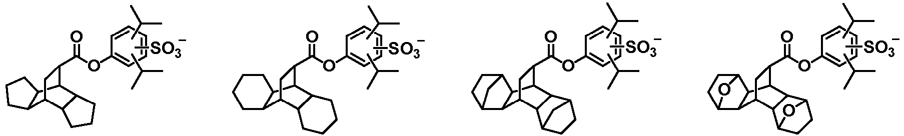

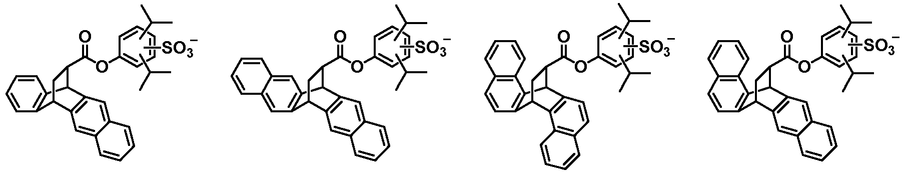

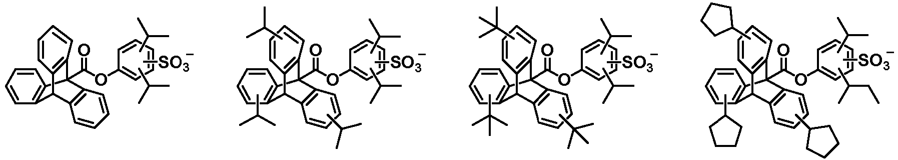

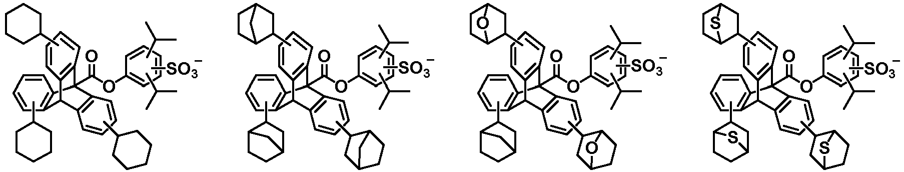

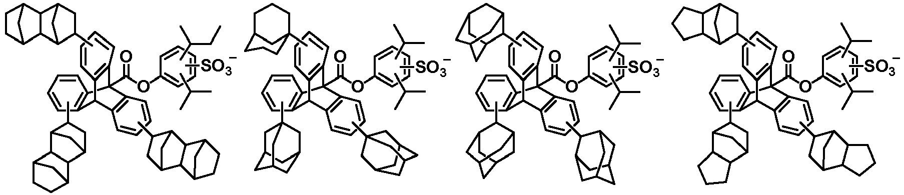

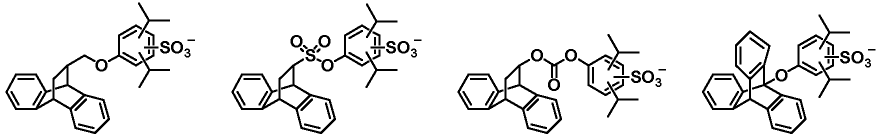

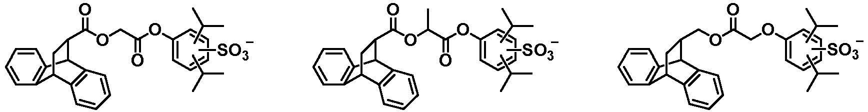

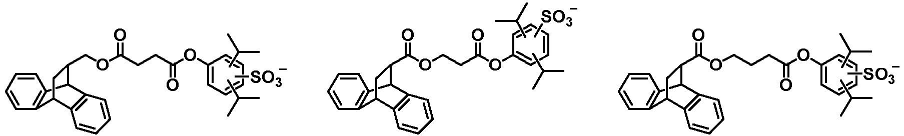

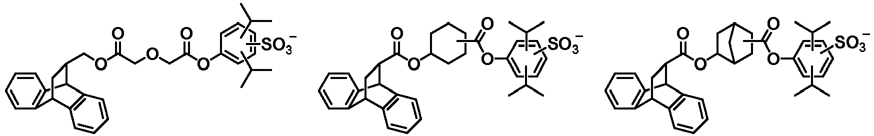

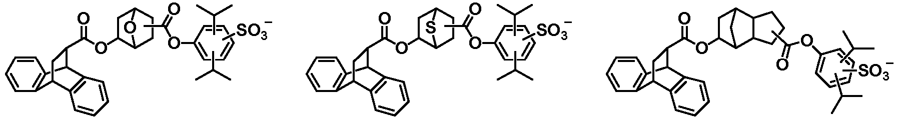

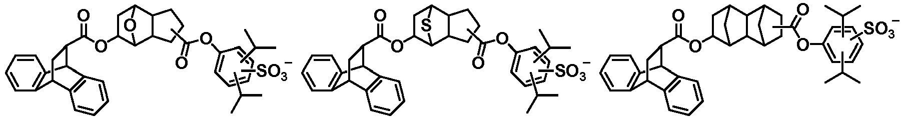

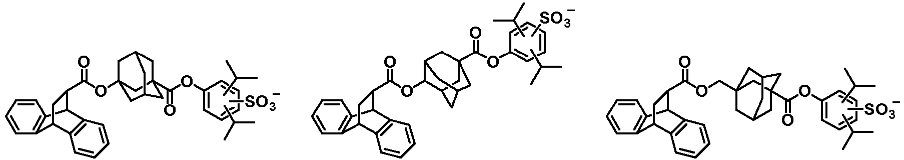

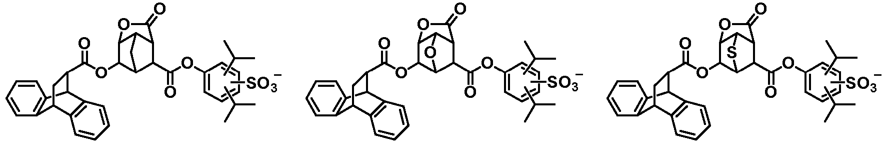

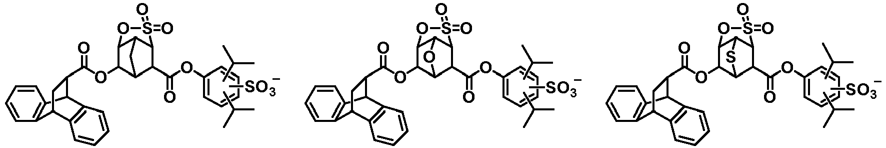

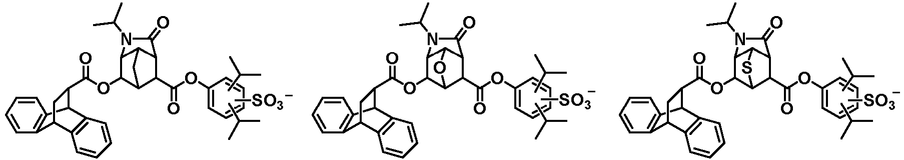

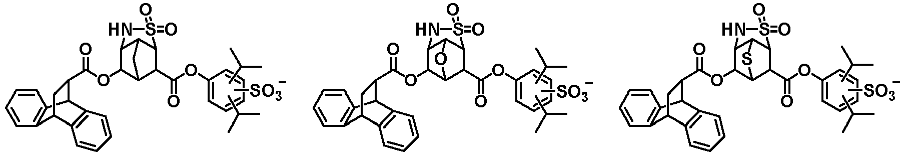

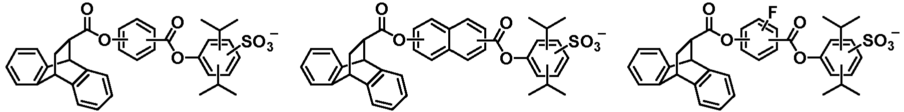

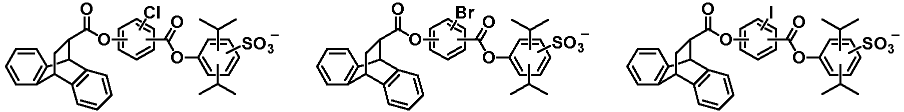

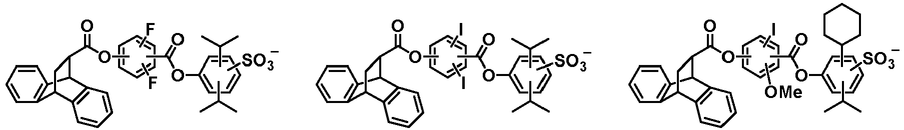

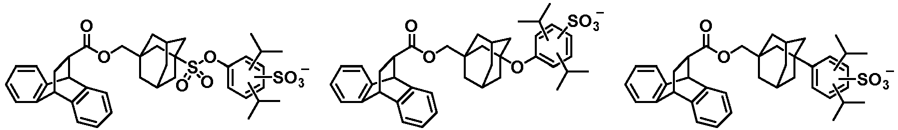

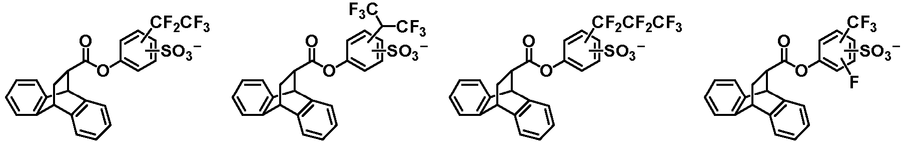

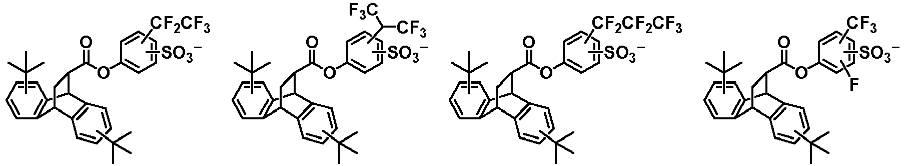

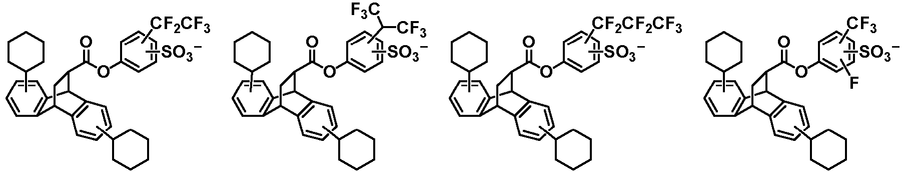

Also preferred as the PAG is a salt compound containing an anion having the formula (D1).

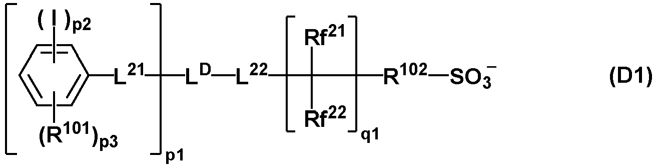

In formula (D1), p1 is 1, 2 or 3, p2 is 1, 2, 3, 4 or 5, p3 is 0, 1, 2 or 3, 1 ≤ p2+p3 ≤ 5, and q1 is 0 or 1.

In formula (D1), L²¹ is a single bond, ether bond, ester bond, sulfonate ester bond, carbonate bond or carbamate bond.

In formula (D1), L²² is an ether bond, ester bond, sulfonate ester bond, carbonate bond or carbamate bond.

In formula (D1), L^{D} is a single bond or a C₁-C₂₀ hydrocarbylene group when p1=1, and a C₁-C₂₀ (p1+1)-valent hydrocarbon group when p1=2 or 3. The hydrocarbylene group and (p1+1)-valent hydrocarbon group may contain at least one moiety selected from ether bond, carbonyl moiety, ester bond, amide bond, sultone ring, lactam ring, carbonate bond, halogen, hydroxy moiety and carboxy moiety.

The C₁-C₂₀ hydrocarbylene group L^{D} may be saturated or unsaturated and straight, branched or cyclic. Examples thereof include C₁-C₂₀ alkanediyl groups such as methanediyl, ethane-1,1-diyl, ethane-1,2-diyl, propane-1,3-diyl, butane-1,4-diyl, pentane-1,5-diyl, hexane-1,6-diyl, heptane-1,7-diyl, octane-1,8-diyl, nonane-1,9-diyl, decane-1,10-diyl, undecane-1,11-diyl, dodecane-1,12-diyl; C₃-C₂₀ cyclic saturated hydrocarbylene groups such as cyclopentanediyl, cyclohexanediyl, norbornanediyl and adamantanediyl; C₂-C₂₀ unsaturated aliphatic hydrocarbylene groups such as vinylene and propene-1,3-diyl; C₆-C₂₀ arylene groups such as phenylene and naphthylene; and combinations thereof. The C₁-C₂₀ (p1+1)-valent hydrocarbon group L^{D} may be saturated or unsaturated and straight, branched or cyclic. Examples thereof include those exemplified above for the C₁-C₂₀ hydrocarbylene group, with one or two hydrogen atoms being eliminated.

In formula (D1), Rf²¹ and Rf²² are each independently hydrogen, fluorine or trifluoromethyl, at least one being fluorine or trifluoromethyl.

In formula (D1), R¹⁰¹ is hydroxy, carboxy, a C₁-C₆ saturated hydrocarbyl group, C₁-C₆ saturated hydrocarbyloxy group, C₂-C₆ saturated hydrocarbylcarbonyloxy group, fluorine, chlorine, bromine, -N(R^{101A})(R^{101B}), -N(R^{101C})-C(=O)-R^{101D} or -N(R^{101C})-C(=O)-O-R^{101D}. R^{101A} and R^{101B} are each independently hydrogen or a C₁-C₆ saturated hydrocarbyl group. R^{101C} is hydrogen or a C₁-C₆ saturated hydrocarbyl group. R^{101D} is a C₁-C₆ saturated hydrocarbyl group or C₂-C₈ unsaturated aliphatic hydrocarbyl group.

The C₁-C₆ saturated hydrocarbyl group represented by R¹⁰¹, R^{101A}, R^{101B} and R^{101C} may be straight, branched or cyclic. Examples thereof include C₁-C₆ alkyl groups such as methyl, ethyl, n-propyl, isopropyl, n-butyl, isobutyl, sec-butyl, tert-butyl, n-pentyl, and n-hexyl; and C₃-C₆ cyclic saturated hydrocarbyl groups such as cyclopropyl, cyclobutyl, cyclopentyl, and cyclohexyl. Examples of the saturated hydrocarbyl moiety in the C₁-C₆ saturated hydrocarbyloxy group represented by R¹⁰¹ are as exemplified above for the saturated hydrocarbyl group. Examples of the saturated hydrocarbyl moiety in the C₂-C₆ saturated hydrocarbylcarbonyloxy group represented by R¹⁰¹ are as exemplified above for the C₁-C₆ saturated hydrocarbyl group, but of 1 to 5 carbon atoms.

The C₂-C₈ unsaturated aliphatic hydrocarbyl group represented by R^{101D} may be straight, branched or cyclic and examples thereof include C₂-C₈ alkenyl groups such as vinyl, propenyl, butenyl, and hexenyl; C₂-C₈ alkynyl groups such as ethynyl, propynyl, and butynyl; and C₃-C₈ cyclic unsaturated aliphatic hydrocarbyl groups such as cyclohexenyl and norbornenyl.

In formula (D1), R¹⁰² is a C₁-C₂₀ saturated hydrocarbylene group or C₆-C₂₀ arylene group. Some or all of the hydrogen atoms in the saturated hydrocarbylene group may be substituted by halogen other than fluorine. Some or all of the hydrogen atoms in the arylene group may be substituted by a substituent selected from C₁-C₂₀ saturated hydrocarbyl groups, C₁-C₂₀ saturated hydrocarbyloxy groups, C₆-C₂₀ aryl groups, halogen, and hydroxy.

The C₁-C₂₀ hydrocarbylene group represented by R¹⁰² may be saturated or unsaturated and straight, branched or cyclic. Examples thereof are as exemplified above for the C₁-C₂₀ hydrocarbylene group L^{B}.

Examples of the C₆-C₂₀ arylene group represented by R¹⁰² include phenylene, naphthylene, phenanthrenediyl, and anthracenediyl. The C₁-C₂₀ saturated hydrocarbyl moiety and hydrocarbyl moiety in the C₁-C₂₀ hydrocarbyloxy moiety, which are substituents on the arylene group, may be straight, branched or cyclic and examples thereof include C₁-C₂₀ alkyl groups such as methyl, ethyl, n-propyl, isopropyl, n-butyl, isobutyl, sec-butyl, tert-butyl, n-pentyl, n-hexyl, n-octyl, n-nonyl, n-decyl, undecyl, dodecyl, tridecyl, tetradecyl, pentadecyl, heptadecyl, octadecyl, nonadecyl, and icosyl; and C₃-C₂₀ cyclic saturated hydrocarbyl groups such as cyclopropyl, cyclopentyl, cyclohexyl, cyclopropylmethyl, 4-methylcyclohexyl, cyclohexylmethyl, norbornyl and adamantyl. Examples of the C₆-C₁₄ arylene moiety which is a substituent on the arylene group include phenylene, naphthylene, phenanthrenediyl and anthracenediyl.

More preferably, the anion has the formula (D2).

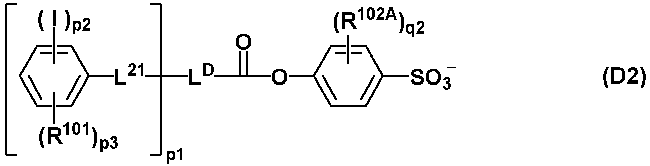

In formula (D2), p1, p2, p3, L²¹, L^{D}, and R¹⁰¹ are as defined above. The subscript q2 is 1, 2, 3 or 4. R^{102A} is a C₁-C₂₀ saturated hydrocarbyl group, C₁-C₂₀ saturated hydrocarbyloxy group, C₆-C₁₄ aryl group, halogen or hydroxy group. When q2 is 2, 3 or 4, a plurality of R^{102A} may be identical or different.

Examples of the anion having formula (D1) are shown below, but not limited thereto.

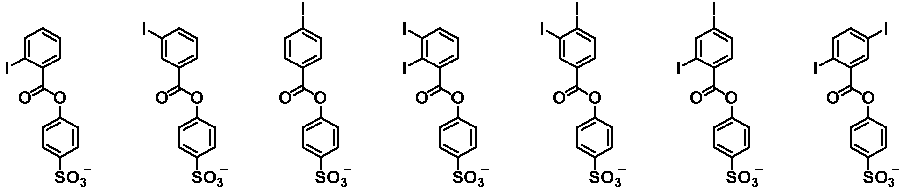

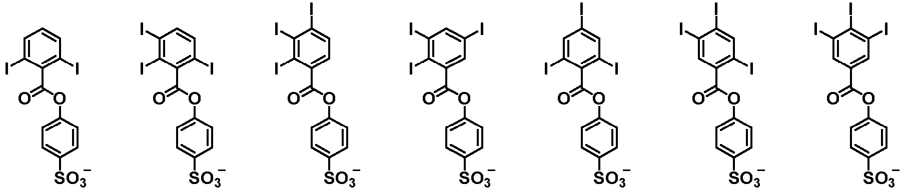

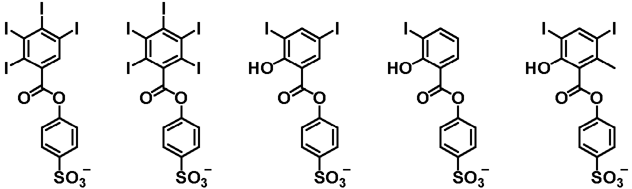

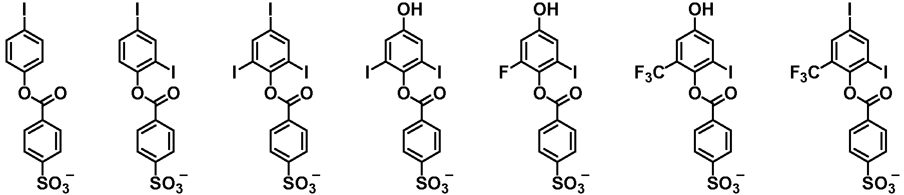

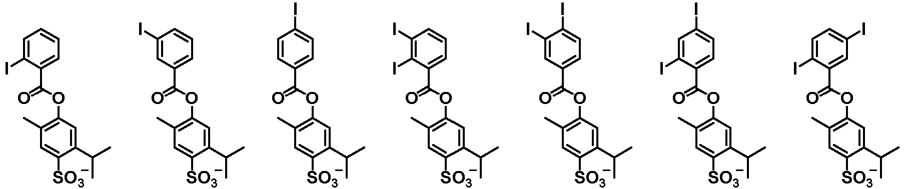

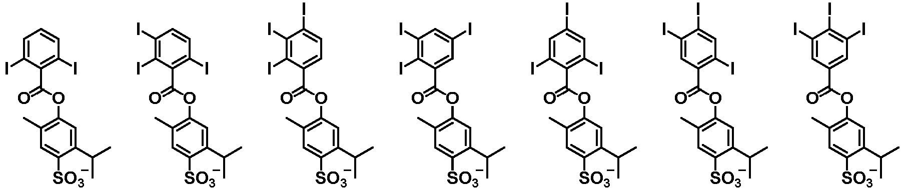

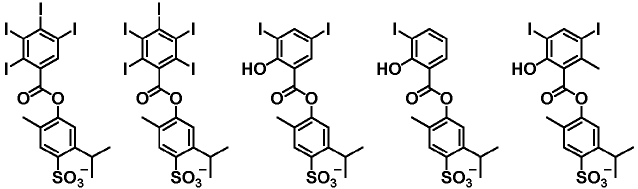

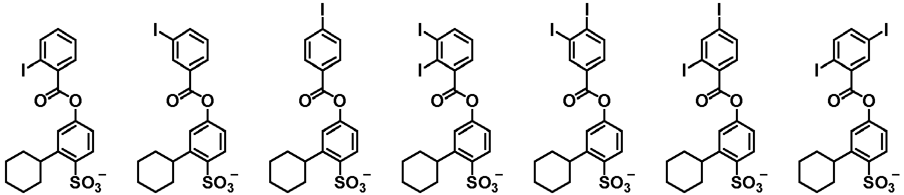

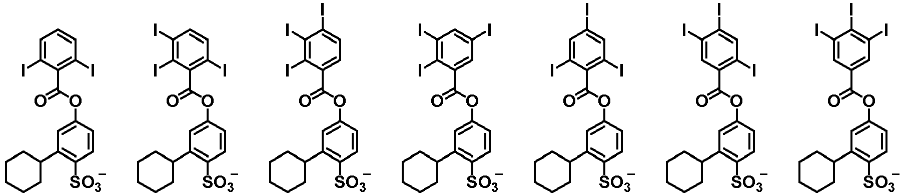

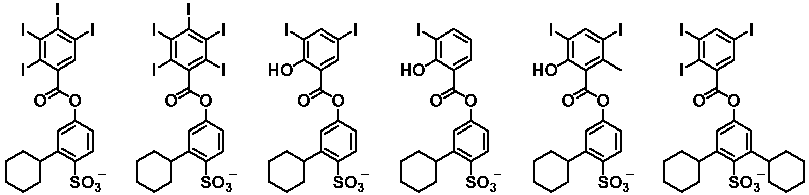

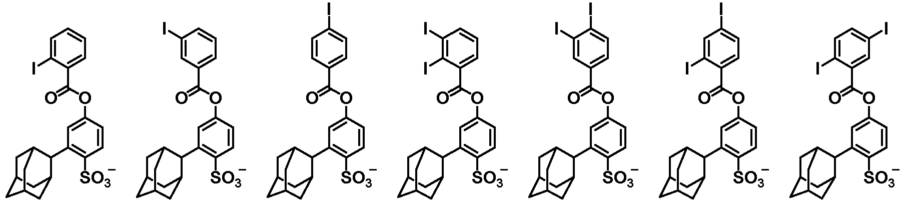

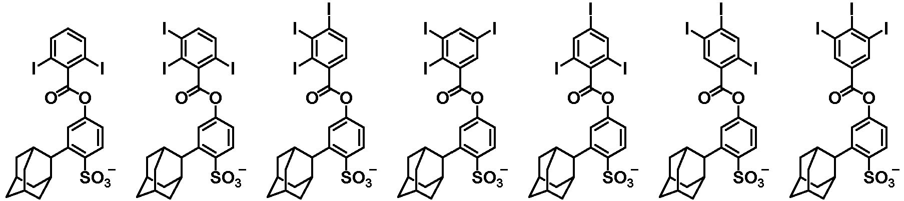

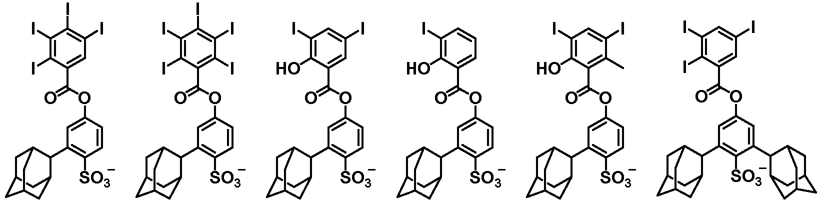

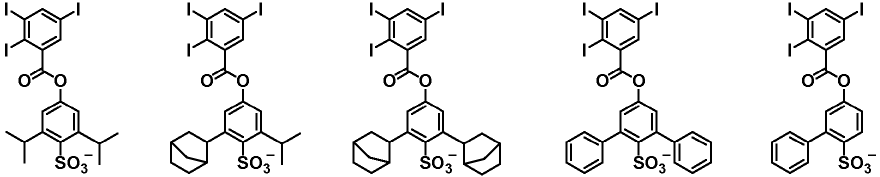

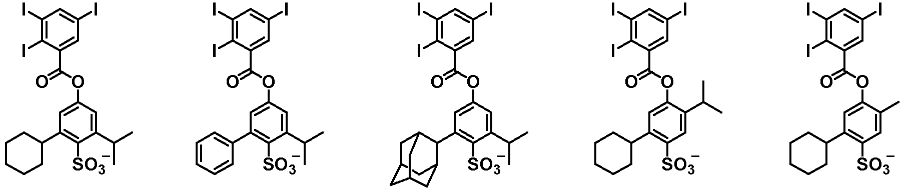

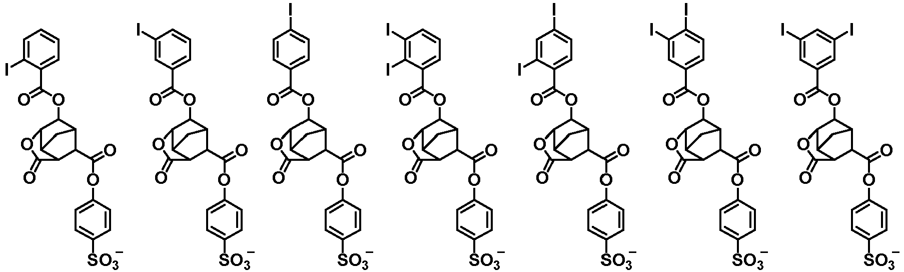

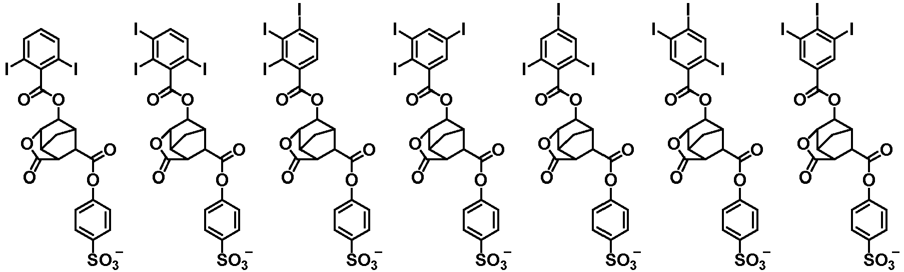

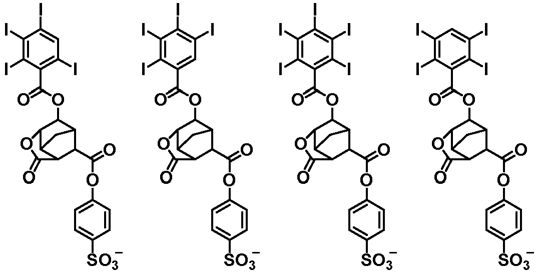

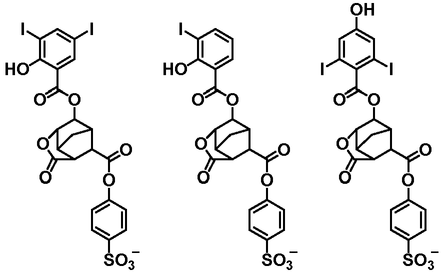

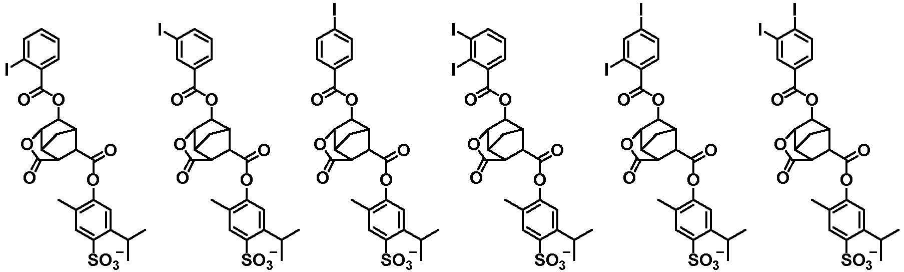

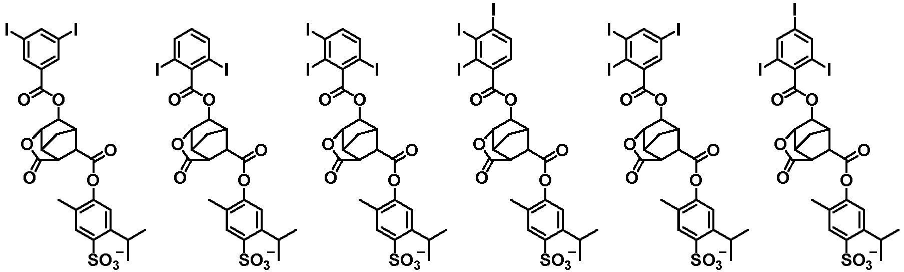

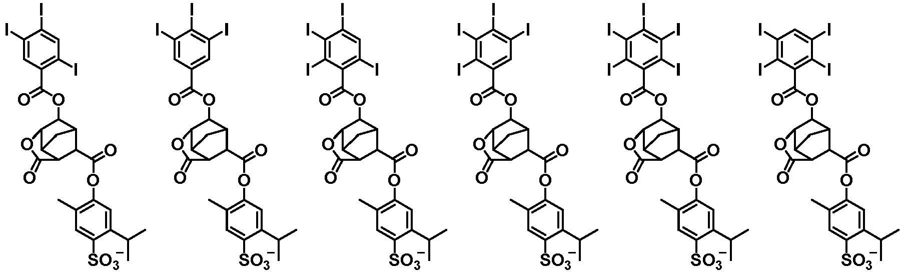

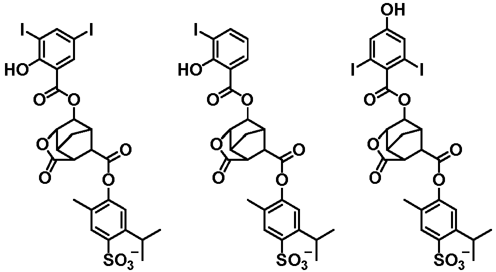

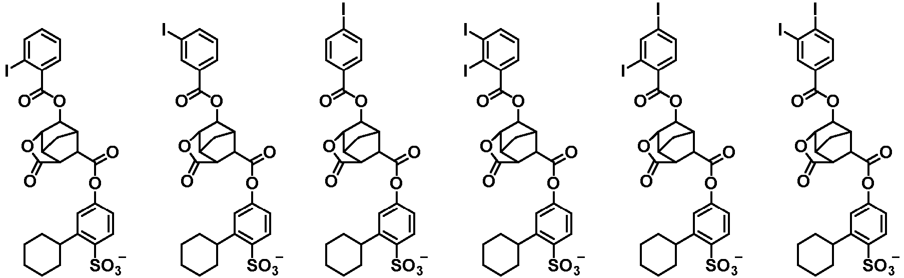

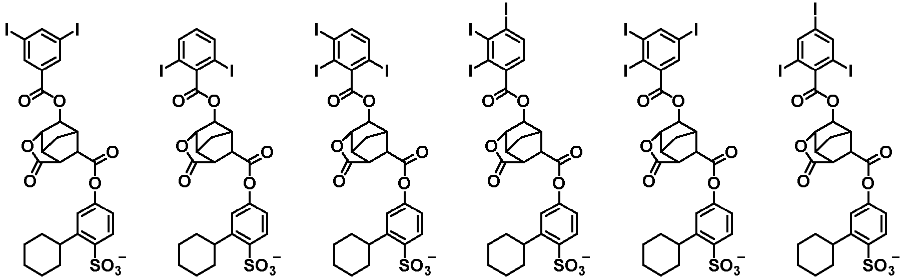

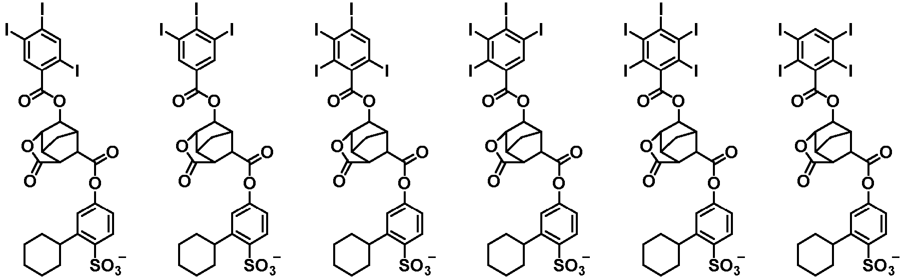

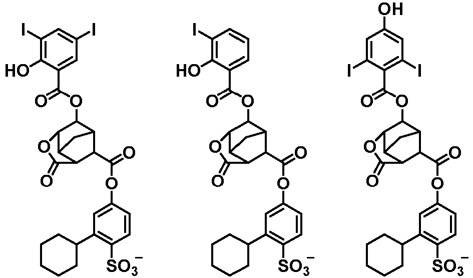

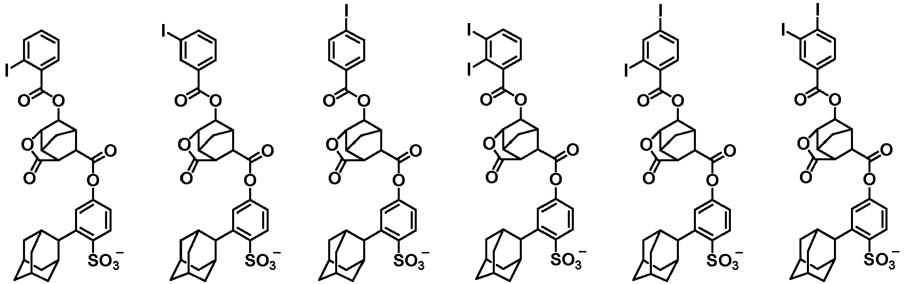

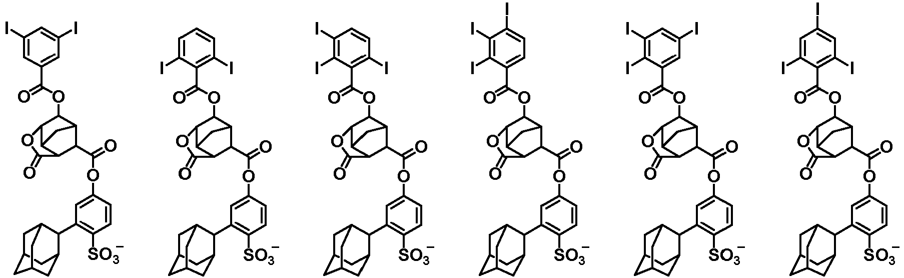

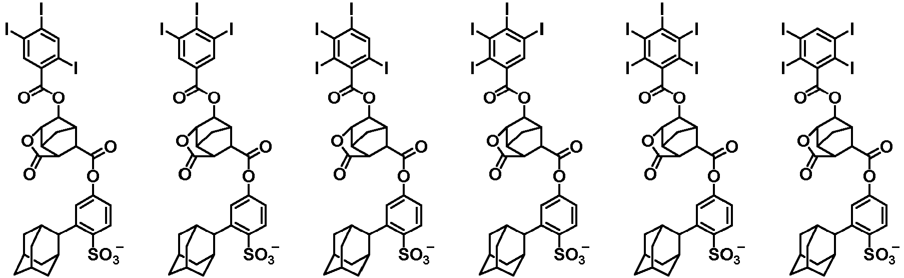

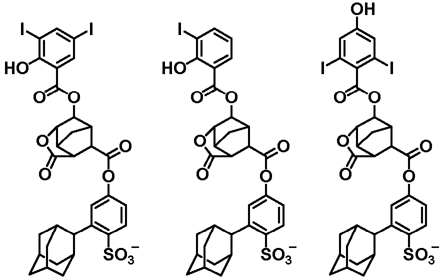

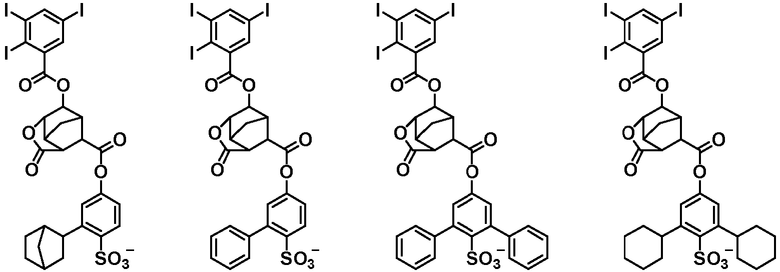

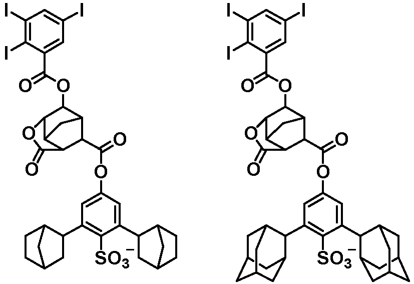

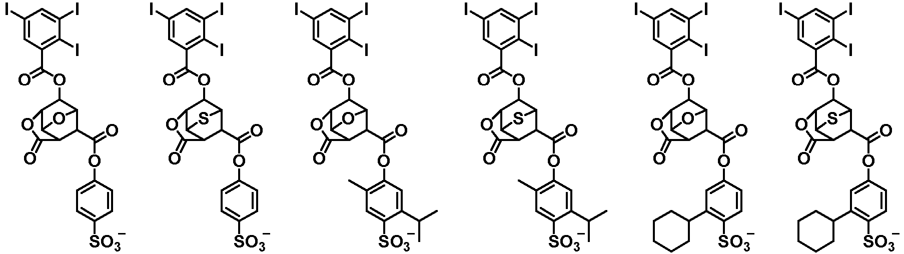

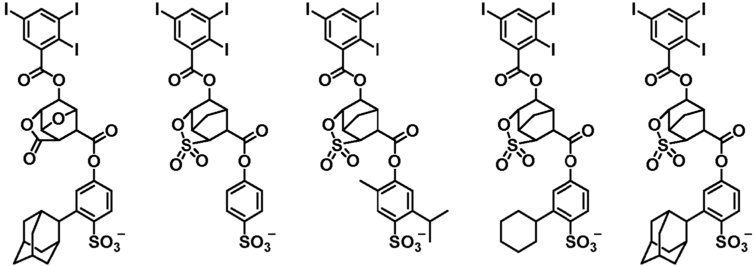

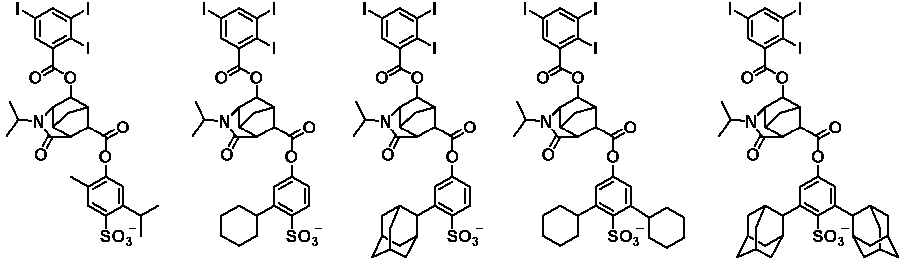

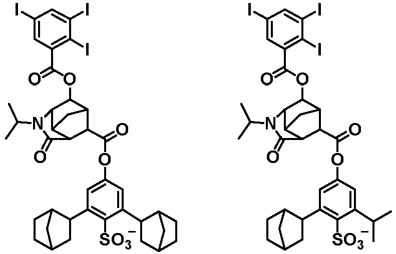

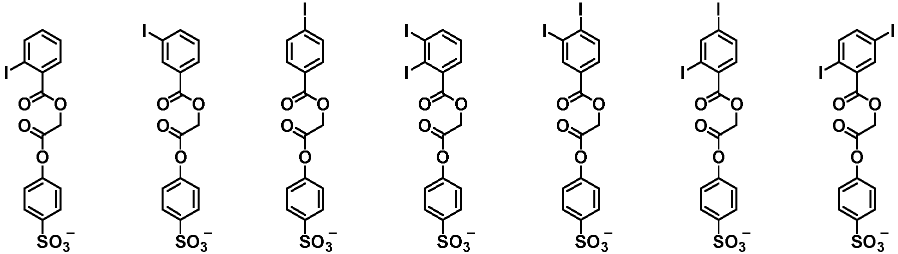

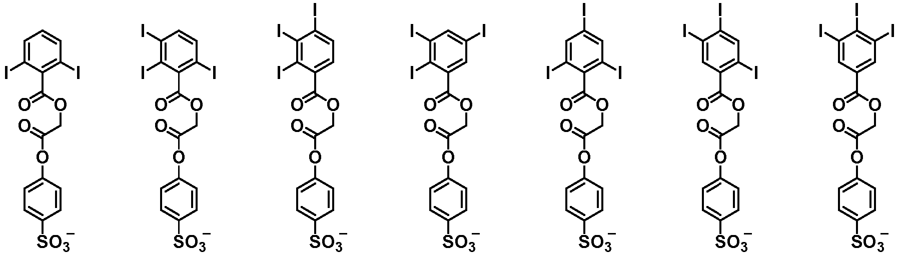

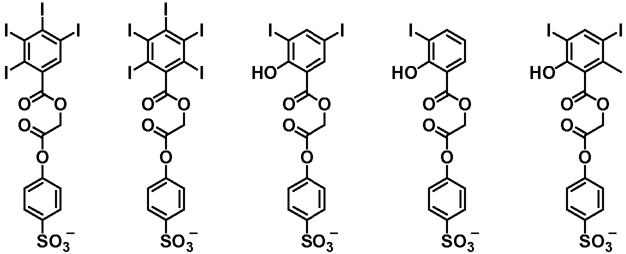

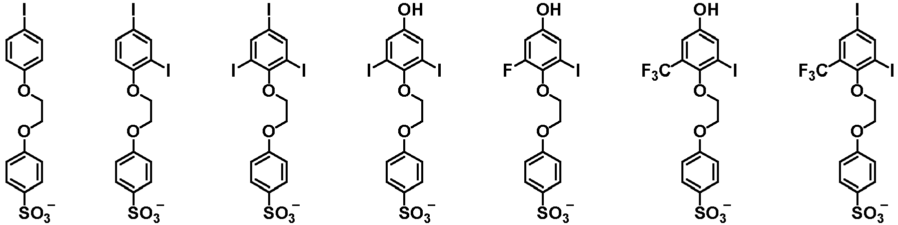

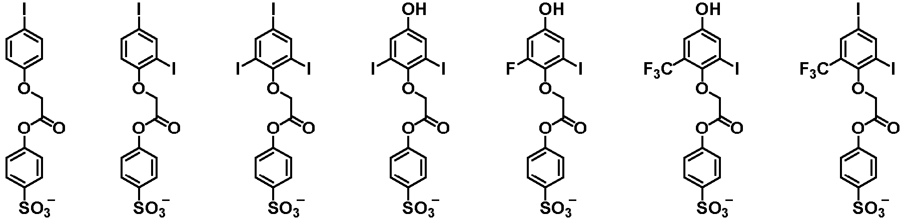

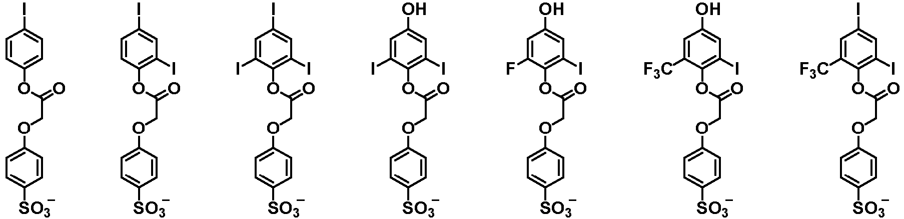

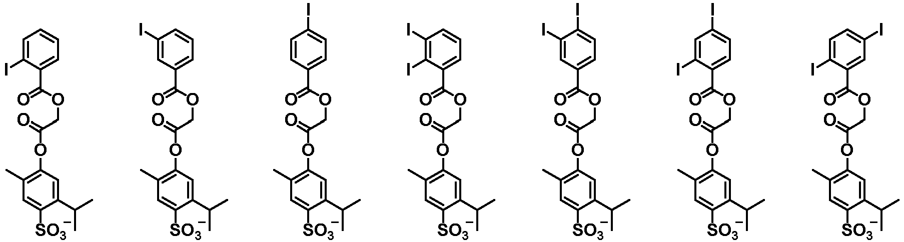

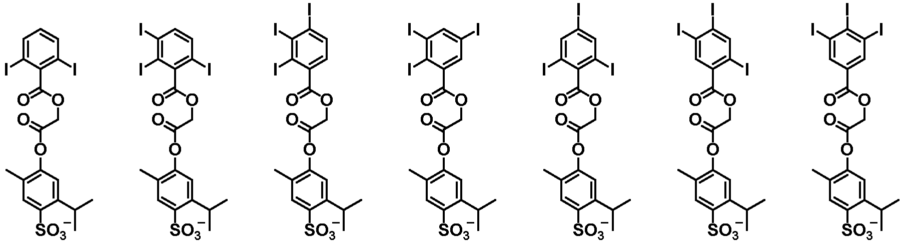

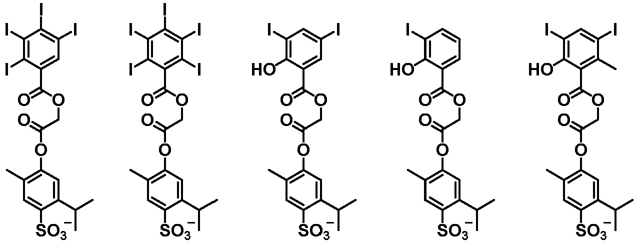

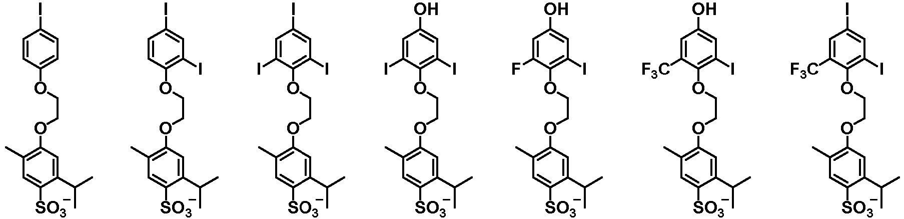

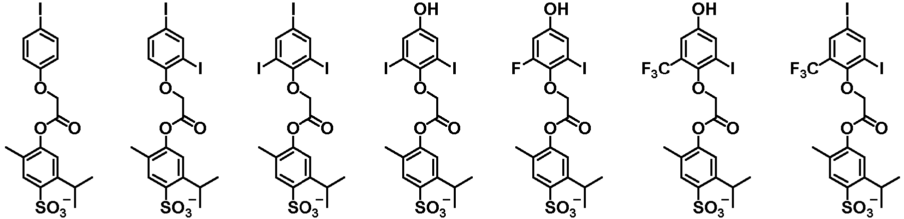

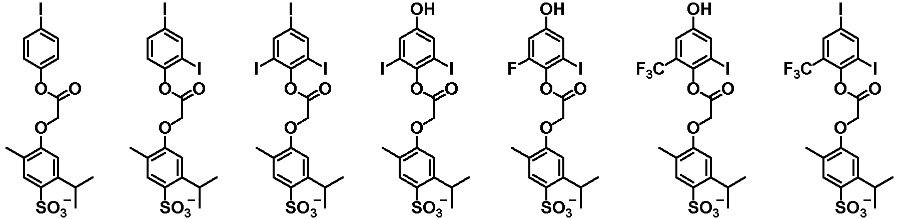

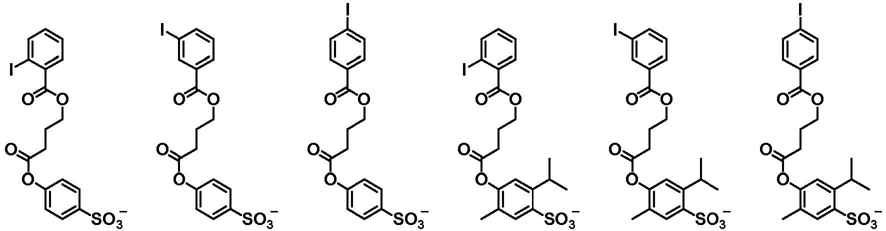

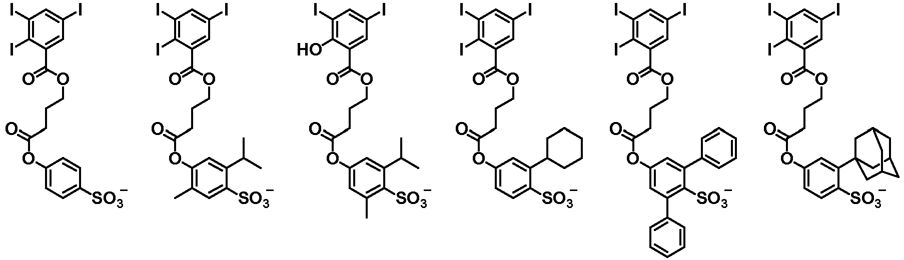

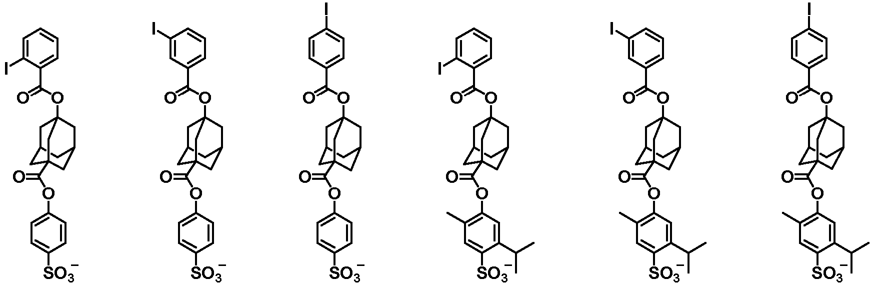

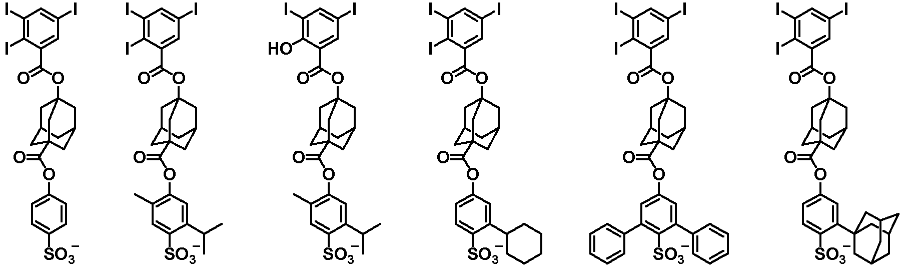

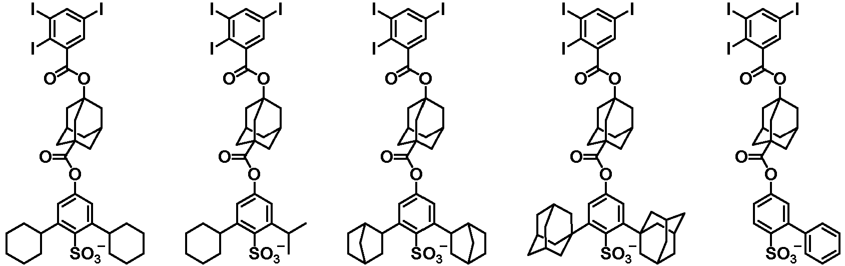

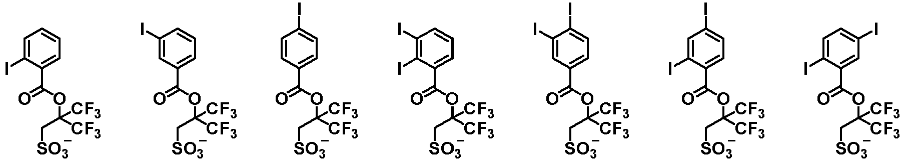

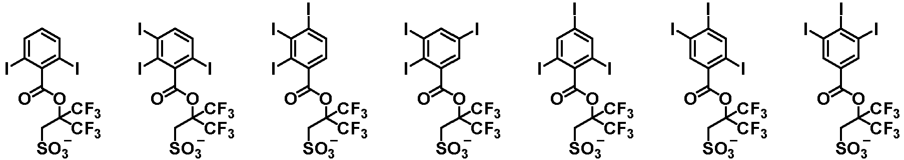

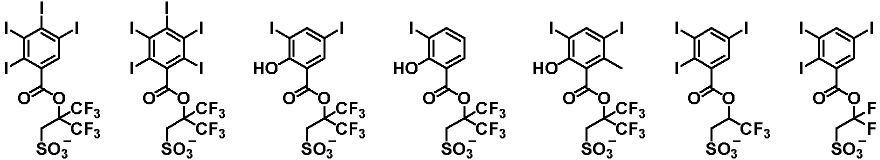

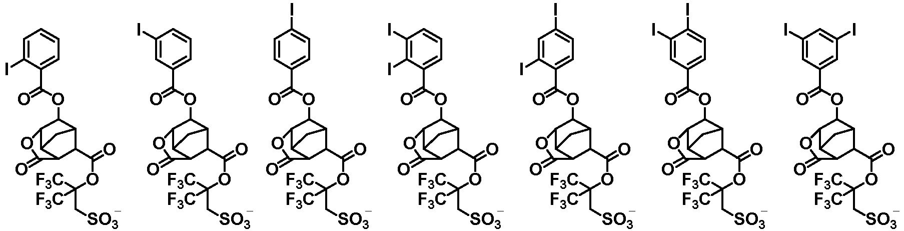

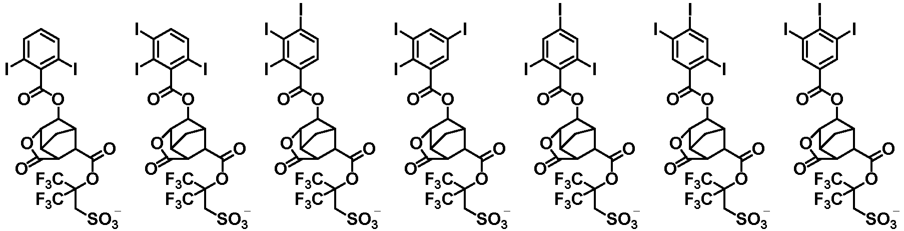

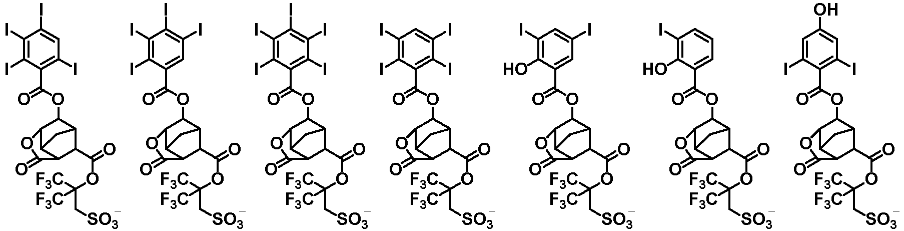

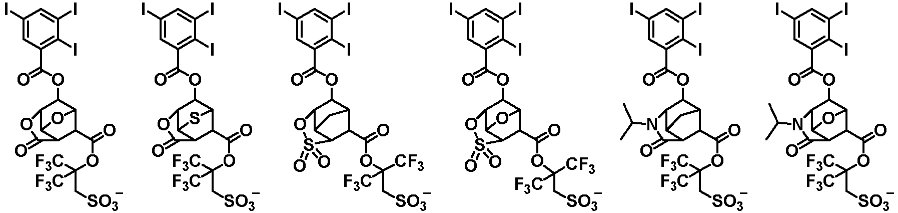

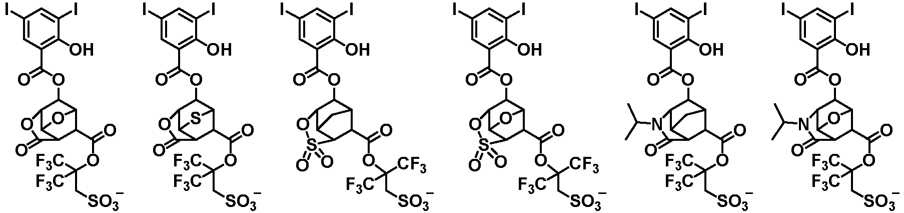

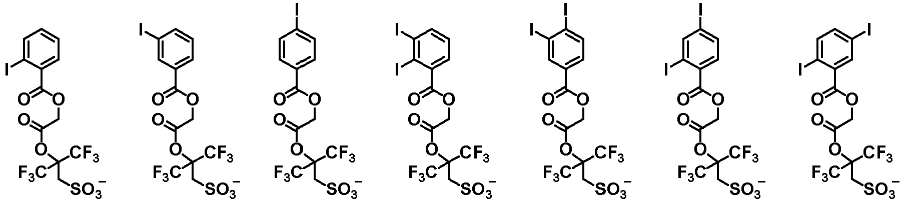

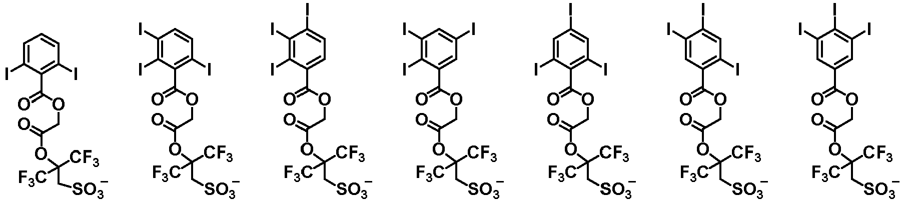

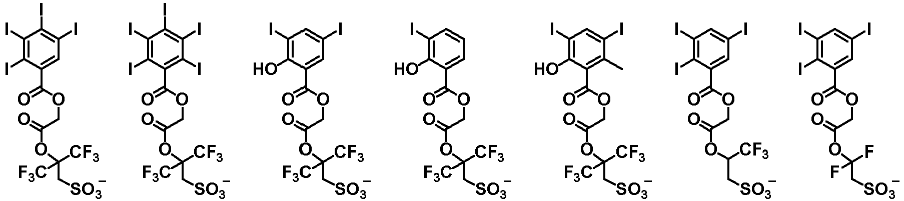

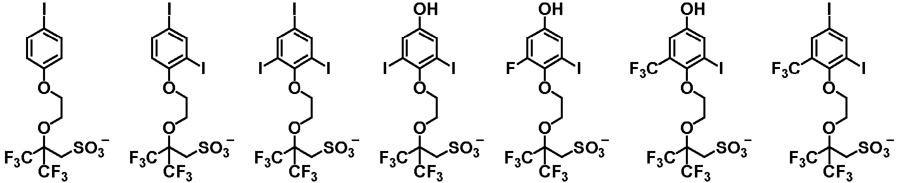

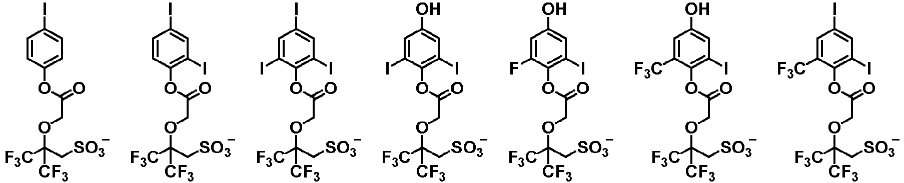

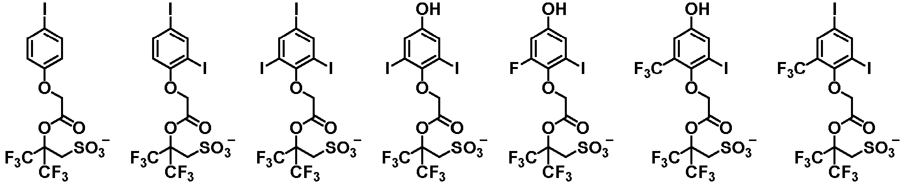

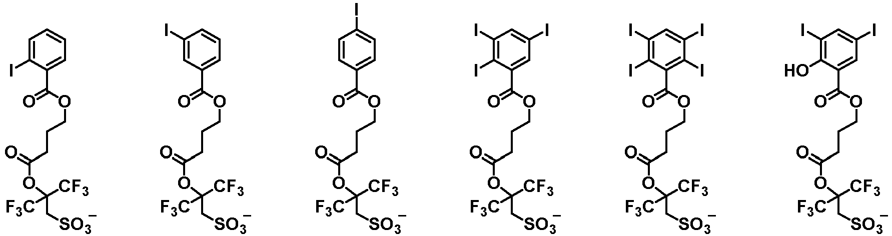

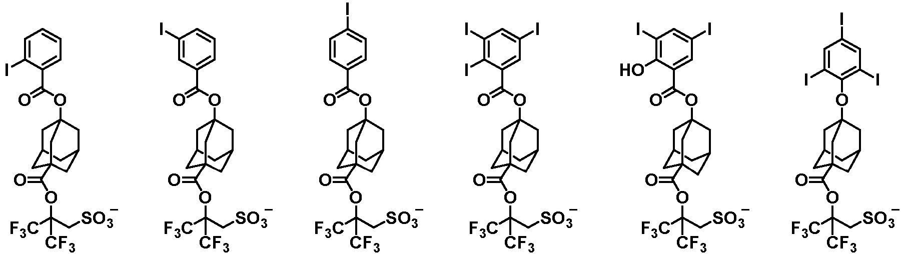

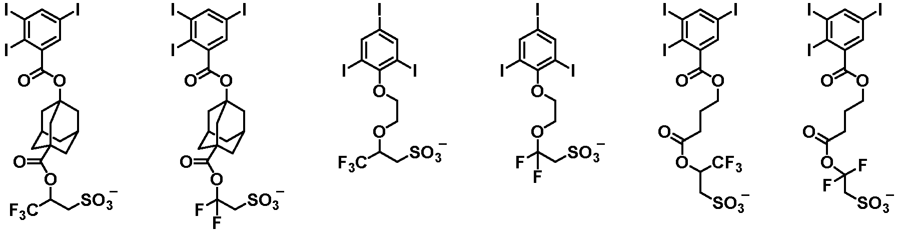

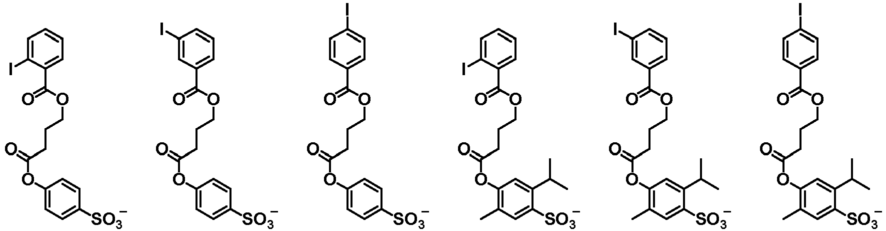

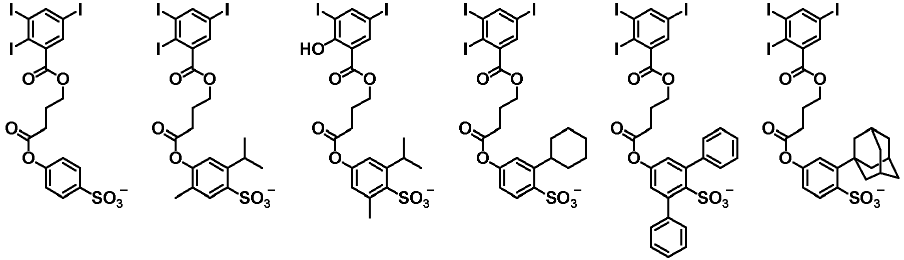

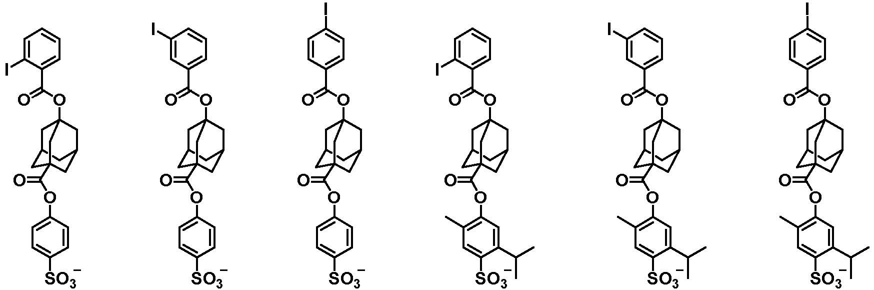

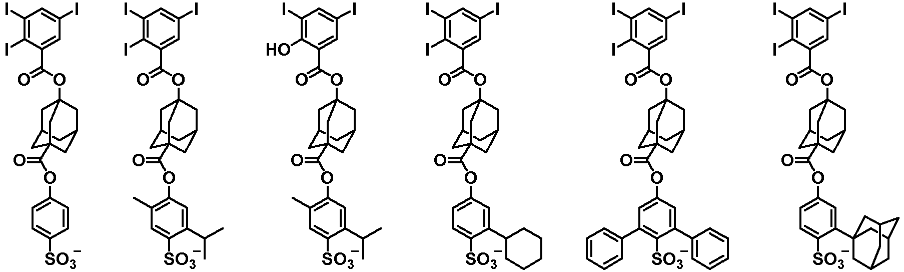

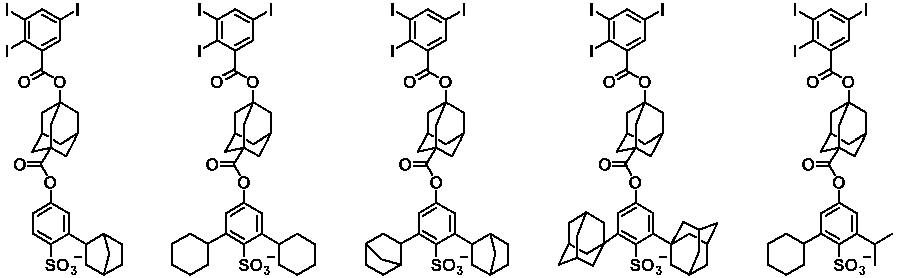

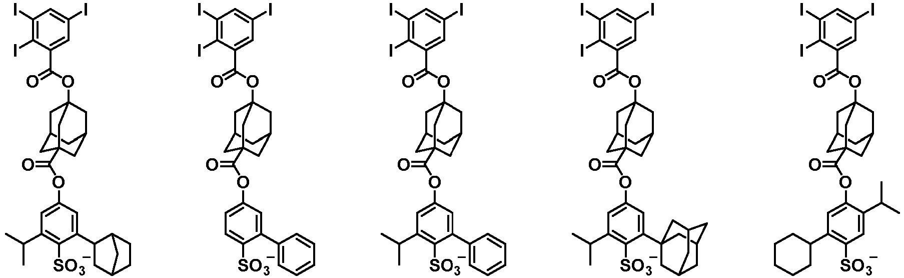

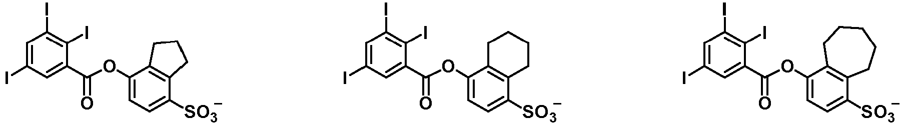

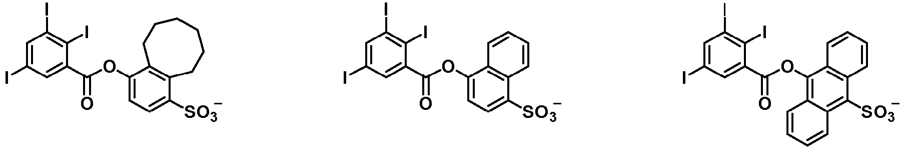

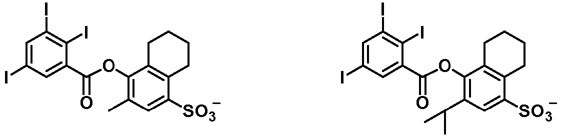

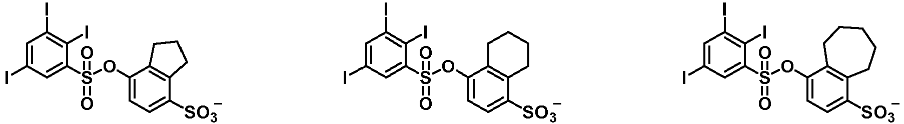

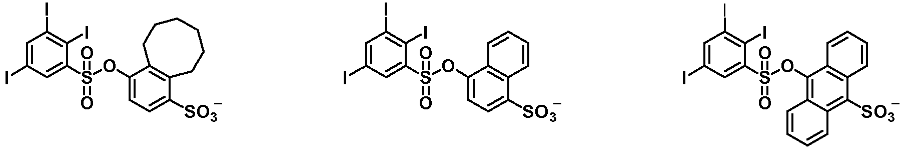

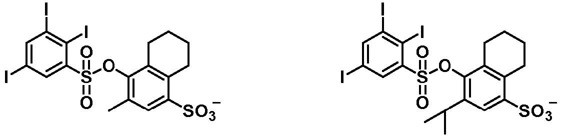

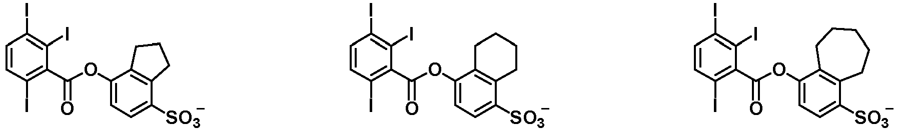

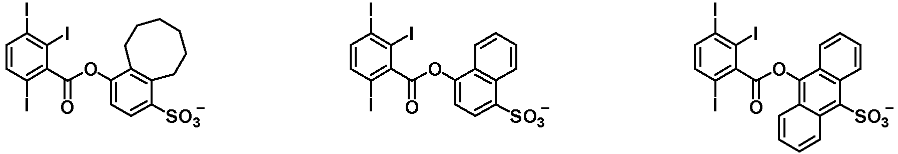

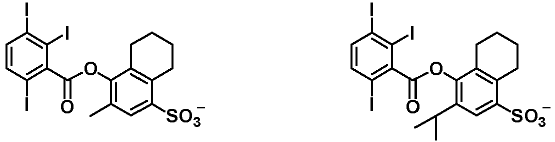

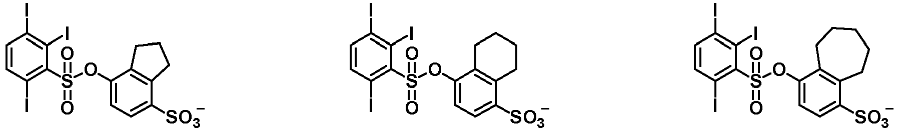

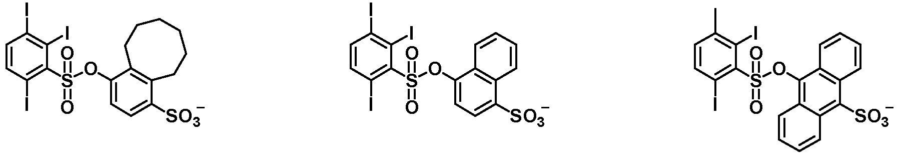

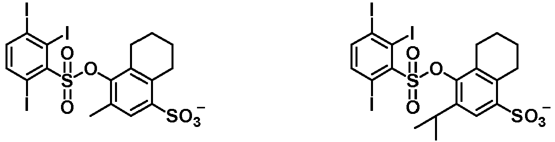

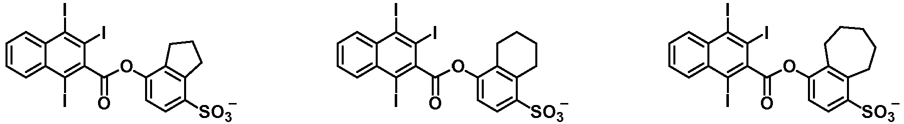

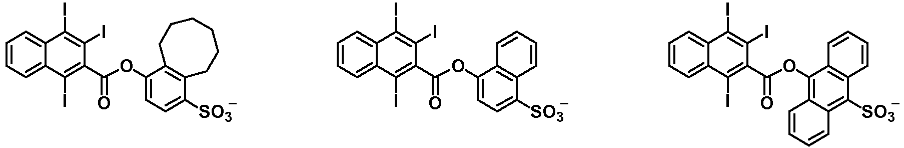

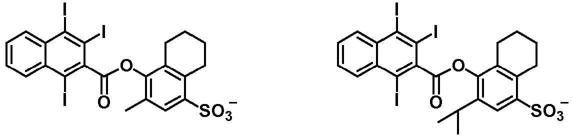

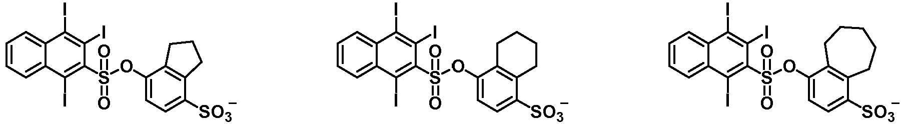

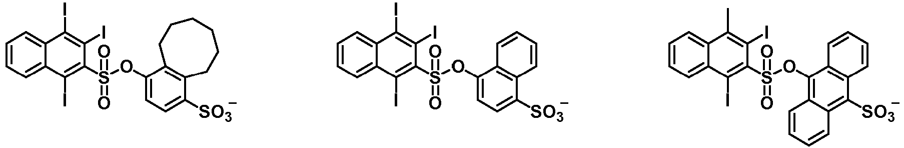

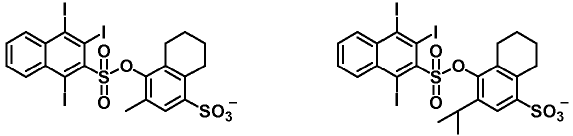

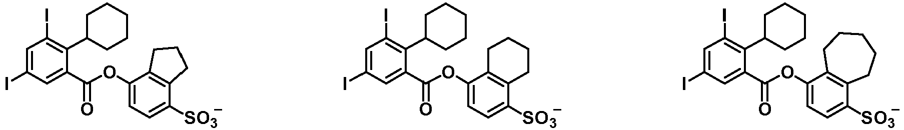

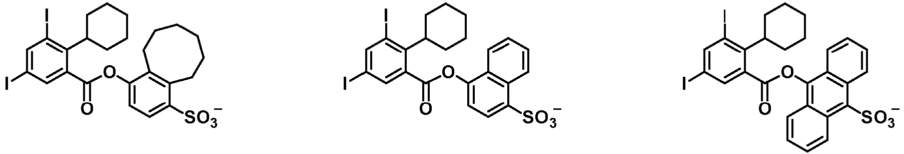

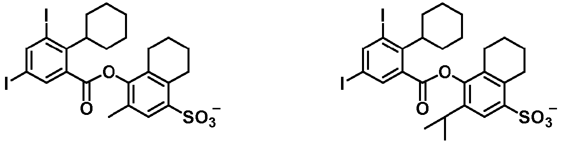

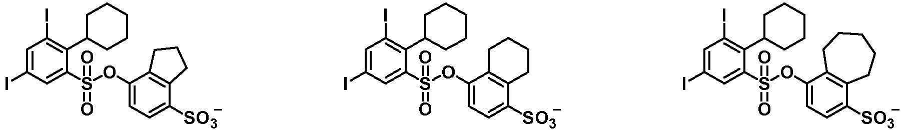

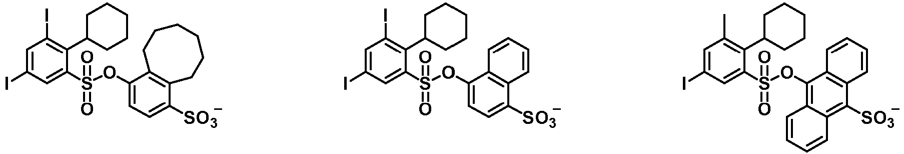

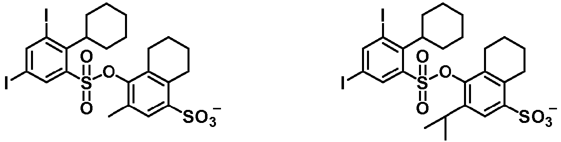

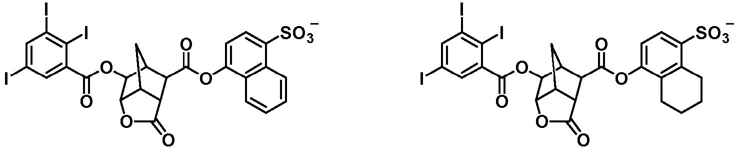

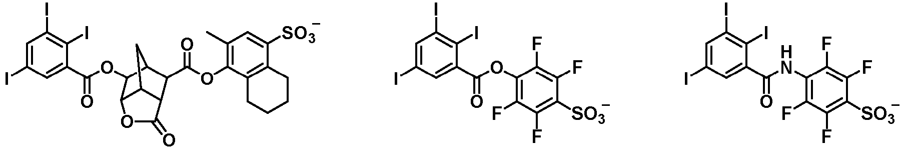

Preferred examples of the cation that pairs with the anion include sulfonium and iodonium cations. Examples of the sulfonium cation are as exemplified above for the sulfonium cation having formulae (Z-1) and (Z-3), but not limited thereto. Examples of the iodonium cation are as exemplified above for the iodonium cation having formula (Z-2), but not limited thereto.

The PAG generates an acid having a pKa value of preferably -2.0 or larger, more preferably -1.0 or larger. The upper limit of pKa is preferably 2.0. Notably, the pKa value is computed using pKa DB in software ACD/Chemsketch ver: 9.04 of Advanced Chemistry Development Inc.

When the resist composition contains the PAG (D), the amount of the PAG (D) used is preferably 1 to 10 parts, more preferably 1 to 5 parts by weight per 80 parts by weight of the base polymer (B). The inclusion of the PAG provides for appropriate adjustment of the amount of acid generated in the exposed region and the degree of dissolution inhibition in the unexposed region. The PAG may be used alone or in admixture.

When the chemically amplified positive resist composition contains both the PAG (D) and the quencher (A), the weight ratio of the PAG to the quencher, (D)/(A) is preferably less than 3/1, more preferably less than 2.5/1, even more preferably less than 2/1. As long as the weight ratio of the PAG to the quencher is in the range, the resist composition is able to fully suppress acid diffusion, leading to improved resolution and dimensional uniformity.

### (E) Fluorinated polymer

The chemically amplified positive resist composition may further comprise a fluorinated polymer for the purposes of enhancing contrast, preventing chemical flare of acid upon exposure to high-energy radiation, preventing mixing of acid from an anti-charging film in the step of coating an anti-charging film-forming material on a resist film, and suppressing unexpected unnecessary pattern degradation. The fluorinated polymer contains repeat units of at least one type selected from repeat units having the formula (E1), repeat units having the formula (E2), repeat units having the formula (E3), and repeat units having the formula (E4). It is noted that repeat units having formulae (E1), (E2), (E3), and (E4) are also referred to as repeat units E1, E2, E3, and E4, respectively, hereinafter. Since the fluorinated polymer also has a surface active function, it can prevent insoluble residues from re-depositing onto the substrate during the development step and is thus effective for preventing development defects.

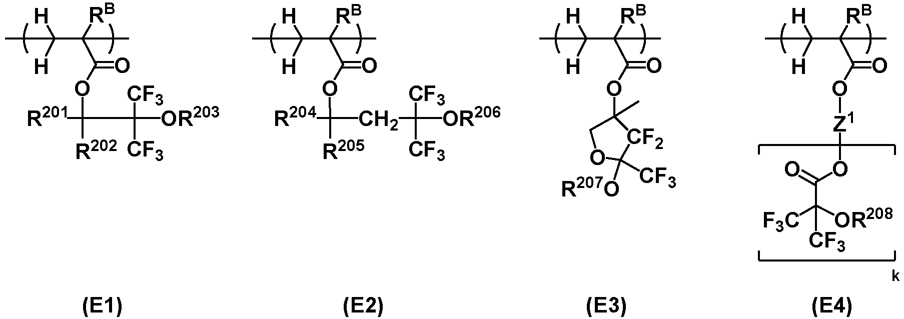

In formulae (E1) to (E4), k is 1, 2 or 3. R^{B} is each independently hydrogen, fluorine, methyl or trifluoromethyl. R²⁰¹, R²⁰², R²⁰⁴ and R²⁰⁵ are each independently hydrogen or a C₁-C₁₀ saturated hydrocarbyl group. R²⁰³, R²⁰⁶, R²⁰⁷ and R²⁰⁸ are each independently hydrogen, a C₁-C₁₅ hydrocarbyl group or fluorinated hydrocarbyl group, or an acid labile group. An ether bond or carbonyl moiety may intervene in a carbon-carbon bond in the hydrocarbyl groups or fluorinated hydrocarbyl groups represented by R²⁰³, R²⁰⁶, R²⁰⁷ and R²⁰⁸. Z¹ is a C₁-C₂₀ (k+1)-valent hydrocarbon group or C₁-C₂₀ (k+1)-valent fluorinated hydrocarbon group.

The C₁-C₁₀ saturated hydrocarbyl group represented by R²⁰¹, R²⁰², R²⁰⁴ and R²⁰⁵ may be straight, branched or cyclic and examples thereof include C₁-C₁₀ alkyl groups such as methyl, ethyl, n-propyl, isopropyl, n-butyl, isobutyl, sec-butyl, tert-butyl, n-pentyl, n-hexyl, n-heptyl, n-octyl, n-nonyl, and n-decyl, and C₃-C₁₀ cyclic saturated hydrocarbyl groups such as cyclopropyl, cyclobutyl, cyclopentyl, cyclohexyl, adamantyl, and norbornyl. Inter alia, C₁-C₆ saturated hydrocarbyl groups are preferred.

The C₁-C₁₅ hydrocarbyl group represented by R²⁰³, R²⁰⁶, R²⁰⁷ and R²⁰⁸ may be straight, branched or cyclic and examples thereof include C₁-C₁₅ alkyl, C₂-C₁₅ alkenyl and C₂-C₁₅ alkynyl groups, with the alkyl groups being preferred. Suitable alkyl groups include methyl, ethyl, n-propyl, isopropyl, n-butyl, isobutyl, sec-butyl, n-pentyl, n-hexyl, n-heptyl, n-octyl, n-nonyl, n-decyl, n-undecyl, n-dodecyl, n-tridecyl, n-tetradecyl and n-pentadecyl. The fluorinated hydrocarbyl groups correspond to the foregoing hydrocarbyl groups in which some or all carbon-bonded hydrogen atoms are substituted by fluorine atoms.

Examples of the C₁-C₂₀ (k+1)-valent hydrocarbon group Z¹ include the foregoing C₁-C₂₀ alkyl groups and C₃-C₂₀ cyclic saturated hydrocarbyl groups, with k number of hydrogen atoms being eliminated. Examples of the C₁-C₂₀ (k+1)-valent fluorinated hydrocarbon group Z¹ include the foregoing (k+1)-valent hydrocarbon groups in which at least one hydrogen atom is substituted by fluorine.

Examples of the repeat units E1 to E4 are given below, but not limited thereto. Herein R^{B} is as defined above.

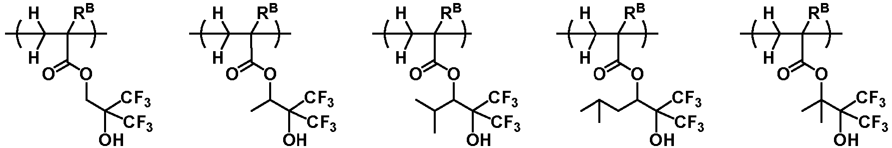

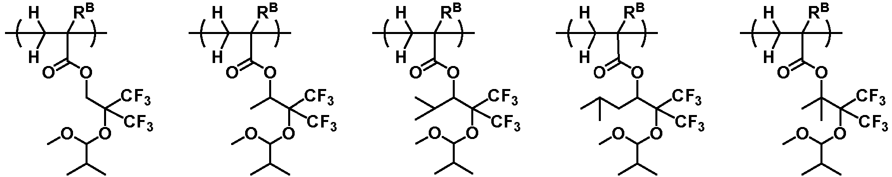

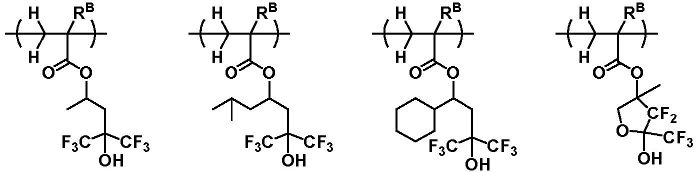

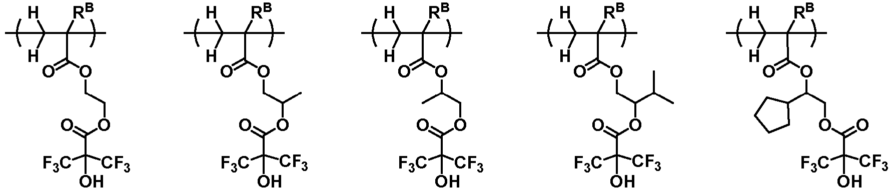

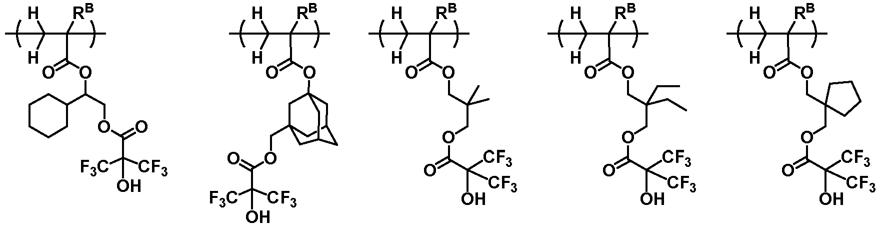

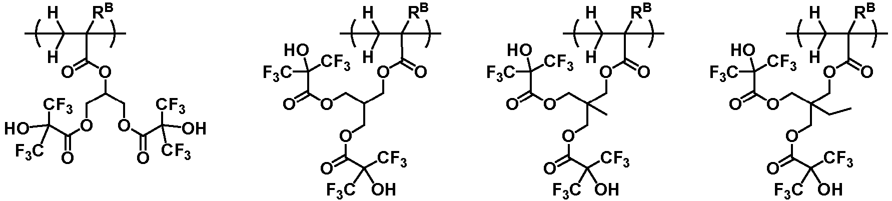

Preferably, the fluorinated polymer further contains repeat units of at least one type selected from repeat units having the formula (E5) and repeat units having the formula (E6). The repeat units having formulae (E5) and (E6) are also referred to as repeat units E5 and E6, respectively.

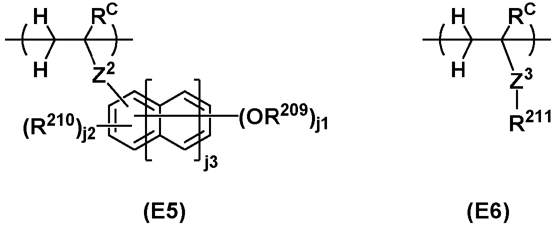

In formulae (E5) and (E6), j1 is 1, 2 or 3, j2 is an integer satisfying: 0 ≤ j2 ≤ 5+2(j3)-j1, and j3 is 0 or 1. R^{C} is each independently hydrogen or methyl. R²⁰⁹ is hydrogen or a C₁-C₅ straight or branched hydrocarbyl group in which a heteroatom-containing moiety may intervene in a carbon-carbon bond. R²¹⁰ is a C₁-C₅ straight or branched hydrocarbyl group in which a heteroatom-containing moiety may intervene in a carbon-carbon bond. R²¹¹ is a C₁-C₂₀ saturated hydrocarbyl group in which at least one hydrogen is substituted by fluorine and some constituent -CH₂- may be replaced by an ester bond or ether bond. Z² is a single bond, *-C(=O)-O- or *-C(=O)-NH-. Z³ is a single bond, -O-, *-C(=O)-O-Z³¹-Z³²- or *-C(=O)-NH-Z³¹-Z³²-. Z³¹ is a single bond or a C₁-C₁₀ saturated hydrocarbylene group. Z³² is a single bond, ester bond, ether bond or sulfonamide bond. The asterisk (*) designates a point of attachment to the carbon atom in the backbone.

Examples of the C₁-C₅ hydrocarbyl groups R²⁰⁹ and R²¹⁰ include alkyl, alkenyl and alkynyl groups, with the alkyl groups being preferred. Suitable alkyl groups include methyl, ethyl, n-propyl, isopropyl, n-butyl, isobutyl, sec-butyl, and n-pentyl. In these groups, a moiety containing a heteroatom such as oxygen, sulfur or nitrogen may intervene in a carbon-carbon bond.

In formula (E5), -OR²⁰⁹ is preferably a hydrophilic group. In this case, R²⁰⁹ is preferably hydrogen or a C₁-C₅ alkyl group in which oxygen intervenes in a carbon-carbon bond.

The C₁-C₂₀ saturated hydrocarbyl group in which at least one hydrogen is substituted by fluorine, represented by R²¹¹, may be straight, branched or cyclic. Examples thereof are C₁-C₂₀ alkyl groups and C₃-C₂₀ cyclic saturated hydrocarbyl groups in which at least one hydrogen atom is substituted by fluorine.

Z² is preferably *-C(=O)-O- or *-C(=O)-NH-. Also preferably R^{C} is methyl. The inclusion of carbonyl in Z² enhances the ability to trap the acid originating from the anti-charging film. A polymer wherein R^{C} is methyl is a robust polymer having a high glass transition temperature (Tg) which is effective for suppressing acid diffusion. As a result, the resist film is improved in stability with time, and neither resolution nor pattern profile is degraded.

The C₁-C₁₀ saturated hydrocarbylene group Z³ may be straight, branched or cyclic. Examples thereof include methanediyl, ethane-1,1-diyl, ethane-1,2-diyl, propane-1,1-diyl, propane-1,2-diyl, propane-1,3-diyl, propane-2,2-diyl, butane-1,1-diyl, butane-1,2-diyl, butane-1,3-diyl, butane-2,3-diyl, butane-1,4-diyl, and 1,1-dimethylethane-1,2-diyl.

Examples of the repeat unit E5 are given below, but not limited thereto. Herein R^{C} is as defined above.

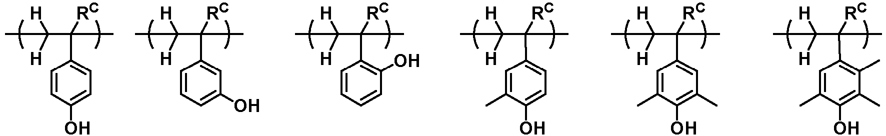

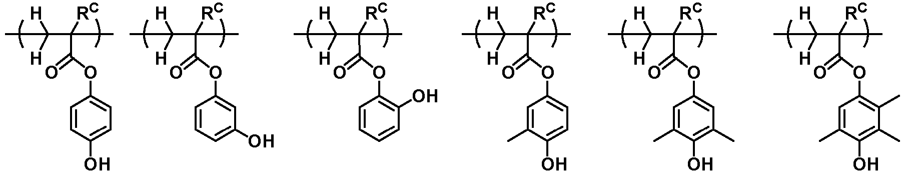

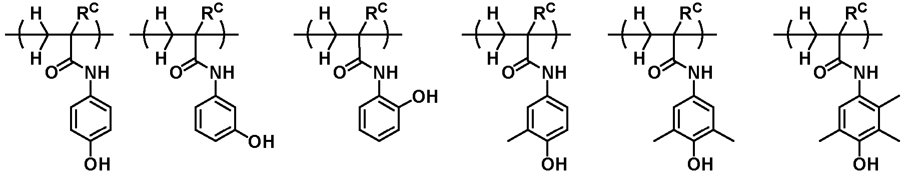

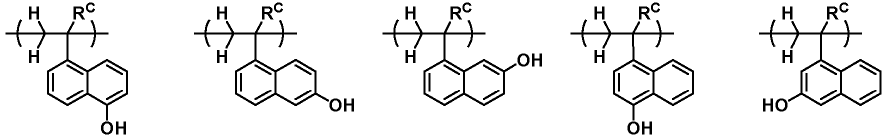

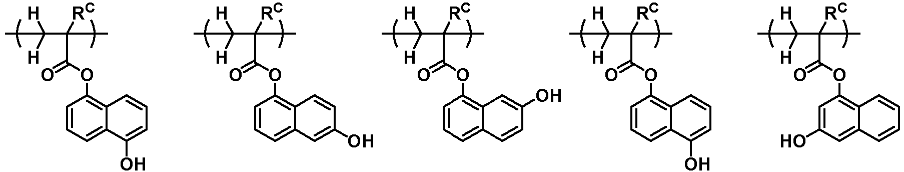

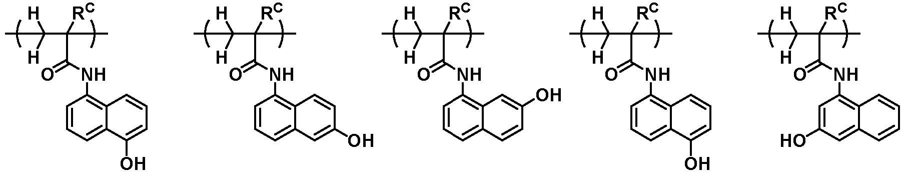

Examples of the repeat unit E6 are given below, but not limited thereto. Herein R^{C} is as defined above.

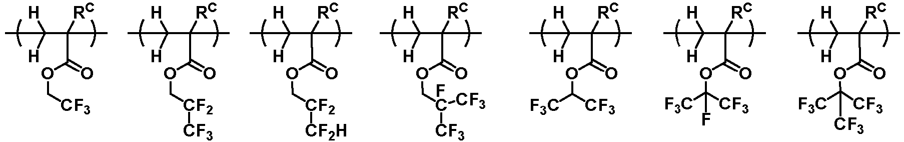

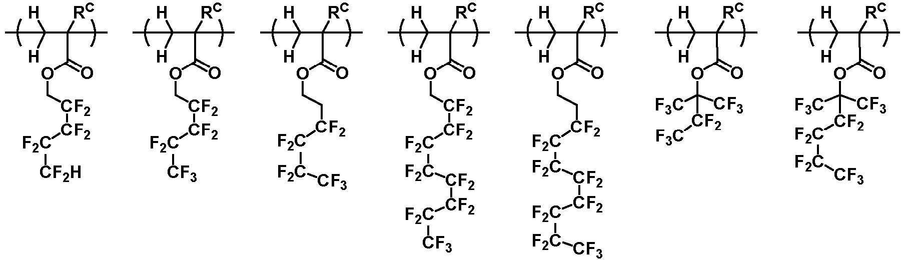

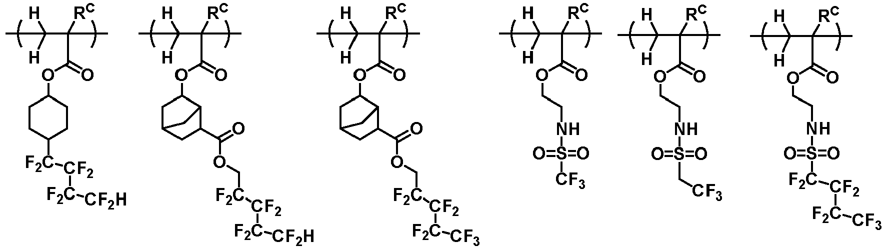

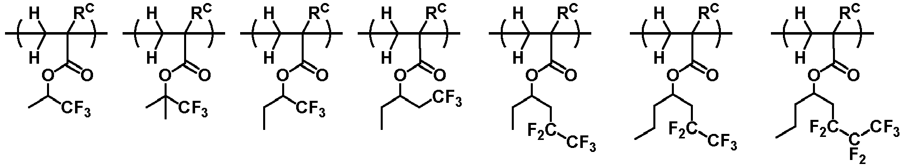

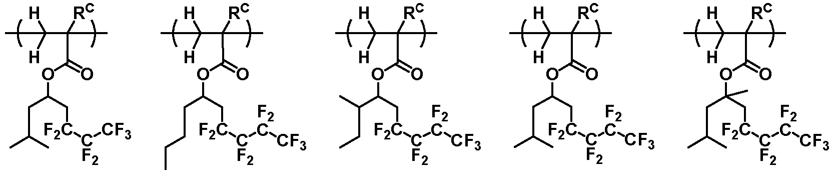

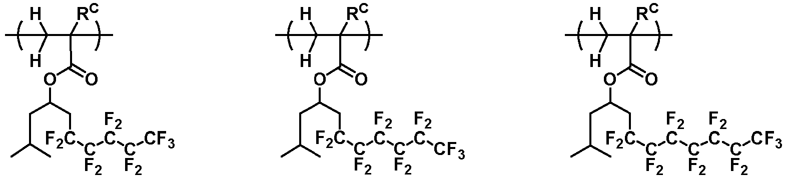

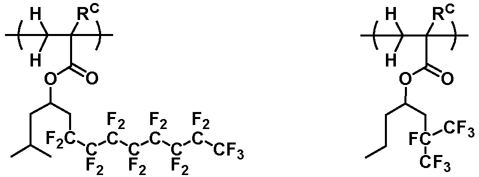

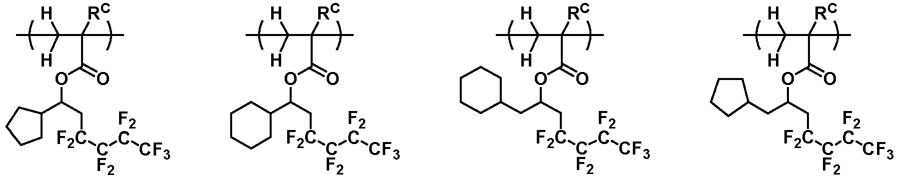

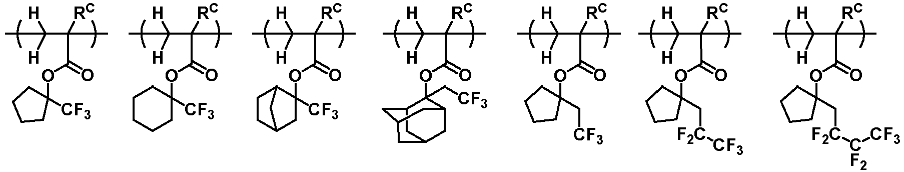

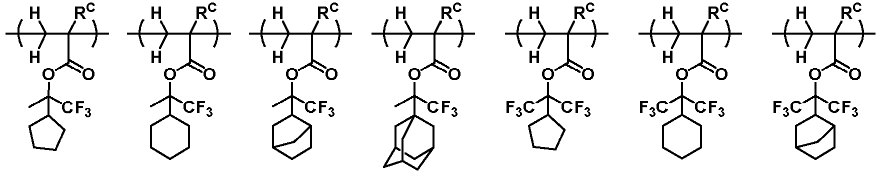

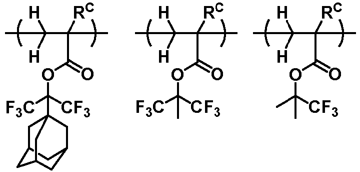

The content of repeat units E1 to E4 is preferably 15 to 95 mol%, more preferably 20 to 85 mol% based on the overall repeat units of the fluorinated polymer. The content of repeat unit E5 and/or E6 is preferably 5 to 85 mol%, more preferably 15 to 80 mol% based on the overall repeat units of the fluorinated polymer. Each of repeat units E1 to E6 may be used alone or in admixture.

The fluorinated polymer may comprise additional repeat units as well as the repeat units E1 to E6. Suitable additional repeat units include those described in USP 9,091,918 (JP-A 2014-177407, paragraphs [0046]-[0078]). When the fluorinated polymer comprises additional repeat units, their content is preferably up to 50 mol% based on the overall repeat units.

The fluorinated polymer may be synthesized by combining suitable monomers optionally protected with a protective group, copolymerizing them in the standard way, and effecting deprotection reaction if necessary. The copolymerization reaction is preferably radical or anionic polymerization though not limited thereto. For the polymerization reaction, reference may be made to JP-A 2004-115630.

The fluorinated polymer should preferably have a Mw of 2,000 to 50,000, and more preferably 3,000 to 20,000. A Mw of at least 2,000 is effective for restraining acid diffusion, avoiding degradation of resolution, and eliminating any loss of age stability. A Mw of up to 50,000 ensures a sufficient solvent solubility to eliminate coating defectiveness. The fluorinated polymer preferably has a dispersity (Mw/Mn) of 1.0 to 2.2, more preferably 1.0 to 1.7.

In the resist composition, the fluorinated polymer (E) is preferably used in an amount of 0.01 to 30 parts by weight, more preferably 0.1 to 20 parts by weight per 80 parts by weight of the base polymer (B). The fluorinated polymer may be used alone or in admixture.

### (F) Other quencher

The chemically amplified positive resist composition may comprise a quencher other than component (A) as component (F) if necessary.

The other quencher is typically selected from conventional basic compounds. Conventional basic compounds include primary, secondary, and tertiary aliphatic amines, mixed amines, aromatic amines, heterocyclic amines, nitrogen-containing compounds with carboxy group, nitrogen-containing compounds with sulfonyl group, nitrogen-containing compounds with hydroxy group, nitrogen-containing compounds with hydroxyphenyl group, alcoholic nitrogen-containing compounds, amide derivatives, imide derivatives, and carbamate derivatives. Also included are primary, secondary, and tertiary amine compounds, specifically amine compounds having a hydroxy, ether bond, ester bond, lactone ring, cyano, or sulfonate ester group as described in JP-A 2008-111103, paragraphs [0146]-[0164], and compounds having a carbamate group as described in JP 3790649. Inter alia, tris[2-(methoxymethoxy)ethyl]amine, tris[2-(methoxymethoxy)ethyl]amine-N-oxide, dibutylaminobenzoic acid, morpholine derivatives, and imidazole derivatives are preferred. Addition of a basic compound may be effective for further suppressing the diffusion rate of acid in the resist film or correcting the pattern profile.

Onium salts such as sulfonium, iodonium and ammonium salts of carboxylic acids which are not fluorinated at α-position as described in USP 8,795,942 (JP-A 2008-158339) may also be used as the other quencher. While an α-fluorinated sulfonic acid, imide acid, and methide acid are necessary to deprotect the acid labile group, an α-non-fluorinated carboxylic acid is released by salt exchange with an α-non-fluorinated onium salt. The α-non-fluorinated carboxylic acid functions as a quencher because it does not induce substantial deprotection reaction.

Examples of the onium salt of α-non-fluorinated carboxylic acid include compounds having the formula (F1).

**R³⁰¹-CO₂⁻ Mq_{A}⁺** **(F1)**

In formula (F1), R³⁰¹ is hydrogen or a C₁-C₄₀ hydrocarbyl group which may contain a heteroatom, exclusive of the hydrocarbyl group in which the hydrogen bonded to the carbon atom at α-position of the carboxy group is substituted by fluorine or fluoroalkyl.

The hydrocarbyl group R³⁰¹ may be saturated or unsaturated and straight, branched or cyclic. Examples thereof include C₁-C₄₀ alkyl groups such as methyl, ethyl, n-propyl, isopropyl, n-butyl, sec-butyl, tert-butyl, n-pentyl, tert-pentyl, n-hexyl, n-octyl, 2-ethylhexyl, n-nonyl, and n-decyl; C₃-C₄₀ cyclic saturated hydrocarbyl groups such as cyclopentyl, cyclohexyl, cyclopentylmethyl, cyclopentylethyl, cyclopentylbutyl, cyclohexylmethyl, cyclohexylethyl, cyclohexylbutyl, norbornyl, tricyclo[5.2.1.0^{2,6}]decyl, adamantyl, and adamantylmethyl; C₂-C₄₀ alkenyl groups such as vinyl, allyl, propenyl, butenyl and hexenyl; C₃-C₄₀ cyclic unsaturated aliphatic hydrocarbyl groups such as cyclohexenyl; C₆-C₄₀ aryl groups such as phenyl, naphthyl, alkylphenyl groups (e.g., 2-methylphenyl, 3-methylphenyl, 4-methylphenyl, 4-ethylphenyl, 4-tert-butylphenyl, 4-n-butylphenyl), di- or trialkylphenyl groups (e.g., 2,4-dimethylphenyl and 2,4,6-triisopropylphenyl), alkylnaphthyl groups (e.g., methylnaphthyl and ethylnaphthyl), dialkylnaphthyl groups (e.g., dimethylnaphthyl and diethylnaphthyl); and C₇-C₄₀ aralkyl groups such as benzyl, 1-phenylethyl and 2-phenylethyl.

In the hydrocarbyl groups, some or all hydrogen may be substituted by a moiety containing a heteroatom such as oxygen, sulfur, nitrogen or halogen, and some -CH₂-may be replaced by a moiety containing a heteroatom such as oxygen, sulfur or nitrogen, so that the group may contain a hydroxy moiety, cyano moiety, carbonyl moiety, ether bond, thioether bond, ester bond, sulfonate ester bond, carbonate bond, lactone ring, sultone ring, carboxylic anhydride (-C(=O)-O-C(=O)-), or haloalkyl moiety. Suitable heteroatom-containing hydrocarbyl groups include heteroaryl groups such as thienyl; alkoxyphenyl groups such as 4-hydroxyphenyl, 4-methoxyphenyl, 3-methoxyphenyl, 2-methoxyphenyl, 4-ethoxyphenyl, 4-tert-butoxyphenyl, 3-tert-butoxyphenyl; alkoxynaphthyl groups such as methoxynaphthyl, ethoxynaphthyl, n-propoxynaphthyl and n-butoxynaphthyl; dialkoxynaphthyl groups such as dimethoxynaphthyl and diethoxynaphthyl; and aryloxoalkyl groups, typically 2-aryl-2-oxoethyl groups such as 2-phenyl-2-oxoethyl, 2-(1-naphthyl)-2-oxoethyl and 2-(2-naphthyl)-2-oxoethyl.

In formula (F1), Mq_{A}⁺ is an onium cation. The onium cation is preferably selected from sulfonium, iodonium and ammonium cations, more preferably sulfonium and iodonium cations. Exemplary sulfonium cations are as exemplified above for the sulfonium cation having formula (Z-1). Exemplary iodonium cations are as exemplified above for the iodonium cation having formula (Z-2).

Examples of the anion in the onium salt having formula (F1) are shown below, but not limited thereto.

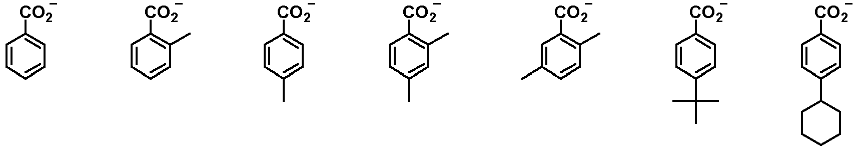

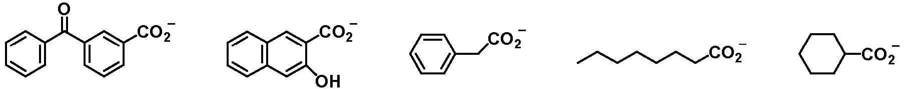

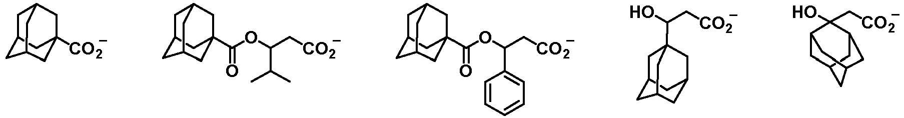

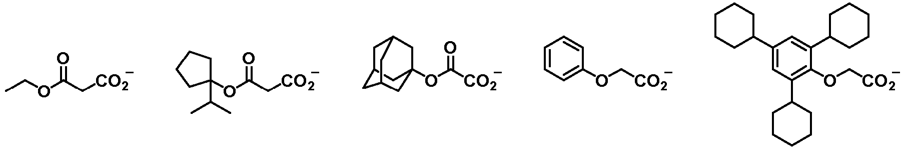

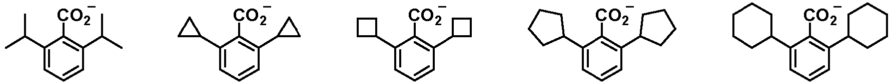

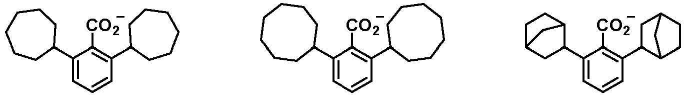

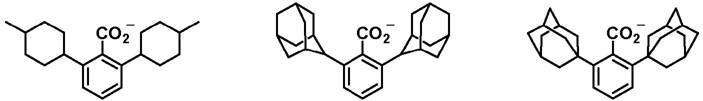

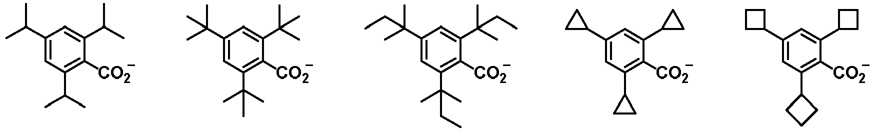

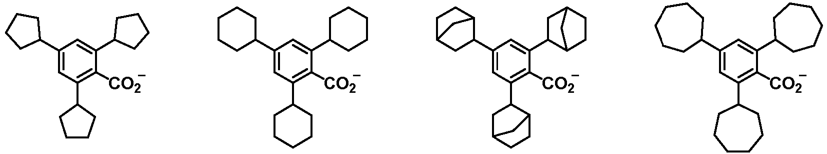

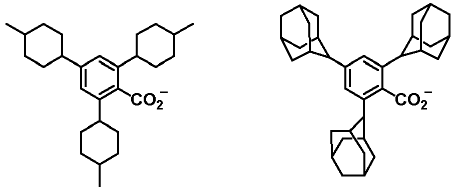

A sulfonium salt of iodized benzene ring-containing carboxylic acid having the formula (F2) is also useful as the quencher.

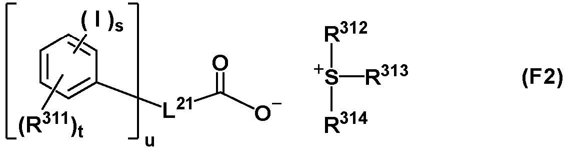

In formula (F2), s is 1, 2, 3, 4 or 5, t is 0, 1, 2 or 3, 1 ≤ s+t ≤ 5, and u is 1, 2 or 3.

In formula (F2), R³¹¹ is hydroxy, fluorine, chlorine, bromine, amino, nitro, cyano, or a C₁-C₆ saturated hydrocarbyl, C₁-C₆ saturated hydrocarbyloxy, C₂-C₆ saturated hydrocarbylcarbonyloxy or C₁-C₄ saturated hydrocarbylsulfonyloxy group, in which some or all hydrogen may be substituted by halogen, or -N(R^{311A})-C(=O)-R^{311B}, or -N(R^{311A})-C(=O)-O-R^{311B}. R^{311A} is hydrogen or a C₁-C₆ saturated hydrocarbyl group. R^{311B} is a C₁-C₆ saturated hydrocarbyl or C₂-C₈ unsaturated aliphatic hydrocarbyl group. A plurality of R³¹¹ may be identical or different when t and/or u is 2 or 3.

In formula (F2), L²¹ is a single bond or a C₁-C₂₀ (u+1)-valent linking group which may contain at least one moiety selected from ether bond, carbonyl moiety, ester bond, amide bond, sultone ring, lactam ring, carbonate bond, halogen, hydroxy moiety, and carboxy moiety. The saturated hydrocarbyl, saturated hydrocarbyloxy, saturated hydrocarbylcarbonyloxy, and saturated hydrocarbylsulfonyloxy groups may be straight, branched or cyclic.

In formula (F2), R³¹², R³¹³ and R³¹⁴ are each independently halogen, or a C₁-C₂₀ hydrocarbyl group which may contain a heteroatom. The hydrocarbyl group may be saturated or unsaturated and straight, branched or cyclic. Examples thereof include C₁-C₂₀ alkyl, C₂-C₂₀ alkenyl, C₆-C₂₀ aryl, and C₇-C₂₀ aralkyl groups. In these groups, some or all hydrogen may be substituted by hydroxy, carboxy, halogen, oxo, cyano, nitro, sultone ring, sulfo, or sulfonium salt-containing moiety, or some -CH₂- may be replaced by an ether bond, ester bond, carbonyl moiety, amide bond, carbonate bond or sulfonate ester bond. Also, R³¹² and R³¹³ may bond together to form a ring with the sulfur atom to which they are attached.

Examples of the compound having formula (F2) include those described in USP 10,295,904 (JP-A 2017-219836). These compounds exert a sensitizing effect due to remarkable absorption and an acid diffusion-controlling effect.

A nitrogen-containing carboxylic acid salt compound having the formula (F3) is also useful as the quencher.

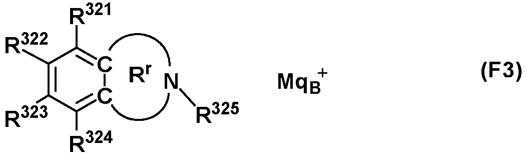

In formula (F3), R³²¹ to R³²⁴ are each independently hydrogen, -L³¹-CO₂⁻, or a C₁-C₂₀ hydrocarbyl group which may contain a heteroatom. R³²¹ and R³²², R³²² and R³²³, or R³²³ and R³²⁴ may bond together to form a ring with the carbon atom to which they are attached. L³¹ is a single bond or a C₁-C₂₀ hydrocarbylene group which may contain a heteroatom. R³²⁵ is hydrogen or a C₁-C₂₀ hydrocarbyl group which may contain a heteroatom.

In formula (F3), the ring R^{r} is a C₂-C₆ ring containing the carbon and nitrogen atoms in the formula, in which some or all of the carbon-bonded hydrogen atoms may be substituted by a C₁-C₂₀ hydrocarbyl group or -L³¹-CO₂⁻ and in which some carbon may be replaced by sulfur, oxygen or nitrogen. The ring may be alicyclic or aromatic and is preferably a 5- or 6-membered ring. Suitable rings include pyridine, pyrrole, pyrrolidine, piperidine, pyrazole, imidazoline, pyridazine, pyrimidine, pyrazine, imidazoline, oxazole, thiazole, morpholine, thiazine, and triazole rings.

The carboxylic onium salt having formula (F3) has at least one -L³¹-CO₂⁻. That is, at least one of R³²¹ to R³²⁴ is -L³¹-CO₂⁻, and/or at least one of hydrogen atoms bonded to carbon atoms in the ring R^{r} is substituted by -L³¹-CO₂⁻.

In formula (F3), Mq_{B}⁺ is a sulfonium, iodonium or ammonium cation, with the sulfonium cation being preferred. Examples of the sulfonium cation are as exemplified above for the sulfonium cations having formulae (Z-1) and (Z-3).

Examples of the anion in the compound having formula (F3) are shown below, but not limited thereto.

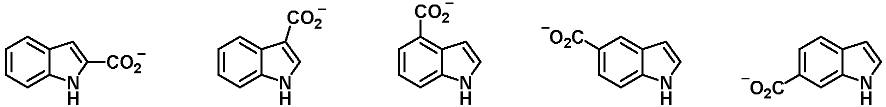

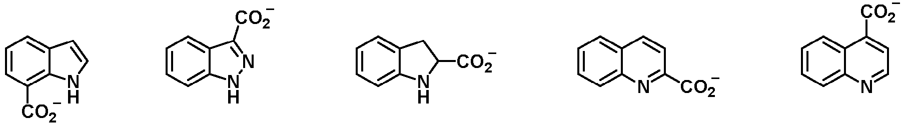

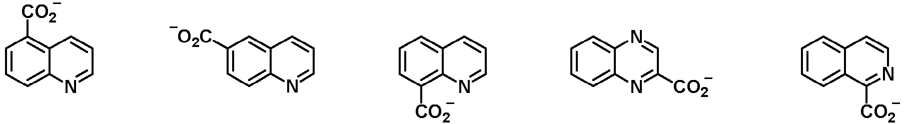

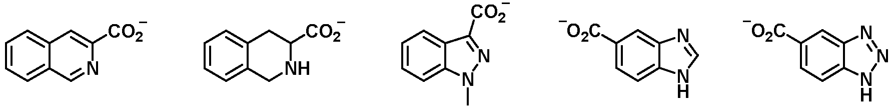

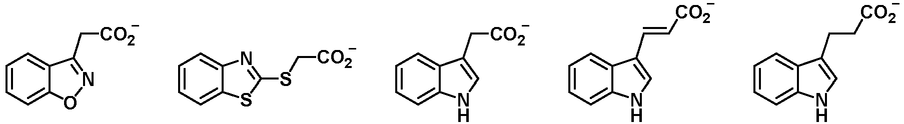

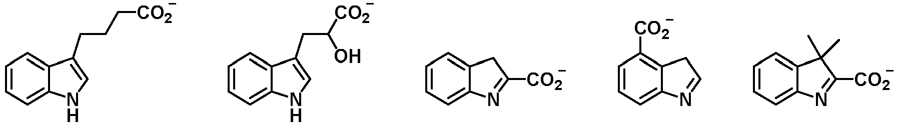

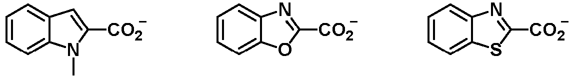

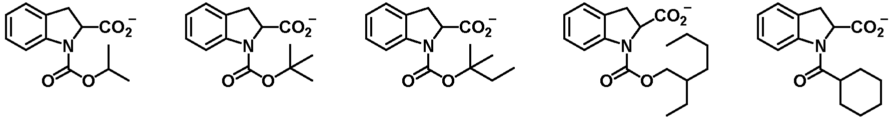

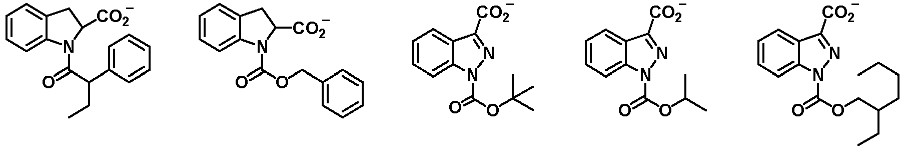

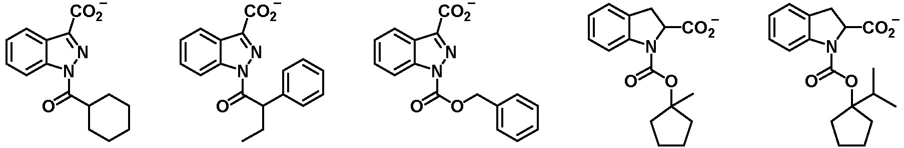

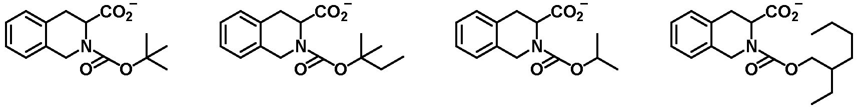

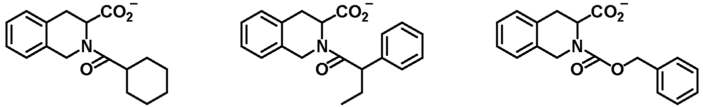

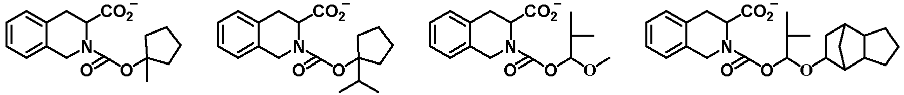

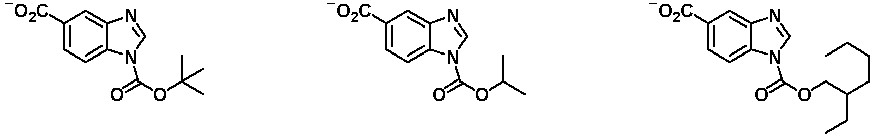

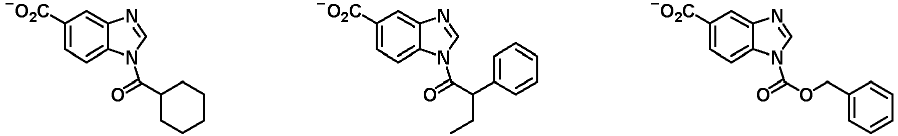

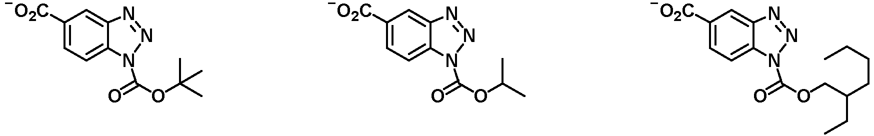

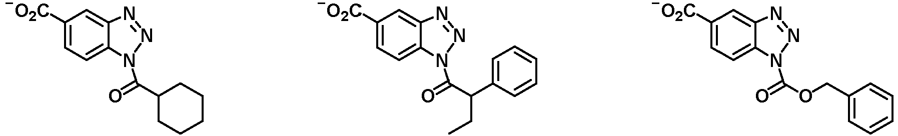

Weak acid betaine compounds are also useful as the quencher. Non-limiting examples thereof are shown below.

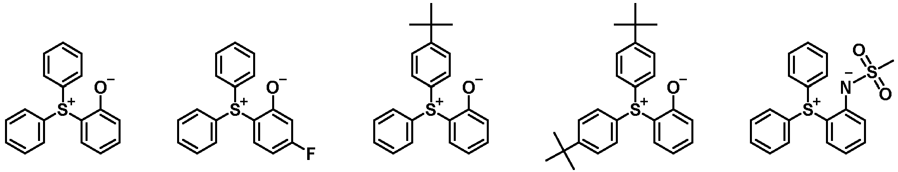

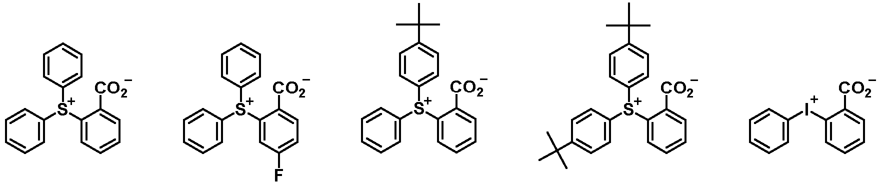

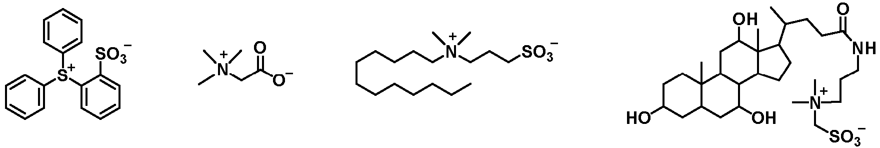

Also useful are quenchers of polymer type as described in USP 7,598,016 (JP-A 2008-239918). The polymeric quencher segregates at the resist surface after coating and thus enhances the rectangularity of resist pattern. When a protective film is applied as is often the case in the immersion lithography, the polymeric quencher is also effective for preventing a film thickness loss of resist pattern or rounding of pattern top.

When used, the other quencher (F) is preferably added in an amount of 0 to 50 parts, more preferably 0.1 to 40 parts by weight per 80 parts by weight of the base polymer (B). The quencher may be used alone or in admixture.

### (G) Surfactant

The positive resist composition may contain any conventional surfactants for facilitating to coat the composition to the substrate. A number of surfactants are known in the art as described in WO 2006/121096, JP-A 2004-115630, JP-A 2005-008766, JP-A 2008-102383, and JP-A 2008-304590, and any suitable one may be chosen therefrom.

When used, the amount of surfactant added is preferably up to 2 parts by weight, more preferably up to 1 part by weight per 80 parts by weight of the base polymer (B). The lower limit is preferably 0.01 part by weight or more. The surfactant may be used alone or in admixture.

### [Pattern forming process]

A further embodiment of the invention is a process of forming a pattern from the chemically amplified positive resist composition defined above by lithography. The preferred process includes the steps of applying the resist composition onto a substrate to form a resist film thereon, exposing the resist film to high-energy radiation, and developing the exposed resist film in an alkaline developer. Any desired steps may be added to the process if necessary.

The substrate used herein may be a substrate for integrated circuitry fabrication, e.g., Si, SiO, SiO₂, SiN, SiON, TiN, WSi, BPSG, SOG, organic antireflective film, etc. or a substrate for mask circuitry fabrication, e.g., Cr, CrO, CrON, MoSi₂, Si, SiO, SiO₂, etc.

The resist composition is applied onto a substrate by a suitable coating technique such as spin coating. The coating is prebaked on a hot plate preferably at a temperature of 60 to 150°C for 1 to 20 minutes, more preferably at 80 to 140°C for 1 to 10 minutes. The resulting resist film preferably has a thickness of 0.03 to 2 µm.

Then the resist film is exposed to a pattern of high-energy radiation, typically UV, deep UV, excimer (KrF or ArF) laser, EUV of wavelength 3 to 15 nm, EB, X-ray, γ-ray or synchrotron radiation. On use of UV, deep UV, excimer laser, EUV, X-ray, γ-ray or synchrotron radiation, the resist film is exposed through a mask having a desired pattern, preferably in a dose of 1 to 300 mJ/cm², more preferably 10 to 200 mJ/cm². On use of EB, a pattern may be written directly, preferably in a dose of 1 to 300 µC/cm², more preferably 10 to 200 µC/cm². The inventive resist composition is quite effective in the EUV and EB lithography.

The exposure may be performed by conventional lithography whereas the immersion lithography of holding a liquid between the resist film and the mask may be employed if desired. The liquid is typically water, and in this case, a protective film which is insoluble in water may be formed on the resist film.

After the exposure, the resist film may be baked (PEB), for example, on a hotplate preferably at 60 to 150°C for 1 to 20 minutes, more preferably at 80 to 140°C for 1 to 10 minutes.

The resist film is then developed with a developer in the form of an aqueous base solution, for example, 0.1 to 5 wt%, preferably 2 to 3 wt% aqueous solution of tetramethylammonium hydroxide (TMAH) for 0.1 to 3 minutes, preferably 0.5 to 2 minutes by conventional techniques such as dip, puddle and spray techniques. In this way, a desired resist pattern is formed on the substrate.

The chemically amplified positive resist composition has high etch resistance and is useful when used to meet the requirement that even when the duration from exposure to PEB is prolonged, the pattern experiences little changes of line width and has reduced LER. The resist composition is effectively applicable to a substrate, specifically a substrate having a surface layer of material to which a resist film is less adherent and which is likely to invite pattern stripping or pattern collapse, and particularly a substrate having sputter deposited on its outermost surface metallic chromium or a chromium compound containing at least one light element selected from oxygen, nitrogen and carbon. The resist composition is useful particularly when a photomask blank is used as the substrate.

### EXAMPLES

Examples of the invention are given below by way of illustration and not by way of limitation. The abbreviation "pbw" is parts by weight. Analysis is made by time-of-flight mass spectrometry (TOF-MS) using MALDI TOF-MS S3000 (JEOL Ltd.).

### [1] Synthesis of iodonium salts

### [Example 1-1]

### Synthesis of iodonium salt SQ-1

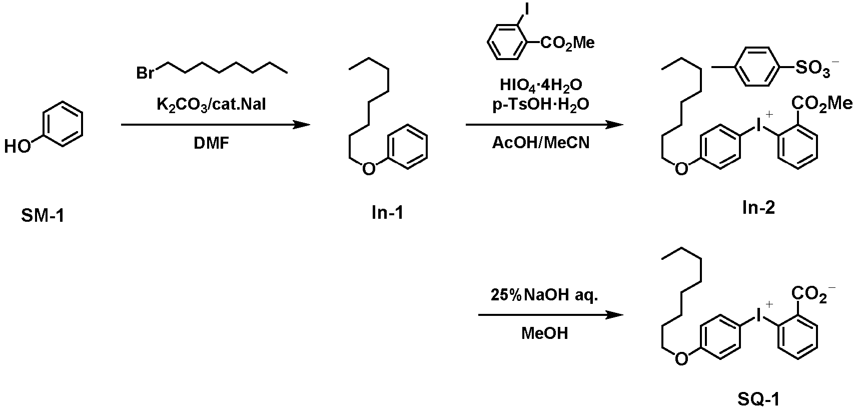

### (1) Synthesis of Intermediate In-1

In a reactor under nitrogen atmosphere, 94.1 g of phenol, 165.9 g of potassium carbonate, and 15.0 g of sodium iodide were dissolved in 500 g of DMF. The internal temperature of the reactor was raised to 70°C, whereupon 231.7 g of n-octyl bromide was added dropwise. Thereafter, with the internal temperature kept at 80°C, the reaction solution was heated and aged for 12 hours. At the end of aging, the reaction solution was cooled and 500 g of water was added to quench the reaction. The desired compound was extracted with 700 g of hexane, followed by ordinary aqueous work-up. The subsequent steps of distilling off the solvent and purifying through a silica gel column gave 204.3 g of Intermediate In-1 as oily matter (yield 99%).

### (2) Synthesis of Intermediate In-2

In a reactor under nitrogen atmosphere, 5.2 g of methyl 2-iodobenzoate was dissolved in 60 g of acetic acid. The solution was stirred at a temperature of 40°C. Then 31.7 g of periodic acid tetrahydrate was slowly added over 1 hour. While the internal temperature of the reactor was kept at 40-45°C, stirring was continued for 6 hours. The reaction solution was allowed to stand at room temperature for 12 hours. Then 90 g of acetonitrile was added and the reactor was cooled at a temperature below 5°C. After cooling, 4.1 g of Intermediate In-1 was added and 1.9 g of p-toluenesulfonic acid monohydrate in 90 g of acetonitrile was added dropwise. While the internal temperature of the reactor was kept below 5°C, the solution was aged for 2 hours. The reaction solution was poured into 500 g of ice water, followed by extraction with 500 g of dichloromethane. The organic layer was washed with an aqueous solution of 50 g of sodium sulfite in 500 g of water and further with 500 g of water. The organic layer was concentrated until the volume of the solvent was reduced to 1/10. To the concentrate was added 100 g of ethyl acetate. The solution was concentrated again, obtaining 4.6 g of Intermediate In-2 as oily matter (yield 36%).

### (3) Synthesis of iodonium salt SQ-1

In a reactor under nitrogen atmosphere, 4.6 g of Intermediate In-2 was dissolved in 20 g of methanol. 1.5 g of 25 wt% aqueous solution of sodium hydroxide was added to the solution, which was aged for 4 hours. After aging, 30 g of water was added to quench the reaction. The target compound was extracted with 40 g of methylene chloride, followed by ordinary aqueous work-up and solvent distillation. The residue was washed with diisopropyl ether, obtaining 2.4 g of the target compound SQ-1 as oily matter (yield 73%).

SQ-1 was analyzed by TOF-MS, with the data shown below.
MALDI TOF-MS:
positive M⁺ 453 (corresponding to C₂₁H₂₆IO₃⁺)

### [Examples 1-2 to 1-6]

### Synthesis of iodonium salts SQ-2 to SQ-6

Iodonium salts SQ-2 to SQ-6 shown below were synthesized using corresponding reactants and well-known organic synthesis reactions.

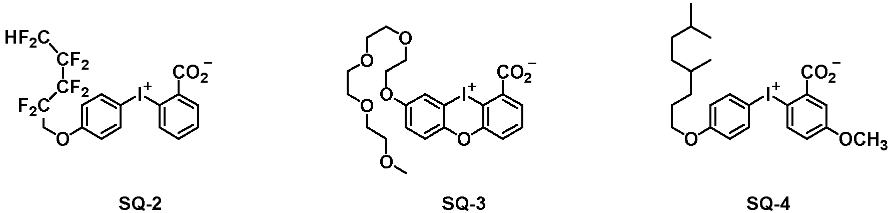

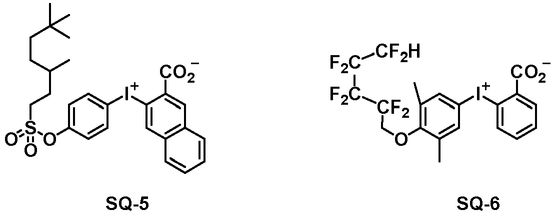

### Synthesis of base polymers

### [Synthesis Example]

### Synthesis of Base Polymers P-1 to P-6

[2] Base polymers P-1 to P-6 were synthesized in a standard way by combining monomers, performing copolymerization reaction in a solvent, pouring the reaction solution to hexane for precipitation, washing the solid precipitate with hexane, isolation and drying. The polymer was analyzed for composition by ¹H-NMR and ¹³C-NMR spectroscopy and for Mw and Mw/Mn by GPC versus polystyrene standards using THF solvent.

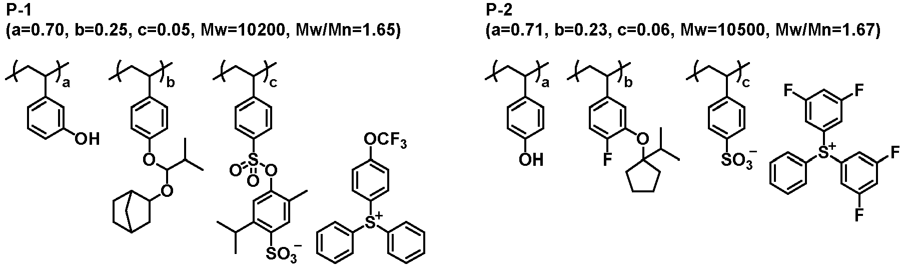

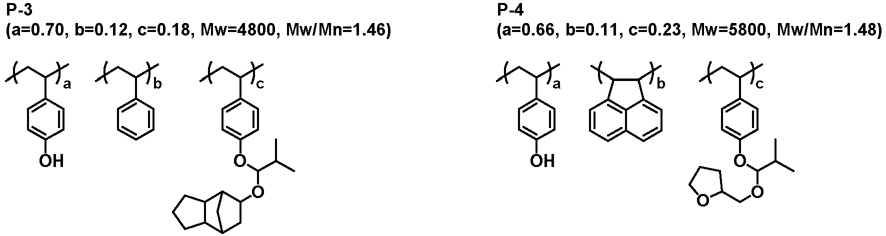

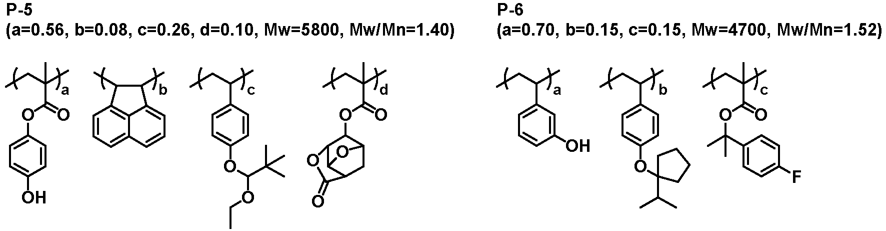

### Preparation of chemically amplified positive resist compositions

### [Examples 2-1 to 2-42 and Comparative Examples 1-1 to 1-35]

[3] A chemically amplified positive resist composition was prepared by dissolving selected components in an organic solvent in accordance with the formulation shown in Tables 1 to 3, and filtering the solution through a nylon filter with a pore size of 5 nm and a UPE filter with a pore size of 1 nm. The organic solvent was a mixture of 940 pbw of PGMEA, 1,870 pbw of EL and 1,870 pbw of PGME.

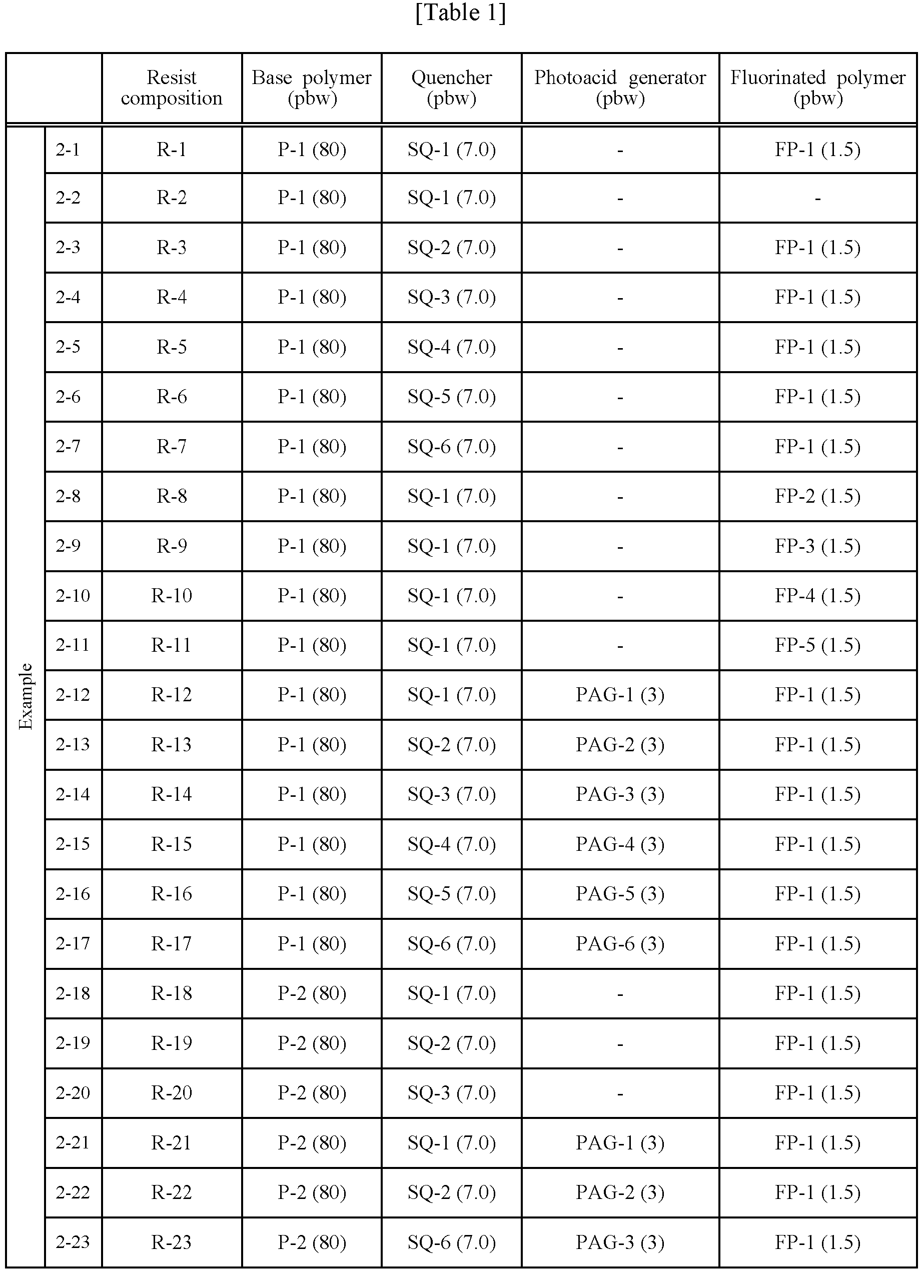

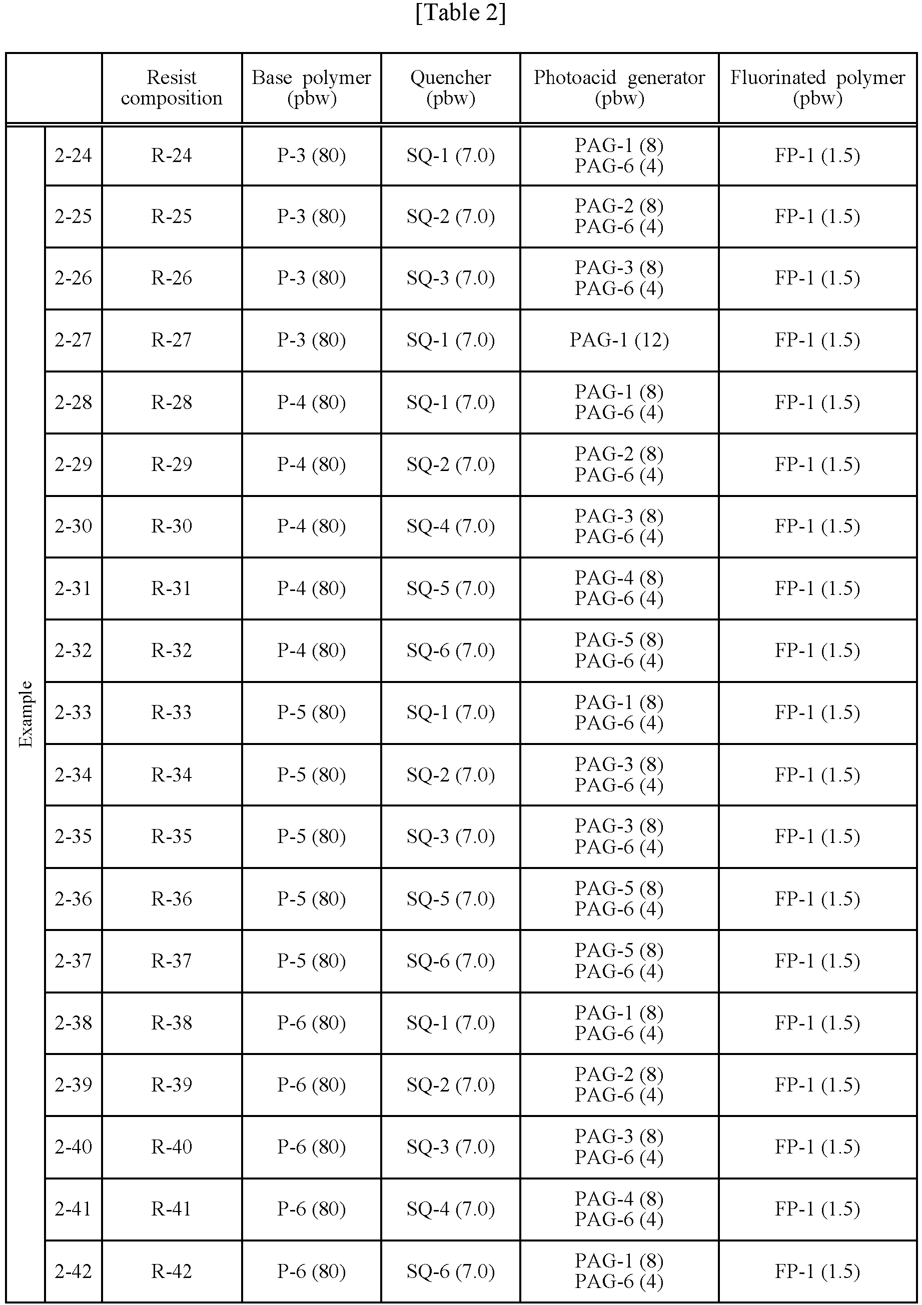

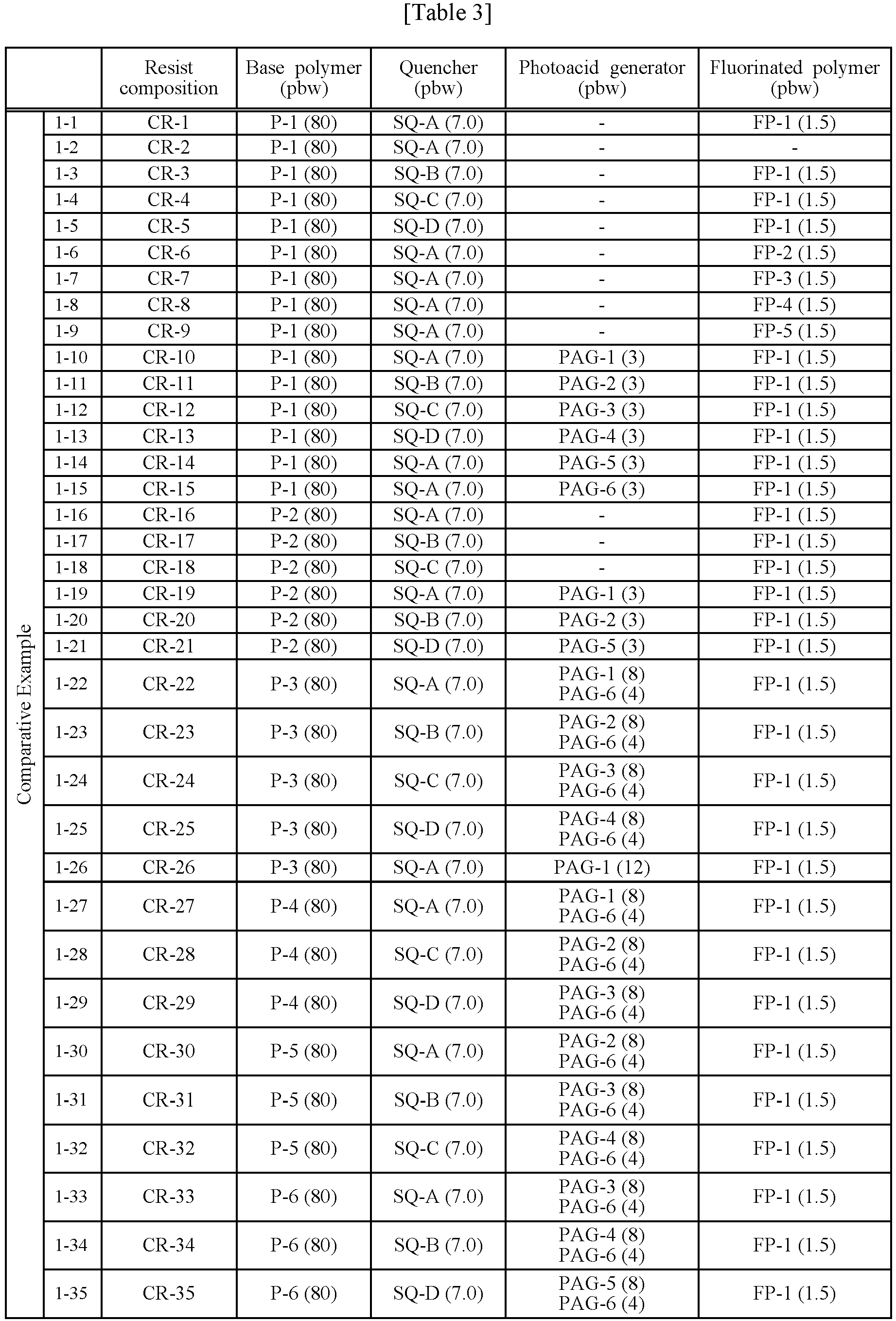

Photoacid generators PAG-1 to PAG-6, comparative quenchers SQ-A to SQ-D, and fluorinated polymers FP-1 to FP-5 in Tables 1 to 3 are identified below.

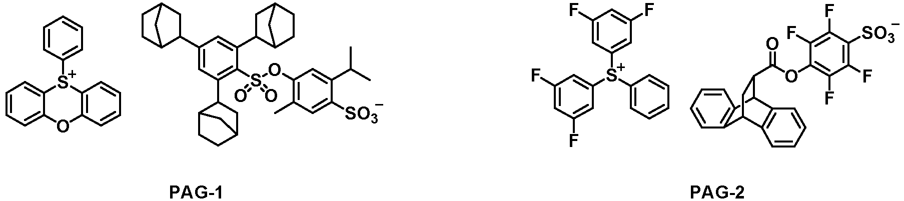

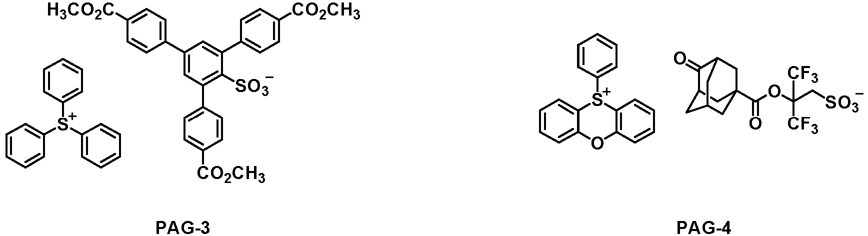

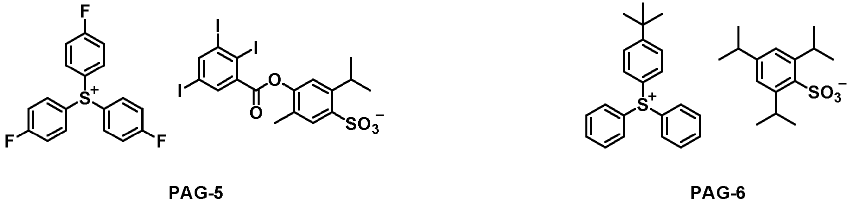

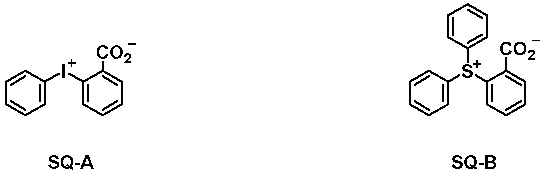

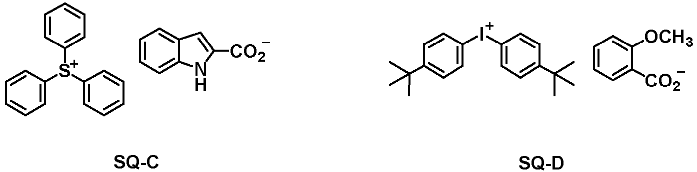

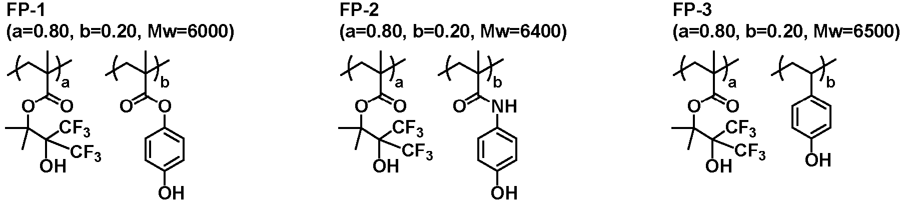

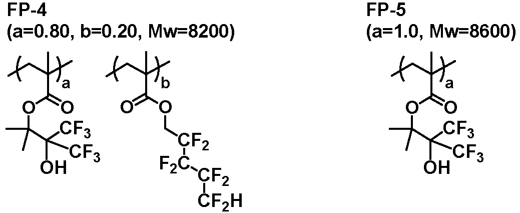

### EB lithography test

### [Examples 3-1 to 3-42 and Comparative Examples 2-1 to 2-35]

[4] A photomask blank of reflection type for an EUV lithography mask was of 152 mm squares and had an outermost surface of chromium compound. Using a coater/developer system ACT-M (Tokyo Electron Ltd.), each of the resist compositions (R-1 to R-42, CR-1 to CR-35) was spin coated onto the photomask blank and prebaked on a hotplate at 110°C for 600 seconds to form a resist film of 80 nm thick. The thickness of the resist film was measured by an optical film thickness measurement system Nanospec (Nanometrics Inc.). Measurement was made at 81 points in the plane of the blank substrate excluding a peripheral band extending 10 mm inward from the blank periphery, and an average film thickness and a film thickness range were computed therefrom.

The resist film was exposed to EB using an EB writer system EBM-5000Plus (NuFlare Technology Inc., accelerating voltage 50 kV), then baked (PEB) at 110°C for 600 seconds, and developed in a 2.38 wt% TMAH aqueous solution, thereby yielding a positive pattern.

The resist pattern was evaluated as follows. The patterned mask blank was observed under a top-down scanning electron microscope (TD-SEM). The optimum dose (Eop) was defined as the exposure dose (µC/cm²) which provided a 1:1 resolution at the top and bottom of a 200-nm 1:1 line-and-space (LS) pattern. The minimum size at the dose which provided a 1:1 resolution of a 200-nm LS pattern was regarded as the resolution (maximum resolution). The edge roughness (LER) of a 200-nm LS pattern was computed from the SEM image. The maximum resolution of IS was defined as the minimum size printed at the dose which provided a 9:1 resolution for a 200-nm 9:1 LS pattern. The pattern was visually observed to judge whether or not the profile was rectangular. The results are shown in Tables 4 and 5.

**[Table 4]**

| | | Resist composition | Optimal exposure dose (µC/cm²) | L/S resolution (nm) | Maximum IS resolution (nm) | LER (nm) | Pattern profile |
|---|---|---|---|---|---|---|---|
| Example | 3-1 | R-1 | 205 | 30 | 16 | 2.8 | rectangular |
| | 3-2 | R-2 | 205 | 30 | 16 | 2.9 | rectangular |
| | 3-3 | R-3 | 200 | 28 | 18 | 2.9 | rectangular |
| | 3-4 | R-4 | 210 | 30 | 20 | 3 | rectangular |
| | 3-5 | R-5 | 200 | 32 | 18 | 2.9 | rectangular |
| | 3-6 | R-6 | 200 | 28 | 20 | 2.7 | rectangular |
| | 3-7 | R-7 | 205 | 30 | 20 | 2.8 | rectangular |
| | 3-8 | R-8 | 200 | 28 | 18 | 2.8 | rectangular |
| | 3-9 | R-9 | 205 | 28 | 16 | 2.9 | rectangular |
| | 3-10 | R-10 | 200 | 32 | 20 | 2.7 | rectangular |
| | 3-11 | R-11 | 210 | 30 | 18 | 2.8 | rectangular |
| | 3-12 | R-12 | 200 | 30 | 20 | 3 | rectangular |
| | 3-13 | R-13 | 205 | 28 | 16 | 2.8 | rectangular |
| | 3-14 | R-14 | 200 | 30 | 20 | 3 | rectangular |
| | 3-15 | R-15 | 205 | 32 | 18 | 2.9 | rectangular |
| | 3-16 | R-16 | 200 | 30 | 16 | 2.7 | rectangular |
| | 3-17 | R-17 | 200 | 30 | 18 | 2.8 | rectangular |
| | 3-18 | R-18 | 205 | 28 | 18 | 2.9 | rectangular |
| | 3-19 | R-19 | 200 | 28 | 16 | 3 | rectangular |
| | 3-20 | R-20 | 205 | 28 | 18 | 2.7 | rectangular |
| | 3-21 | R-21 | 200 | 30 | 16 | 2.9 | rectangular |
| | 3-22 | R-22 | 205 | 28 | 18 | 2.9 | rectangular |
| | 3-23 | R-23 | 205 | 28 | 20 | 2.9 | rectangular |
| | 3-24 | R-24 | 205 | 30 | 16 | 2.7 | rectangular |
| | 3-25 | R-25 | 210 | 30 | 18 | 2.8 | rectangular |
| | 3-26 | R-26 | 205 | 28 | 18 | 2.9 | rectangular |
| | 3-27 | R-27 | 205 | 32 | 18 | 2.8 | rectangular |
| | 3-28 | R-28 | 200 | 30 | 20 | 2.7 | rectangular |
| | 3-29 | R-29 | 205 | 28 | 16 | 2.9 | rectangular |
| | 3-30 | R-30 | 205 | 28 | 16 | 2.8 | rectangular |
| | 3-31 | R-31 | 200 | 30 | 16 | 2.9 | rectangular |
| | 3-32 | R-32 | 200 | 30 | 18 | 3 | rectangular |
| | 3-33 | R-33 | 205 | 28 | 20 | 2.8 | rectangular |
| | 3-34 | R-34 | 200 | 28 | 18 | 2.7 | rectangular |
| | 3-35 | R-35 | 210 | 30 | 18 | 2.9 | rectangular |
| | 3-36 | R-36 | 205 | 30 | 20 | 2.8 | rectangular |
| | 3-37 | R-37 | 200 | 28 | 16 | 3.1 | rectangular |
| | 3-38 | R-38 | 205 | 32 | 16 | 2.8 | rectangular |
| | 3-39 | R-39 | 205 | 28 | 16 | 2.9 | rectangular |
| | 3-40 | R-40 | 200 | 30 | 20 | 2.8 | rectangular |
| | 3-41 | R-41 | 205 | 30 | 20 | 3 | rectangular |
| | 3-42 | R-42 | 200 | 28 | 18 | 2.9 | rectangular |

**[Table 5]**

| | | Resist composition | Optimal exposure dose (µC/cm²) | L/S resolution (nm) | Maximum IS resolution (nm) | LER (nm) | Pattern profile |
|---|---|---|---|---|---|---|---|
| Comparative Example | 2-1 | CR-1 | 200 | 34 | 22 | 3.4 | rounded top |
| | 2-2 | CR-2 | 210 | 34 | 24 | 3.3 | footing |
| | 2-3 | CR-3 | 200 | 36 | 22 | 3.4 | rounded top |
| | 2-4 | CR-4 | 205 | 34 | 22 | 3.3 | footing |
| | 2-5 | CR-5 | 200 | 34 | 22 | 3.3 | rounded top |
| | 2-6 | CR-6 | 210 | 36 | 24 | 3.4 | rounded top |
| | 2-7 | CR-7 | 205 | 36 | 24 | 3.4 | rounded top |
| | 2-8 | CR-8 | 200 | 34 | 22 | 3.4 | rounded top |
| | 2-9 | CR-9 | 210 | 36 | 22 | 3.3 | rounded top |
| | 2-10 | CR-10 | 205 | 36 | 24 | 3.4 | footing |
| | 2-11 | CR-11 | 200 | 36 | 22 | 3.5 | rectangular |
| | 2-12 | CR-12 | 205 | 34 | 24 | 3.6 | rounded top |
| | 2-13 | CR-13 | 210 | 34 | 24 | 3.6 | rounded top |
| | 2-14 | CR-14 | 200 | 36 | 22 | 3.4 | rounded top |
| | 2-15 | CR-15 | 210 | 36 | 24 | 3.6 | rounded top |
| | 2-16 | CR-16 | 200 | 34 | 22 | 3.5 | footing |
| | 2-17 | CR-17 | 205 | 34 | 24 | 3.3 | rounded top |
| | 2-18 | CR-18 | 200 | 36 | 22 | 3.4 | rounded top |
| | 2-19 | CR-19 | 210 | 36 | 24 | 3.5 | footing |
| | 2-20 | CR-20 | 205 | 36 | 22 | 3.4 | rounded top |
| | 2-21 | CR-21 | 200 | 34 | 22 | 3.6 | footing |
| | 2-22 | CR-22 | 210 | 34 | 24 | 3.3 | rounded top |
| | 2-23 | CR-23 | 200 | 36 | 24 | 3.6 | footing |
| | 2-24 | CR-24 | 200 | 34 | 22 | 3.4 | rounded top |
| | 2-25 | CR-25 | 210 | 38 | 24 | 3.4 | rounded top |
| | 2-26 | CR-26 | 210 | 36 | 22 | 3.6 | rounded top |
| | 2-27 | CR-27 | 200 | 34 | 22 | 3.4 | footing |
| | 2-28 | CR-28 | 205 | 34 | 22 | 3.3 | rounded top |
| | 2-29 | CR-29 | 200 | 36 | 22 | 3.5 | rounded top |
| | 2-30 | CR-30 | 210 | 34 | 22 | 3.6 | rounded top |
| | 2-31 | CR-31 | 205 | 36 | 24 | 3.6 | footing |
| | 2-32 | CR-32 | 200 | 34 | 22 | 3.5 | rounded top |
| | 2-33 | CR-33 | 210 | 34 | 24 | 3.6 | rounded top |
| | 2-34 | CR-34 | 205 | 34 | 22 | 3.4 | rounded top |
| | 2-35 | CR-35 | 200 | 36 | 24 | 3.3 | footing |

The chemically amplified positive resist compositions (R-1 to R-42) within the scope of the invention exhibit satisfactory resolution, LER and pattern rectangularity. Comparative resist compositions (CR-1 to CR-35) are insufficient in optimization of acid diffusion and show poor resolution, LER and pattern rectangularity.

The resist pattern forming process using the chemically amplified positive resist compositions within the scope of the invention is effective in photolithography for the fabrication of semiconductor devices and the processing of photomask blanks of transmission and reflection types.

## Claims

1. A iodonium salt having the formula (A): 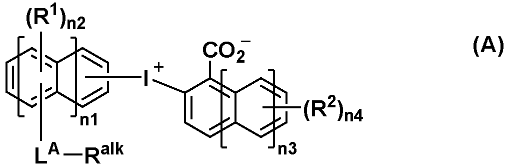 wherein n1 is 0 or 1, n2 is 0, 1, 2, 3 or 4, n3 is 0 or 1, n4 is 0, 1, 2, 3 or 4,
L^{A} is a single bond, ether bond, ester bond, sulfonic ester bond, carbonate bond or carbamate bond,
R^{alk} is a C₆-C₁₈ alkyl group or C₄-C₁₈ fluorinated alkyl group; when R^{alk} is a C₆-C₁₈ alkyl group, the alkyl group has at least one straight chain structure of 6 or more carbon atoms; when R^{alk} is a C₄-C₁₈ fluorinated alkyl group, the fluorinated alkyl group has at least two groups selected from -CF₂- and -CF₃; the alkyl or fluorinated alkyl group may have some -CH₂- replaced by an ether bond or carbonyl group and may contain a cyclic structure selected from cyclopentane, cyclohexane, adamantane, norbornyl and benzene rings at its end or in a carbon-carbon bond,
R¹ is halogen, nitro, cyano, hydroxy, a C₁-C₂₀ hydrocarbyl group which may contain a heteroatom, C₁-C₂₀ hydrocarbyloxy group which may contain a heteroatom, or C₁-C₂₀ hydrocarbylthio group which may contain a heteroatom; when n2 is 2, 3 or 4, a plurality of R¹ may be identical or different and may bond together to form a ring with the carbon atoms to which they are attached,
R² is halogen, nitro, cyano, hydroxy, a C₁-C₂₀ hydrocarbyl group which may contain a heteroatom, C₁-C₂₀ hydrocarbyloxy group which may contain a heteroatom, or C₁-C₂₀ hydrocarbylthio group which may contain a heteroatom; when n4 is 2, 3 or 4, a plurality of R² may be identical or different and may bond together to form a ring with the carbon atoms to which they are attached,
two aromatic rings attached to I⁺ in the formula may bond together to form a ring with the iodine atom to which they are attached.

2. The iodonium salt of claim 1, having the formula (A1): 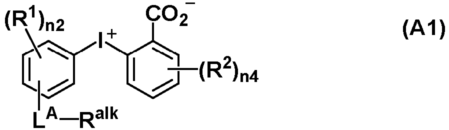 wherein n2, n4, L^{A}, R^{alk}, R¹ and R² are as defined above.

3. A quencher in the form of the iodonium salt of claim 1 or 2.

4. A chemically amplified positive resist composition comprising the quencher of claim 3.

5. The resist composition of claim 4, further comprising a base polymer containing a polymer adapted to be decomposed under the action of acid to increase its solubility in alkaline developer,
wherein the polymer comprises repeat units having the formula (B2-1) or (B2-2): 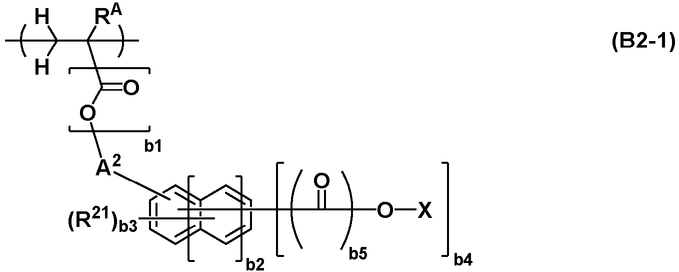
wherein R^{A} is hydrogen, fluorine, methyl or trifluoromethyl,
b1 is 0 or 1, b2 is 0, 1 or 2, b3 is an integer meeting 0 ≤ b3 ≤ 5+2(b2)-b4, b4 is 1, 2 or 3, b5 is 0 or 1,
R²¹ is halogen, an optionally halogenated C₁-C₆ saturated hydrocarbyl group, optionally halogenated C₁-C₆ saturated hydrocarbyloxy group, or optionally halogenated C₂-C₈ saturated hydrocarbylcarbonyloxy group,
A² is a single bond or C₁-C₁₀ saturated hydrocarbylene group in which some -CH₂-may be replaced by -O-,
X is an acid labile group when b4=1, and X is hydrogen or an acid labile group, at least one being an acid labile group, when b4=2 or 3. 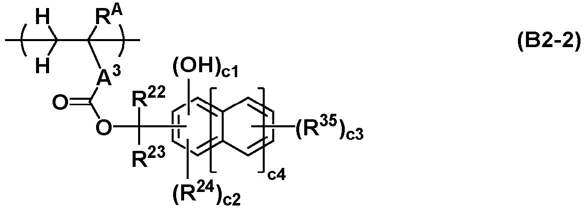
wherein c1 is 0, 1 or 2, c2 is 0, 1 or 2, c3 is 0, 1, 2, 3, 4 or 5, c4 is 0, 1 or 2,
R^{A} is hydrogen, fluorine, methyl or trifluoromethyl,
R²² and R²³ are each independently a C₁-C₁₀ hydrocarbyl group which may contain a heteroatom, R²² and R²³ may bond together to form a ring with the carbon atom to which they are attached,
R²⁴ is each independently fluorine, a C₁-C₅ fluorinated alkyl group or C₁-C₅ fluorinated alkoxy group,
R²⁵ is each independently a C₁-C₁₀ hydrocarbyl group which may contain a heteroatom, and
A³ is a single bond, phenylene, naphthylene or *-C(=O)-O-A³¹-, A³¹ is a C₁-C₂₀ aliphatic hydrocarbylene group which may contain a hydroxy moiety, ether bond, ester bond or lactone ring, a phenylene group or a naphthylene group, * designates a point of attachment to the carbon atom in the backbone.

6. The resist composition of claim 5 wherein the polymer comprises repeat units having the formula (B1): 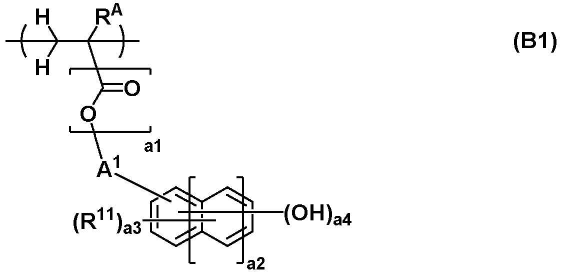 wherein a1 is 0 or 1, a2 is 0, 1 or 2, a3 is an integer meeting 0 ≤ a3 ≤ 5+2(a2)-a4, a4 is 1, 2 or 3,
R^{A} is hydrogen, fluorine, methyl or trifluoromethyl,
R¹¹ is halogen, nitro group, carboxy group, an optionally halogenated C₁-C₆ saturated hydrocarbyl group, optionally halogenated C₁-C₆ saturated hydrocarbyloxy group, or optionally halogenated C₂-C₈ saturated hydrocarbylcarbonyloxy group, and
A¹ is a single bond or C₁-C₁₀ saturated hydrocarbylene group in which some -CH₂-may be replaced by -O-.

7. The resist composition of claim 5 or 6 wherein the polymer comprises repeat units of at least one type selected from repeat units having the formula (B3), repeat units having the formula (B4), and repeat units having the formula (B5): 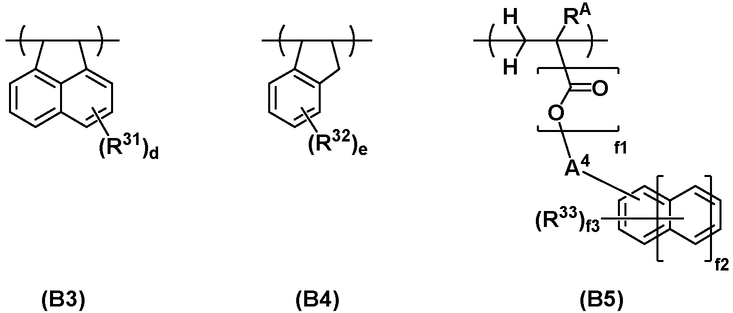 wherein d is 0, 1, 2, 3, 4, 5 or 6, e is 0, 1, 2, 3 or 4, f1 is 0 or 1, f2 is 0, 1 or 2, f3 is 0, 1, 2, 3, 4 or 5,
R^{A} is hydrogen, fluorine, methyl or trifluoromethyl,
R³¹ and R³² are each independently hydroxy, halogen, an optionally halogenated C₁-C₆ saturated hydrocarbyl group, optionally halogenated C₁-C₆ saturated hydrocarbyloxy group, or optionally halogenated C₂-C₈ saturated hydrocarbylcarbonyloxy group,
R³³ is a C₁-C₂₀ saturated hydrocarbyl group, C₁-C₂₀ saturated hydrocarbyloxy group, C₂-C₂₀ saturated hydrocarbylcarbonyloxy group, C₂-C₂₀ saturated hydrocarbyloxyhydrocarbyl group, C₂-C₂₀ saturated hydrocarbylthiohydrocarbyl group, halogen, nitro, or cyano, R³³ may also be hydroxy when f2=1 or 2, and
A⁴ is a single bond or C₁-C₁₀ saturated hydrocarbylene group in which some -CH₂-may be replaced by -O-.

8. The resist composition of any one of claims 5 to 7 wherein the polymer comprises repeat units of at least one type selected from repeat units having the formula (B6), repeat units having the formula (B7), repeat units having the formula (B8), repeat units having the formula (B9), and repeat units having the formula (B10): 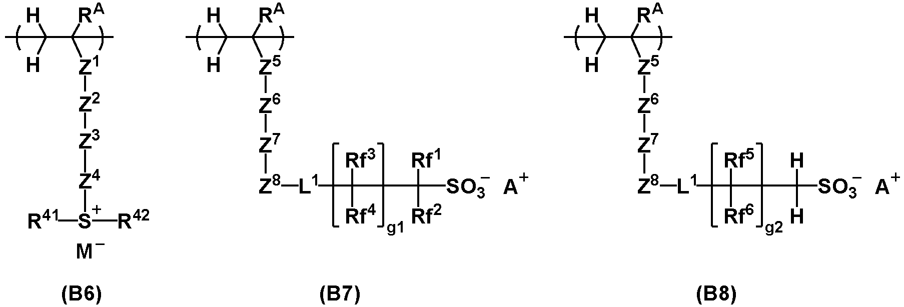 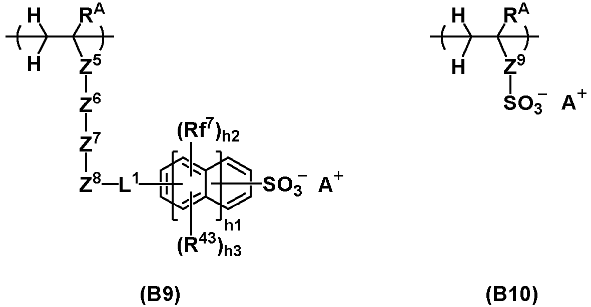 wherein g1 and g2 are each independently 0, 1, 2 or 3, h1 is 0 or 1, h2 is 0, 1, 2, 3 or 4, h3 is 0, 1, 2, 3 or 4, with the proviso that when h1=0, 0 ≤ h2+h3 ≤ 4, and when h1=1, 0 ≤ h2+h3 ≤ 6,
R^{A} is hydrogen, fluorine, methyl or trifluoromethyl,
Z¹ is a single bond or an optionally substituted phenylene group,
Z² is a single bond, **-C(=O)-O-Z²¹-, **-C(=O)-NH-Z²¹-, or **-O-Z²¹-, Z²¹ is a C₁-₆ aliphatic hydrocarbylene group, phenylene group or a divalent group obtained by combining the foregoing, which may contain halogen, carbonyl moiety, ester bond, ether bond or hydroxy moiety,
Z³ is a single bond, ether bond, ester bond, amide bond, sulfonate ester bond, sulfonamide bond, carbonate bond or carbamate bond,
Z⁴ is a single bond, or a C₁-C₆ aliphatic hydrocarbylene group, phenylene group or a divalent group obtained by combining the foregoing, which may contain halogen, carbonyl moiety, ester bond, ether bond or hydroxy moiety,
Z⁵ is each independently a single bond, an optionally substituted phenylene, naphthylene, or *-C(=O)-O-Z⁵¹-, Z⁵¹ is a C₁-C₁₀ aliphatic hydrocarbylene group which may contain halogen, hydroxy moiety, ether bond, ester bond or lactone ring, or phenylene or naphthylene group,
Z⁶ is a single bond, ether bond, ester bond, amide bond, sulfonate ester bond, sulfonamide bond, carbonate bond or carbamate bond,
Z⁷ is each independently a single bond, ***-Z⁷¹-C(=O)-O-, ***-C(=O)-NH-Z⁷¹-, or ***-O-Z⁷¹-, Z⁷¹ is a C₁-C₂₀ hydrocarbylene group which may contain a heteroatom,
Z⁸ is each independently a single bond, ****-Z⁸¹-C(=O)-O-, ****-C(=O)-NH-Z⁸¹-, or ****-O-Z⁸¹-, Z⁸¹ is a C₁-C₂₀ hydrocarbylene group which may contain a heteroatom,
Z⁹ is a single bond, methylene, ethylene, phenylene, fluorinated phenylene, trifluoromethyl-substituted phenylene, *-C(=O)-O-Z⁹¹-, *-C(=O)-N(H)-Z⁹¹-, or *-O-Z⁹¹-, Z⁹¹ is a C₁-C₆ aliphatic hydrocarbylene group, phenylene group, fluorinated phenylene group or trifluoromethyl-substituted phenylene group, which may contain a carbonyl moiety, ester bond, ether bond or hydroxy moiety,
* is a point of attachment to the carbon atom in the backbone, ** is a point of attachment to Z¹, *** is a point of attachment to Z⁶, **** is a point of attachment to Z⁷, L¹ is a single bond, ether bond, ester bond, carbonyl group, sulfonate ester bond, sulfonamide bond, carbonate bond or carbamate bond,
Rf¹ and Rf² are each independently fluorine or a C₁-C₆ fluorinated saturated hydrocarbyl group,
Rf³ and Rf⁴ are each independently hydrogen, fluorine, or a C₁-C₆ fluorinated saturated hydrocarbyl group,
Rf⁵ and Rf⁶ are each independently hydrogen, fluorine, or a C₁-C₆ fluorinated saturated hydrocarbyl group, excluding that all Rf⁵ and Rf⁶ are hydrogen at the same time,
Rf⁷ is fluorine, a C₁-C₆ fluorinated alkyl group, C₁-C₆ fluorinated alkoxy group, or C₁-C₆ fluorinated alkylthio group,
R⁴¹ and R⁴² are each independently a C₁-C₂₀ hydrocarbyl group which may contain a heteroatom, R⁴¹ and R⁴² may bond together to form a ring with the sulfur atom to which they are attached,
R⁴³ is halogen exclusive of fluorine, or a C₁-C₂₀ hydrocarbyl group which may contain a heteroatom; when h3 is 2, 3 or 4, a plurality of R⁴³ may be identical or different and may bond together to form a ring with the carbon atoms to which they are attached,
M⁻ is a non-nucleophilic counter ion, and
A⁺ is an onium cation.

9. The resist composition of any one of claims 5 to 8 wherein repeat units having an aromatic ring structure account for at least 60 mol% of the overall repeat units of the polymer in the base polymer.

10. The resist composition of any one of claims 4 to 9, further comprising (C) an organic solvent.

11. The resist composition of any one of claims 4 to 10, further comprising a photoacid generator.

12. The resist composition of any one of claims 4 to 11, further comprising (E) a fluorinated polymer comprising repeat units of at least one type selected from repeat units having the formula (E1), repeat units having the formula (E2), repeat units having the formula (E3) and repeat units having the formula (E4) and optionally repeat units of at least one type selected from repeat units having the formula (E5) and repeat units having the formula (E6): 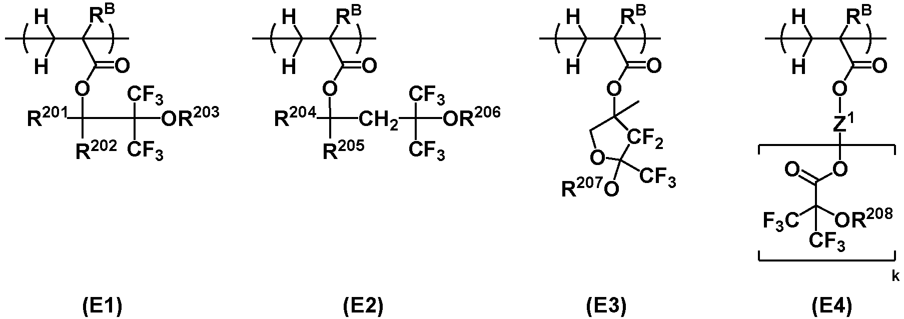 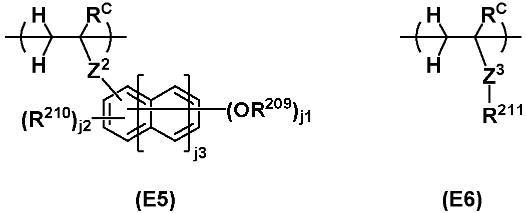 wherein j1 is 1, 2 or 3, j2 is an integer meeting 0 ≤ j2 ≤ 5+2(j3)-j 1, j3 is 0 or 1, k is 1, 2 or 3,
R^{B} is each independently hydrogen, fluorine, methyl or trifluoromethyl,
R^{C} is each independently hydrogen or methyl,
R²⁰¹, R²⁰², R²⁰⁴ and R²⁰⁵ are each independently hydrogen or a C₁-C₁₀ saturated hydrocarbyl group,
R²⁰³, R²⁰⁶, R²⁰⁷ and R²⁰⁸ are each independently hydrogen, a C₁-C₁₅ hydrocarbyl group, C₁-C₁₅ fluorinated hydrocarbyl group, or acid labile group, and when R²⁰³, R²⁰⁶, R²⁰⁷ and R²⁰¹ each are a hydrocarbyl or fluorinated hydrocarbyl group, an ether bond or carbonyl moiety may intervene in a carbon-carbon bond,
R²⁰⁹ is hydrogen or a C₁-C₅ straight or branched hydrocarbyl group in which a heteroatom-containing moiety may intervene in a carbon-carbon bond,
R²¹⁰ is a C₁-C₅ straight or branched hydrocarbyl group in which a heteroatom-containing moiety may intervene in a carbon-carbon bond,
R²¹¹ is a C₁-C₂₀ saturated hydrocarbyl group in which at least one hydrogen is substituted by fluorine, and in which some constituent -CH₂- may be replaced by an ester bond or ether bond,
Z¹ is a C₁-C₂₀ (k+1)-valent hydrocarbon group or C₁-C₂₀ (k+1)-valent fluorinated hydrocarbon group,
Z² is a single bond, *-C(=O)-O- or *-C(=O)-NH-, * designates a point of attachment to the carbon atom in the backbone,
Z³ is a single bond, -O-, *-C(=O)=O-Z³¹-Z³²- or *-C(=O)-NH-Z³¹-Z³²-, Z³¹ is a single bond or C₁-C₁₀ saturated hydrocarbylene group, Z³² is a single bond, ester bond, ether bond, or sulfonamide bond, and * designates a point of attachment to the carbon atom in the backbone.

13. A resist pattern forming process comprising the steps of:
applying the chemically amplified positive resist composition of any one of claims 4 to 12 onto a substrate to form a resist film thereon,
exposing the resist film patternwise to high-energy radiation, and
developing the exposed resist film in an alkaline developer.

14. The process of claim 13 wherein the high-energy radiation is EUV of wavelength 3 to 15 nm or EB.

15. The process of claim 13 or 14 wherein the substrate has the outermost surface of a chromium-containing material.
